(19) Europäisches Patentamt
European Patent Office
Office européen des brevets

(11) **EP 4 585 599 A1**

(12) **EUROPEAN PATENT APPLICATION**
published in accordance with Art. 153(4) EPC

(43) Date of publication:
**16.07.2025 Bulletin 2025/29**

(21) Application number: **23881982.5**

(22) Date of filing: **27.10.2023**

(51) International Patent Classification (IPC):
***C07D 513/04*** *(2006.01)* ***C07D 513/14*** *(2006.01)*
***C07D 417/14*** *(2006.01)* ***C07D 417/04*** *(2006.01)*
***C07D 401/04*** *(2006.01)* ***A61K 31/429*** *(2006.01)*
***A61K 31/428*** *(2006.01)* ***A61P 35/00*** *(2006.01)*

(52) Cooperative Patent Classification (CPC):
**A61K 31/428; A61K 31/429; A61K 31/444; A61K 31/4545; A61K 31/496; A61K 31/497; A61K 31/501; A61K 31/506; A61K 31/519; A61K 31/55; A61K 31/553; A61K 31/675; A61P 35/00; C07D 213/80; C07D 277/82;** (Cont.)

(86) International application number:
**PCT/CN2023/127292**

(87) International publication number:
**WO 2024/088407 (02.05.2024 Gazette 2024/18)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority:
**28.10.2022 CN 202211339071**
**12.01.2023 CN 202310041397**
**31.01.2023 CN 202310048633**
**20.02.2023 CN 202310138583**
**31.05.2023 CN 202310634655**
**11.07.2023 CN 202310847363**
**18.10.2023 CN 202311351811**

(71) Applicant: **Hangzhou Synrx Therapeutics Biomedical Technology Co., Ltd.**
**Hangzhou, Zhejiang 311121 (CN)**

(72) Inventors:
- **HE, Hu**
  **Hangzhou, Zhejiang 311121 (CN)**
- **GE, Chongxun**
  **Hangzhou, Zhejiang 311121 (CN)**
- **LIU, Song**
  **Hangzhou, Zhejiang 311121 (CN)**
- **SHI, Song**
  **Hangzhou, Zhejiang 311121 (CN)**
- **ZHANG, Yilan**
  **Hangzhou, Zhejiang 311121 (CN)**
- **JIANG, Kai**
  **Hangzhou, Zhejiang 311121 (CN)**
- **WU, Liu**
  **Hangzhou, Zhejiang 311121 (CN)**
- **XU, Xiaokang**
  **Hangzhou, Zhejiang 311121 (CN)**

(74) Representative: **Lavoix**
**Bayerstraße 83**
**80335 München (DE)**

# (54) NITROGEN-CONTAINING FUSED RING COMPOUND, INTERMEDIATE THEREOF, PREPARATION METHOD THEREFOR AND USE THEREOF

(57) Disclosed are a nitrogen-containing fused ring compound as shown informula (I), an intermediate thereof, a preparation method therefor and the use thereof. The compound has a DNA polymerase θ inhibitory activity, and can be used for treatingdiseases mediated by the DNA polymerase θ, such as tumors.

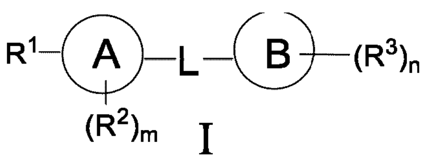

I

EP 4 585 599 A1

(52) Cooperative Patent Classification (CPC): (Cont.)
**C07D 401/04; C07D 417/04; C07D 417/14; C07D 471/04; C07D 487/04; C07D 491/048; C07D 491/056; C07D 495/04; C07D 498/04; C07D 513/04; C07D 513/14; C07D 513/22; C07F 7/18; C07F 9/6541; C07F 9/6558; C07F 9/6561; C07F 9/6568**

## Description

This application claims the priorities to Chinese Patent Application No. 2022113390715 filed on October 28, 2022, Chinese Patent Application No. 2023100413978 filed on January 12, 2023, Chinese Patent Application No. 2023100486339 filed on January 31, 2023, Chinese Patent Application No. 2023101385833 filed on February 20, 2023, Chinese Patent Application No. 2023106346553 filed on May 31, 2023, Chinese Patent Application No. 2023108473638 filed on July 11, 2023, and Chinese Patent Application No. 2023113518111 filed on October 18, 2023. The present application makes reference to the full text of the Chinese Patent Application above.

## FIELD OF THE INTVENTION

The present invention relates to a fused-ring nitrogen-containing compound, intermediates thereof, preparation method therefor, and application thereof.

## BACKGROUND OF THE INVENTION

DNA damage is an important mechanism for many chemotherapy drugs to exert anti-tumor effects. DNA double-strand breaks (DSBs) are one of the most common types of damage in cells, which can be directly caused by ionizing radiation or induced by ultraviolet light, reactive oxygen species (ROS), or other mutagens, such as common chemotherapy drugs like cisplatin, 5-FU, and etoposide. Unrepaired DNA damage may lead to functional blockage such as cell transcription and replication, inducing apoptosis or necrosis, and clearance by immune cells. In tumor cells, there is an efficient damage repair pathway or alternative repair pathway. It is one of the important strategies for cancer treatment to target the key proteins of the DNA damage repair pathway, allowing for the constant accumulation of unrepaired DNA damage to eventually lead to cell death.

The DNA DSBs are typically repaired in three ways, including non-homologous end joining (NHEJ), homologous recombination (HR), and alternative non-homologous end joining (Alt-NHEJ, also known as microhomology-mediated end joining (MMEJ) or theta-mediated end joining (TMEJ)). Here, the HR repair pathway only occurs in phases G2 and S to complete error-free repair in the presence of sister chromatids. In mammals, more than 90% of DSB damage is repaired by the NHEJ pathway. This repair is error-prone, resulting in the deletion mutation of < 30 bp, or the insertion mutation of < 5 bp, or the micro-homologous sequence of < 2 bp. Recent studies have shown that, in the case of HR and/or NHEJ deficiency, cells are highly dependent on the third way, i.e., MMEJ, to repair the broken DNAs. Inhibition of MMEJ leads to apoptosis. MMEJ, which is also an error-prone repair pathway, relies on DNA polymerase theta to link theta mediated end joining (TMEJ) sequences, thereby completing the repair of DNA double-strand breaks.

DNA polymerase theta (Pol theta or Pol θ), a key protein in the MMEJ repair pathway, is a unique multifunctional polymerase composed of an N-terminus helicase domain, a central domain, and a C-terminus polymerase domain. Basic studies have found that the polymerase domain is essential for DNA extension of DSB damage repair sites, and the helicase domain and the central domain play a key role in the recognition and binding of Pol θ to a substrate. Pol θ can eliminate the interaction between DNA and damage repair complexes (such as competition with RAD51 for binding to single-stranded DNA) and inhibit the HR repair pathway. In addition, the helicase domain of Pol θ is also involved in DNA replication blockage, and its function deficiency leads to increased replication pressure and apoptosis of tumor cells.

Pol θ is not expressed or expressed at a low level in normal tissue cells, but is highly expressed in a variety of tumors such as lung cancer, breast cancer, HR-deficient ovarian cancer, gastric cancer, and colon cancer, and it is associated with worse prognosis. Pol θ is overexpressed in more than 70% of breast cancers. These facts indicate that Pol θ may play an important role in these cancers and is a potential tumor-specific target.

Studies on knockdown or knockout of Pol θ in tumor cells have found that Pol θ deficiency can sensitize these cells to radioactive rays, induce DSB production, enhance replication fork instability, and make tumor cells sensitive to genotoxic agents, which may enhance the effect of radiotherapy and chemotherapy, and Pol θ is a potential drug target. The studies have also found that Pol θ shows a combined lethal effect with HR deficiency, and its small molecule inhibitors can kill HR-deficient tumor cells in vitro and in vivo. In particular, in tumors resistant to PARP inhibitors (Olaparib, etc.) with HR deficiency reverse mutations, Pol θ inhibitors can sensitize cells to PARPi again, providing valuable therapeutic opportunities. In addition, given that Pol θ is a key protein in the MMEJ pathway, its functional impairment also leads to intensified genomic instability in cancer cells and increased somatic mutations, facilitating the production of tumor neoantigens. In addition, Pol θ has been reported to be involved in cGAS-STING-mediated immune activation. Hence, targeting Pol θ also has the potential to enhance immunotherapy.

In summary, Pol θ is a target with great potential for cancer treatment, and in the treatment of lung cancer, breast cancer, HR-deficient ovarian cancer, gastric cancer, colon cancer, prostatic cancer, pancreatic cancer and other tumors, it is promising to design a Polθ protein-targeting ATPase activity inhibitor to inhibit intracellular MMEJ, and administrate it alone or in combined trial with the additional chemotherapy, radiotherapy, antibody therapy, immunotherapy, etc., to

achieve the purpose of killing tumor cells.

## SUMMARY OF THE INVENTION

The technical problem to be solved by the present invention is to overcome the defect of relatively unitary structure of polymerase theta inhibitor compounds in the prior art. To this end, the present invention provides a type of fused-ring nitrogen-containing compounds, intermediates thereof, a preparation method therefor, and application thereof. The ATPase activity and protein inhibition effect of these compounds are greatly improved compared with the prior art.

The present invention solves the above technical problems by means of the following technical solutions.

The present invention provides a type of compounds of formula I, a pharmaceutically acceptable salt thereof, or an isotopic compound thereof.

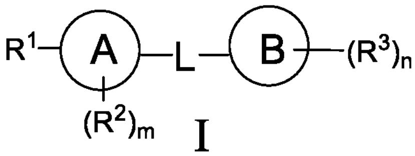

I

wherein:

m is 0, 1, 2, or 3; n is 1, 2, or 3;

L is

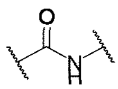, 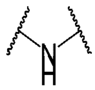, 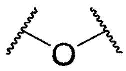, -CO- or 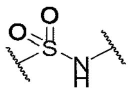;

ring A is a 6-12 membered aromatic ring or a 5-12 membered heteroaromatic ring; a heteroatom in the 5-12 membered heteroaromatic ring is one or more of N, O, and S, and the number of heteroatom(s) is 1, 2, or 3;

$R^1$ is 6-12 membered aryl unsubstituted or substituted by one or more $R^{1-1}$, or 5-12 membered heteroaryl unsubstituted or substituted by one or more $R^{1-2}$; a heteroatom in the 5-12 membered heteroaryl is one or more of N, O, and S, and the number of heteroatom(s) is 1, 2, or 3;

each of $R^{1-1}$ and $R^{1-2}$ is independently selected from deuterium, halogen, cyano, amino, hydroxy, $C_1$-$C_6$ alkyl unsubstituted or substituted by one or more $R^{1-1-1}$, $C_1$-$C_6$ alkoxy unsubstituted or substituted by one or more $R^{1-2-2}$, $C_2$-$C_6$ alkenyl unsubstituted or substituted by one or more $R^{1-1-3}$, $C_2$-$C_6$ alkynyl unsubstituted or substituted by one or more $R^{1-1-4}$, 3-6 membered cycloalkyl unsubstituted or substituted by one or more $R^{1-1-5}$, 4-6 membered heterocyclyl unsubstituted or substituted by one or more $R^{1-1-6}$, COOR$^{1-1-7}$, -C(O)R$^{1-1-8}$, -C(O)NR$^{1-1-9}$R$^{1-1-9}$,

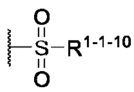, or 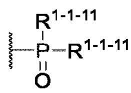;

a heteroatom in the 4-6 membered heterocyclyl is one or more selected from N, O, and S, and the number of heteroatom(s) is 1, 2, or 3;

each of $R^{1-1-1}$, $R^{1-1-2}$, $R^{1-1-3}$, $R^{1-1-4}$, $R^{1-1-5}$ and $R^{1-1-6}$ is independently selected from deuterium, hydroxy, cyano, or halogen;

each of $R^{1-1-7}$, $R^{1-1-8}$ and $R^{1-1-9}$ is independently selected from hydrogen or $C_1$-$C_6$ alkyl;

each of $R^{1-1-10}$ and $R^{1-1-11}$ is independently selected from hydroxy or $C_1$-$C_6$ alkyl;

each of $R^2$ is independently selected from

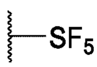,

deuterium, halogen, cyano, amino, hydroxy, $C_1$-$C_6$ alkyl unsubstituted or substituted by one or more $R^{2-1}$, $C_1$-$C_6$ alkoxy unsubstituted or substituted by one or more $R^{2-2}$, $C_2$-$C_6$ alkenyl unsubstituted or substituted by one or more $R^{2-3}$, $C_2$-$C_6$ alkynyl unsubstituted or substituted by one or more $R^{2-4}$, 3-6 membered cycloalkyl unsubstituted or substituted by one or more $R^{2-5}$ or 4-6 membered heterocyclyl unsubstituted or substituted by one or more $R^{2-6}$; a

heteroatom in the 4-6 membered heterocyclyl is one or more selected from N, O, and S, and the number of heteroatom(s) number is 1, 2, or 3;

each of $R^{2-1}$, $R^{2-2}$, $R^{2-3}$, $R^{2-4}$, $R^{2-5}$ and $R^{2-6}$ is independently selected from deuterium, hydroxy, cyano or halogen;

ring B is a 5-20 membered fused heteroaromatic ring unsubstituted or substituted by one or more $R^{b-1}$; a heteroatom in the 5-20 membered fused-ring heteroaromatic ring is one or more selected from N, O, and S, and the number of heteroatom(s) is 1, 2, 3, 4, 5, 6, 7 or 8, a single heteroaromatic ring in the 5-20 membered fused-ring heteroaromatic ring is a 5-7 membered heteroaromatic ring, and the number of rings in the 5-20 membered fused-ring heteroaromatic ring is 2, 3 or 4;

each of $R^{b-1}$ is independently selected from deuterium, oxo(=O), halogen, cyano, hydroxy, $C_1$-$C_6$ alkyl unsubstituted or substituted by one or more $R^{b-1-1}$, $C_1$-$C_6$ alkoxy unsubstituted or substituted by one or more $R^{b-1-2}$, -$NR^{b-1-3}R^{b-1-3}$, -C(O)$NR^{b-1-4}R^{b-1-4}$, -$COOR^{b-1-5}$,

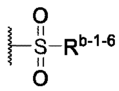 , or 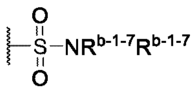 ;

each of $R^{b-1-1}$ and $R^{b-1-2}$ is independently selected from hydroxy, halogen or -$NR^{b-1-1-1}R^{b-1-1-1}$;

each of $R^{b-1-3}$, $R^{b-1-4}$, $R^{b-1-5}$ and $R^{b-1-7}$ is independently selected from hydrogen or $C_1$-$C_6$ alkyl;

each $R^{b-1-6}$ is independently selected from hydroxy or $C_1$-$C_6$ alkyl;

each $R^{b-1-1-1}$ is independently selected from hydrogen, $C_1$-$C_6$ alkyl or -C(O)$R^{b-1-1-1-1}$;

$R^{b-1-1-1-1}$ is $C_1$-$C_6$ alkyl;

$R^3$ is hydrogen,

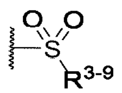

, 3-12 membered cycloalkyl unsubstituted or substituted by one or more $R^{3-1}$, 4-12 membered heterocyclyl unsubstituted or substituted by one or more $R^{3-2}$, 4-12 membered cycloalkenyl unsubstituted or substituted by one or more $R^{3-3}$, 4-12 membered heterocycloalkenyl unsubstituted or substituted by one or more $R^{3-4}$, 6-10 membered aryl unsubstituted or substituted by one or more $R^{3-5}$, 5-10 membered heteroaryl unsubstituted or substituted by one or more $R^{3-6}$, -C(O)$R^4$, -NH$(CH_2)_pR^5$, -O$(CH_2)_qR^6$, $C_1$-$C_6$ alkyl unsubstituted or substituted by one or more $R^{3-7}$, or -$OR^7$ unsubstituted or substituted by one or more $R^{3-8}$; a heteroatom in each of the 4-12 membered heterocyclyl, 4-12 membered heterocycloalkenyl and 5-10 membered heteroaryl is one or more selected from N, O, and S, and the number of heteroatom(s) is 1, 2, 3 or 4;

p is 0, 1, 2, or 3;

q is 1, 2, or 3;

each of $R^{3-1}$, $R^{3-2}$, $R^{3-3}$, $R^{3-4}$, $R^{3-7}$ and $R^{3-8}$ is independently selected from deuterium, hydroxy, halogen, cyano, amino, -NHC(O)$R^{3-2-1}$, $C_1$-$C_6$ alkyl unsubstituted or substituted by one or more $R^{3-2-2}$, $C_1$-$C_6$ alkoxy, $C_2$-$C_6$ alkenyl, $C_2$-$C_6$ alkynyl, oxo(=O), -C(O)$NH_2$, 3-6 membered cycloalkyl unsubstituted or substituted by one or more $R^{3-2-3}$, 4-6 membered heterocyclyl, -$S(O)_2R^{3-4-1}$, -$NR^{3-4-2}R^{3-4-2}$,

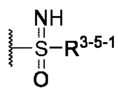 or 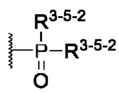

; a heteroatom in the 4-6 membered heterocyclyl is one or more selected from N, O, and S, and the number of heteroatom(s) is 1 or 2;

each of $R^{3-5}$ and $R^{3-6}$ is independently selected from deuterium,

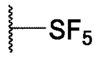 ,

hydroxy, halogen, cyano, amino, -NHC(O)$R^{3-2-1}$, $C_1$-$C_6$ alkyl unsubstituted or substituted by one or more $R^{3-2-2}$, $C_2$-$C_6$ alkenyl, $C_2$-$C_6$ alkynyl, -C(O)$NH_2$, 3-6 membered cycloalkyl unsubstituted or substituted by one or more $R^{3-2-3}$, 4-6 membered heterocyclyl unsubstituted or substituted by one or more $R^{3-7}$, -$S(O)_2R^{3-4-1}$, -$NR^{3-4-2}R^{3-4-2}$,

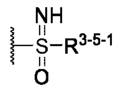

or

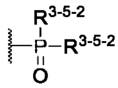 ;

a heteroatom in the 4-6 membered heterocyclyl is one or more selected from N, O, and S, and the number of heteroatom(s) is 1 or 2;
each of $R^{3-2-1}$ and $R^{3-4-2}$ is independently selected from hydrogen, $-S(O)_2R^{3-4-1}$, 3-6 membered cycloalkyl unsubstituted or substituted by one or more $R^{3-2-1-1}$, or $C_1$-$C_6$ alkyl;
each of $R^{3-2-1-1}$ is independently $C_1$-$C_6$ alkyl;
each of $R^{3-2-3}$ and $R^{3-5-1}$ is independently selected from hydroxy or $C_1$-$C_6$ alkyl;
each of $R^{3-5-2}$ is independently selected from hydroxy, $C_1$-$C_6$ alkyl or 1-10 membered heteroalkyl, or two $R^{3-5-2}$s form 4-10 membered heterocyclyl; a heteroatom, except for phosphorus, in the 1-10 membered heteroalkyl further comprises one or more selected from N, O, and S, and the number of heteroatom(s) is 1, 2, 3 or 4; a heteroatom, except for phosphorus, in the 4-10 membered heterocyclyl further comprises one or more of N, O, and S, and the number of heteroatom(s) is 1, 2, 3 or 4;
each of $R^{3-6-1}$ is independently $C_1$-$C_6$ alkyl;
each of $R^{3-2-2}$ is independently selected from deuterium, hydroxy or halogen (for example, each of $R^{3-2-2}$ is independently selected from hydroxy or halogen);
$R^{3-4-1}$ is hydroxy, amino or $C_1$-$C_6$ alkyl;
$R^{3-9}$ is $C_1$-$C_6$ alkyl;
$R^4$ is 4-6 membered heterocyclyl unsubstituted or substituted by one or more $R^{4-1}$; a heteroatom in the 4-6 membered heterocyclyl is one or more selected from N, O, and S, and the number of heteroatom(s)is 1 or 2;
each of $R^{4-1}$ is independently hydroxy;
$R^5$ is 3-6 membered cycloalkyl unsubstituted or substituted by one or more $R^{5-1}$, $-S(O)_2R^{5-2}$, or 4-6 membered heterocyclyl unsubstituted or substituted by one or more $R^{5-3}$; a heteroatom in the 4-6 membered heterocyclyl is one or more selected from N, O, and S, and the number of heteroatom(s) is 1 or 2;
$R^{5-2}$ is $C_1$-$C_6$ alkyl;
each of $R^{5-3}$ is independently $-S(O)_2R^{5-3-1}$; each of $R^{5-3-1}$ is independently $C_1$-$C_6$ alkyl;
$R^6$ is 3-6 membered cycloalkyl or 4-6 membered heterocyclyl unsubstituted or substituted by one or more $R^{6-1}$; a heteroatom in the 4-6 membered heterocyclyl is selected from one or more of N, O, and S, and the number of heteroatom(s) is 1 or 2;
each of $R^{5-1}$ and $R^{6-1}$ is independently hydroxy; and
$R^7$ is 3-6 membered cycloalkyl.

In a preferred embodiment, in the compound of formula I, the pharmaceutically acceptable salt thereof or the isotopic compound thereof, some groups can be defined as follows, and other groups can be described as in any of the above embodiments (hereinafter referred to as "in a preferred embodiment"):

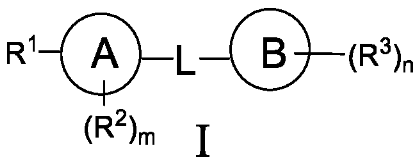
I .

wherein:

m is 0, 1, 2, or 3; n is 1, 2, or 3;
L is

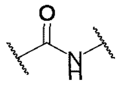, 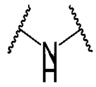, 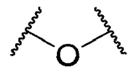, -CO- or 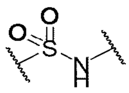;

ring A is a 6-12 membered aromatic ring or a 5-12 membered heteroaromatic ring; a heteroatom in the 5-12 membered heteroaromatic ring is one or more selected from N, O, and S, and the number of heteroatom(s) is 1, 2, or 3;

$R^1$ is 6-12 membered aryl unsubstituted or substituted by one or more $R^{1-1}$ or 5-12 membered heteroaryl unsubstituted or substituted by one or more $R^{1-2}$; a heteroatom in the 5-12 membered heteroaryl is one or more selected from N, O, and S, and the number of heteroatom(s) is 1, 2, or 3;

each of $R^{1-1}$ and $R^{1-2}$ is independently selected from deuterium, halogen, cyano, amino, hydroxy, $C_1$-$C_6$ alkyl unsubstituted or substituted by one or more $R^{1-1-1}$, $C_1$-$C_6$ alkoxy unsubstituted or substituted by one or more $R^{1-2-2}$, $C_2$-$C_6$ alkenyl unsubstituted or substituted by one or more $R^{1-1-3}$, $C_2$-$C_6$ alkynyl unsubstituted or substituted by one or more $R^{1-1-4}$, 3-6 membered cycloalkyl unsubstituted or substituted by one or more $R^{1-1-5}$, 4-6 membered heterocyclyl unsubstituted or substituted by one or more $R^{1-1-6}$, $COOR^{1-1-7}$, $-C(O)R^{1-1-8}$, $-C(O)NR^{1-1-9}R^{1-1-9}$,

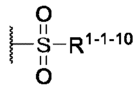, or 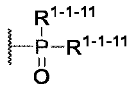;

a heteroatom in the 4-6 membered heterocyclyl is one or more selected from N, O, and S, and the number of heteroatom(s) is 1, 2, or 3;

each of $R^{1-1-1}$, $R^{1-1-2}$, $R^{1-1-3}$, $R^{1-1-4}$, $R^{1-1-5}$ and $R^{1-1-6}$ is independently selected from deuterium, hydroxy, cyano, or halogen;

each of $R^{1-1-7}$, $R^{1-1-8}$ and $R^{1-1-9}$ is independently selected from hydrogen or $C_1$-$C_6$ alkyl;

each of $R^{1-1-10}$ and $R^{1-1-11}$ is independently selected from hydroxy or $C_1$-$C_6$ alkyl;

each of $R^2$ is independently selected from deuterium, halogen, cyano, amino, hydroxy, $C_1$-$C_6$ alkyl unsubstituted or substituted by one or more $R^{2-1}$, $C_1$-$C_6$ alkoxy unsubstituted or substituted by one or more $R^{2-2}$, $C_2$-$C_6$ alkenyl unsubstituted or substituted by one or more $R^{2-3}$, $C_2$-$C_6$ alkynyl unsubstituted or substituted by one or more $R^{2-4}$, 3-6 membered cycloalkyl unsubstituted or substituted by one or more $R^{2-5}$ or 4-6 membered heterocyclyl unsubstituted or substituted by one or more $R^{2-6}$; a heteroatom in the 4-6 membered heterocyclyl is one or more selected from N, O, and S, and the number of heteroatom(s) is 1, 2, or 3;

each of $R^{2-1}$, $R^{2-2}$, $R^{2-3}$, $R^{2-4}$, $R^{2-5}$ and $R^{2-6}$ is independently selected from deuterium, hydroxy, cyano or halogen;

ring B is a 5-20 membered fused-ring heteroaromatic ring unsubstituted or substituted by one or more $R^{b-1}$; a heteroatom in the 5-20 membered fused-ring heteroaromatic ring is one or more selected from N, O, and S, and the number of heteroatom(s) is 1, 2, 3, 4, 5, or 6, a single heteroaromatic ring in the 5-20 membered fused-ring heteroaromatic ring is a 5-7 membered heteroaromatic ring, and the number of rings in the 5-20 membered fused-ring heteroaromatic ring is 2, 3 or 4;

each of $R^{b-1}$ is independently selected from deuterium, oxo(=O), halogen, cyano, hydroxy, $C_1$-$C_6$ alkyl unsubstituted or substituted by one or more $R^{b-1-1}$, $C_1$-$C_6$ alkoxy unsubstituted or substituted by one or more $R^{b-1-2}$, $-NR^{b-1-3}R^{b-1-3}$, $-C(O)NR^{b-1-4}R^{b-1-4}$, $-COOR^{b-1-5}$,

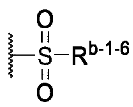 or 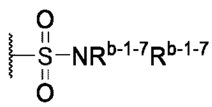;

each of $R^{b-1-1}$ and $R^{b-1-2}$ is independently selected from hydroxy, halogen or $-NR^{b-1-1-1}R^{b-1-1-1}$;

each of $R^{b-1-3}$, $R^{b-1-4}$, $R^{b-1-5}$ and $R^{b-1-7}$ is independently selected from hydrogen or $C_1$-$C_6$ alkyl;

each $R^{b-1-6}$ is independently selected from hydroxy or $C_1$-$C_6$ alkyl;

each $R^{b-1-1-1}$ is independently selected from hydrogen, $C_1$-$C_6$ alkyl or $-C(O)R^{b-1-1-1-1}$;

$R^{b-1-1-1-1}$ is $C_1$-$C_6$ alkyl;

$R^3$ is hydrogen, 3-12 membered cycloalkyl unsubstituted or substituted by one or more $R^{3-1}$, 4-12 membered heterocyclyl unsubstituted or substituted by one or more $R^{3-2}$, 4-12 membered cycloalkenyl unsubstituted or substituted by one or more $R^{3-3}$, 4-12 membered heterocycloalkenyl unsubstituted or substituted by one or more $R^{3-4}$, 6-10 membered aryl unsubstituted or substituted by one or more $R^{3-5}$, 5-10 membered heteroaryl unsubstituted or substituted by one or more $R^{3-6}$, $-C(O)R^4$, $-NH(CH_2)_pR^5$, $-O(CH_2)_qR^6$, $C_1$-$C_6$ alkyl unsubstituted or substituted by one or more $R^{3-7}$, or $-OR^7$ unsubstituted or substituted by one or more $R^{3-8}$; a heteroatom in each of the 4-12

membered heterocyclyl, 4-12 membered heterocycloalkenyl and 5-10 membered heteroaryl is one or more selected from N, O, and S, and the number of heteroatom(s) is 1, 2, 3 or 4;
p is 0, 1, 2, or 3;
q is 1, 2, or 3;
each of $R^{3-1}$, $R^{3-2}$, $R^{3-3}$, $R^{3-4}$, $R^{3-7}$ and $R^{3-8}$ is independently selected from deuterium, hydroxy, halogen, cyano, $-NHC(O)R^{3-2-1}$, $C_1$-$C_6$ alkyl unsubstituted or substituted by one or more $R^{3-2-2}$, $C_2$-$C_6$ alkenyl, $C_2$-$C_6$ alkynyl, oxo(=O), $-C(O)NH_2$, 3-6 membered cycloalkyl unsubstituted or substituted by one or more $R^{3-2-3}$, 4-6 membered heterocyclyl, $-S(O)_2R^{3-4-1}$, $-NR^{3-4-2}R^{3-4-2}$,

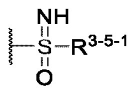 or 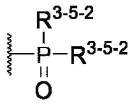 ;

a heteroatom in the 4-6 membered heterocyclyl is one or more selected from N, O, and S, and the number of heteroatom(s) is 1 or 2;
each of $R^{3-5}$ and $R^{3-6}$ is independently selected from deuterium, hydroxy, halogen, cyano, amino, $-NHC(O)R^{3-2-1}$, $C_1$-$C_6$ alkyl unsubstituted or substituted by one or more $R^{3-2-2}$, $C_2$-$C_6$ alkenyl, $C_2$-$C_6$ alkynyl, $-C(O)NH_2$, 3-6 membered cycloalkyl unsubstituted or substituted by one or more $R^{3-2-3}$, 4-6 membered heterocyclyl, $-S(O)_2R^{3-4-1}$, $-NR^{3-4-2}R^{3-4-2}$,

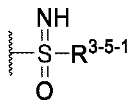 or 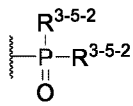 ;

a heteroatom in the 4-6 membered heterocyclyl is selected from one or more of N, O, and S, and the number of heteroatom(s) is 1 or 2;
each of $R^{3-2-1}$ and $R^{3-4-2}$ is independently selected from hydrogen or $C_1$-$C_6$ alkyl;
each of $R^{3-2-3}$, $R^{3-5-1}$ and $R^{3-5-2}$ is independently selected from hydroxy or $C_1$-$C_6$ alkyl;
each of $R^{3-2-2}$ is independently selected from hydroxy or halogen;
$R^{3-4-1}$ is hydroxy, amino or $C_1$-$C_6$ alkyl;
$R^4$ is 4-6 membered heterocyclyl; a heteroatom in the 4-6 membered heterocyclyl is one or more selected from N, O, and S, and the number of heteroatom(s) is 1 or 2;
$R^5$ is 3-6 membered cycloalkyl unsubstituted or substituted by one or more $R^{5-1}$;
$R^6$ is 3-6 membered cycloalkyl unsubstituted or substituted by one or more $R^{6-1}$
each of $R^{5-1}$ and $R^{6-1}$ is independently hydroxy; and
$R^7$ is 3-6 membered cycloalkyl.

In a preferred embodiment, the compound of formula I' is the compound of formula I',

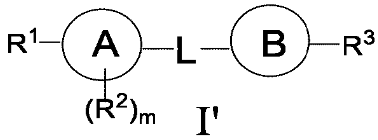

I'

In a preferred embodiment, in the ring A, the 5-12 membered heteroaromatic ring can be a 5-10 membered heteroaromatic ring, and a heteroatom in the 5-10 membered heteroaromatic ring can be selected from one or two of N, O and S; the 5-10 membered heteroaromatic ring is preferably a 5-6 membered heteroaromatic ring, a 5 membered-fused-6 membered heteroaromatic ring or a 6 membered-fused-6 membered heteroaromatic ring;

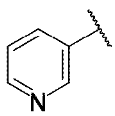 , 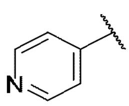 , 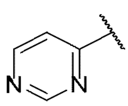 , 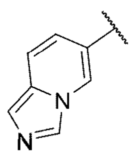 , 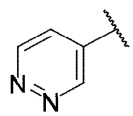 , 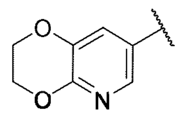 ,

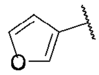, 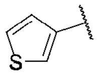, 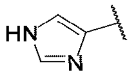, 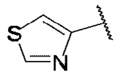, 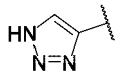 or 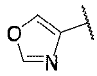

is preferred, for example,

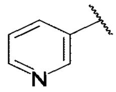,

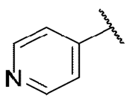, 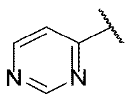 or 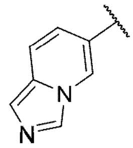.

In a preferred embodiment, in $R^1$, the 6-12 membered aryl can be 6-10 membered aryl, or can be phenyl or naphthyl, for example, phenyl.

In a preferred embodiment, in $R^1$, the 5-12 membered heteroaryl can be 5-10 membered heteroaryl, a heteroatom in the 5-10 membered heteroaryl can be N and/or O, and the number of heteroatom(s) can be 1 or 2; the 5-12 membered heteroaryl is preferably 5-6 membered heteroaryl or 5 membered-fused-6 membered heteroaryl, more preferably

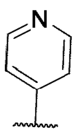, 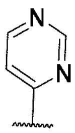, 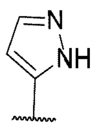, 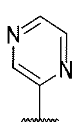, 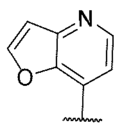 or 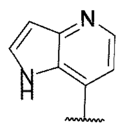,

for example,

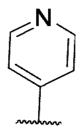.

In a preferred embodiment, in each $R^{1-1}$, the halogen can be fluorine, chlorine, bromine or iodine, for example, fluorine.

In a preferred embodiment, in each $R^{1-1}$, the $C_1$-$C_6$ alkyl can be $C_1$-$C_4$ alkyl, or can be methyl, ethyl, n-propyl, isopropyl, n-butyl, isobutyl, pr-butyl, sec-butyl or tert-butyl.

In a preferred embodiment, in each $R^{1-1}$, the $C_1$-$C_6$ alkoxy can be $C_1$-$C_4$ alkoxy, or can be methoxy, ethoxy, n-propoxy, iso-propoxy, n-butoxy, isobutoxy, pr-butoxy, sec-butoxy or tert-butoxy, for example, methoxy.

In a preferred embodiment, in each $R^{1-2}$, the halogen can be fluorine, chlorine, bromine or iodine, for example, fluorine or chlorine.

In a preferred embodiment, in each $R^{1-2-2}$, the $C_1$-$C_6$ alkyl can be $C_1$-$C_4$ alkyl, or can be methyl, ethyl, n-propyl, isopropyl, n-butyl, isobutyl, pr-butyl, sec-butyl or tert-butyl, preferably methyl.

In a preferred embodiment, in each $R^{1-2-2}$, the $C_1$-$C_6$ alkoxy can be $C_1$-$C_4$ alkoxy, or can be methoxy, ethoxy, n-propoxy, iso-propoxy, n-butoxy, isobutoxy, pr-butoxy, sec-butoxy or tert-butoxy, for example, methoxy.

In a preferred embodiment, in each $R^{1-1-1}$, the halogen can be fluorine, chlorine, bromine or iodine, preferably fluorine.

In a preferred embodiment, in each $R^{1-1-2}$, the halogen can be fluorine, chlorine, bromine or iodine, for example, fluorine.

In a preferred embodiment, in each $R^2$, the $C_1$-$C_6$ alkyl can be $C_1$-$C_4$ alkyl, or can be methyl, ethyl, n-propyl, isopropyl, n-butyl, isobutyl, pr-butyl, sec-butyl or tert-butyl, for example, methyl.

In a preferred embodiment, in each $R^2$, the halogen can be fluorine, chlorine, bromine or iodine, for example, fluorine.

In a preferred embodiment, in the ring B, the number of heteroatom(s) in the 5-20 membered fused-ring

heteroaromatic ring can be 1, 2, 3 or 4; the 5-20 membered fused-ring heteroaromatic ring is preferably 5 membered-fused-5 membered heteroaromatic ring, a 5 membered-fused-6 membered heteroaromatic ring, a 6 membered-fused-6 membered heteroaromatic ring, a 5 membered-fused-6 membered-fused-6 membered-fused-6 membered heteroaromatic ring or a 5 membered-fused-6 membered-fused-7 membered-fused-6 membered heteroaromatic ring, more preferably

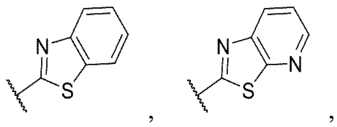

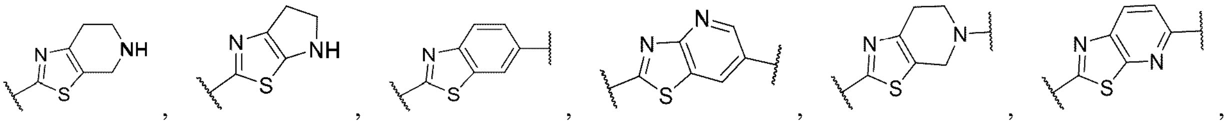

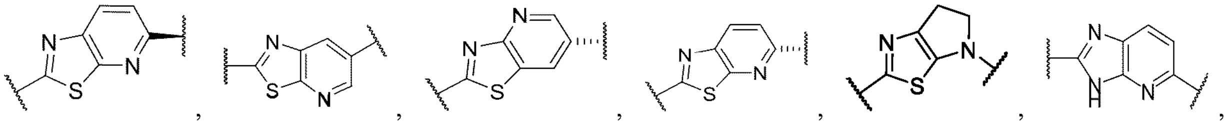

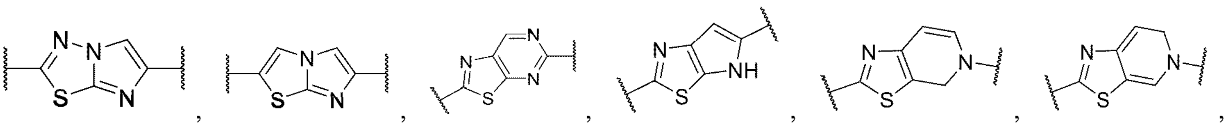

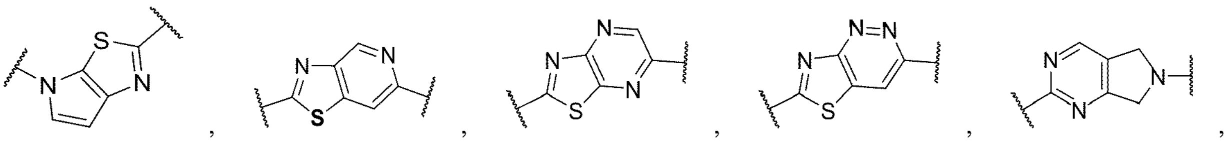

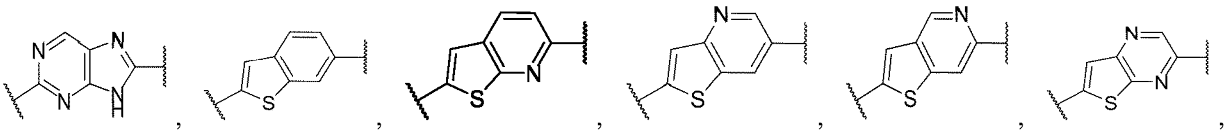

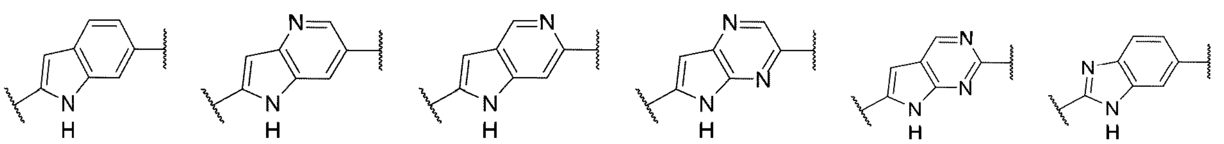

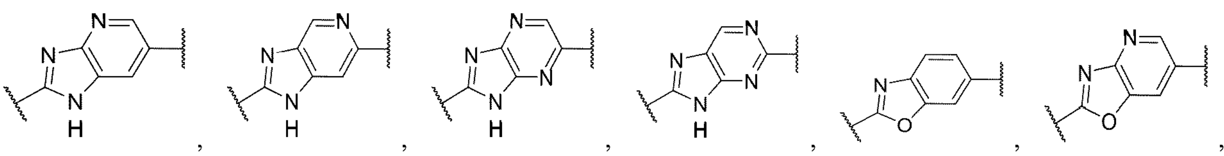

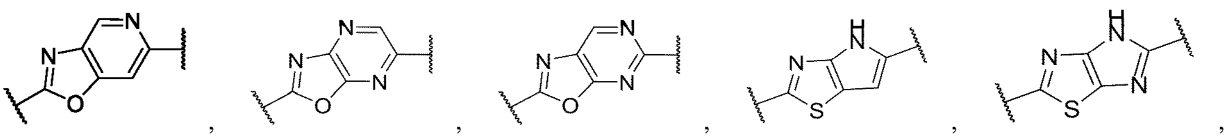

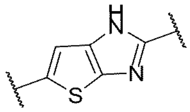 , 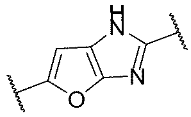 , 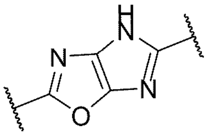 , 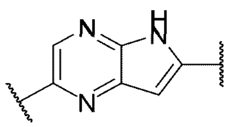 , 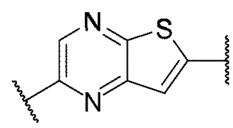 ,

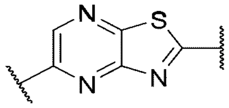 , 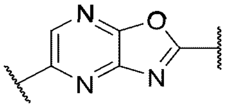 , 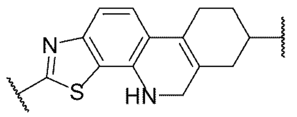 , 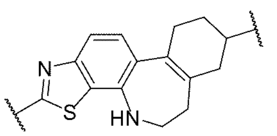 ,

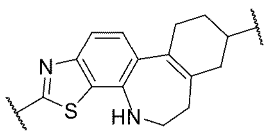 , 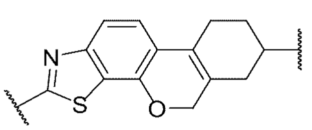 , 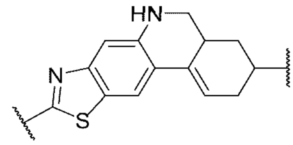 , 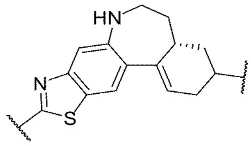 ,

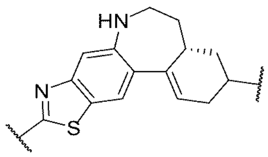 , 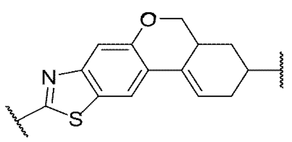 , 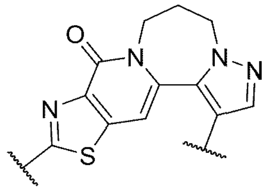 , 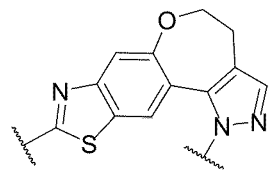 ,

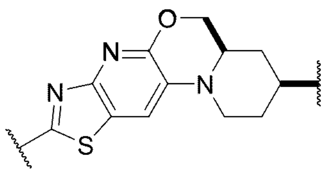 , 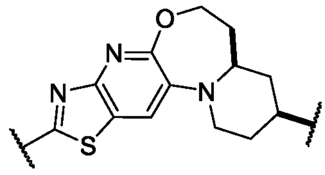 , 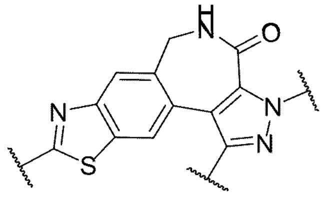 ,

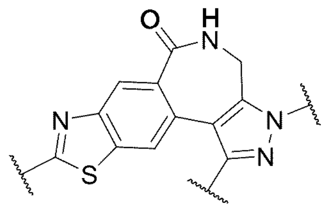 , 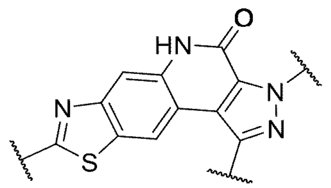 , 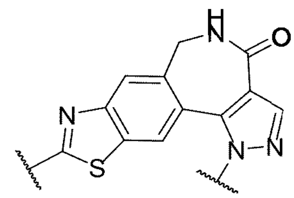

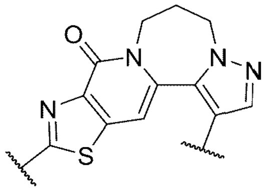 , 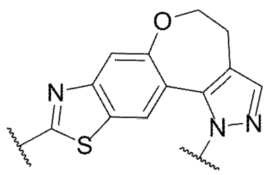 , 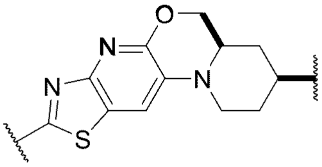 ,

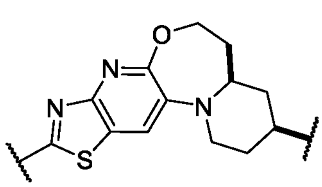 , 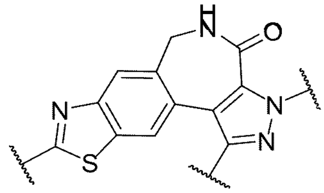 , 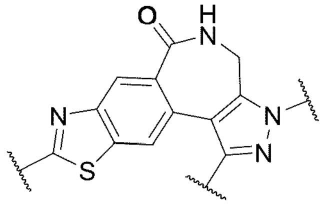 ,

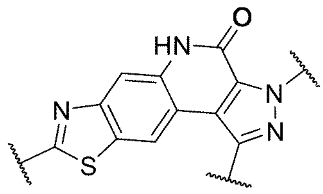, 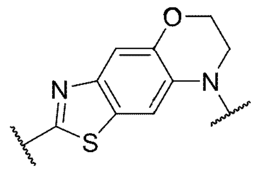, 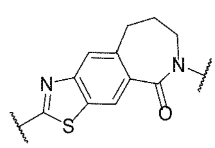, 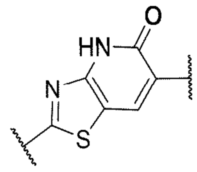,

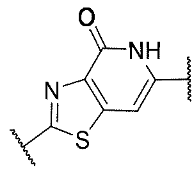 or 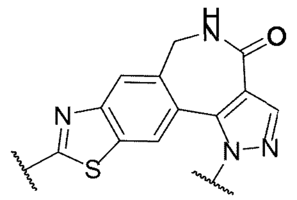,

for example,

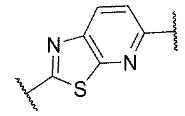, 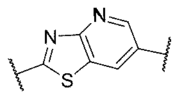,

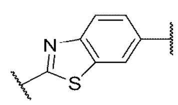, 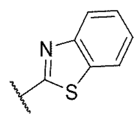, 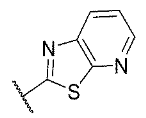, 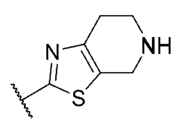, 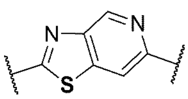 or 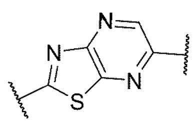

(for another example,

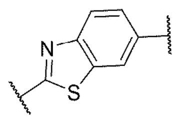, 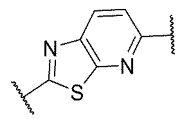, 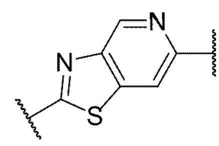, 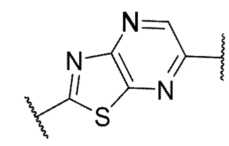,

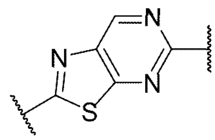, 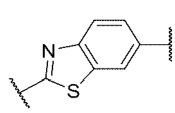, 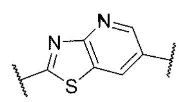, 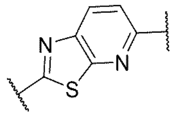

).

In a preferred embodiment, in each $R^{b-1}$, the halogen can be fluorine, chlorine, bromine or iodine, for example, fluorine.

In a preferred embodiment, in each $R^{b-1}$, the $C_1$-$C_6$ alkyl can be $C_1$-$C_4$ alkyl, or can be methyl, ethyl, n-propyl, isopropyl, n-butyl, isobutyl, pr-butyl, sec-butyl or tert-butyl, for example, methyl.

In a preferred embodiment, in each $R^{b-1}$, the $C_1$-$C_6$ alkoxy can be $C_1$-$C_4$ alkoxy, or can be methoxy, ethoxy, n-propoxy, iso-propoxy, n-butoxy, isobutoxy, pr-butoxy, sec-butoxy or tert-butoxy, for example, methoxy or ethoxy.

In a preferred embodiment, in each $R^{b-1-1}$, the halogen can be fluorine, chlorine, bromine or iodine.

In a preferred embodiment, in each $R^{1-2-2}$, the halogen can be fluorine, chlorine, bromine or iodine.

In a preferred embodiment, in each $R^{b-1-2-1}$, the $C_1$-$C_6$ alkyl can be $C_1$-$C_4$ alkyl, or can be methyl, ethyl, n-propyl, isopropyl, n-butyl, isobutyl, pr-butyl, sec-butyl or tert-butyl, preferably methyl.

In a preferred embodiment, in each $R^{b-1-4}$ and $R^{b-1-5}$, the $C_1$-$C_6$ alkyl can be $C_1$-$C_4$ alkyl, or can be methyl, ethyl, n-propyl, isopropyl, n-butyl, isobutyl, pr-butyl, sec-butyl or tert-butyl, preferably methyl.

In a preferred embodiment, in $R^3$, the 3-12 membered cycloalkyl can be 3-6 membered cycloalkyl, or can be cyclopropyl, cyclobutyl, cyclopentyl or cyclohexyl, preferably cyclobutyl or cyclohexyl, for example, cyclohexyl.

In a preferred embodiment, in $R^3$, the 3-12 membered cycloalkyl is 7-10 membered cycloalkyl, a ring in the 7-10

membered cycloalkyl can be a monocyclic, spiro, fused or bridged ring, the number of rings in the spiro, fused or bridged ring can be 2, the spiro ring can be 4 membered-spiro-4 membered cycloalkyl, 4 membered-spiro-5 membered cycloalkyl or 4 membered-spiro-6 membered cycloalkyl, the fused ring can be 5 membered-fused-5 membered cycloalkyl or 5 membered-fused-6 membered cycloalkyl, and the bridged ring can be 5 membered-bridged-7 membered cycloalkyl, and

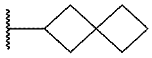, 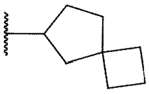,

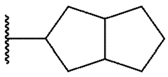, 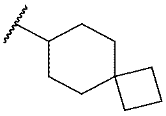, 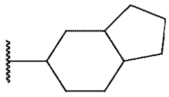 or 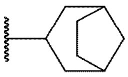

is preferred.

In a preferred embodiment, in $R^3$, a ring in the 4-12 membered heterocyclyl can be a monocyclic, spiro, fused or bridged ring, the number of rings in the spiro, fused or bridged ring can be 2, the spiro ring can be a 3 membered-spiro-5 membered heterocyclyl, 3 membered-spiro-6 membered heterocyclyl, 4 membered-spiro-5 membered heterocyclyl, 4 membered-spiro-6 membered heterocyclyl, 4 membered-spiro-4 membered heterocyclyl, 4 membered-spiro-7 membered heterocyclyl, 5 membered-spiro-4 membered heterocyclyl, 5 membered-spiro-7 membered heterocyclyl, 5 membered-spiro-5 membered heterocyclyl or 5 membered-spiro-6 membered heterocyclyl, the fused ring can be 3 membered-fused-5 membered heterocyclyl, 4 membered-fused-5 membered heterocyclyl or 5 membered-fused-5 membered heterocyclyl, the bridged ring can be 5 membered-bridged-6 membered heterocyclyl, and the number of heteroatom(s) in the 4-12 membered heterocyclyl can be 1 or 2;

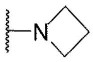,

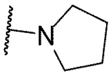, 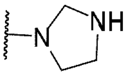, 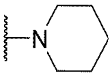, 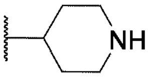, 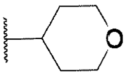, 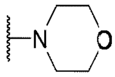, 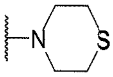, 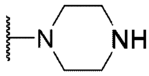,

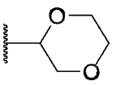, 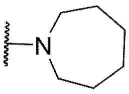, 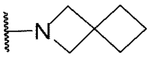, 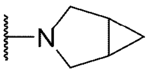, 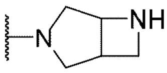, 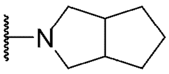, 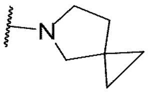,

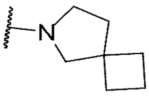, 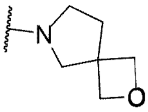, 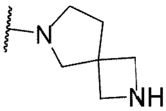, 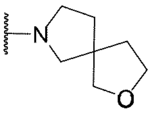, 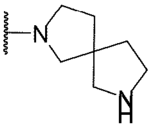, 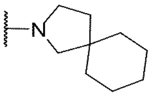, 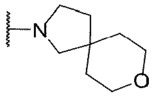,

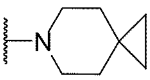, 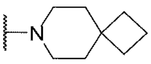, 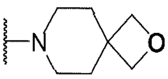, 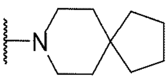, 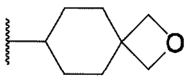, 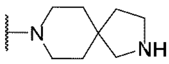,

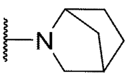 or 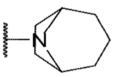

is preferred, for example,

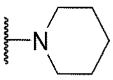, 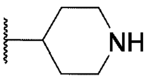, 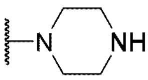 or

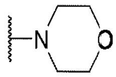,

for another example,

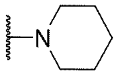.

In a preferred embodiment, in $R^3$, in the 4-12 membered heterocyclyl, the monocyclic ring is preferably 4-7 membered heterocyclyl, and the number of heteroatom(s) can be 1 or 2; the heteroatom is preferably N and/or O.

In a preferred embodiment, in $R^3$, a ring in the 4-10 membered cycloalkenyl can be a monocyclic, spiro, fused or bridged ring, the number of rings in the spiro, fused or bridged ring can be 2, the spiro ring can be 4 membered-spiro-4 membered cycloalkenyl, 4 membered-spiro-5 membered cycloalkenyl or 4 membered-spiro-6 membered cycloalkenyl, the fused ring can be 5 membered-fused-5 membered cycloalkenyl or 5 membered-fused-6 membered cycloalkenyl, the bridged ring can be 5 membered-bridged-7 membered cycloalkenyl, and

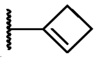, 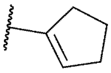, 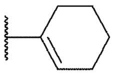, 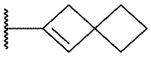, 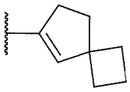, 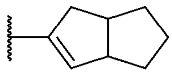,

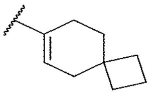, 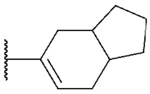 or 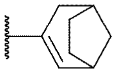

is preferred, for example,

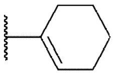.

In a preferred embodiment, in $R^3$, a ring in the 4-10 membered heterocycloalkenyl is a monocyclic or spiro ring, the number of rings in the spiro ring can be 2, the spiro ring can be 4 membered-spiro-4 membered heterocycloalkenyl, 4 membered-spiro-5 membered heterocycloalkenyl or 4 membered-spiro-6 membered heterocycloalkenyl, and the number of heteroatom(s) in the 4-10 membered heterocycloalkenyl can be 1;

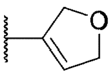, 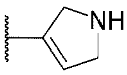, 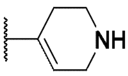, 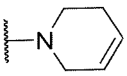, 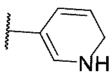, 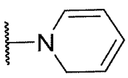, 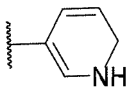, 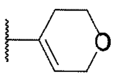,

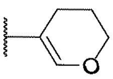, 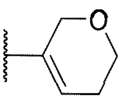, 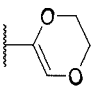, 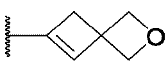, 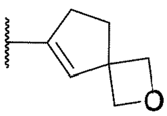, 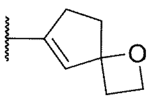, 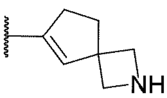,

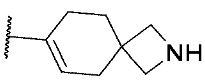 or 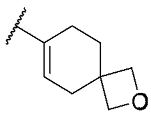

is preferred, for example,

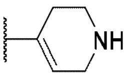 or 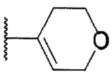.

In a preferred embodiment, in $R^3$, the 6-10 membered aryl can be aryl or naphthyl, preferably aryl.

In a preferred embodiment, in $R^3$, a heteroatom in the 5-10 membered heteroaryl can be N, and the number of heteroatom(s) can be 1, 2, or 3.

In a preferred embodiment, in $R^3$, the 5-10 membered heteroaryl can be monocyclic- or fused-ring heteroaryl, the number of rings in the fused ring can be 2, the fused ring can be 5 membered-fused-5 membered heteroaryl or 5 membered-fused-6 membered heteroaryl (preferably, both rings in the fused ring each have an aromatic ring), a heteroatom in the 5-10 membered heteroaryl can be N and/or O, and the number of heteroatom(s) can be 1;

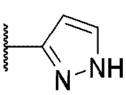, 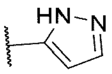, 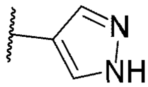, 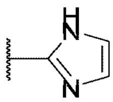, 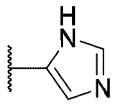, 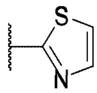,

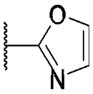, 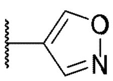, 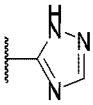, 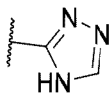, 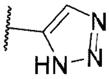, 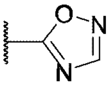, 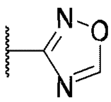, 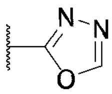, 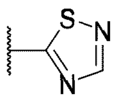,

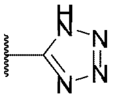, 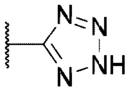, 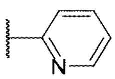, 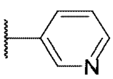, 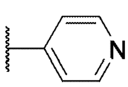, 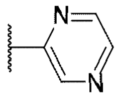, 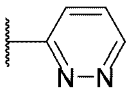, 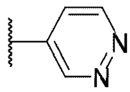,

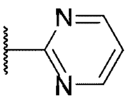, 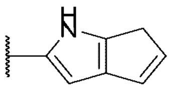, 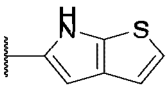, 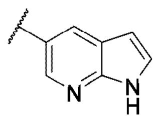, 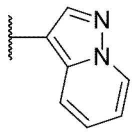, 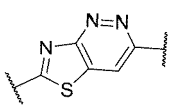, 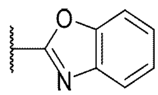,

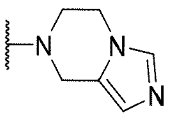, 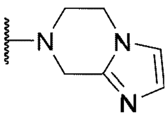, 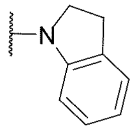, 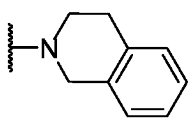 or 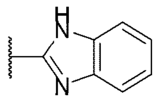,

for example,

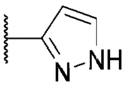, 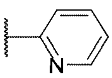

; for another example,

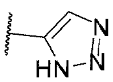.

In a preferred embodiment, in $R^3$, the $C_1$-$C_6$ alkyl can be $C_1$-$C_4$ alkyl, or can be methyl, ethyl, n-propyl, isopropyl, n-butyl, isobutyl, pr-butyl, sec-butyl or tert-butyl, preferably methyl or isopropyl, for example, methyl.

In a preferred embodiment, in each $R^{3-1}$, the halogen can be fluorine, chlorine, bromine or iodine, preferably fluorine.

In a preferred embodiment, in each $R^{3-1}$, the $C_1$-$C_6$ alkyl can be $C_1$-$C_4$ alkyl, or can be methyl, ethyl, n-propyl, isopropyl, n-butyl, isobutyl, pr-butyl, sec-butyl or tert-butyl, preferably methyl.

In a preferred embodiment, in each $R^{3-2}$, the halogen can be fluorine, chlorine, bromine or iodine, for example, fluorine.

In a preferred embodiment, in each $R^{3-2}$, the $C_1$-$C_6$ alkyl can be $C_1$-$C_4$ alkyl, or can be methyl, ethyl, n-propyl, isopropyl, n-butyl, isobutyl, pr-butyl, sec-butyl or tert-butyl, preferably methyl or isopropyl, for example, methyl.

In a preferred embodiment, in each $R^{3-2}$, the $C_2$-$C_6$ alkenyl can be $C_2$-$C_4$ alkenyl, or can be

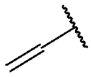,

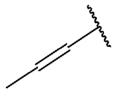, 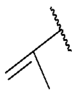, 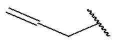, 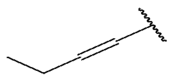, 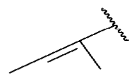, 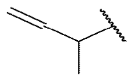 or 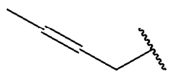,

preferably

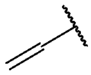.

In a preferred embodiment, in each $R^{3-2}$, the $C_2$-$C_6$ alkynyl can be $C_2$-$C_4$ alkynyl, or can be

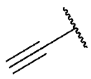,

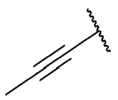, 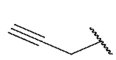, 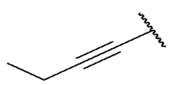, 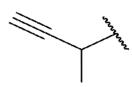 or 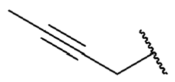,

preferably

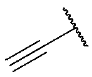.

In a preferred embodiment, in each $R^{3-2}$, the 3-6 membered cycloalkyl can be cyclopropyl, cyclobutyl, cyclopentyl or cyclohexyl, preferably cyclobutyl.

In a preferred embodiment, in each $R^{3-2}$, the number of heteroatoms in the 4-6 membered heterocyclyl can be 1 or 2, and the 4-6 membered heterocyclyl is preferably

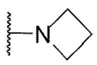, 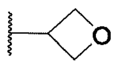, 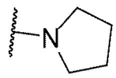, 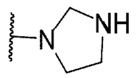,

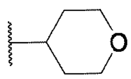, 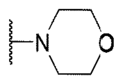, 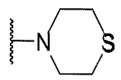, 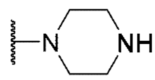, 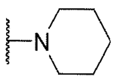 or 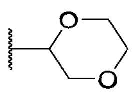.

In a preferred embodiment, in $R^{3-2-1}$, the $C_1$-$C_6$ alkyl can be $C_1$-$C_4$ alkyl, or can be methyl, ethyl, n-propyl, isopropyl, n-butyl, isobutyl, pr-butyl, sec-butyl or tert-butyl, preferably methyl.

In a preferred embodiment, in each $R^{3-2-2}$, the halogen can be fluorine, chlorine, bromine or iodine, preferably fluorine.

In a preferred embodiment, in each $R^{3-2-1-1}$, the $C_1$-$C_6$ alkyl can be $C_1$-$C_4$ alkyl, or can be methyl, ethyl, n-propyl, isopropyl, n-butyl, isobutyl, pr-butyl, sec-butyl or tert-butyl, preferably methyl.

In a preferred embodiment, in each $R^{3-2-3}$, the $C_1$-$C_6$ alkyl can be $C_1$-$C_4$ alkyl, or can be methyl, ethyl, n-propyl, isopropyl, n-butyl, isobutyl, pr-butyl, sec-butyl or tert-butyl, preferably methyl.

In a preferred embodiment, in each $R^{3-3}$, the halogen can be fluorine, chlorine, bromine or iodine, preferably fluorine.

In a preferred embodiment, in each $R^{3-4}$, the $C_1$-$C_6$ alkyl can be $C_1$-$C_4$ alkyl, or can be methyl, ethyl, n-propyl, isopropyl, n-butyl, isobutyl, pr-butyl, sec-butyl or tert-butyl, preferably methyl.

In a preferred embodiment, in each $R^{3-4}$, the halogen can be fluorine, chlorine, bromine or iodine.

In a preferred embodiment, in each $R^{3-4}$, the number of heteroatom(s) in the 4-6 membered heterocyclyl can be 1

or 2, or the 4-6 membered heterocyclyl can be

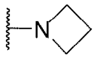, 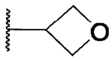, 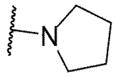, 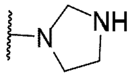,

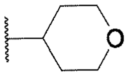, 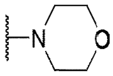, 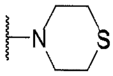, 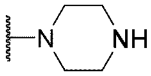, 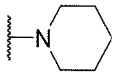, 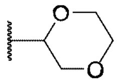

, preferably

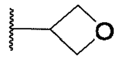.

In a preferred embodiment, in $R^{3-4-1}$, the $C_1$-$C_6$ alkyl can be $C_1$-$C_4$ alkyl, or can be methyl, ethyl, n-propyl, isopropyl, n-butyl, isobutyl, pr-butyl, sec-butyl or tert-butyl, preferably methyl.
In a preferred embodiment, in each $R^{3-4-2}$, the $C_1$-$C_6$ alkyl can be $C_1$-$C_4$ alkyl, or can be methyl, ethyl, n-propyl, isopropyl, n-butyl, isobutyl, pr-butyl, sec-butyl or tert-butyl, preferably methyl.
In a preferred embodiment, in $R^{3-5-1}$, the $C_1$-$C_6$ alkyl can be $C_1$-$C_4$ alkyl, or can be methyl, ethyl, n-propyl, isopropyl, n-butyl, isobutyl, pr-butyl, sec-butyl or tert-butyl, preferably methyl.
In a preferred embodiment, in $R^{3-5-2}$, the $C_1$-$C_6$ alkyl can be $C_1$-$C_4$ alkyl, or can be methyl, ethyl, n-propyl, isopropyl, n-butyl, isobutyl, pr-butyl, sec-butyl or tert-butyl, preferably methyl or ethyl.
In a preferred embodiment, in $R^{3-5-2}$, the 1-10 membered heteroalkyl can be 1-4 membered heteroalkyl.
In a preferred embodiment, in $R^{3-5-2}$, a heteroatom, except for phosphorus, in the 1-10 membered heteroalkyl is further selected from N and/or O.
In a preferred embodiment, in $R^{3-5-2}$, the number of heteroatoms in the 1-10 membered heteroalkyl is 3.
In a preferred embodiment, in $R^{3-5-2}$, the 4-10 membered heterocyclyl can be 4-8 membered heteroalkyl, for example,

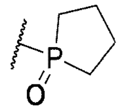.

In a preferred embodiment, in $R^{3-5-2}$, a heteroatom, except for phosphorus, in the 4-10 membered heterocyclyl is further selected from N and/or O.
In a preferred embodiment, in $R^{3-5-2}$, the number of heteroatom(s) in the 4-10 membered heterocyclyl is 1 or 3.
In a preferred embodiment, in each $R^{3-6}$, the halogen can be fluorine, chlorine, bromine or iodine, preferably chlorine.
In a preferred embodiment, in each $R^{3-6}$, the $C_1$-$C_6$ alkyl can be $C_1$-$C_4$ alkyl, or can be methyl, ethyl, n-propyl, isopropyl, n-butyl, isobutyl, pr-butyl, sec-butyl or tert-butyl, preferably methyl.
In a preferred embodiment, in each $R^{3-6}$, a heteroatom in the 4-6 membered heterocyclyl can be selected from N, and the number of heteroatom(s) can be 1 or 2, for example,

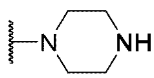.

In a preferred embodiment, in each $R^{3-6}$, the 3-6 membered cycloalkyl can be cyclopropyl, cyclobutyl, cyclopentyl or cyclohexyl, preferably cyclopropyl.
In a preferred embodiment, in each $R^{3-6-1}$, the $C_1$-$C_6$ alkyl can be $C_1$-$C_4$ alkyl, or can be methyl, ethyl, n-propyl, isopropyl, n-butyl, isobutyl, pr-butyl, sec-butyl or tert-butyl, preferably methyl.
In a preferred embodiment, in each $R^{3-7}$, the halogen can be fluorine, chlorine, bromine or iodine.
In a preferred embodiment, in each $R^{3-7}$, a heteroatom in the 4-6 membered heterocyclyl can be selected from one or more of N, O and S, the number of heteroatom(s) can be 1 or 2, or the 4-6 membered heterocyclyl can be

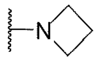, 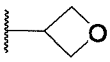, 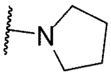, 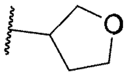, 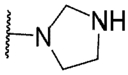, 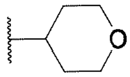, 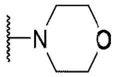,

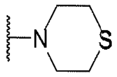, 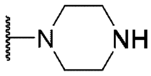, 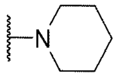 or 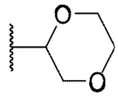

, for example,

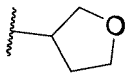.

In a preferred embodiment, in each $R^{3-9}$, the $C_1$-$C_6$ alkyl can be $C_1$-$C_4$ alkyl, or can be methyl, ethyl, n-propyl, isopropyl, n-butyl, isobutyl, pr-butyl, sec-butyl or tert-butyl, preferably methyl.

In a preferred embodiment, in $R^4$, the number of heteroatom(s) in the 4-6 membered heterocyclyl can be 1 or 2, or the 4-6 membered heterocyclyl can be

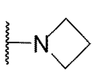, 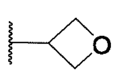, 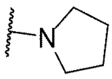, 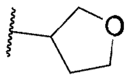, 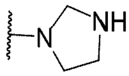,

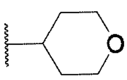, 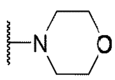, 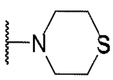, 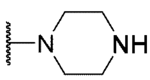, 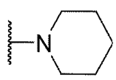 or 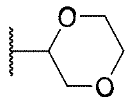;

, for example,

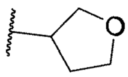.

In a preferred embodiment, in $R^5$, the 3-6 membered cycloalkyl can be cyclopropyl, cyclobutyl, cyclopentyl or cyclohexyl, preferably cyclobutyl.

In a preferred embodiment, in $R^5$, a heteroatom in the 4-6 membered heterocyclyl can be selected from one or more of N, O and S, and the number of heteroatom(s) can be 1 or 2, for example,

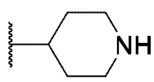.

In a preferred embodiment, in each $R^{5-2}$, the $C_1$-$C_6$ alkyl can be $C_1$-$C_4$ alkyl, or can be methyl, ethyl, n-propyl, isopropyl, n-butyl, isobutyl, pr-butyl, sec-butyl or tert-butyl, preferably methyl.

In a preferred embodiment, in each $R^{5-3-1}$, the $C_1$-$C_6$ alkyl can be $C_1$-$C_4$ alkyl, or can be methyl, ethyl, n-propyl, isopropyl, n-butyl, isobutyl, pr-butyl, sec-butyl or tert-butyl, preferably methyl.

In a preferred embodiment, in $R^6$, the number of heteroatom(s) in the 4-6 membered heterocyclyl can be 1 or 2.

In a preferred embodiment, in $R^6$, the 3-6 membered cycloalkyl can be cyclopropyl, cyclobutyl, cyclopentyl or cyclohexyl, preferably cyclohexyl.

In a preferred embodiment, in $R^7$, the 3-6 membered cycloalkyl can be cyclopropyl, cyclobutyl, cyclopentyl or cyclohexyl, preferably cyclohexyl.

In a preferred embodiment, the pharmaceutically acceptable salt can be trifluorohydrochloride.

In a preferred embodiment, the pharmaceutically acceptable salt can be trifluoroacetate or formate.

In a preferred embodiment, the compound of formula I, the pharmaceutically acceptable salt thereof or the isotopic compound thereof is a compound of formula Ia, Ib, Ic, Id or Ie:

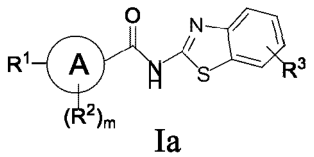
Ia
,
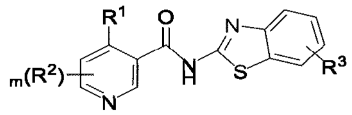
Ib
,
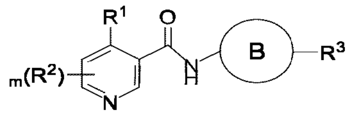
Ic
,

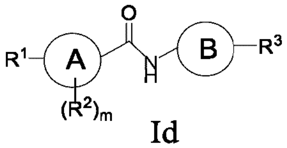
Id
or
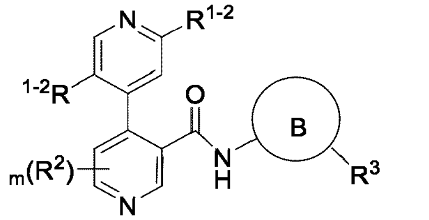
Ie
.

In a preferred embodiment, ring A is a 5-12 membered heteroaromatic ring; a heteroatom in the 5-12 membered heteroaromatic ring is selected from one or more of N, O and S, and the number of heteroatom(s) is 1, 2 or 3.

In a preferred embodiment, each $R^{1-1}$ is independently selected from deuterium, halogen, cyano, amino, $C_1$-$C_6$ alkyl unsubstituted or substituted by one or more $R^{1-1-1}$ or $C_1$-$C_6$ alkoxy unsubstituted or substituted by one or more $R^{1-1-2}$.

In a preferred embodiment, each $R^{1-2}$ is independently selected from deuterium, halogen, cyano, amino, $C_1$-$C_6$ alkyl unsubstituted or substituted by one or more $R^{1-1-1}$ or $C_1$-$C_6$ alkoxy unsubstituted or substituted by one or more $R^{1-1-2}$.

In a preferred embodiment, each $R^{1-2}$ is independently selected from deuterium, halogen, $C_1$-$C_6$ alkyl unsubstituted or substituted by one or more $R^{1-1-1}$ or $C_1$-$C_6$ alkoxy unsubstituted or substituted by one or more $R^{1-1-2}$.

In a preferred embodiment, each $R^{1-1-1}$ is independently selected from deuterium or halogen.

In a preferred embodiment, each $R^{1-1-2}$ is independently selected from deuterium or halogen.

In a preferred embodiment, each $R^2$ is independently selected from deuterium, or $C_1$-$C_6$ alkyl unsubstituted or substituted by one or more $R^{2-1}$.

In a preferred embodiment, each $R^2$ is independently selected from deuterium or $C_1$-$C_6$ alkyl.

In a preferred embodiment, each $R^2$ is independently selected from $C_1$-$C_6$ alkyl unsubstituted or substituted by one or more $R^{2-1}$.

In a preferred embodiment, each $R^{2-1}$ is independently deuterium.

In a preferred embodiment, m is 0, 1, 2 or 3.

In a preferred embodiment, each $R^{b-1}$ is independently selected from deuterium, oxo(=O), halogen, cyano, hydroxyl, $C_1$-$C_6$alkyl unsubstituted or substituted by one or more $R^{b-1-1}$ or $C_1$-$C_6$ alkoxy unsubstituted or substituted by one or more $R^{b-1-2}$.

In a preferred embodiment, each $R^{b-1-1}$ is independently selected from hydroxyl, halogen or amino unsubstituted or substituted by 1 or 2 $R^{b-1-2-1}$.

In a preferred embodiment, each $R^{b-1-2}$ is independently selected from hydroxyl, halogen or amino unsubstituted or substituted by 1 or 2 $R^{b-1-2-1}$.

In a preferred embodiment, each $R^{b-1-2-1}$ is independently selected from hydrogen or $C_1$-$C_6$ alkyl.

In a preferred embodiment, $R^3$ is hydrogen, 3-6 membered cycloalkyl unsubstituted or substituted by one or more $R^{3-1}$, 4-12 membered heterocyclyl unsubstituted or substituted by one or more $R^{3-2}$, 4-10 membered cycloalkenyl unsubstituted or substituted by one or more $R^{3-3}$, 4-10 membered heterocycloalkenyl unsubstituted or substituted by one or more $R^{3-4}$, 6-10 membered aryl unsubstituted or substituted by one or more $R^{3-5}$, 5-10 membered heteroaryl unsubstituted or substituted by one or more $R^{3-6}$, $-C(O)R^4$, $-NH(CH_2)_pR^5$, $-O(CH_2)_qR^6$, $C_1$-$C_6$ alkyl unsubstituted or substituted by one or more $R^{3-7}$, or $-OR^7$ unsubstituted or substituted by one or more $R^{3-8}$; a heteroatom in the 4-12 membered heterocyclyl, 4-10 membered heterocycloalkenyl or 5-10 membered heteroaryl is selected from one or more of N, O, and S, and the number of heteroatom(s) is 1, 2 3 or 4.

In a preferred embodiment, each $R^{3-1}$ is independently selected from deuterium, hydroxyl, halogen or $C_1$-$C_6$ alkyl.

In a preferred embodiment, each $R^{3-1}$ is independently selected from hydroxyl or deuterium.

In a preferred embodiment, each $R^{3-2}$ is independently selected from deuterium, hydroxyl, halogen, cyano, amino, $-NHC(O)R^{3-2-1}$, $C_1$-$C_6$ alkyl unsubstituted or substituted by one or more $R^{3-2-2}$, $C_2$-$C_6$ alkenyl, $C_2$-$C_6$ alkynyl, oxo(=O), $-C(O)NH_2$, 3-6 membered cycloalkyl unsubstituted or substituted by one or more $R^{3-2-3}$ or 4-6 membered heterocyclyl; a heteroatom in the 4-6 membered heterocyclyl is selected from one or more of N, O, and S, and the number of heteroatom(s) is 1 or 2.

In a preferred embodiment, each $R^{3-2}$ is independently selected from hydroxyl, deuterium or $C_1$-$C_6$ alkyl.

In a preferred embodiment, $R^{3-2-1}$ is $C_1$-$C_6$ alkyl.

In a preferred embodiment, each $R^{3-2-2}$ is independently selected from hydroxyl or halogen.

In a preferred embodiment, each $R^{3-2-2}$ is independently selected from deuterium, hydroxyl or halogen.

In a preferred embodiment, each $R^{3-2-2}$ is independently deuterium.

In a preferred embodiment, each $R^{3-2-3}$ is independently selected from $C_1$-$C_6$ alkyl or hydroxyl.

In a preferred embodiment, each $R^{3-3}$ is independently selected from deuterium, hydroxyl, halogen, cyano, amino, $-S(O)_2R^{3-4-1}$or $-NR^{3-4-2}R^{3-4-2}$.

In a preferred embodiment, each $R^{3-3}$ is independently selected from hydroxyl or deuterium.

In a preferred embodiment, each $R^{3-4}$ is independently selected from deuterium, hydroxyl, $C_1$-$C_6$ alkyl, oxo(=O), cyano, halogen, 4-6 membered heterocyclyl, $-S(O)_2R^{3-4-1}$ or $-NR^{3-4-2}R^{3-4-2}$.

In a preferred embodiment, $R^{3-4-1}$ is $C_1$-$C_6$ alkyl.

In a preferred embodiment, each $R^{3-4-2}$ is independently selected from hydrogen or $C_1$-$C_6$ alkyl.

In a preferred embodiment, each $R^{3-5}$ is independently selected from deuterium or

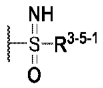

.

In a preferred embodiment, each $R^{3-5}$ is independently selected from deuterium,

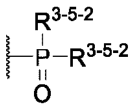 or 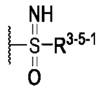 .

In a preferred embodiment, each $R^{3-5}$ is independently selected from

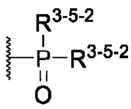 or 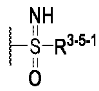 .

In a preferred embodiment, $R^{3-5-1}$ is $C_1$-$C_6$ alkyl.

In a preferred embodiment, $R^{3-5-1}$ and $R^{3-5-2}$ are each independently $C_1$-$C_6$ alkyl.

In a preferred embodiment, each $R^{3-6}$ is independently selected from deuterium, halogen or $C_1$-$C_6$ alkyl.

In a preferred embodiment, each $R^{3-6}$ is independently selected from deuterium, halogen or $C_1$-$C_6$ alkyl unsubstituted or substituted by one or more $R^{3-2-2}$.

In a preferred embodiment, each $R^{3-7}$ is independently selected from deuterium, hydroxyl, halogen or 4-6 membered heterocyclyl; a heteroatom in the 4-6 membered heterocyclyl is O, and the number of heteroatom(s) is 1.

In a preferred embodiment, $R^{3-8}$ is hydroxyl.

In a preferred embodiment, $R^5$ is 3-6 membered cycloalkyl substituted by one or more $R^{5-1}$.

In a preferred embodiment, $R^6$ is 3-6 membered cycloalkyl substituted by one or more $R^{6-1}$.

In a preferred embodiment, ring A is a 5-10 membered heteroaromatic ring; a heteroatom in the 5-10 membered heteroaromatic ring is selected from one or two of N, O or S, and the number of heteroatom(s) is 1 or 2.

In a preferred embodiment, $R^1$ is phenyl unsubstituted or substituted by one or more $R^{1-1}$ or 5-10 membered heteroaryl unsubstituted or substituted by one or more $R^{1-2}$; a heteroatom in the 5-10 membered heteroaryl is N and/or O, and the number of heteroatom(s) is 1 or 2.

In a preferred embodiment, each $R^{1-1}$ is independently selected from halogen or $C_1$-$C_6$ alkoxy unsubstituted or substituted by one or more $R^{1-1-2}$.

In a preferred embodiment, each $R^{1-2}$ is independently selected from halogen or $C_1$-$C_6$ alkoxy unsubstituted or substituted by one or more $R^{1-1-2}$.

In a preferred embodiment, each $R^{1-1-2}$ is independently halogen.

In a preferred embodiment, each $R^2$ is independently $C_1$-$C_6$ alkyl.

In a preferred embodiment, m is 0 or 1.

In a preferred embodiment, ring B is a 5-12 membered fused-ring heteroaromatic ring unsubstituted or substituted by one or more $R^{b-1}$; a heteroatom in the 5-12 membered fused-ring heteroaromatic ring is N and/or S, the number of heteroatom(s) is 1, 2, 3 or 4, a single heteroaryl ring in the 5-12 membered fused-ring heteroaromatic ring is a

5-7 membered heteroaromatic ring, the number of rings in the 5-12 membered fused-ring heteroaromatic ring is 2, and

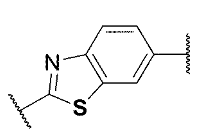 , 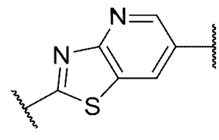 or

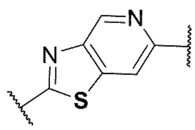

is preferred; preferably, ring B is

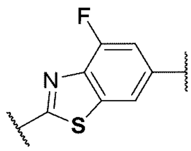 , 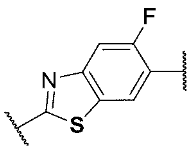 , 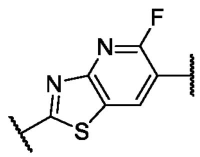 ,

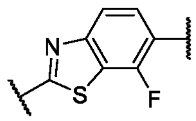 , 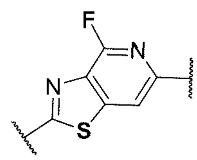 , 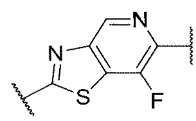 or 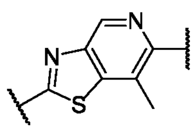 .

In a preferred embodiment, each $R^{b-1}$ is independently selected from halogen or $C_1$-$C_6$alkyl.

In a preferred embodiment, $R^3$ is hydrogen, 3-6 membered cycloalkyl unsubstituted or substituted by one or more $R^{3-1}$, 4-6 membered heterocyclyl unsubstituted or substituted by one or more $R^{3-2}$, 4-6 membered cycloalkenyl unsubstituted or substituted by one or more $R^{3-3}$, 4-6 membered heterocycloalkenyl unsubstituted or substituted by one or more $R^{3-4}$, phenyl unsubstituted or substituted by one or more $R^{3-5}$, 5-6 membered heteroaryl unsubstituted or substituted by one or more $R^{3-6}$, -C(O)$R^4$ or $C_1$-$C_6$ alkyl unsubstituted or substituted by one or more $R^{3-7}$; a heteroatom in the 4-6 membered heterocyclyl, 4-6 membered heterocycloalkenyl or 5-6 membered heteroaryl is N and/or O, and the number of heteroatom(s) is 1 or 2.

In a preferred embodiment, $R^3$ is hydrogen, 3-6 membered cycloalkyl unsubstituted or substituted by one or more $R^{3-1}$, 4-6 membered heterocyclyl unsubstituted or substituted by one or more $R^{3-2}$, 4-6 membered cycloalkenyl unsubstituted or substituted by one or more $R^{3-3}$, 4-6 membered heterocycloalkenyl unsubstituted or substituted by one or more $R^{3-4}$, phenyl unsubstituted or substituted by one or more $R^{3-5}$, 5-6 membered heteroaryl unsubstituted or substituted by one or more $R^{3-6}$, -C(O)$R^4$ or $C_1$-$C_6$ alkyl unsubstituted or substituted by one or more $R^{3-7}$; a heteroatom in the 4-6 membered heterocyclyl, 4-6 membered heterocycloalkenyl or 5-6 membered heteroaryl is N and/or O, and the number of heteroatom(s) is 1, 2 or 3.

In a preferred embodiment, $R^3$ is hydrogen, 3-6 membered cycloalkyl unsubstituted or substituted by one or more $R^{3-1}$, 4-6 membered heterocyclyl unsubstituted or substituted by one or more $R^{3-2}$, 4-6 membered cycloalkenyl unsubstituted or substituted by one or more $R^{3-3}$, 4-6 membered heterocycloalkenyl unsubstituted or substituted by one or more $R^{3-4}$, phenyl unsubstituted or substituted by one or more $R^{3-5}$, 5-6 membered heteroaryl unsubstituted or substituted by one or more $R^{3-6}$, -C(O)$R^4$ or $C_1$-$C_6$ alkyl unsubstituted or substituted by one or more $R^{3-7}$; a heteroatom in the 4-6 membered heterocyclyl or 4-6 membered heterocycloalkenyl is N and/or O, and the number of heteroatom(s) is 1 or 2; or a heteroatom in the 5-6 membered heteroaryl is N and/or O, and the number of heteroatom(s) is 1, 2 or 3.

In a preferred embodiment, each $R^{3-1}$ is independently hydroxyl.

In a preferred embodiment, each $R^{3-2}$ is independently selected from hydroxyl or $C_1$-$C_6$ alkyl.

In a preferred embodiment, each $R^{3-3}$ is independently hydroxyl.

In a preferred embodiment, each $R^{3-4}$ is independently $C_1$-$C_6$ alkyl.

In a preferred embodiment, each $R^{3-5}$ is independently

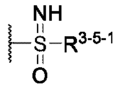 .

In a preferred embodiment, each $R^{3-6}$ is independently selected from halogen or $C_1$-$C_6$ alkyl.

In a preferred embodiment, each $R^{3-7}$ is independently 4-6 membered heterocyclyl; and a heteroatom in the 4-6 membered heterocyclyl is O, and the number of heteroatom(s) is 1.

In a preferred embodiment, $R^4$ is 4-6 membered heterocyclyl; and a heteroatom in the 4-6 membered heterocyclyl is O, and the number of heteroatom(s) is 1.

In a preferred embodiment, ring A is a 6-12 membered aromatic ring or a 5-6 membered heteroaromatic ring; and a heteroatom in the 5-6 membered heteroaromatic ring is selected from one or two of N, O, and S, and the number of heteroatom(s) is 1, 2, or 3.

In a preferred embodiment, $R^1$ is 6-12 membered aryl unsubstituted or substituted by one or more $R^{1-1}$ or 5-6 membered heteroaryl unsubstituted or substituted by one or more $R^{1-2}$; and a heteroatom in the 5-6 membered heteroaromatic ring is selected from one or two of N, O, and S, and the number of heteroatom(s) is 1, 2, or 3.

In a preferred embodiment, each $R^{1-1}$ and $R^{1-2}$ is independently selected from halogen or $C_1$-$C_6$ alkoxy.

In a preferred embodiment, ring B is a 5-20 membered fused-ring heteroaromatic ring unsubstituted or substituted by one or more $R^{b-1}$; a heteroatom in the 5-20 membered fused-ring heteroaromatic ring is selected from one or more of N, O, and S, and the number of heteroatom(s) is 1, 2, 3, 4, 5, 6, 7 or 8, a single heteroaromatic ring in the 5-20 membered fused-ring heteroaromatic ring is a 5-7 membered heteroaromatic ring, and the number of rings in the 5-20 membered fused-ring heteroaromatic ring is 3 or 4.

In a preferred embodiment, each $R^{b-1}$ is independently selected from deuterium, cyano, halogen or $C_1$-$C_6$ alkyl unsubstituted or substituted by one or more $R^{b-1-1}$.

In a preferred embodiment, $R^3$ is hydrogen,

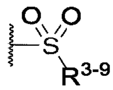

, 3-6 membered cycloalkyl unsubstituted or substituted by one or more $R^{3-1}$, 4-9 membered heterocyclyl unsubstituted or substituted by one or more $R^{3-2}$, 4-6 membered cycloalkenyl unsubstituted or substituted by one or more $R^{3-3}$, 4-6 membered heterocycloalkenyl unsubstituted or substituted by one or more $R^{3-4}$, phenyl unsubstituted or substituted by one or more $R^{3-5}$, 5-10 membered heteroaryl unsubstituted or substituted by one or more $R^{3-6}$, -C(O)$R^4$ or $C_1$-$C_6$ alkyl unsubstituted or substituted by one or more $R^{3-7}$; a heteroatom in the 4-6 membered heterocycloalkenyl and in the 5-10 membered heteroaryl is N and/or O, and the number of heteroatom(s) is 1 or 2; and a heteroatom in the 4-9 membered heterocyclyl is selected from one or two of N, O, and S, and the number of heteroatom(s) is 1 or 2.

In a preferred embodiment, each $R^{3-2}$ is independently selected from deuterium, oxo, halogen, $C_1$-$C_6$ alkoxy, -$NR^{3-4-2}R^{3-4-2}$, -$S(O)_2R^{3-4-1}$, hydroxyl or $C_1$-$C_6$ alkyl unsubstituted or substituted by one or more $R^{3-2-2}$.

In a preferred embodiment, each $R^{3-3}$ is independently selected from deuterium, oxo, amino or hydroxyl.

In a preferred embodiment, each $R^{3-4}$ is independently selected from deuterium, oxo or $C_1$-$C_6$alkyl.

In a preferred embodiment, $R^{3-4-1}$ is $C_1$-$C_6$ alkyl.

In a preferred embodiment, each $R^{3-4-2}$ is independently selected from -$S(O)_2R^{3-4-1}$, hydrogen or $C_1$-$C_6$ alkyl.

In a preferred embodiment, each $R^{3-5}$ is independently selected from deuterium,

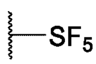 , 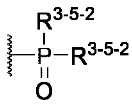

or

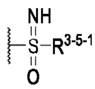 .

In a preferred embodiment, each $R^{3-5-2}$ is independently selected from hydroxyl, $C_1$-$C_6$ alkyl or 1-10 membered heteroalkyl, or two $R^{3-5-2}$s form 4-10 membered heterocyclyl; a heteroatom, except for phosphorus, in the 1-10 membered heteroalkyl further comprises selected from one or more of N, O, and S, and the number of heteroatom(S) is 1, 2, 3 or 4; a

heteroatom, except for phosphorus, in the 4-10 membered heterocyclyl further comprises selected from one or more of N, O, and S, and the number of heteroatom(S) is 1, 2, 3 or 4.

In a preferred embodiment, each $R^{3-6}$ is independently selected from deuterium, halogen, $C_1$-$C_6$ alkyl unsubstituted or substituted by one or more $R^{3-2-2}$ or 4-6 membered heterocyclyl unsubstituted or substituted by one or more $R^{3-6-1}$; and a heteroatom in the 4-6 membered heterocyclyl is selected from one or two of N, O, and S, and the number of heteroatoms is 1 or 2.

In a preferred embodiment, each $R^{3-6-1}$ is independently $C_1$-$C_6$ alkyl.

In a preferred embodiment, each $R^{3-2-2}$ is independently selected from deuterium or hydroxyl.

In a preferred embodiment, each $R^{3-7}$ is independently selected from deuterium, hydroxyl, -$S(O)_2R^{3-4-1}$, 4-6 membered heterocyclyl; and a heteroatom in the 4-6 membered heterocyclyl is O, and a heteroatom number is 1.

In a preferred embodiment, $R^3$ is hydrogen,

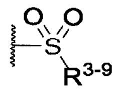

, 3-6 membered cycloalkyl unsubstituted or substituted by one or more $R^{3-1}$, 4-9 membered heterocyclyl unsubstituted or substituted by one or more $R^{3-2}$, 4-6 membered cycloalkenyl unsubstituted or substituted by one or more $R^{3-3}$, 4-6 membered heterocycloalkenyl unsubstituted or substituted by one or more $R^{3-4}$, phenyl unsubstituted or substituted by one or more $R^{3-5}$, 5-10 membered heteroaryl unsubstituted or substituted by one or more $R^{3-6}$, -C(O)$R^4$ or $C_1$-$C_6$ alkyl unsubstituted or substituted by one or more $R^{3-7}$; a heteroatom in the 4-6 membered heterocycloalkenyl and in the 5-10 membered heteroaryl is N and/or O, and the number of heteroatom(s) is 1, 2 or 3; and a heteroatom in the 4-9 membered heterocyclyl is selected from one or two of N, O, and S, and the number of heteroatom(s) is 1 or 2.

In a preferred embodiment, $R^3$ is hydrogen,

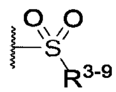

, 3-6 membered cycloalkyl unsubstituted or substituted by one or more $R^{3-1}$, 4-9 membered heterocyclyl unsubstituted or substituted by one or more $R^{3-2}$, 4-6 membered cycloalkenyl unsubstituted or substituted by one or more $R^{3-3}$, 4-6 membered heterocycloalkenyl unsubstituted or substituted by one or more $R^{3-4}$, phenyl unsubstituted or substituted by one or more $R^{3-5}$, 5-10 membered heteroaryl unsubstituted or substituted by one or more $R^{3-6}$, -C(O)$R^4$ or $C_1$-$C_6$ alkyl unsubstituted or substituted by one or more $R^{3-7}$; a heteroatom in the 4-6 membered heterocycloalkenyl is N and/or O, and the number of heteroatom(s) is 1 or 2; a heteroatom in the 5-10 membered heteroaryl is N and/or O, and the number of heteroatom(s) is 1, 2 or 3; and a heteroatom in the 4-9 membered heterocyclyl is selected from one or two of N, O, and S, and the number of heteroatom(s) is 1 or 2.

In a preferred embodiment, $R^3$ is hydrogen, 3-6 membered cycloalkyl unsubstituted or substituted by one or more $R^{3-1}$, 4-12 membered heterocyclyl unsubstituted or substituted by one or more $R^{3-2}$, 4-10 membered cycloalkenyl unsubstituted or substituted by one or more $R^{3-3}$, 4-10 membered heterocycloalkenyl unsubstituted or substituted by one or more $R^{3-4}$, 6-10 membered aryl unsubstituted or substituted by one or more $R^{3-5}$, 5-10 membered heteroaryl unsubstituted or substituted by one or more $R^{3-6}$, -C(O)$R^4$, $C_1$-$C_6$ alkyl unsubstituted or substituted by one or more $R^{3-7}$ or -O$R^7$ unsubstituted or substituted by one or more $R^{3-8}$; a heteroatom in the 4-12 membered heterocyclyl or 4-10 membered heterocycloalkenyl is one or more of N, O, and S, and the number of heteroatom(s) is 1, or 2; and a heteroatom in the 5-10 membered heteroaryl is N and/or O, and the number of heteroatom(s) is 1, 2, 3 or 4.

In a preferred embodiment, ring B is a 5-20 membered fused-ring heteroaromatic ring unsubstituted or substituted by one or more $R^{b-1}$; a heteroatom in the 5-20 membered fused-ring heteroaromatic ring is selected from one or more of N, O, and S, and the number of heteroatom(s) is 1, 2, 3, 4, 5, 6, 7 or 8, a single heteroaromatic ring in the 5-20 membered fused-ring heteroaromatic ring is a 5-7 membered heteroaromatic ring, and the number of rings in the 5-20 membered fused-ring heteroaromatic ring is 2, 3 or 4.

In a preferred embodiment, ring B is a 5-20 membered fused-ring heteroaromatic ring unsubstituted or substituted by one or more $R^{b-1}$; a heteroatom in the 5-20 membered fused-ring heteroaromatic ring is selected from one or more of N, O, and S, and the number of heteroatom(s) is 1, 2, 3, 4, 5, 6, 7 or 8, a single heteroaromatic ring in the 5-20 membered fused-ring heteroaromatic ring is a 5-7 membered heteroaromatic ring, the number of rings in the 5-20 membered fused-ring heteroaromatic ring is 2, 3 or 4, and the unsubstituted 5-20 membered fused-ring heteroaromatic ring is

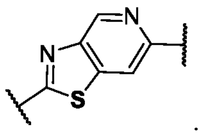

.

In a preferred embodiment, ring A is a 5-12 membered heteroaromatic ring; and a heteroatom in the 5-12 membered heteroaromatic ring is one or more of N, O and S, and the number of heteroatom(s) is 1, 2 or 3.

$R^1$ is 5-12 membered heteroaryl unsubstituted or substituted by one or more $R^{1-2}$; a heteroatom in the 5-12 membered heteroaromatic ring is one or two selected from N, O, and S, and the number of heteroatom(s) is 1, 2 or 3;

$R^3$ is hydrogen,

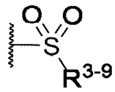

,

3-12 membered cycloalkyl unsubstituted or substituted by one or more $R^{3-1}$, 4-12 membered heterocyclyl substituted by one or more $R^{3-2}$, 4-12 membered cycloalkenyl unsubstituted or substituted by one or more $R^{3-3}$, 4-12 membered heterocycloalkenyl unsubstituted or substituted by one or more $R^{3-4}$, 6-10 membered aryl unsubstituted or substituted by one or more $R^{3-5}$, 5-10 membered heteroaryl unsubstituted or substituted by one or more $R^{3-6}$, $-C(O)R^4$, $-NH(CH_2)_pR^5$, $-O(CH_2)_qR^6$, $C_1$-$C_6$ alkyl, or $-OR^7$ unsubstituted or substituted by one or more $R^{3-8}$; a heteroatom in each of the 4-12 membered heterocyclyl, 4-12 membered heterocycloalkenyl and 5-10 membered heteroaryl is one or more selected from N, O, and S, and the number of heteroatom(s) is 1, 2, 3 or 4;

each of $R^{3-5}$ and $R^{3-6}$ is independently selected from deuterium,

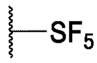

,

hydroxy, amino, $-NHC(O)R^{3-2-1}$, $C_1$-$C_6$ alkyl, $C_2$-$C_6$ alkenyl, $C_2$-$C_6$ alkynyl, $-C(O)NH_2$, 3-6 membered cycloalkyl substituted by one or more $R^{3-2-3}$, 4-6 membered heterocyclyl unsubstituted or substituted by one or more $R^{3-6-1}$, $-S(O)_2R^{3-4-1}$, $-NR^{3-4-2}R^{3-4-2}$,

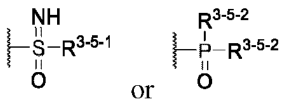

;

; and a heteroatom in the 4-6 membered heterocyclyl is one or more selected from N, O, and S, and the number of heteroatom(s) is 1 or 2.

In an embodiment,

L is

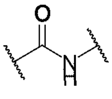

;

ring A is a 5-12 membered heteroaromatic ring; a heteroatom in the 5-12 membered heteroaromatic ring is one or more of N, O, and S, and the number of heteroatom(s) is 1, 2, or 3;

$R^1$ is 6-12 membered aryl unsubstituted or substituted by one or more $R^{1-1}$, or 5-12 membered heteroaryl unsubstituted or substituted by one or more $R^{1-2}$; a heteroatom in the 5-12 membered heteroaryl is one or more of N, O, and S, and the number of heteroatom(s) is 1, 2, or 3;

each of $R^{1-1}$ and $R^{1-2}$ is independently selected from deuterium, halogen, cyano, amino, $C_1$-$C_6$ alkyl unsubstituted or substituted by one or more $R^{1-1-1}$, or $C_1$-$C_6$ alkoxy unsubstituted or substituted by one or more $R^{1-2}$;

each of $R^{1-1-1}$ and $R^{1-1-2}$ is independently selected from deuterium or halogen;

each of $R^2$ is independently selected from deuterium or $C_1$-$C_6$ alkyl;

m is 0, 1, 2 or 3; and

ring B is a 5-20 membered fused-ring heteroaromatic ring unsubstituted or substituted by one or more $R^{b-1}$; a heteroatom in the 5-20 membered fused-ring heteroaromatic ring is one or more of N, O, and S, and the number of heteroatom(s) is 1, 2, 3, 4, 5 or 6, a single heteroaromatic ring in the 5-20 membered fused-ring heteroaromatic ring is a 5-7 membered heteroaryl ring, and the number of rings in the 5-20 membered fused-ring heteroaromatic ring is 2, 3 or 4;

each $R^{b-1}$ is independently selected from deuterium, oxo(=O), halogen, cyano, hydroxyl, $C_1$-$C_6$ alkyl unsubstituted or substituted by one or more $R^{b-1-1}$ or $C_1$-$C_6$ alkoxy unsubstituted or substituted by one or more $R^{b-1-2}$;

each of $R^{b-1-1}$ and $R^{b-1-2}$ is independently selected from hydroxy, halogen or amino unsubstituted or substituted by 1 or 2 $R^{b-1-2-1}$;

each of $R^{b-1-2-1}$ is independently selected from hydrogen or $C_1$-$C_6$ alkyl;

$R^3$ is hydrogen, 3-6 membered cycloalkyl unsubstituted or substituted by one or more $R^{3-1}$, 4-12 membered heterocyclyl unsubstituted or substituted by one or more $R^{3-2}$, 4-10 membered cycloalkenyl unsubstituted or substituted by one or more $R^{3-3}$, 4-10 membered heterocycloalkenyl unsubstituted or substituted by one or more $R^{3-4}$, 6-10 membered aryl unsubstituted or substituted by one or more $R^{3-5}$, 5-10 membered heteroaryl unsubstituted or substituted by one or more $R^{3-6}$, $-C(O)R^4$, $-NH(CH_2)_pR^5$, $-O(CH_2)_qR^6$, $C_1$-$C_6$ alkyl unsubstituted or substituted by one or more $R^{3-7}$, or $-OR^7$ unsubstituted or substituted by one or more $R^{3-8}$; a heteroatom in the 4-12 membered heterocyclyl, 4-10 membered heterocycloalkenyl or 5-10 membered heteroaryl is one or more selected from N, O, and S, and the number of heteroatom(s) is 1, 2 or 3;

p is 0, 1, 2, or 3;

q is 1, 2, or 3;

each of $R^{3-1}$ is independently selected from deuterium, hydroxy, halogen or $C_1$-$C_6$ alkyl;

each of $R^{3-2}$ is independently selected from deuterium, hydroxy, halogen, cyano, amino, $-NHC(O)R^{3-2-1}$, $C_1$-$C_6$ alkyl unsubstituted or substituted by one or more $R^{3-2-2}$, $C_2$-$C_6$ alkenyl, $C_2$-$C_6$ alkynyl, oxo(=O), $-C(O)NH_2$, 3-6 membered cycloalkyl unsubstituted or substituted by one or more $R^{3-2-3}$ or 4-6 membered heterocyclyl; a heteroatom in the 4-6 membered heterocyclyl is one or more selected from N, O, and S, and the number of heteroatom(s) is 1 or 2;

$R^{3-2-1}$ is $C_1$-$C_6$ alkyl;

each of $R^{3-2-2}$ is independently selected from hydroxy or halogen;

each of $R^{3-2-3}$ is independently selected from $C_1$-$C_6$ alkyl or hydroxy;

each of $R^{3-3}$ is independently selected from deuterium, hydroxy, halogen, cyano, amino, $-S(O)_2R^{3-4-1}$ or $-NR^{3-4-2}R^{3-4-2}$;

each of $R^3$ is independently selected from deuterium, hydroxy, $C_1$-$C_6$ alkyl, oxo(=O), cyano, halogen, 4-6 membered heterocyclyl, $-S(O)_2R^{3-4-1}$ or $-NR^{3-4-2}R^{3-4-2}$;

$R^{3-4-1}$ is $C_1$-$C_6$ alkyl;

each $R^{3-4-2}$ is independently selected from hydrogen or $C_1$-$C_6$ alkyl;

each $R^{3-5}$ is independently selected from deuterium or

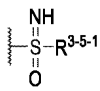 ;

$R^{3-5-1}$ is $C_1$-$C_6$ alkyl;

each $R^{3-6}$ is independently selected from deuterium, halogen or $C_1$-$C_6$ alkyl;

each $R^{3-7}$ is independently selected from deuterium, hydroxy, halogen or 4-6 membered heterocyclyl; a heteroatom in the 4-6 membered heterocyclyl is O, and the number of heteroatom(s) is 1;

$R^{3-8}$ is hydroxy;

$R^4$ is 4-6 membered heterocyclyl; a heteroatom in the 4-6 membered heterocyclyl is one or more selected from N, O, and S, and the number of heteroatom(s) is 1 or 2;

$R^5$ is 3-6 membered cycloalkyl unsubstituted or substituted by one or more $R^{5-1}$;

each $R^{5-1}$ is independently hydroxy;

$R^6$ is 3-6 membered cycloalkyl unsubstituted or substituted by one or more $R^{6-1}$

each $R^{6-1}$ is independently hydroxy;

$R^7$ is 3-6 membered cycloalkyl.

In an embodiment,

L is

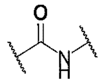;

ring A is a 5-10 membered heteroaromatic ring; a heteroatom in the 5-10 membered heteroaromatic ring is N, and the number of heteroatom(s) is 1 or 2;

$R^1$ is 6-10 membered aryl unsubstituted or substituted by one or more $R^{1-1}$ or 5-6 membered heteroaryl unsubstituted or substituted by one or more $R^{1-2}$; a heteroatom in the 5-6 membered heteroaryl is N, and the number of heteroatom(s) is 1 or 2;

each of $R^{1-1}$ and $R^{1-2}$ is independently selected from deuterium, halogen, $C_1$-$C_6$ alkyl unsubstituted or substituted by one or more $R^{1-1-1}$, $C_1$-$C_6$ alkoxy unsubstituted or substituted by one or more $R^{1-1-2}$ or

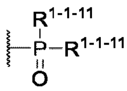;

each of $R^{1-1-1}$ and $R^{1-1-2}$ is independently halogen;

each of $R^{1-1-11}$ is independently selected from hydroxy or $C_1$-$C_6$ alkyl;

$R^2$ is independently selected from deuterium or $C_1$-$C_6$ alkyl unsubstituted or substituted by one or more $R^{2-1}$; each of $R^{2-1}$ is independently selected from halogen or deuterium;

m is 0 or 1;

ring B is a 5-12 membered fused-ring heteroaromatic ring unsubstituted or substituted by one or more $R^{b-1}$; a heteroatom in the 5-12 membered fused-ring heteroaromatic ring is N and/or S, the number of heteroatom(s) is 1, 2, 3 or 4, and the number of rings in the 5-12 membered fused-ring heteroaromatic ring is 2;

each of $R^{b-1}$ is independently selected from deuterium, cyano, halogen, or $C_1$-$C_6$ alkyl unsubstituted or substituted by one or more $R^{b-1-1}$;

each of $R^{b-1-1}$ is independently halogen;

$R^3$ is hydrogen,

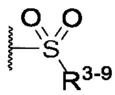,

3-6 membered cycloalkyl unsubstituted or substituted by one or more $R^{3-1}$, 4-9 membered heterocyclyl unsubstituted or substituted by one or more $R^{3-2}$, 4-6 membered cycloalkenyl unsubstituted or substituted by one or more $R^{3-3}$, 4-6 membered heterocycloalkenyl unsubstituted or substituted by one or more $R^{3-4}$, phenyl unsubstituted or substituted by one or more $R^{3-5}$, 5-10 membered heteroaryl unsubstituted or substituted by one or more $R^{3-6}$, -C(O)$R^4$ or $C_1$-$C_6$ alkyl unsubstituted or substituted by one or more $R^{3-7}$; a heteroatom in the 4-6 membered heterocycloalkenyl and 5-10 membered heteroaryl is N and/or O, and the number of heteroatoms is 1 or 2; a heteroatom in the 4-9 membered heterocyclyl is one or two selected from N, O, and S, and the number of heteroatom(s) is 1 or 2;

each $R^{3-1}$ is independently selected from deuterium or hydroxy;

each $R^{3-2}$ is independently selected from deuterium, oxo, halogen, $C_1$-$C_6$ alkoxy, -$NR^{3-4-2}R^{3-4-2}$, -$S(O)_2R^{3-4-1}$, hydroxyl or $C_1$-$C_6$ alkyl unsubstituted or substituted by one or more $R^{3-2-2}$;

each $R^{3-3}$ is independently selected from deuterium, oxo, amino or hydroxyl;

each $R^{3-4}$ is independently selected from deuterium, oxo or $C_1$-$C_6$ alkyl;

$R^{3-4-1}$ is $C_1$-$C_6$ alkyl;

each $R^{3-4-2}$ is independently selected from -$S(O)_2R^{3-4-1}$, hydrogen or $C_1$-$C_6$ alkyl;

each $R^{3-5}$ is independently selected from deuterium,

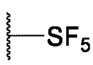, 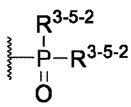 or 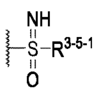;

$R^{3-5-1}$ is independently $C_1$-$C_6$ alkyl;

each $R^{3-5-2}$ is independently selected from hydroxy, $C_1$-$C_6$ alkyl or 1-10 membered heteroalkyl, or two $R^{3-5-2}$s form 4-10 membered heterocyclyl; a heteroatom, except for phosphorus, in the 1-10 membered heteroalkyl further

comprises one or more selected from N, O, and S, and the number of heteroatom(s) is 1, 2, 3 or 4; a heteroatom, except for phosphorus, in the 4-10 membered heterocyclyl further comprises one or more selected from N, O, and S, and the number of heteroatom(s) is 1, 2, 3 or 4;

each $R^{3-6}$ is independently selected from deuterium, halogen, $C_1$-$C_6$ alkyl unsubstituted or substituted by one or more $R^{3-2-2}$ or 4-6 membered heterocyclyl unsubstituted or substituted by one or more $R^{3-6-1}$; a heteroatom in the 4-6 membered heterocyclyl is one or two selected from N, O, and S, and the number of heteroatom(s) is 1 or 2;

each $R^{3-6-1}$ is independently $C_1$-$C_6$ alkyl;

each $R^{3-2-2}$ is independently selected from deuterium or hydroxy;

each $R^{3-7}$ is independently selected from deuterium, hydroxy, $-S(O)_2R^{3-4-1}$, or 4-6 membered heterocyclyl; a heteroatom in the 4-6 membered heterocyclyl is O, and the number of heteroatom(s) is 1;

$R^4$ is 4-6 membered heterocyclyl; a heteroatom in the 4-6 membered heterocyclyl is O, and the number of heteroatom(s) is 1; $R^4$ is 4-6 membered heterocyclyl; a heteroatom in the 4-6 membered heterocyclyl is one or more selected from N, O, and S, and the number of heteroatom(s) is 1 or 2;

$R^5$ is 3-6 membered cycloalkyl unsubstituted or substituted by one or more $R^{5-1}$;

each $R^{5-1}$ is independently hydroxy;

$R^6$ is 3-6 membered cycloalkyl unsubstituted or substituted by one or more $R^{6-1}$;

each $R^{6-1}$ is independently hydroxy;

$R^7$ is 3-6 membered cycloalkyl.

In an embodiment,

L is

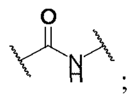
;

ring A is a 5-10 membered heteroaromatic ring; a heteroatom in the 5-10 membered heteroaromatic ring is N, and the number of heteroatom(s) is 1 or 2;

$R^1$ is 6-10 membered aryl unsubstituted or substituted by one or more $R^{1-1}$ or 5-6 membered heteroaryl unsubstituted or substituted by one or more $R^{1-2}$; a heteroatom in the 5-6 membered heteroaryl is N, and the number of heteroatom(s) is 1 or 2;

each of $R^{1-1}$ and $R^{1-2}$ is independently selected from deuterium, halogen, $C_1$-$C_6$ alkyl unsubstituted or substituted by one or more $R^{1-1-1}$, $C_1$-$C_6$ alkoxy unsubstituted or substituted by one or more $R^{1-1-2}$ or

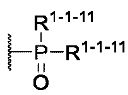
;

each of $R^{1-1-1}$ and $R^{1-1-2}$ is independently halogen;

each of $R^{1-1-11}$ is independently selected from hydroxy or $C_1$-$C_6$ alkyl;

$R^2$ is independently selected from deuterium or $C_1$-$C_6$ alkyl unsubstituted or substituted by one or more $R^{2-1}$; each of $R^{2-1}$ is independently halogen;

m is 0 or 1;

ring B is 5-12 membered fused-ring heteroaromatic ring unsubstituted or substituted by one or more $R^{b-1}$; a heteroatom in the 5-12 membered fused-ring heteroaromatic ring is N and/or S, the number of heteroatom(s) is 1, 2, 3 or 4, and the number of rings in the 5-12 membered fused-ring heteroaromatic ring is 2;

each $R^{b-1}$ is independently selected from deuterium, halogen, or $C_1$-$C_6$ alkyl unsubstituted or substituted by one or more $R^{b-1-1}$;

each of $R^{b-1-1}$ is independently halogen;

$R^3$ is hydrogen, 3-6 membered cycloalkyl unsubstituted or substituted by one or more $R^{3-1}$, 4-6 membered heterocyclyl unsubstituted or substituted by one or more $R^{3-2}$, 4-6 membered cycloalkenyl unsubstituted or substituted by one or more $R^{3-3}$, 4-6 membered heterocycloalkenyl unsubstituted or substituted by one or more $R^{3-4}$, phenyl unsubstituted or substituted by one or more $R^{3-5}$, 5-6 membered heteroaryl unsubstituted or substituted by one or more $R^{3-6}$, $-C(O)R^4$ or $C_1$-$C_6$ alkyl unsubstituted or substituted by one or more $R^{3-7}$; a heteroatom in the 4-6 membered heterocyclyl, 4-6 membered heterocycloalkenyl or 5-6 membered heteroaryl is N and/or O, and the number of heteroatom(s) is 1 or 2;

each $R^{3-1}$ is independently selected from deuterium or hydroxy;
each $R^{3-2}$ is independently selected from deuterium, hydroxy or $C_1$-$C_6$ alkyl;
each $R^{3-3}$ is independently selected from deuterium or hydroxy;
each $R^{3-4}$ is independently selected from deuterium or $C_1$-$C_6$ alkyl;
each $R^{3-5}$ is independently selected from deuterium or

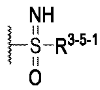;

$R^{3-5-1}$ is $C_1$-$C_6$ alkyl;
each $R^{3-6}$ is independently selected from deuterium, halogen or $C_1$-$C_6$ alkyl;
each $R^{3-7}$ is independently selected from deuterium, or 4-6 membered heterocyclyl; a heteroatom in the 4-6 membered heterocyclyl is O, and the number of heteroatom(s) is 1;
$R^4$ is 4-6 membered heterocyclyl; and a heteroatom in the 4-6 membered heterocyclyl is O, and the number of heteroatom(s) is 1.

In an embodiment,

L is

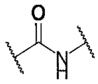;

ring A is a 5-10 membered heteroaromatic ring; a heteroatom in the 5-10 membered heteroaromatic ring is N, and the number of heteroatom(s) is 1 or 2;
$R^1$ is 6-10 membered aryl unsubstituted or substituted by one or more $R^{1-1}$ or 5-6 membered heteroaryl unsubstituted or substituted by one or more $R^{1-2}$; a heteroatom in the 5-6 membered heteroaryl is N, and the number of heteroatom(s) is 1 or 2;
each of $R^{1-1}$ and $R^{1-2}$ is independently selected from deuterium, halogen, $C_1$-$C_6$ alkyl unsubstituted or substituted by one or more $R^{1-1-1}$, $C_1$-$C_6$ alkoxy unsubstituted or substituted by one or more $R^{1-1-2}$ or

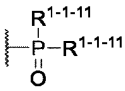;

each of $R^{1-1-1}$ and $R^{1-1-2}$ is independently selected from halogen or deuterium;
each of $R^{1-1-11}$ is independently selected from hydroxy or $C_1$-$C_6$ alkyl;
$R^2$ is independently selected from deuterium or $C_1$-$C_6$ alkyl unsubstituted or substituted by one or more $R^{2-1}$; each of $R^{2-1}$ is independently selected from halogen or deuterium;
m is 0 or 1;
ring B is 5-12 membered fused-ring heteroaromatic ring unsubstituted or substituted by one or more $R^{b-1}$; a heteroatom in the 5-12 membered fused-ring heteroaromatic ring is N and/or S, the number of heteroatom(s) is 1, 2, 3 or 4, and the number of rings in the 5-12 membered fused-ring heteroaromatic ring is 2;
each $R^{b-1}$ is independently selected from deuterium, cyano, halogen, or $C_1$-$C_6$ alkyl unsubstituted or substituted by one or more $R^{b-1-1}$;
each of $R^{b-1-1}$ is independently halogen;
$R^3$ is hydrogen,

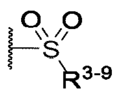,

3-6 membered cycloalkyl unsubstituted or substituted by one or more $R^{3-1}$, 4-9 membered heterocyclyl unsubstituted or substituted by one or more $R^{3-2}$, 4-6 membered cycloalkenyl unsubstituted or substituted by one or more $R^{3-3}$, 4-6 membered heterocycloalkenyl unsubstituted or substituted by one or more $R^{3-4}$, phenyl unsubstituted or substituted by one or more $R^{3-5}$, 5-10 membered heteroaryl unsubstituted or substituted by one or more $R^{3-6}$, -C(O)$R^4$ or $C_1$-$C_6$

alkyl unsubstituted or substituted by one or more $R^{3-7}$ a heteroatom in the 4-6 membered heterocycloalkenyl or 5-10 membered heteroaryl is N and/or O, and the number of heteroatom(s) is 1 or 2; a heteroatom in the 4-9 membered heterocyclyl is one or two selected from N, O, and S, and the number of heteroatom(s) is 1 or 2;
each $R^{3-1}$ is independently selected from deuterium or hydroxy;
each $R^{3-2}$ is independently selected from deuterium, oxo, halogen, $C_1$-$C_6$ alkoxy, $-NR^{3-4-2}R^{3-4-2}$, $-S(O)_2R^{3-4-1}$, hydroxy or $C_1$-$C_6$ alkyl unsubstituted or substituted by one or more $R^{3-2-2}$;
each $R^{3-3}$ is independently selected from deuterium, oxo, amino or hydroxy;
each $R^{3-4}$ is independently selected from deuterium, oxo or $C_1$-$C_6$ alkyl;
$R^{3-4-1}$ is $C_1$-$C_6$ alkyl;
each $R^{3-4-2}$ is independently selected from $-S(O)_2R^{3-4-1}$, hydrogen or $C_1$-$C_6$ alkyl;
each $R^{3-5}$ is independently selected from deuterium,

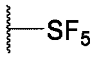 , 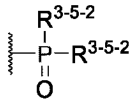 or 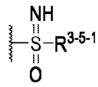 ;

$R^{3-5-1}$ is $C_1$-$C_6$ alkyl;
each $R^{3-6}$ is independently selected from deuterium, halogen, $C_1$-$C_6$ alkyl unsubstituted or substituted by one or more $R^{3-2-2}$ or 4-6 membered heterocyclyl unsubstituted or substituted by one or more $R^{3-6-1}$; a heteroatom in the 4-6 membered heterocyclyl is one or two selected from N, O, and S, and the number of heteroatom(s) is 1 or 2;
each $R^{3-5-2}$ is independently selected from hydroxy, $C_1$-$C_6$ alkyl or 1-10 membered heteroalkyl, or two $R^{3-5-2}$ form 4-10 membered heterocyclyl; a heteroatom, except for phosphorus, in the 1-10 membered heteroalkyl further comprises one or more selected from N, O, and S, and the number of heteroatom(s) is 1, 2, 3 or 4; a heteroatom, except for phosphorus, in the 4-10 membered heterocyclyl further comprises one or more selected from N, O, and S, and the number of heteroatom(s) is 1, 2, 3 or 4;
each $R^{3-6-1}$ is independently $C_1$-$C_6$ alkyl;
each $R^{3-2-2}$ is independently selected from deuterium or hydroxy;
each $R^{3-7}$ is independently selected from deuterium, hydroxy, $-S(O)_2R^{3-4-1}$, or 4-6 membered heterocyclyl; a heteroatom in the 4-6 membered heterocyclyl is O, and the number of heteroatom(s) is 1;
$R^4$ is 4-6 membered heterocyclyl; and a heteroatom in the 4-6 membered heterocyclyl is O, and the number of heteroatom(s) is 1.

In an embodiment,

L is

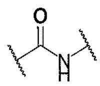 ;

ring A is a 5-10 membered heteroaromatic ring; a heteroatom in the 5-10 membered heteroaromatic ring is N, and the number of heteroatom(s) is 1 or 2;
$R^1$ is 6-10 membered aryl unsubstituted or substituted by one or more $R^{1-1}$ or 5-6 membered heteroaryl unsubstituted or substituted by one or more $R^{1-2}$; a heteroatom in the 5-6 membered heteroaryl is N, and the number of heteroatom(s) is 1;
each of $R^{1-1}$ and $R^{1-2}$ is independently selected from halogen or $C_1$-$C_6$ alkoxy unsubstituted or substituted by one or more $R^{1-1-2}$;
each $R^{1-1-2}$ is independently halogen;
$R^2$ is independently $C_1$-$C_6$ alkyl;
m is 0 or 1;
ring B is a 5-12 membered fused-ring heteroaromatic ring unsubstituted or substituted by one or more $R^{b-1}$; a heteroatom in the 5-12 membered fused-ring heteroaromatic ring is N and/or S, and the number of heteroatom(s) is 1, 2, 3 or 4, a single heteroaryl ring in the 5-12 membered fused-ring heteroaromatic ring is a 5-7 membered heteroaryl ring, and the number of rings in the 5-12 membered fused-ring heteroaromatic ring is 2;
each $R^{b-1}$ is independently halogen;
$R^3$ is hydrogen, 3-6 membered cycloalkyl unsubstituted or substituted by one or more $R^{3-1}$, 4-6 membered heterocyclyl unsubstituted or substituted by one or more $R^{3-2}$, 4-6 membered cycloalkenyl unsubstituted or substituted by one or more $R^{3-3}$, 4-6 membered heterocycloalkenyl unsubstituted or substituted by one or more $R^{3-4}$, phenyl unsubstituted or substituted by one or more $R^{3-5}$, 5-6 membered heteroaryl unsubstituted or substituted by one or more $R^{3-6}$, or -C(O)$R^4$; a heteroatom in the 4-6 membered heterocyclyl, 4-6 membered heterocycloalkenyl or 5-6 membered heteroaryl is N and/or O, and the number of heteroatom(s) is 1 or 2;

each $R^{3-1}$ is independently hydroxy;

each $R^{3-2}$ is independently selected from hydroxy or $C_1$-$C_6$ alkyl;

each $R^{3-3}$ is independently hydroxy;

each $R^{3-4}$ is independently $C_1$-$C_6$ alkyl;

each $R^{3-5}$ is independently

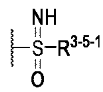 ;

$R^{3-5-1}$ is $C_1$-$C_6$ alkyl;

each $R^{3-6}$ is independently selected from halogen or $C_1$-$C_6$ alkyl;

$R^4$ is 4-6 membered heterocyclyl; and a heteroatom in the 4-6 membered heterocyclyl is O, and the number of heteroatom(s) is 1.

In an embodiment,

L is

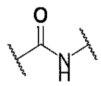 ;

ring A is a 5-10 membered heteroaromatic ring; a heteroatom in the 5-10 membered heteroaromatic ring is N, and the number of heteroatom(s) is 1 or 2;

$R^1$ is 6-10 membered aryl unsubstituted or substituted by one or more $R^{1-1}$ or 5-6 membered heteroaryl unsubstituted or substituted by one or more $R^{1-2}$; a heteroatom in the 5-6 membered heteroaryl is N, and the number of heteroatom(s) is 1;

each of $R^{1-1}$ and $R^{1-2}$ is independently selected from halogen or $C_1$-$C_6$ alkoxy unsubstituted or substituted by one or more $R^{1-1-2}$;

each $R^{1-1-2}$ is independently selected from deuterium or halogen;

each $R^2$ is independently selected from $C_1$-$C_6$ alkane unsubstituted or substituted by one or more $R^{2-1}$;

each $R^{2-1}$ is independently deuterium;

m is 0 or 1;

ring B is a 5-12 membered fused-ring heteroaromatic ring unsubstituted or substituted by one or more $R^{b-1}$; a heteroatom in the 5-12 membered fused-ring heteroaromatic ring is N and/or S, and the number of heteroatom(s) is 1, 2, 3 or 4, a single heteroaryl ring in the 5-12 membered fused-ring heteroaromatic ring is a 5-7 membered heteroaromatic ring, and the number of rings in the 5-12 membered fused-ring heteroaromatic ring is 2;

each $R^{b-1}$ is independently halogen;

$R^3$ is hydrogen, 3-6 membered cycloalkyl unsubstituted or substituted by one or more $R^{3-1}$, 4-6 membered heterocyclyl unsubstituted or substituted by one or more $R^{3-2}$, 4-6 membered cycloalkenyl unsubstituted or substituted by one or more $R^{3-3}$, 4-6 membered heterocycloalkenyl unsubstituted or substituted by one or more $R^{3-4}$, phenyl unsubstituted or substituted by one or more $R^{3-5}$, 5-6 membered heteroaryl unsubstituted or substituted by one or more $R^{3-6}$, or -C(O)$R^4$; a heteroatom in the 4-6 membered heterocyclyl, 4-6 membered heterocycloalkenyl or 5-6 membered heteroaryl is N and/or O, and the number of heteroatom(s) is 1 or 2;

each $R^{3-1}$ is independently selected from hydroxy or deuterium;

each $R^{3-2}$ is independently selected from hydroxy, deuterium or $C_1$-$C_6$ alkyl;

each $R^{3-3}$ is independently selected from hydroxy or deuterium;

each $R^{3-4}$ is independently $C_1$-$C_6$ alkyl;

each $R^{3-5}$ is independently selected from

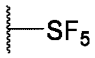, 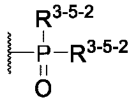 or 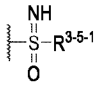;

$R^{3-5-1}$ is $C_1$-$C_6$ alkyl;

each $R^{3-5-2}$ is independently selected from hydroxy, $C_1$-$C_6$ alkyl or 1-10 membered heteroalkyl, or two $R^{3-5-2}$s form 4-10 membered heterocyclyl; a heteroatom, except for phosphorus, in the 1-10 membered heteroalkyl further comprises one or more selected from N, O, and S, and the number of heteroatom(s) is 1, 2, 3 or 4; a heteroatom, except for phosphorus, in the 4-10 membered heterocyclyl further comprises one or more selected from N, O, and S, and the number of heteroatom(s) is 1, 2, 3 or 4;

each $R^{3-6}$ is independently halogen or is unsubstituted or substituted by one or more $R^{3-2-2}$;

each $R^{3-2-2}$ is independently deuterium;

$R^4$ is 4-6 membered heterocyclyl; and a heteroatom in the 4-6 membered heterocyclyl is O, and the number of heteroatom(s) is 1.

In an embodiment,

L is

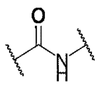;

ring A is a 5-6 membered heteroaromatic ring; a heteroatom in the 5-6 membered heteroaromatic ring is N, and the number of heteroatom(s) is 1 or 2;

$R^1$ is phenyl unsubstituted or substituted by one or more $R^{1-1}$ or 5-6 membered heteroaryl unsubstituted or substituted by one or more $R^{1-2}$; a heteroatom in the 5-6 membered heteroaryl is N, and the number of heteroatom(s) is 1;

each of $R^{1-1}$ and $R^{1-2}$ is independently selected from halogen or $C_1$-$C_6$ alkoxy unsubstituted or substituted by one or more $R^{1-1-2}$;

each $R^{1-1-2}$ is independently halogen;

$R^2$ is independently $C_1$-$C_6$ alkyl;

m is 0 or 1;

ring B is a 5 membered-fused-6 membered heteroaromatic ring (both the 5 membered and the 6 membered rings are aromatic rings); a heteroatom in the 5 membered-fused-6 membered heteroaromatic ring is N and/or S, and the number of heteroatom(s) is 1, 2 or 3;

$R^3$ is hydrogen, 3-6 membered cycloalkyl unsubstituted or substituted by one or more $R^{3-1}$, 4-6 membered heterocyclyl unsubstituted or substituted by one or more $R^{3-2}$ or 4-6 membered cycloalkenyl unsubstituted or substituted by one or more $R^{3-3}$; a heteroatom in the 4-6 membered heterocyclyl is N and/or O, and the number of heteroatom(s) is 1 or 2;

each $R^{3-1}$ is independently hydroxy;

each $R^{3-2}$ is independently selected from hydroxy or $C_1$-$C_6$ alkyl;

each $R^{3-3}$ is independently hydroxyl.

In an embodiment,

L is

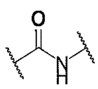;

ring A is a 5-6 membered heteroaromatic ring; a heteroatom in the 5-6 membered heteroaromatic ring is N, and the number of heteroatom(s) is 1;

$R^1$ is phenyl unsubstituted or substituted by one or more $R^{1-1}$ or 5-6 membered heteroaryl unsubstituted or substituted by one or more $R^{1-2}$; a heteroatom in the 5-6 membered heteroaryl is N, and the number of heteroatom(s) is 1;

each of $R^{1-1}$ and $R^{1-2}$ is independently selected from halogen or $C_1$-$C_6$ alkoxy unsubstituted or substituted by one or more $R^{1-1-2}$;

more $R^{1-1-2}$;

each $R^{1-1-2}$ is independently selected from halogen or deuterium;
$R^2$ is independently $C_1$-$C_6$ alkane unsubstituted or substituted by one or more $R^{2-1}$;
each $R^{2-1}$ is independently deuterium;
m is 0 or 1;
ring B is 5 membered-fused-6 membered cycloheteroaromatic ring; a heteroatom in the 5 membered-fused-6 membered heteroaromatic ring is N and/or S, and the number of heteroatom(s) is 1, 2, 3 or 4;
$R^3$ is 3-6 membered cycloalkyl unsubstituted or substituted by one or more $R^{3-1}$, 4-6 membered cycloalkenyl unsubstituted or substituted by one or more $R^{3-3}$, phenyl unsubstituted or substituted by one or more $R^{3-5}$ or 5-6 membered heteroaryl unsubstituted or substituted by one or more $R^{3-6}$; a heteroatom in the 4-6 membered heterocyclyl is N and/or O, and the number of heteroatom(s) is 1 or 2; a heteroatom in the 5-6 membered heteroaromatic ring is N, and the number of heteroatoms is 2;
each $R^{3-1}$ is independently hydroxy;
each $R^{3-3}$ is independently hydroxy;
each $R^{3-5}$ is independently selected from

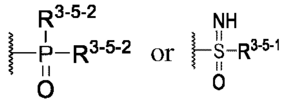 or ;

$R^{3-5-1}$ and $R^{3-5-2}$ are each independently $C_1$-$C_6$ alkyl;
each $R^{3-6}$ is independently halogen or is unsubstituted or substituted by one or more $R^{3-2-2}$; each $R^{3-2-2}$ is independently deuterium.

In an embodiment,

L is

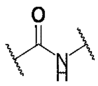 ;

ring A is a 5-6 membered heteroaromatic ring; a heteroatom in the 5-6 membered heteroaromatic ring is N, and the number of heteroatom(s) is 1;
$R^1$ is phenyl unsubstituted or substituted by one or more $R^{1-1}$ or 5-6 membered heteroaryl unsubstituted or substituted by one or more $R^{1-2}$; a heteroatom in the 5-6 membered heteroaryl is N, and the number of heteroatom(s) is 1;
each of $R^{1-1}$ and $R^{1-2}$ is independently selected from halogen or $C_1$-$C_6$ alkoxy unsubstituted or substituted by one or more $R^{1-1-2}$;
each $R^{1-1-2}$ is independently halogen;
$R^2$ is independently $C_1$-$C_6$ alkyl;
m is 0 or 1;
ring B is 5 membered-fused-6 membered cycloheteroaromatic ring; a heteroatom in the 5 membered-fused-6 membered heteroaromatic ring is N and/or S, and the number of heteroatom(s) is 1, 2 or 3;
$R^3$ is 3-6 membered cycloalkyl unsubstituted or substituted by one or more $R^{3-1}$ or 4-6 membered cycloalkenyl unsubstituted or substituted by one or more $R^{3-3}$;
each $R^{3-1}$ is independently hydroxy;
each $R^{3-3}$ is independently hydroxyl.

In an embodiment,

L is

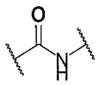 ;

ring A is a 5-6 membered heteroaromatic ring; a heteroatom in the 5-6 membered heteroaromatic ring is N, and the number of heteroatom(s) is 1;

$R^1$ is phenyl unsubstituted or substituted by one or more $R^{1-1}$ or 5-6 membered heteroaryl unsubstituted or substituted by one or more $R^{1-2}$; a heteroatom in the 5-6 membered heteroaryl is N, and the number of heteroatom(s) is 1;
each of $R^{1-1}$ and $R^{1-2}$ is independently selected from halogen or $C_1$-$C_6$ alkoxy unsubstituted or substituted by one or more $R^{1-1-2}$;
each $R^{1-1-2}$ is independently selected from halogen or deuterium;
$R^2$ is independently $C_1$-$C_6$ alkane unsubstituted or substituted by one or more $R^{2-1}$;
each $R^{2-1}$ is independently deuterium;
m is 0 or 1;
ring B is a 5-12 membered fused-ring heteroaromatic ring unsubstituted or substituted by one or more $R^{b-1}$;
the 5-12 membered fused-ring heteroaromatic ring is

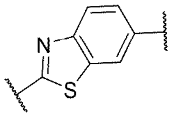, 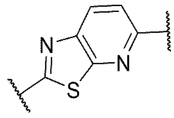, 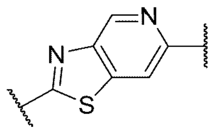,

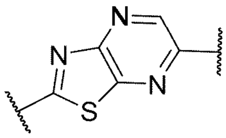, 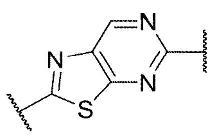, 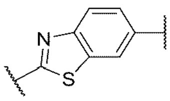, 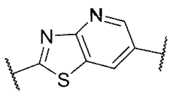, 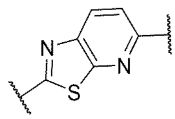;

each $R^{b-1}$ is independently halogen;
$R^3$ is 3-6 membered cycloalkyl unsubstituted or substituted by one or more $R^{3-1}$, 4-6 membered cyclene unsubstituted or substituted by one or more $R^{3-3}$, phenyl unsubstituted or substituted by one or more $R^{3-5}$ or 5-6 membered heteroaryl unsubstituted or substituted by one or more $R^{3-6}$; a heteroatom in the 4-6 membered heterocyclyl is N and/or O, and the number of heteroatom(s) is 1 or 2; a heteroatom in the 5-6 membered heteroaromatic ring is N, and the number of heteroatoms is 2;
each $R^{3-1}$ is independently hydroxy;
each of $R^{3-3}$ is independently hydroxy;
each $R^{3-5}$ is independently selected from

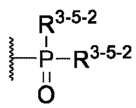 or 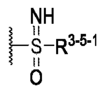;

$R^{3-5-1}$ and $R^{3-5-2}$ are each independently $C_1$-$C_6$ alkyl;
each $R^{3-6}$ is independently halogen or is unsubstituted or substituted by one or more $R^{3-2-2}$;
each $R^{3-2-2}$ is independently deuterium.

In an embodiment,

L is

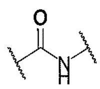;

ring A is a 6-12 membered aromatic ring or a 5-6 membered heteroaromatic ring; a heteroatom in the 5-6 membered heteroaromatic ring is selected from one or two of N, O, and S, and the number of heteroatom(s) is 1, 2, or 3;
$R^1$ is 6-12 membered aryl unsubstituted or substituted by one or more $R^{1-1}$ or 5-6 membered heteroaryl unsubstituted or substituted by one or more $R^{1-2}$; a heteroatom in the 5-6 membered heteroaromatic ring is one or two selected from N, O, and S, and the heteroatom(s) is 1, 2, or 3;
each of $R^{1-1}$ and $R^{1-2}$ is independently selected from halogen or $C_1$-$C_6$ alkoxy;
ring B is a 5-20 membered fused-ring heteroaromatic ring unsubstituted or substituted by one or more $R^{b-1}$; a heteroatom in the 5-20 membered fused-ring heteroaromatic ring is selected from one or more of N, O, and S, and the number of heteroatom(s) is 1, 2, 3, 4, 5, 6, 7 or 8, a single heteroaromatic ring in the 5-20 membered fused-ring

heteroaromatic ring is a 5-7 membered heteroaromatic ring, and the number of rings in the 5-20 membered fused-ring heteroaromatic ring is 3 or 4;
$R^3$ is hydrogen.

In an embodiment,

L is

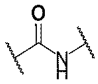
;

ring A is a 5-10 membered heteroaromatic ring; a heteroatom in the 5-10 membered heteroaromatic ring is N, and the number of heteroatom(s) is 1 or 2;
$R^1$ is 6-10 membered aryl unsubstituted or substituted by one or more $R^{1-1}$ or 5-6 membered heteroaryl unsubstituted or substituted by one or more $R^{1-2}$; a heteroatom in the 5-6 membered heteroaryl is N, and the number of heteroatom(s) is 1 or 2;
each of $R^{1-1}$ and $R^{1-2}$ is independently selected from deuterium, halogen, $C_1$-$C_6$ alkyl unsubstituted or substituted by one or more $R^{1-1-1}$, $C_1$-$C_6$ alkoxy unsubstituted or substituted by one or more $R^{1-1-2}$ or

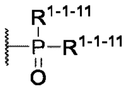
;

each of $R^{1-1-1}$ and $R^{1-1-2}$ is independently selected from halogen or deuterium;
each of $R^{1-1-11}$ is independently selected from hydroxy or $C_1$-$C_6$ alkyl;
$R^2$ is independently selected from deuterium or $C_1$-$C_6$ alkyl unsubstituted or substituted by one or more $R^{2-1}$; each of $R^{2-1}$ is independently selected from halogen or deuterium;
m is 0 or 1;
ring B is a 5-12 membered fused-ring heteroaromatic ring substituted by one or more $R^{b-1}$; a heteroatom in the 5-12 membered fused-ring heteroaromatic ring is N and/or S, the number of heteroatom(s) is 1, 2, 3 or 4, and the number of rings in the 5-12 membered fused-ring heteroaromatic ring is 2;
each $R^{b-1}$ is independently selected from halogen, or $C_1$-$C_6$ alkyl unsubstituted or substituted by one or more $R^{b-1-1}$;
each of $R^{b-1-1}$ is independently halogen;
$R^3$ is hydrogen, 3-6 membered cycloalkyl unsubstituted or substituted by one or more $R^{3-1}$, 4-12 membered heterocyclyl unsubstituted or substituted by one or more $R^{3-2}$, 4-10 membered cycloalkenyl unsubstituted or substituted by one or more $R^{3-3}$, 4-10 membered heterocycloalkenyl unsubstituted or substituted by one or more $R^{3-4}$, 6-10 membered aryl unsubstituted or substituted by one or more $R^{3-5}$, 5-10 membered heteroaryl unsubstituted or substituted by one or more $R^{3-6}$, -C(O)$R^4$, $C_1$-$C_6$ alkyl unsubstituted or substituted by one or more $R^{3-7}$ or -O$R^7$ unsubstituted or substituted by one or more $R^{3-8}$; a heteroatom in the 4-12 membered heterocyclyl or 4-10 membered heterocycloalkenyl is one or more of N, O, and S, and the number of heteroatom(s) is 1 or 2; a heteroatom in the 5-10 membered heteroaryl is N and/or O, and the number of heteroatom(s) is 1, 2, 3 or 4;
each $R^{3-1}$ is independently selected from deuterium or hydroxy;
each $R^{3-2}$ is independently selected from deuterium, oxo, halogen, $C_1$-$C_6$ alkoxy, -N$R^{3-4-2}R^{3-4-2}$, -$S(O)_2R^{3-4-1}$, hydroxy or $C_1$-$C_6$ alkyl unsubstituted or substituted by one or more $R^{3-2-2}$;
each $R^{3-3}$ is independently selected from deuterium, oxo, amino or hydroxy;
each $R^{3-4}$ is independently selected from deuterium, oxo or $C_1$-$C_6$ alkyl;
$R^{3-4-1}$ is $C_1$-$C_6$ alkyl;
each $R^{3-4-2}$ is independently selected from -$S(O)_2R^{3-4-1}$, hydrogen or $C_1$-$C_6$ alkyl;
each $R^{3-5}$ is independently selected from deuterium,

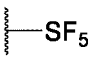
, 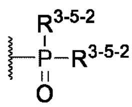
or 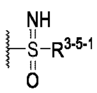
;

$R^{3-5-1}$ is $C_1$-$C_6$ alkyl;
each $R^{3-6}$ is independently selected from deuterium, halogen, $C_1$-$C_6$ alkyl unsubstituted or substituted by one or more

$R^{3-2-2}$ or 4-6 membered heterocyclyl unsubstituted or substituted by one or more $R^{3-6-1}$; a heteroatom in the 4-6 membered heterocyclyl is one or two selected from N, O, and S, and the number of heteroatom(s) is 1 or 2;
each $R^{3-5-2}$ is independently selected from hydroxy, $C_1$-$C_6$ alkyl or 1-10 membered heteroalkyl, or two $R^{3-5-2}$ form 4-10 membered heterocyclyl; a heteroatom, except for phosphorus, in the 1-10 membered heteroalkyl further comprises one or more selected from N, O, and S, and the number of heteroatom(s) is 1, 2, 3 or 4; a heteroatom, except for phosphorus, in the 4-10 membered heterocyclyl further comprises one or more selected from N, O, and S, and the number of heteroatom(s) is 1, 2, 3 or 4;
each $R^{3-6-1}$ is independently $C_1$-$C_6$ alkyl;
each $R^{3-2-2}$ is independently selected from deuterium or hydroxy;
each $R^{3-7}$ is independently selected from deuterium, hydroxy, $-S(O)_2R^{3-1-1}$, or 4-6 membered heterocyclyl; a heteroatom in the 4-6 membered heterocyclyl is O, and the number of heteroatom(s) is 1;
each $R^{3-8}$ is independently selected from deuterium or hydroxy;
$R^4$ is 4-6 membered heterocyclyl; a heteroatom in the 4-6 membered heterocyclyl is O, and the number of heteroatom(s) is 1;
$R^7$ is 3-6 membered cycloalkyl.

In an embodiment,

L is

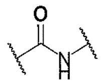;

ring A is a 5-10 membered heteroaromatic ring; a heteroatom in the 5-10 membered heteroaromatic ring is N, and the number of heteroatom(s) is 1 or 2;
$R^1$ is 6-10 membered aryl unsubstituted or substituted by one or more $R^{1-1}$ or 5-6 membered heteroaryl unsubstituted or substituted by one or more $R^{1-2}$; a heteroatom in the 5-6 membered heteroaryl is N, and the number of heteroatom(s) is 1 or 2;
each of $R^{1-1}$ and $R^{1-2}$ is independently selected from deuterium, halogen, $C_1$-$C_6$ alkyl unsubstituted or substituted by one or more $R^{1-1-1}$, or $C_1$-$C_6$ alkoxy unsubstituted or substituted by one or more $R^{1-1-2}$;
each of $R^{1-1-1}$ and $R^{1-1-2}$ is independently selected from halogen or deuterium;
each of $R^{1-1-11}$ is independently selected from hydroxy or $C_1$-$C_6$ alkyl;
$R^2$ is independently selected from deuterium or $C_1$-$C_6$ alkyl unsubstituted or substituted by one or more $R^{2-1}$; each of $R^{2-1}$ is independently selected from halogen or deuterium;
m is 0 or 1;
ring B is a 5-12 membered fused-ring heteroaromatic ring substituted by one or more $R^{b-1}$; a heteroatom in the 5-12 membered fused-ring heteroaromatic ring is N and/or S, the number of heteroatom(s) is 1, 2, 3 or 4, and the number of rings in the 5-12 membered fused-ring heteroaromatic ring is 2;
each $R^{b-1}$ is independently selected from halogen, or $C_1$-$C_6$ alkyl unsubstituted or substituted by one or more $R^{b-1-1}$; each of $R^{b-1-1}$ is independently halogen;
$R^3$ is hydrogen, 3-6 membered cycloalkyl unsubstituted or substituted by one or more $R^{3-1}$, 4-12 membered heterocyclyl unsubstituted or substituted by one or more $R^{3-2}$, 4-10 membered cycloalkenyl unsubstituted or substituted by one or more $R^{3-3}$, 4-10 membered heterocycloalkenyl unsubstituted or substituted by one or more $R^{3-4}$, 6-10 membered aryl unsubstituted or substituted by one or more $R^{3-5}$, 5-10 membered heteroaryl unsubstituted or substituted by one or more $R^{3-6}$, $-C(O)R^4$, $C_1$-$C_6$ alkyl unsubstituted or substituted by one or more $R^{3-7}$ or $-OR^7$ unsubstituted or substituted by one or more $R^{3-8}$; a heteroatom in the 4-12 membered heterocyclyl or 4-10 membered heterocycloalkenyl is one or more of N, O, and S, and the number of heteroatom(s) is 1 or 2; a heteroatom in the 5-10 membered heteroaryl is N and/or O, and the number of heteroatom(s) is 1, 2, 3 or 4;
each $R^{3-1}$ is independently selected from deuterium or hydroxy;
each $R^{3-2}$ is independently selected from deuterium, oxo, halogen, $C_1$-$C_6$ alkoxy, $-NR^{3-4-2}R^{3-4-2}$, $-S(O)_2R^{3-4-1}$, hydroxy or $C_1$-$C_6$ alkyl unsubstituted or substituted by one or more $R^{3-2-2}$;
each $R^{3-3}$ is independently selected from deuterium, oxo, amino or hydroxy;
each $R^{3-4}$ is independently selected from deuterium, oxo or $C_1$-$C_6$ alkyl;
$R^{3-4-1}$ is $C_1$-$C_6$ alkyl;
each $R^{3-4-2}$ is independently selected from $-S(O)_2R^{3-4-1}$, hydrogen or $C_1$-$C_6$ alkyl;
each $R^{3-5}$ is independently selected from deuterium,

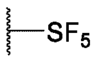, 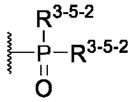 or 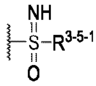;

$R^{3-5-1}$ is $C_1$-$C_6$ alkyl;
each $R^{3-6}$ is independently selected from deuterium, halogen, $C_1$-$C_6$ alkyl unsubstituted or substituted by one or more $R^{3-2-2}$ or 4-6 membered heterocyclyl unsubstituted or substituted by one or more $R^{3-6-1}$; a heteroatom in the 4-6 membered heterocyclyl is one or two selected from N, O, and S, and the number of heteroatom(s) is 1 or 2;
each $R^{3-5-2}$ is independently selected from hydroxy, $C_1$-$C_6$ alkyl or 1-10 membered heteroalkyl, or two $R^{3-5-2}$s form 4-10 membered heterocyclyl; a heteroatom, except for phosphorus, in the 1-10 membered heteroalkyl further comprises one or more selected from N, O, and S, and the number of heteroatom(s) is 1, 2, 3 or 4; a heteroatom, except for phosphorus, in the 4-10 membered heterocyclyl further comprises one or more selected from N, O, and S, and the number of heteroatom(s) is 1, 2, 3 or 4;
each $R^{3-6-1}$ is independently $C_1$-$C_6$ alkyl;
each $R^{3-2-2}$ is independently selected from deuterium or hydroxy;
each $R^{3-7}$ is independently selected from deuterium, hydroxy, -$S(O)_2R^{3-4-1}$, or 4-6 membered heterocyclyl; a heteroatom in the 4-6 membered heterocyclyl is O, and the number of heteroatom(s) is 1;
each $R^{3-8}$ is independently selected from deuterium or hydroxy;
$R^4$ is 4-6 membered heterocyclyl; a heteroatom in the 4-6 membered heterocyclyl is O, and the number of heteroatom(s) is 1;
$R^7$ is 3-6 membered cycloalkyl.

In an embodiment,

L is

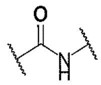;

ring A is a 5-6 membered heteroaromatic ring; a heteroatom in the 5-6 membered heteroaromatic ring is N, and the number of heteroatom(s) is 1;
$R^1$ is 5-6 membered heteroaryl unsubstituted or substituted by one or more $R^{1-2}$; a heteroatom in the 5-6 membered heteroaryl is N, and the number of heteroatom(s) is 1 or 2;
each $R^{1-2}$ is independently selected from deuterium, halogen, $C_1$-$C_6$ alkyl unsubstituted or substituted by one or more $R^{1-1-1}$, $C_1$-$C_6$ alkoxy unsubstituted or substituted by one or more $R^{1-1-2}$ or

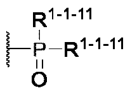;

each of $R^{1-1-1}$ and $R^{1-1-2}$ is independently selected from halogen or deuterium;
each of $R^{1-1-11}$ is independently selected from hydroxy or $C_1$-$C_6$ alkyl;
$R^2$ is independently selected from deuterium or $C_1$-$C_6$ alkyl unsubstituted or substituted by one or more $R^{2-1}$; each of $R^{2-1}$ is independently selected from halogen or deuterium;
m is 0 or 1;
ring B is a 5-12 membered fused-ring heteroaromatic ring substituted by one or more $R^{b-1}$; a heteroatom in the 5-12 membered fused-ring heteroaromatic ring is N and/or S, the number of heteroatom(s) is 1, 2, 3 or 4, and the number of rings in the 5-12 membered fused-ring heteroaromatic ring is 2;
each $R^{b-1}$ is independently selected from halogen, or $C_1$-$C_6$ alkyl unsubstituted or substituted by one or more $R^{b-1-1}$; each of $R^{b-1-1}$ is independently halogen;
$R^3$ is 3-6 membered cycloalkyl unsubstituted or substituted by one or more $R^{3-1}$, 4-9 membered heterocyclyl unsubstituted or substituted by one or more $R^{3-2}$, 4-6 membered cycloalkenyl unsubstituted or substituted by one or more $R^{3-3}$, 4-6 membered heterocycloalkenyl unsubstituted or substituted by one or more $R^{3-4}$, phenyl unsubstituted or substituted by one or more $R^{3-5}$, or 5-10 membered heteroaryl unsubstituted or substituted by one or more $R^{3-6}$; a heteroatom in the 4-9 membered heterocyclyl or 4-6 membered heterocycloalkenyl is N and/or O, and the number of heteroatom(s) is 1 or 2; a heteroatom in the 5-10 membered heteroaryl is selected from N, and the number

of heteroatom(s) is 1, 2, 3 or 4;
each $R^{3-1}$ is independently selected from deuterium or hydroxy;
each $R^{3-2}$ is independently selected from deuterium, oxo, halogen, $C_1$-$C_6$ alkoxy, -$NR^{3-4-2}R^{3-4-2}$, -$S(O)_2R^{3-4-1}$, hydroxy or $C_1$-$C_6$ alkyl unsubstituted or substituted by one or more $R^{3-2-2}$;
each $R^{3-3}$ is independently selected from deuterium, oxo, amino or hydroxy;
each $R^{3-4}$ is independently selected from deuterium, oxo or $C_1$-$C_6$ alkyl;
$R^{3-4-1}$ is $C_1$-$C_6$ alkyl;
each $R^{3-4-2}$ is independently selected from -$S(O)_2R^{3-4-1}$, hydrogen or $C_1$-$C_6$ alkyl;
each $R^{3-5}$ is independently selected from deuterium,

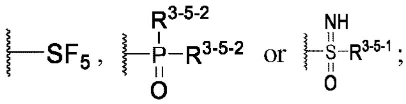

$R^{3-5-1}$ is $C_1$-$C_6$ alkyl;
each $R^{3-6}$ is independently selected from deuterium, halogen, $C_1$-$C_6$ alkyl unsubstituted or substituted by one or more $R^{3-2-2}$ or 4-6 membered heterocyclyl unsubstituted or substituted by one or more $R^{3-6-1}$; a heteroatom in the 4-6 membered heterocyclyl is one or two selected from N, O, and S, and the number of heteroatom(s) is 1 or 2;
each $R^{3-5-2}$ is independently selected from hydroxy, $C_1$-$C_6$ alkyl or 1-10 membered heteroalkyl, or two $R^{3-5-2}$ form 4-10 membered heterocyclyl; a heteroatom, except for phosphorus, in the 1-10 membered heteroalkyl further comprises one or more selected from N, O, and S, and the number of heteroatom(s) is 1, 2, 3 or 4; a heteroatom, except for phosphorus, in the 4-10 membered heterocyclyl further comprises one or more selected from N, O, and S, and the number of heteroatom(s) is 1, 2, 3 or 4;
each $R^{3-6-1}$ is independently $C_1$-$C_6$ alkyl;
each $R^{3-2-2}$ is independently selected from deuterium or hydroxyl.

In an embodiment,

L is

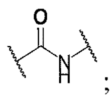

ring A is a 5-6 membered heteroaromatic ring; a heteroatom in the 5-6 membered heteroaromatic ring is N, and the number of heteroatom(s) is 1;
$R^1$ is 5-6 membered heteroaryl unsubstituted or substituted by one or more $R^{1-2}$; a heteroatom in the 5-6 membered heteroaryl is N, and the number of heteroatom(s) is 1 or 2;
each $R^{1-2}$ is independently selected from deuterium, halogen, $C_1$-$C_6$ alkyl unsubstituted or substituted by one or more $R^{1-1-1}$, or $C_1$-$C_6$ alkoxy unsubstituted or substituted by one or more $R^{1-1-2}$;
each of $R^{1-1-1}$ and $R^{1-1-2}$ is independently selected from halogen or deuterium;
each of $R^{1-1-11}$ is independently selected from hydroxy or $C_1$-$C_6$ alkyl;
$R^2$ is independently selected from deuterium or $C_1$-$C_6$ alkyl unsubstituted or substituted by one or more $R^{2-1}$; each of $R^{2-1}$ is independently selected from halogen or deuterium;
m is 0 or 1;
ring B is a 5-12 membered fused-ring heteroaromatic ring substituted by one or more $R^{b-1}$; a heteroatom in the 5-12 membered fused-ring heteroaromatic ring is N and/or S, the number of heteroatom(s) is 1, 2, 3 or 4, and the number of rings in the 5-12 membered fused-ring heteroaromatic ring is 2;
each $R^{b-1}$ is independently selected from halogen, or $C_1$-$C_6$ alkyl unsubstituted or substituted by one or more $R^{b-1-1}$; each of $R^{b-1-1}$ is independently halogen;
$R^3$ is 3-6 membered cycloalkyl unsubstituted or substituted by one or more $R^{3-1}$, 4-9 membered heterocyclyl unsubstituted or substituted by one or more $R^{3-2}$, 4-6 membered cycloalkenyl unsubstituted or substituted by one or more $R^{3-3}$, 4-6 membered heterocycloalkenyl unsubstituted or substituted by one or more $R^{3-4}$, phenyl unsubstituted or substituted by one or more $R^{3-5}$, or 5-10 membered heteroaryl unsubstituted or substituted by one or more $R^{3-6}$; a heteroatom in the 4-9 membered heterocyclyl or 4-6 membered heterocycloalkenyl is N and/or O, and the number of heteroatom(s) is 1 or 2; a heteroatom in the 5-10 membered heteroaryl is selected from N, and the number of heteroatom(s) is 1, 2, 3 or 4;

each $R^{3-1}$ is independently selected from deuterium or hydroxy;
each $R^{3-2}$ is independently selected from deuterium, oxo, halogen, $C_1$-$C_6$ alkoxy, $-NR^{3-4-2}R^{3-4-2}$, $-S(O)_2R^{3-4-1}$, hydroxy or $C_1$-$C_6$ alkyl unsubstituted or substituted by one or more $R^{3-2-2}$;
each $R^{3-3}$ is independently selected from deuterium, oxo, amino or hydroxy;
each $R^{3-4}$ is independently selected from deuterium, oxo or $C_1$-$C_6$ alkyl;
$R^{3-4-1}$ is $C_1$-$C_6$ alkyl;
each $R^{3-4-2}$ is independently selected from $-S(O)_2R^{3-4-1}$, hydrogen or $C_1$-$C_6$ alkyl;
each $R^{3-5}$ is independently selected from deuterium,

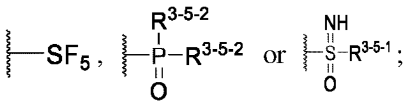

$R^{3-5-1}$ is $C_1$-$C_6$ alkyl;
each $R^{3-6}$ is independently selected from deuterium, halogen, $C_1$-$C_6$ alkyl unsubstituted or substituted by one or more $R^{3-2-2}$ or 4-6 membered heterocyclyl unsubstituted or substituted by one or more $R^{3-6-1}$; a heteroatom in the 4-6 membered heterocyclyl is one or two selected from N, O, and S, and the number of heteroatom(s) is 1 or 2;
each $R^{3-5-2}$ is independently selected from hydroxy, $C_1$-$C_6$ alkyl or 1-10 membered heteroalkyl, or two $R^{3-5-2}$ form 4-10 membered heterocyclyl; a heteroatom, except for phosphorus, in the 1-10 membered heteroalkyl further comprises one or more selected from N, O, and S, and the number of heteroatom(s) is 1, 2, 3 or 4; a heteroatom, except for phosphorus, in the 4-10 membered heterocyclyl further comprises one or more selected from N, O, and S, and the number of heteroatom(s) is 1, 2, 3 or 4;
each $R^{3-6-1}$ is independently $C_1$-$C_6$ alkyl;
each $R^{3-2-2}$ is independently selected from deuterium or hydroxyl.

In an embodiment,

m is 0 or 1; n is 1;
L is

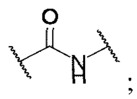

ring A is a 5-6 membered heteroaromatic ring; a heteroatom in the 5-6 membered heteroaromatic ring is N, and the number of heteroatom(s) is 1;
$R^1$ is 5-6 membered heteroaryl unsubstituted or substituted by one or more $R^{1-2}$; a heteroatom in the 5-6 membered heteroaryl is N, and the number of heteroatom(s) is 1, 2 or 3;
each $R^{1-2}$ is independently selected from deuterium, halogen, cyano, amino, hydroxy, $C_1$-$C_6$ alkyl unsubstituted or substituted by one or more $R^{1-1-1}$, $C_1$-$C_6$ alkoxy unsubstituted or substituted by one or more $R^{1-1-2}$, $C_2$-$C_6$ alkenyl unsubstituted or substituted by one or more $R^{1-1-3}$, or $C_2$-$C_6$ alkynyl unsubstituted or substituted by one or more $R^{1-1-4}$;
each of $R^{1-1-1}$ $R^{1-1-2}$, $R^{1-1-3}$ and $R^{1-1-4}$ is independently selected from deuterium, hydroxy, cyano or halogen; each $R^2$ is independently selected from deuterium, halogen, cyano, amino, hydroxy, $C_1$-$C_6$ alkyl unsubstituted or substituted by one or more $R^{2-1}$, $C_1$-$C_6$ alkoxy unsubstituted or substituted by one or more $R^{2-2}$, $C_2$-$C_6$ alkenyl unsubstituted or substituted by one or more $R^{2-3}$, $C_2$-$C_6$ alkynyl unsubstituted or substituted by one or more $R^{2-4}$, 3-6 membered cycloalkyl unsubstituted or substituted by one or more $R^{2-5}$ or 4-6 membered heterocyclyl unsubstituted or substituted by one or more $R^{2-6}$; a heteroatom in the 4-12 membered heterocyclyl is one or more selected from N, and the number of heteroatom(s) is 1, 2, or 3;
each of $R^{2-1}$, $R^{2\ 2}$, $R^{2-3}$, $R^{2-4}$, $R^{2-5}$ and $R^{2-6}$ is independently selected from deuterium, hydroxy, cyano or halogen;
ring B is a 5-20 membered fused-ring heteroaromatic ring unsubstituted or substituted by one or more $R^{b-1}$; a heteroatom in the 5-20 membered fused-ring heteroaromatic ring is one or more selected from N, O, and S, the number of heteroatom(s) is 1, 2, 3, 4, 5, 6, 7 or 8, a single heteroaromatic ring in the 5-20 membered fused-ring heteroaromatic ring is a 5-7 membered heteroaromatic ring, and the number of rings in the 5-20 membered fused-ring heteroaromatic ring is 2 or 3; and when the number of rings in the 5-20 membered fused-ring heteroaromatic ring is 2, ring B is a 5-20 membered fused-ring heteroaromatic ring substituted by one or more $R^{b-1}$;
each $R^{b-1}$ is independently selected from halogen, or $C_1$-$C_6$ alkyl unsubstituted or substituted by one or more $R^{b-1-1}$;
each $R^{b-1-1}$ is independently selected from hydroxy or halogen;

$R^3$ is hydrogen, 3-12 membered cycloalkyl unsubstituted or substituted by one or more $R^{3-1}$, 4-12 membered heterocyclyl unsubstituted or substituted by one or more $R^{3-2}$, 4-12 membered cycloalkenyl unsubstituted or substituted by one or more $R^{3-3}$, 4-12 membered heterocycloalkenyl unsubstituted or substituted by one or more $R^{3-4}$, 6-10 membered aryl unsubstituted or substituted by one or more $R^{3-5}$, 5-10 membered heteroaryl unsubstituted or substituted by one or more $R^{3-6}$, -C(O)$R^4$, $C_1$-$C_6$ alkyl unsubstituted or substituted by one or more $R^{3-7}$ or -O$R^7$ unsubstituted or substituted by one or more $R^{3-8}$; a heteroatom in the 4-12 membered heterocyclyl, 4-12 membered heterocycloalkenyl or 5-10 membered heteroaryl is one or more selected from N, O, and S, and the number of heteroatom(s) is 1, 2, 3 or 4;

each of $R^{3-1}$, $R^{3-2}$, $R^{3-3}$ and $R^{3-4}$ is independently selected from deuterium, hydroxy, halogen, cyano, amino, -NHC(O)$R^{3-2-1}$, $C_1$-$C_6$ alkyl unsubstituted or substituted by one or more $R^{3-2-2}$, $C_1$-$C_6$ alkoxy, $C_2$-$C_6$ alkenyl, $C_2$-$C_6$ alkynyl, oxo(=O), -C(O)$NH_2$, 3-6 membered cycloalkyl unsubstituted or substituted by one or more $R^{3-2-3}$, 4-6 membered heterocyclyl, -S(O)$_2R^{3-4-1}$, -N$R^{3-4-2}R^{3-4-2}$,

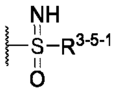 or 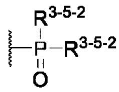 ;

; a heteroatom in the 4-6 membered heterocyclyl is one or more selected from N, O, and S, and the number of heteroatom(s) is 1 or 2; a heteroatom in the 5-6 membered heteroaryl is N, and the number of heteroatom(s) is 1 or 2;

each $R^{3-7}$ is independently selected from deuterium, hydroxy, halogen, cyano or amino;

each $R^{3-8}$ is independently selected from hydroxy or 4-6 membered heterocyclyl;

each of $R^{3-5}$ and $R^{3-6}$ is independently selected from deuterium,

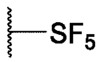 ,

hydroxy, halogen, cyano, amino, -NHC(O)$R^{3-2-1}$, $C_1$-$C_6$ alkyl unsubstituted or substituted by one or more $R^{3-2-2}$, $C_2$-$C_6$ alkenyl, $C_2$-$C_6$ alkynyl, -C(O)$NH_2$, 3-6 membered cycloalkyl unsubstituted or substituted by one or more $R^{3-2-3}$, 4-6 membered heterocyclyl unsubstituted or substituted by one or more $R^{3-6-1}$,

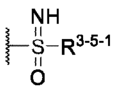 or 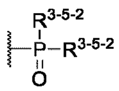 ;

a heteroatom in the 4-6 membered heterocyclyl is one or more selected from N, O, and S, and the number of heteroatom(s) is 1 or 2;

each of $R^{3-2-1}$ and $R^{3-4-2}$ is independently selected from hydrogen or $C_1$-$C_6$ alkyl;

each of $R^{3-2-3}$ and $R^{3-5-1}$ is independently selected from hydroxy or $C_1$-$C_6$ alkyl;

each of $R^{3-5-2}$ is independently selected from hydroxy, $C_1$-$C_6$ alkyl or 1-10 membered heteroalkyl, or two $R^{3-5-2}$s form 4-10 membered heterocyclyl; a heteroatom, except for phosphorus, in the 1-10 membered heteroalkyl further comprises one or more selected from N, O, and S, and the number of heteroatom(s) is 1, 2, 3 or 4; a heteroatom, except for phosphorus, in the 4-10 membered heterocyclyl further comprises one or more of N, O, and S, and the number of heteroatom(s) is 1, 2, 3 or 4;

each of $R^{3-6-1}$ is independently $C_1$-$C_6$ alkyl;

each $R^{3-2-2}$ is independently selected from deuterium, hydroxy or halogen;

$R^{3-4-1}$ is hydroxy, amino or $C_1$-$C_6$ alkyl;

$R^4$ is 4-6 membered heterocyclyl unsubstituted or substituted by one or more $R^{4-1}$; a heteroatom in the 4-6 membered heterocyclyl is selected from N, and the number of heteroatom(s) is 1 or 2;

each of $R^{4-1}$ is independently hydroxy;

$R^7$ is 3-6 membered cycloalkyl.

In an embodiment,

m is 0 or 1; n is 1;

L is

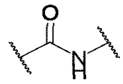;

ring A is a 5-6 membered heteroaromatic ring; a heteroatom in the 5-6 membered heteroaromatic ring is N, and the number of heteroatom(s) is 1;

$R^1$ is 5-6 membered heteroaryl unsubstituted or substituted by one or more $R^{1-2}$; a heteroatom in the 5-6 membered heteroaryl is N, and the number of heteroatom(s) is 1;

each $R^{1-2}$ is independently selected from deuterium, halogen or $C_1$-$C_6$ alkoxy unsubstituted or substituted by one or more $R^{1-1-2}$;

each $R^{1-1-2}$ is independently selected from deuterium, hydroxy, cyano or halogen;

each $R^2$ is independently selected from deuterium, or $C_1$-$C_6$ alkyl unsubstituted or substituted by one or more $R^{2-1}$;

each $R^{2-1}$ is independently selected from deuterium, hydroxy, cyano or halogen;

ring B is a 5-20 membered fused-ring heteroaromatic ring unsubstituted or substituted by one or more $R^{b-1}$; a heteroatom in the 5-20 membered fused-ring heteroaromatic ring is one or more selected from N, O, and S, the number of heteroatom(s) is 1, 2, 3, 4, 5, 6, 7 or 8, a single heteroaromatic ring in the 5-20 membered fused-ring heteroaromatic ring is a 5-7 membered heteroaromatic ring, and the number of rings in the 5-20 membered fused-ring heteroaromatic ring is 2 or 3; and when the number of rings in the 5-20 membered fused-ring heteroaromatic ring is 2, ring B is a 5-20 membered fused-ring heteroaromatic ring substituted by one or more $R^{b-1}$;

each $R^{b-1}$ is independently selected from halogen or $C_1$-$C_6$ alkyl;

$R^3$ is 3-12 membered cycloalkyl unsubstituted or substituted by one or more $R^{3-1}$, 4-12 membered heterocyclyl unsubstituted or substituted by one or more $R^{3-2}$, 4-12 membered cycloalkenyl unsubstituted or substituted by one or more $R^{3-3}$, 4-12 membered heterocycloalkenyl unsubstituted or substituted by one or more $R^{3-4}$, 6-10 membered aryl unsubstituted or substituted by one or more $R^{3-5}$, 5-10 membered heteroaryl unsubstituted or substituted by one or more $R^{3-6}$, or $-OR^7$ unsubstituted or substituted by one or more $R^{3-8}$; a heteroatom in each of the 4-12 membered heterocyclyl, the 4-12 membered heterocycloalkenyl and the 5-10 membered heteroaryl is one or more selected from N, O, and S, and the number of heteroatom(s) is 1, 2, 3 or 4;

each of $R^{3-1}$, $R^{3-2}$, $R^{3-3}$ and $R^{3-4}$ is independently selected from deuterium, hydroxy, halogen, cyano, amino, $-NHC(O)R^{3-2-1}$, $C_1$-$C_6$ alkyl unsubstituted or substituted by one or more $R^{3-2-2}$, $C_1$-$C_6$ alkoxy, $C_2$-$C_6$ alkenyl, $C_2$-$C_6$ alkynyl, oxo(=O),

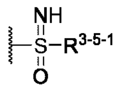 or 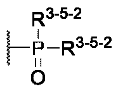;

each $R^{3-8}$ is independently selected from hydroxy or 4-6 membered heterocyclyl;

each of $R^{3-5}$ and $R^{3-6}$ is independently selected from deuterium,

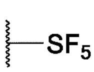,

hydroxy, halogen, cyano, amino, $C_1$-$C_6$ alkyl unsubstituted or substituted by one or more $R^{3-2-2}$, $C_2$-$C_6$ alkenyl, $C_2$-$C_6$ alkynyl, $-C(O)NH_2$, 3-6 membered cycloalkyl unsubstituted or substituted by one or more $R^{3-2-3}$, 4-6 membered heterocyclyl unsubstituted or substituted by one or more $R^{3-6-1}$,

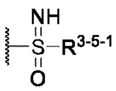 or 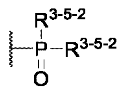;

a heteroatom in the 4-6 membered heterocyclyl is one or more selected from N, O, and S, and the number of heteroatom(s) is 1 or 2; each $R^{3-2-1}$ is independently selected from hydrogen or $C_1$-$C_6$ alkyl;

each of $R^{3-2-3}$ and $R^{3-5-1}$ is independently selected from hydroxy or $C_1$-$C_6$ alkyl;

each of $R^{3-5-2}$ is independently selected from hydroxy, $C_1$-$C_6$ alkyl or 1-10 membered heteroalkyl, or two $R^{3-5-2}$ form 4-10 membered heterocyclyl; a heteroatom, except for phosphorus, in the 1-10 membered heteroalkyl further comprises one or more selected from N, O, and S, and the number of heteroatom(s) is 1, 2, 3 or 4; a heteroatom, except for phosphorus, in the 4-10 membered heterocyclyl further comprises one or more of N, O, and S, and the number of heteroatom(s) is 1, 2, 3 or 4;

each of $R^{3-6-1}$ is independently $C_1$-$C_6$ alkyl;

each $R^{3-2-2}$ is independently selected from deuterium, hydroxy or halogen;
$R^7$ is 3-6 membered cycloalkyl.

In an embodiment,

m is 0 or 1; n is 1;
L is

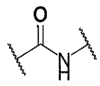;

ring A is a 5-6 membered heteroaromatic ring; a heteroatom in the 5-6 membered heteroaromatic ring is N, and the number of heteroatom(s) is 1;
$R^1$ is 5-6 membered heteroaryl unsubstituted or substituted by one or more $R^{1-2}$; a heteroatom in the 5-6 membered heteroaryl is N, and the number of heteroatom(s) is 1;
each $R^{1-2}$ is independently selected from deuterium, halogen, or $C_1$-$C_6$ alkoxy unsubstituted or substituted by one or more $R^{1-1-2}$;
each $R^{1-1-2}$ is independently selected from deuterium, hydroxy, cyano or halogen;
each $R^2$ is independently selected from deuterium, or $C_1$-$C_6$ alkyl unsubstituted or substituted by one or more $R^{2-1}$;
each $R^{2-1}$ is independently selected from deuterium, hydroxy, cyano or halogen;
ring B is 5-12 membered fused-ring heteroaromatic ring substituted by one or more $R^{b-1}$, a heteroatom in the 5-12 membered fused-ring heteroaromatic ring is selected from N and S, and the number of heteroatom(s) is 1, 2, 3 or 4, (for example,

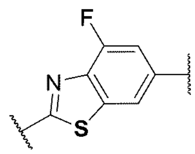, 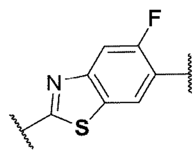, 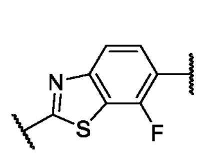, 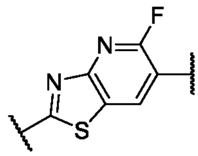,

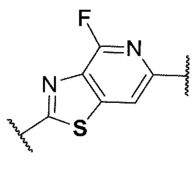, 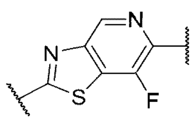 or 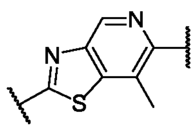

);
each $R^{b-1}$ is independently selected from halogen or $C_1$-$C_6$ alkyl (for example, fluorine or methyl);
$R^3$ is 3-6 membered cycloalkyl unsubstituted or substituted by one or more $R^{3-1}$, 4-6 membered heterocyclyl unsubstituted or substituted by one or more $R^{3-2}$, 4-6 membered cycloalkenyl unsubstituted or substituted by one or more $R^{3-3}$, 4-6 membered heterocycloalkenyl unsubstituted or substituted by one or more $R^{3-4}$, phenyl unsubstituted or substituted by one or more $R^{3-5}$, or 5-6 membered heteroaryl unsubstituted or substituted by one or more $R^{3-6}$; a heteroatom in the 4-6 membered heterocyclyl or 4-6 membered heterocycloalkenyl is N and/or O, and the number of heteroatom(s) is 1 or 2; a heteroatom in the 5-6 membered heteroaryl is N, and the number of heteroatom(s) is 1, 2 or 3;
each of $R^{3-1}$, $R^{3-2}$, $R^{3-3}$ and $R^{3-4}$ is independently selected from deuterium, hydroxy or $C_1$-$C_6$ alkyl;
each $R^{3-5}$ is independently selected from

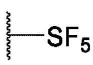, 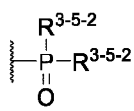 or 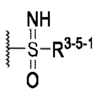;

$R^{3-5-1}$ is $C_1$-$C_6$ alkyl;
each of $R^{3-5-2}$ is independently selected from hydroxy or $C_1$-$C_6$ alkyl;
each $R^{3-6}$ is independently selected from halogen, or $C_1$-$C_6$ alkoxy unsubstituted or substituted by one or more $R^{3-2-2}$;

and
each $R^{3-2-2}$ is independently deuterium.

In an embodiment, $R^3$ is hydrogen, 3-6 membered cycloalkyl unsubstituted or substituted by one or more $R^{3-1}$, 4-12 membered heterocyclyl unsubstituted or substituted by one or more $R^{3-2}$, 4-10 membered cycloalkenyl unsubstituted or substituted by one or more $R^{3-3}$, 4-10 membered heterocycloalkenyl unsubstituted or substituted by one or more $R^{3-4}$, 6-10 membered aryl unsubstituted or substituted by one or more $R^{3-5}$, 5-10 membered heteroaryl unsubstituted or substituted by one or more $R^{3-6}$, or -C(O)$R^4$; a heteroatom in the 4-12 membered heterocyclyl, 4-10 membered heterocycloalkenyl or 5-10 membered heteroaryl is one or more of N, O and S, and the number of heteroatom(s) is 1, 2, 3 or 4.

In an embodiment, $R^3$ is hydrogen, 3-6 membered cycloalkyl unsubstituted or substituted by one or more $R^{3-1}$, 4-12 membered heterocyclyl unsubstituted or substituted by one or more $R^{3-2}$, 4-10 membered cycloalkenyl unsubstituted or substituted by one or more $R^{3-3}$, 4-10 membered heterocycloalkenyl unsubstituted or substituted by one or more $R^{3-4}$, 6-10 membered aryl unsubstituted or substituted by one or more $R^{3-5}$, 5-10 membered heteroaryl unsubstituted or substituted by one or more $R^{3-6}$, -C(O)$R^4$, -NH$(CH_2)_pR^5$, -O$(CH_2)_qR^6$, $C_1$-$C_6$ alkyl unsubstituted or substituted by one or more $R^{3-7}$, or -O$R^7$ unsubstituted or substituted by one or more $R^{3-8}$; a heteroatom in the 4-12 membered heterocyclyl, 4-10 membered heterocycloalkenyl or 5-10 membered heteroaryl is one or more of N, O, and S, and the number of heteroatom(s) is 1, 2, 3 or 4.

In an embodiment, $R^1$ is

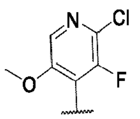 , 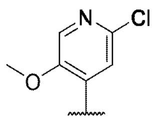 , 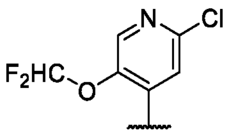 , 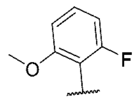 ,

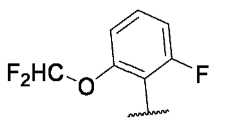 , 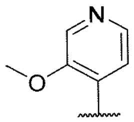 , 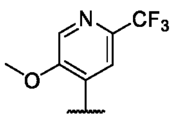 , 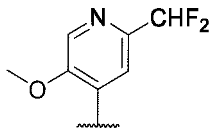 , 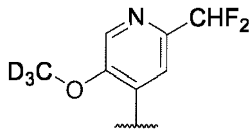 ,

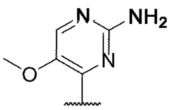 , 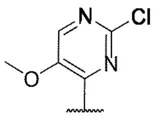 , 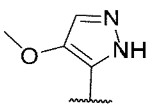 , 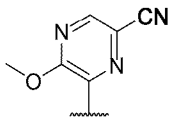 , 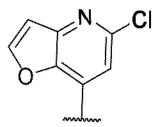 , 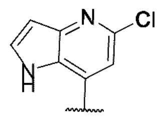 ,

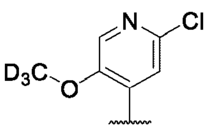 , 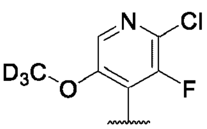 , 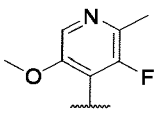 or 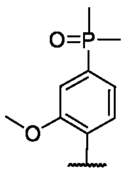 .

In an embodiment,

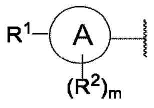

is

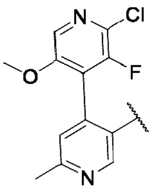 , 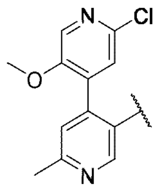 , 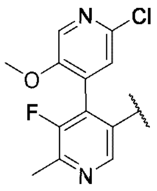 , 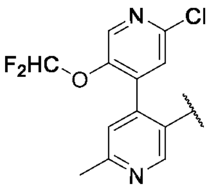 ,

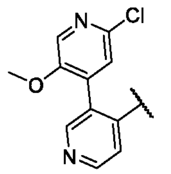 , 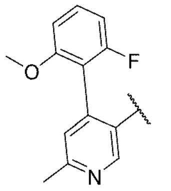 , 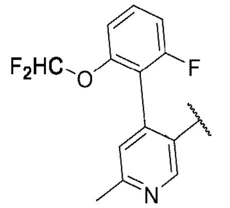 , 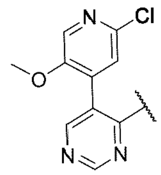 , 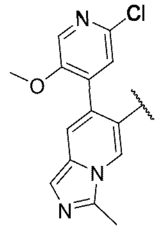 , 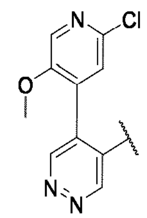 ,

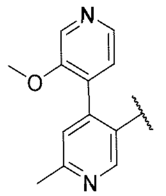 , 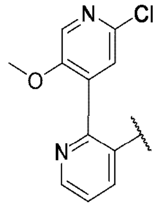 , 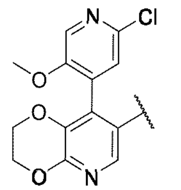 , 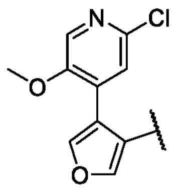 , 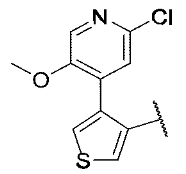 , 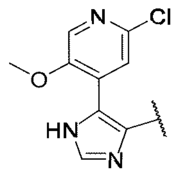 ,

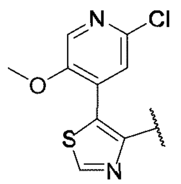 , 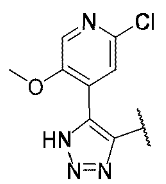 , 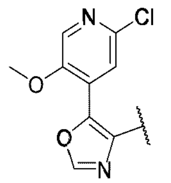 , 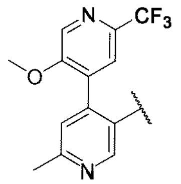 , 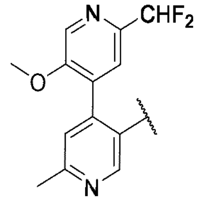 , 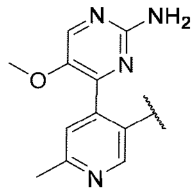 ,

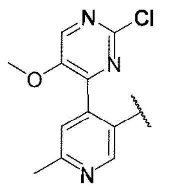 , 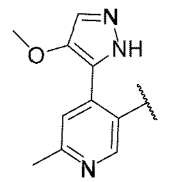 , 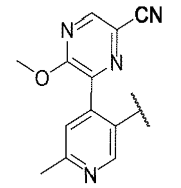 , 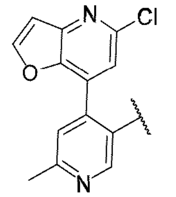 , 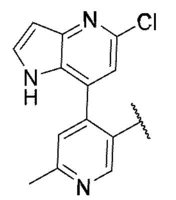 , 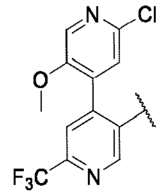 ,

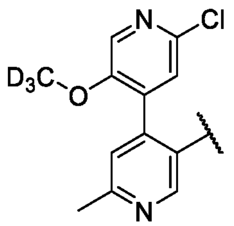 , 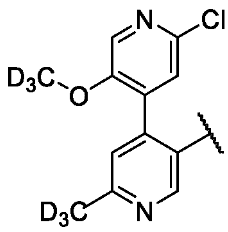 , 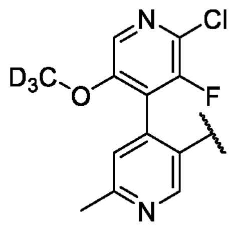 , 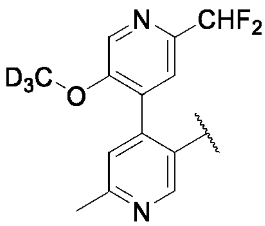 ,

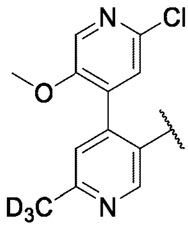, 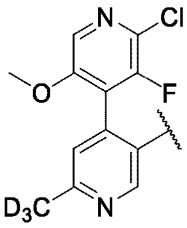, 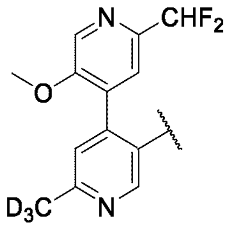, 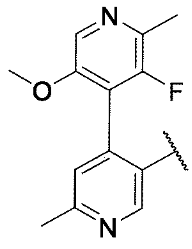, 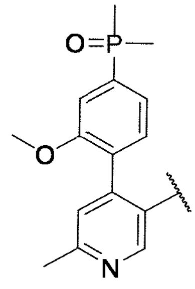

or

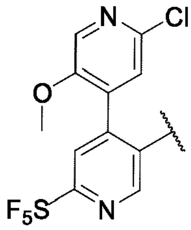.

In an embodiment,

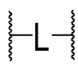 is 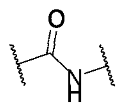.

In an embodiment,

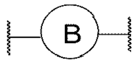

is

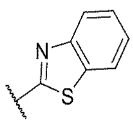, 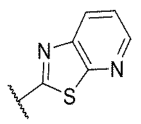, 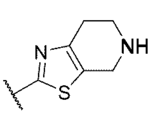, 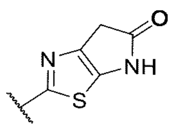,

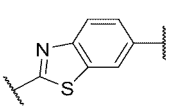, 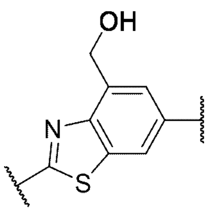, 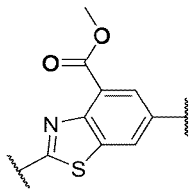, 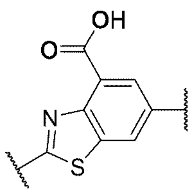, 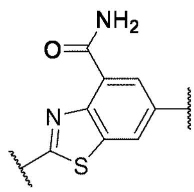,

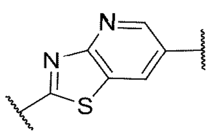, 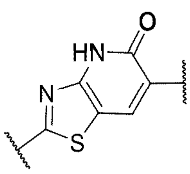, 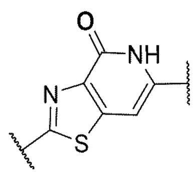, 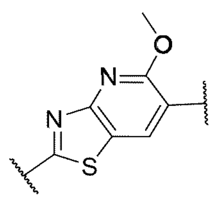, 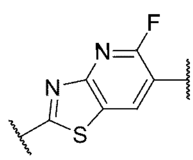,

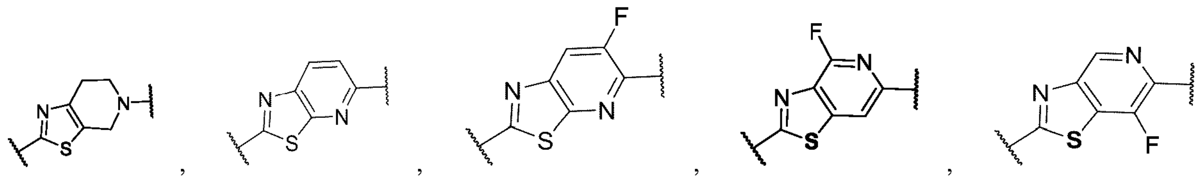
, , , , ,

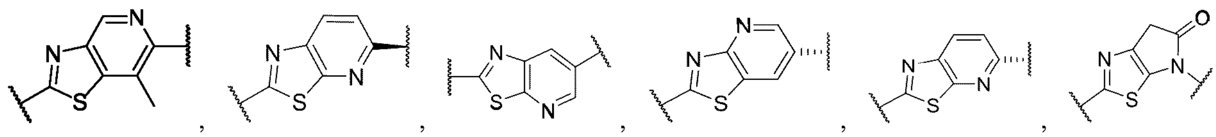
, , , , , ,

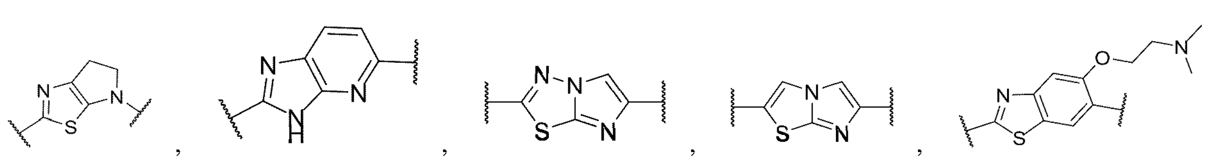
, , , , ,

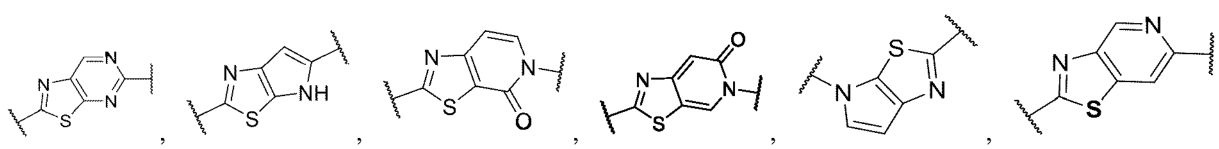
, , , , , ,

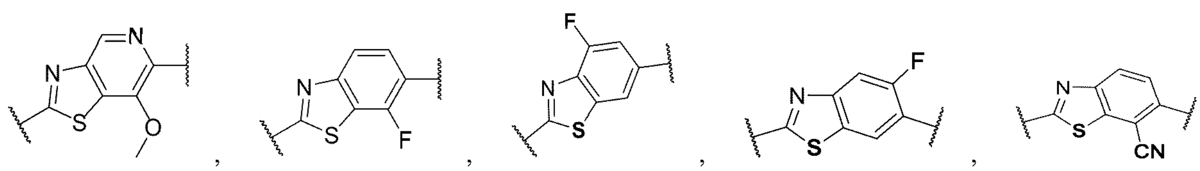
, , , , ,

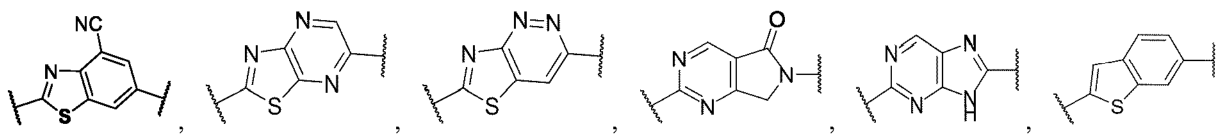
, , , , , ,

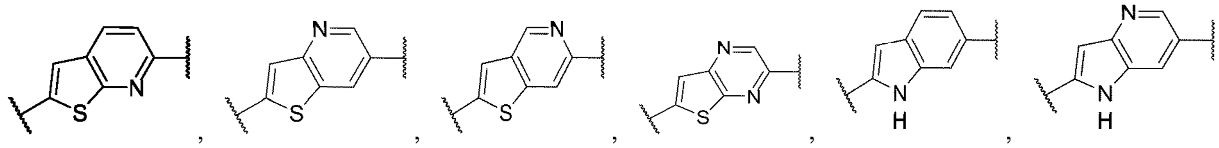
, , , , , ,

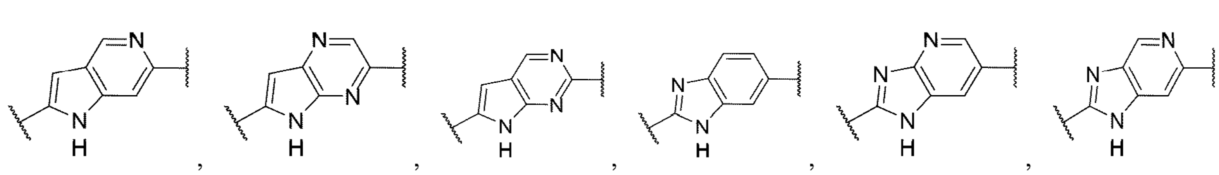
, , , , , ,

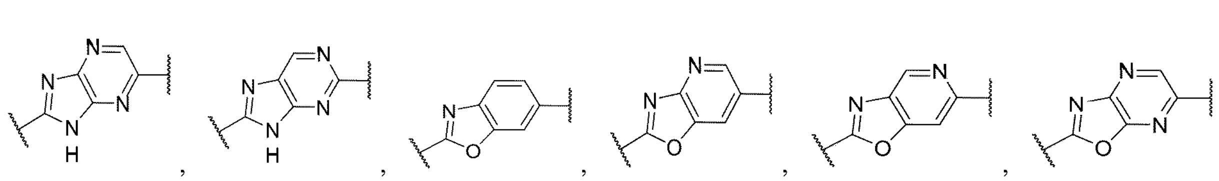
, , , , , ,

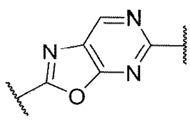, 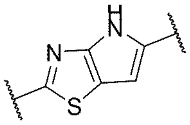, 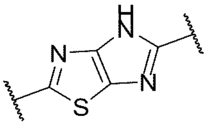, 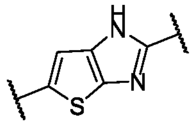, 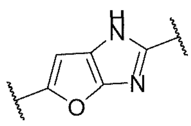,

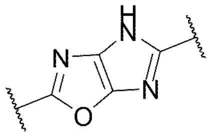, 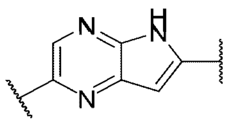, 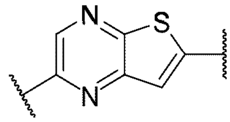, 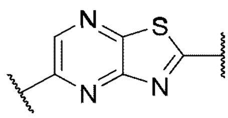, 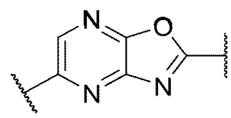,

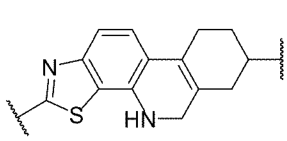, 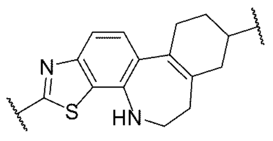, 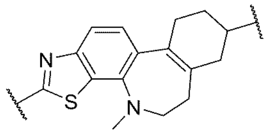, 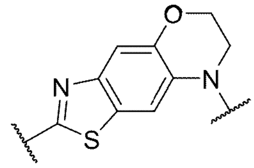,

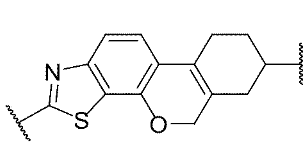, 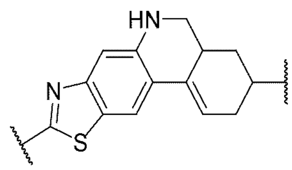, 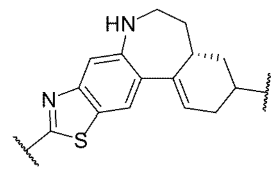, 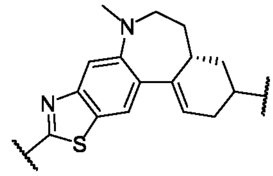,

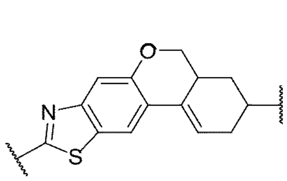, 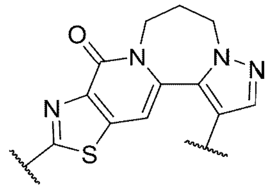, 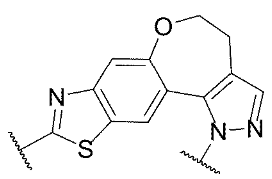, 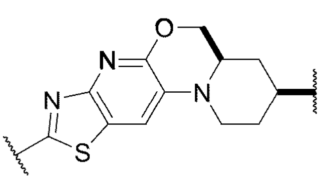,

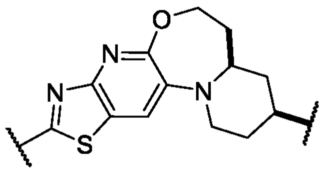, 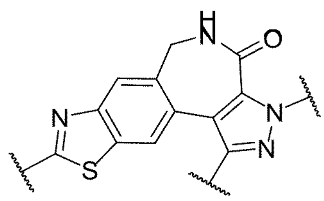, 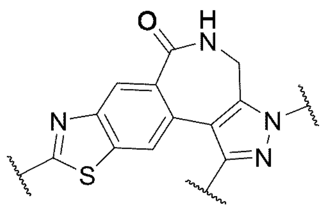,

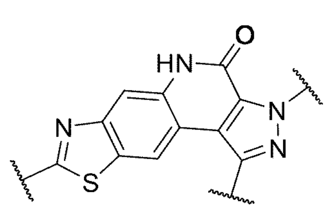, 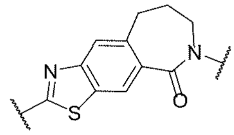 or 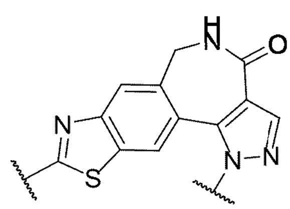.

In an embodiment, $R^3$ is hydrogen, methyl,

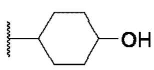, 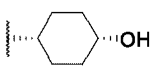, 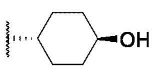, 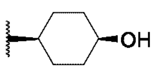,

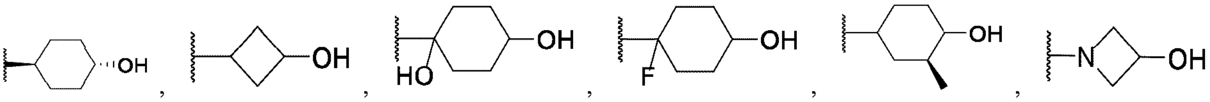, , , , , ,

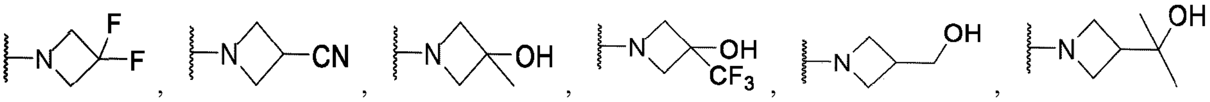, , , , , ,

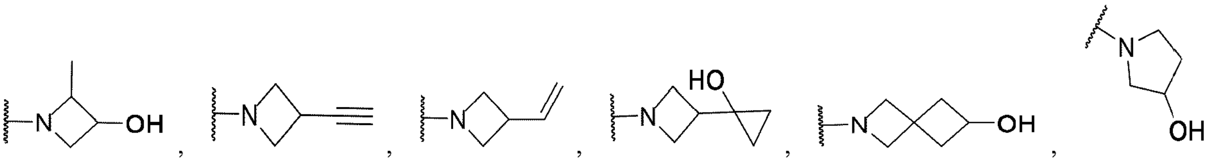, , , , , ,

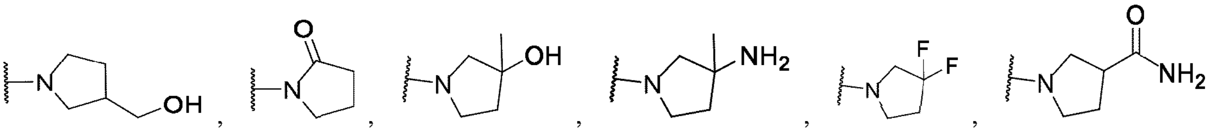, , , , , ,

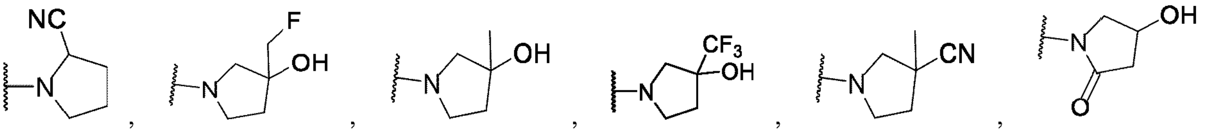, , , , , ,

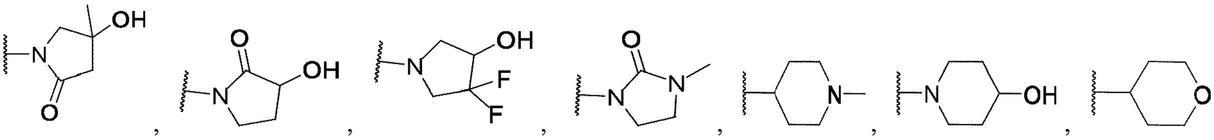, , , , , , ,

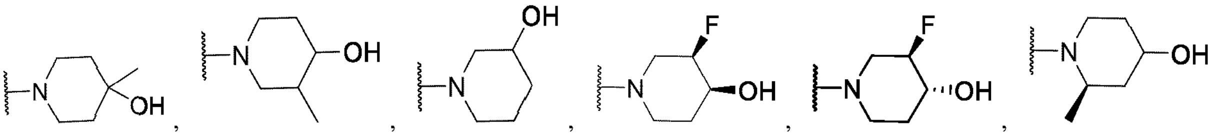, , , , , ,

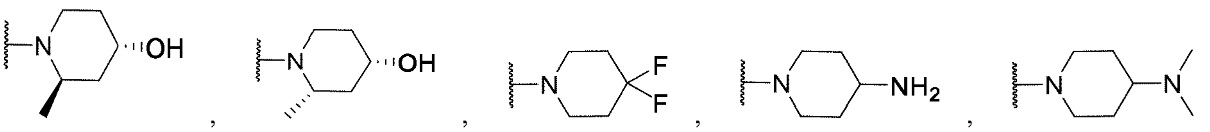, , , , ,

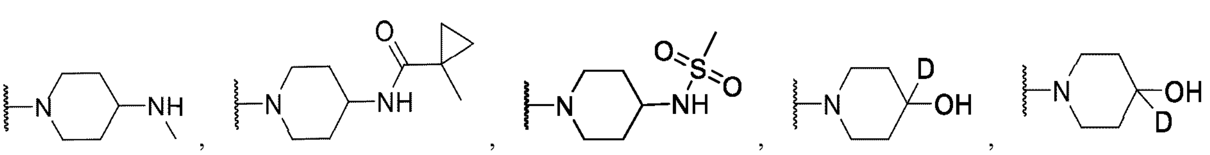, , , , ,

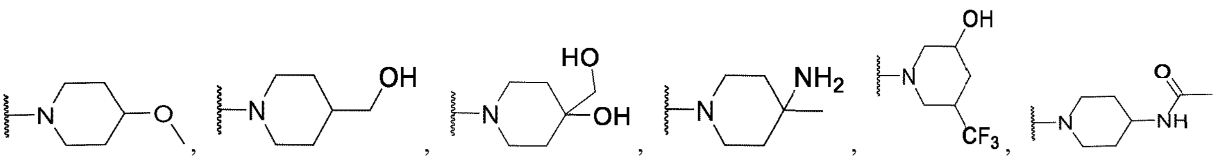, , , , , ,

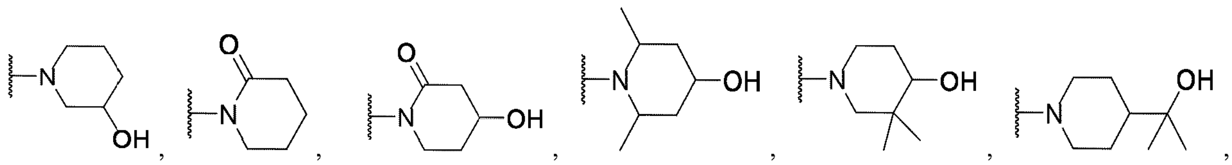
, , , , , ,

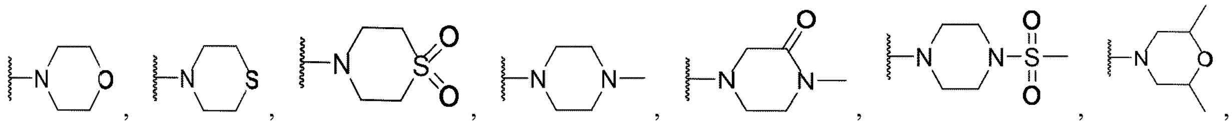
, , , , , , ,

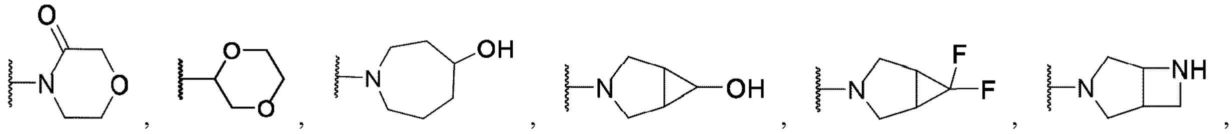
, , , , , ,

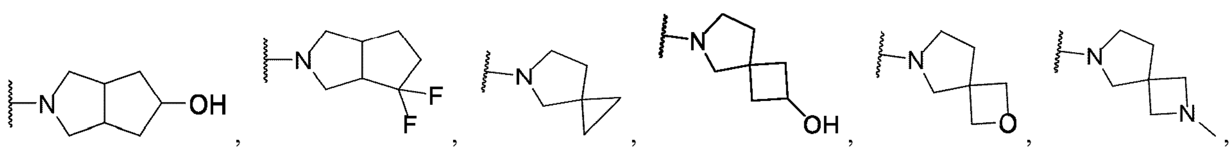
, , , , , ,

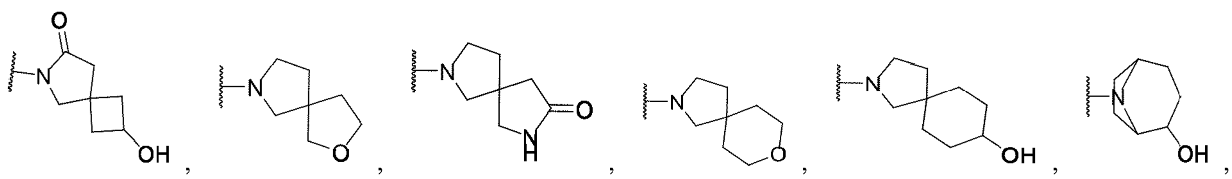
, , , , , ,

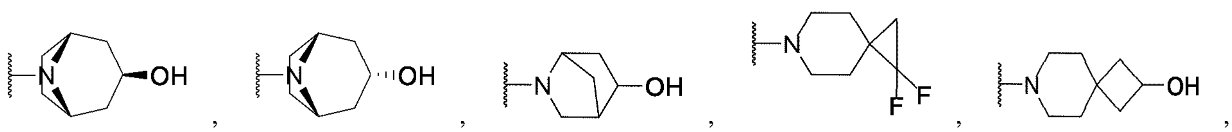
, , , , ,

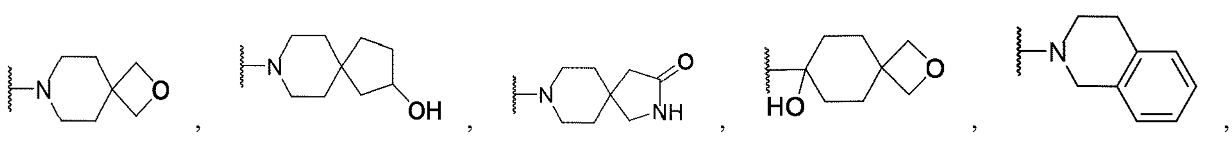
, , , , ,

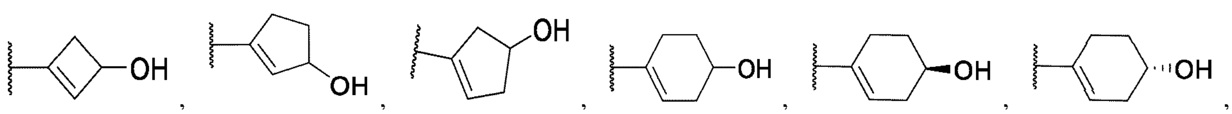
, , , , , ,

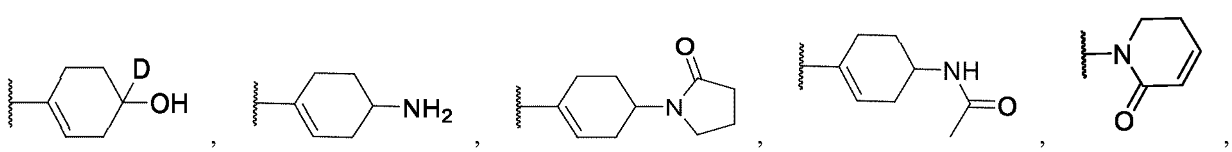
, , , , ,

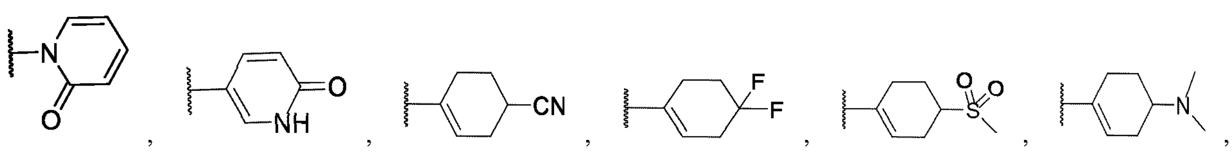
, , , , , ,

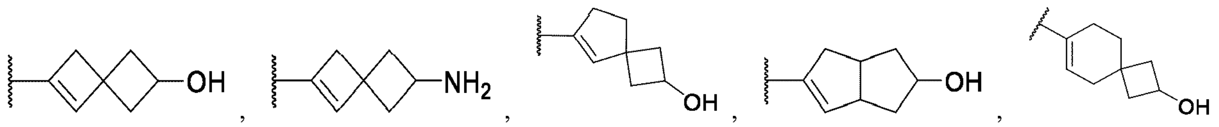 , , , , ,

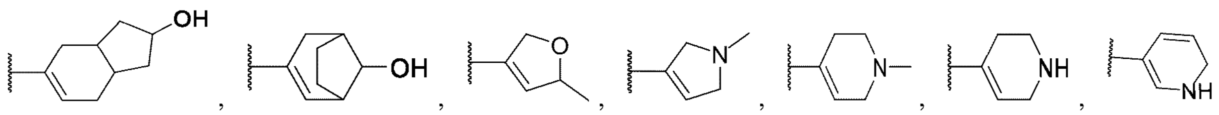 , , , , , , ,

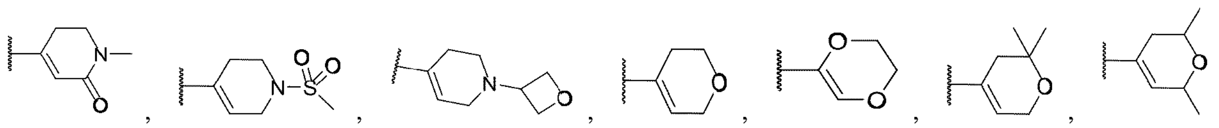 , , , , , , ,

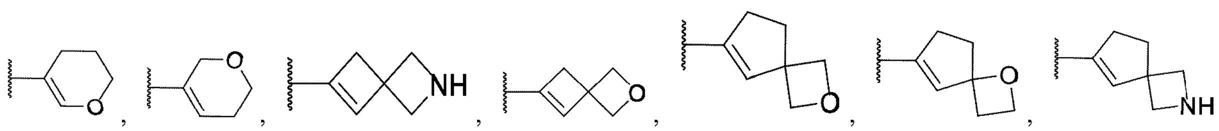 , , , , , , ,

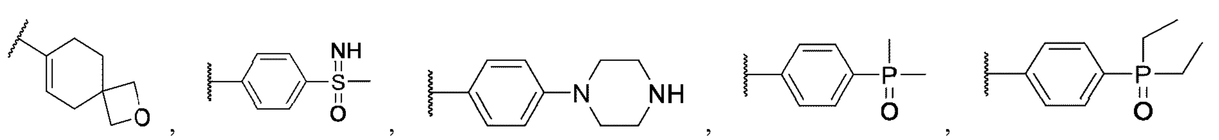 , , , , ,

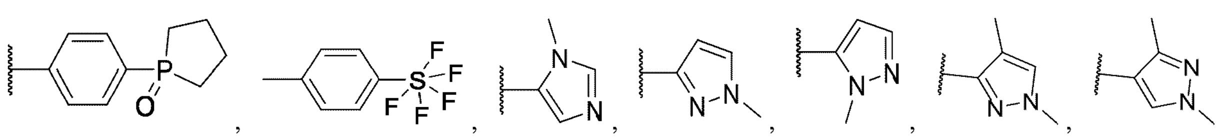 , , , , , , ,

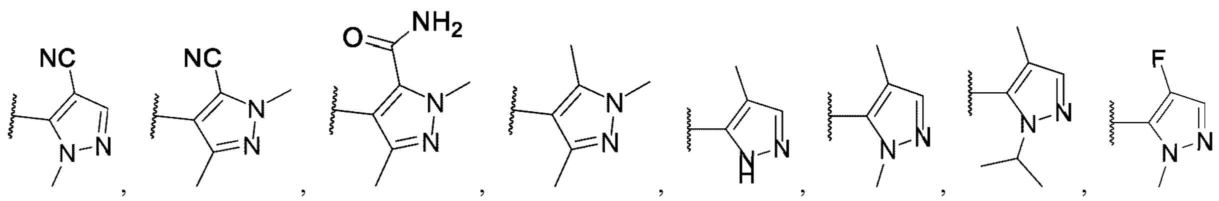 , , , , , , , ,

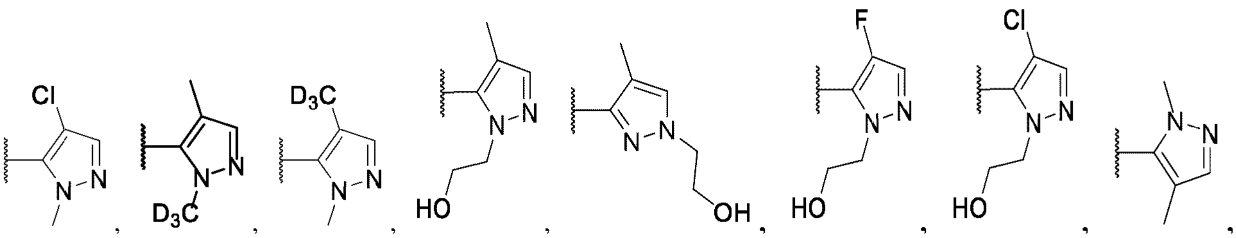 , , , , , , , ,

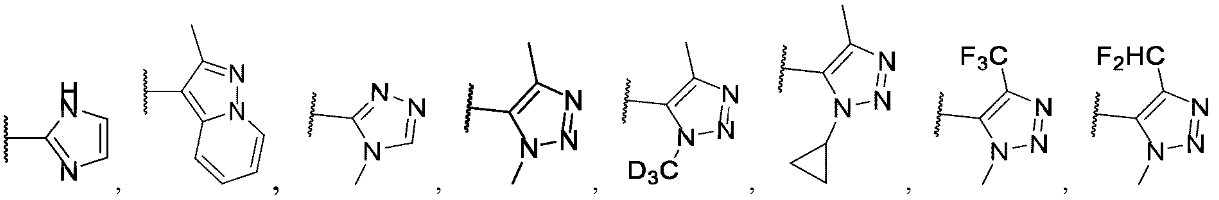 , , , , , , , ,

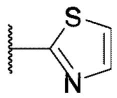, 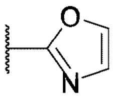, 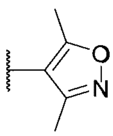, 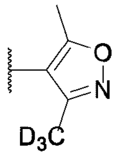, 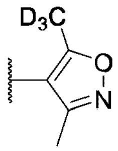, 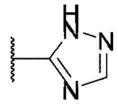, 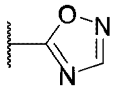, 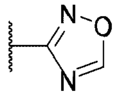, 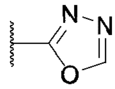,

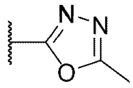, 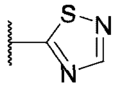, 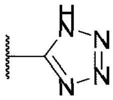, 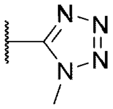, 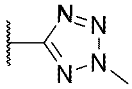, 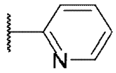, 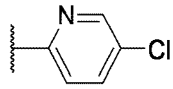, 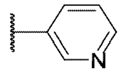,

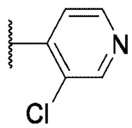, 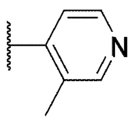, 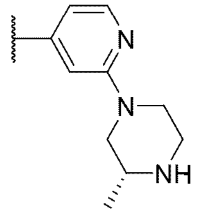, 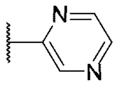, 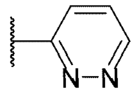, 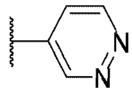, 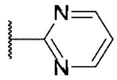,

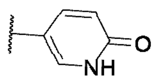, 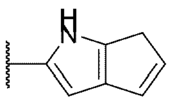, 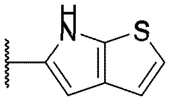, 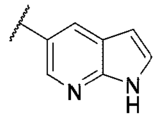, 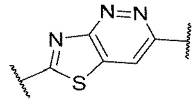, 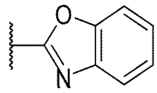,

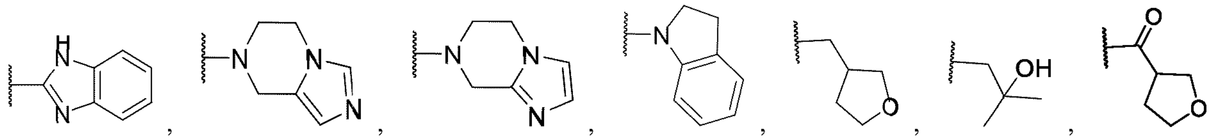, , , , , , ,

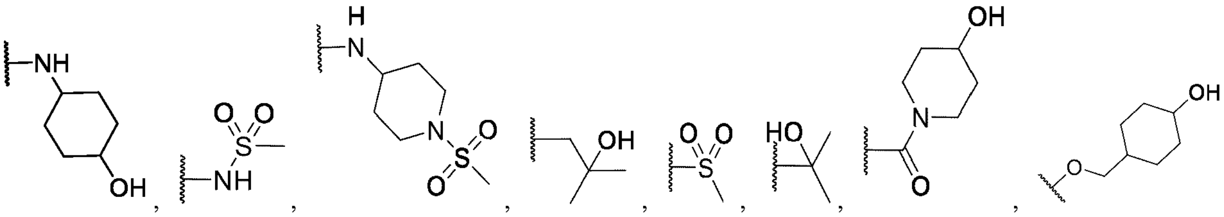, , , , , , , ,

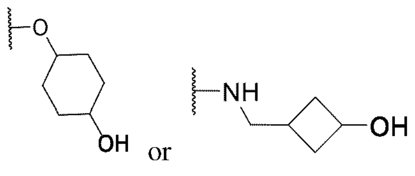 or .

In an embodiment, the compound of formula I is any one of the following compounds:

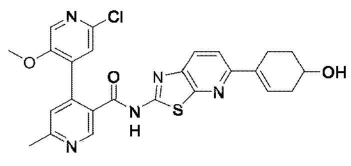, 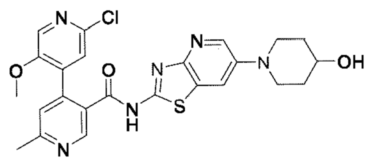, 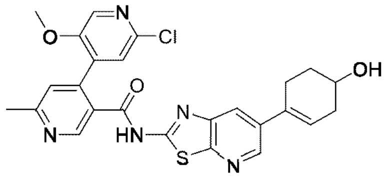,

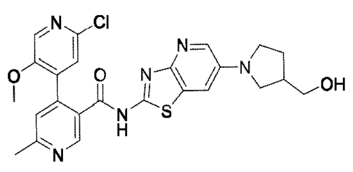,
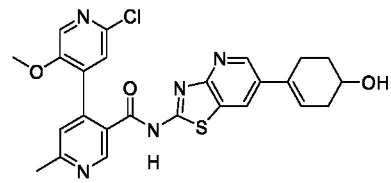,
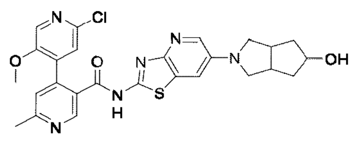,

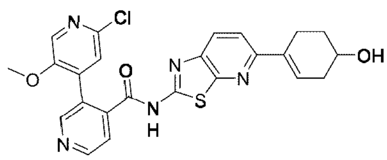,
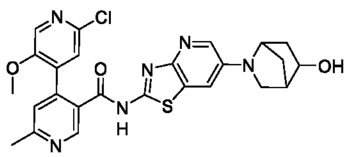,
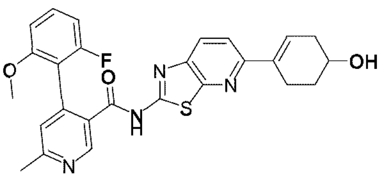,

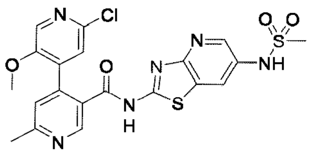,
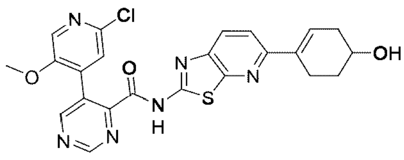,
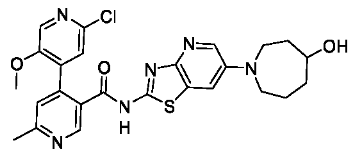,

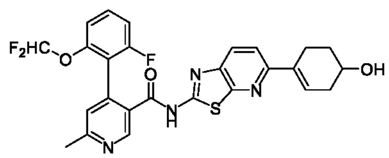,
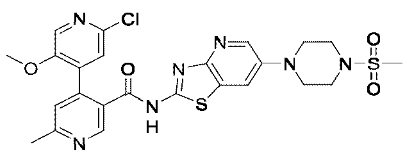,
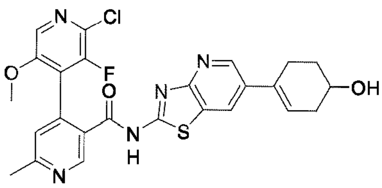,

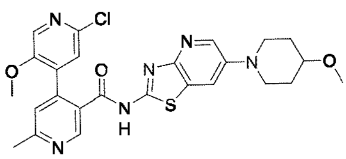,
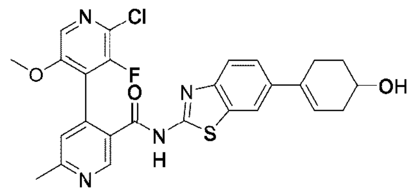,
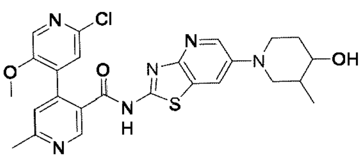,

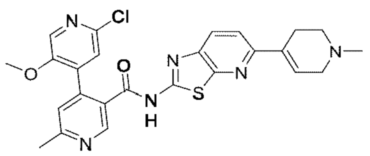,
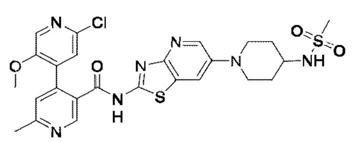,
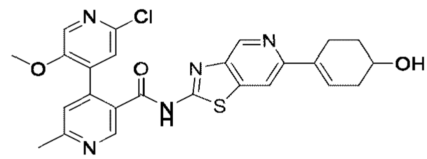,

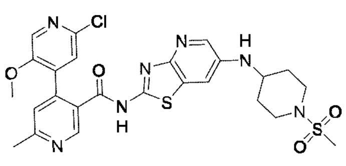,
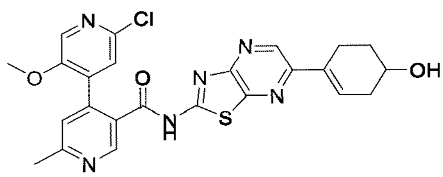,
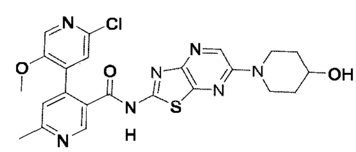,

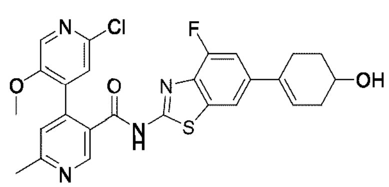,
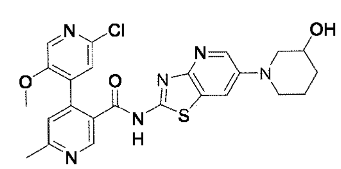,
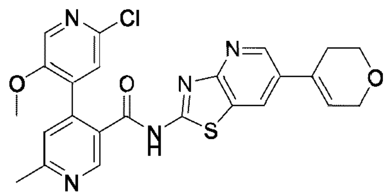,

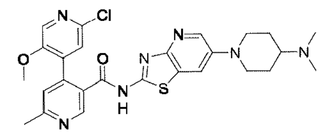, 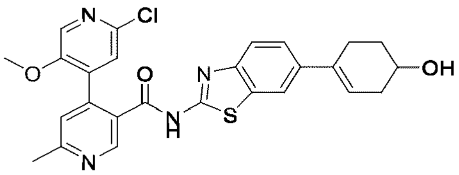, 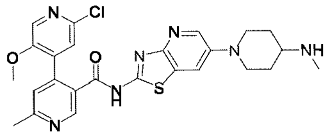,

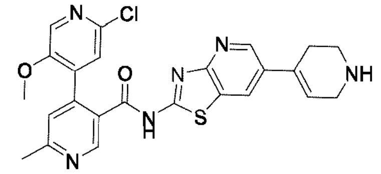, 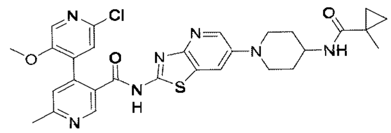, 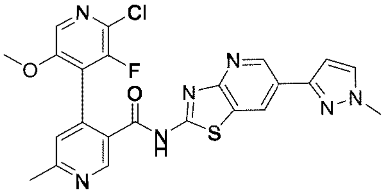,

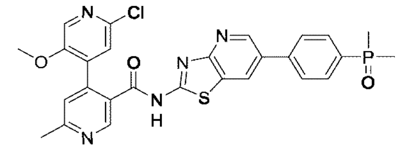, 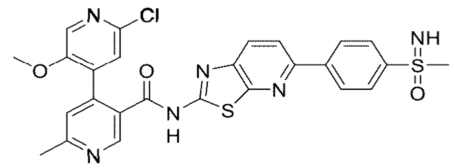, 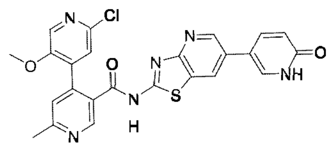,

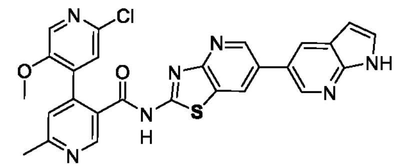, 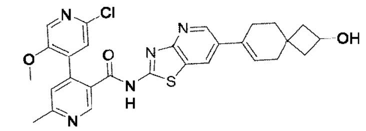, 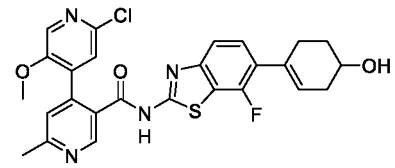,

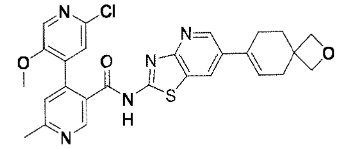, 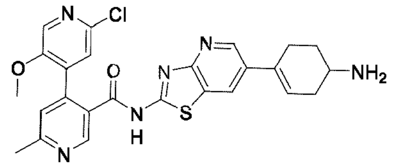, 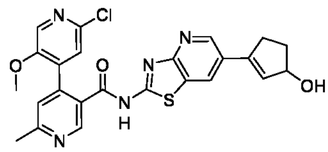,

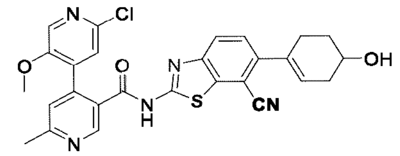, 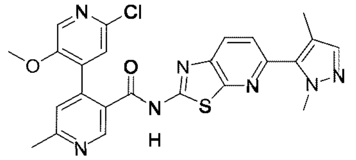, 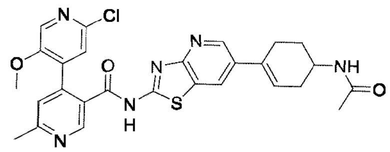,

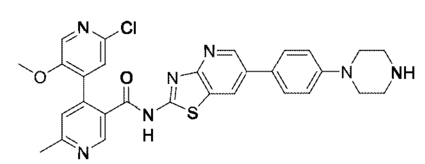, 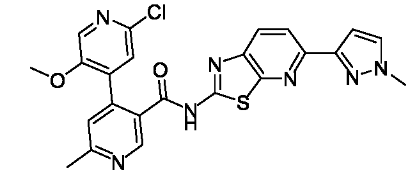, 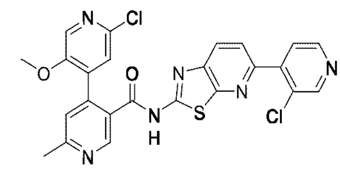,

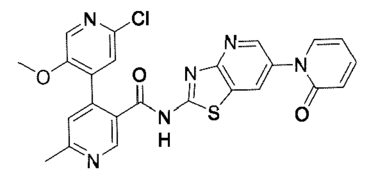, 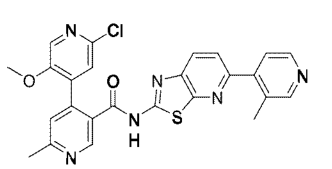, 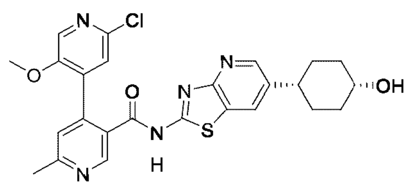,

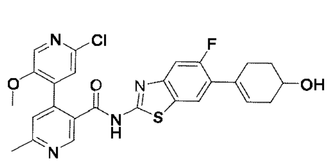,
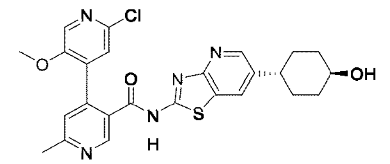,
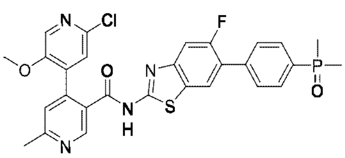,

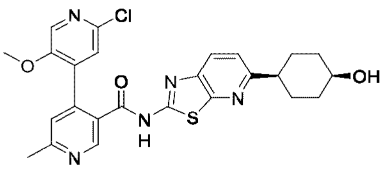,
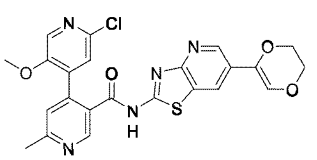,
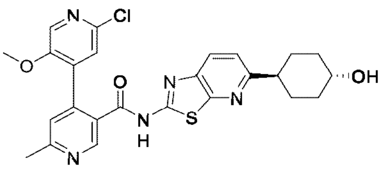,

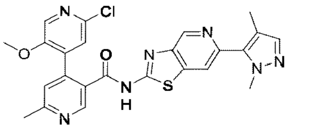,
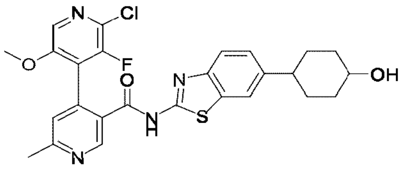,
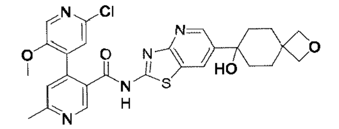,

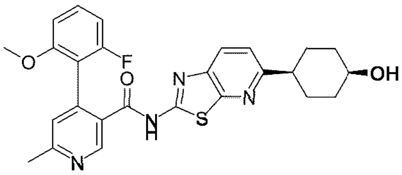,
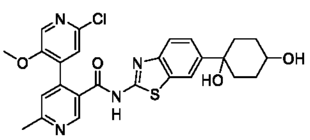,
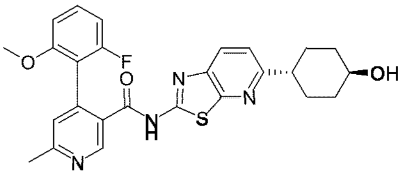,

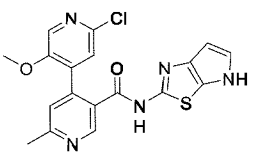,
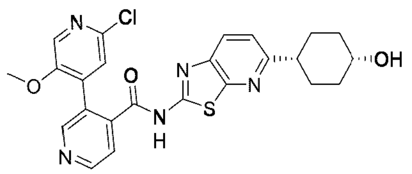,
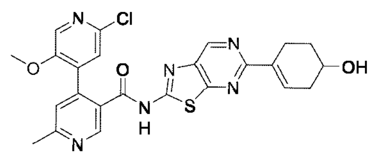,

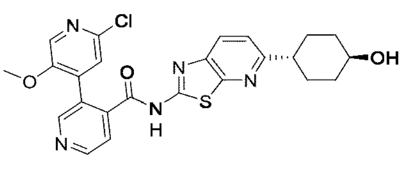,
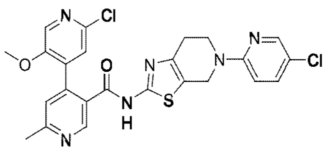,
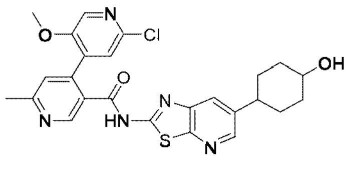,

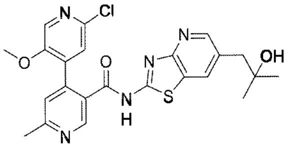,
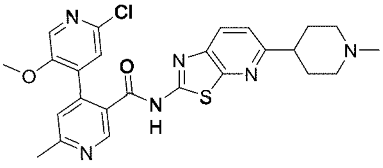,
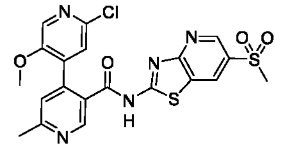,

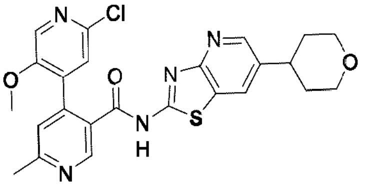,
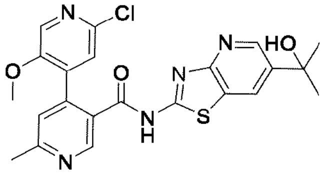,
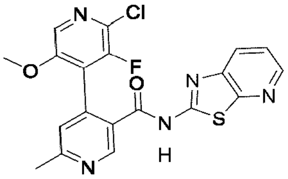,

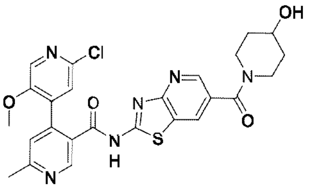 , 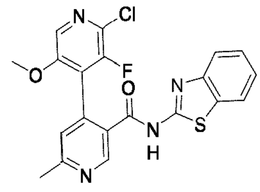 , 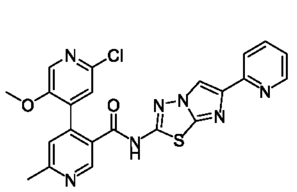 ,

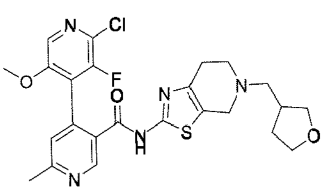 , 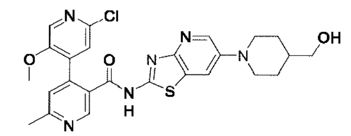 , 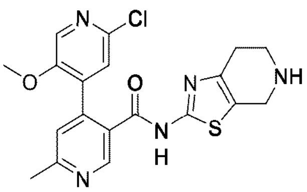 ,

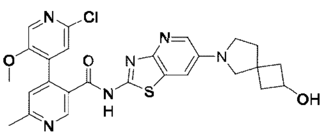 , 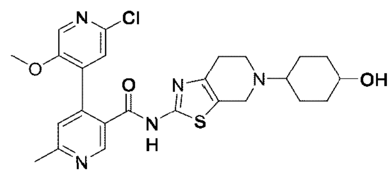 , 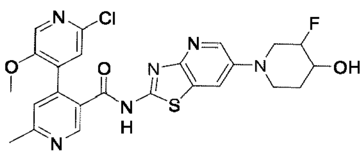 ,

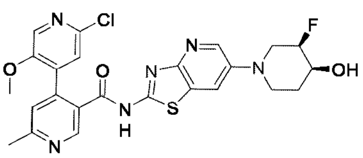 , 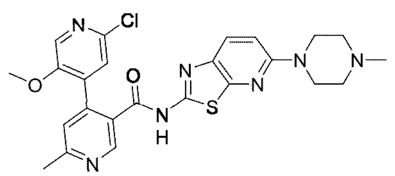 , 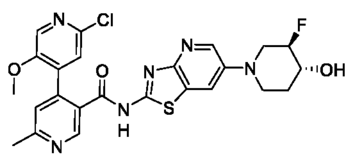 ,

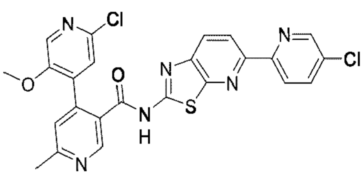 , 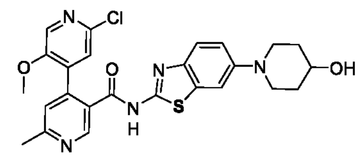 , 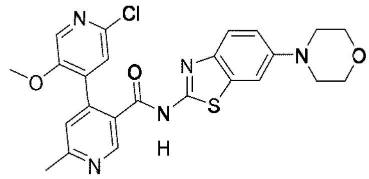 ,

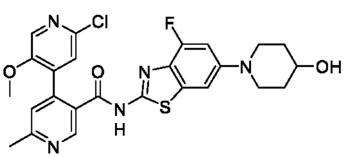 , 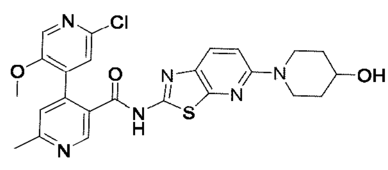 , 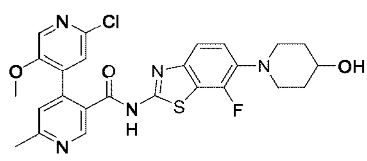 ,

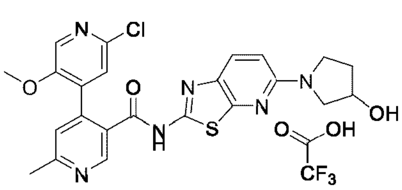 , 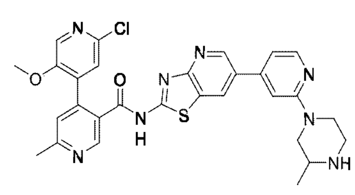 , 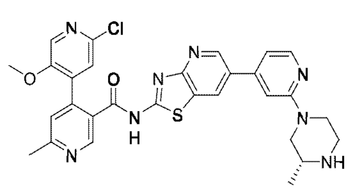 ,

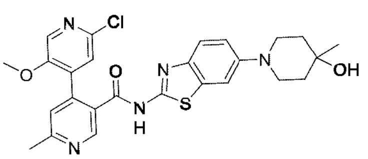 , 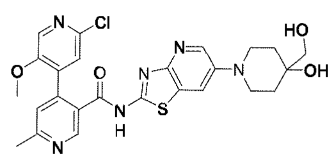 , 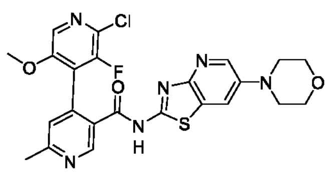 ,

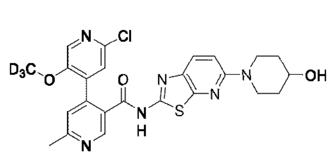,
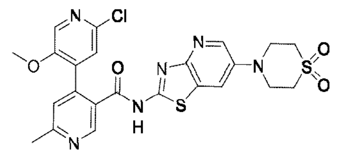,
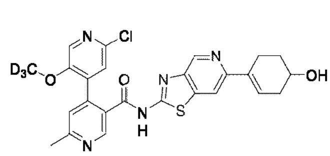,

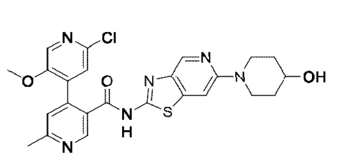,
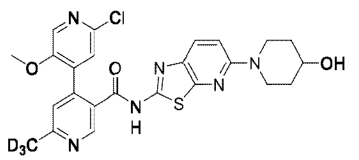,
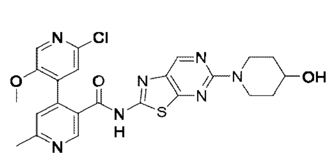,

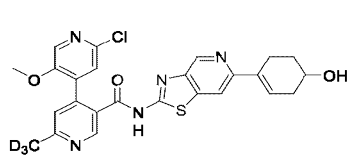,
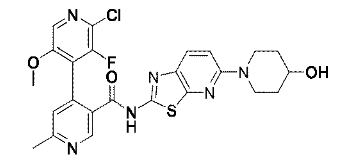,
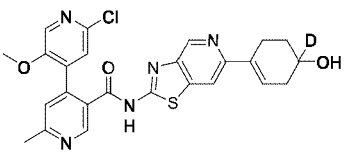,

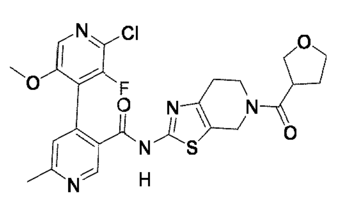,
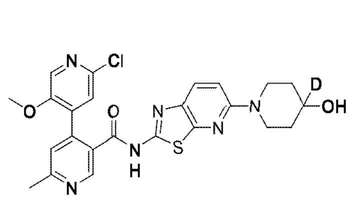,
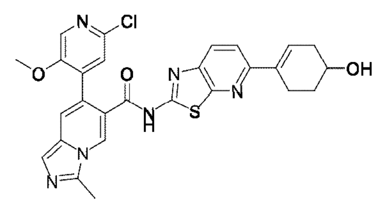,

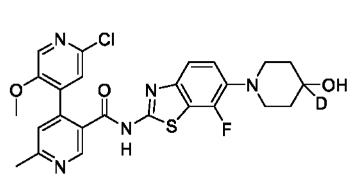,
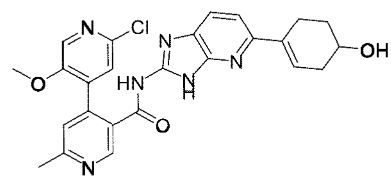,
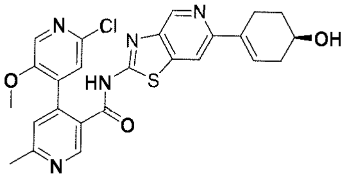,

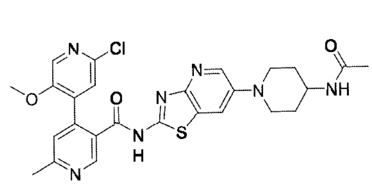,
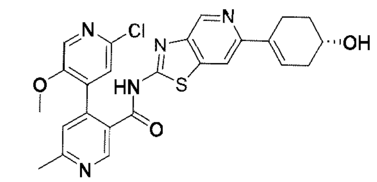,
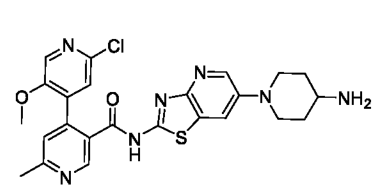,

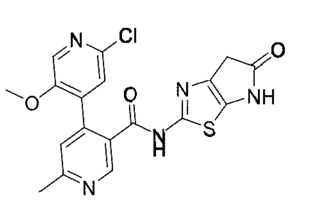,
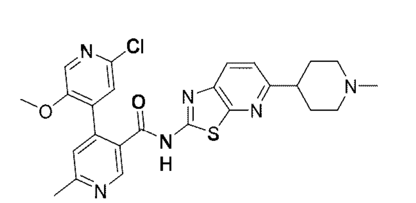,
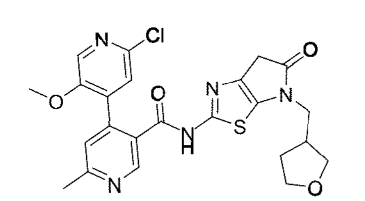,

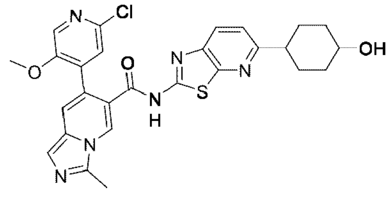 , 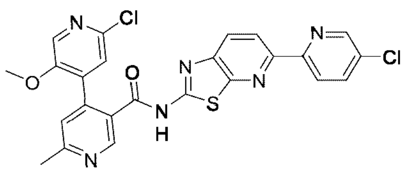 , 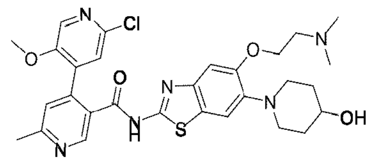 ,

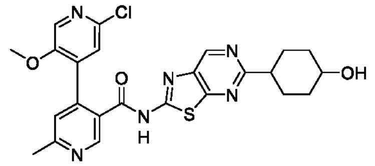 , 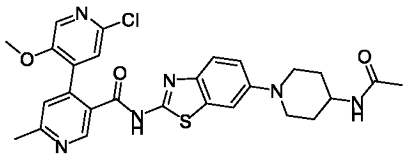 , 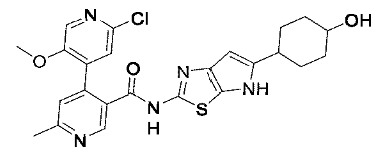 ,

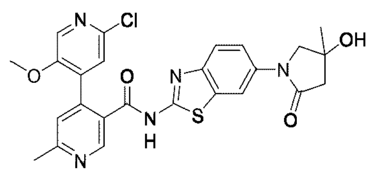 , 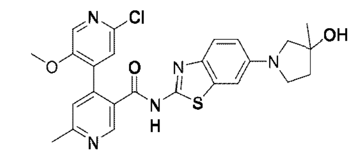 , 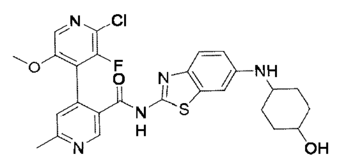 ,

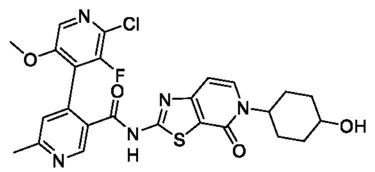 , 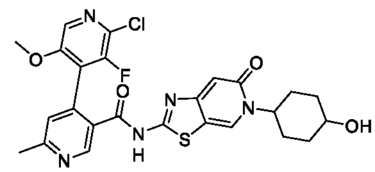 , 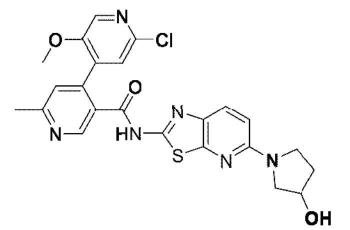 ,

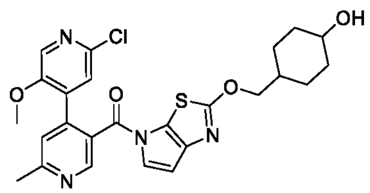 , 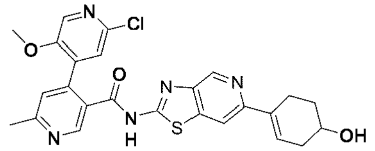 , 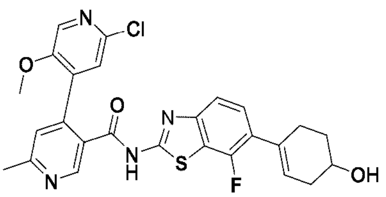 ,

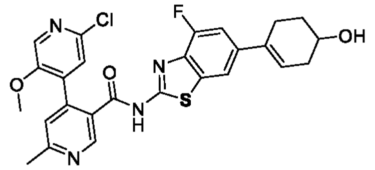 , 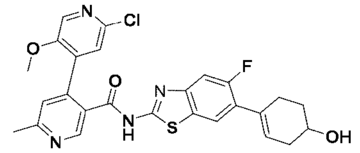 , 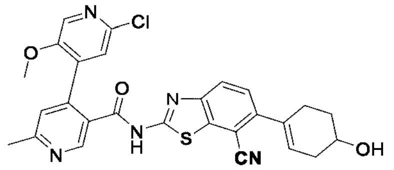 ,

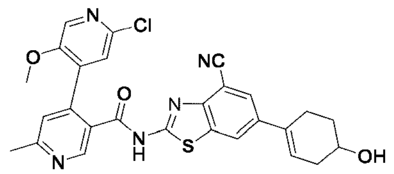 , 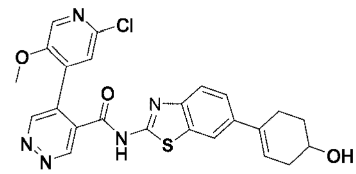 , 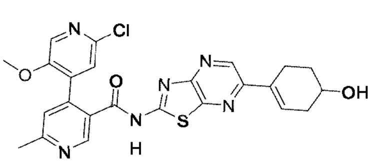 ,

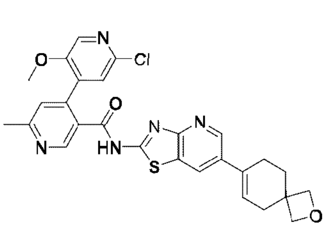 ,
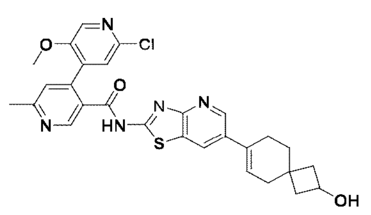 ,
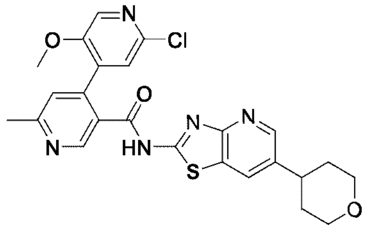 ,

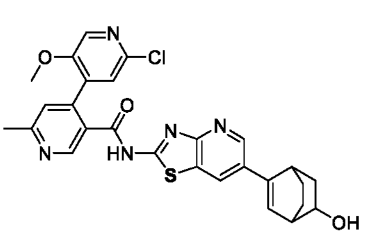 ,
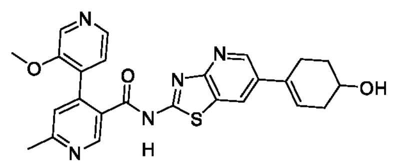 ,
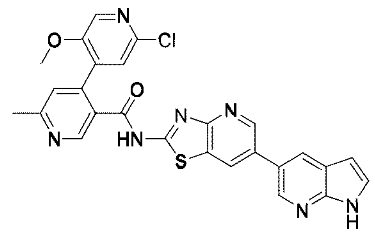 ,

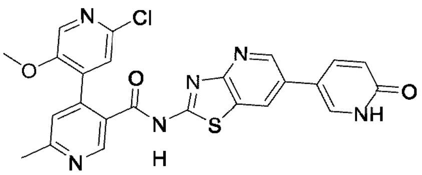 ,
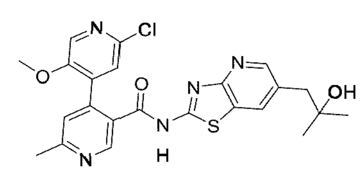 ,
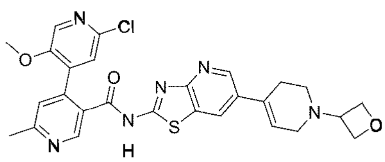 ,

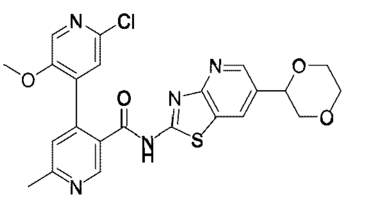 ,
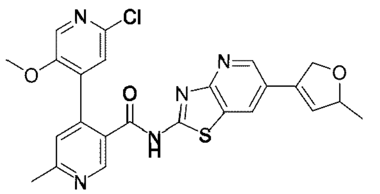 ,
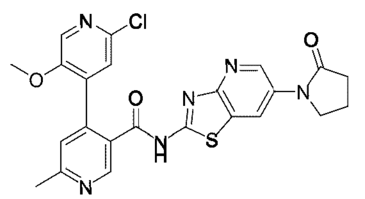 ,

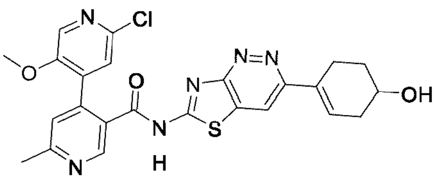 ,
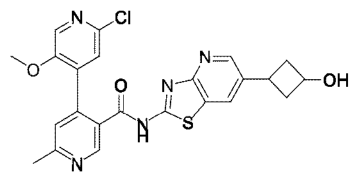 ,
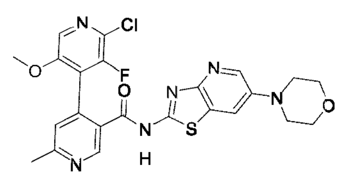 ,

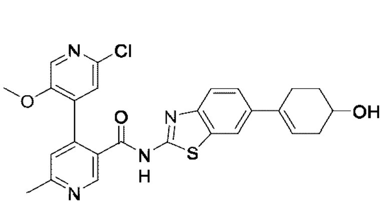 ,
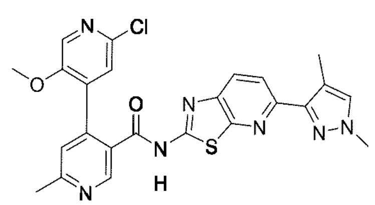 ,
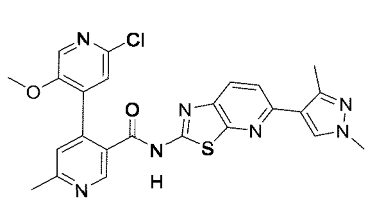 ,

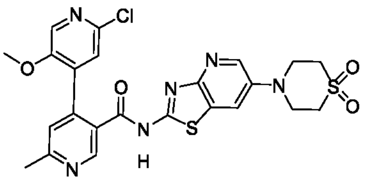 ,
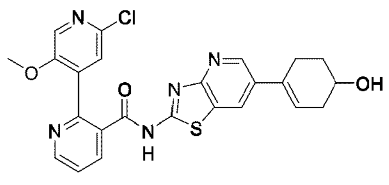 ,
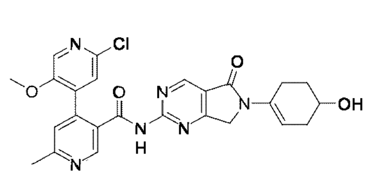 ,

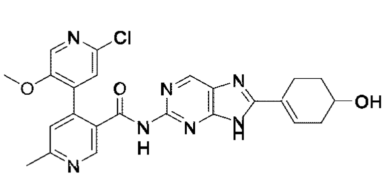 , 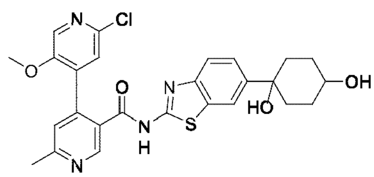 , 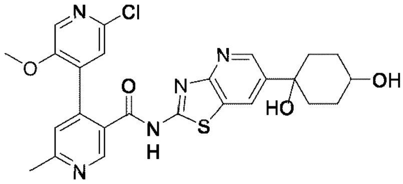 ,

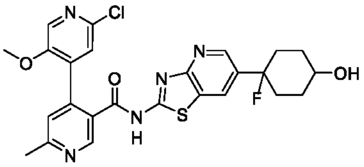 , 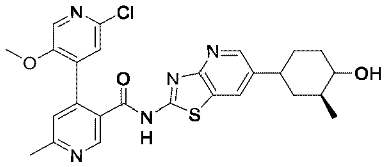 , 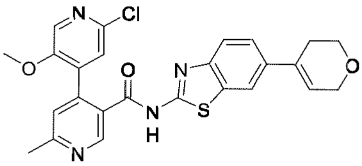 ,

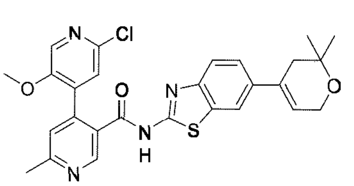 , 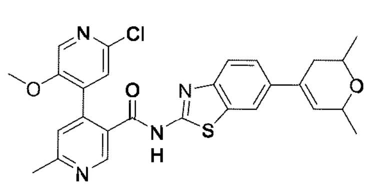 , 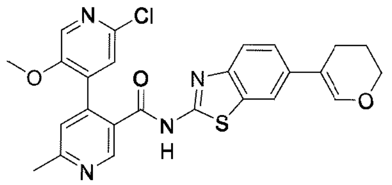 ,

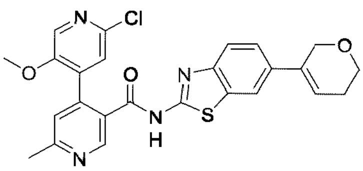 , 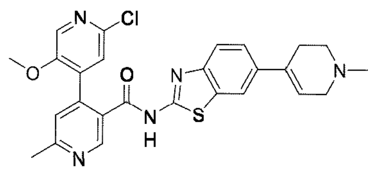 , 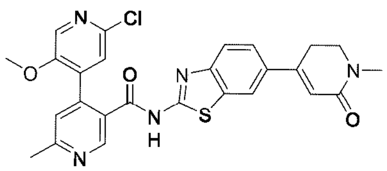 ,

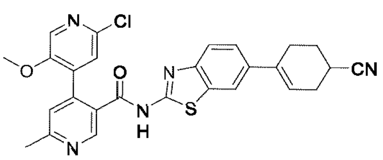 , 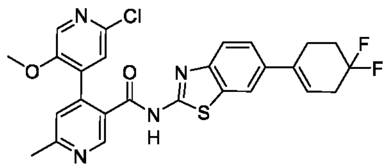 , 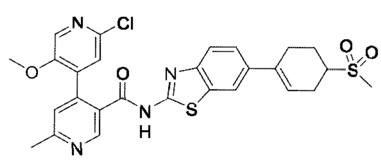 ,

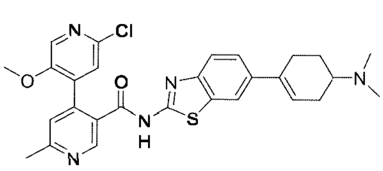 , 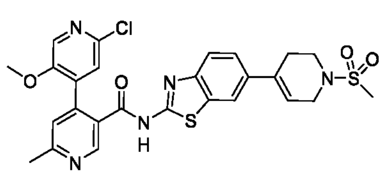 , 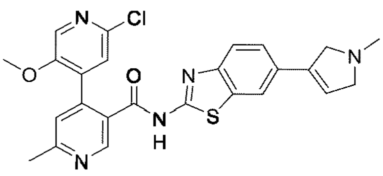 ,

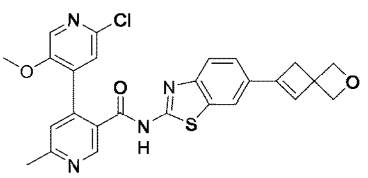 , 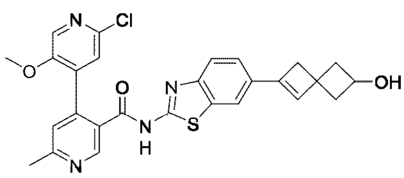 , 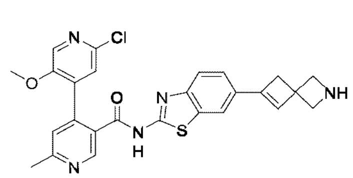 ,

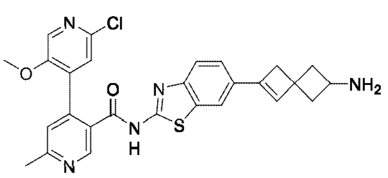 , 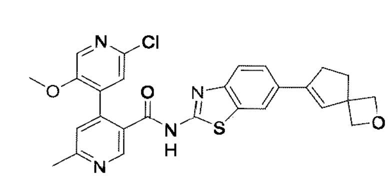 , 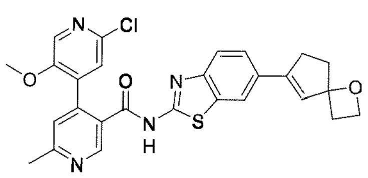 ,

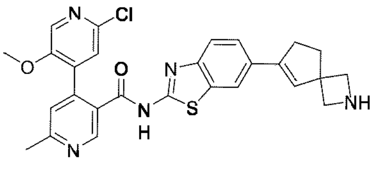,
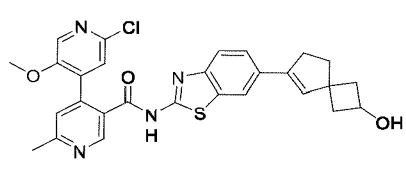,
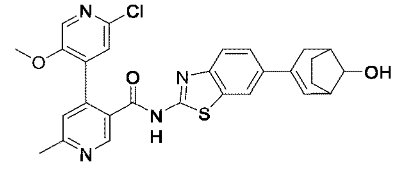,

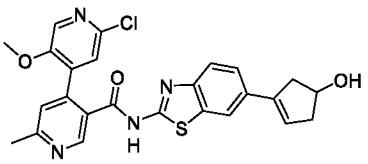,
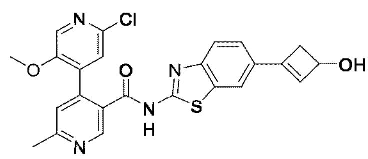,
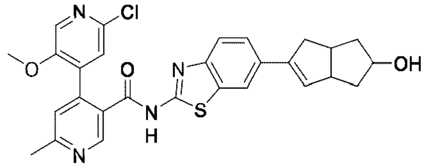,

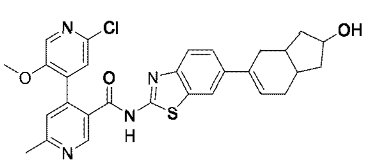,
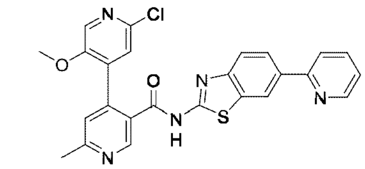,
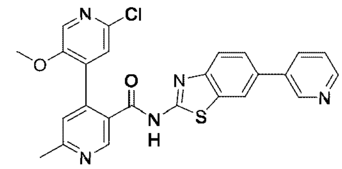,

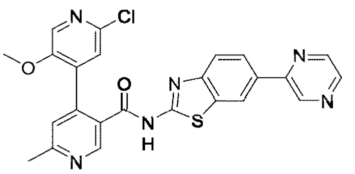,
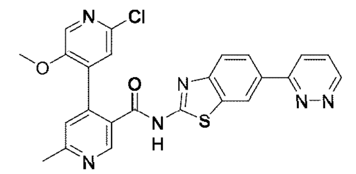,
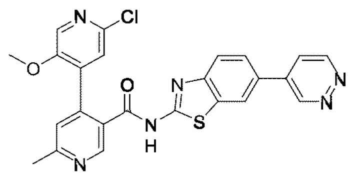,

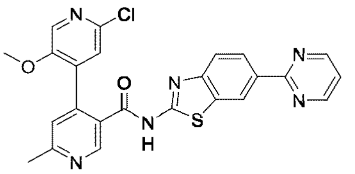,
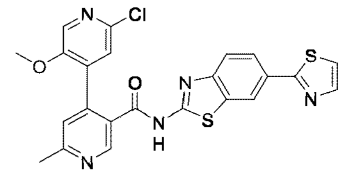,
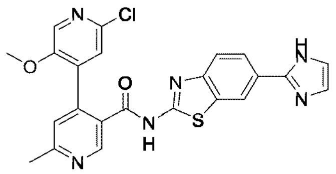,

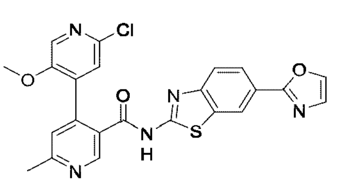,
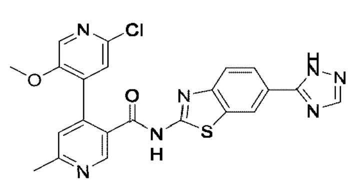,
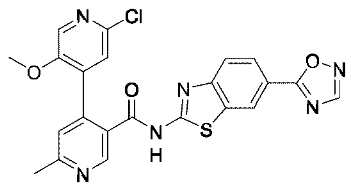,

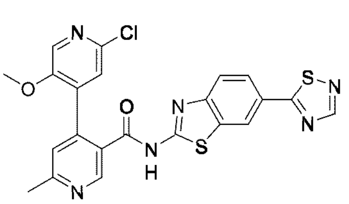,
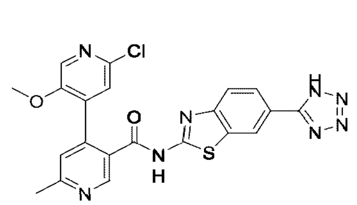,
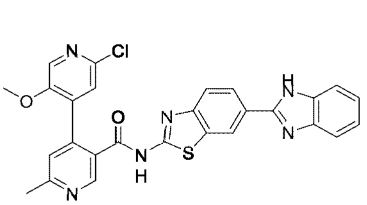,

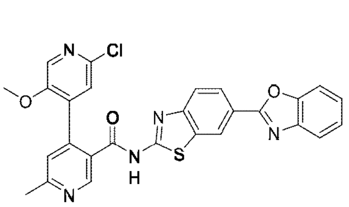,
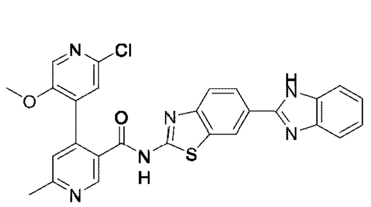,
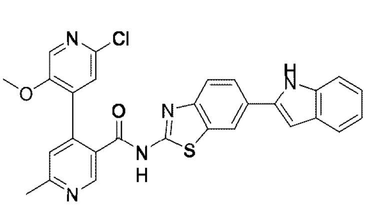,

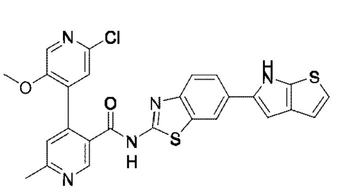,
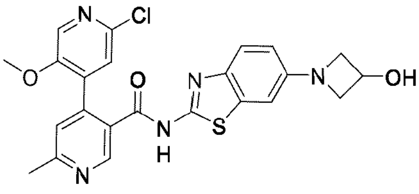,
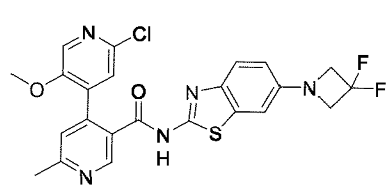,
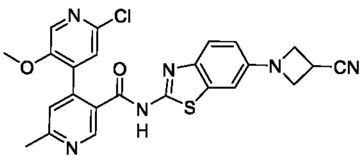,
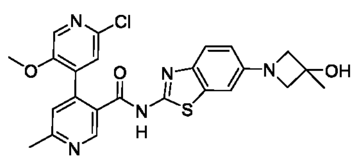,
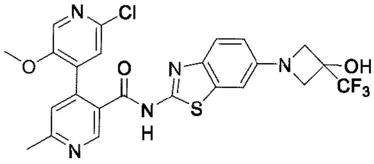,
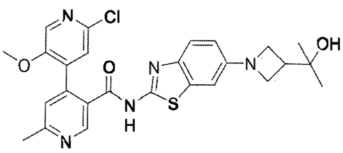,
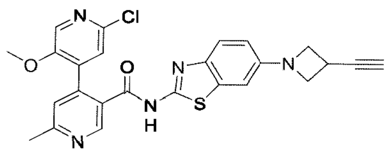,
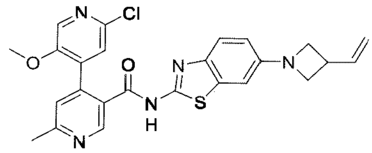,
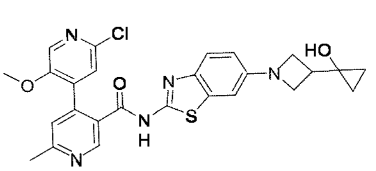,
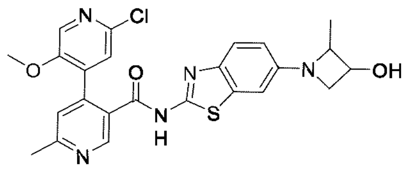,
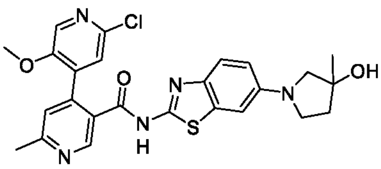,
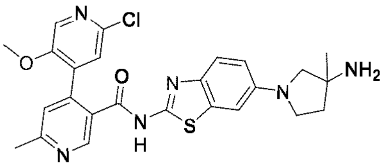,
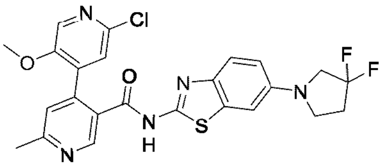,
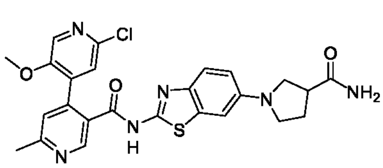,
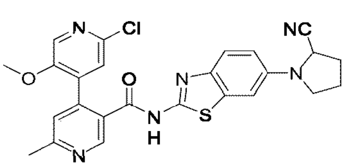,
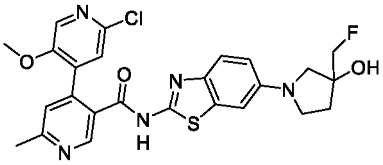,
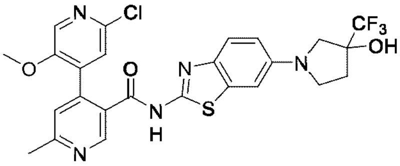,
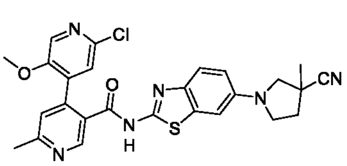,
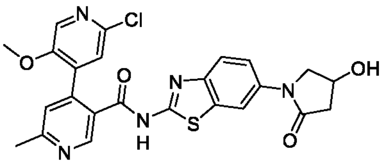,
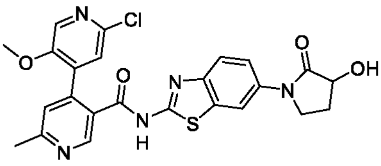,
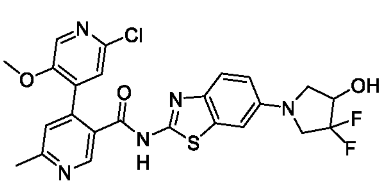,
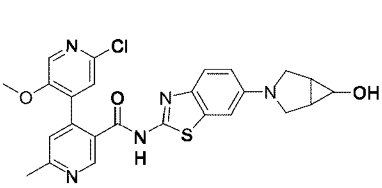,
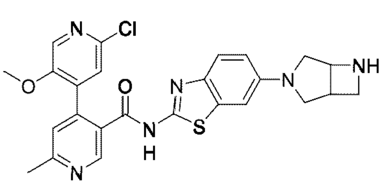,

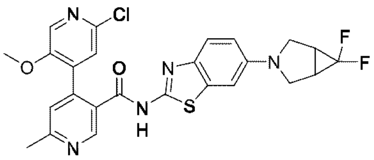 ,
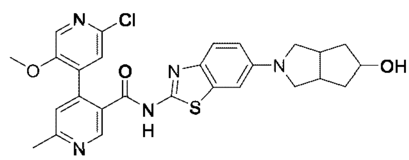 ,
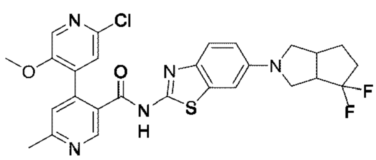 ,

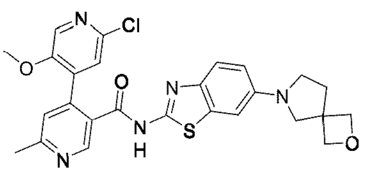 ,
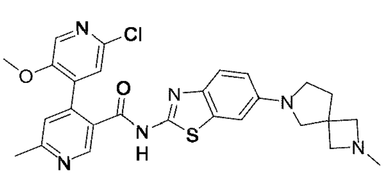 ,
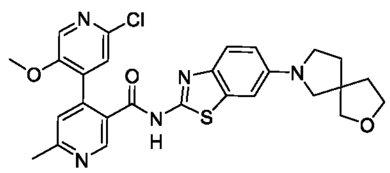 ,

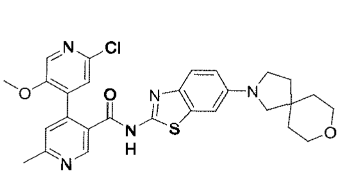 ,
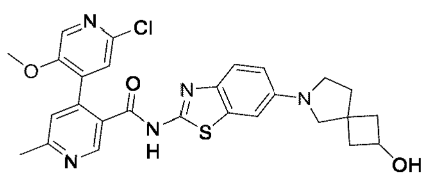 ,
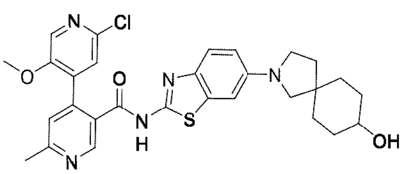 ,

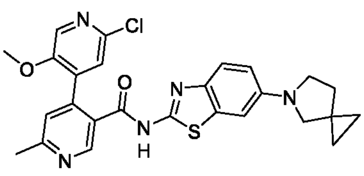 ,
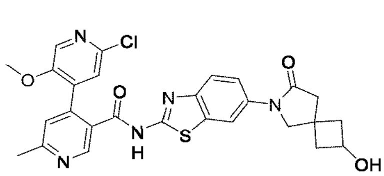 ,
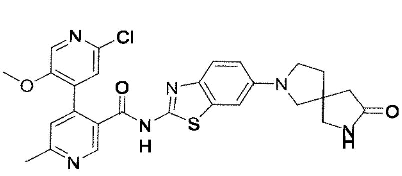 ,

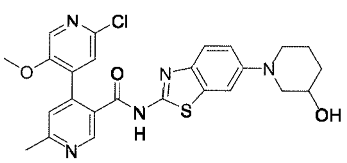 ,
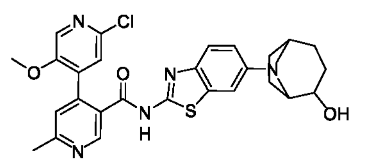 ,
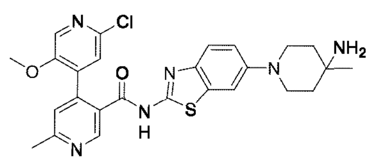 ,

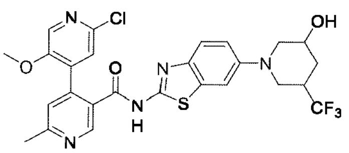 ,
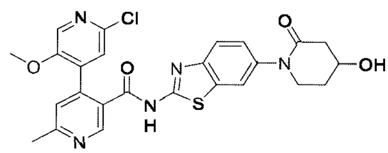 ,
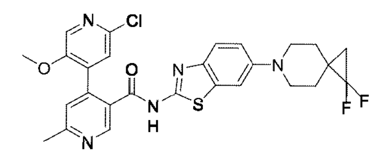 ,

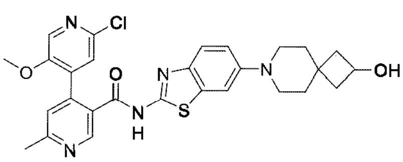 ,
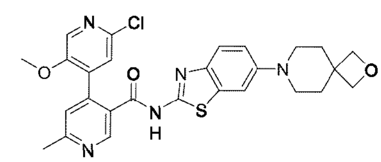 ,
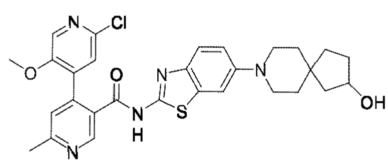 ,

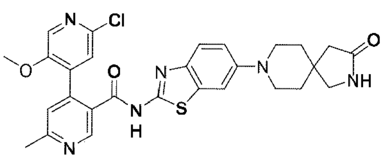 ,
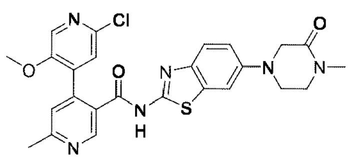 ,
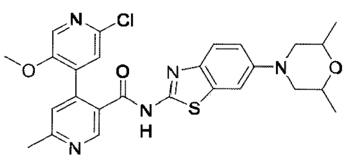 ,

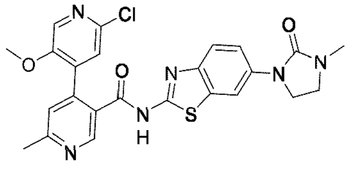 , 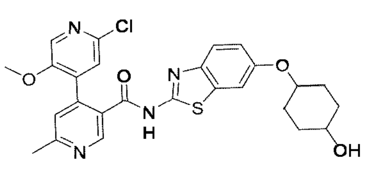 , 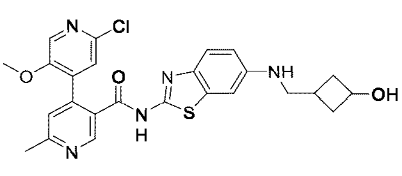 ,

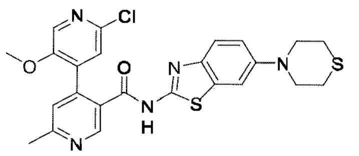 , 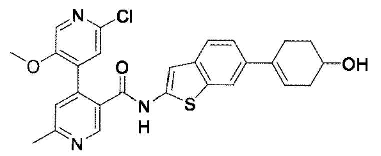 , 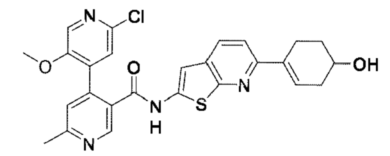 ,

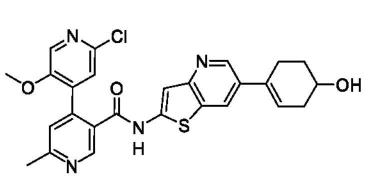 , 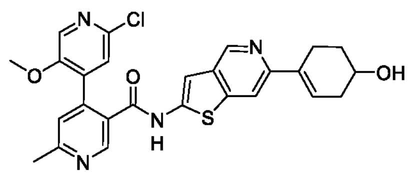 , 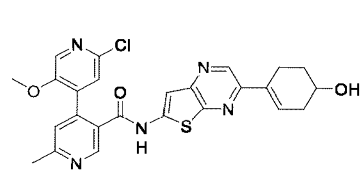 ,

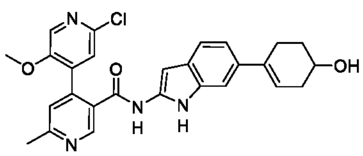 , 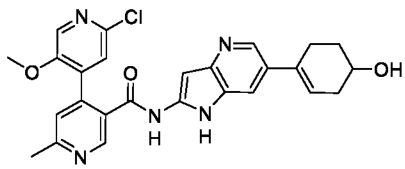 , 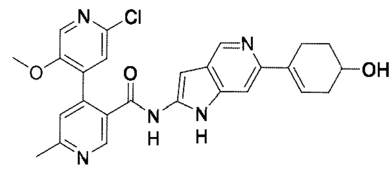 ,

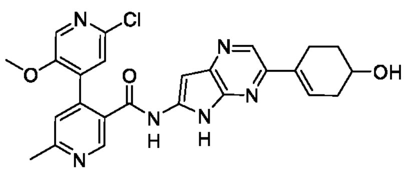 , 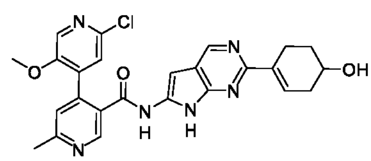 , 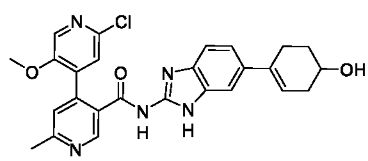 ,

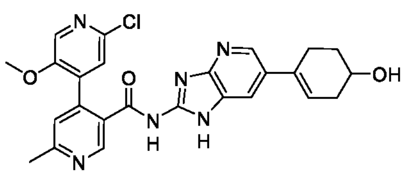 , 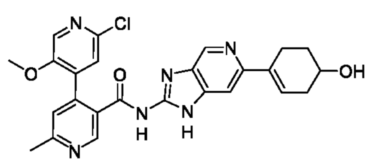 , 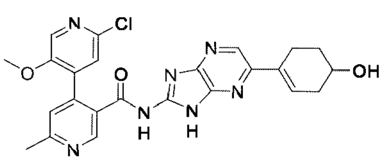 ,

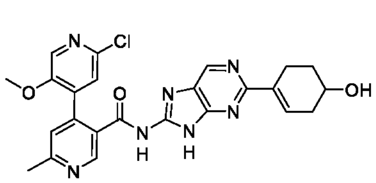 , 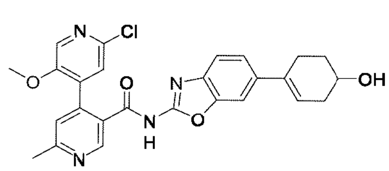 , 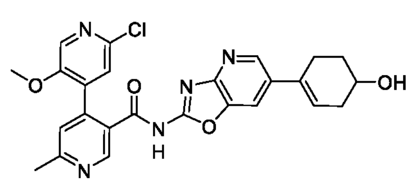 ,

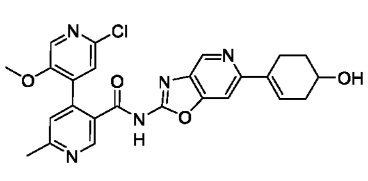 , 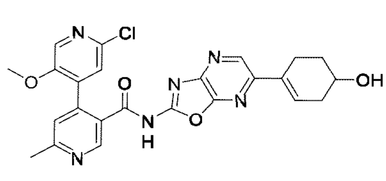 , 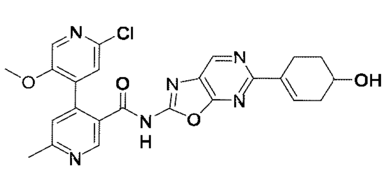 ,

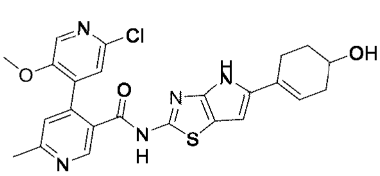,
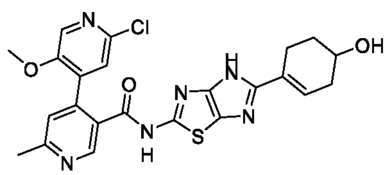,
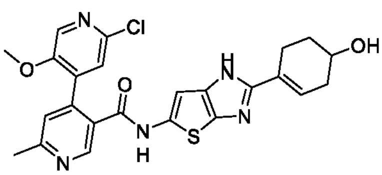,

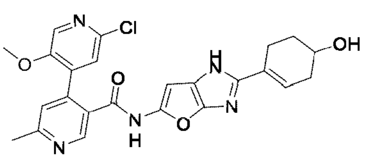,
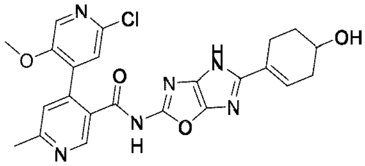,
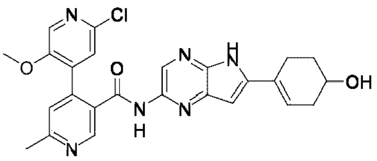,

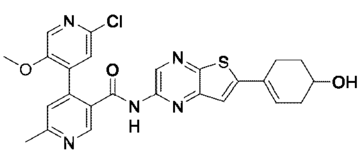,
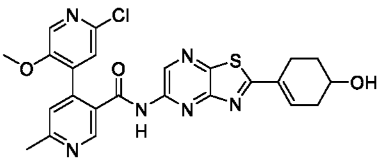,
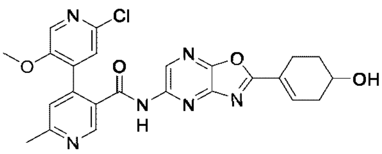,

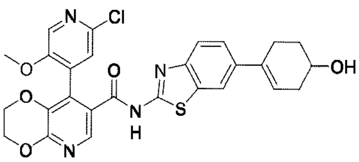,
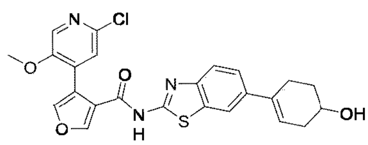,
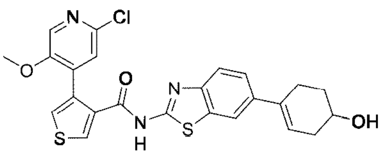,

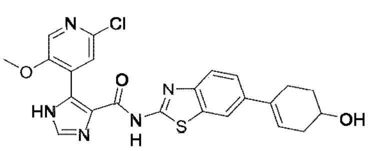,
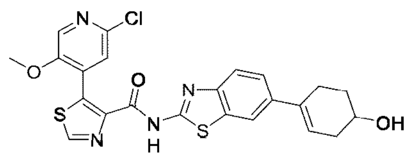,
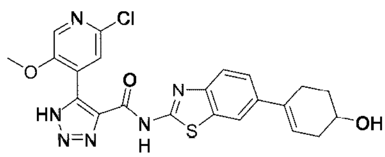,

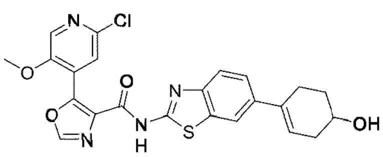,
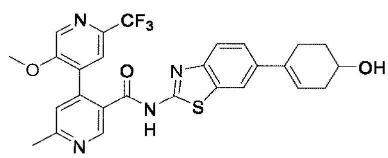,
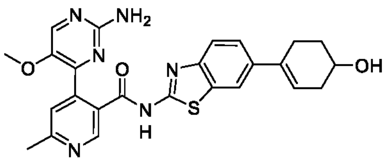,

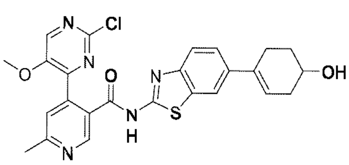,
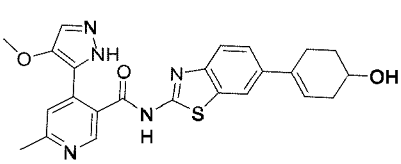,
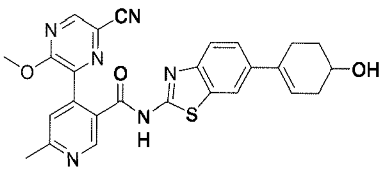,

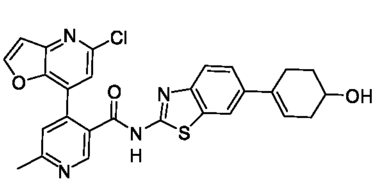,
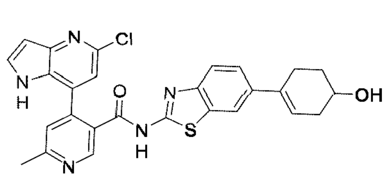,
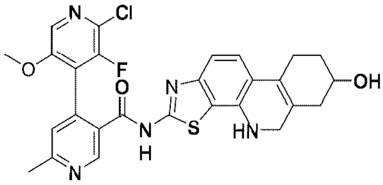,

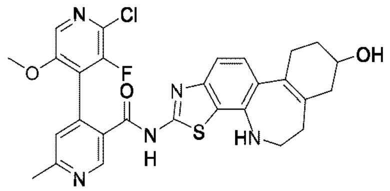 , 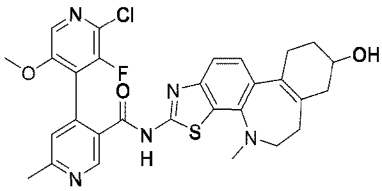 , 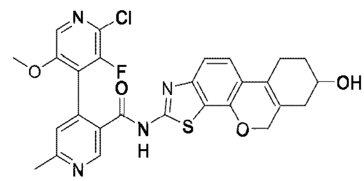 ,

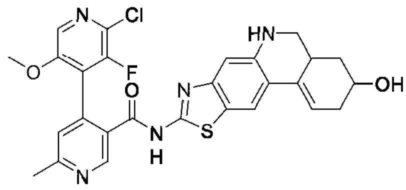 , 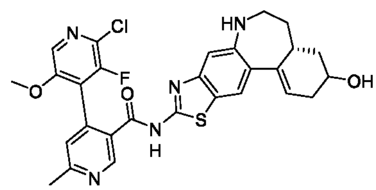 , 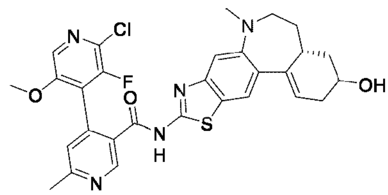 ,

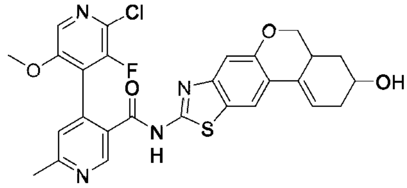 , 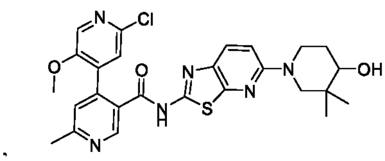 , 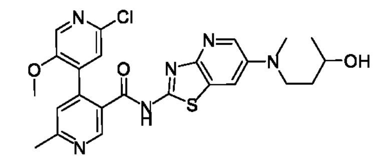

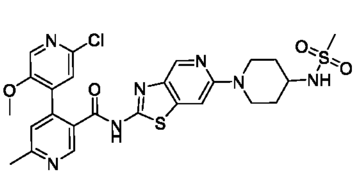 , 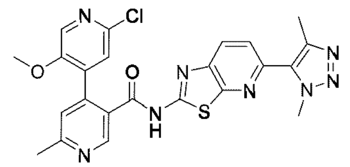 , 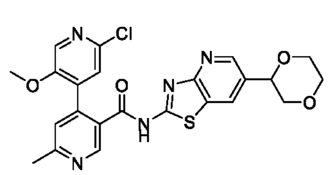 ,

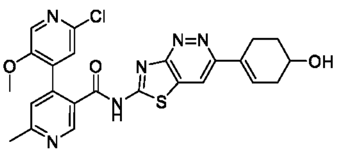 , 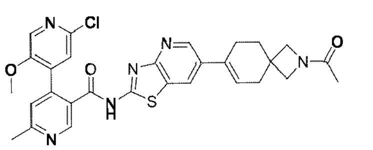 , 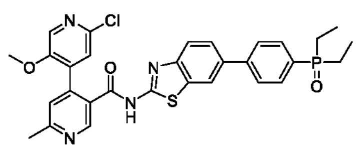 ,

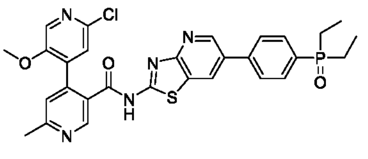 , 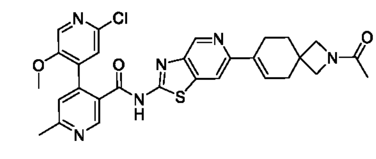 , 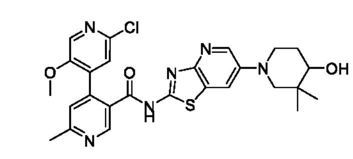 ,

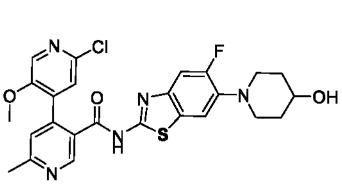 , 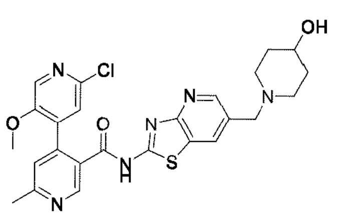 , 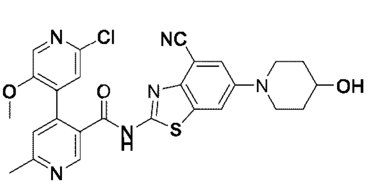 ,

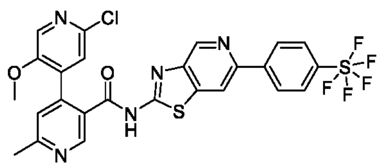 , 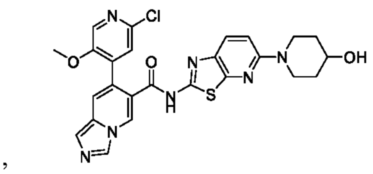 , 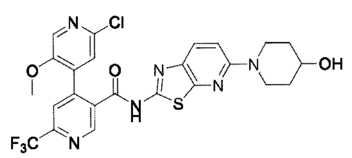 ,

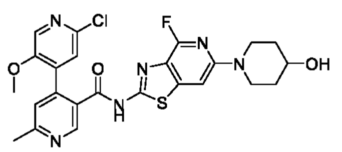 , 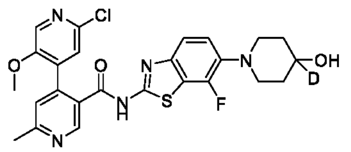 , 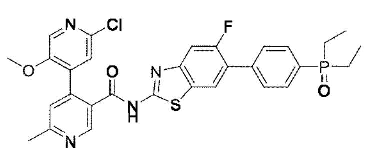 ,

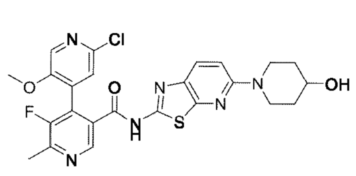 , 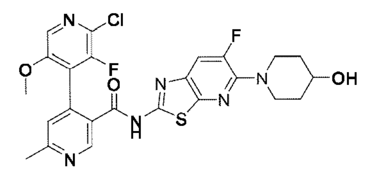 , 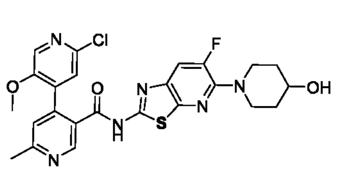 ,

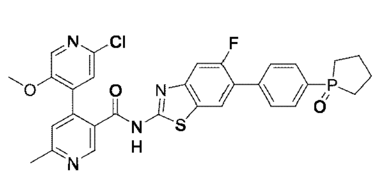 , 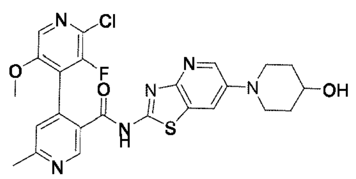 , 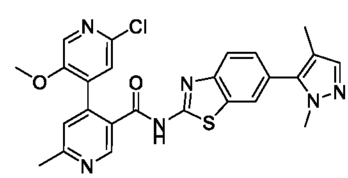 ,

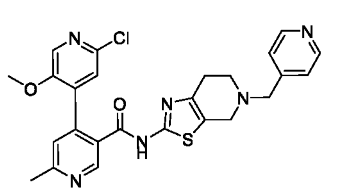 , 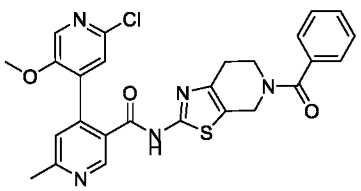 , 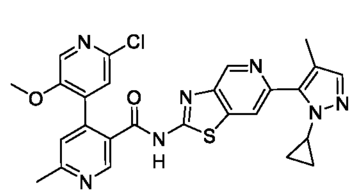 ,

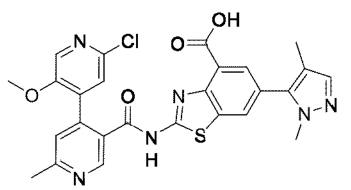 , 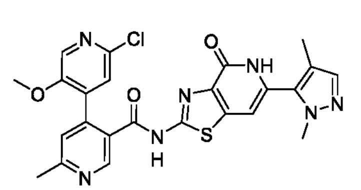 , 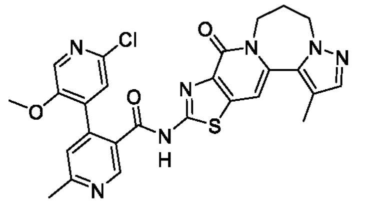 ,

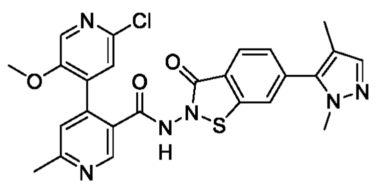 , 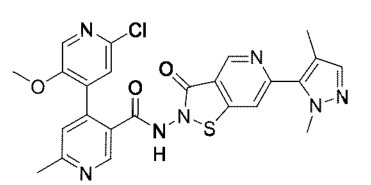 , 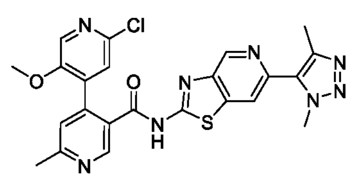 ,

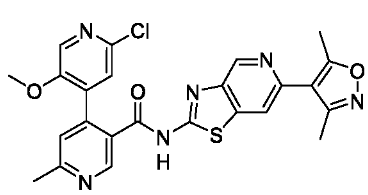 , 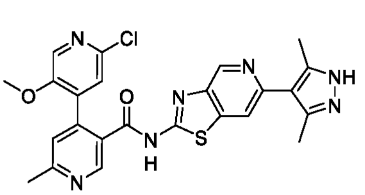 , 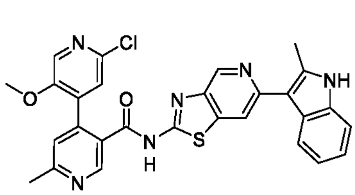 ,

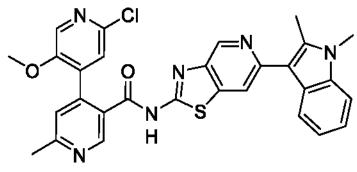,
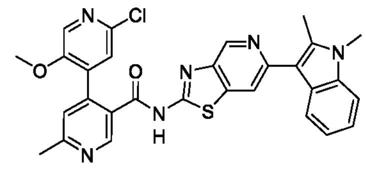,
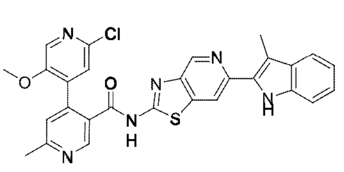,

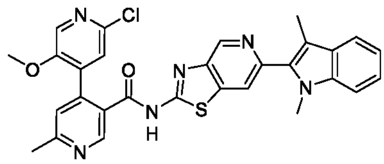,
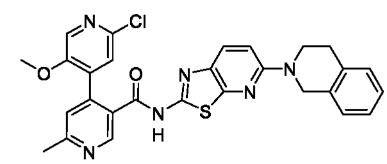,
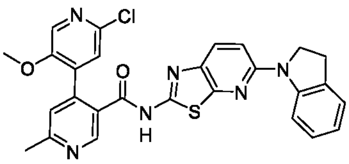,

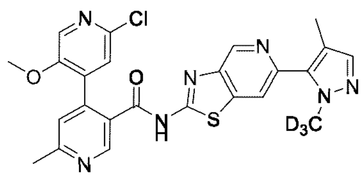,
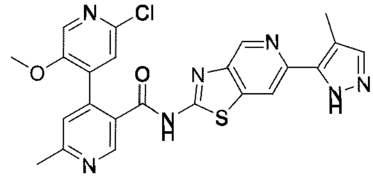,
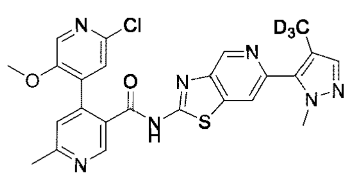,

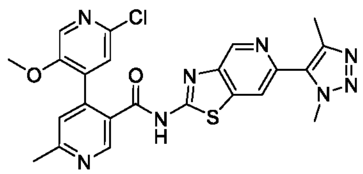,
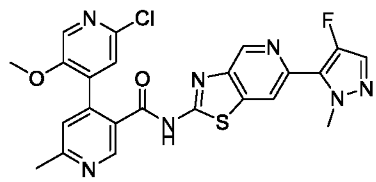,
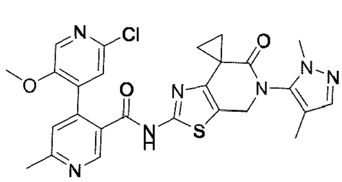,

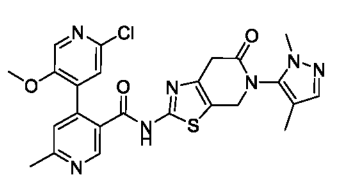,
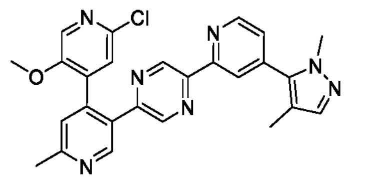,
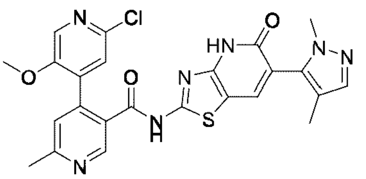,

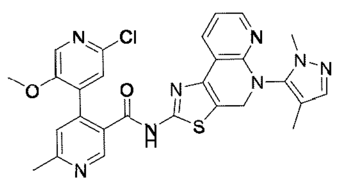,
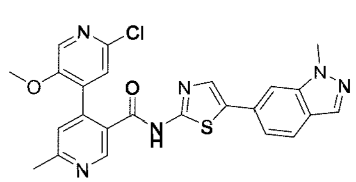,
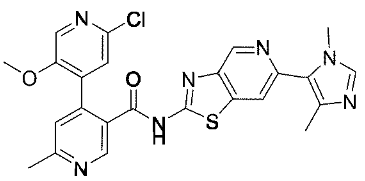,

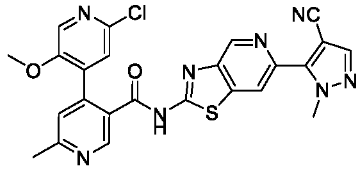,
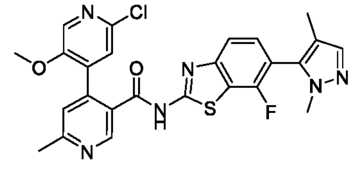,
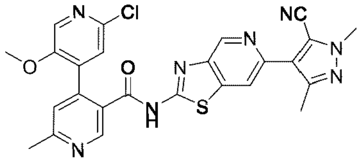,

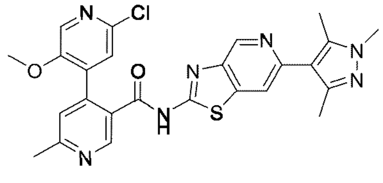,
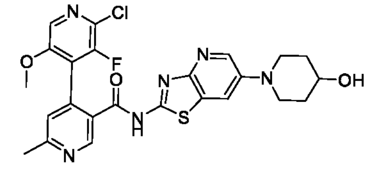,
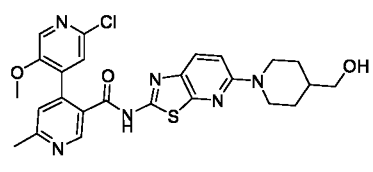,

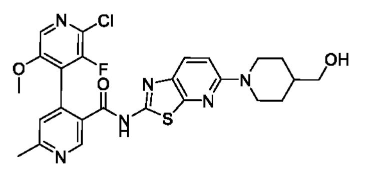,
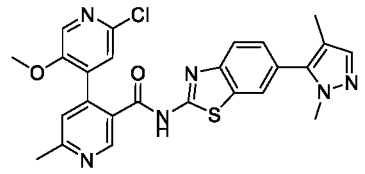,
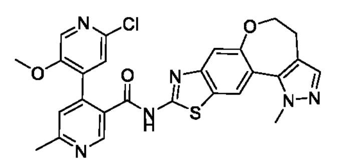,
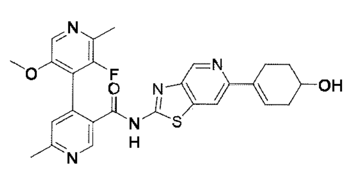,
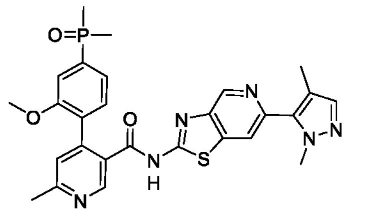,
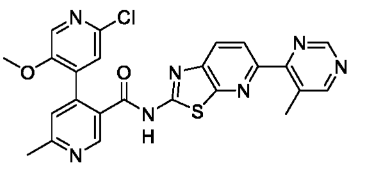,
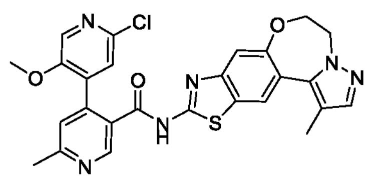,
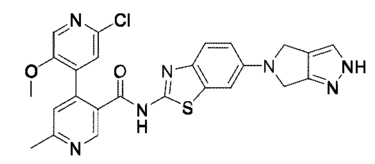,
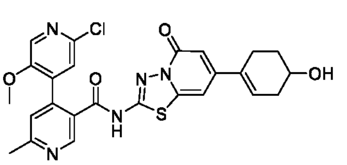,
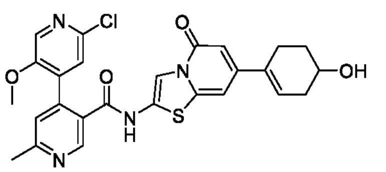,
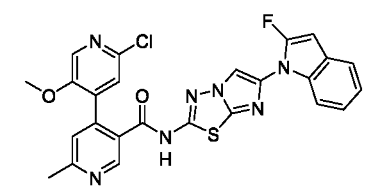,
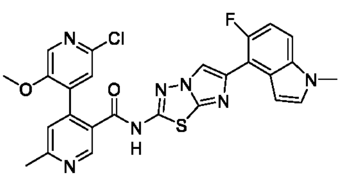,
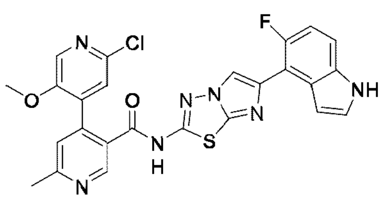,
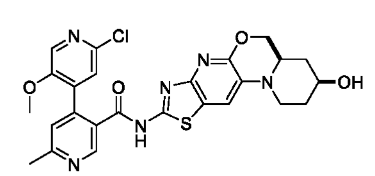,
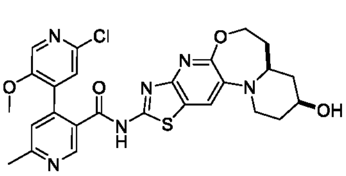,
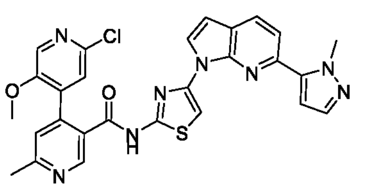,
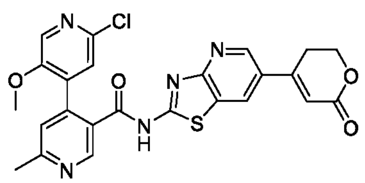,
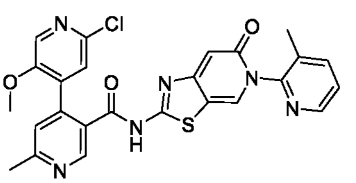,
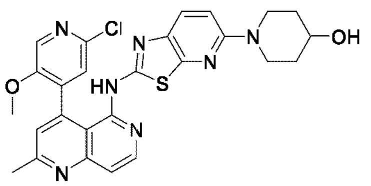,
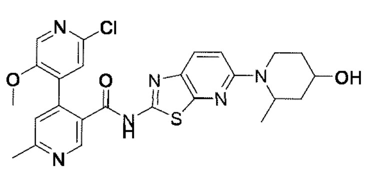,
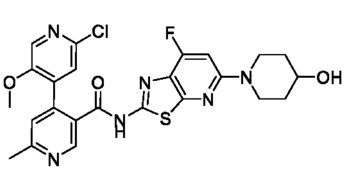,
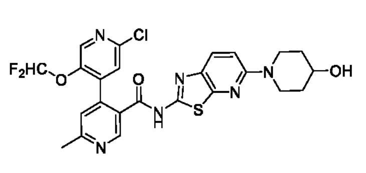,
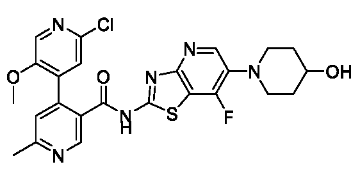,
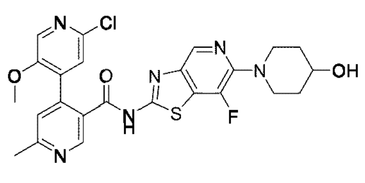,

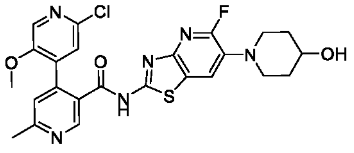 , 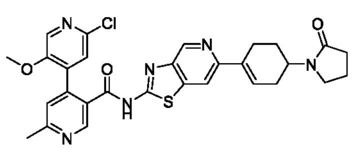 , 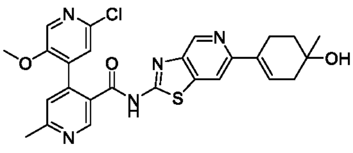 ,

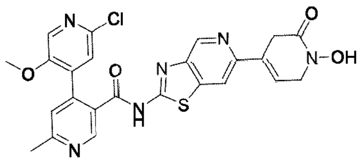 , 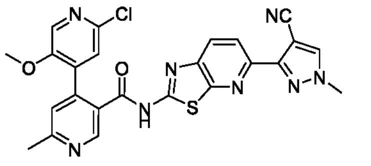 , 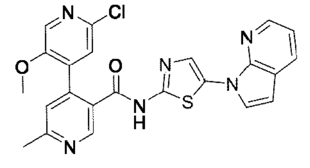 ,

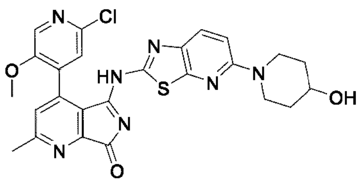 , 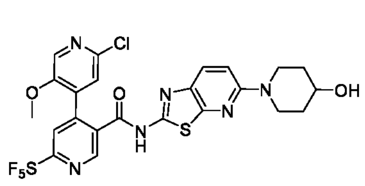 , 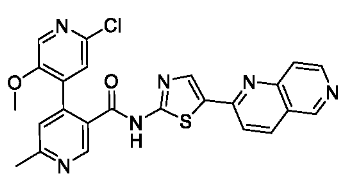 ,

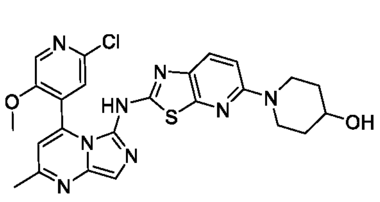 , 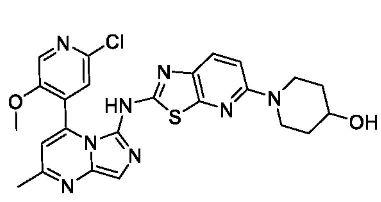 , 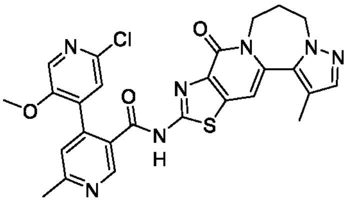 ,

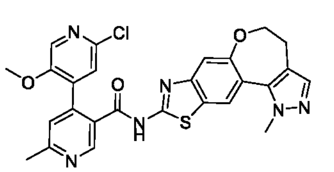 , 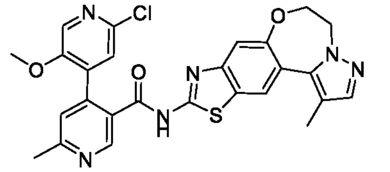 , 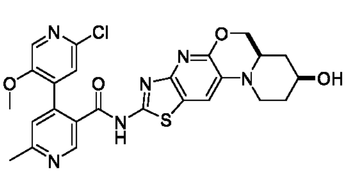 ,

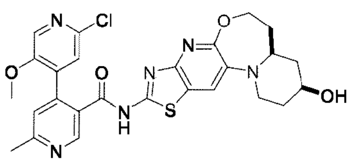 , 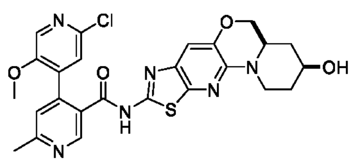 , 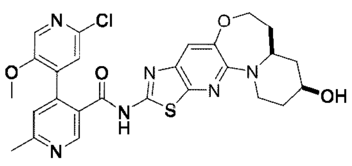 ,

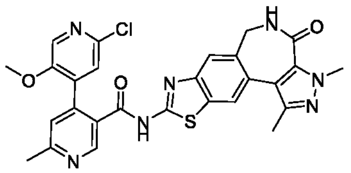 , 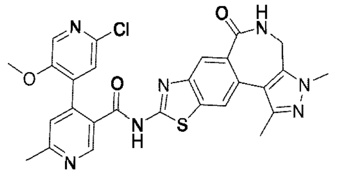 , 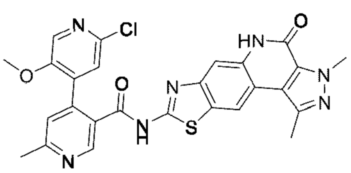 ,

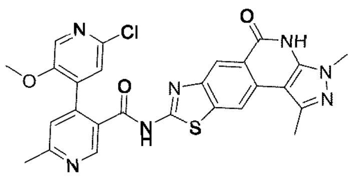 , 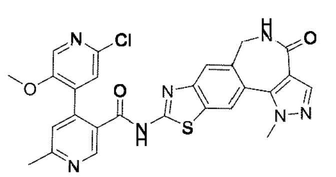 , 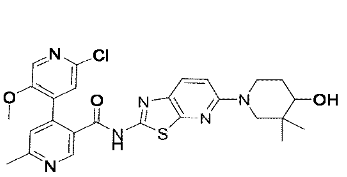 ,

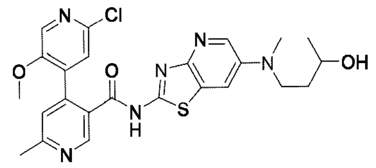 ,
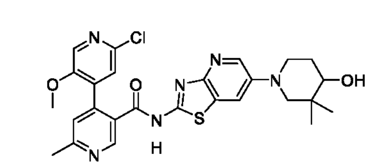 ,
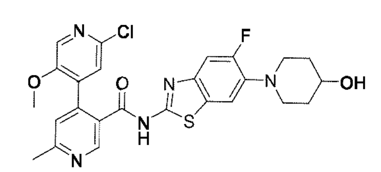 ,

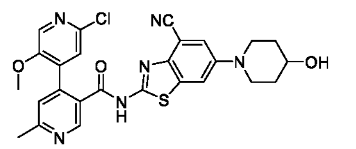 ,
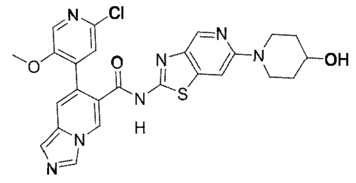 ,
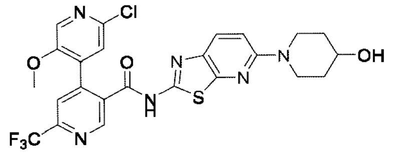 ,

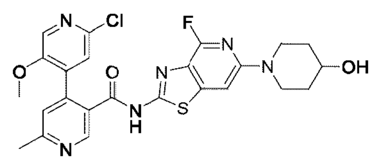 ,
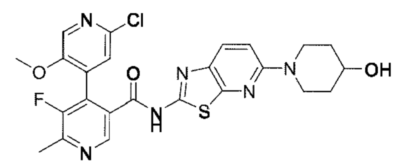 ,
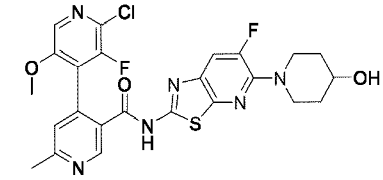 ,

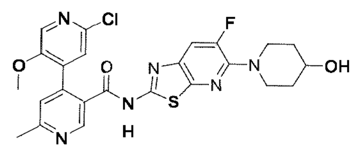 ,
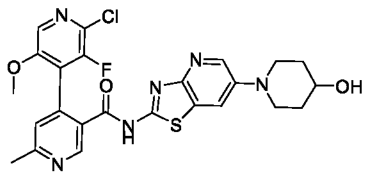 ,
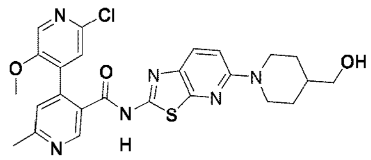 ,

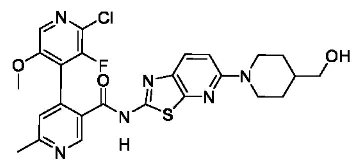 ,
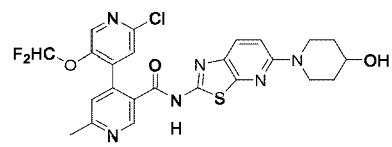 ,
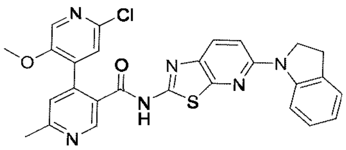 ,

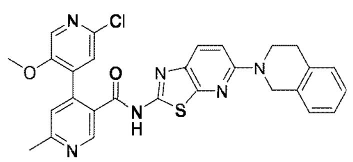 ,
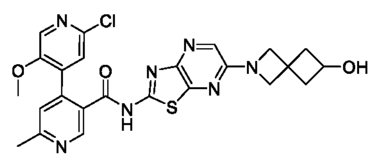 ,
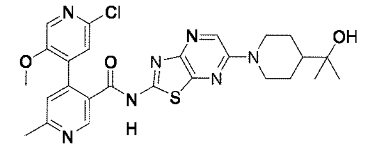 ,

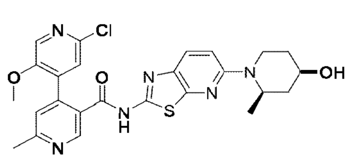 ,
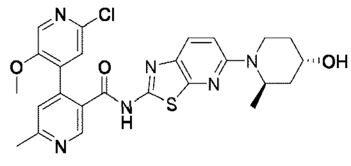 ,
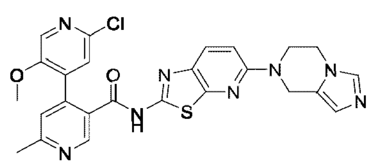 ,

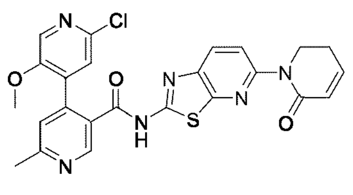 ,
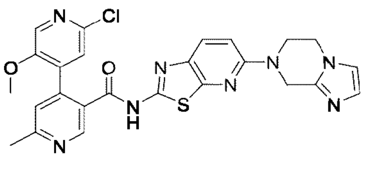 ,
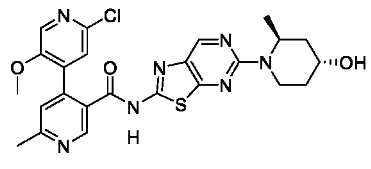 ,

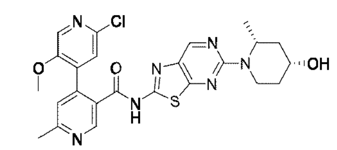 , 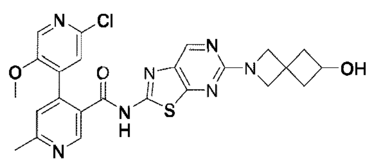 , 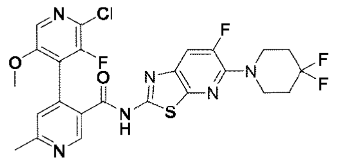 ,

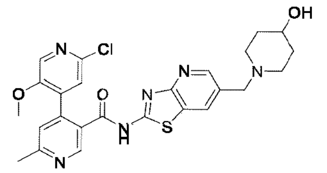 , 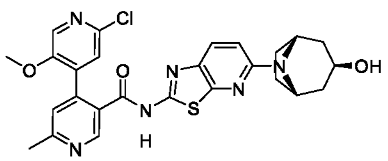 , 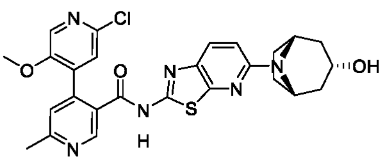 ,

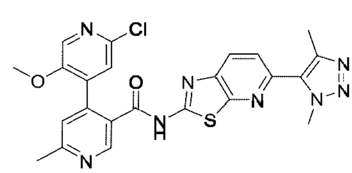 , 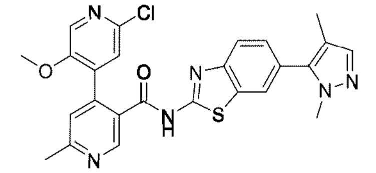 , 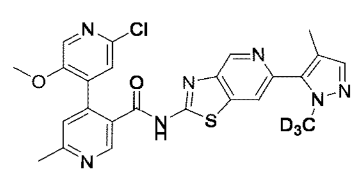 ,

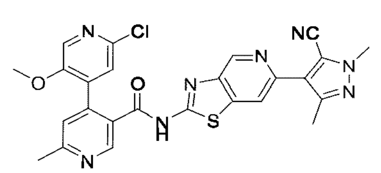 , 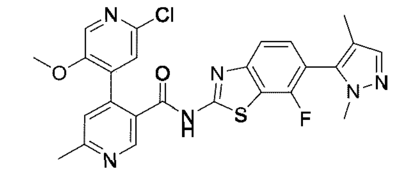 , 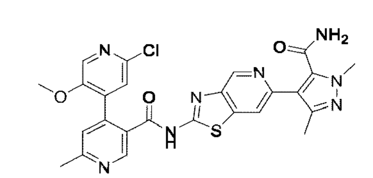 ,

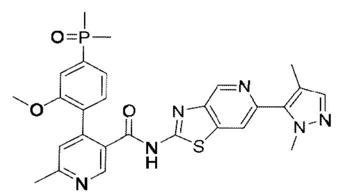 , 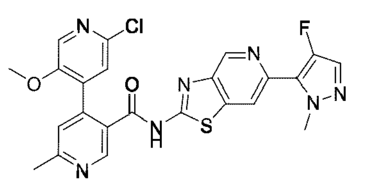 , 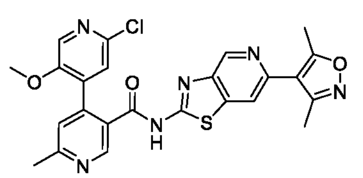 ,

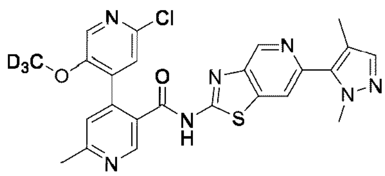 , 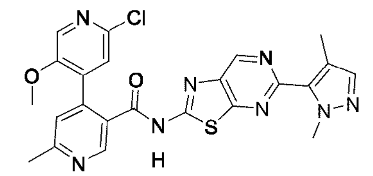 , 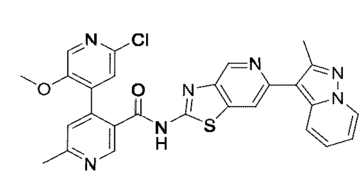 ,

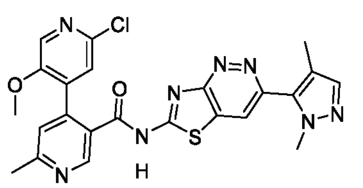 , 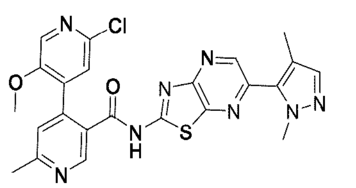 , 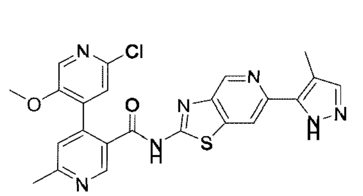 ,

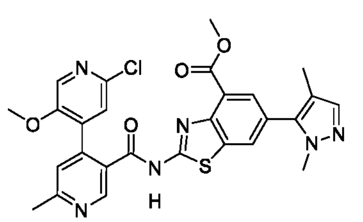 , 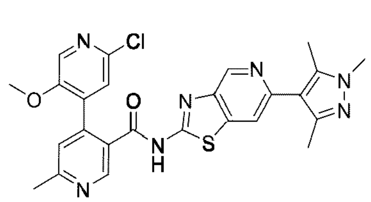 , 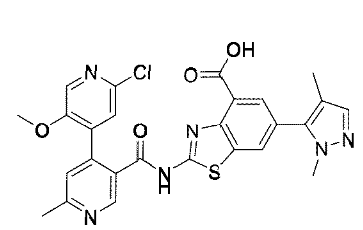 ,

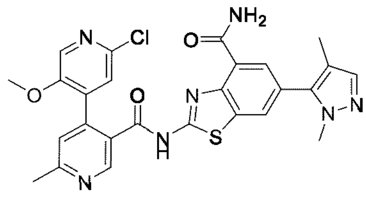,
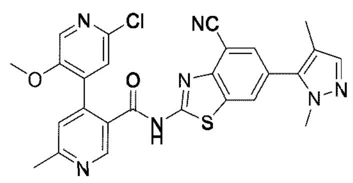,
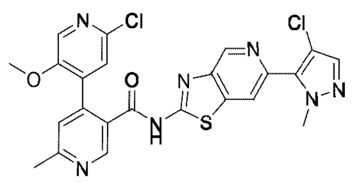,

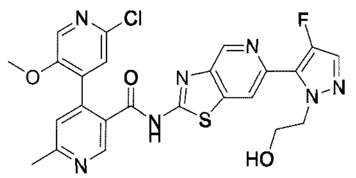,
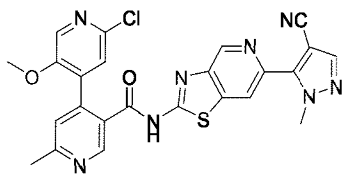,
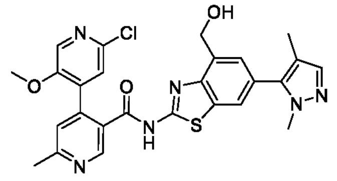,

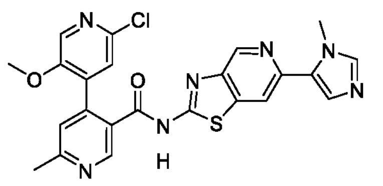,
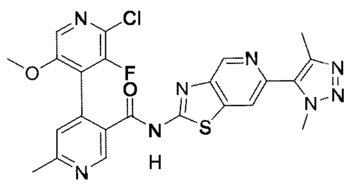,
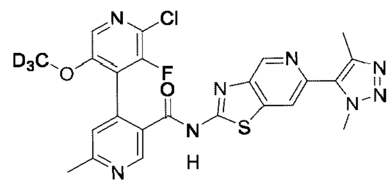,

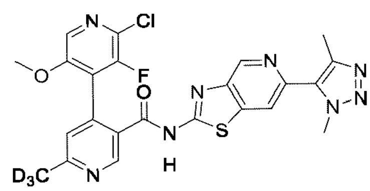,
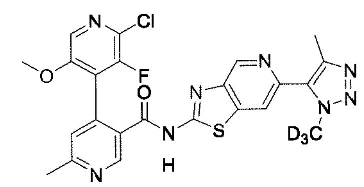,
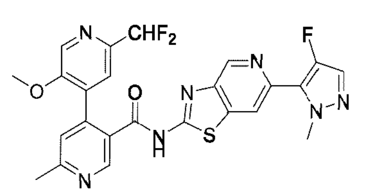,

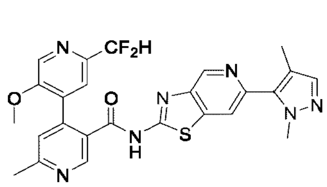,
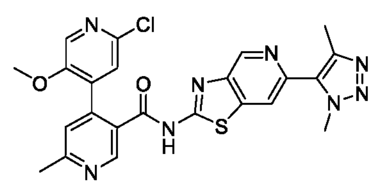,
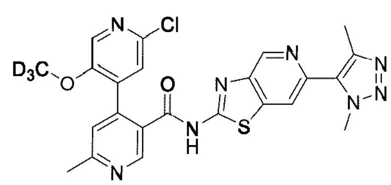,

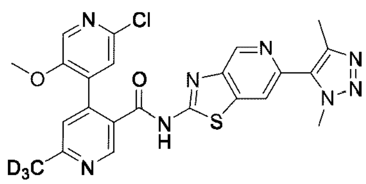,
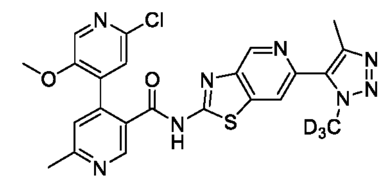,
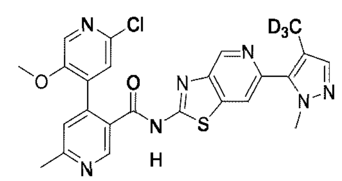,

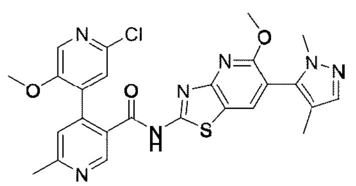,
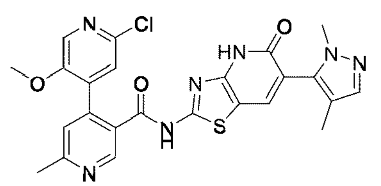,
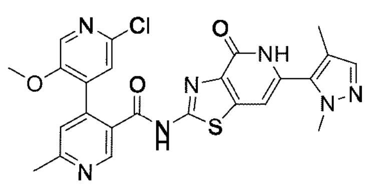,

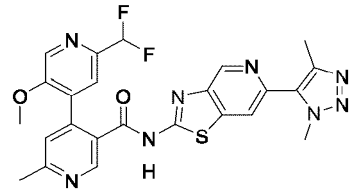,
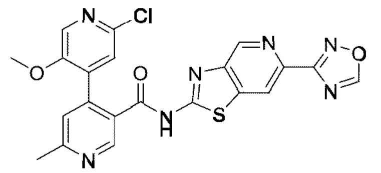,
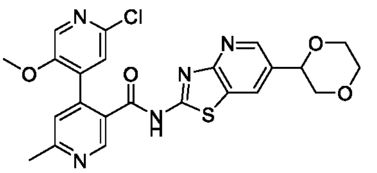,

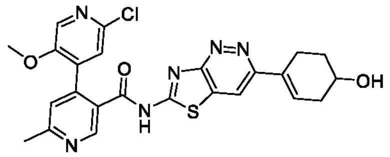,
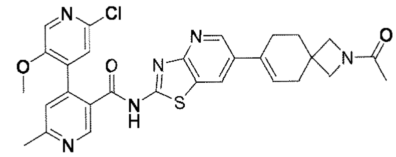,
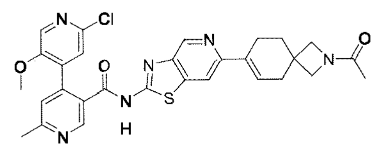,

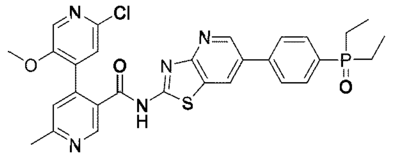,
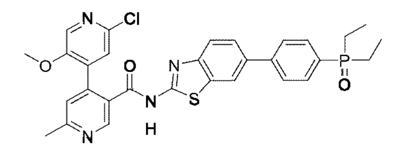,
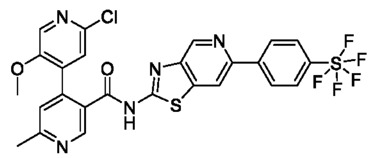,

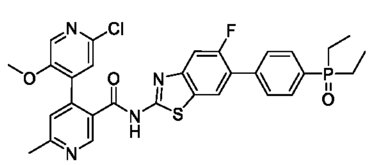,
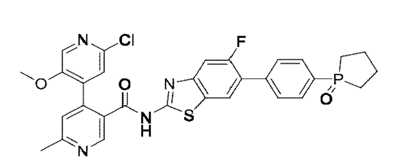,
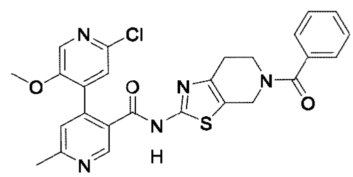,

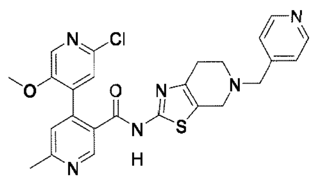,
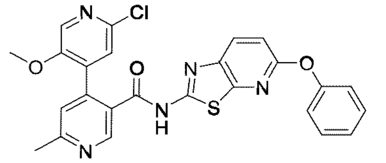,
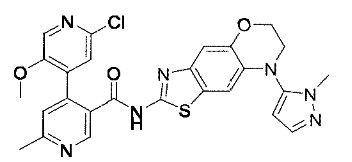,

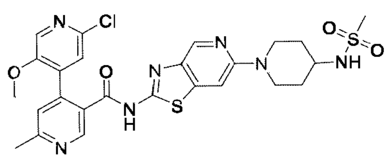,
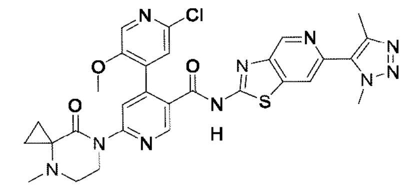,
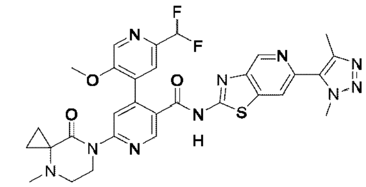,

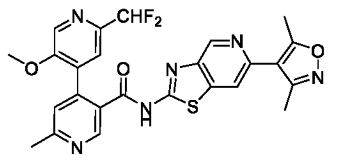,
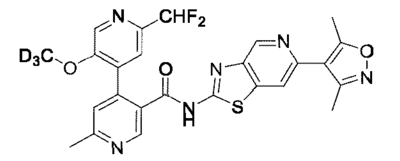,
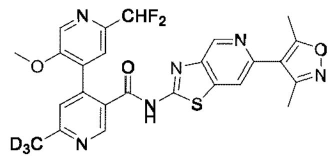,

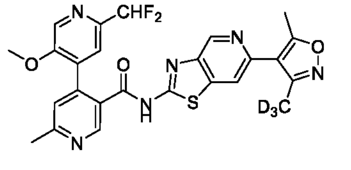,
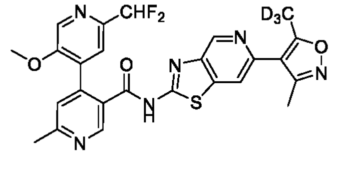,
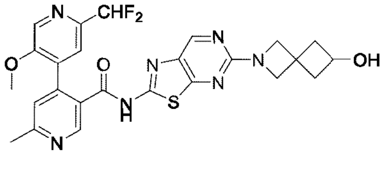,

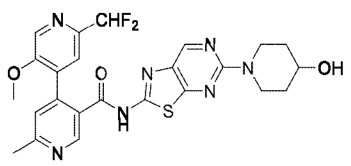,
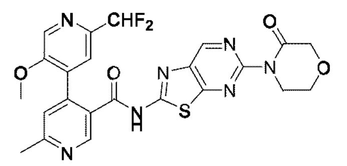,
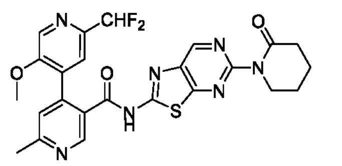,

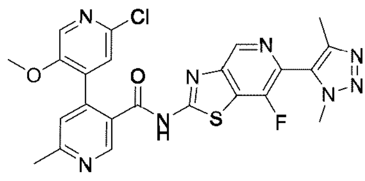,
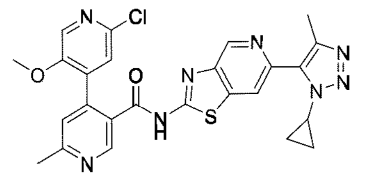,
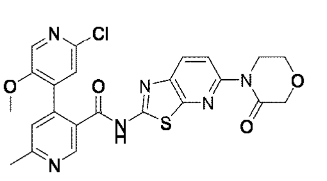,

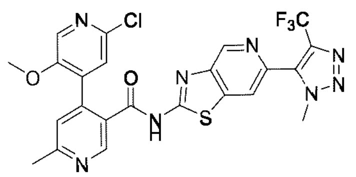,
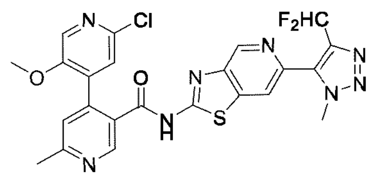,
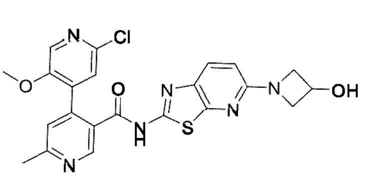,

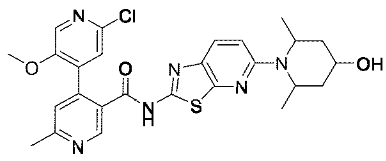,
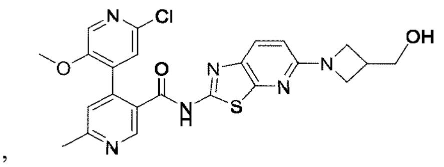,
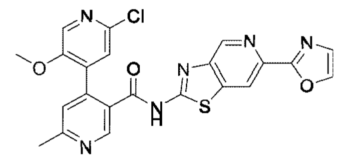,

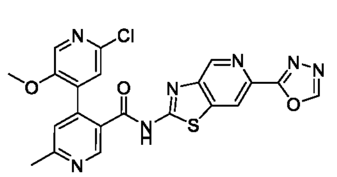,
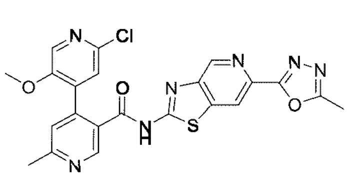,
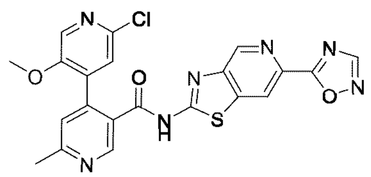,

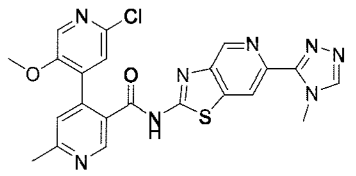,
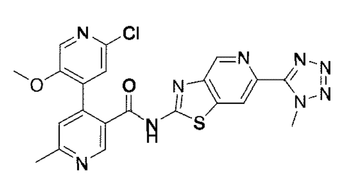,
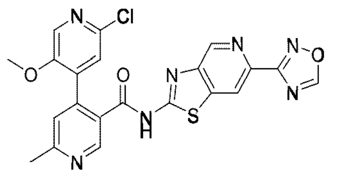,

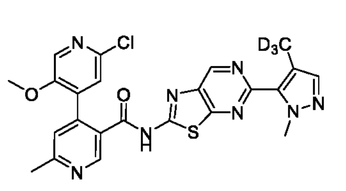,
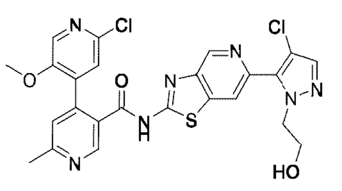,
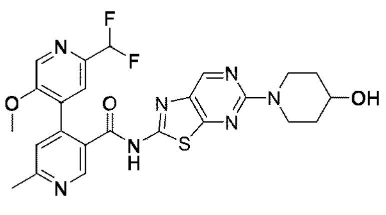,

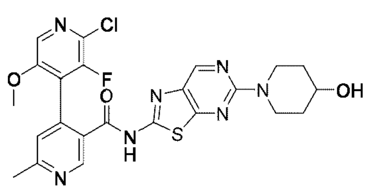,
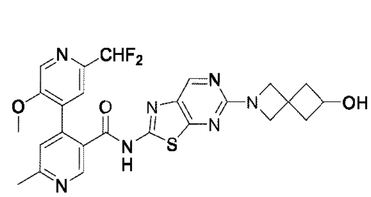,
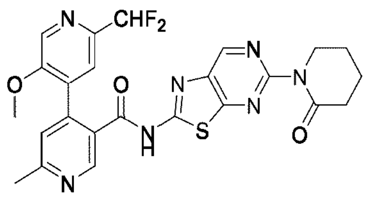,

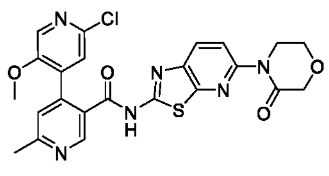,
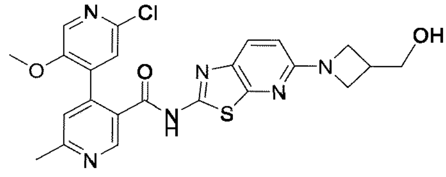,
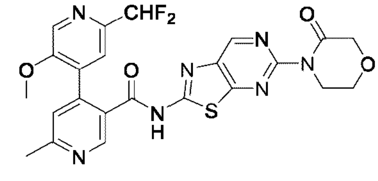,

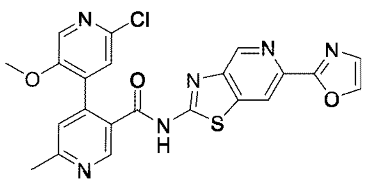,
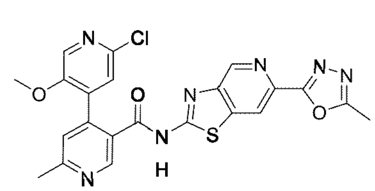,
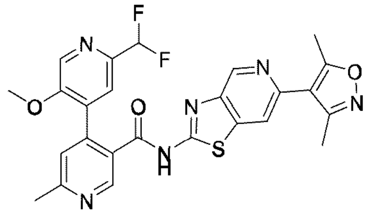,

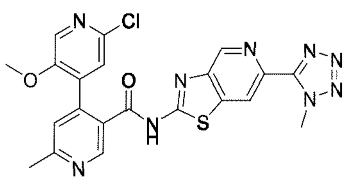,
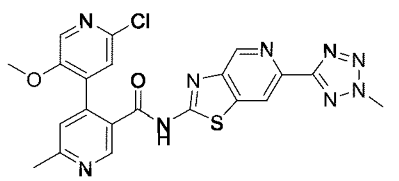,
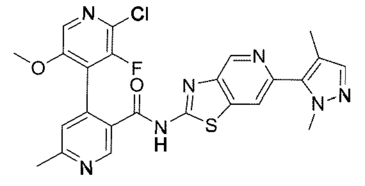,

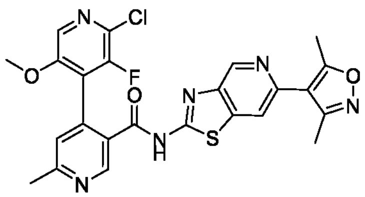,
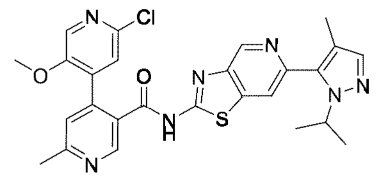,
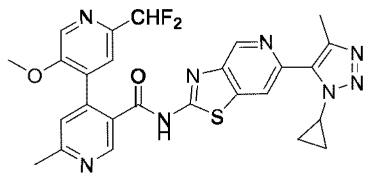,

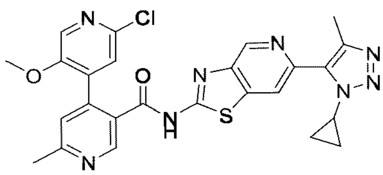,
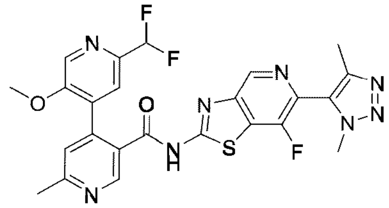,
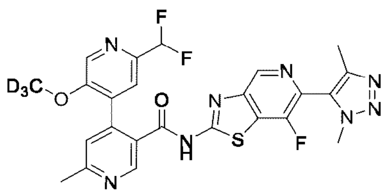,

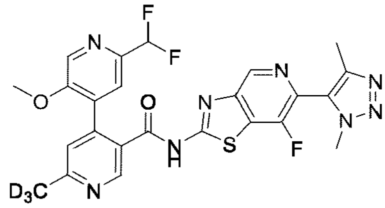,
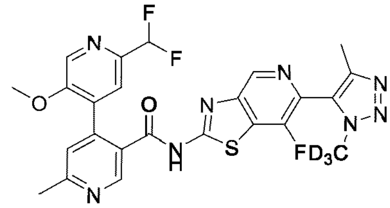,
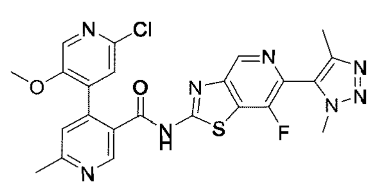,

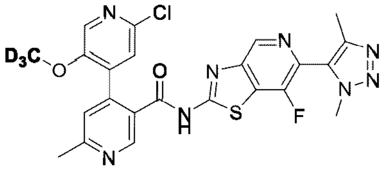,
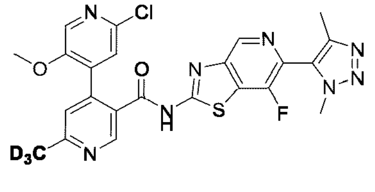,
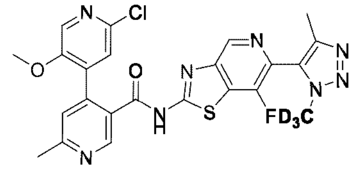,

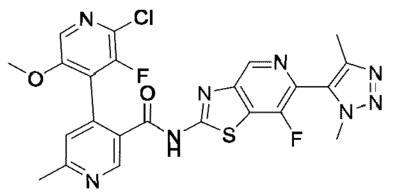, 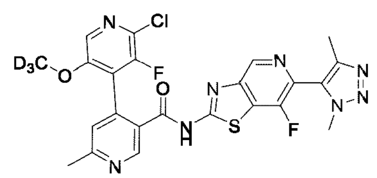, 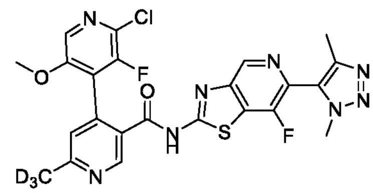,

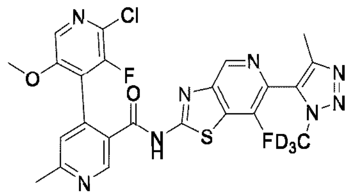, 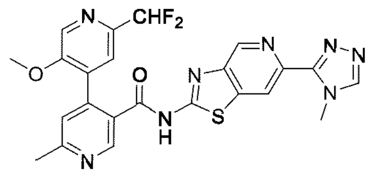, 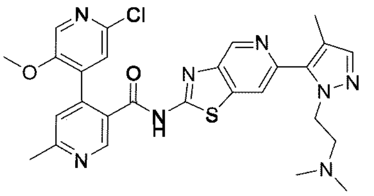,

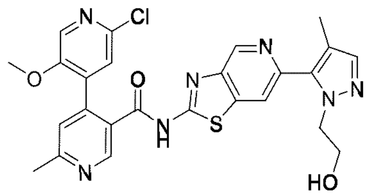, 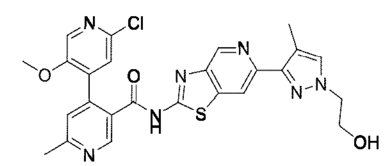, 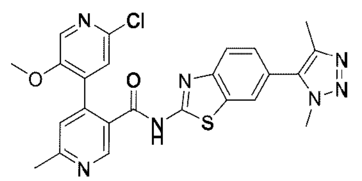,

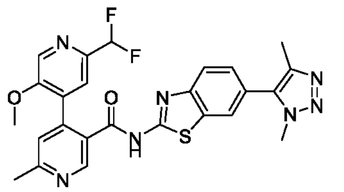, 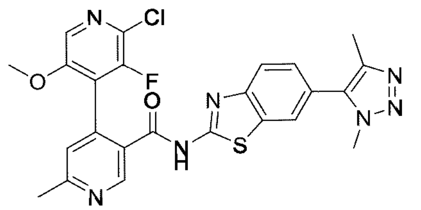, 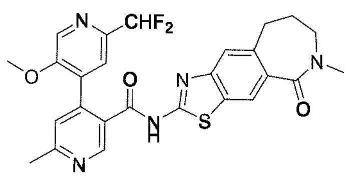,

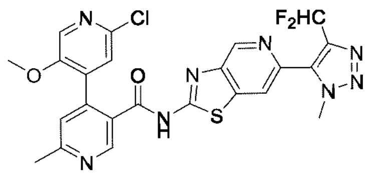, 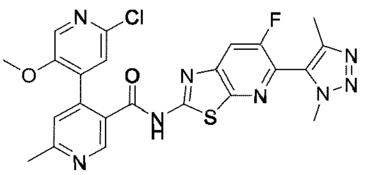, 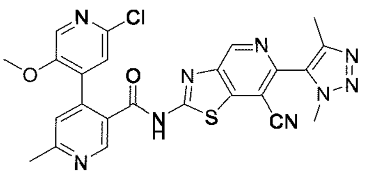,

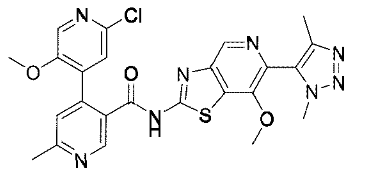, 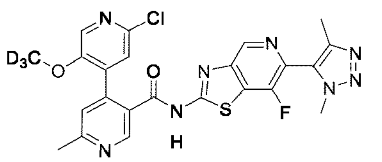, 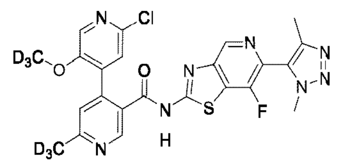,

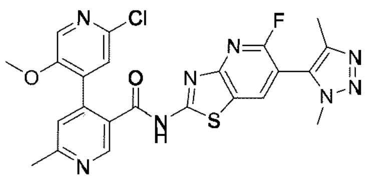 and 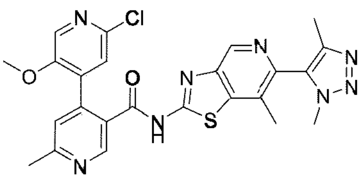.

The present invention provides a preparation method for a compound of formula I', including a scheme a, a scheme b or a scheme c;

the scheme a comprises the step of: subjecting a compound of formula II and a compound of formula III in a solvent to a condensation reaction shown below to obtain the compound of formula I',

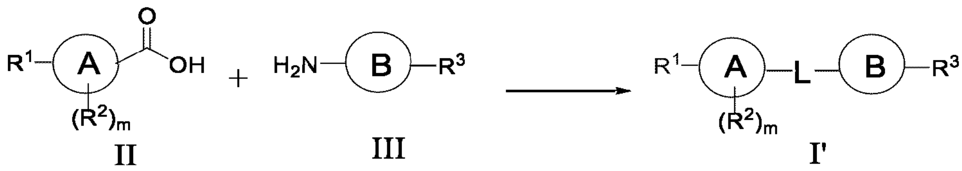

II III I' :

the scheme b comprises the steps of: (a) subjecting a compound of formula II and a compound of formula III' in a solvent to a condensation reaction shown below to obtain a compound of formula I",

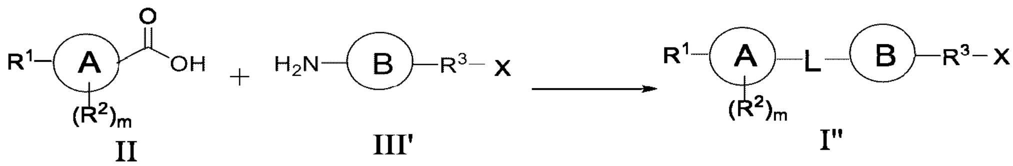

II III' I"

wherein X is dimethyl tert-butylsilyl ether (OTBS) or tert-butyloxycarboryl (Boc), and
(b) subjecting the compound of formula I" prepared by step (a) in a solvent in the presence of an acid to a deprotection reaction shown below to obtain the compound of formula I';

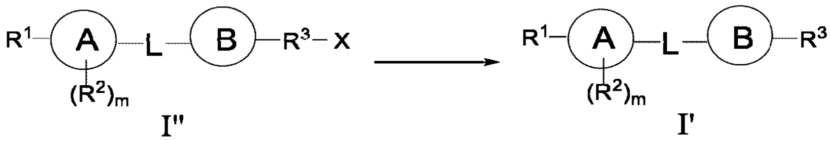

I" I'

the scheme c comprises the steps of: subjecting a compound of formula IV and a compound of formula V in a solvent in the presence of a condensing agent to a condensation reaction, to obtain the compound of formula I',

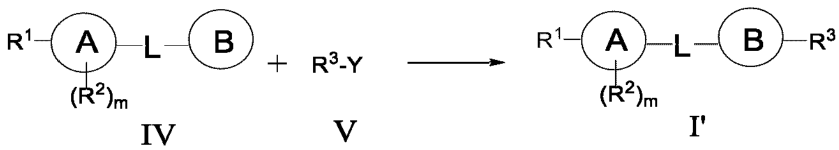

IV V I'

Y is hydrogen or carbonyl;
wherein $R^1$, $R^2$, m, a ring A, L, a ring B and $R^3$ are as previously defined.

In the scheme a or b, in the condensation reaction, a molar ratio of the compound of formula II to the compound of formula III may be 1:(0.5-1.5), for example, 1:1, 1:1.35 or 1:0.83.

In the scheme a or b, in the condensation reaction, a molar ratio of the compound of formula II to the compound of formula III may be 1:(0.5-1.5) , for example, 1:1 or 0.67.

In the scheme a or b, in the condensation reaction, the solvent is a conventional organic solvent in the art, preferably an amide solvent and/or a cyanogen solvent; the amide solvent is preferably N,N-dimethyl formamide; and the cyanogen solvent is preferably acetonitrile.

In the scheme a or b, in the condensation reaction, the condensing agent is a conventional condensing agent in the art, for example, N,N,N',N'-tetramethylchlorformamidine hexafluorphosphate and N-methylimidazole. In the scheme a or b, in the condensation reaction, a molar ratio of the compound of formula II to the condensing agent may be 1:(2-5), for example, 1:4 or 1:4.3, 1:3.7 or 1:4.1.

In the scheme a or b, in the condensation reaction, a reaction temperature for the condensation reaction is a conventional temperature for such a reaction in the art, for example, room temperature.

In the scheme a or b, the condensation reaction may further comprise post-treatment, which is a conventional post-treatment step for organic reaction in the art and may comprise the steps of: one or more of concentrating, washing with water, extracting, drying, column chromatography, and preparative chromatography.

In the scheme c, in the condensation reaction, when Y is carbonyl, a molar ratio of the compound of formula IV to the compound of formula V may be 1:(0.3-0.6) , for example, 1:0.46.

In the scheme c, in the condensation reaction, when Y is carbonyl, the solvent is a conventional organic solvent in the art, preferably an alcohol solvent, for example, methanol.

In the scheme c, in the condensation reaction, when Y is carbonyl, the condensing agent is a conventional condensing agent in the art, for example, sodium cyanoborohydride and tetraethyl titanate.

In the scheme c, in the condensation reaction, when Y is carbonyl, a molar ratio of the compound of formula IV to the condensing agent may be 1:(2-5), for example, 1:3.2.

In the scheme c, in the condensation reaction, when Y is carbonyl, the reaction temperature for the condensation reaction is a conventional temperature for such a reaction in the art, for example, room temperature.

In the scheme c, in the condensation reaction, when Y is carbonyl, the condensation reaction further comprises post-treatment, which may be a conventional post-treatment step for organic reactions in the art, and may comprise the steps of: one or more of filtering, concentrating and preparative chromatography.

In the scheme c, in the condensation reaction, when Y is hydrogen, a molar ratio of the compound of formula IV to the compound of formula V may be 1:(1-1.5) , for example, 1:1.49.

In the scheme c, in the condensation reaction, when Y is hydrogen, the solvent is a conventional solvent in the art, preferably an amide solvent and/or a cyanogen solvent; the amide solvent is preferably N,N-dimethyl formamide; and the cyanogen solvent is preferably acetonitrile.

In the scheme c, in the condensation reaction, when Y is hydrogen, the condensing agent is a conventional condensing agent in the art, for example, N,N,N',N'-tetramethylchlorformamidine hexafluorphosphate and N-methylimidazole.

In the scheme c, in the condensation reaction, when Y is hydrogen, a molar ratio of the compound of formula IV to the condensing agent may be 1:(2-5) , for example, 1:4.2.

In the scheme c, in the condensation reaction, when Y is hydrogen, a reaction temperature for the condensation reaction is a conventional temperature for such a reaction in the art, for example, room temperature.

In the scheme c, in the condensation reaction, when Y is hydrogen, the condensation reaction may further comprise post-treatment, which is a conventional post-treatment step for organic reactions in the art, and may comprise the steps of: one or more of extracting, drying, concentrating and preparative chromatography. The present invention further provides a compound of formula VI,

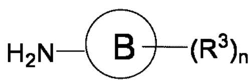

VI ;

wherein ring B is a 5-20 membered fused-ring heteroaromatic ring unsubstituted or substituted by one or more $R^{b-1}$; a heteroatom in the 5-20 membered fused-ring heteroaromatic ring is selected from one or more of N, O, and S, the number of heteroatom(s) is 1, 2, 3, 4, 5 or 6, a single heteroaromatic ring in the 5-20 membered fused-ring heteroaromatic ring is a 5-7 membered heteroaromatic ring, and the number of rings in the 5-20 membered fused-ring heteroaromatic ring is 3 or 4; and $R^3$ and n are as previously defined.

The present invention further provides a compound represented below:

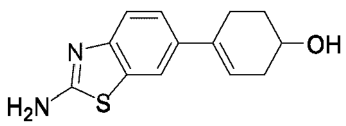 , 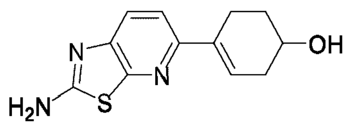 , 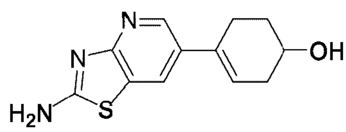 ,

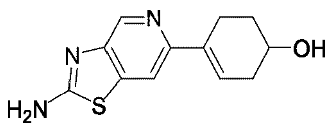 , 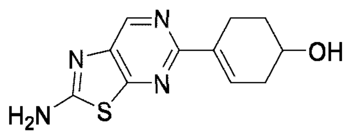 , 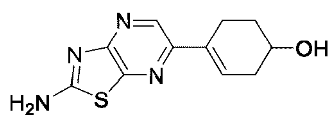 ,

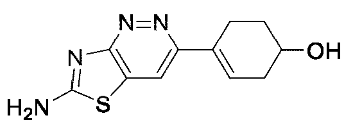 , 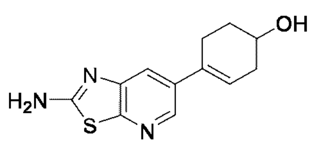 , 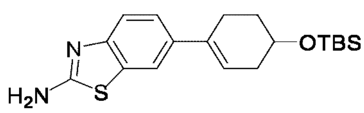 ,

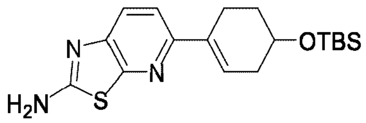 , 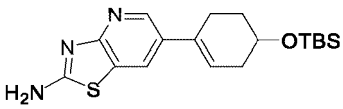 , 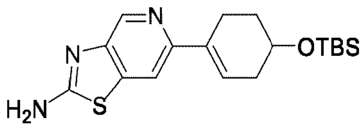 ,

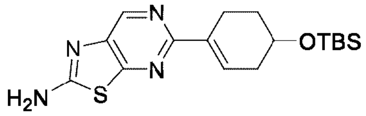 , 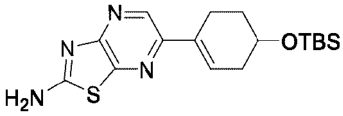 , 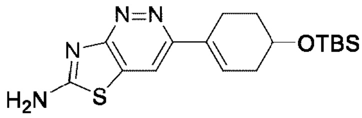 ,

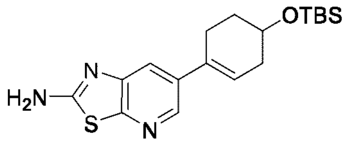 , 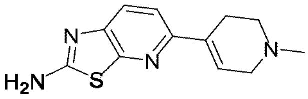 , 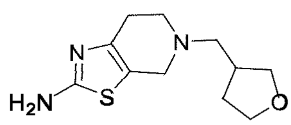 ,

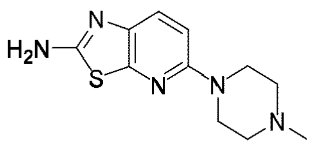 , 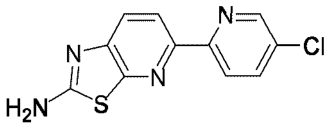 , 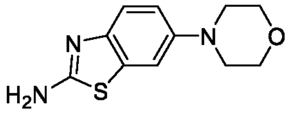 ,

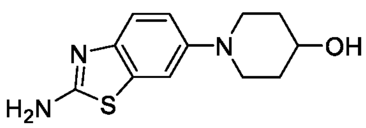 , 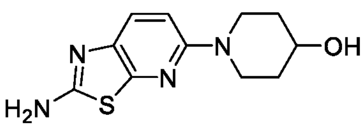 , 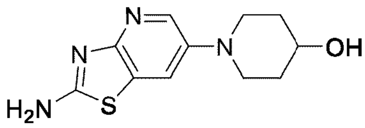 ,

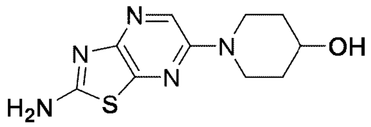 , 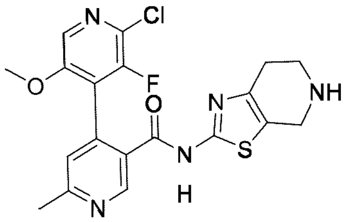 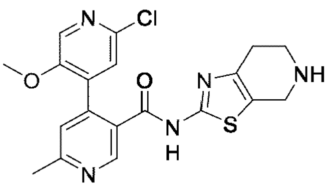 ,

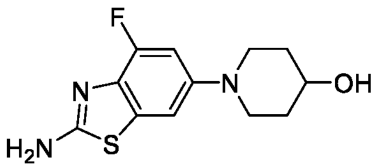 , 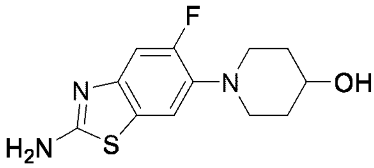 , 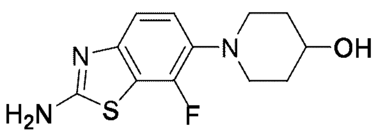 ,

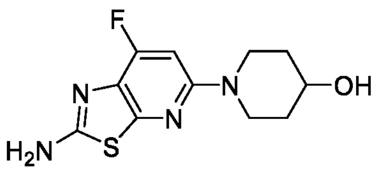 , 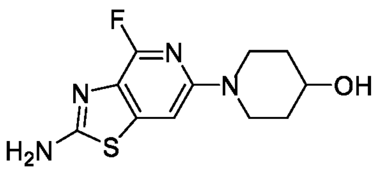 , 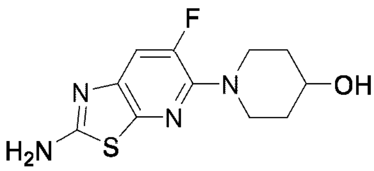 ,

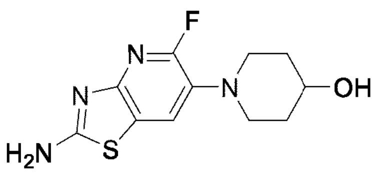 , 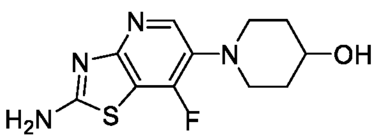 , 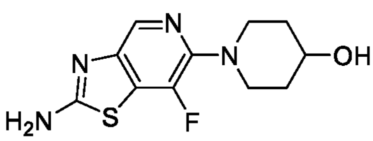 ,

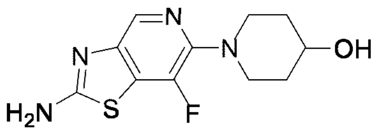 , 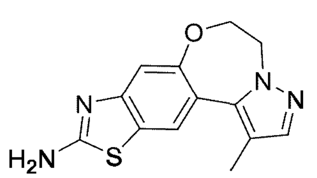 , 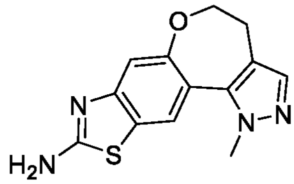 ,

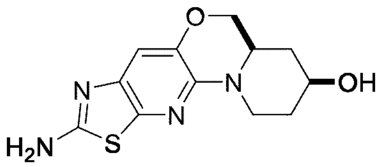 , 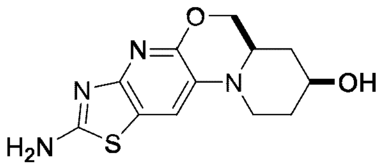 , 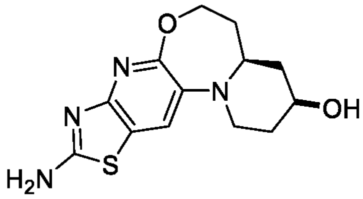 ,

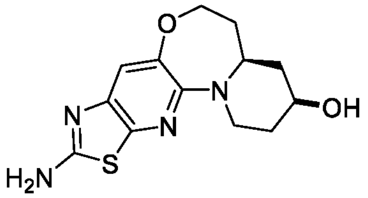 , 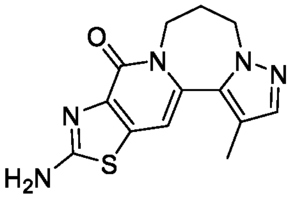 , 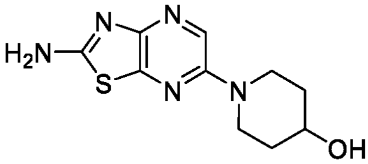 ,

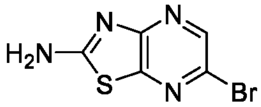 , 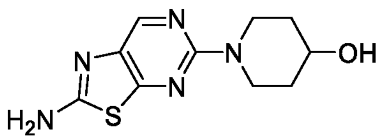 , 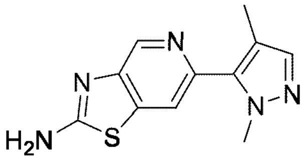 ,

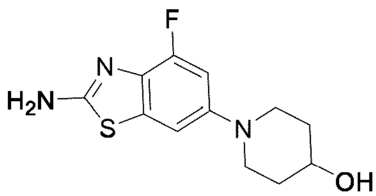 , 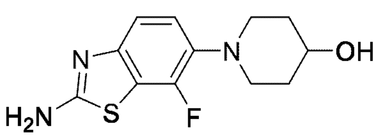 , 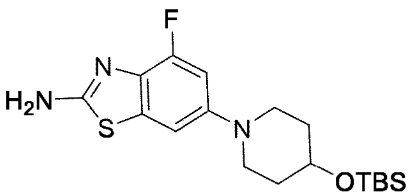 ,

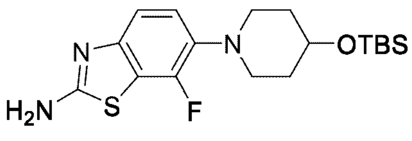 , 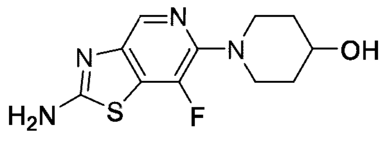 , 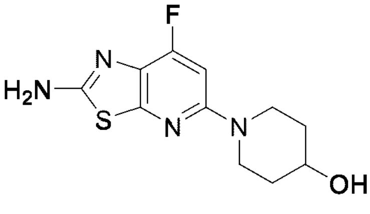 ,

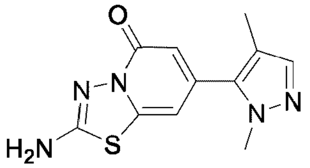 , 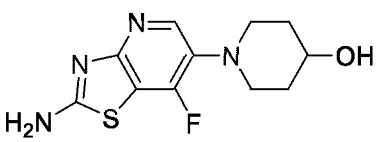 , 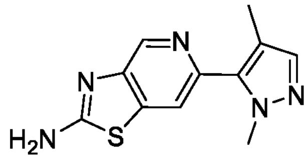 ,

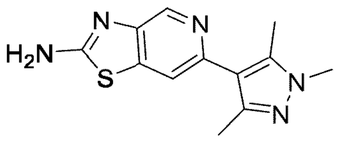 , 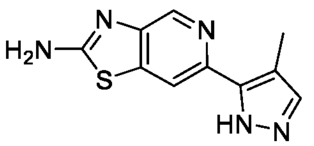 , 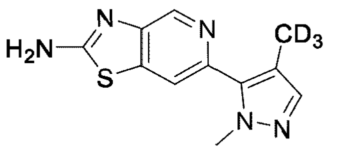 ,

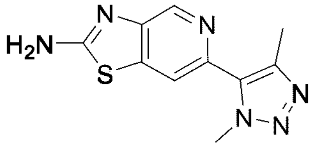 , 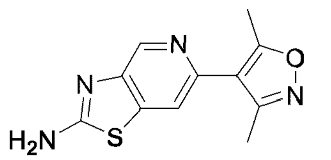 , 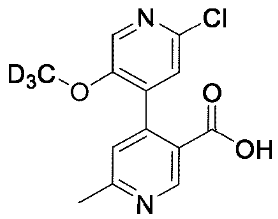 ,

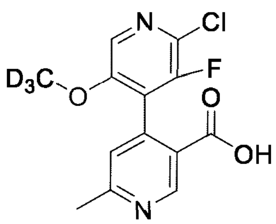 , 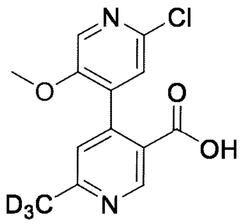 , 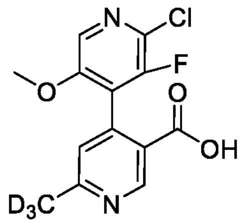 , 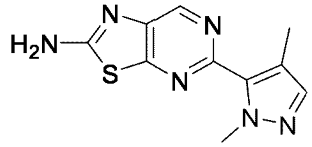 ,

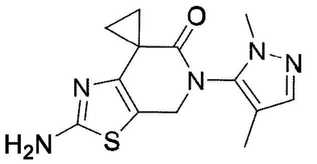 , 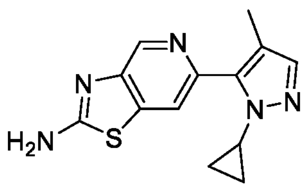 , 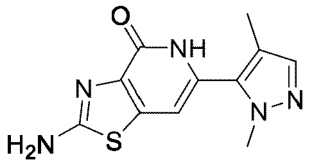 ,

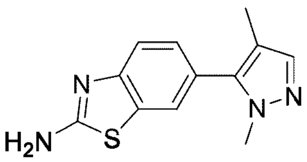 , 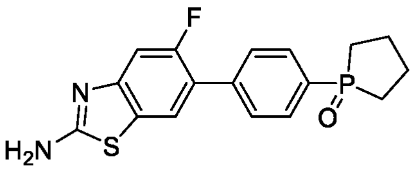 , 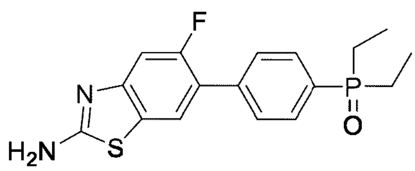 ,

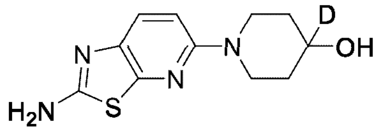 , 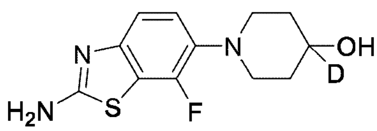 , 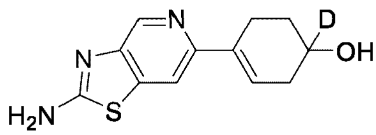 ,

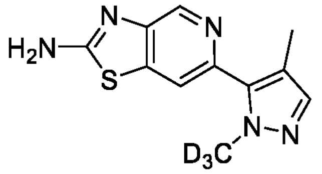 , 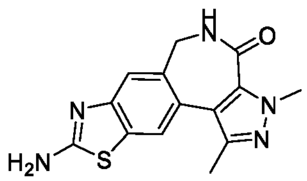 , 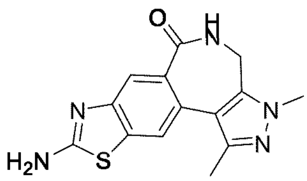 ,

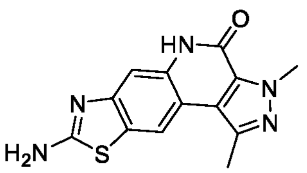 , 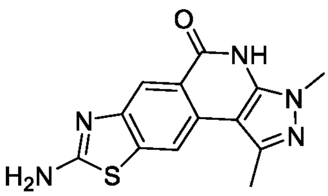 , 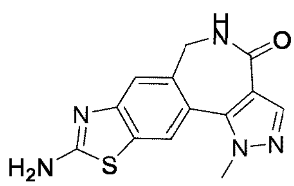 ,

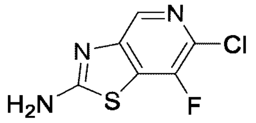, 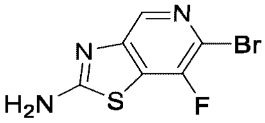, 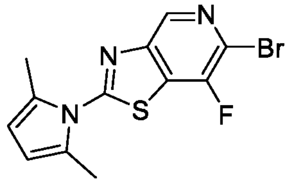, 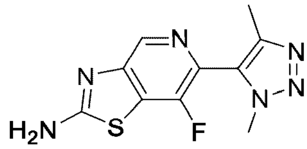,

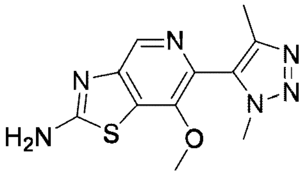, 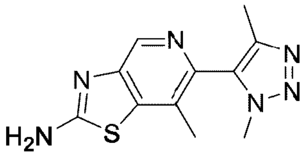 or 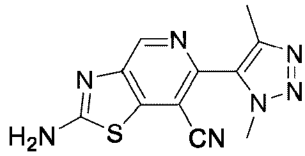.

The present invention provides a pharmaceutical composition, comprising:

(1) a (therapeutically effective amount of) substance **A**, wherein the substance **A** is the compound of formula **I**, the pharmaceutically acceptable salt thereof or the isotopic compound thereof as previously defined, and
(2) a pharmaceutically acceptable excipient.

The present invention provides use of a substance **A** in preparation of a polymerase theta inhibitor, wherein the substance **A** is the compound of formula **I**, the pharmaceutically acceptable salt thereof or the isotopic compound thereof as previously defined.

The present invention provides use of a substance **A** in preparation of a medicament, wherein the substance **A** is the compound of formula I, the pharmaceutically acceptable salt thereof or the isotopic compound thereof as previously defined; and the medicament may be for use in treatment of lung cancer, breast cancer, HR-deficient ovarian cancer, gastric cancer, prostate cancer, pancreatic cancer, or colon cancer.

Interpretation of Terms

The term "a group B unsubstituted or substituted by more than one a group A" means that one or more hydrogen atoms in the group B are independently substituted by the group A, or B is not substituted. When a plurality of groups A appear at the same time, their definitions are independent of each other without mutual influence, unless otherwise specified. For example, "$C_6$-$C_{10}$ aryl substituted by 3 halogens" means that $C_6$-$C_{10}$ aryl is to be substituted by 3 halogens, the definitions of the 3 halogens are independent of each other without mutual influence, and the halogens include but are not limited to:

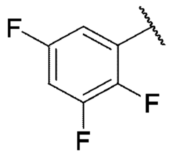, 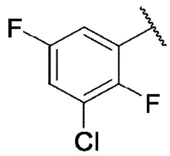

or the like.

The term "a plurality of" means 2 or more, for example 2, 3, 4, or 5.

The term "pharmaceutically acceptable" means a relatively non-toxic and safe condition suitable for use in patients.

The term "pharmaceutically acceptable salt" means the salt resulting from the reaction of a compound with a pharmaceutically acceptable acid or alkali. When the compound contains relatively acidic functional groups, an alkali addition salt may be obtained by contacting the compound with a sufficient amount of a pharmaceutically acceptable alkali in a suitable inert solvent. The pharmaceutically acceptable alkali addition salt includes, but is not limited to: a sodium salt, a potassium salt, a calcium salt, an aluminum salt, a magnesium salt, a bismuth salt, an ammonium salt or the like. When the compound contains relatively alkaline functional groups, an acid addition salt may be obtained by contacting the compound with a sufficient amount of a pharmaceutically acceptable acid in a suitable inert solvent. The pharmaceutically acceptable acid addition salt includes, but is not limited to: hydrochloride, sulfate, trifluoroacetate, formate, mesylate or the like. For details, see Handbook of Pharmaceutical Salts: Properties, Selection, and Use (P. Heinrich Stahl, Camille G. Wermuth, 2011, 2nd Revised Edition).

The "-" in the group means the fact that the group is attached to the rest of the molecule via this site. For example, $CH_3$-C(=O)- means acetyl.

The term "halogen" means fluorine, chlorine, bromine or iodine. Halogen substitution in the present invention includes, but is not limited to, substitution by one, two, or three halogens, and in general, multiple substitutions occur on one carbon atom.

The term "oxo" means =O, indicating the substitution of two hydrogen on the same carbon atom by an oxygen atom. That is, a methylene group is substituted by a carbonyl group.

The term "alkyl" means a linear or branched saturated monovalent hydrocarbon group with a specified number of carbon atoms (for example, $C_1$-$C_6$). Alkyl groups include, but are not limited to: methyl, ethyl, n-propyl, isopropyl, n-butyl, isobutyl, sec-butyl, tert-butyl, n-amyl, n-hexyl or the like.

The term "alkoxy" means the group $R^X$-O-, and $R^X$ is defined in the same way as the term "alkyl". Alkoxy groups include, but are not limited to: methoxy, ethoxy, n-propoxy, isopropoxy or the like.

The term "alkenyl" means a linear or branched unsaturated monovalent hydrocarbon group with a specified number of carbon atoms (for example, $C_2$-$C_6$), with one or more (for example, 1, 2, or 3) carbon-carbon $sp^2$ double bonds. Alkenyl groups include, but are not limited to: vinyl,

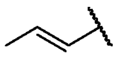, 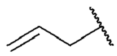

or the like.

The term "alkynyl" means a linear or branched unsaturated monovalent hydrocarbon group with a specified number of carbon atoms (for example, $C_2$-$C_6$), with one or more (for example, 1, 2, or 3) carbon-carbon $sp^3$ triple bonds. Alkynyl groups include, but are not limited to: acetylenyl,

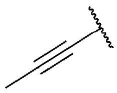, 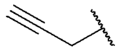

or the like.

Unless otherwise specially specified in the present invention, the term "cycloalkyl" means a cyclic saturated monovalent hydrocarbon group with a specified number of carbon atoms (for example, 3-10 membered), which is monocyclic. Monocyclic rings include, but are not limited to:

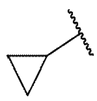, 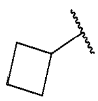, 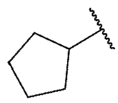,

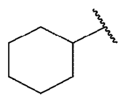

or the like.

Unless otherwise specially specified in the present invention, the term "aryl" means a cyclic unsaturated monovalent hydrocarbon group with a specified number of carbon atoms (for example, 6-10 membered), which is monocyclic or polycyclic (for example, 2 or 3 rings). In case of a polycyclic ring, monocyclic rings share two atoms and a bond, and is (at least one/each ring is) aromatic. The aryl group is attached to the rest of a molecule by a ring that is aromatic or not. Aryl groups include, but are not limited to: phenyl, naphthyl,

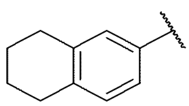, 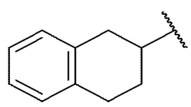

or the like.

Unless otherwise specially specified in the present invention, the term "cycloalkenyl" means a cyclic unsaturated monovalent hydrocarbon group with a specified number of carbon atoms (for example, 4-10 membered), which is monocyclic or polycyclic (for example, 2 or 3 rings, or a bridged, spiro or fused ring). Monocyclic cycloalkenyl groups

include, but are not limited to:

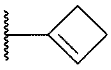, 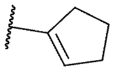, 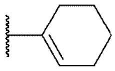

or the like. Bridged-ring cycloalkenyl groups include, but are not limited to:

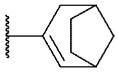

or the like. Spiro-ring cycloalkenyl groups include, but are not limited to,

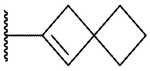, 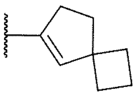, 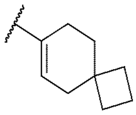,

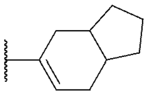

or the like. Fused-ring cycloalkenyl groups include, but are not limited to,

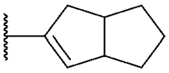

or the like.

Unless otherwise specially specified in the present invention, the term "heterocyclyl" means a monocyclo, bridged, spiro, or fused ring with a specified number of cyclic atoms (for example, 3-10 membered), a specified number of heteroatom(s) (for example, 1, 2 or 3), and a specified heteroatomic species (one or more of N, O and S). A monocyclic heterocyclyl group is attached to the rest of a molecule by a carbon atom or heteroatom. The monocyclic-ring heterocyclyl includes but is not limited to:

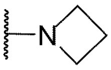, 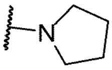,

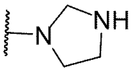, 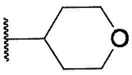, 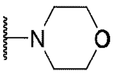, 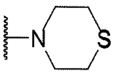, 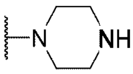, 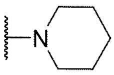 or 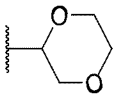

etc. The spiro-ring heterocyclyl includes but is not limited to:

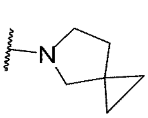, 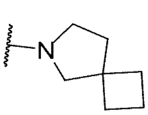, 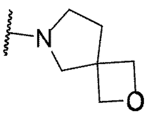,

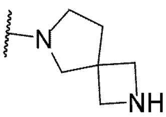, 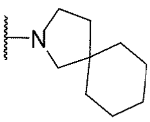, 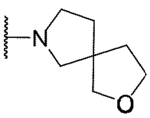, 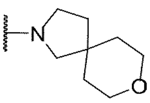, 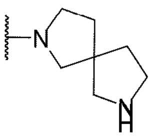, 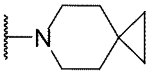, 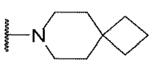,

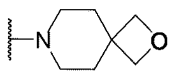, 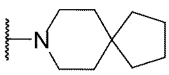 or 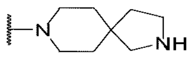

etc. The bridged-ring heterocyclyl includes but is not limited to:

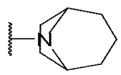

etc. The fused-ring heterocyclyl includes but is not limited to

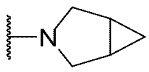,

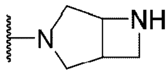 or 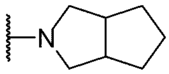

etc.

Unless otherwise specially specified in the present invention, the term "heterocycloalkenyl" means a cyclic unsaturated monovalent group with a specified number of cyclic atoms (for example, 5-10 membered), a specified number of heteroatom(s) (for example, 1, 2 or 3), and a specified heteroatomic species (one or more of N, O and S), which are monocyclic or polycyclic (for example, 2 or 3, a spiro-ring). The monocyclic-ring hetercycloalkenyl includes but is not limited to:

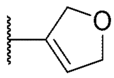, 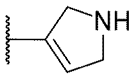, 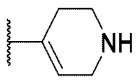,

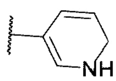, 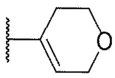, 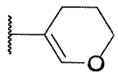, 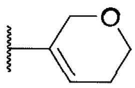

etc. The spiro-ring heterocycloalkenyl includes but is not limited to

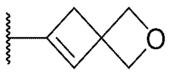, 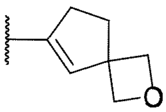, 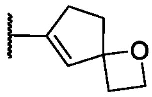, 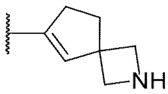 or 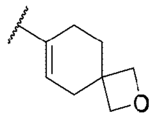

etc.

Unless otherwise specially specified in the present invention, the term "heteroaryl" means a cyclic unsaturated monovalent group with a specified number of cyclic atoms (for example, 5-10 membered), a specified number of heteroatom(s) (for example, 1, 2 or 3), and a specified heteroatom species (one or more of N, O and S), which are monocyclic or polycyclic, where the monocyclic rings share two atoms and a bond, and at least one ring is aromatic. The heteroaryl group is attached to the rest of a molecule by a carbon atom or heteroatom, by a ring with or without heteroatoms, or by a ring that is aromatic or not. Heteroaryl includes but is not limited to:

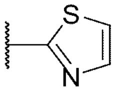, 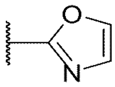, 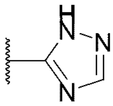, 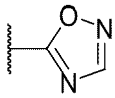, 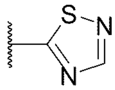, 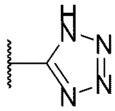, 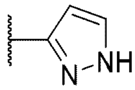,

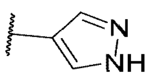, 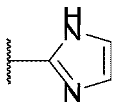, 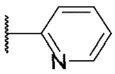, 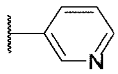, 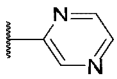, 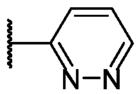, 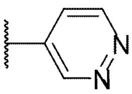, 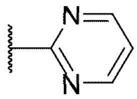,

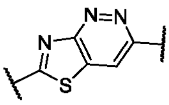, 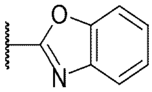, 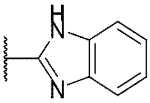, 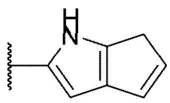, 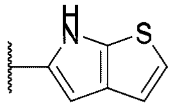 or 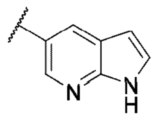

etc.

Unless otherwise specially specified in the present invention, the term "fused-ring heteroaromatic ring" means that the "heteroaromatic ring" forms a fused ring with an additional ring, and the "fused-ring heteroaromatic ring" comprises at least two rings, at least one of which is a heteroaromatic ring. Here, the rest of the definition of the "heteroaromatic ring" is the same as that of "heteroaryl".

The term "isotopic compound" means a compound in which one or more atoms have the isotopic abundance that differ from their natural abundance. For example, one or more atoms in a compound are substituted by an atom with a lower mass in nature, for instance, one hydrogen atom in a compound is substituted by deuterium, or C is substituted by $^{13}C$.

In the present invention, "room temperature" means "20-40°C".

Without departing from the common knowledge in the art, all the preferred conditions above can be randomly combined to implement the preferred embodiments of the present invention.

The reagents and raw materials used in the present invention are commercially available.

The positive progressive effect of the present invention lies in that: the ATPase activity and protein inhibition effect of the compound of the present invention are greatly improved compared with the prior art.

## DETAILED DESCRIPTION OF THE PREFERRED EMBODIMENTS

The present invention will be further illustrated below by the way of embodiments, which are not intended to limit the present invention. For the test methods without specific conditions indicated in the following embodiments, the specific conditions were selected according to conventional methods and conditions, or according to the product instructions.

All the compounds of the present invention may be synthesized with different methods by a person skilled in the field of organic chemistry. The following describes general synthesis schemes for preparing the compounds of the present invention. These schemes are generic, which, however, does not imply the limitation to the possible techniques used by a person skilled in the art to prepare the compounds disclosed herein. The different methods for preparing the compounds of the present invention are obvious to a person skilled in the art. In addition, the various steps in synthesis may be performed alternately in sequence to obtain one or more of the desired compounds. Examples of the preparation of the compounds of the present invention by the method described in the general schemes are given in the Preparation and Examples sections described below. The preparation of compounds containing chiral center embodiments may be carried out by means of the techniques mastered by a person skilled in the art. For example, chiral compounds may be prepared by separating racemic products by chiral resolution by means of HPLC; or, example compounds may be prepared by known methods to obtain chiral compounds.

The chemical reactions and synthesis techniques described herein are performed in the reagents and solvents described herein, and the corresponding reaction yields are also affected by the reagents and solvents used. In addition, it should be understood that in the synthesis method described below, all the reaction conditions mentioned, including the choice of solvent, the reaction atmosphere, the reaction temperature, the experimental duration, and the feeding sequence for reaction, should be regarded as the standard operating conditions for the reaction, which should be easily identifiable by a person skilled in the art. Meanwhile, it is also understandable by a technician in the field of organic synthesis. The functional groups present on each part of a molecule must be compatible with the reagents used and the reaction itself. In case of the limitation of the incompatibility of some functional groups on each part of the molecule with the reaction conditions, an alternative method must be used, which is obvious to a person skilled in the art. To derive the desired compounds of the present invention, obviously, it is necessary to make judgment on the adjustment of the sequence of synthesis steps, or on the selection of a specific synthesis process scheme. This is understandable and easily recognizable to a person skilled in the field of organic synthesis. It should also be appreciated that another major consideration for any synthetic route designed in the art is the rational selection of a protective group to protect the tolerance of reactive functional groups present in the compounds described in the present invention. For details, a reference can be made to the work of Greene et al., authorities in the field of chemistry (*Protective Groups in Organic Synthesis*, Third Edition, Wiley and Sons (1999)).

Examples

The compounds and the intermediates used in the preparation of the compounds may be prepared using the procedures shown in the following examples and related procedures. The methods and conditions used in these examples and the actual compounds prepared in these examples are not meant to be limited, but imply to elaborate how to prepare the relevant compounds. The starting materials and reagents used in these examples, when not prepared by the procedures described herein, are often commercially available, or have been reported in the relevant chemical literature, or may be prepared by using the procedures described in the chemical literature.

In the examples given herein, the term "drying and concentrating" generally means the addition of anhydrous sodium sulfate or magnesium sulfate drying solution to an organic solvent, followed by filtration and removal of the solvent from the filtrate (usually under reduced pressure and at a temperature suitable for the stability of the compound being prepared). In case of chromatographic column methods, conventional column chromatography or rapid column chromatography is typically used for column separation and purification, or a medium-pressure chromatograph (Biotage Isola One) preloaded with silica-gel column is used for elution in a specified solvent or solvent mixture. In some cases, the final product is rapidly purified by preparative thin-layer chromatography (TLC) using silica gel plates of 20 cm x 20 cm x 0.5 mm or 20 cm x 20 cm x 1 mm in an appropriate solvent system. Preparative high-performance liquid chromatography (HPLC) is performed using a reversed-phase column (Waters Sunfire C18, Waters Xbridge C18, or a similar reversed-phase column) with a size appropriate for the amount of compound being separated; elution is typically performed by adding methanol or acetonitrile of gradient concentrations to an aqueous phase, with an eluent containing 0.05% or 0.1% formic acid, trifluoroacetic acid, or 10 mM ammonium acetate, at an elution rate matching the size of the reversed-phase column used and the resolution of the product to be prepared.

**List of Abbreviations**

| Abbreviation | Explanation | Abbreviation | Explanation |
|---|---|---|---|
| MS | Mass spectrometry | NMR | Nuclear magnetic resonance |
| TLC | Thin-layer chromatography | HPLC | High performance liquid chromatography |
| LCMS | Liquid chromatography-mass spectrometry | DMF | N,N-dimethylformamide |
| DCE | 1,2-dichloroethane | PE | Petroleum ether |
| DCM | Dichloromethane | DMSO | Dimethyl sulfoxide |
| IPA | Isopropanol | DEA | N,N-diethylaniline |
| MTBE | Methyl tert-butyl ether | THF | Tetrahydrofuran |
| $Pd(dppf)Cl_2$ | [1,1'-di(diphenylphosphino) -ferrocen]palladium dichlorid(II) | HATU | 2-(7-azobenzotriazol)-N,N,N',N'-tetramethyluronium hexafluorophosphate |
| TFA | Trifluoroacetic acid | TEA | Triethylamine |
| LiHMDS | Lithium bis(trimethylsilyl)amide | DIPEA | N,N-diisopropylethylamine |
| DMAP | 4-dimethylaminopyridine | TsOH | P-toluenesulfonic acid |
| NCS | N-chlorosuccinimide | DMF-DMA | N,N-dimethylformamide dimethylacetal |
| NBS | N-bromosuccinimide | LDA | Lithium diisopropylamide |
| Xantphos | 4,5-bisdiphenylphosphino-9 ,9-dimethylxanthene | $Pd_2(dba)_3$ | Tris(dibenzylideneacetone) dipalladium |
| SEMCl | 2-(trimethylsilyl)ethoxymet hyl chloride | TBAF | Tetrabutylammonium fluoride |
| MsCl | Methylsufonyl chloride | Ruphos | 2-dicyclohexylphosphino-2' ,6'-diisopropoxy-1,1'-biphe nyl |

(continued)

| Abbreviation | Explanation | Abbreviation | Explanation |
|---|---|---|---|
| Ruphos PG3 | Methanesulfonato(2-dicycl ohexylphosphino-2',6'-diisopropoxy-1,1'-biphenyl)(2-a mino-1,1'-biphenyl-2-yl)pal ladium (II) | rt | Room temperature |
| EA | Ethyl acetate | $T_3P$ | 1-propylphosphoric anhydride |
| LiOH | Lithium hydroxide | $K_2CO_3$ | Potassium carbonate |
| $CH_3I$ | Iodomethane | nBuLi | n-butyllithium |
| $I_2$ | Iodine | TBSCl | Tert-butyldimethylchlorosil ane |
| $LiAH_4$ | Lithium aluminium hydride | NaH | Sodium hydride |
| $CS_2$ | Carbon disulfide | BrCN | Cyanogen bromide |
| $PhNTf_2$ | N-phenylbis(trifluorometha nesulfonyl)imide | TCFH | N,N,N',N'-tetramethylchlor formamidine hexafluorphosphate |
| NMI | N-methylimidazole | $Ac_2O$ | Acetic anhydride |
| $PtO_2$ | Platinum dioxide | ACN | Acetonitrile |
| Raney Ni | Raney nickel | $K_3PO_4$ | Potassium phosphate |
| CuI | Cuprous iodide | $PPh_3$ | Triphenyl phosphine |
| $Cs_2CO_3$ | Cesium carbonate | $NaIO_4$ | Sodium periodate |
| $K_2OsO_4$ | Potassium osmate | DIAD | Diisopropyl azodicarboxylate |
| *t*-BuOK | Potassium tert-butoxide | $Boc_2O$ | Di-tert-butyl dicarbonate |
| EtONa | Sodium ethoxide | $NaBH_4$ | Sodium borohydride |
| $Pd(PPh_3)_4$ | Tetrakis(triphenylphosphin e)palladium | TsCl | Paratoluensulfonyl chloride |
| Pd/C | Palladium on carbon | $NaBH_3CN$ | Cyanosodium borohydride |
| $Tf_2O$ | Trifluoromethanesulfonic anhydride | Py | Pyridine |
| $BnNH_2$ | Benzylamine | NIS | N-iodosuccinimide |
| *t*-BuONa | Sodium tert-butoxide | | |

**Intermediate 1: 2'-chloro-3'-fluoro-5'-methoxy-6-methyl-[4,4'-bipyridine]-3-carboxylic acid**

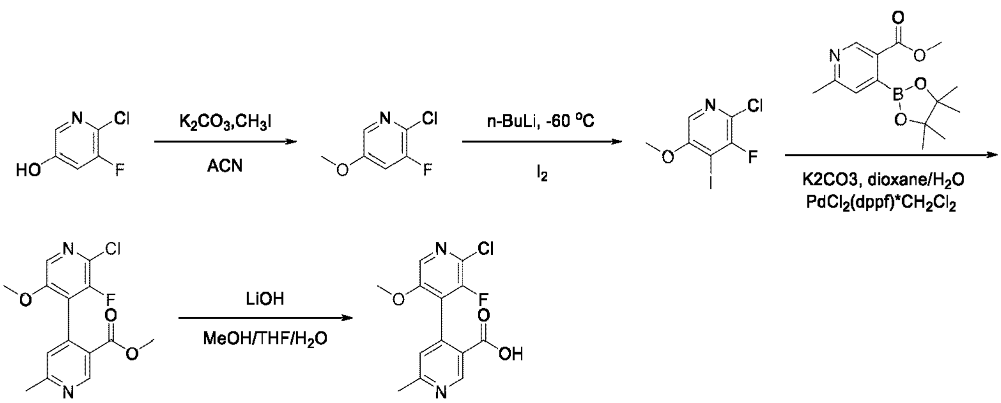


**Step 1: 2-chloro-3-fluoro-5-methoxypyridine**

6-chloro-5-fluoropyridin-3-ol (10 g, 67.8 mmol) was dissolved in acetonitrile (100 mL); potassium carbonate (32.7

g, 237 mmol) was added; then, iodomethane (33.7 g, 237 mmol) was added; and the resulting mixture was allowed to react at room temperature for 18 hours. After complete reaction, water was added; and the resulting mixture was extracted with ethyl acetate, washed with saturated saline, dried over anhydrous sodium sulfate, and concentrated under reduced pressure. The residue was purified by column chromatography (eluent: petroleum ether/ethyl acetate, gradient: 10%-40% ethyl acetate) to obtain the title compound (colorless liquid, 7.1 g, yield: 64.8%). LC/MS (ESI) m/z: 161.8 $[M+H]^+$.

**Step 2: 2-chloro-3-fluoro-4-iodo-5-methoxypyridine**

Under the protection of nitrogen, 2-chloro-3-fluoro-5-methoxypyridine (11 g, 68.1 mmol) was dissolved in tetrahydrofuran (100 mL), and cooled to -60°C; with the temperature controlled to be below -60°C, n-butyllithium in an n-hexane solution (2.5 mol/L, 40.8 mL, 102.1 mmoL) was slowly added; with the temperate held at -60°C, the resulting mixture was allowed to react for half an hour; then, the solution of iodine (19.0 g, 74.9 mmol) in tetrahydrofuran (20 mL) was added; and the resulting mixture was allowed to react at -60°C for 1 hour. After complete reaction, saturated ammonium chloride was slowly added for quenching; water was added; the resulting mixture was extracted with ethyl acetate; the organic phase was washed with saturated saline, dried over anhydrous sodium sulfate, and then filtered; and the filtrate was concentrated under reduced pressure. The residue was purified by column chromatography (eluent: petroleum ether/ethyl acetate, gradient: 10%-40% ethyl acetate) to obtain the title compound (white solid, 12.5 g, yield: 6.4%). LC/MS (ESI) m/z: 288.1 $[M+H]^+$.

**Step 3: 2'-chloro-3'-fluoro-5'-methoxy-6-methyl-[4,4'-bipyridine]-3-methyl carboxylate**

Under the protection of nitrogen, 2-chloro-3-fluoro-4-iodo-5-methoxypyridine (288 mg, 1.0 mmol), 6-methyl-4-(4,4,5,5-tetramethyl-1,3,2-dioxyboric acid -2-yl)methyl nicotinate (277 mg, 1.0 mmol), a [1,1'-bis(diphenylphosphino)ferrocen]palladium dichloride dichloromethane complex (164 mg, 0.20 mmol), potassium carbonate (415 mg, 3.0 mmol) were dissolved in the mixed solvent of 1,4-dioxane (10 mL) and water (2 mL). Under the protection of nitrogen, the mixture was stirred at 65°C to react for 2 hours. Water was added; the mixture was extracted with ethyl acetate; the organic phase was washed with saturated saline, dried over anhydrous sodium sulfate, and then filtered; and the filtrate was concentrated under reduced pressure. The residue was purified by column chromatography (eluent: petroleum ether/ethyl acetate, gradient: 20%-50% ethyl acetate) to obtain the title compound (white solid, 50 mg, yield: 16.1%). LC/MS (ESI) m/z: 311.0 $[M+H]^+$.

**Step 4: 2'-chloro-3'-fluoro-5'-methoxy-6-methyl-[4,4'-bipyridine]-3-carboxylic acid**

2'-chloro-3'-fluoro-5'-methoxy-6-methyl-[4,4'-bipyridine]-3-methyl carboxylate (500 mg, 1.60 mmol) was dissolved in the mixed solvent of tetrahydrofuran (5 mL), methanol (5 mL) and water (5 mL); and lithium hydroxide monohydrate (67 mg, 1.60 mmol) was added. The mixture was allowed to react at room temperature for 5 hours, and concentrated under reduced pressure to obtain the title compound (white solid, 445 mg, yield: 93.1%). LC/MS (ESI) m/z: 296.9 $[M+H]^+$.

**Intermediate 2: 2'-chloro-5'-methoxy-6-methyl-[4,4'-bipyridine]-3-carboxylic acid**

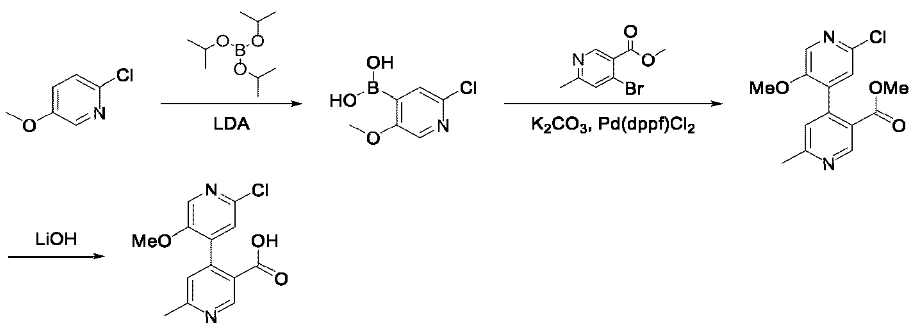


**Step 1: (2-chloro-5-methoxypyridin-4-yl)boric acid**

2-chloro-5-methoxypyridine (10.0 g, 69.5 mmol) was dissolved in 250 mL of tetrahydrofuran, and cooled to -65°C

under the protection of nitrogen; and with the temperature held below -60°C, 2 mol/L lithium diisopropylamide (70 mL) was added dropwise slowly. After addition, the above low temperature was held, to allow for reaction for 2 hours. Then, at -65°C, triisopropyl borate (26.2 g, 139 mmol) was added dropwise slowly; with the temperature still held at -65°C, the mixture was stirred for 1 hour; and then the temperature was increased to room temperature, and the mixture was stirred overnight. In an ice-water bath, 100 mL of water was carefully added to the reaction mixture for quenching; the resulting aqueous solution was extracted with ethyl acetate for twice; the organic phase was discarded; the aqueous phase was adjusted pH to 5-6 with 2 M hydrochloric acid aqueous solution, allowing for the precipitation of a large amount of solids; and the solid phase was filtered under reduced pressure, and dried to obtain the title compound (11.4 g, white solid, yield: 88.1%). LC/MS (ESI) m/z: 188.0 $[M+H]^+$.

**Step 2: 2'-chloro-5'-methoxy-6-methyl-[4,4'-bipyridine]-3-methyl carboxylate**

Methyl 4-bromo-6-methylnicotinate (5 g, 21.7 mmol), (2-chloro-5-methoxypyridin-4-yl)boric acid (4.07 g, 21.7 mmol), and potassium carbonate (9 g, 65.2 mmol) were dissolved in 180 mL of the mixed solution of 1,4-dioxane and 36 mL of water; and under the protection of nitrogen, bis(triphenylphosphine)palladium dichloride (1.59 g, 2.17 mmol) was added; and the resulting mixture was heated to 80°C to react for 2 hours. The reaction mixture was cooled to room temperature, filtered, and then stratified and extracted with water and ethyl acetate; the organic phase was concentrated and then purified by silica-gel column chromatography (petroleum etherethyl acetate system: 0-50% ethyl acetate) to obtain the title compound (5.20 g, white solid, yield: 81.7%). LC/MS (ESI) m/z: 293.0 $[M+1]^+$.

**Step 3: 2'-chloro-5'-methoxy-6-methyl-[4,4'-bipyridine]-3-carboxylic acid**

2'-chloro-5'-methoxy-6-methyl-[4,4'-bipyridine]-3-methyl carboxylate (5.2 g, 17.7 mmol) was dissolved in 50 mL of tetrahydrofuran and 50 mL of water; lithium hydroxide (0.64 g, 26.6 mmol) was added; the resulting mixture was stirred overnight at room temperature, concentrated under reduced pressure to remove tetrahydrofuran, then adjusted to pH of 5-6 with hydrogen chloride to allow for the precipitation of a large amount of white solids, and underwent suction filtration under reduced pressure; and the resulting filter cake was dried to obtain the title compound (4.82 g, white solid, yield: 96.7%). LC/MS (ESI) m/z: 279.1 $[M+H]^+$.

**Intermediate 3: 6-bromothiazolo[4,5-b]pyridin-2-amine**

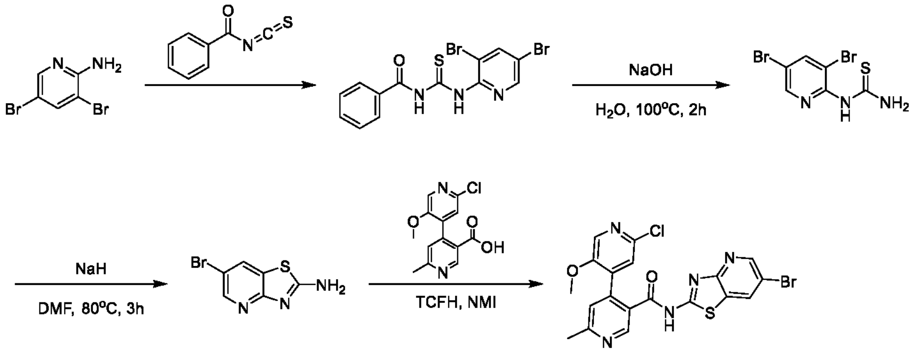


**Step 1: *N*-((3,5-dibromopyridin-2-yl)carbamothioyl)benzamide**

Benzoyl isothiocyanate (130 g, 794 mmol) was added to the solution of 3,5-dibromopyridin-2-amine (100 g, 397 mmol) in acetone (1.2 L). Then, the mixture was stirred at room temperature for 16 hours. After complete reaction, the reaction mixture was filtered; and the filter cake was washed with acetone, and dried to obtain the title compound (faint yellow solid, 149 g, yield: 90.4%).

**Step 2: 1-(3,5-dibromopyridin-2-yl)thiourea**

N-((3,5-dibromopyridin-2-yl)carbamothioyl)benzamide (149 g, 359 mmol) was dissolved in sodium hydroxide (598 mL, 3 M) in water, to react at 100°C for 2 hours. After complete reaction, the reaction mixture was filtered; and the filter cake was washed with water and ethanol, and dried to obtain the title compound (yellow solid, 101 g, yield: 90.5%).

**Step 3: 6-bromothiazolo[4,5-b]pyridin-2-amine**

60% sodium hydride (26 g, 650 mmol) was added to the solution of 1-(3,5-dibromopyridin-2-yl)thiourea (101 g, 325 mmol) in N,N-dimethylformamide (600 mL). Under the protection of nitrogen, the reaction mixture was stirred at 80°C to react for 3 hours. After complete reaction, saturated ammonium chloride in water was slowly added for quenching; the resulting mixture was filtered; and the filter cake was washed with water and ethanol. The filter cake was dried to obtain the title compound (brown solid, 56 g, yield: 74.9%). $^1$H NMR (400 MHz, DMSO-$d_6$) δ 8.32 (s, 1H), 8.27 (s, 1H), 8.12 (s, 2H). LC/MS (ESI) m/z: 229.9 [M+H]$^+$.

**Step 4: *N*-(6-bromothiazolo[4,5-b]pyridin-2-yl)-2'-chloro-5'-methoxy-6-methyl-[4,4'-bipyridine]-3-car boxamide**

6-bromothiazolo[4, 5-b]pyridin-2-amine (10 g, 43.4 mmol) and 2'-chloro-5'-methoxy-6-methyl-4, 4'-bipyridin-3-carboxylic acid (13.3 g, 47.8 mmol) were dissolved in 300 mL of acetonitrile and 180 mL of DMF; N-methylimidazole (10.4 mL, 130.4 mmol) was added; with the temperature increased to 75°C, the resulting mixture was stirred for 30 minutes, and then *N,N,N',N'*-tetramethylchlorformamidine hexafluorphosphate (12.2 g, 43.4 mmol) was added; the temperature was held at 75°C to allow for reaction for 3 hours. Then, ethyl acetate and water was added to the reaction mixture for stratification; the organic phase was washed with saturated sodium chloride solution, dried over anhydrous sodium sulfate, filtered, and concentrated under reduced pressure; and the resulting residue was purified by column chromatography (eluent: dichloromethane/methanol, gradient: 3%-5% methanol) to obtain the title compound (faint yellow solid, 8 g, yield: 37.5%). LC/MS (ESI) m/z: 491 [M+H]$^+$.

**Example 1: 2'-chloro-N-(5-((1r,4r)-4-hydroxycyclohexyl)thiazol[5,4-b]pyridin-2-yl)-5'-methoxy-6-met hyl-[4,4'-bipyridine]-3-carboxamide and 2'-chloro-N-(5-((1s,4s)-4-hydroxycyclohexyl)thiazol[5,4-b]py ridin-2-yl)-5'-methoxy-6-methyl-[4,4'-bipyridine]-3-carboxamide**

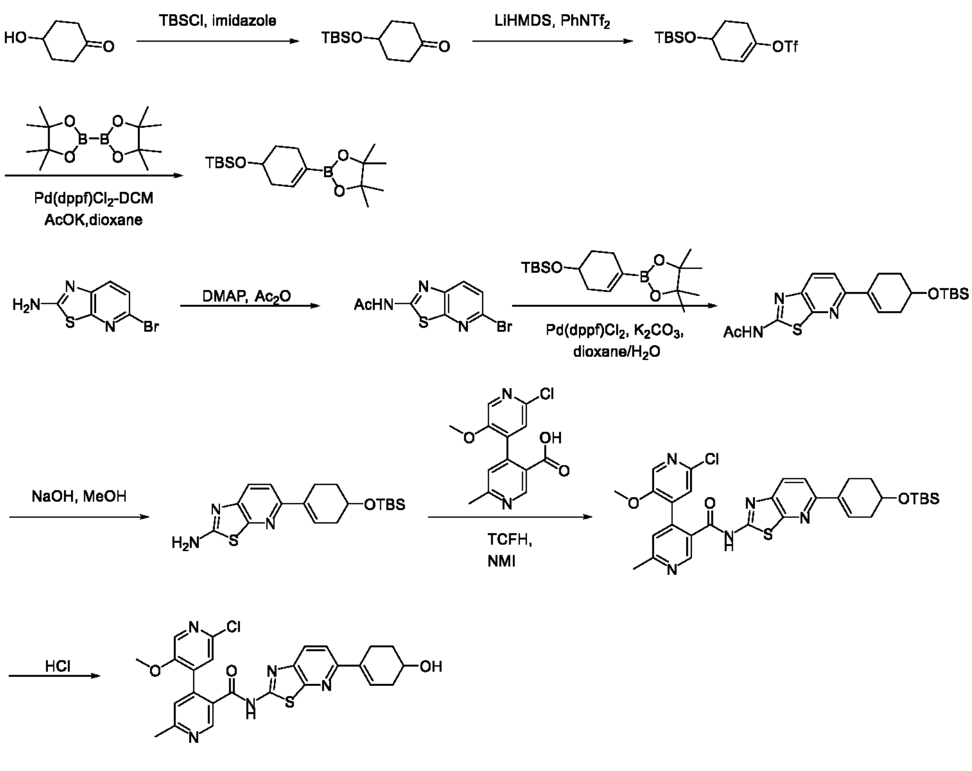


**Step 1: 4-(tert-butyldimethylsiloxy)cyclohexan-1-one**

4-hydroxycyclohexanone (25.0 g, 219 mmol) was dissolved in 450 mL of dichloromethane; imidazole (14.9 g, 219 mmol) and tert-butyldimethylchlorosilane (33.0 g, 219 mmol) were added; and the reaction was allowed to occur at room temperature for 18 hours. The reaction mixture was washed with water and saturated saline, and underwent rotary evaporation to remove dichloromethane; and the residue was purified by silica-gel column chromatography (eluent:

petroleum ether/ethyl acetate = 20/1, V/V) to obtain 4-(tert-butyldimethylsiloxy)cyclohexan-1-one (42.0 g, colorless oil, yield: 84%).

**Step 2: 4-(tert-butyldimethylsiloxy)cyclohex-1-en-1-yl trifluoromethanesulfonate**

4-(tert-butyldimethylsiloxy)cyclohexan-1-one (20.0 g, 87.6 mmol) was dissolved in 400 mL of anhydrous tetrahydrofuran; under the protection of nitrogen, the anhydrous operation was conducted; the resulting mixture was cooled to -65°C; 1 mol/L lithium bis(trimethylsilyl)amide in tetrahydrofuran solution (175 mL, 175 mmol) was added dropwise slowly; and after the addition was completed, the reaction was allowed to occur at -65°C for 1 hour. After that, 200 mL of N-phenylbis(trifluoromethanesulfonyl)imide in tetrahydrofuran solution (62.6 g, 175 mmol) was added dropwise at -65°C; and the temperature of the reaction mixture was allowed to slowly rise to -20°C over 1 hour. Afterwards, saturated saline was added to the reaction mixture for quenching; the reaction mixture was extracted with ethyl acetate, and underwent rotary evaporation to remove the solvent; and the residue was purified by silica-gel column chromatography (eluent: petroleum ether/ethyl acetate = 30/1, *V/V*) to obtain 4-(tert-butyldimethylsiloxy)cyclohex-1-en-1-yl trifluoromethanesulfonate (24.0 g, yellow solid, yield: 76.1%).

**Step 3: tert-butyldimethyl((4-(4,4,5,5-tetramethyl-1,3,2- dioxaborolan-2-yl)cyclohexyl-3-en-1-yl)oxy) silane**

4-(tert-butyldimethylsiloxy)cyclohex-1-en-1-yl trifluoromethanesulfonate (22.0 g, 61.0 mmol) and bis(pinacolato)diboron (18.6 g, 73.2 mmol) were dissolved in 286 mL of anhydrous 1,4-dioxane; potassium acetate (44.9 g, 458 mmol) and a [1,1'-bis(diphenylphosphino)ferrocen] palladium dichloride dichloromethane complex (1.50 g, 1.83 mmol) were added; and under the protection of nitrogen, the anhydrous operation was conducted, and with the temperature increased to 80°C, the reaction was allowed to occur for 18 hours. The reaction mixture was cooled to room temperature, underwent rotary evaporation to remove 1,4-dioxane, added with water, and extracted with ethyl acetate; the organic phase was washed with water and saturated saline; and the residue was purified by silica-gel column chromatography (eluent: dichloromethane/methanol = 50/1, V/V) to obtain tert-butyldimethyl(4-(4,4,5,5-tetramethyl-1,3,2-dioxaborinan-2-yl)cyclohexyl-3-en-1-yl)oxysilane (7.60 g, yellow oily liquid, yield: 79.0%). $^1H$ NMR (400 MHz, $CDCl_3$) δ 6.45 - 6.41 (m, 1H), 3.93 - 3.76 (m, 1H), 2.41 - 2.24 (m, 2H), 2.19 - 1.99 (m, 2H), 1.86 - 1.73 (m, 1H), 1.58 - 1.45 (m, 1H), 1.25 (s, 12H), 0.88 (s, 9H), 0.05 (s, 6H).

**Step 4: 5-bromothiazolo[5, 4-b]pyridin-2-yl acetamide**

5-bromo-2-aminothiazol[5,4-B]opyridine (1.40 g, 6.09 mmol), 4-dimethylaminopyridine (850 mg, 7.00 mmol), acetic anhydride (710 mg, 7.00 mmol) and 20 mL of dichloromethane were added to a 100 mL reaction flask, to react at room temperature for 18 hours; the reaction mixture was concentrated under reduced pressure; and the residue was purified by silica-gel column chromatography (eluent: dichloromethane/methanol = 30/1, *V/V*) to obtain 5-bromothiazolo [5, 4-b]pyridin-2-ylacetamide (800 mg, yellow solid, yield: 48.3%). LC/MS (ESI) m/z: 272.2 $[M+H]^+$.

**Step 5: N-(5-(4-(tert-butyldimethylsiloxy)cyclohexyl-1-en-1-yl)thiazolo[5,4-b]pyridin-2-yl)acetamide**

5-bromothiazolo[5,4-b]pyridin-2-ylacetamide (300 mg, 1.10 mmol) and tert-butyldimethyl (4-(4,4,5,5-tetramethyl-1,3,2-dioxaborinan-2-yl)cyclohexyl-3-en-1-yl)oxysilane (410 mg, 1.21 mmol) were dissolved in 27 mL of 1,4-dioxane and 9 mL of water; potassium carbonate (305 mg, 2.21 mmol) and 1,1-bis(diphenylphosphino)ferrocene palladium dichloride (80.7 mg, 0.110 mmol) were added; and under the protection of nitrogen, the anhydrous operation was conducted, and with the temperature increased to 90°C, the reaction was allowed to occur for 2 hours. The reaction mixture was cooled to room temperature, underwent rotary evaporation to remove 1,4-dioxane, added with water, and extracted with ethyl acetate; the organic phase was washed with water and saturated saline; and the residue was purified by silica-gel column chromatography (eluent: dichloromethane/methanol = 30/1, V/V) to obtain N-(5-(4-(tert-butyldimethylsiloxy)cyclohexyl-1-en-1-yl)thiazolo[5,4-b]pyridin-2-yl)acetamide (380 mg, yellow solid, yield: 85.4%). LC/MS (ESI) m/z: 404.2 $[M+H]^+$.

**Step 6: 5-(4-((tert-butyldimethylsilyl)oxy)cyclohex-1-en-1-yl)thiazol[5,4-b]pyridin-2-amine**

N-(5-(4-(tert-butyldimethylsiloxy)cyclohexyl-1-en-1-yl)thiazolo[5,4-b]pyridin-2-yl)acetamide (380 mg, 0.941 mmol) and sodium hydroxide (377 mg, 9.42 mmol) were dissolved in 15 mL of methanol and 5 mL of water; and the reaction was allowed to occur at 85°C for 3 hours. The reaction mixture was concentrated under reduced pressure; and the residue was purified by silica-gel column chromatography (eluent: dichloromethane/methanol = 20/1, V/V) to obtain 5-(4-((tert-butyldimethylsilyl)oxy)cyclohex-1-en-1-yl)thiazol[5,4-b]pyridin-2-amine (220 mg, yellow solid, yield: 65%).

LC/MS (ESI) m/z: 362.2 [M+H]+.

**Step 7: N-(5-(4-((tert-butyldimethylsilyl)oxy)cyclohex-1-en-1-yl)thiazol[5,4-b]pyridin-2-yl)-2'-chloro-5'-methoxy-6-methyl-[4,4'-bipyridine]-3-carboxamide**

5-(4-((tert-butyldimethylsilyl)oxy)cyclohex-1-en-1-yl)thiazol[5,4-b]pyridin-2-amine (120 mg, 0.332 mmol), 2'-chloro-5'-methoxy-6-methyl-[4,4'-bipyridine]-3-carboxylic acid (92.5 mg, 0.332 mmol), and methylimidazole (81.8mg, 0.996 mmol) were dissolved in 5 mL of acetonitrile and 2 mL of N,N-dimethylformamide; and 1 mL of N,N,N',N'-tetramethylchlorformamidine hexafluorphosphate in acetonitrile solution (93.1 mg, 0.332 mmol) was added dropwise, to allow for reaction at room temperature for 18 hours. The reaction mixture was concentrated under reduced pressure, added with water and extracted with ethyl acetate; the residue was purified by silica-gel column chromatography (eluent: petroleum ether/ethyl acetate = 3/1, V/V) to obtain N-(5-(4-((tert-butyldimethylsilyl)oxy)cyclohex-1-en-1-yl)thiazol[5,4-b]pyridin-2-yl)-2'-chloro-5'-methoxy-6 -methyl-[4,4'-bipyridine]-3-carboxamide (80.2 mg, yellow solid, yield: 38.7%). LC/MS (ESI) m/z: 622.2 [M+H]+.

**Step 8: 2'-chloro-N-(5-(4-hydroxycyclohex-1-en-1-yl)thiazol[5,4-b]pyridin-2-yl)-5'-methoxy-6-methyl-[4,4'-bipyridine]-3-carboxamide**

N-(5-(4-((tert-butyldimethylsilyl)oxy)cyclohex-1-en-1-yl)thiazol[5,4-b]pyridin-2-yl)-2'-chloro-5'-methoxy-6 -methyl-[4,4'-bipyridine]-3-carboxamide (80.1 mg, 0.129 mmol) was dissolved in 3 mL of dichloromethane and 3 mL of trifluoroacetic acid, to allow for reaction at room temperature for 18 hours. The reaction mixture was concentrated under reduced pressure, added with saturated sodium bicarbonate in water, extracted with ethyl acetate for stratification, and concentrated under reduced pressure to obtain residues, which were purified by prep-HPLC (eluent: acetonitrile/0.1% formic acid aqueous solution = 25%-90%, V/V) to obtain 2'-chloro-N-(5-(4-hydroxycyclohex-1-en-1-yl)thiazol[5,4-b]pyridin-2-yl)-5'-methoxy-6-methyl-[4,4'-bipyridi ne]-3-carboxamide (40.1 mg, yellow solid, yield: 61.3%). $^1$H NMR (400 MHz, DMSO-$d_6$) $\delta$ 13.02 (s, 1H), 8.85 (s, 1H), 8.17 (s, 1H), 8.04 (d, *J*= 8.6 Hz, 1H), 7.65 (d, *J*= 8.6 Hz, 1H), 7.58 (s, 1H), 7.47 (s, 1H), 6.66-6.60 (m, 1H), 4.71 (d, *J*= 3.9 Hz, 1H), 3.85 - 3.80 (m, 1H), 3.62 (s, 3H), 2.75 - 2.64 (m, 1H), 2.61 (s, 3H), 2.54 - 2.50 (m, 1H), 2.47 (s, 1H), 2.17 - 2.05 (m, 1H), 1.95 - 1.85 (m, 1H), 1.65 - 1.58 (m, 1H). LC/MS (ESI) m/z:508.2 [M+H]+.

The example compounds in Table 1 were synthesized using commercially available raw materials with reference to the synthesis steps of Example 1.

Table 1:

| Examples | Chemical structure | LC/MS(ESI) m/z [M+H]+ | $^1$H NMR |
|---|---|---|---|
| 2 | 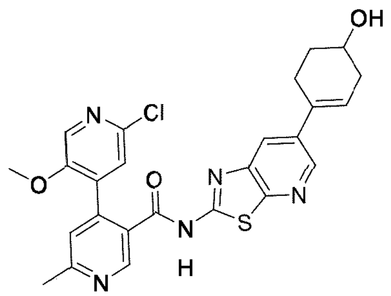 | 508.2 | $^1$HNMR (400MHz, DMSO-$d_6$) $\delta$ 13.08 (s, 1H), 8.86 (s, 1H), 8.56 (d, *J* = 1.8 Hz, 1H), 8.17 (s, 1H), 8.10 - 8.04 (m, 1H), 7.59 (s, 1H), 7.47 (s, 1H), 6.23 - 6.18 (m, 1H), 4.72 (d, *J* = 4.0 Hz, 1H), 3.88 - 3.79 (m, 1H), 3.61 (s, 3H), 2.61 (s, 3H), 2.59 - 2.55 (m, 1H), 2.55 - 2.51 (m, 1H), 2.48 - 2.40 (m, 1H), 2.15 - 2.08 (m, 1H), 1.96 - 1.87(m, 1H), 1.70 - 1.59 (m, 1H). |
| 3 | 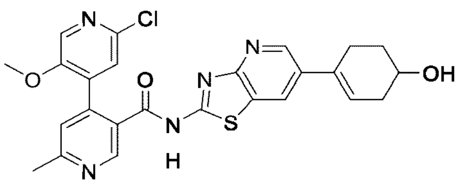 | 508.1 | $^1$H NMR (400 MHz, DMSO-$d_6$) $\delta$ 13.19 (s, 1H), 8.87 (s, 1H), 8.64 (d, *J* = 1.8 Hz, 1H), 8.44 (d, *J* = 1.8 Hz, 1H), 8.17 (s, 1H), 7.59 (s, 1H), 7.47 (s, 1H), 6.18 - 6.14 (m, 1H), 4.71 (d, *J* = 4.0 Hz, 1H), 3.86 - 3.76 (m, 1H), 3.60 (s, 3H), 2.61 (s, 3H), 2.57 - 2.40 (m, 3H), 2.14 - 2.04 (m, 1H), 1.95 - 1.87 (m, 1H), 1.68 - 1.54 (m, 1H). |

(continued)

| Exam ples | Chemical structure | LC/MS(ESI) m/z $[M+H]^+$ | $^1H$ NMR |
|---|---|---|---|
| 4 | 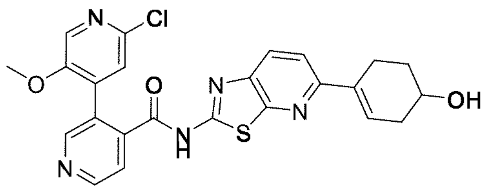 | 494.1 | $^1H$ NMR (400 MHz, DMSO-$d_6$) δ 13.06 (s, 1H), 8.67 (d, J =4.8 Hz, 1H), 8.72 (s, 1H), 8.15 (s, 1H), 8.02 (d, *J* = 8.6 Hz, 1H), 7.81 (d, *J* = 4.9 Hz, 1H), 7.64(d, J = 8.4 Hz, 1H),7.62 (s, 1H), 6.63 - 6.58 (m, 1H), 4.70 (d, *J* = 3.4 Hz, 1H), 3.85 - 3.76 (m, 1H), 3.61 (s, 3H), 2.76 - 2.66 (m, 1H), 2.53 - 2.43 (m, 2H), 2.17 - 2.04 (m, 1H), 1.97 - 1.85 (m, 1H), 1.67 - 1.54 (m, 1H). |
| 5 | 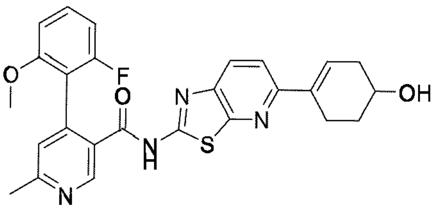 | 491.1 | $^1H$ NMR (400 MHz, $CD_3OD$) δ 8.94 (s, 1H), 7.96 (d, *J* = 8.6 Hz, 1H), 7.70 (s, 1H), 7.62 (d, *J* = 8.6 Hz, 1H), 7.48 - 7.23 (m, 1H), 6.93 - 6.86 (m, 2H), 6.59 - 6.55 (m, 1H), 4.09 - 3.87 (m, 1H), 3.66 (s, 3H), 2.86 - 2.70 (m, 4H), 2.65 - 2.51 (m, 2H), 2.29 - 2.17 (m, 1H), 2.11 - 1.97 (m, 1H), 1.85 - 1.69 (m, 1H). |
| 6 | 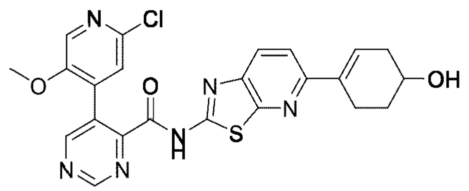 | 495.1 | $^1H$ NMR (400 MHz, DMSO-$d_6$) δ 13.22 (s, 1H), 9.47 (s, 1H), 9.11 (s, 1H), 8.23 (s, 1H), 8.09 (d, *J* = 8.0 Hz, 1H), 7.74 (s, 1H), 7.67 (d, *J* = 8.5 Hz, 1H), 6.65 - 6.61 (m, 1H), 4.71 (d, *J* = 4.0 Hz, 1H), 3.87 - 3.76 (m, 1H), 3.66 (s, 3H), 2.72 - 2.65 (m, 1H), 2.50 - 2.43(m, 2 H), 2.17 - 2.04 (m, 1H), 1.94 - 1.86 (m, 1H), 1.65 - 1.55 (m, 1H) |
| 7 | 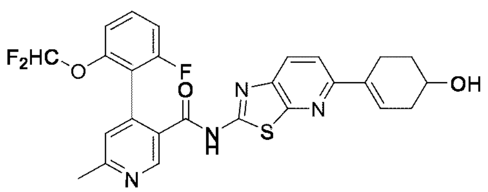 | 527.6 | $^1H$ NMR (400 MHz, DMSO-d6) δ 13.11 (s, 1H), 8.98 (s, 1H), 8.02 (d, *J* = 8.3 Hz, 1H), 7.64 (d, *J* = 8.6 Hz, 1H), 7.54 (dd, *J* = 15.2, 8.3 Hz, 1H), 7.39 (s, 1H), 7.24 (t, *J* = 8.8 Hz, 1H), 7.14 (t, *J* = 72.8 Hz, 1H), 7.12 (d, *J* = 8.4 Hz, 1H), 6.63 - 6.59 (m, 1H), 4.71 (d, *J* = 4.0 Hz, 1H), 3.85 - 3.76 (m, 1H), 2.75 - 2.65 (m, 1H), 2.60 (s, 3H), 2.49 - 2.42 (m, 2H), 2.15 - 2.05 (m, 1H), 1.95 - 1.85 (m, 1H), 1.65 - 1.55 (m, 1H). |
| 8 | 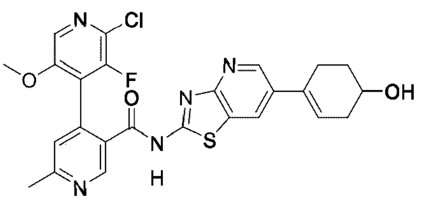 | 526.2 | $^1H$ NMR (400 MHz, DMSO-$d_6$) δ 13.33 (s, 1H), 9.05 (s, 1H), 8.63 (s, 1H), 8.40 (s, 1H), 8.18 (s, 1H), 7.50 (s, 1H), 6.17 - 6.13 (m, 1H), 4.71 (d, *J* = 4.0 Hz, 1H), 3.86 - 3.78 (m, 1H), 3.72 (s, 3H), 2.61 (s, 3H), 2.57 - 2.52 (m, 2H), 2.45 - 2.41 (m, 1H), 2.13 - 2.05 (m, 1H), 1.93 - 1.87 (m, 1H), 1.68 - 1.63 (m, 1H). |

(continued)

| Exam ples | Chemical structure | LC/MS(ESI) m/z $[M+H]^+$ | $^1H$ NMR |
|---|---|---|---|
| 9 | 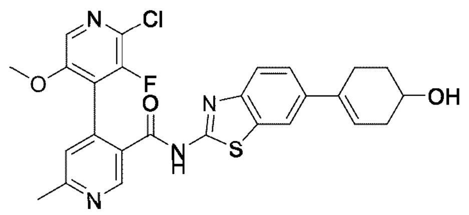 | 485.0 | $^1H$ NMR (400 MHz, DMSO-$d_6$) δ 8.14 (s, 1H), 7.23 (s, 1H), 7.05 (s, 1H), 6.82 (d, $J$ = 8.5 Hz, 1H), 6.73 - 6.65 (m, 2H), 5.29 - 5.23 (m, 1H), 4.07 (d, $J$ = 2.8 Hz, 1H), 3.23 - 3.09 (m, 1H), 2.95 (s, 3H), 1.87 (s, 3H), 1.84 - 1.80 (m, 1H), 1.79 - 1.69 (m, 2H), 1.44 - 1.32 (m, 1H), 1.29 - 1.17 (m, 1H), 1.03 - 0.91 (m, 1H). |
| 10 | 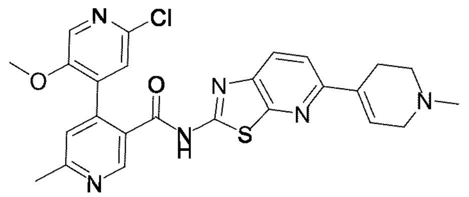 | 507.2 | $^1H$ NMR (400 MHz, DMSO-$d_6$) δ 8.87 (s, 1H), 8.18 (s, 1H), 8.16 (s, 1H) 8.03 (d, $J$ = 8.0 Hz, 1H), 7.67 (d, $J$ = 8.0 Hz, 1H), 7.56 (s, 1H), 7.44 (s, 1H), 6.71 - 6.69 (m, 1H), 3.62 (s, 3H), 3.17 - 3.13 (m, 3H), 2.68-2.55 (m, 3H), 2.60 (s, 3H), 2.36 (s, 3H). |
| 11 | 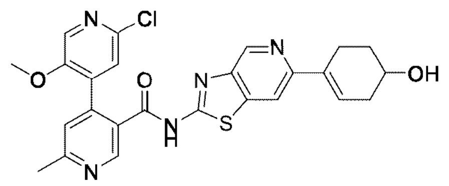 | 508.2 | $^1H$ NMR (400 MHz, DMSO-$d_6$)δ 13.07 (s, 1H), 8.94 (s, 1H), 8.86 (s, 1H), 8.17 (s, 1H), 8.12 (s, 1H), 7.58 (s, 1H), 7.47 (s, 1H), 6.67 - 6.63 (m, 1H), 4.74 - 4.62 (m, 1H), 3.85 - 3.78 (m, 1H), 3.60 (s, 3H), 2.72 - 2.65 (m, 1H), 2.61 (s, 3H), 2.52 - 2.43 (m, 2H), 2.15 - 2.06 (m, 1H), 1.95 - 1.88 (m, 1H), 1.68 - 1.57 (m, 1H). |
| 12 | 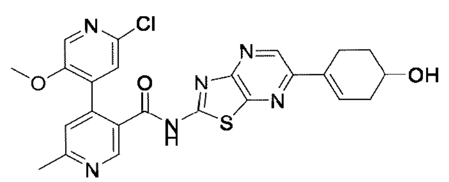 | 509.1 | $^1H$ NMR (400 MHz, DMSO-$d_6$) δ 13.39 (s, 1H), 8.88 (s, 1H), 8.83 (s, 1H), 8.18 (s, 1H), 7.61 (s, 1H), 7.49 (s, 1H), 6.75 - 6.71 (m, 1H), 4.74 (d, $J$ = 3.2 Hz, 1H), 3.87 - 3.78 (m, 1H), 3.63 (s, 3H), 2.77 - 2.63 (m, 3H), 2.61 (s, 3H), 2.18 - 2.08 (m, 1H), 1.95 - 1.87 (m, 1H), 1.68 - 1.59 (m, 1H). |
| 13 | 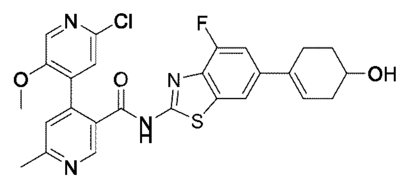 | 525.2 | $^1H$ NMR (400 MHz, DMSO-$d_6$) δ 13.16 (s, 1H), 8.86 (s, 1H), 8.17 (s, 1H), 7.84 (s, 1H), 7.58 (s, 1H), 7.46 (s, 1H), 7.38 (d, $J$ = 12.7 Hz, 1H), 6.17 - 6.14 (m, 1H), 4.69 (d, $J$ = 3.9 Hz, 1H), 3.83 - 3.76 (m, 1H), 3.60 (s, 3H), 2.60 (s, 3H), 2.58 - 2.52 (m, 1H), 2.48 - 2.40 (m, 2H), 2.12 - 2.02 (m, 1H), 1.93 - 1.85 (m, 1H), 1.67 - 1.56 (m, 1H). |
| 14 | 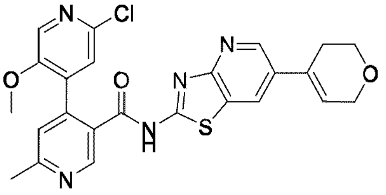 | 494.1 | $^1H$ NMR (400 MHz, DMSO-$d_6$) δ 13.21 (s, 1H), 8.90 (s, 1H), 8.66 (s, 1H), 8.43 (s, 1H), 8.16 (s, 1H), 7.56 (s, 1H), 7.43 (s, 1H), 6.40 - 6.36 (m, 1H), 4.27 - 4.24 (m, 2H), 3.86 (t, $J$ = 5.3 Hz, 2H), 3.61 (s, 3H), 2.60 (s, 3H), 2.56 - 2.52 (m, 2H). |

(continued)

| Examples | Chemical structure | LC/MS(ESI) m/z [M+H]+ | 1H NMR |
|---|---|---|---|
| 15 | 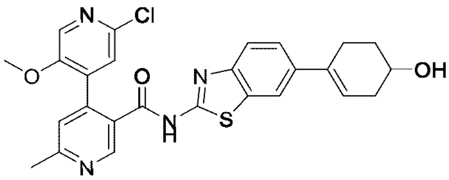 | 507.3 | $^1$H NMR (400 MHz, DMSO-$d_6$) δ 12.90 (s, 1H), 8.84 (s, 1H), 8.16 (s, 1H), 7.98 (s, 1H), 7.68 (d, *J* = 8.3 Hz, 1H), 7.57 (s, 1H), 7.52 (d, *J* = 8.6 Hz, 1H), 7.45 (s, 1H), 6.10 - 6.05 (m, 1H), 4.74 - 4.62 (m, 1H), 3.84 - 3.76 (m, 1H), 3.60 (s, 3H), 2.60 (s, 3H), 2.56 - 2.52 (m, 1H), 2.48 - 2.40 (m, 2H), 2.11 - 2.03 (m, 1H), 1.95 - 1.86 (m, 1H), 1.67 - 1.56 (m, 1H). |
| 16 | 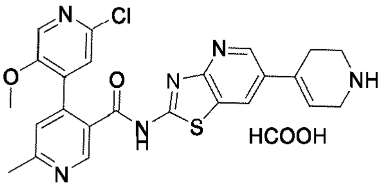 HCOOH | 493.2 | $^1$H NMR (400 MHz, DMSO-$d_6$) δ 9.02 (s, 1H), 8.51 (s, 1H), 8.28 (s, 2H), 8.21 (s, 1H), 8.13 (s, 1H), 7.42 (s, 1H), 7.26 (s, 1H), 6.26 - 6.23 (m, 1H), 3.65 - 3.63 (m, 2H), 3.62 (s, 3H), 3.20 (t, *J* = 5.6 Hz, 2H), 2.65 - 2.59 (m, 2H), 2.56 (s, 3H). |
| 17 | 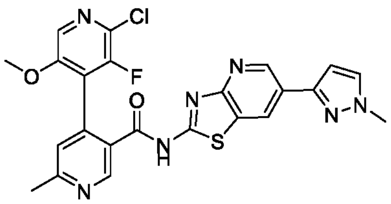 | 510.1 | $^1$H NMR (400 MHz, DMSO-$d_6$) δ 13.36 (s, 1H), 9.04 (s, 1H), 8.99 (s, 1H), 8.74 (s, 1H), 8.17 (s, 1H), 7.77 (s, 1H), 7.48 (s, 1H), 6.79 (s, 1H), 3.92 (s, 3H), 3.74 (s, 3H), 2.63 (s, 3H). |
| 18 | 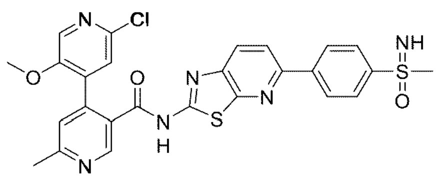 | 564.1 | $^1$H NMR (400 MHz, DMSO-$d_6$) δ 13.18 (s, 1H), 8.89 (s, 1H), 8.35 (d, J = 4 Hz, 2H), 8.23 - 8.15 (m, 3H), 8.04 (d, J = 8 Hz, 2H), 7.59 (s, 1H), 7.46 (s, 1H), 4.29 (s, 1H), 3.63 (s, 3H), 3.12 (s, 3H), 2.61 (s, 3H) |
| 19 | 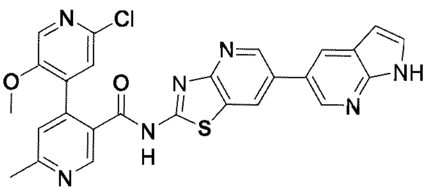 | 528.1 | $^1$H NMR (400 MHz, DMSO-$d_6$) δ 13.25 (s, 1H), 11.79 (s, 1H), 8.93 - 8.88 (m, 2H), 8.76 (s, 1H), 8.60 (d, *J* = 2.2 Hz, 1H), 8.32 (d, *J* = 2.2 Hz, 1H), 8.18 (s, 1H), 7.59 (s, 1H), 7.57 - 7.53 (m, 1H), 7.46 (s, 1H), 6.55 - 6.51 (m, 1H), 3.62 (s, 3H), 2.61 (s, 3H). |
| 20 | 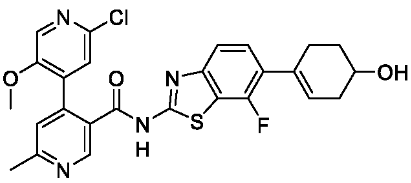 | 525.1 | $^1$H NMR (400 MHz, DMSO-$d_6$) δ 13.17 (s, 1H), 8.86 (s, 1H), 8.17 (s, 1H), 7.62 - 7.58 (m, 2H), 7.47 (s, 1H), 7.44 - 7.38 (m, 1H), 5.89 -5.83 (m, 1H), 4.73 (s, 1H), 3.83 (s, 1H), 3.60 (s, 3H), 2.61 (s, 3H), 2.41 - 2.47 (m, 2H), 2.13 - 2.03 (m, 1H), 1.90 - 1.84 (m, 1H), 1.66 - 1.58 (m, 1H), 1.24 (s, 1H). |
| 21 | 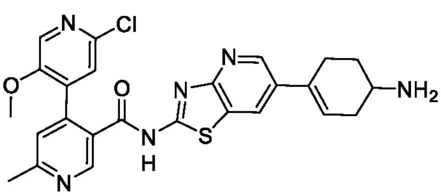 | 507.1 | $^1$H NMR (400 MHz, DMSO-$d_6$) δ 9.10 (s, 1H), 8.36 (s, 1H), 8.10 (s, 1H), 7.99 (s, 1H), 7.32 (s, 1H), 7.14 (s, 1H), 6.08 - 6.03 (m, 1H), 3.62 (s, 3H), 3.25 -3.19 (m, 1H), 2.62 - 2.54(m, 2H), 2.53 (s, 3H), 2.18 - 2.08 (m, 1H), 2.05-1.98 (m, 1H), 1.70 - 1.60 (m, 1H), 1.27 -1.21 (m, 1H). |

(continued)

| Examples | Chemical structure | LC/MS(ESI) m/z [M+H]+ | 1H NMR |
|---|---|---|---|
| 22 | 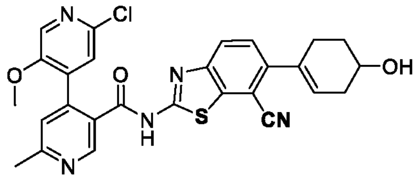 | 532.1 | 1H NMR (400 MHz, DMSO-$d_6$) δ 13.31 (s, 1H), 8.87 (s, 1H), 8.18 (s, 1H), 8.02 (d, *J* = 8.5 Hz, 1H), 7.61 (s, 1H), 7.54 (d, *J* = 8.5 Hz, 1H), 7.49 (s, 1H), 5.97 - 5.93 (m, 1H), 4.79 - 4.75 (m, 1H), 3.86 - 3.83 (m, 1H), 3.60 (s, 3H), 2.61 (s, 3H), 2.46 - 2.41 (m, 3H), 2.13 - 2.08 (m, 1H), 1.90 - 1.85 (m, 1H), 1.69 - 1.64 (m, 1H). |
| 23 | 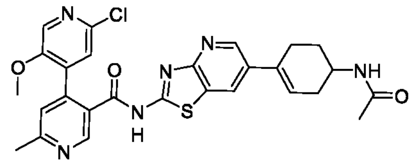 | 549.2 | 1H NMR (400 MHz, DMSO-$d_6$) δ 13.19 (s, 1H), 8.88 (s, 1H), 8.65 (s, 1H), 8.44 (s, 1H), 8.16 (s, 1H), 7.86 (d, J = 7.0 Hz, 1H), 7.58 (s, 1H), 7.46 (s, 1H), 6.23 - 6.18 (m, 1H), 3.93 - 3.83 (m, 1H), 3.60 (s, 3H), 2.60 (s, 3H), 2.58 - 2.55 (m, 1H), 2.55 -2.53 (m, 2H), 2.15 - 2.05 (m, 1H), 1.96 - 1.89 (m, 1H), 1.82 (s, 3H), 1.68 - 1.59 (m, 1H). |
| 24 | 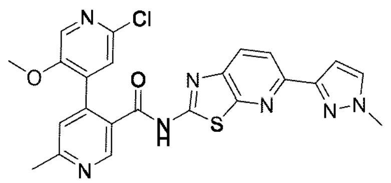 | 492.3 | 1H NMR (400 MHz, DMSO-$d_6$) δ 13.09 (s, 1H), 8.87 (s, 1H), 8.18 (s, 1H), 8.15 (d, *J* = 8.5 Hz, 1H), 8.04 (d, *J* = 8.5 Hz, 1H), 7.79 (d, *J* = 2.1 Hz, 1H), 7.60 (s, 1H), 7.50 (s, 1H), 6.86 (d, *J* = 2.1 Hz, 1H), 3.94 (s, 3H), 3.63 (s, 3H), 2.62 (s, 3H). |
| 25 | 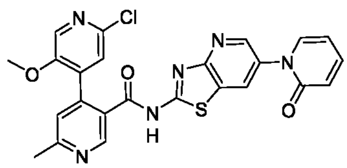 | 505.2 | 1H NMR (400 MHz, DMSO-$d_6$) δ 13.36 (s, 1H), 8.91 (s, 1H), 8.57 (s, 2H), 8.16 (s, 1H), 7.78 (d, *J* = 6.5 Hz, 1H), 7.63 - 7.52 (m, 2H), 7.47 (s, 1H), 6.54 (d, *J* = 9.4 Hz, 1H), 6.38 (t, *J* = 6.8 Hz, 1H), 3.61 (s, 3H), 2.61 (s, 3H). |

**Example 26 and Example 27: 2'-chloro-N-(6-((1s, 4s)-4-hydroxycyclohexyl)thiazolo[4,5-b]pyridin-2 -yl)-5'-methoxy-6-methyl-[4,4'-bipyridine]-3-carboxamide and 2'-chloro-N-(6-((1r, 4r)-4-hydroxycycl ohexyl)thiazolo [4,5-b]pyridin-2-yl)-5'-methoxy-6-methyl-[4,4'-bipyridine]-3-carboxamide**

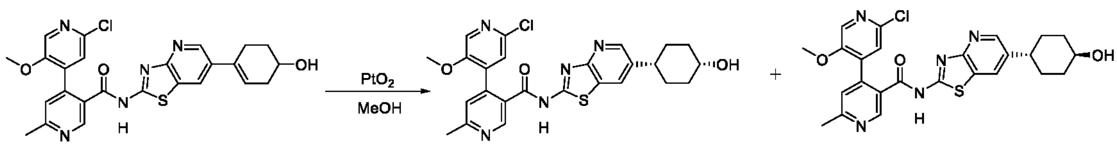


**Step 1: 2'-chloro-N-(6-((1s, 4s)-4-hydroxycyclohexyl)thiazolo[4,5-b]pyridin-2-yl)-5'-methoxy-6-methy l-[4,4'-bipyridine]-3-carboxamide** and **2'-chloro-N-(6-((1r, 4r)-4-hydroxycyclohexyl)thiazolo[4,5-b]py ridin-2-yl)-5'-methoxy-6-methyl-[4,4'-bipyridine]-3-carboxamide**

2'-chloro-N-(6-(4-hydroxycyclohex-1-en-1-yl)thiazolo[4,5-b]pyridin-2-yl)-5'-methoxy-6-methyl-[4,4'-bipyri dine]-3-carboxamide (40 mg, 78.7 μmol) was dissolved in methanol (10 mL); platinum oxide (40 mg, 176 μmol) was added; the resulting mixture was subjected to reaction for 6 hours in the presence of a hydrogen balloon; the reaction mixture was filtered; the filtrate was concentrated under reduced pressure, and treated by pre-HPLC to subsequently obtain two isomers, which were freeze-dried respectively to obtain 2'-chloro-N-(6-((1s, 4s)-4-hydroxycyclohexyl)thiazolo[4,5-b]pyridin-2-yl)-5'-methoxy-6-methyl-[4,4'-bipyridine]-3-carboxamide (white powder, 2.8 mg, yield: 7.0%, $^1$H NMR (400 MHz, $CD_3OD$) δ 8.86 (s, 1H), 8.47 (s, 1H), 8.39 (s, 1H), 8.04 (s, 1H), 7.51 (s, 1H), 7.45 (s, 1H), 3.70 (s, 3H), 3.68-3.64 (m, 1H), 2.80-2.74 (m, 1H), 2.71 (s, 3H), 2.14-2.08 (m, 2H), 2.04-1.97 (m, 2H), 1.73-1.61 (m, 2H), 1.43-1.45 (m, 2H). LC/MS (ESI) m/z: 510.2 $[M+H]^+$), and 2'-chloro-N-(6-((1r, 4r)-4-hydroxycyclohexyl)thiazolo[4,5-b]pyridin-2-yl)-5'-methoxy-6-methyl-[4,4'-bipyridine]-3-carboxamide (white powder, 5.6 mg, yield: 13.9%), $^1$H NMR (400 MHz, $CD_3OD$) δ 8.85 (s, 1H), 8.46 (s, 1H), 8.32 (s, 1H), 8.04 (s, 1H), 7.50 (s, 1H), 7.43 (s, 1H), 4.11-4.07 (m, 1H), 3.70 (s, 3H), 2.78-2.74 (m, 1H), 2.69 (s, 3H), 2.05-1.90 (m, 4H), 1.78-1.68 (m, 4H). LC/MS (ESI) m/z: 510.1$[M+H]^+$.

The example compounds in Table 2 were synthesized with reference to the synthesis steps of Examples 26 and 27.

Table 2:

| Examples | Chemical structure | LC/MS(ESI) m/z $[M+H]^+$ | $^1$H NMR |
|---|---|---|---|
| 28 | 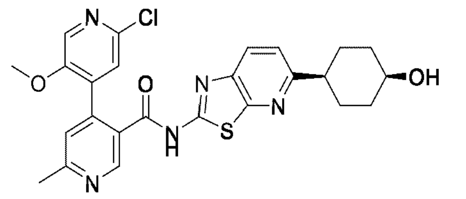 | 510.2 | $^1$H NMR (400 MHz, DMSO-$d_6$) δ 12.97 (s, 1H), 8.84 (s, 1H), 8.16 (s, 1H), 8.02 (d, *J* = 8.3 Hz, 1H), 7.58 (s, 1H), 7.46 (s, 1H), 7.38 (d, *J* = 8.4 Hz, 1H), 4.58 (d, *J* = 4.4 Hz, 1H), 3.61 (s, 3H), 3.51 - 3.44 (m, 1H), 2.76 - 2.68 (m, 1H), 2.60 (s, 3H), 1.95 - 1.85 (m, 4H), 1.66 - 1.57 (m, 2H), 1.38 - 1.22 (m, 2H). |
| 29 | 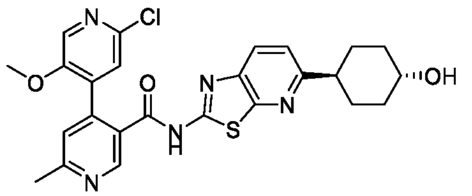 | 510.2 | $^1$H NMR (400 MHz, DMSO-$d_6$) δ 12.97 (s, 1H), 8.85 (s, 1H), 8.16 (s, 1H), 8.03 (d, *J* = 8.4 Hz, 1H), 7.58 (s, 1H), 7.46 (s, 1H), 7.38 (d, *J* = 8.4 Hz, 1H), 4.36 (d, *J* = 3.1 Hz, 1H), 3.89 (s, 1H), 3.61 (s, 3H), 2.83 - 2.77 (m, 1H), 2.59 (s, 3H), 2.03 - 1.93 (m, 2H), 1.76 - 1.72 (m, 2H), 1.62 - 1.53 (m, 4H). |
| 30 | 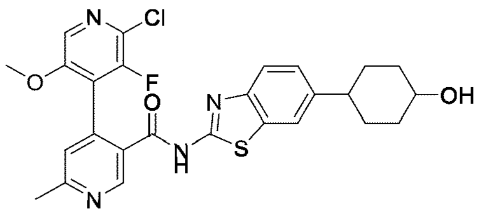 | 527.3 | $^1$H NMR (400 MHz, DMSO-$d_6$) δ 13.00 (s, 1H), 9.02 (s, 1H), 8.17 (s, 1H), 7.78 (s, 1H), 7.64 (d, *J* = 8.0 Hz, 1H), 7.48 (s, 1H), 7.31 (d, *J* = 8.0 Hz, 1H), 4.57 (d, *J* = 4.4 Hz, 1H), 3.70 (s, 3H), 3.52 - 3.41 (m, 1H), 2.60 (s, 3H), 2.57 - 2.54 (m, 1H), 1.94 - 1.91 (m, 2H), 1.82 - 1.78 (m, 2H), 1.57 - 1.48 (m, 2H), 1.34 - 1.26 (m, 2H) |
| 31 | 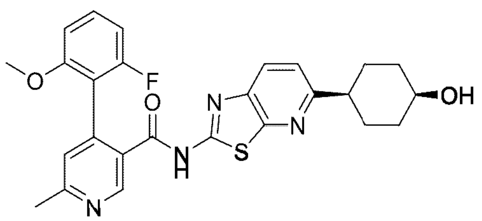 | 493.2 | $^1$H NMR (400 MHz, $CD_3OD$) δ 8.93 (s, 1H), 7.99 (d, *J* = 8.4 Hz, 1H), 7.66 (s, 1H), 7.49 - 7.41 (m, 1H), 7.38 (d, *J* = 8.4 Hz, 1H), 6.95 - 6.80 (m, 2H), 3.69 - 3.58 (m, 4H), 2.85 - 2.75 (m, 1H), 2.74 (s, 3H), 2.13 - 2.05 (m, 2H), 2.03 - 1.93 (m, 2H), 1.78 - 1.66 (m, 2H), 1.50 - 1.37 (m, 2H). |

(continued)

| Examples | Chemical structure | LC/MS(ESI) m/z [M+H]+ | 1H NMR |
|---|---|---|---|
| 32 | 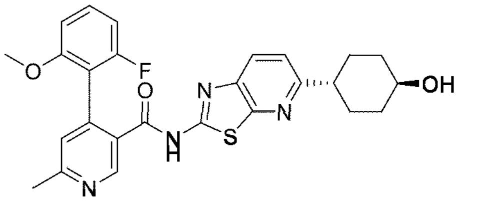 | 493.2 | $^1$H NMR (400 MHz, $CD_3OD$) δ 8.95 (s, 1H), 8.02 (d, *J* = 8.4 Hz, 1H), 7.71 (s, 1H), 7.50 - 7.41 (m, 2H), 6.95 - 6.85 (m, 2H), 4.08 - 4.03 (m, 1H), 3.66 (s, 3H), 2.92 - 2.84 (m, 1H), 2.75 (s, 3H), 2.16 - 2.00 (m, 2H), 1.98 - 1.86 (m, 2H), 1.78 - 1.66 (m, 4H). |
| 33 | 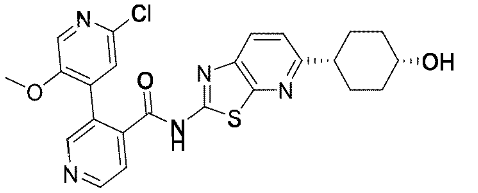 | 496.1 | $^1$H NMR (400 MHz, DMSO-$d_6$) δ 8.67 (d, *J* = 5.0 Hz, 1H), 8.48 (s, 1H), 8.09 (s, 1H), 7.84 (d, *J* = 5.0 Hz, 1H), 7.65 (d, *J* = 8.1 Hz, 1H), 7.41 (s, 1H), 7.11 (d, *J* = 8.3 Hz, 1H), 4.59 - 4.53 (m, 1H), 3.60 (s, 3H), 3.37 - 3.33 (m, 1H), 2.67 - 2.57 (m, 1H), 1.95 - 1.89 (m, 2H), 1.86 - 1.80 (m, 2H), 1.62 - 1.52 (m, 2H), 1.34 - 1.20 (m, 2H). |
| 34 | 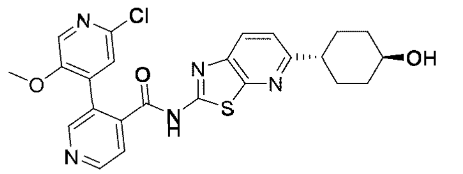 | 496.2 | $^1$H NMR (400 MHz, $CDCl_3$) δ 8.82 (d, *J* = 5.0 Hz, 1H), 8.68 (s, 1H), 8.04 (s, 1H), 7.62 (d, *J* = 4.9 Hz, 1H), 7.37 (d, *J* = 8.4 Hz, 1H), 7.25 (s, 1H), 7.17 (d, *J* = 8.5 Hz, 1H), 4.16 - 4.12 (m, 1H), 3.72 (s, 3H), 2.90 - 2.78 (m, 1H), 2.08 - 1.97 (m, 2H), 1.95 - 1.86 (m, 2H), 1.84 - 1.66 (m, 4H). |
| 35 | 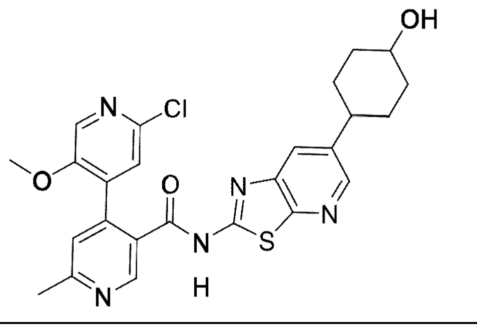 | 510.2 | $^1$HNMR (400MHz, DMSO-$d_6$) δ 9.00 (s, 1H), 8.15 (s, 1H), 8.12 (s, 1H), 7.64 (s, 1H), 7.40 (s, 1H), 7.24 (s, 1H), 4.39 (d, *J* = 3.6 Hz, 1H), 3.92 - 3.88 (m, 1H), 3.62 (s, 3H), 2.67 - 2.59(m, 1H), 2.55 (s, 3H), 1.94 - 1.84 (m, 2H), 1.80 - 1.72( m, 2H), 1.62 - 1.52 (m, 4H). |
| 36 | 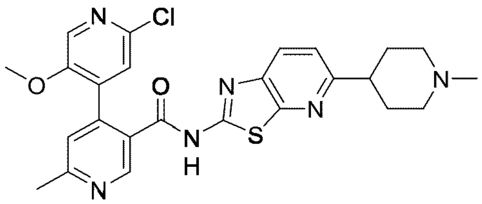 | 509 | $^1$H NMR (400 MHz, $CD_3OD$) δ 8.83 (s, 1H), 8.07(s, 1H), 8.04 (d, *J* = 8.4 Hz, 1H), 7.54 (s, 1H), 7.49 (s, 1H), 7.42 (d, *J* = 8.4 Hz, 1H), 3.70 (s, 3H), 3.65 - 3.58 (m, 2H), 3.17 - 3.06 (m, 3H), 2.93 (s, 3H), 2.70 (s, 3H), 2.27 - 2.13 (m, 4H) |
| 37 | 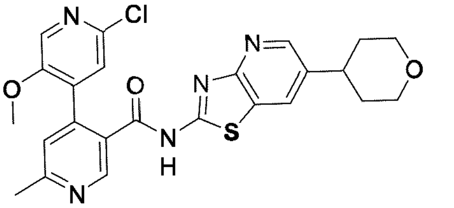 | 496.1 | $^1$H NMR (400 MHz, DMSO- $d_6$) δ 13.14 (s, 1H), 8.87 (s, 1H), 8.49 (s, 1H), 8.36 (s, 1H), 8.16 (s, 1H), 7.59 (s, 1H), 7.47 (s, 1H), 3.98 - 3.94 (m, 2H), 3.59 (s, 3H), 3.50 - 3.43 (m, 2H), 2.97 - 2.93 (m, 1H), 2.61 (s, 3H), 1.78 - 1.72 (m, 4H). |

**Example 38: 2'-chloro-3'-fluoro-5'-methoxy-6-methyl-N-(thiazol[5,4-b]pyridin-2-yl)-[4,4'-bipyridine]-3-carboxamide**

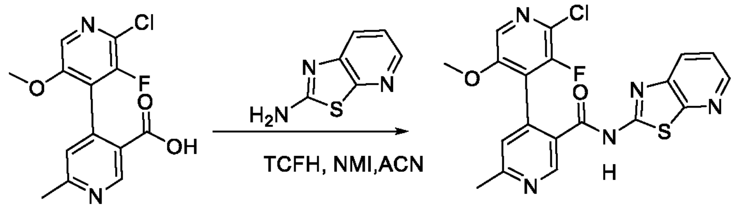


**Step 1: 2'-chloro-3'-fluoro-5'-methoxy-6-methyl-N-(thiazol[5,4-b]pyridin-2-yl)-[4,4'-bipyridine]-3-car boxamide**

4-(2-chloro-3-fluoro-5-methoxypyridin-4-yl)-6-methylpyridin-3-carboxylic acid (40.0 mg, 0.135 mmol) and benzo[d][1,3]thiazol-2-amine (20.4 mg, 0.135 mmol) were dissolved in acetonitrile (2 mL). N-methylimidazole (33.21 mg, 0.404 mmol) was added; under stirring, N,N,N',N'-tetramethylchlorformamidine hexafluorphosphate (37.83 mg, 0.135 mmol) was dissolved in acetonitrile (1 mL), to allow for reaction under a room temperature condition for 1 hour. The reaction mixture was concentrated under reduced pressure to obtain residues, which were processed by reversed-phase C18 column chromatography to obtain 2'-chloro-3'-fluoro-5'-methoxy-6-methyl-N-(thiazol[5,4-b]pyridin-2-yl)-[4,4'-bipyridine]-3-formyl. $^1$H NMR (400 MHz, DMSO-$d_6$) δ 13.22 (s, 1H), 9.03 (s, 1H), 8.48 (d, *J* = 3.9 Hz, 1H), 8.19 (s, 1H), 8.13 (d, *J* = 6.0 Hz, 1H), 7.53 - 7.48 (m, 2H), 3.73 (s, 3H), 2.61 (s, 3H). LC/MS (ESI) m/z: 430.1 $[M+H]^+$.

The example compounds in Table 3 were synthesized using commercially available raw materials with reference to the synthesis steps of Example 38.

Table 3:

| Examples | Chemical structure | LC/MS(ESI) m/z $[M+H]^+$ | $^1$H NMR |
|---|---|---|---|
| 39 | 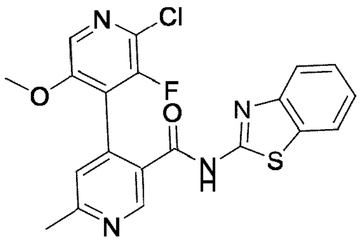 | 429.0 | $^1$H NMR (400 MHz, DMSO-$d_6$) δ 9.02 (s, 1H), 8.18 (s, 1H), 7.96 (d, *J* = 7.8 Hz, 1H), 7.77 (d, *J* = 8.1 Hz, 1H), 7.51 (s, 1H), 7.49 - 7.44 (m, 1H), 7.38 - 7.28 (m, 1H), 3.72 (s, 3H), 2.61 (s, 3H) |

**Example 40: 2'-chloro-3'-fluoro-5'-methoxy-6-methyl-N-(5-((tetrahydrofuran-3-yl)methyl)-4,5,6,7-he xahydrothiazolo[5,4-c]pyridin-2-yl)-[4,4'-bipyridine]-3-carboxamide**

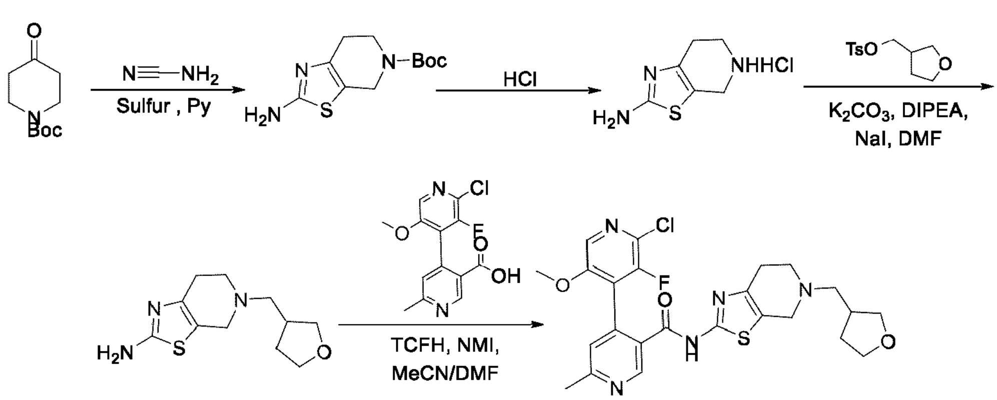

**Step 1: 2-amino-6,7-dihydrothiazolo[5,4-c]pyridin-5(4H)-tert-butyl carboxylate**

N-tert-butoxycarbonyl-4-piperidone (2 g, 10.1 mmol), cyanamide (844 mg, 20.2 mmol), and powdered sulfur (684 mg, 20.2 mmol) were dissolved in 10 mL of pyridine, and heated to 130°C to react for 2 hours. The reaction mixture was cooled to room temperature, filtered, and then extracted with water and ethyl acetate; and the organic phase was spin-dried to obtain the title compound (2.11 g, black brown solid, yield: 82.3%). LC/MS (ESI) m/z: 256.1 $[M+H]^+$.

**Step 2: 4,5,6,7-tetrahydrothiazol[5,4-c]pyridin-2-amine hydrochloride**

2-amino-6,7-dihydrothiazolo[5,4-c]pyridin-5(4H)-tert-butyl carboxylate (2 g, 7.83 mmol) and 4mol/L hydrogen chloride 1,4-dioxane solution (20 mL) were dissolved in 10 mL of 1,4-dioxane, to allow for reaction at room temperature for 2 hours. The reaction mixture was filtered under reduced pressure; the solid phase was washed with ethyl acetate and dried under reduced pressure, to subsequently obtain the title compound (1.5 g, white solid, yield: 100%). LC/MS (ESI) m/z: 156.1 $[M+H]^+$.

**Step 3: 5-((tetrahydrofuran -3-yl)methyl)-4,5,6,7-tetrahydrothiazol[5,4-c]pyridin-2-amine**

4,5,6,7-tetrahydrothiazol[5,4-c]pyridin-2-amine hydrochloride (500 mg, 2.61 mmol) was dissolved in 10 mL of methanol; ammonia was added dropwise to allow for dissociation; the resulting mixture was spin-dried under reduced pressure, and dissolved in N,N-dimethylformamide (10 mL); subsequently, (tetrahydrofuran-3-yl)methyl-4-tosylate (668 mg, 2.61 mmol), potassium carbonate (1.08 g, 7.82 mmol), N,N-diisopropylethylamine (674 mg, 5.22 mmol), and sodium iodide (78.2 mg, 0.522 mmol) were added in sequence. The temperature of the mixture was increased to 60°C to allow for reaction for 24 hours. The reaction mixture was filtered, and then concentrated under reduced pressure; the filtrate was concentrated; the residue was purified by silica-gel column chromatography (dichloromethane methanol system, gradient: 0-5% methanol) to obtain the title compound (133 mg, white solid, yield: 26.7%). LC/MS (ESI) m/z: 240.1 $[M+H]^+$.

**Step 4: 2'-chloro-3'-fluoro-5'-methoxy-6-methyl-N-(5-((tetrahydrofuran -3-yl)methyl)-4,5,6,7-hexahy drothiazolo [5,4-c]pyridin-2-yl)-[4,4'-bipyridine]-3-carboxamide**

5-((tetrahydrofuran -3-yl)methyl)-4,5,6,7-tetrahydrothiazol[5,4-c]pyridin-2-amine (80 mg, 0.270 mmol), 2'-chloro-3'-fluoro-5'-methoxy-6-methyl-[4,4'-bipyridine]-3-carboxylic acid (70.1 mg, 0.2 mmol), N-methylimidazole (44.1 mg, 0.539 mmol), and N,N,N',N'-tetramethylchlorformamidine hexafluorphosphate (90.1 mg, 0.324 mmol) were dissolved in the mixed solvent of acetonitrile (1 mL) and N,N-dimethylformamide (1 mL), and stirred at room temperature to react for 24 hours. The reaction mixture was washed with a small amount of water, and extracted with ethyl acetate; then, the organic phase was dried over anhydrous sodium sulfate, and filtered; the filtrate was concentrated and spin-dried; and the residue was purified by pre-HPLC to obtain the title compound (30 mg, faint yellow solid, yield: 20%). $^1H$ NMR (400 MHz, $CDCl_3$) $\delta$ 9.38 (s, 1H), 7.97 (s, 1H), 7.47 (s, 1H), 4.52 - 4.40(m, 2H), 3.99 - 3.89 (m, 2H), 3.81 - 3.76 (m, 2 H), 3.75 (s, 3H), 3.59 - 3.51 (m, 2H), 3.27 - 3.24 (m, 2H), 3.06 - 3.01 (m, 2H), 2.84 (s, 3H), 2.81 - 2.72 (m, 1H), 2.26 - 2.23 (m, 1H), 1.75 - 1.71 (m, 1H). LC/MS (ESI) m/z: 518.2 $[M+H]^+$.

**Example 41: 2'-chloro-5'-methoxy-6-methyl-N-(4,5,6,7-tetrahydrothiazol[5,4-c]pyridin-2-yl)-[4,4'-bip yridine]-3-carboxamide**

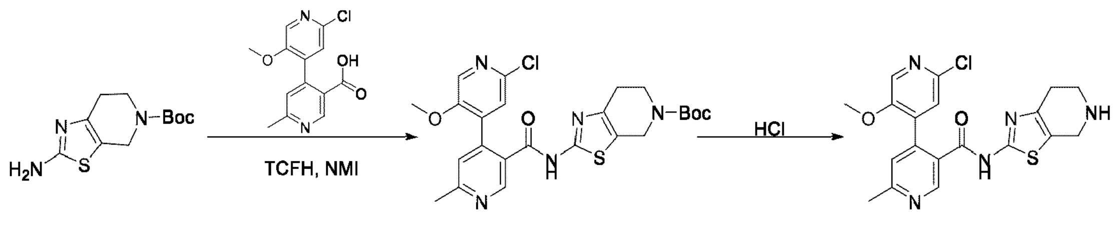


**Step 1: 2-(2'-chloro-5'-methoxy-6-methyl-[4,4'-bipyridine]-3-formamido)-6,7-dihydrothiazol[5,4-c]py ridin-5(4H)-tert-butyl carboxylate**

2-amino-6,7-dihydrothiazol[5,4-c]pyridin-5(4H)-tert-butyl carboxylate (200 mg, 0.783 mmol) and 2'-chloro-5'-methoxy-6-methyl-[4,4'-bipyridine]-3-carboxylic acid (190 mg, 0.783 mmol) were dissolved in acetonitrile (5 mL); and

under the protection of nitrogen, N,N,N',N'-tetramethylchlorformamidine hexafluorphosphate (219 mg, 0.783 mmol) and N-methylimidazole (193 mg, 2.35 mmol) were added, to allow for reaction under room temperature condition for 16 hours. The reaction mixture was concentrated under reduced pressure to obtain residues, which were processed by silica-gel column chromatography (petroleum etherethyl acetate system) to obtain 2-(2'-chloro-5'-methoxy-6-methyl-[4,4'-bipyridine]-3-formamido)-6,7-dihydrothiazol[5,4-c]pyridin-5(4H)-te rt-butyl carboxylate (100 mg, yellow oil), yield: 28.4%. LC/MS m/z (ESI): 244.0 $[M+H]^+$.

**Step 2: 2'-chloro-5'-methoxy-6-methyl-N-(4,5,6,7-tetrahydrothiazol[5,4-c]pyridin-2-yl)-[4,4'-bipyridin e]-3-carboxamide**

2-(2'-chloro-5'-methoxy-6-methyl-[4,4'-bipyridine]-3-formamido)-6,7-dihydrothiazol[5,4-c]pyridin-5(4H)-te rt-butyl carboxylate (100 mg, 0.194 mmol) was dissolved in acetonitrile (1 mL); and 2 mol/L ethyl acetate hydrochloride solution (1 mL) was added; and the resulting mixture was stirred under room temperature condition for 2 hours. The reaction mixture was concentrated under reduced pressure to obtain residues, which were processed by reversed-phase C18 column chromatography to obtain 2'-chloro-5'-methoxy-6-methyl-N-(4,5,6,7-tetrahydrothiazol[5,4-c]pyridin-2-yl)-[4,4'-bipyridine]-3-carboxa mide (16.9 mg, white solid, yield: 21.0%). $^1H$ NMR (400 MHz, $CD_3OD$) δ 8.75 (s, 1H), 8.05 (s, 1H), 7.49 (s, 1H), 7.43 (s, 1H), 4.29 (s, 2H), 3.68 (s, 3H), 3.48 (t, *J* = 6.0 Hz, 2H), 2.95 (t, *J* = 6.0 Hz, 2H), 2.67 (s, 3H). LC/MS m/z (ESI): 290.2 $[M+H]^+$.

**Example 42: 2'-chloro-N-(5-(4-hydroxycyclohexyl)-4,5,6,7-tetrahydrothiazol[5,4-c]pyridin-2-yl)-5'-m ethoxy-6-methyl-[4,4'-bipyridine]-3-carboxamide**

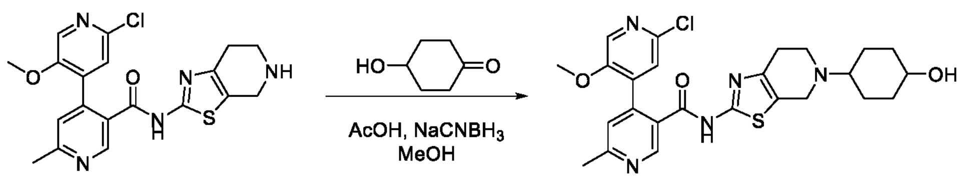


**Step 1: 2'-chloro-N-(5-(4-hydroxycyclohexyl)-4,5,6,7-tetrahydrothiazol[5,4-c]pyridin-2-yl)-5'-methoxy -6-methyl-[4,4'-bipyridine]-3-carboxamide**

2'-chloro-5'-methoxy-6-methyl-N-(4,5,6,7-tetrahydrothiazol[5,4-c]pyridin-2-yl)-[4,4'-bipyridine]-3-carboxa mide (170 mg, 0.447 mmol) and tetraethyl titanate (0.043 mL, 0.205 mmol) were dissolved in methanol (5 mL); then, (4-hydroxycyclohexan-1-one (0.043 mL, 0.205 mmol) was added; the resulting mixture was stirred at room temperature for half an hour. Then, sodium cyanoborohydride (77.2 mg, 1.23 mmol) was added, to allow for reaction at room temperature for16 hours. The reaction mixture was filtered; the stock solution was concentrated under reduced pressure to obtain residues, which were treated by reversed-phase preparation (acetonitrile-water system) using reversed-phase C18 columns to obtain 2'-chloro-N-(5-(4-hydroxycyclohexyl)-4,5,6,7-tetrahydrothiazol[5,4-c]pyridin-2-yl)-5'-methoxy-6-methyl-[4, 4'-bipyridine]-3-carboxamide (3.5 mg, white solid, yield: 1.6%). $^1H$ NMR (400 MHz, $CD_3OD$) δ 8.75 (s, 1H), 8.05 (s, 1H), 7.49 (s, 1H), 7.43 (s, 1H), 4.34 (s, 2H), 4.02 - 3.97 (m, 1H), 3.67 (s, 3H), 3.55 - 3.48, (m, 2H), 3.20-3.16(m, 1H), 3.04 - 2.98 (m, 2H), 2.67 (s, 3H), 2.01 - 1.92, (m, 4H), 1.91 - 1.85, (m, 2H), 1.69 - 1.59 (m, 2H). LC/MS m/z (ESI): 559.8 $[M+H]^+$.

**Example 43: 2'-chloro-5'-methoxy-6-methyl-N-(5-(4-methylpiperazin-1-yl)thiazol[5,4-b]pyridin-2-yl)-[4,4'-bipyridine]-3-carboxamide**

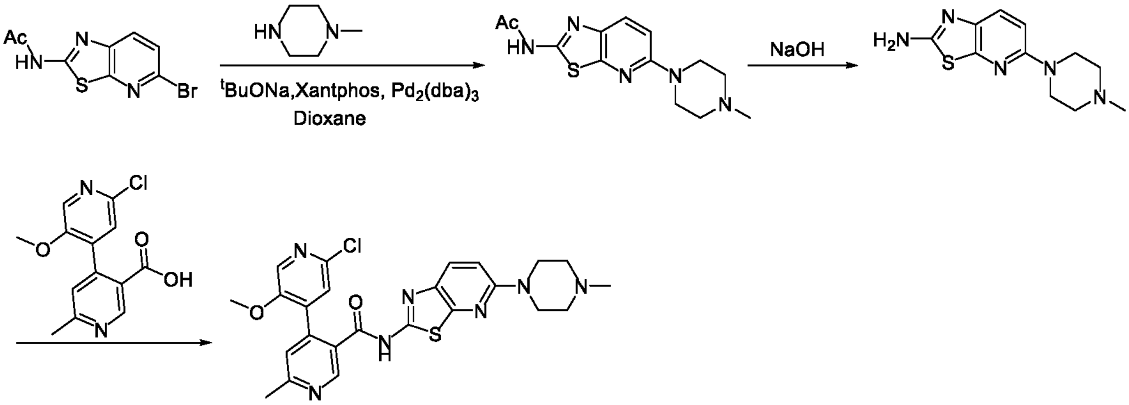


**Step 1: N-(5-(4-methylpiperazin-1-yl)thiazol[5,4-b]pyridin-2-yl)acetamide**

N-(5-bromothiazol[5,4-b]pyridin-2-yl)acetamide (100 mg, 0.367 mmol), 1-methylpiperazine (55 mg, 0.551 mmol), and sodium tert-butoxide (106 mg, 1.101 mmol) were dissolved in dioxane (8 mL); under the protection of nitrogen, tris(dibenzylideneacetone)dipalladium (34 mg, 0.037 mmol) and 4,5-bisdiphenylphosphino-9,9-dimethylxanthene (42 mg, 0.073 mmol) were added; and under the protection of nitrogen, the resulting mixture was heated to 100°C to react for 10 hours. After complete reaction, the reaction mixture was filtered; and the filtrate was concentrated. The residue was purified by column chromatography (eluent: dichloromethane/methanol, gradient: 0-10% methanol) to obtain the title compound (yellow oil, 70 mg, yield: 65.5%). LC/MS (ESI) m/z: 292 $[M+H]^+$.

**Step 2: 5-(4-methylpiperazin-1-yl)thiazol[5,4-b]pyridin-2-amine**

N-(5-(4-methylpiperazin-1-yl)thiazol[5,4-b]pyridin-2-yl)acetamide (65 mg, 0.223 mmol) was dissolved in ethanol (3 mL) and water (3 mL); sodium hydroxide (18 mg, 0.446 mmol) was added to the mixture, and stirred at 60°C for 10 hours. The reaction mixture was cooled to room temperature, and acidified to pH-7 with 1N hydrochloric acid to obtain residues, which were purified by reversed-phase C18 column chromatography to obtain the title compound (yellow solid, 40 mg, yield: 70.9%). LC/MS (ESI) m/z: 250 $[M+H]^+$.

**Step 3: 2'-chloro-5'-methoxy-6-methyl-N-(5-(4-methylpiperazin-1-yl)thiazol[5,4-b]pyridin-2-yl)-[4,4'-bipyridine]-3-carboxamide**

5-(4-methylpiperazin-1-yl)thiazol[5,4-b]pyridin-2-amine (40 mg, 0.161 mmol) and 2'-chloro-5'-methoxy-6-methyl-[4,4'-bipyridine]-3-carboxylic acid (54 mg, 0.193 mmol) were dissolved in acetonitrile (2 mL) and N,N-dimethylformamide (2 mL); and under the protection of nitrogen, N,N,N',N'-tetramethylchlorformamidine hexafluorphosphate (68 mg, 0.241 mmol) and N-methylimidazole (40 mg, 0.481 mmol) were added, to allow for reaction under room temperature condition for 10 hours. The reaction mixture was concentrated under reduced pressure; and the residue was purified by pre-HPLC to obtain the title compound (yellow solid, 11 mg, yield: 13.5%). $^1$H NMR (400 MHz, $CD_3OD$) δ 8.85 (s, 1H), 8.08 (s, 1H), 7.92 (d, *J* = 9.0 Hz, 1H), 7.55 (s, 1H), 7.53 (s, 1H), 7.07 (d, *J* = 9.1 Hz, 1H), 4.56 - 4.53 (m, 2H), 3.71 (s, 3H), 3.62 - 3.60 (m, 2H), 3.26 - 3.20 (m, 4H), 2.96 (s, 3H), 2.71 (s, 3H). LC/MS (ESI) m/z: 510.1 $[M+H]^+$.

**Example 44: 2'-chloro-N-(5-(5-chloropyridin-2-yl)thiazol[5,4-b]pyridin-2-yl)-5'-methoxy-6-methyl-[4, 4'-bipyridine]-3-carboxamide**

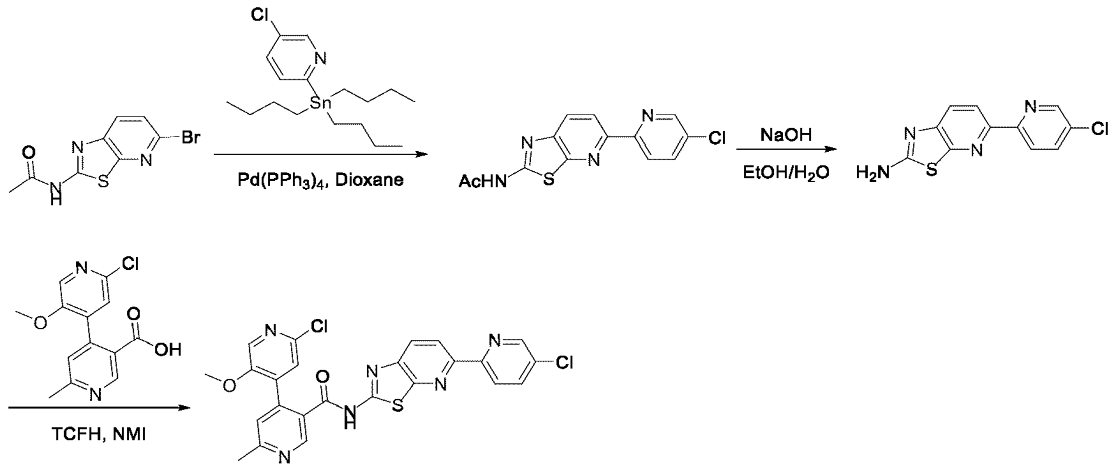


**Step 1: N-(5-(5-chloropyridin-2-yl)thiazol[5,4-b]pyridin-2-alkyl)acetamide**

N-(5-bromothiazol[5,4-b]pyridin-2-yl)acetamide (100 mg, 0.367 mmol) and 5-chloro-2-(tributylstannyl)pyridine (148 mg, 0.367 mmol) were dissolved in dioxane (8 mL); and under the protection of nitrogen, tetrakis(triphenylphosphine) palladium (42 mg, 0.037 mmol) was added, to allow for reaction at 120°C for 10 hours. After complete reaction, the reaction mixture was filtered; and the filtrate was concentrated. The residue was purified by reversed-phase C18 column chromatography to obtain the title compound (yellow solid, 80 mg, yield: 71.4%). LC/MS (ESI) m/z: 305 $[M+H]^+$.

**Step 2: 5-(5-chloropyridin-2-yl)thiazol[5,4-b]pyridin-2-amine**

Sodium hydroxide (21 mg, 0.526 mmol) was added to the mixture obtained by dissolving N-(5-(5-chloropyridin-2-yl)thiazol[5,4-b]pyridin-2-alkyl)acetamide (80 mg, 0.263 mmol) in ethanol (3 mL) and water (3 mL), and stirred at 60°C for 10 hours. The mixture was acidified to pH-7 with 1N hydrochloric acid to obtain residues, which were purified by reversed-phase C18 column chromatography to obtain the title compound (yellow solid, 50 mg, yield: 72.5%). LC/MS (ESI) m/z: 263 $[M+H]^+$.

**Step 3: 2'-chloro-N-(5-(5-chloropyridin-2-yl)thiazol[5,4-b]pyridin-2-yl)-5'-methoxy-6-methyl-[4,4'-bip yridine]-3-carboxamide**

5-(5-chloropyridin-2-yl)thiazol[5,4-b]pyridin-2-amine (50 mg, 0.191 mmol) and 2'-chloro-5'-methoxy-6-methyl-[4,4'-bipyridine]-3-carboxylic acid (64 mg, 0.228 mmol) were dissolved in acetonitrile (2 mL) and N,N-dimethylformamide (2 mL); and under the protection of nitrogen, N,N,N',N'-tetramethylchlorformamidine hexafluorphosphate (80 mg, 0.285 mmol) and N-methylimidazole (47 mg, 0.571 mmol) were added, to allow for reaction under room temperature condition for 10 hours. The reaction mixture was concentrated under reduced pressure; and the residue was purified by pre-HPLC to obtain the title compound (yellow solid, 10 mg, yield: 10.0%). $^1H$ NMR (400 MHz, DMSO-$d_6$) δ 13.23 (s, 1H), 8.88 (s, 1H), 8.75 (s, 1H), 8.49(d, $J$ = 8.0 Hz, 1H), 8.44 (d, $J$ = 8.0 Hz, 1H), 8.27 (d, $J$ = 8.4 Hz, 1H), 8.18 (s, 1H), 8.08 (d, J= 7.9 Hz, 1H), 7.61 (s, 1H), 7.49 (s, 1H), 3.63 (s, 3H), 2.62 (s, 3H). LC/MS (ESI) m/z: 523 $[M+H]^+$.

**Example 45: 2-chloro-5-methoxy-6-methyl-N-(6-morpholinylbenzothiazol-2-yl)-4, 4'-bipyridin-3-car boxamide**

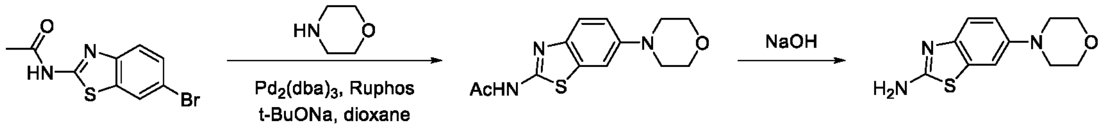


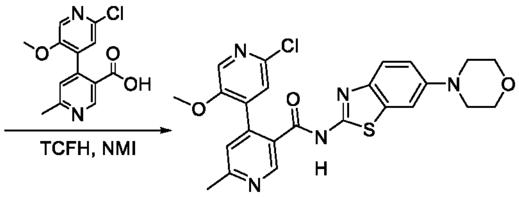


**Step 1: N-(6-morpholinylbenzothiazol-2-yl)acetamide**

6-bromo-2-acetamidobenzothiazole (500 mg, 1.84 mmol), morpholine (0.18 mL, 2.03 mmol), sodium tert-butoxide (354 mg, 3.69 mmol), 2-dicyclohexylphosphino-2',6'-diisopropoxy-1,1'-biphenyl (86.1 mg, 0.18 mmol), and tris(dibenzylideneacetone)dipalladium (84.4 mg, 0.09 mmol) were dissolved in 10 mL of anhydrous 1,4-dioxane; the mixture was subjected to nitrogen replacement three times; and under the protection of nitrogen, the temperature was increased to 100°C to allow for reaction for 3 hours. The reaction mixture was cooled to room temperature, and then concentrated under reduced pressure to obtain residues, which were purified by column chromatography (eluent: dichloromethane/methanol, gradient: 3%-5% methanol) to obtain the title compound (white solid, 360 mg, yield: 70.4%). LC/MS (ESI) m/z: 278.0 $[M+H]^+$.

**Step 2: 6-morpholinobenzo[d]thiazol-2-amine**

The resulting N-(6-morpholinylbenzothiazol-2-yl)acetamide (360 mg, 1.3 mmol) was dissolved in 3 mL of water and 9 mL of methanol; sodium hydroxide (519 mg, 13.0 mmol) was added; the temperature was increased to allow for reaction at 90°C for 3 hours; and the reaction mixture was concentrated under reduced pressure. The reaction mixture was concentrated under reduced pressure to obtain residues, which were purified by column chromatography (eluent: dichloromethane/methanol, gradient: 3%-5% methanol) to obtain the title compound (white solid, 240 mg, yield: 78.5%). LC/MS (ESI) m/z: 236.0 $[M+H]^+$.

**Step 3: 5-(2, 7-diazaspiro[3.5]nonadien-2-yl)-1, 3,4-thiadiazol-2-yl-2-chloro-5-methoxy-6-methyl-4,4' -bipyridin-3-carboxamide**

6-morpholinobenzo[d]thiazol-2-amine (240 mg, 1.02 mmol) and 2-chloro-5-methoxy-6-methyl-4, 4-bipyridin-3-carboxylic acid (284 mg, 1.02 mmol) were dissolved in 10 mL of acetonitrile and 3 mL of DMF; N-methylimidazole (0.24 mL, 3.06 mmol) was added and stirred for 10 minutes; and then, N,N,N',N'-tetramethylchlorformamidine hexafluorphosphate (286 mg, 1.02 mmol) was added, to allow for reaction at room temperature for 18 hours. Ethyl acetate and water were added to the reaction mixture for stratification; the organic phase was washed with saturated sodium chloride solution, dried over anhydrous sodium sulfate, filtered, and concentrated under reduced pressure; and the residue was purified by pre-HPLC (eluent: acetonitrile/0.1% formic acid aqueous solution, gradient: 25%-90%) to obtain the title compound (yellow solid, 175 mg, yield: 34.6%). $^1$HNMR (400MHz, DMSO-$d_6$) δ 12.74 (s, 1H), 8.82 (s, 1H), 8.16 (s, 1H), 7.62 (d, $J$ = 9.0 Hz, 1H), 7.56 (s, 1H), 7.47 (d, $J$ = 2.2 Hz, 1H), 7.44 (s, 1H), 7.15 (dd, $J$ = 9.0, 2.4 Hz, 1H), 3.78 - 3.75 (m, 4H), 3.60 (s, 3H), 3.15 - 3.12 (m, 4H), 2.60 (s, 3H). LC/MS (ESI) m/z: 496.1 $[M+H]^+$. The example compounds in Table 4 were synthesized using commercially available raw materials with reference to the synthesis steps of Example 45.

Table 4:

| Examples | Chemical structure | LC/MS(ESI) m/z [M+H]+ | 1H NMR |
|---|---|---|---|
| 46 | 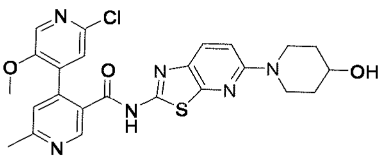 | 511.2 | $^1H$ NMR (400 MHz, DMSO-$d_6$) $\delta$ 12.67 (s, 1H), 8.81 (s, 1H), 8.16 (s, 1H), 7.84 (d, $J$ = 9.1 Hz, 1H), 7.56 (s, 1H), 7.44 (s, 1H), 6.99 (d, $J$= 9.2 Hz, 1H), 4.71 (d, $J$ = 4.1 Hz, 1H), 4.05 - 4.00 (m, 2H), 3.76 - 3.66 (m, 1H), 3.62 (s, 3H), 3.19 - 3.08 (m, 2H), 2.60 (s, 3H), 1.81 - 1.75 (m, 2H), 1.44 - 1.31 (m, 2H). |
| 47 | 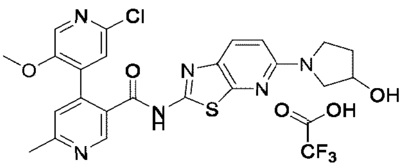 | 497.1 | $^1H$ NMR (400 MHz, DMSO-$d_6$) $\delta$ 12.66 (s, 1H), 8.83 (s, 1H), 8.17 (s, 1H), 7.85 (d, $J$ = 8.9 Hz, 1H), 7.57 (s, 1H), 7.48 (s, 1H), 6.60 (d, $J$= 9.0 Hz, 1H), 4.43 - 4.39 (m, 1H), 3.62 (s, 3H), 3.57 - 3.42 (m, 3H), 3.37 - 3.33 (m, 1H), 2.61 (s, 3H), 2.08 - 1.98 (m, 1H), 1.94 - 1.90 (m, 1H). |
| 48 | 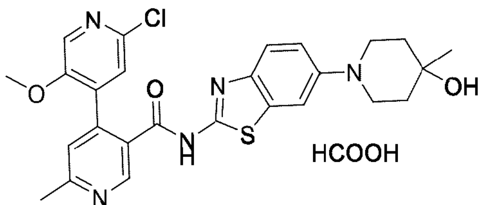 HCOOH | 524.2 | $^1H$ NMR (400 MHz, DMSO-$d_6$) $\delta$ 13.16 (s, 1H), 8.90 (s, 1H), 8.60 8.40 (m, 1H), 8.18 (s, 1H), 7.95 - 7.85 (m, 2H), 7.61 (s, 1H), 7.56 (s, 1H), 3.77 - 3.65 (m, 2H), 3.59 (s, 3H), 3.49 - 3.43 (m, 2H), 2.64 (s, 3H), 2.30 - 2.10 (m, 2H), 1.85 - 1.70 (m, 2H), 1.26 (s, 3H). |
| 49 | 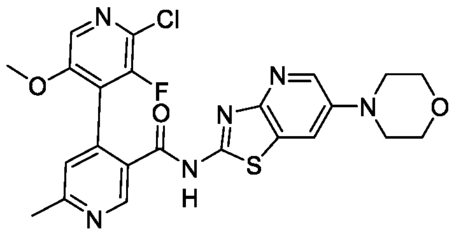 | 515.1 | $^1H$ NMR (400 MHz, DMSO-$d_6$) $\delta$ 9.15 (s, 1H), 8.28 (s, 1H), 8.21 (s, 1H), 8.15 (s, 1H), 7.76 (s, 1H), 7.32 (s, 1H), 3.79 - 3.75 (m, 4H), 3.73 (s, 3H), 3.16 - 3.10 (m, 4H), 2.57 (s, 3H). |
| 50 | 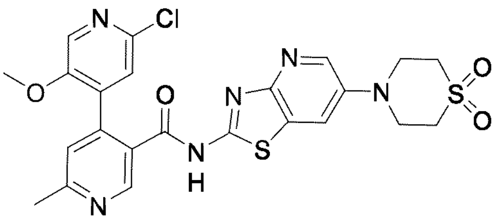 | 545.1 | $^1H$ NMR (400 MHz, DMSO-$d_6$) $\delta$ 13.02 (s, 1H), 8.85 (s, 1H), 8.43 (d, $J$ = 2.7 Hz, 1H), 8.16 (s, 1H), 8.09 (d, $J$ = 2.7 Hz, 1H), 7.58 (s, 1H), 7.46 (s, 1H), 3.85 - 3.80 (m, 4H), 3.60 (s, 3H), 3.24 - 3.18 (m, 4H), 2.60 (s, 3H). |
| 51 | 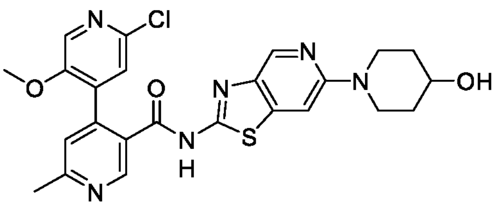 | 511.2 | $^1H$ NMR (400 MHz, DMSO-$d_6$) $\delta$ 12.83 (s, 1H), 8.83 (s, 1H), 8.59 (s, 1H), 8.17 (s, 1H), 7.57 (s, 1H), 7.46 (s, 1H), 7.39 (s, 1H), 4.80 - 4.50 (m, 1H), 4.02 - 3.99 (m, 2H), 3.73 - 3.66 (m, 1H), 3.61 (s, 3H), 3.09 (t, $J$ = 11.2 Hz, 2H), 2.60 (s, 3H), 1.81 - 1.78 (m, 2H), 1.43 - 1.35 (m, 2H). |

(continued)

| Exam ples | Chemical structure | LC/MS(ESI) m/z [M+H]+ | 1H NMR |
|---|---|---|---|
| 52 | 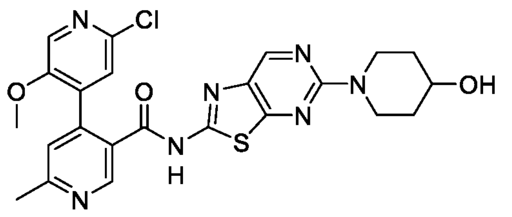 | 512.2 | $^1$H NMR (400 MHz, DMSO-$d_6$) δ 12.87 (s, 1H), 8.82 (s, 1H), 8.75 (s, 1H), 8.17 (s, 1H), 7.57 (s, 1H), 7.46 (s, 1H), 4.75 - 4.70 (m, 1H), 4.33 - 4.24 (m, 2H), 3.80 - 3.70 (m, 1H), 3.62 (s, 3H), 3.37 - 3.33 (m, 1H), 3.30 - 3.27 (m, 1H), 2.60 (s, 3H), 1.84 - 1.75 (m, 2H), 1.41 - 1.30 (m, 2H). |
| 53 | 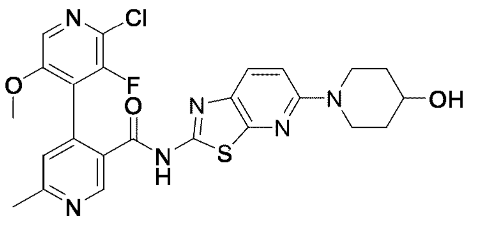 | 529.1 | $^1$H NMR (400 MHz, DMSO-$d_6$) δ 12.81 (s, 1H), 8.98 (s, 1H), 8.18 (s, 1H), 7.83 (d, *J* = 9.0 Hz, 1H), 7.48 (s, 1H), 6.98 (d, *J* = 9.1 Hz, 1H), 4.70 (d, *J* = 4.1 Hz, 1H), 4.05 - 3.98 (m, 2H), 3.73(s, 3H), 3.71 - 3.67 (m, 1H), 3.15 - 2.05 (m, 2H), 2.60 (s, 3H), 1.83 - 1.75 (m, 2H), 1.45 - 1.30 (m, 2H). |

**Example 54: 2'-chloro-3'-fluoro-5'-methoxy-6-methyl-N-(5-(tetrahydrofuran -3-carbonyl)-4,5,6,7-tet rahydrothiazol[5,4-c]pyridin-2-yl)-[4,4'-bipyridine]-3-carboxamide**

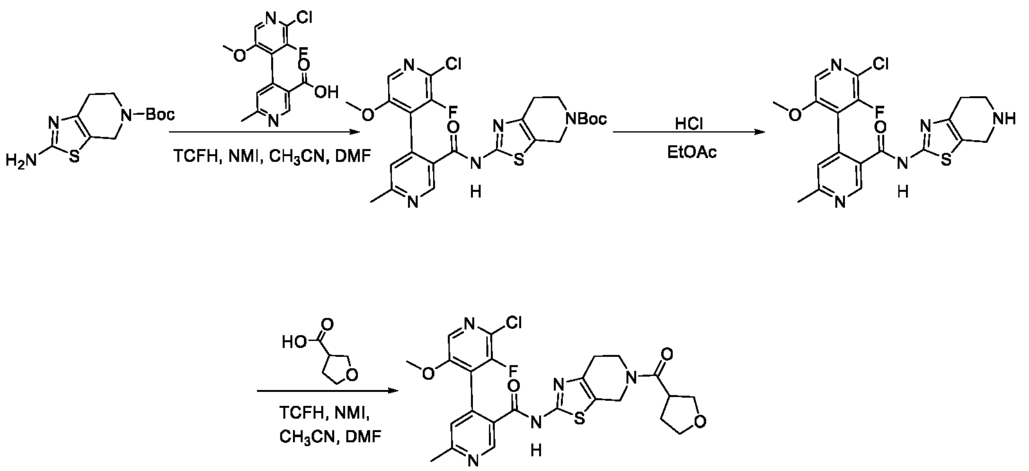


**Step 1: 2-(2'-chloro-3'-fluoro-5'-methoxy-6-methyl-[4,4'-bipyridine]-3-formamido)-6,7-dihydrothiazol [5,4-c]pyridin-5(4H)-tert-butyl carboxylate**

2'-chloro-3'-fluoro-5'-methoxy-6-methyl-[4,4'-bipyridine]-3-carboxylic acid (58 mg, 0.195 mmol), N-methylimidazole (48 mg, 0.585 mmol) and N,N,N',N'-tetramethylchlorformamidine hexafluorphosphate (66 mg, 0.235 mmol) were sequentially added to 2-amino-6,7-dihydrothiazol[5,4-c]pyridin-5(4H)-tert-butyl carboxylate (50 mg, 0.196 mmol) in the mixed solution of N,N-dimethylformamide (1 mL) and acetonitrile (1 mL). Then, the mixture was stirred at room temperature for 16 hours. After complete reaction, water was added; the resulting mixture was extracted with ethyl acetate; the organic phase was washed with saturated saline, dried over anhydrous sodium sulfate, and concentrated under reduced pressure. The residue was purified by column chromatography (eluent: petroleum ether/ethyl acetate, gradient: 0-100% ethyl acetate) to obtain the title compound (faint yellow oil, 20 mg, yield: 19.1%). LC/MS (ESI) m/z: 534.2 $[M+H]^+$.

**Step 2: 2'-chloro-3'-fluoro-5'-methoxy-6-methyl-N-(4,5,6,7-tetrahydrothiazol[5,4-c]pyridin-2-yl)-[4,4' -bipyridine]-3-carboxamide**

4M hydrogen ethyl acetate solution (1 mL) was added to the solution of 2-(2'-chloro-3'-fluoro-5'-methoxy-6-methyl-[4,4'-bipyridine]-3-formamido)-6,7-dihydrothiazol[5,4-c]pyridin -5(4H)-tert-butyl carboxylate (20 mg, 0.037 mmol) in ethyl acetate (1 mL). The reaction mixture was stirred at room temperature to react for 1 hour. After complete reaction, the reaction mixture was concentrated under reduced pressure to obtain the title compound (faint yellow oil, 16 mg, yield: 100.0%). This product was directly used in the next step of reaction without purification. LC/MS (ESI) m/z: 434.1 $[M+H]^+$.

**Step 3: 2'-chloro-3'-fluoro-5'-methoxy-6-methyl-N-(5-(tetrahydrofuran -3-carbonyl)-4,5,6,7-tetrahyd rothiazol [5,4-c]pyridin-2-yl)-[4,4'-bipyridine]-3-carboxamide**

3-tetrahydrofuroic acid (6 mg, 0.055 mmol), N-methylimidazole (9 mg, 0.111 mmol) and N,N,N',N'-tetramethylchlorformamidine hexafluorphosphate (12 mg, 0.044 mmol) were sequentially added to

2'-chloro-3'-fluoro-5'-methoxy-6-methyl-N-(4,5,6,7-tetrahydrothiazol[5,4-c]pyridin-2-yl)-[4,4'-bipyridine]-3-carboxamide (16 mg, 0.037 mmol) in the mixed solution of N,N-dimethylformamide (1 mL) and acetonitrile (1 mL). Then, the mixture was stirred at room temperature for 16 hours. After complete reaction, water was added; the resulting mixture was extracted with ethyl acetate; the organic phase was washed with saturated saline, dried over anhydrous sodium sulfate, and concentrated under reduced pressure. The residue was purified by pre-HPLC to obtain the title compound (white solid, 4.4 mg, yield: 23.9%). $^1H$ NMR (400 MHz, DMSO-$d_6$) $\delta$ 12.74 (s, 1H), 8.95 (s, 1H), 8.17 (s, 1H), 7.45 (s, 1H), 4.76 - 4.52 (m, 2H), 3.94 - 3.78 (m, 3H), 3.74 - 3.64 (m, 6H), 3.52 - 3.42 (m, 1H), 2.76 - 2.72 (m, 1H), 2.69 - 2.61 (m, 1H), 2.59 (s, 3H), 2.11 - 1.92 (m, 2H). LC/MS (ESI) m/z: 532.2 $[M+H]^+$.

**Example 55: 7-(2-chloro-5-methoxypyridin-4-yl)-N-(5-(4-hydroxycyclohex-1-en-1-yl)thiazol[5,4-b]pyr idin-2-yl)-3-methylimidazol[1,5-a]pyridin-6-carboxamide**

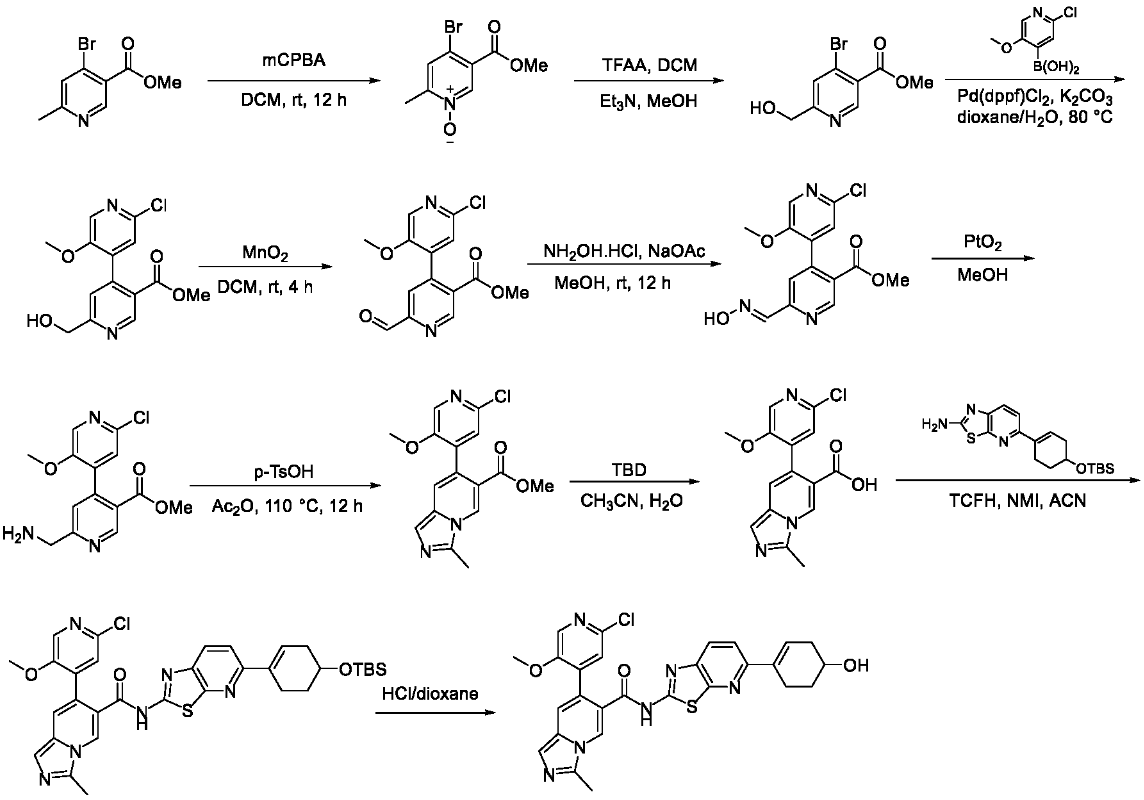


**Step 1: 4-bromo-5-(methoxycarbonyl)-2-methylpyridin-1-oxide**

At 0°C, 3-chlorophenyl peroxybenzoic acid (2.65 g, 13.0 mmol) was added to the solution of 4-bromo-6-methylpyridin-3-methyl carboxylate (2.00 g, 8.69 mmol) in dichloromethane (30 mL). Then, the reaction mixture was stirred at 50°C for 2 hours. After complete reaction, the reaction mixture was cooled to room temperature. The mixture was filtered through diatomite, and washed with saturated sodium bicarbonate in water(100 mL×2) and saline (100 mL). The organic phases were combined, dried over anhydrous sodium sulfate, and concentrated on a rotary evaporator. The residue was purified by column chromatography (eluent: petroleum ether/ethyl acetate, gradient: 0-80% ethyl acetate) to obtain the title compound (white solid, 1.60 g, yield: 74.8%). $^1$H NMR (400 MHz, $CDCl_3$) δ 8.70 (s, 1H), 7.57 (s, 1H), 3.96 (s, 3H), 2.53 (s, 3H).

**Step 2: 4-bromo-6-(hydroxymethyl)methyl nicotinate**

At 0°C, trifluoroacetic anhydride (3.41 g, 16.3 mmol) was added to the solution of 4-bromo-5-(methoxycarbonyl)-2-methylpyridine1-oxide (1.6 g, 6.50 mmol) in dichloromethane (30 mL). Under a refluxing condition, the mixture was stirred. After the reaction was completed under TLC monitoring (PE:EA=1:1), the resulting solution was concentrated on a rotary evaporator. The residue was dissolved in methanol (30 mL), into which triethylamine was added dropwise till the solution was neutral; and the solution was stirred at room temperature for 4 hours. Methanol, methyl trifluoroacetate, trifluoroacetic acid and triethylamine are each removed by means of rotary evaporation. The high-speed chromatography (eluent: petroleum ether/ethyl acetate, gradient: 0-50% ethyl acetate) was performed to obtain the title compound (yellow solid, 1.35 g, 84.4%). $^1$H NMR (400 MHz, $CDCl_3$) δ 8.95 (s, 1H), 7.66 (s, 1H), 4.80 (s, 2H), 3.98 (s, 3H), 3.18 (s, 1H).

**Step 3: 2'-chloro-6-(hydroxymethyl)-5'-methoxy-[4,4'-bipyridine]-3-methyl carboxylate**

Potassium carbonate (2.27 g, 16.5 mmol) and Pd(dppf)$Cl_2$ (0.20 g, 0.274 mmol) were added to the solution obtained by dissolving 4-bromo-6-(hydroxymethyl)pyridin-3-carboxylmethyl formate (1.35 g, 5.49 mmol) and (2-chloro-5-methoxypyridin-4-yl)boranediol (1.08 g, 5.76 mmol) in dioxane/water (24 mL) (V:V = 5:1); and the reaction mixture was stirred at 80°C for 1 hour under the protection of nitrogen. After the reaction was completed, the reaction mixture was cooled to room temperature and concentrated in vacuum, to remove most of the solvent. The residue was poured into water (10 mL), and extracted with ethyl acetate (20 mL). The organic layer was dried over anhydrous sodium sulfate, filtered and concentrated. The high-speed chromatography (eluent: petroleum ether/ethyl acetate, gradient: 0-40% ethyl acetate) to obtain the title compound (yellow solid, 1.50 g, 88.6%). $^1$H NMR (400 MHz, $CDCl_3$) δ 9.11 (s, 1H), 8.05 (s, 1H), 7.22 (s, 1H), 7.19 (s, 1H), 4.87 (s, 2H), 3.81 (s, 3H), 3.77 (s, 3H), 3.42 (s, 1H).

**Step 4: 2'-chloro-6-formyl-5'-methoxy-[4,4'-bipyridine]-3-methyl carboxylate**

At room temperature, active manganese dioxide (0.841 mL, 48.6 mmol) was added to the solution of 4-(2-chloro-5-methoxypyridin-4-yl)-6-(hydroxymethyl)pyridin-3-methyl carboxylate (1.5 g, 4.859 mmol) in dichloromethane (30 mL), and stirred at 50°C for 1 hour. After the reaction was completed, the reaction mixture was filtered and the filtrate was concentrated. The high-speed chromatography (eluent: petroleum ether/ethyl acetate, gradient: 0-30% ethyl acetate) to obtain the title compound (white solid, 1.20 g, 3.91 mmol, 80.5%). $^1$H NMR (400 MHz, $CDCl_3$) δ 10.16 (s, 1H), 9.27 (s, 1H), 8.08 (s, 1H), 7.87 (s, 1H), 7.23 (s, 1H), 3.83 (s, 3H), 3.81 (s, 3H).

**Step 5: methyl(E)-2'-chloro-6-((hydroxyimino)methyl)-5'-methoxy-[4,4'-bipyridine]-3-carboxylate**

At room temperature, sodium acetate (0.960 g, 11.7 mmol) was dissolved in methanol (20 mL); hydroxylamine hydrochloride (0.820 g, 11.7 mmol) was added; and the resulting mixture was stirred for 0.5 hours. The produced precipitates were removed through filtering; and the filtrate was dropped into the solution of 4-bromo-6-formylpyridin-3-methyl carboxylate (135 mg, 0.553 mmol) in methanol (20 mL). Then, the reaction mixture was stirred at room temperature for 1 hour, concentrated and dissolved in ethyl acetate (60 mL). Then, the mixture was washed with a saturated sodium bicarbonate solution, dried over anhydrous sodium sulfate, filtered and concentrated, to obtain the title compound (white solid, 1.10 g, 87.4%). $^1$H NMR (400 MHz, $CDCl_3$) δ 9.14 (s, 2H), 8.35 (s, 1H), 8.05 (s, 1H), 7.75 (s, 1H), 7.23 (s, 1H), 3.81 (s, 3H), 3.77 (s, 3H).

**Step 6: 6-(aminomethyl)-2-chloro-5'-methoxy-[4,4'-bipyridine]-3-methyl carboxylate**

At room temperature, platinum dioxide (0.021 mL, 0.932 mmol) was added to the solution obtained by dissolving 4-(2-chloro-5-methoxypyridin-4-yl)-6-[(E)-(hydroxyimino)methyl]pyridin-3-methyl carboxylate (1 g, 3.108 mmol) in methanol (30 mL). Then, the mixture was subjected to replacement with a hydrogen balloon at room temperature three times; and stirred in the presence of hydrogen for 4 hours. After the reaction was completed, the reaction mixture was filtered and the filtrate was concentrated to obtain the title compound (yellow solid, 950 mg, 99.32%). LC/MS (ESI) m/z: 308

[M+H]+.

**Step 7: 7-(2-chloro-5-methoxypyridin-4-yl)-3-methylimidazo[1,5-a]pyridin-6-methyl carboxylate**

At room temperature, 4-methylbenzenesulfonic acid (0.497 mL, 3.087 mmol) was added to the solution of 6-(aminomethyl)-4-(2-chloro-5-methoxypyridin-4-yl)pyridin-3-methyl carboxylate (0.95 g, 3.09 mmol) in acetic anhydride (20 mL). Then, the reaction mixture was stirred at 110°C for 12 hours. After the reaction was completed, the reaction mixture was concentrated. The high-speed chromatography (eluent: petroleum ether/ethyl acetate, gradient: 0-60% ethyl acetate) was performed to obtain the title compound (yellow solid, 680 mg, 66.4%).$^1$H NMR (400 MHz, $CDCl_3$) δ 8.42 (s, 1H), 7.98 (s, 1H), 7.46 (s, 1H), 7.34 (s, 1H), 7.28 (s, 1H), 3.81 (s, 3H), 3.74 (s, 3H), 2.76 (s, 3H).

**Step 8: 7-(2-chloro-5-methoxypyridin-4-yl)-3-methylimidazo[1,5-a]pyridin-6-carboxylic acid**

At room temperature, 1,3,4,6,7,8-hexahydro-2H-pyrimidin[1,2-a]pyrimidine (235 mg, 1.69 mmol) was added to the solution of 7-(2-chloro-5-methoxypyridin-4-yl)-3-methylimidazo[1,5-a]pyridin-6-methyl carboxylate (280 mg, 0.844 mmol) in acetonitrile (9 mL) and water (3 mL). Then, the mixture was stirred at 50°C for 1 hour. After the reaction was completed, the reaction mixture was concentrated. The high-speed chromatography (eluent: dichloromethane/ethyl acetate (with 0.3% acetic acid added into ethyl acetate), gradient: 0-80% ethyl acetate) to obtain the title compound (yellow solid, 160 mg, 59.7%). $^1$H NMR (400 MHz, DMSO-$d_6$) δ 12.49 (m, 1H), 8.61 (s, 1H), 8.18 (s, 1H), 7.63 (s, 1H), 7.52 (s, 1H), 7.45 (s, 1H), 3.83 (s, 3H), 2.74 (s, 3H).

**Step 9: N-(5-(4-((tert-butyldimethylsilyl)oxy)cyclohex-1-en-1-yl)thiazol[5,4-b]pyridin-2-yl)-7-(2-chlor o-5-methoxypyridin-4-yl)-3-methylimidazol[1,5-a]pyridin-6-carboxamide**

7-(2-chloro-5-methoxypyridin-4-yl)-3-methylimidazo[1,5-a]pyridin-6-carboxylic acid (80 mg, 0.252 mmol), 5-(4-((tert-butyldimethylsilyl)oxy)cyclohex-1-en-1-yl)thiazol[5,4-b]pyridin-2-amine (91 mg, 0.252 mmol) and N-methylimidazole (62.1 mg, 0.756 mmol) were dissolved in acetonitrile (5 mL), stirred at 40°C for 5 minutes;; and finally N,N,N',N'-tetramethylchlorformamidine hexafluorphosphate (77.7 mg, 0.277 mmol) dissolved in acetonitrile (2 mL) was added. Under the protection of nitrogen, the mixture was stirred at 40°C to react for 1 hour. After complete reaction, water was added; and the resulting mixture was extracted with ethyl acetate, washed with saturated saline, dried over anhydrous sodium sulfate, and concentrated under reduced pressure. The residue was purified by column chromatography (eluent: dichloromethane/methanol, gradient: 0%-8% methanol) to obtain the title compound (white powder, 56 mg, yield: 33.6%). LC/MS (ESI) m/z: 661.3 [M+H]+.

**Step 10: 7-(2-chloro-5-methoxypyridin-4-yl)-N-(5-(4-hydroxycyclohex-1-en-1-yl)thiazol[5,4-b]pyridin -2-yl)-3-methylimidazol[1,5-a]pyridin-6-carboxamide**

N-(5-(4-((tert-butyldimethylsilyl)oxy)cyclohex-1-en-1-yl)thiazol[5,4-b]pyridin-2-yl)-7-(2-chloro-5-methoxy pyridin-4-yl)-3-methylimidazol[1,5-a]pyridin-6-carboxamide (40 mg, 0.061 mmol) was dissolved in 1,4-dioxane (2 mL), and 4M 1,4-dioxane hydrochloride solution (2 mL) was slowly added to react for 1 hour at room temperature; then; the reaction mixture was concentrated under reduced pressure; the residue was added to a saturated sodium bicarbonate solution, extracted with ethyl acetate, washed with saturated saline, dried over anhydrous sodium sulfate, and concentrated under reduced pressure; and the residue was treated by pre-HPLC and then freeze-dried to obtain the target compound (white powder, 12 mg, yield: 36.3%) $^1$H NMR (400 MHz, DMSO-$d_6$) δ 8.74 (s, 1H), 8.07 (s, 1H), 7.99 (d, $J$ = 8.3 Hz, 1H), 7.68 (s, 1H), 7.62 (d, $J$ = 8.5 Hz, 1H), 7.55 (s, 1H), 7.44 (s, 1H), 6.62 - 6.57 (m, 1H), 4.74 - 4.66 (m, 1H), 3.85 - 3.78 (m, 1H), 3.60 (s, 3H), 2.71 (s, 3H), 2.71 - 2.63 (m, 1H), 2.55 - 2.51 (m, 1H), 2.48 - 2.42 (m, 1H), 2.17 - 2.05 (m,1H), 1.94 - 1.86 (m, 1H), 1.65 - 1.55 (m, 1H). LC/MS (ESI) m/z: 547.2 [M+H]+.

**Example 56: 2'-chloro-N-(5-(4-hydroxycyclohex-1-en-1-yl)-3H-imidazo[4,5-b]pyridin-2-yl)-5'-methox y-6-methyl-[4,4'-bipyridine]-3-carboxamide**

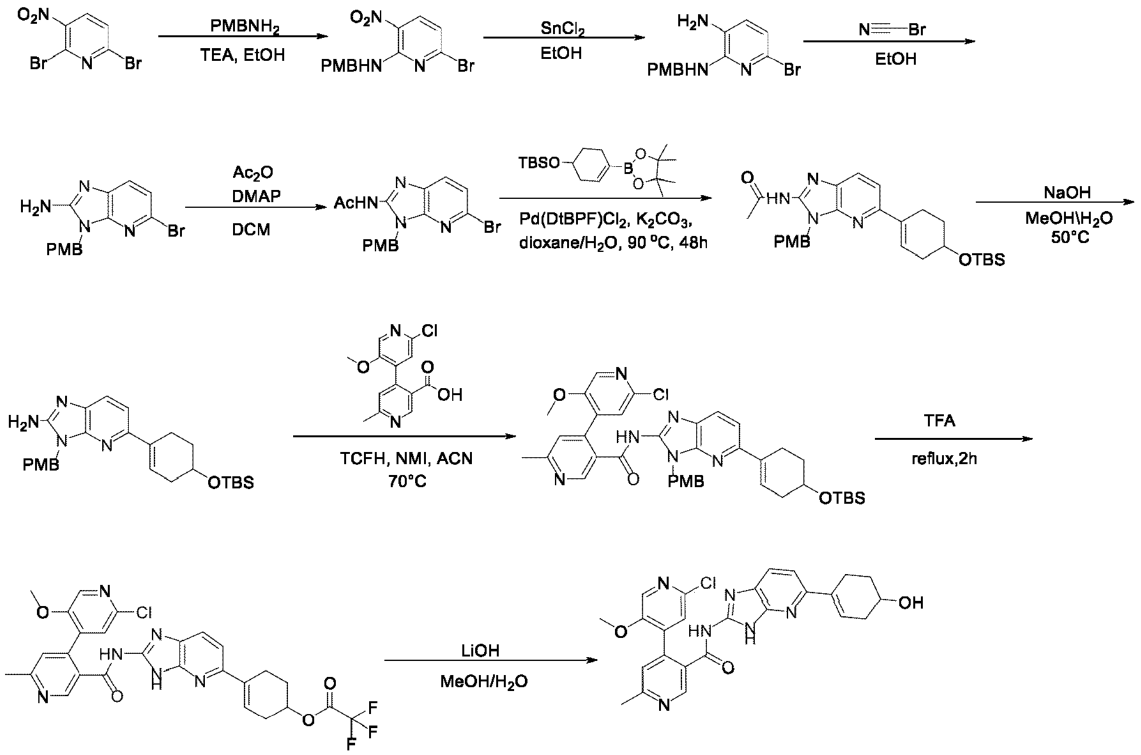


**Step 1: 6-bromo-N-(4-methoxybenzyl)-3-nitropyridin-2-amine**

2,6-dibromo-3-nitropyridine (1.0 g, 3.55 mmol) was dissolved in ethanol (15 mL); triethylamine (0.718 g, 7.09 mmol) was added, then p-methoxy benzylamine (0.487 g, 3.55 mmol) was added, and the resulting mixture was allowed to react at room temperature for 18 hours. After complete reaction, the reaction mixture was concentrated under reduced pressure; and the solid was washed with ethyl acetate to obtain the title compound (yellow solid, 0.58 g, yield: 48.3%). LC/MS (ESI) m/z: 338 $[M+H]^+$.

**Step 2: 6-bromo-$N^2$-(4-methoxybenzyl)pyridin-2,3-diamine**

6-bromo-N-(4-methoxybenzyl)-3-nitropyridin-2-amine (4.8 g, 14.19 mmol) was dissolved in ethanol (50 mL); stannous chloride dihydrate (16.0 g, 70.95 mmol) was added; and the mixture was allowed to react at 50°C for 3 hours. After complete reaction, the reaction mixture was cooled to room temperature; after 10% sodium bicarbonate solution was added, a large amount of white solids appeared; after no more solids appeared, the solids were filtered, and washed with ethanol; the filtrate was concentrated under reduced pressure, filtered by adding diatomite, and washed with ethanol; the filtrate was concentrated under reduced pressure again, to obtain the title compound (dark yellow solid, 1.2 g, yield: 27.5%). LC/MS (ESI) m/z: 308 $[M+H]^+$.

**Step 3: 5-bromo-3-(4-methoxybenzyl)-3H-imidazo[4,5-b]pyridin-2-amine**

6-bromo-$N^2$-(4-methoxybenzyl)pyridin-2,3-diamine (1.2 g, 3.89 mmol) was dissolved in ethanol (20 mL); nitrile bromide dichloromethane solution (0.493 g, 4.65 mmol) was added; and the mixture was allowed to react at room temperature overnight. After complete reaction, the reaction mixture was adjusted to alkalinity with the methanol solution of ammonia, then concentrated under reduced pressure, purified by silica-gel column chromatography (eluent: dichloromethane/methanol =5/1, V/V) to obtain the title compound (faint yellow solid, 720 mg, yield: 55.4%). LC/MS (ESI) m/z: 333 $[M+H]^+$.

**Step 4: N-(5-bromo-3-(4-methoxybenzyl)-3H-imidazo[4,5-b]pyridin-2-yl)acetamide**

5-bromo-3-(4-methoxybenzyl)-3H-imidazo[4,5-b]pyridin-2-amine (0.72 g, 2.16 mmol), 4-dimethylaminopyridine (290 mg, 2.38 mmol), acetic anhydride (243 mg, 2.38 mmol) and 20 mL of dichloromethane were added into a 100 mL reaction flask to react at room temperature for 18 hours, and concentrated under reduced pressure; the residue was purified by silica-gel column chromatography (eluent: dichloromethane/methanol = 30/1, V/V) to obtain N-(5-bromo-3-(4-methoxybenzyl)-3H-imidazo[4,5-b]pyridin-2-yl)acetamide (600 mg, yellow solid, yield: 74.0%). LC/MS (ESI) m/z: 375 $[M+H]^+$.

**Step 5: N-(5-(4-(tert-butyldimethylsilyl)oxy)cyclohex-1-en-1-yl)-3-(4-methoxybenzyl)-3H-imidazo[4,5-b]pyridin-2-yl)acetamide**

N-(5-bromo-3-(4-methoxybenzyl)-3H-imidazo[4,5-b]pyridin-2-yl)acetamide (600 mg, 1.60 mmol) and tert-butyldimethyl((4-(4,4,5,5-tetramethyl-1,3,2-dioxaborinan-2-yl)cyclohex-3-en-1-yl)oxy)silane (812.1 mg, 2.40 mmol) were dissolved in 27 mL of 1,4-dioxane and 9 mL of water; potassium carbonate (663.4 mg, 4.80 mmol) and 1,1-bis(di-tert-butylphosphine)ferrocene palladium dichloride (51.7 mg, 0.08 mmol) were added; and under the protection of nitrogen, the temperature was increased to 90°C to allow for reaction for 3 hours. The reaction mixture was cooled to room temperature, underwent rotary evaporation to remove 1,4-dioxane, added with water, and extracted with ethyl acetate; the organic phase was washed with water and saturated saline; and the organic phase was concentrated under reduced pressure to obtain the crude product N-(5-(4-(tert-butyldimethylsilyl)oxy)cyclohex-1-en-1-yl)-3-(4-methoxybenzyl)-3H-imidazo[4,5-b]pyridin-2-yl)acetamide (1.0 g, yellow solid), which was directly used in the next step. LC/MS (ESI) m/z: 507 $[M+H]^+$.

**Step 6: 5-(4-((tert-butyldimethylsilyl)oxy)cyclohex-1-en-1-yl)-3-(4-methoxybenzyl)-3H-imidazo[4,5-b] pyridin-2-amine**

The crude product N-(5-(4-(tert-butyldimethylsilyl)oxy)cyclohex-1-en-1-yl)-3-(4-methoxybenzyl)-3H-imidazo [4,5-b]pyridin-2-yl)acetamide (1.0 g) and sodium hydroxide (400 mg, 10 mmol) were dissolved in 15 mL of methanol and 5 mL of water, to allow for reaction at 50°C for 3 hours. The reaction mixture was concentrated under reduced pressure; and the residue was purified by silica-gel column chromatography (eluent: dichloromethane/methanol = 20/1, *V*/*V*) to obtain crude product 5-(4-((tert-butyldimethylsilyl)oxy)cyclohex-1-en-1-yl)-3-(4-methoxybenzyl)-3H-imidazo[4,5-b]pyridin-2-a mine (600 mg, yellow solid). LC/MS (ESI) m/z: 465 $[M+H]^+$.

**Step 7: N-(5-(4-(tert-butyldimethylsilyl)oxy)cyclohex-1-en-1-yl)-3-(4-methoxybenzyl)-3H-imidazo[4,5-b]pyridin-2-yl)-2'-chloro-5'-methoxy-6-methyl-[4,4'-bipyridine]-3-carboxamide**

5-(4-((tert-butyldimethylsilyl)oxy)cyclohex-1-en-1-yl)-3-(4-methoxybenzyl)-3H-imidazo[4,5-b]pyridin-2-a mine (200 mg, 0.430 mmol), 2'-chloro-5'-methoxy-6-methyl-[4,4'-bipyridine]-3-carboxylic acid (179.8 mg, 0.645 mmol), and methylimidazole (105.8 mg, 1.29 mmol) were dissolved in 10 mL of acetonitrile, and stirred at 70°C for 10 minutes; and then, N,N,N',N'-tetramethylchlorformamidine hexafluorphosphate (361.9 mg, 1.29 mmol) was added, to allow for reaction at 70°C for 18 hours. The reaction mixture was concentrated under reduced pressure, added with water, extracted with ethyl acetate; and the residue was purified by silica-gel column chromatography (eluent: petroleum ether/ethyl acetate = 3/1, *V*/*V*) to obtain N-(5-(4-(tert-butyldimethylsilyl)oxy)cyclohex-1-en-1-yl)-3-(4-methoxybenzyl)-3H-imidazo[4,5-b]pyridin-2-yl)-2'-chloro-5'-methoxy-6-methyl-[4,4'-bipyridine]-3-carboxamide (200 mg, yellow solid, yield: 64.1%). LC/MS (ESI) m/z: 725$[M+H]^+$.

**Step 8: 4-(2-(2'-chloro-5'-methoxy-6-methyl-[4,4'-bipyridine]-3-carboxamide)-3H-imidazo[4,5-b]pyri din-5-yl)cyclohex-3-en-1-yl2,2,2-trifluoroacetate**

N-(5-(4-(tert-butyldimethylsilyl)oxy)cyclohex-1-en-1-yl)-3-(4-methoxybenzyl)-3H-imidazo[4,5-b]pyridin-2-yl)-2'-chloro-5'-methoxy-6-methyl-[4,4'-bipyridine]-3-carboxamide (150 mg, 0.207 mmol) was dissolved in 5 mL of trifluoroacetic acid, to allow for reaction at 75°C for 2 hours. The reaction mixture was concentrated under reduced pressure, added with dichloromethane, and concentrated, and this process was repeated three times to obtain a crude product 4-(2-(2'-chloro-5'-methoxy-6-methyl-[4,4'-bipyridine]-3-carboxamide)-3H-imidazo[4,5-b]pyridin-5-yl)cyclo hex-3-en-1-yl2,2,2-trifluoroacetate (121.5 mg, yellow solid, yield: 100%). LC/MS (ESI) m/z: 587 $[M+H]^+$.

**Step 9: 2'-chloro-N-(5-(4-hydroxycyclohex-1-en-1-yl)-3H-imidazo[4,5-b]pyridin-2-yl)-5'-methoxy-6-m ethyl-[4,4'-bipyridine]-3-carboxamide**

The crude product 4-(2-(2'-chloro-5'-methoxy-6-methyl-[4,4'-bipyridine]-3-carboxamide)-3H-imidazo[4,5-b] pyridin-5-yl)cyclo hex-3-en-1-yl2,2,2-trifluoroacetate (121.5 mg, 0.207 mmol) was dissolved in 3 mL of methanol and 1 mL of lithium hydroxide (49.6 mg, 2.07 mmol) in water, to allow for reaction at room temperature for 10 minutes. The reaction mixture was added with 5 mL of water, extracted with ethyl acetate for stratification, and concentrated under reduced pressure to obtain residues, which were purified by prep-HPLC (eluent: acetonitrile/0.1% formic acid aqueous solution = 0%-70%, V/V) to obtain 2'-chloro-N-(5-(4-hydroxycyclohex-1-en-1-yl)-3H-imidazo[4,5-b]pyridin-2-yl)-5'-methoxy-6-methyl-[4,4'-bi pyridine]-3-carboxamide (63.9 mg, grey-white solid, yield: 62.9%). $^1$H NMR (400 MHz, DMSO-$d_6$) δ 12.50 - 12.05 (m, 2H), 8.90 (s, 1H), 8.17 (s, 1H), 7.66 (d, *J* = 8.3 Hz, 1H), 7.53 (s, 1H), 7.39 (s, 1H), 7.29 (d, *J* = 8.3 Hz, 1H), 6.50 - 6.44 (m, 1H), 4.70 - 4.63 (m, 1H), 3.85 - 3.75 (m, 1H), 3.65 (s, 3H), 2.76 - 2.65 (m, 1H), 2.59 (s, 3H), 2.49 - 2.41 (m, 2H), 2.15 - 2.05 (m, 1H), 1.95 - 1.85 (m, 1H), 1.68 - 1.51 (m, 1H). LC/MS (ESI) m/z: 491.2 [M+H]$^+$.

**Example 57: N-(6-(4-acetaminopiperidin-1-yl)thiazolo[4,5-b]pyridin-2-yl)-2'-chloro-5'-methoxy-6-me thyl-[4,4'-bipyridine]-3-carboxamide**

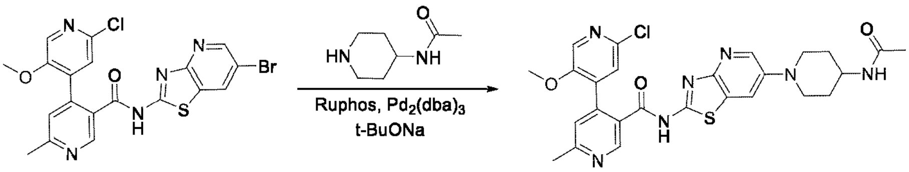


**Step 1: *N*-(6-(4-acetaminopiperidin-1-yl)thiazolo[4,5-b]pyridin-2-yl)-2'-chloro-5'-methoxy-6-methyl-[4,4'-bipyridine]-3-carboxamide**

Under the protection of nitrogen, *N*-(6-bromothiazolo[4,5-b]pyridin-2-yl)-2'-chloro-5'-methoxy-6-methyl-[4,4'-bipyridine]-3-carboxamide (120 mg, 0.245 mmol), N-(piperidin-4-yl)acetamide (34.8 mg, 0.245 mmol), sodium tert-butoxide (66 mg, 0.735 mmol), 2-dicyclohexylphosphino-2 ',6'-diisopropoxybiphenyl (69 mg, 0.15 mmol), and tris(dibenzylideneacetone)dipalladium (137 mg, 0.15 mmol) were dissolved in a 1,4-dioxane (8 mL). The reaction was allowed to stirred at 100°C for 2 hours under the protection of nitrogen. After complete reaction, water was added; the resulting mixture was extracted with ethyl acetate; and the organic phase was washed with saturated saline, dried over anhydrous sodium sulfate, and concentrated under reduced pressure. The resulting residue was purified by column chromatography (eluent: dichloromethane, methanol, gradient: 0%-8% methanol) to obtain the crude title compound, which was treated by pre-HPLC and then freeze-dried to obtain the target compound (yellow solid, 15 mg, yield: 24%). $^1$H NMR (400 MHz, DMSO-$d_6$) δ 12.97 (s, 1H), 8.85 (s, 1H), 8.35 (s, 1H), 8.16 (s, 1H), 7.96 (s, 1H), 7.84 (d, J = 7.3 Hz, 1H), 7.57 (s, 1H), 7.45 (s, 1H), 3.77-3.73 (m, 1H), 3.73-3.65(m, 2H),3.61 (s, 3H), 2.87 (t, J = 11.3 Hz, 2H), 2.60 (s, 3H), 1.88-1.84 (m, 2H), 1.80 (s, 3H), 1.55-1.45 (m, 2H). LC/MS (ESI) m/z: 552.2 [M+H]$^+$.

The example compounds in Table 5 were synthesized using commercially available raw materials with reference to the synthesis steps of Example 57.

Table 5:

| Exam ples | Chemical structure | LC/MS(ESI) m/z [M+H]$^+$ | $^1$H NMR |
|---|---|---|---|
| 58 | 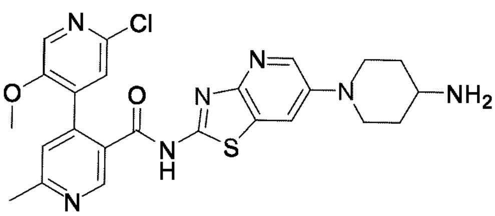 | 510.1 | $^1$H NMR (400 MHz, DMSO-$d_6$) δ 8.95 (s, 1H), 8.30 (s, 1H), 8.23 (s, 1H), 8.14 (s, 1H), 7.81 (s, 1H), 7.46 (s, 1H), 7.31 (s, 1H), 3.72 - 3.66 (m, 2H), 3.61 (s, 3H), 3.14 - 3.08 (m, 1H), 2.83 - 2.77 (m, 2H), 2.57 (s, 3H), 1.97 - 1.90 (m, 2H), 1.65 - 1.55 (m, 2H). |
| 59 | 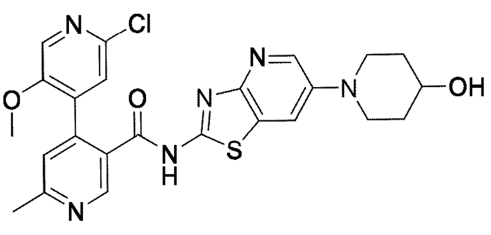 | 511.1 | $^1$H NMR (400 MHz, DMSO-$d_6$) δ 12.95 (s, 1H), 8.84 (s, 1H), 8.35 (s, 1H), 8.16 (s, 1H), 7.95 (s, 1H), 7.57 (s, 1H), 7.45 (s, 1H), 4.71 (d, J = 3.5 Hz, 1H), 3.69 - 3.64 (m, 1H), 3.60 (s, 3H), 3.58 - 3.54 (m, 2H), 2.93 (t, J = 10.8 Hz, 2H), 2.60 (s, 3H), 1.90 - 1.82 (m, 2H), 1.57 - 1.47 (m, 2H). |

(continued)

| Examples | Chemical structure | LC/MS(ESI) m/z [M+H]+ | $^1$H NMR |
|---|---|---|---|
| 60 | 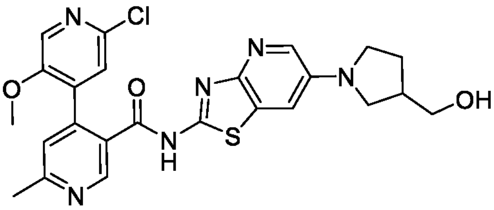 | 511.1 | $^1$H NMR (400 MHz, DMSO-d6) δ 8.86 (s, 1H), 8.16 (s, 1H), 7.94 (s, 1H), 7.54 (s, 1H), 7.46 (s, 1H), 7.41 (s, 1H), 4.77 - 4.71 (m, 1H), 3.61 (s, 3H), 3.48 - 3.38 (m, 4H), 3.13 - 3.06 (m, 2H), 2.59 (s, 3H), 2.10 - 2.04 (m, 2H), 1.80 - 1.75 (m, 1H). |
| 61 | 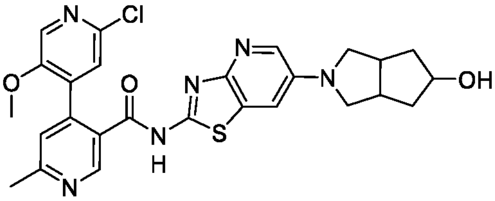 | 537.2 | $^1$H NMR (400 MHz, DMSO-$d_6$) δ 12.95 (s, 1H), 8.85 (s, 1H), 8.17 (s, 1H), 8.06 (s, 1H), 7.67 (s, 1H), 7.58 (s, 1H), 7.47 (s, 1H), 4.12 - 4.06 (m, 1H), 3.61 (s, 3H), 3.43 - 3.37 (m, 3H), 3.31 - 3.25 (m, 2H), 2.75 - 2.70 (m, 2H), 2.61 (s, 3H), 2.14 - 2.05 (m, 2H), 1.45 - 1.37 (m, 2H). |
| 62 | 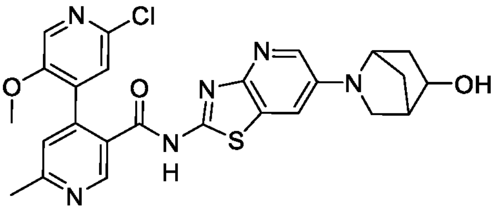 | 523.1 | $^1$H NMR (400 MHz, $CDCl_3$) δ 8.86 (s, 1H), 7.96 (s, 1H), 7.86 (s, 1H), 7.15 (s, 1H), 7.11 (s, 1H), 7.09 (s, 1H), 4.45 - 4.40 (m, 1H), 4.06 - 4.01 (m, 1H), 3.66 (s, 3H), 3.54 - 3.50 (m, 1H), 3.40 - 3.34 (m, 1H), 2.69 - 2.65 (m, 1H), 2.61 (s, 3H), 2.08 - 2.02 (m, 1H), 1.92 - 1.85 (m, 1H), 1.67 - 1.61 (m, 2H), 1.42 - 1.36 (m, 1H). |
| 63 | 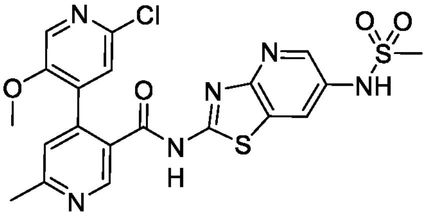 | 505.0 | $^1$H NMR (400 MHz, DMSO-$d_6$) δ 13.20 (s, 1H), 9.98 (s, 1H), 8.87 (s, 1H), 8.41 (s, 1H), 8.30 (s, 1H), 8.16 (s, 1H), 7.59 (s, 1H), 7.48 (s, 1H), 3.60 (s, 3H), 3.05 (s, 3H), 2.61 (s, 3H). |
| 64 | 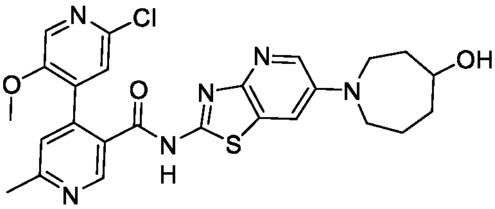 | 525.2 | $^1$H NMR (400 MHz, $CDCl_3$) δ 9.00 (s, 1H), 8.10 (s, 1H), 8.04 (s, 1H), 7.39 (s, 1H), 7.25 - 7.23 (m, 1H), 7.19 (s, 1H), 4.03 - 3.94 (m, 1H), 3.75 (s, 3H), 3.66 - 3.60 (m, 1H), 3.52 - 3.42 (m, 3H), 2.70 (s, 3H), 2.18 - 2.02 (m, 2H), 1.97 - 1.87 (m, 2H), 1.86 - 1.74 (m, 2H). |
| 65 | 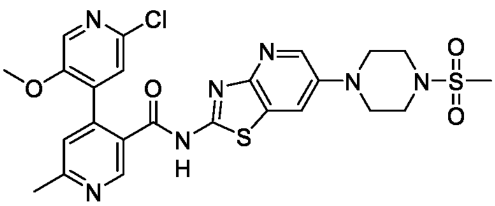 | 574.2 | $^1$H NMR (400 MHz, DMSO-d6) δ 13.09 (s, 1H), 8.87 (s, 1H), 8.42 (s, 1H), 8.17 (s, 1H), 8.09 (s, 1H), 7.59 (s, 1H), 7.50 (s, 1H), 3.60 (s, 3H), 3.32 (d, *J* = 11.8 Hz, 8H), 2.95 (s, 3H), 2.62 (s, 3H). |

(continued)

| Examples | Chemical structure | LC/MS(ESI) m/z [M+H]+ | 1H NMR |
|---|---|---|---|
| 66 | 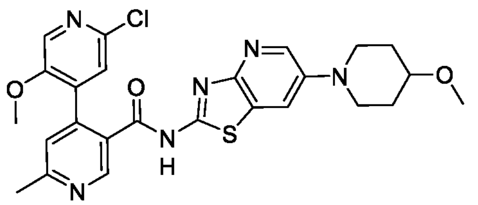 | 525.2 | 1H NMR (400 MHz, DMSO-d6) δ 12.97 (s, 1H), 8.85 (s, 1H), 8.35 (s, 1H), 8.16 (s, 1H), 7.96 (s, 1H), 7.57 (s, 1H), 7.45 (s, 1H), 3.61 (s, 3H), 3.55 - 3.48 (m, 2H), 3.40 - 3.34 (m, 1H), 3.28 (s, 3H), 3.03 - 2.93 (m, 2H), 2.60 (s, 3H), 2.02 - 1.93 (m, 2H), 1.62 - 1.53 (m, 2H). |
| 67 | 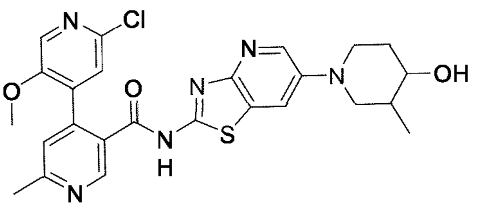 | 525.2 | 1H NMR (400 MHz, $CDCl_3$) δ 8.95 (s, 1H), 8.30 (s, 1H), 8.03 (s, 1H), 7.59 (s, 1H), 7.24 (s, 1H), 7.17 (s, 1H), 3.74 (s, 3H), 3.71 - 3.53 (m, 1H), 3.44 - 3.18 (m, 2H), 3.00 - 2.86 (m, 1H), 2.68 (s, 3H), 2.64 - 2.52 (m, 1H), 2.12 - 2.04 (m, 2H), 1.97 - 1.92 (m, 1H), 1.13 - 1.03 (m, 3H). |
| 68 | 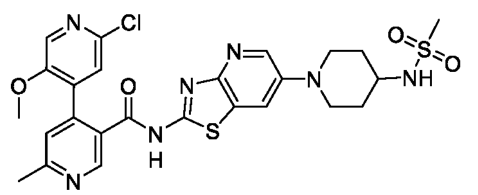 | 588.2 | 1H NMR (400 MHz, DMSO-$d_6$) δ 12.97 (s, 1H), 8.84 (s, 1H), 8.35 (s, 1H), 8.16 (s, 1H), 7.95 (s, 1H), 7.57 (s, 1H), 7.45 (s, 1H), 7.14 (d, *J*= 6.0 Hz, 1H), 3.72 - 3.64 (m, 2H), 3.60 (s, 3H), 2.95 (s, 3H), 2.87 (t, *J* = 11.6 Hz, 2H), 2.60 (s, 3H), 2.56 - 2.53 (m, 1H), 1.98 - 1.90 (m, 2H), 1.66 - 1.52 (m, 2H). |
| 69 | 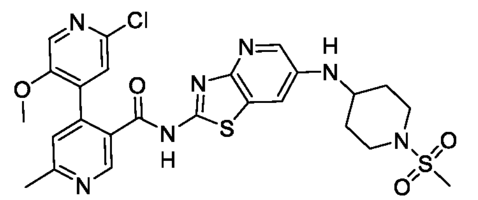 | 588.0 | 1H NMR (400 MHz, DMSO-$d_6$) δ 12.84 (s, 1H), 8.83 (s, 1H), 8.16 (s, 1H), 8.04 (s, 1H), 7.57 (s, 1H), 7.54 (s, 1H), 7.45 (s, 1H), 5.98 (d, *J* = 8.0 Hz, 1H), 3.61 (s, 3H), 3.54 (d, *J* = 10.8 Hz, 2H), 3.48 - 3.40 (m, 1H), 2.95 - 2.87 (m, 5H), 2.60 (s, 3H), 2.03 (d, *J* = 12.2 Hz, 2H), 1.52 - 1.39 (m, 2H). |
| 70 | 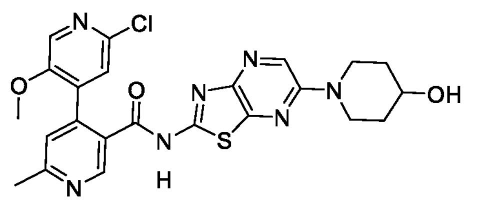 | 512.1 | 1H NMR (400 MHz, DMSO-$d_6$) δ 12.95 (s, 1H), 8.84 (s, 1H), 8.33 (s, 1H), 8.16 (s, 1H), 7.56 (s, 1H), 7.44 (s, 1H), 4.74 (d, *J* = 4.1 Hz, 1H), 4.14 - 3.95 (m, 2H), 3.78 - 3.70 (m, 1H), 3.62 (s, 3H), 3.26 - 3.20 (m, 2H), 2.60 (s, 3H), 1.88 - 1.79 (m, 2H), 1.48 - 1.36 (m, 2H). |
| 71 | 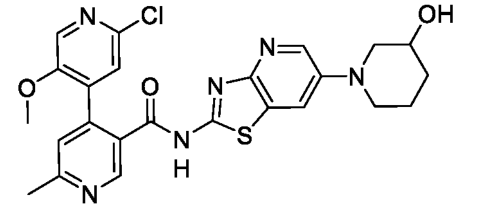 | 511.1 | 1H NMR (400 MHz, $CDCl_3$) δ 9.16 (s, 1H), 8.45 (s, 1H), 8.02 (s, 1H), 7.75 (s, 1H), 7.31 - 7.27 (m, 2H), 4.05 - 3.99 (m, 1H), 3.72 (s, 3H), 3.60 - 3.51 (m, 1H), 3.30 - 3.12 (m, 4H), 2.77 (s, 3H), 2.00 - 1.91 (m, 2H), 1.75 - 1.65 (m, 2H). |

(continued)

| Examples | Chemical structure | LC/MS(ESI) m/z [M+H]+ | $^1$H NMR |
|---|---|---|---|
| 72 | 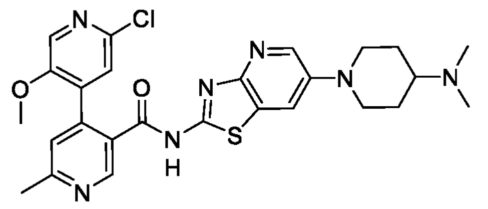 | 538.3 | $^1$H NMR (400 MHz, DMSO-$d_6$) δ 8.89 (s, 1H), 8.30 (s, 1H), 8.15 (s, 1H), 7.88 (s, 1H), 7.52 (s, 1H), 7.39 (s, 1H), 3.73 - 3.70 (m, 2H), 3.61 (s, 3H), 2.73 (t, J = 11.5 Hz, 2H), 2.59 (s, 3H), 2.29 - 2.23 (m, 1H), 2.22(s, 6H), 1.88 - 1.85 (m, 2H), 1.57 - 1.49 (m, 2H). |
| 73 | 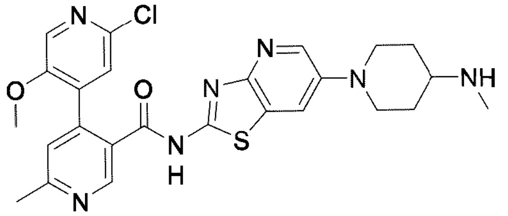 | 524.3 | $^1$H NMR (400 MHz, DMSO-$d_6$) δ 8.94 (s, 1H), 8.23 (s, 1H), 8.14 (s, 1H), 7.81 (s, 1H), 7.46 (s, 1H), 7.31 (s, 1H), 3.68 - 3.65 (m, 2H), 3.61 (s, 3H), 2.83 - 2.70 (m, 3H), 2.57 (s, 3H), 2.43 (s, 3H), 1.99 - 1.96 (m, 2H), 1.54 - 1.41 (m, 2H). |
| 74 | 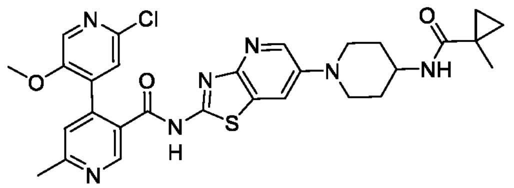 | 592.2 | $^1$H NMR (400 MHz, DMSO-$d_6$) δ 12.96 (s, 1H), 8.84 (s, 1H), 8.36 (d, *J* = 2.4 Hz, 1H), 8.17 (s, 1H), 7.97 (d, *J* = 2.8 Hz, 1H), 7.58 (s, 1H), 7.46 (s, 1H), 7.25 (d, *J* = 7.6 Hz, 1H), 3.89 - 3.78 (m, 1H), 3.78 - 3.71 (m, 2H), 3.60 (s, 3H), 2.91 - 2.78 (m, 2H), 2.60 (s, 3H), 1.81 - 1.71 (m, 2H), 1.70 - 1.58 (m, 2H), 1.24 (s, 3H), 0.98 - 0.91 (m, 2H), 0.52 - 0.44 (m, 2H). |

**Example 75: 2'-chloro-*N*-(6-(4-(dimethylphosphoryl)phenyl)thiazolo[4,5-b]pyridin-2-yl)-5'-methoxy-6-methyl-[4,4'-bipyridine]-3-carboxamide**

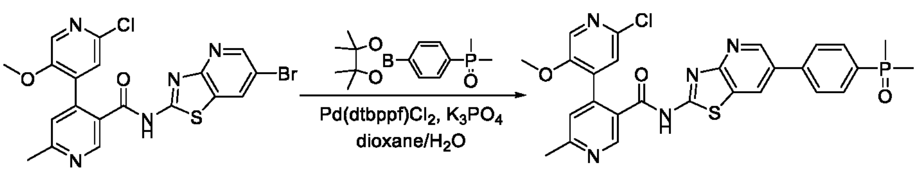


**Step 1: 2'-chloro-N-(6-(4-(dimethylphosphoryl)phenyl)thiazolo[4,5-b]pyridin-2-yl)-5'-methoxy-6-met hyl-[4,4'-bipyridine]-3-carboxamide**

N-(6-bromothiazolo[4,5-b]pyridin-2-yl)-2'-chloro-5'-methoxy-6-methyl-[4,4'-bipyridine]-3-carboxamide (150 mg, 0.306 mmol), dimethyl(4-(4,4,5,5-tetramethyl-1,3,2-dioxaboracyclopentan-2-yl)phenyl)phosphine oxide (85.6 mg, 0.306 mmol), dichloro[1,1'-bis(di-tert-butylphosphine)ferrocenpalladium (II) (19.9 mg, 0.031 mmol) and potassium phosphate (194 mg, 0.917 mmol) were dissolved in 1,4-dioxane (10 mL) and water (2 mL), to allow for reaction at 90°C for 2 hours under the protection of nitrogen. After the reaction was completed, the reaction mixture was added with water and extracted with ethyl acetate; and the organic phase was washed with saturated sodium chloride solution, dried over anhydrous sodium sulfate, filtered, and concentrated under reduced pressure to obtain residues, which were purified by silica-gel chromatography (eluent: petroleum ether/ethyl acetate, gradient: 50-70% ethyl acetate) to obtain 2'-chloro-*N*-(6-(4-(dimethylphosphoryl)phenyl)thiazolo[4,5-b]pyridin-2-yl)-5'-methoxy-6-methyl-[4,4'-bipyr idine]-3-carboxamide (white solid, 50 mg, yield: 29.0%). $^1$H NMR (400 MHz, DMSO- $d_6$) δ 13.31 (s, 1H), 8.91 (d, *J* = 18.9 Hz, 2H), 8.83 (s, 1H), 8.18 (s, 1H), 7.95 - 7.85 (m, 4H), 7.61 (s, 1H), 7.49 (s, 1H), 3.61 (s, 3H), 2.62 (s, 3H), 1.71 (s, 3H), 1.68 (s, 3H). LC/MS (ESI) m/z: 564.2 [M+H]$^+$.

The example compounds in Table 6 were synthesized using commercially available raw materials with reference

to the synthesis steps of Example 75.

Table 6:

| Examples | Chemical structure | LC/MS(ESI) m/z [M+H]+ | $^1$H NMR |
|---|---|---|---|
| 76 | 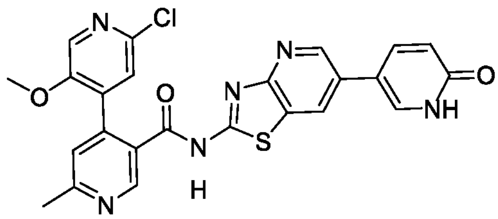 | 505.2 | $^1$H NMR (400 MHz, DMSO-$d_6$) δ 9.17 (s, 1H), 8.98 (d, *J* = 7.7 Hz, 2H), 8.20 (s, 1H), 8.17 - 8.09 (m, 2H), 7.94 (s, 1H), 7.64 (s, 1H), 6.77 (d, *J* = 9.2 Hz, 1H), 3.61 (s, 3H), 2.75 (s, 3H). |
| 77 | 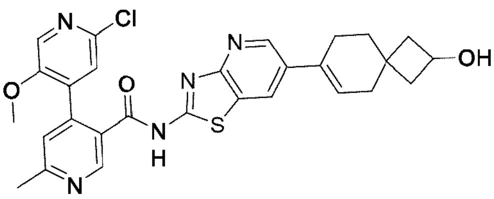 | 548.3 | $^1$H NMR (400 MHz, DMSO-$d_6$) δ 13.18 (s, 1H), 8.87 (s, 1H), 8.63 (s, 1H), 8.43 (s, 1H), 8.16 (s, 1H), 7.58 (s, 1H), 7.46 (s, 1H), 6.22 - 6.18 (m, 1H), 4.91 (d, *J* = 6.0 Hz, 1H), 4.24 - 4.12 (m, 1H), 3.60 (s, 3H), 2.60 (s, 3H), 2.47 - 2.42 (m, 2H), 2.26 - 2.22 (m, 2H), 2.19 - 2.07 (m, 2H), 1.75 - 1.62 (m, 4H). |
| 78 | 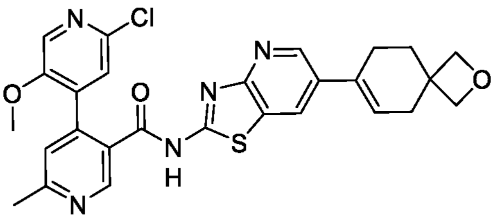 | 534.2 | $^1$H NMR (400 MHz, DMSO-$d_6$) δ 13.20 (s, 1H), 8.88 (s, 1H), 8.63 (s, 1H), 8.43 (s, 1H), 8.16 (s, 1H), 7.58 (s, 1H), 7.46 (s, 1H), 6.23 - 6.20 (m, 1H), 4.39 (d, *J* = 5.5 Hz, 2H), 4.33 (d, *J* = 5.5 Hz, 2H), 3.60 (s, 3H), 2.60 (s, 3H), 2.53 (s, 4H), 2.04 - 1.99 (m, 2H). |
| 79 | 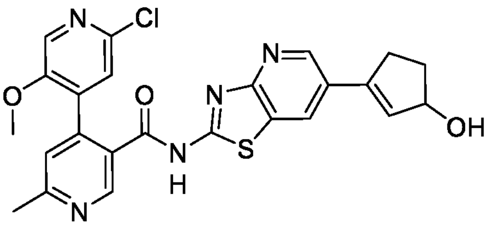 | 494.0 | $^1$H NMR (400 MHz, DMSO-$d_6$) δ 13.22 (s, 1H), 8.90 (s, 1H), 8.73 (s, 1H), 8.49 (s, 1H), 8.16 (s, 1H), 7.56 (s, 1H), 7.43 (s, 1H), 6.40 - 6.37(m, 1H), 4.90 - 4.80 (m, 2H), 3.61 (s, 3H), 2.90 - 2.80 (m, 1H), 2.64 - 2.57 (m, 4H), 2.33 - 2.27 (m, 1H), 1.80 - 1.65 (m, 1H). |
| 80 | 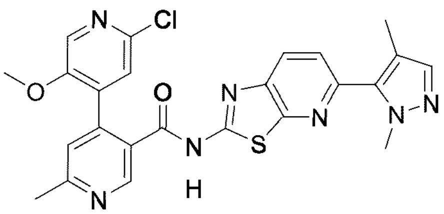 | 506.0 | $^1$H NMR (400 MHz, DMSO-$d_6$) δ 13.17 (s, 1H), 8.87 (s, 1H), 8.26 (d, *J* = 8.0 Hz, 1H), 8.18 (s, 1H), 7.67 (d, *J* = 8.0 Hz, 1H), 7.60 (s, 1H), 7.48 (s, 1H), 7.37 (s, 1H), 3.91 (s, 3H), 3.63 (s, 3H), 2.61 (s, 3H), 2.12 (s, 3H). |
| 81 | 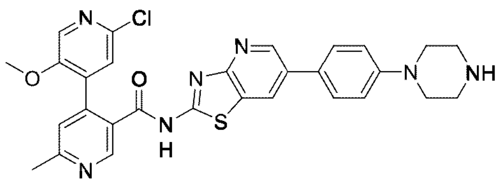 | 572.3 | $^1$H NMR (400 MHz, DMSO-$d_6$) δ 13.24 (s, 1H), 9.04 - 8.97 (m, 2H), 8.89 (s, 1H), 8.85 (d, J = 2.1 Hz, 1H), 8.69 (d, J = 2.0 Hz, 1H), 8.18 (s, 1H), 7.70 (s, 1H), 7.68 (s, 1H), 7.60 (s, 1H), 7.49 (s, 1H), 7.15 (s, 1H), 7.13 (s, 1H), 3.62 (s, 3H), 3.47 - 3.43 (m, 4H), 3.27 - 3.22 (m, 4H), 2.62 (s, 3H) |

(continued)

| Examples | Chemical structure | LC/MS(ESI) m/z [M+H]+ | 1H NMR |
|---|---|---|---|
| 82 | 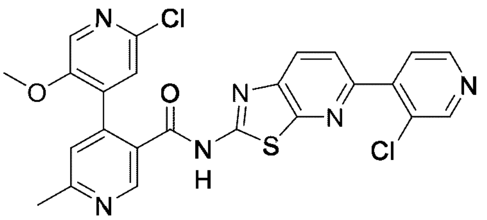 | 523.0 | 1H NMR (400 MHz, DMSO-$d_6$) δ 13.24 (s, 1H), 8.89 (s, 1H), 8.79 (s, 1H), 8.68 - 8.62 (m, 1H), 8.28 (d, *J* = 9.2 Hz, 1H), 8.18 (s, 1H), 7.91 (d, *J* = 9.2 Hz, 1H), 7.75 - 7.71 (m, 1H), 7.60 (s, 1H), 7.48 (s, 1H), 3.63 (s, 3H), 2.61 (s, 3H). |
| 83 | 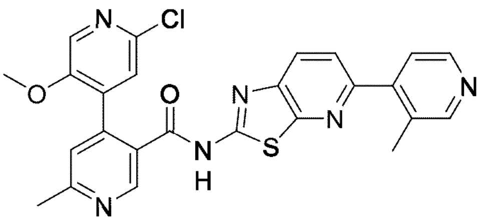 | 503.0 | 1H NMR (400 MHz, DMSO-$d_6$) δ13.18 (s, 1H), 8.88 (s, 1H), 8.56 (s, 1H), 8.54 - 8.49 (m, 1H), 8.25 (d, *J* = 8.0 Hz, 1H), 8.18 (s, 1H), 7.77 (d, J = 8.0 Hz, 1H), 7.59 (s, 1H), 7.53 - 7.45 (m, 2H), 3.63 (s, 3H), 2.61 (s, 3H), 2.40 (s, 3H). |
| 84 | 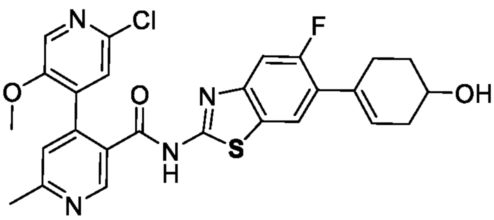 | 525.2 | 1H NMR (400 MHz, DMSO-$d_6$) δ 13.00 (s, 1H), 8.85 (s, 1H), 8.16 (s, 1H), 7.89 (d, J = 7.5 Hz, 1H), 7.57(s, 1H), 7.56 (d, J = 14.6 Hz, 1H), 7.46 (s, 1H), 5.85 - 5.81 (m, 1H), 4.71 (d, J = 3.2 Hz, 1H), 3.87 - 3.78 (m, 1H), 3.59 (s, 3H), 2.60 (s, 3H), 2.47 - 2.40 (m, 3H), 2.11 - 2.04 (m, 1H), 1.92 - 1.83 (m, 1H), 1.67 - 1.56 (m, 1H). |
| 85 | 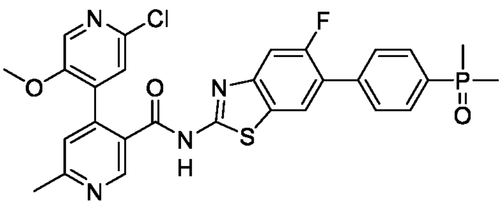 | 581.2 | 1H NMR (400 MHz, DMSO-$d_6$) δ 13.09 (s, 1H), 8.87 (s, 1H), 8.21 (d, *J* = 7.7 Hz, 1H), 8.17 (s, 1H), 7.92 - 7.84 (m, 2H), 7.77 - 7.69 (m, 3H), 7.59 (s, 1H), 7.47 (s, 1H), 3.61 (s, 3H), 2.61 (s, 3H), 1.71 (s, 3H), 1.68 (s, 3H). |
| 86 | 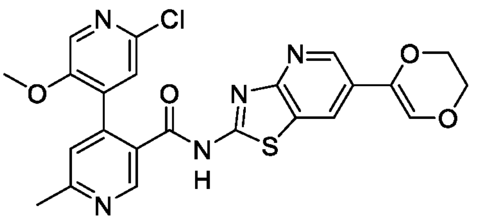 | 496.1 | 1H NMR (400 MHz, DMSO-$d_6$) δ 13.20 (s, 1H), 8.87 (s, 1H), 8.66 (d, J = 2.1 Hz, 1H), 8.40 (d, J = 2.1 Hz, 1H), 8.17 (s, 1H), 7.59 (s, 1H), 7.47 (s, 1H), 7.07 (s, 1H), 4.32 - 4.23 (m, 2H), 4.17 - 4.12 (m, 2H), 3.61 (s, 3H), 2.61 (s, 3H). |
| 87 | 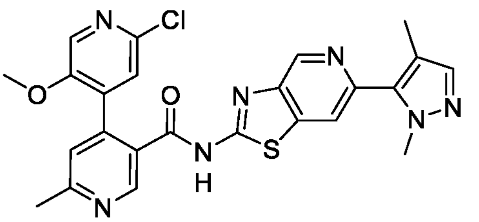 | 506 | 1H NMR (400 MHz, DMSO-$d_6$) δ 13.29 (s, 1H), 9.19 (s, 1H), 8.90 (s, 1H), 8.29 (s, 1H), 8.18 (s, 1H), 7.61 (s, 1H), 7.52 (s, 1H), 7.36 (s, 1H), 3.90 (s, 3H), 3.62 (s, 3H), 2.63 (s, 3H), 2.11 (s, 3H). |

**Example 88: 2'-chloro-*N*-(6-(7-hydroxy-2-oxaspiro[3.5]non-7-yl)thiazolo[4,5-b]pyridin-2-yl)-5'-metho xy-6-methyl-[4,4'-bipyridine]-3-carboxamide**

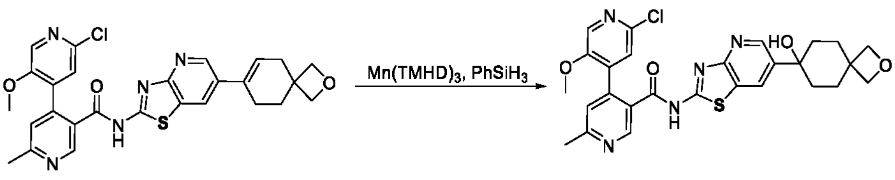


**Step 1: 2'-chloro-N-(6-(7-hydroxy-2-oxaspiro[3.5]non-7-yl)thiazolo[4,5-b]pyridin-2-yl)-5'-methoxy-6-methyl-[4,4'-bipyridine]-3-carboxamide**

In the presence of oxygen and at 0°C, tris(2,2,6,6-tetramethyl-3,5-heptanedionato)manganese (34 mg, 0.06 mmol) was added to the solution of N-(6-(2-oxaspiro[3.5]non-6-en-7-yl)thiazolo[4,5-b]pyridin-2-yl)-2'-chloro-5'-methoxy-6-methyl-[4,4'-bipyri dine]-3-carboxamide (100 mg, 0.19 mmol) in isopropanol (18 mL) and dichloromethane (2 mL); still at 0°C, the reaction was allowed to occur for 10 min; phenylsilane (0.1 mL, 0.94 mmol) was then added dropwise to the reaction mixture; then, still at 0°C, the reaction was allowed to occur 3 hours; and based on LCMS detection, the reaction was completed. Water and dichloromethane were added; and the organic phase was then concentrated to obtain a crude product. The crude product was prepared into the title compound (white solid, 11.7 mg, 11.3%). $^{1}$H NMR (400 MHz, DMSO-$d_6$) δ 13.14 (s, 1H), 8.86 (s, 1H), 8.68 (s, 1H), 8.47 (s, 1H), 8.16 (s, 1H), 7.59 (s, 1H), 7.47 (s, 1H), 5.08 (s, 1H), 4.41 (s, 2H), 4.26 (s, 2H), 3.59 (s, 3H), 2.61 (s, 3H), 1.98 - 1.88 (m, 4H), 1.82 - 1.76 (m, 2H), 1.67 - 1.64 (m, 2H). LC/MS (ESI) m/z: 552.2 $[M+H]^+$.

The example compounds in Table 7 were synthesized using commercially available raw materials with reference to the synthesis steps of Example 88.

Table 7:

| Examples | Chemical structure | LC/MS(ESI) m/z $[M+H]^+$ | $^{1}$H NMR |
|---|---|---|---|
| 89 | 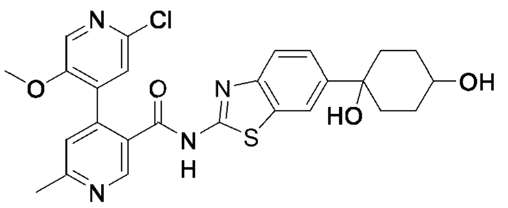 | 525.2 | $^{1}$H NMR (400 MHz, DMSO-$d_6$) δ 12.87 (s, 1H), 8.86 (s, 1H), 8.16 (s, 1H), 8.04 (s, 1H), 7.70 (d, *J* = 7.5 Hz, 1H), 7.56 (d, *J* = 8.6 Hz, 2H), 7.48 (s, 1H), 3.87 - 3.84 (m, 1H), 3.60 (s, 3H), 2.61 (s, 3H), 2.19 - 2.13 (m, 2H), 1.93 - 1.86 (m, 2H), 1.55 - 1.35 (m, 4H). |

**Example 90: 2'-chloro-5'-methoxy-6-methyl-N-(4H-pyrrolo[3,2-d]thiazol-2-yl)-[4,4'-bipyridine]-3-car boxamide**

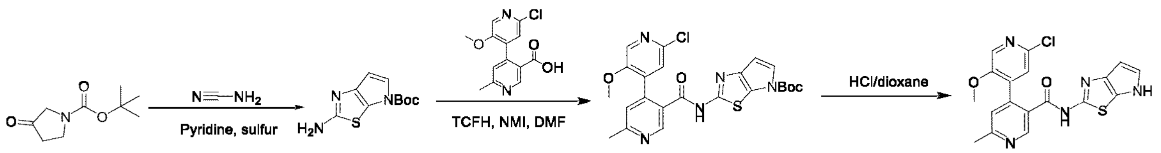


**Step 1: 2-amino-4H-pyrrolo[3,2-d]thiazol-4-tert-butyl carboxylate**

1-tert-butoxycarbyl-3-pyrrolidone (5.00 g, 27.0 mmol), cyanamide (2.27 g, 54.0 mmol), and sulphur (1.84 g, 54.0 mmol) were dissolved in pyridine (50 mL), to allow for reaction at 100°C for 2 hours under the protection of nitrogen. After the reaction was completed, saturated sodium bicarbonate in water (200 mL) was added to the reaction mixture, which was then extracted with ethyl acetate; and the organic phase was washed with a saturated sodium chloride solution, dried over anhydrous sodium sulfate, filtered, and concentrated under reduced pressure to obtain residues, which were purified by silica-gel chromatography (eluent: petroleum ether/ethyl acetate, gradient: 40- 60% ethyl acetate) to obtain 2-amino-4H-pyrrolo[3,2-d]thiazol-4-tert-butyl carboxylate (yellow solid, 1.80 g, yield: 27.9%). LC/MS (ESI) m/z: 240.1 $[M+H]^+$.

**Step 2: 2-(2'-chloro-5'-methoxy-6-methyl-[4,4'-bipyridine]-3-formamido)-4H-pyrrolo[3,2-d]thiazol-4-tert-butyl carboxylate**

2-amino-4H-pyrrolo[3,2-d]thiazol-4-tert-butyl carboxylate (100 mg, 0.418 mmol), 2'-chloro-5'-methoxy-6-methyl-[4,4'-bipyridine]-3-carboxylic acid (116 mg, 0.418 mmol), *N,N,N',N'*-tetramethylchlorformamidine hexafluorphosphate (117 mg, 0.418 mmol) and N-methylimidazole (103 mg, 1.25 mmol) were dissolved in DMF(10 mL), to allow for reaction at room temperature for 12 hours. After the reaction was completed, the reaction mixture was added with water and extracted with ethyl acetate; and the organic phase was washed with saturated sodium chloride solution, dried over anhydrous sodium sulfate, filtered, and concentrated under reduced pressure to obtain residues, which were purified by silica-gel chromatography (eluent: petroleum ether/ethyl acetate, gradient: 50-70% ethyl acetate) to obtain 2-(2'-chloro-5'-methoxy-6-methyl-[4,4'-bipyridine]-3-formamido)-4H-pyrrolo[3,2-d]thiazol-4-tert-butyl carboxylate (white solid, 70 mg, yield: 33.5%). LC/MS (ESI) m/z: 500.1 $[M+H]^+$.

**Step 3: 2'-chloro-5'-methoxy-6-methyl-N-(4H-pyrrolo[3,2-d]thiazol-2-yl)-[4,4'-bipyridine]-3-carboxamide**

A dioxane hydrochloride solution (4.0 M, 10 mL) was added to 2-(2'-chloro-5'-methoxy-6-methyl-[4,4'-bipyridine]-3-formamido)-4H-pyrrolo[3,2-d]thiazol-4-tert-butyl carboxylate (70 mg, 0.140 mmol), to allow for reaction at room temperature for 3 hours. The reaction mixture was concentrated under reduced pressure to obtain residues, which were purified by prep-HPLC (SilaSep™ C18 silica flash cartridge, 25%-85% MeCN in $H_2O$ with 0.01% FA) to obtain 2'-chloro-5'-methoxy-6-methyl-N-(4H-pyrrolo[3,2-d]thiazol-2-yl)-[4,4'-bipyridine]-3-carboxamide (white solid, 20 mg, yield: 35.7%). $^1H$ NMR (400 MHz, DMSO-$d_6$) δ 12.44 (s, 1H), 11.13 (s, 1H), 8.78 (s, 1H), 8.16 (s, 1H), 7.53 (s, 1H), 7.42 (s, 1H), 7.00 (t, *J* = 2.7 Hz, 1H), 6.31 (dd, *J* = 2.9, 1.8 Hz, 1H), 3.60 (s, 3H), 2.58 (s, 3H). LC/MS (ESI) m/z: 400.0 $[M+H]^+$.

**Example 91: 2'-chloro-N-(5-(4-hydroxycyclohex-1-en-1-yl)thiazolo[5,4-d]pyrimidin-2-yl)-5'-methoxy-6-methyl-[4,4'-bipyridine]-3-carboxamide**

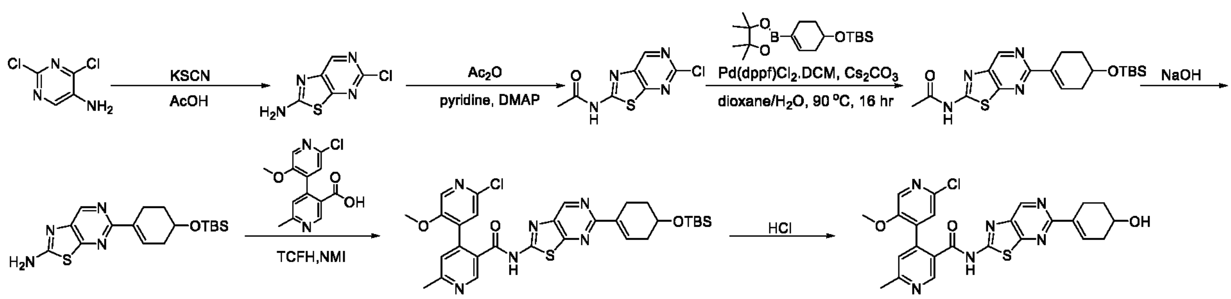


**Step 1: 5-chlorothiazol[5,4-d]pyrimidin-2-amine**

Potassium thiocyanate (3.11 g, 32.0 mmol) was added to the solution of 2,4-dichloropyrimidin-5-amine (5 g, 30.5 mmol) in acetic acid (30 mL). Then, the mixture was stirred at 110°C for 3 hours. After complete reaction, water was added; the resulting mixture was filtered; and the filter cake was washed with water and cold diethyl ether, and dried to obtain the title compound (orange solid, 5 g, yield: 87.9%). LC/MS (ESI) m/z: 187.0 $[M+H]^+$.

**Step 2: *N*-(5-chlorothiazolo[5,4-d]pyrimidin-2-yl)acetamide**

Acetic anhydride (3.5 mL, 37.3 mmol) and 4-dimethylaminopyridine (0.60 g, 4.93 mmol) were added to the solution of 5-chlorothiazol[5,4-d]pyrimidin-2-amine (4.6 g, 24.6 mmol) in 1,2-dichloroethane (50 mL). Then, the mixture was allowed for reaction at 80°C for 5 hours. After complete reaction, methanol was added; and the resulting mixture was filtered, pulped with 10% methanol, and dried to obtain the title compound (white solid, 5.2 g, yield: 92.3%). LC/MS (ESI) m/z: 229.0 $[M+H]^+$.

**Step 3: N-(5-(4-((tert-butyldimethylsilicyl)oxy)cyclohex-1-en-1-yl)thiazolo[5,4-d]pyrimidin-2-yl)aceta mide**

Tert-butyldimethyl((4-(4,4,5,5-tetramethyl-1,3,2-dioxaborinan-2-yl)cyclohex-3-en-1-yl)oxy) silane (4.44 g, 13.1 mmol), cesium carbonate (1.71 g, 5.25 mmol) and $Pd(dppf)Cl_2.CH_2Cl_2$ (0.71 g, 0.875 mmol) were added to N-(5-chlorothiazolo[5,4-d]pyrimidin-2-yl)acetamide (1 g, 4.37 mmol) in the mixed solution of 1,4 dioxane (100 mL) and water (25 mL). Under the protection of nitrogen, the reaction mixture was stirred at 90°C to react for 16 hours. After complete reaction, the reaction mixture was concentrated under reduced pressure. The residue was purified by column chromatography (eluent: petroleum ether/ethyl acetate, gradient: 0-50% ethyl acetate) to obtain the title compound (white solid, 900 mg, yield: 50.9%). LC/MS (ESI) m/z: 405.1 $[M+H]^+$.

**Step 4: 5-(4-((tert-butyldimethylsilicyl)oxy)cyclohex-1-en-1-yl)thiazolo[5,4-d]pyrimidin-2-amine**

Sodium hydroxide (890 mg, 22.3 mmol) was added to N-(5-(4-((tert-butyldimethylsilicyl)oxy)cyclohex-1-en-1-yl) thiazolo[5,4-d]pyrimidin-2-yl)acetamide (900 mg, 2.22 mmol) in the mixed solution of methanol (12 mL) and water (4 mL). The reaction mixture was stirred at 80°C to react for 3 hours. After complete reaction, the reaction mixture was concentrated under reduced pressure. The residue was purified by column chromatography (eluent: petroleum ether/ethyl acetate, gradient: 0-70% ethyl acetate) to obtain the title compound (white solid, 220 mg, yield: 27.3%). LC/MS (ESI) m/z: 363.2 $[M+H]^+$.

**Step 5: *N*-(5-(4-((tert-butyldimethylsilicyl)oxy)cyclohex-1-en-1-yl)thiazolo[5,4-d]pyrimidin-2-yl)-2'-ch loro-5'-methoxy-6-methyl-[4,4'-bipyridine]-3-carboxamide**

2'-chloro-5'-methoxy-6-methyl-[4,4'-bipyridine]-3-carboxylic acid (254 mg, 0.911 mmol) and N-methylimidazole (149 mg, 1.82 mmol) were sequentially added to 5-(4-((tert-butyldimethylsilicyl)oxy)cyclohex-1-en-1-yl)thiazolo[5,4-d] pyrimidin-2-amine (220 mg, 0.607 mmol) in the mixed solution of N,N-dimethylformamide (3 mL) and acetonitrile (5 mL). Then, at 70°C, *N,N,N',N'*-tetramethylchlorformamidine hexafluorphosphate (187 mg, 0.666 mmol) was added to the mixture. The mixture was stirred at 70°C for 2 hours. After complete reaction, water was added; the resulting mixture was extracted with ethyl acetate; the organic phase was washed with saturated saline, dried over anhydrous sodium sulfate, and concentrated under reduced pressure. The residue was purified by column chromatography (eluent: petroleum ether/ethyl acetate, gradient: 0-70% ethyl acetate) to obtain the title compound (white solid, 200 mg, yield: 52.9%). LC/MS (ESI) m/z: 623.2 $[M+H]^+$.

**Step 6: 2'-chloro-N-(5-(4-hydroxycyclohex-1-en-1-yl)thiazolo[5,4-d]pyrimidin-2-yl)-5'-methoxy-6-met hyl-[4,4'-bipyridine]-3-carboxamide**

4 M dioxane hydrochloride solution (2 mL) was added to the solution of N-(5-(4-((tert-butyldimethylsilicyl)oxy) cyclohex-1-en-1-yl) thiazolo[5,4-d]pyrimidin-2-yl) -2'-chloro-5'-methoxy-6-methyl-[4,4'-bipyridine]-3-carboxamide (100 mg, 0.160 mmol) in dichloromethane (1 mL). The reaction mixture was stirred at room temperature to react for 1 hour. After complete reaction, the reaction mixture was concentrated under reduced pressure; and the residue was purified by pre-HPLC to obtain the title compound (white solid, 35.1 mg, yield: 43.0%). $^1$H NMR (400 MHz, DMSO-$d_6$) $\delta$ 13.27 (s, 1H), 9.12 (s, 1H), 8.86 (s, 1H), 8.17 (s, 1H), 7.60 (s, 1H), 7.48 (s, 1H), 7.17 - 7.13 (m, 1H), 4.72 (d, J = 4.0 Hz, 1H), 3.87 - 3.78 (m, 1H), 3.62 (s, 3H), 2.83 - 2.73 (m, 1H), 2.61 (s, 3H), 2.57 - 2.53 (m, 2H), 2.20-2.10 (m, 1H), 1.95 - 1.85 (m, 1H), 1.66 - 1.56 (m, 1H). LC/MS (ESI) m/z: 509.2 $[M+H]^+$.

**Example 92: 2'-chloro-N-(5-(5-chloropyridin-2-yl)-4,5,6,7-tetrahydrothiazol[5,4-c]pyridin-2-)-5'-meth oxy-6-methyl-[4,4'-bipyridine]-3-carboxamide**

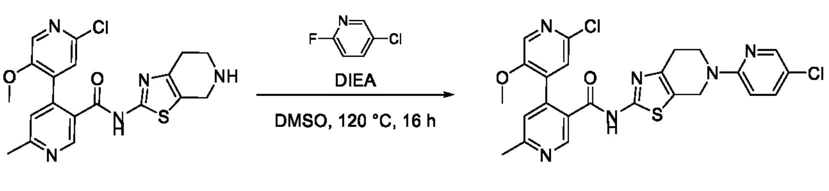


**Step 1: 2'-chloro-N-(5-(5-chloropyridin-2-yl)-4,5,6,7-tetrahydrothiazol[5,4-c]pyridin-2-)-5'-methoxy-6 -methyl-[4,4'-bipyridine]-3-carboxamide**

N,N-diisopropylethylamine (0.572 mL, 3.462 mmol) was added to the solution of 2'-chloro-5'-methoxy-6-methyl-N-(4,5,6,7-tetrahydrothiazol[5,4-c]pyridin-2-yl)-[4,4'-bipyridine]-3-carboxa mide (480 mg, 1.154 mmol) and 5-chloro-2-fluoropyridine (455.41 mg, 3.462 mmol) in dimethyl sulfoxide (5 mL); and the reaction mixture was stirred at 120°C for 16 hours. After complete reaction, the reaction mixture was filtered; and the stock solution obtained after filtering was purified

by pre-HPLC to obtain the title compound (yellow solid, 75 mg, yield: 12.3%). $^1$H NMR (400 MHz, DMSO-$d_6$) $\delta$ 12.60 (s, 1H), 8.76 (s, 1H), 8.20-8.00 (m, 2H), 7.63 (d, $J$ = 9.4 Hz, 1H), 7.52 (s, 1H), 7.42 (s, 1H), 7.00 (d, $J$ = 8.8 Hz, 1H), 4.69 (s, 2H), 3.98 - 3.86 (m, 2H), 3.60 (s, 3H), 2.78 - 2.70 (m, 2H), 2.58 (s, 3H). LC/MS (ESI) m/z: 527 [M+H]$^+$.

**Example 93: 2'-chloro-N-(6-(2-hydroxy-2-methylpropyl)thiazolo[4,5-b]pyridin-2-yl)-5'-methoxy-6-me thyl-[4,4'-bipyridine]-3-carboxamide**

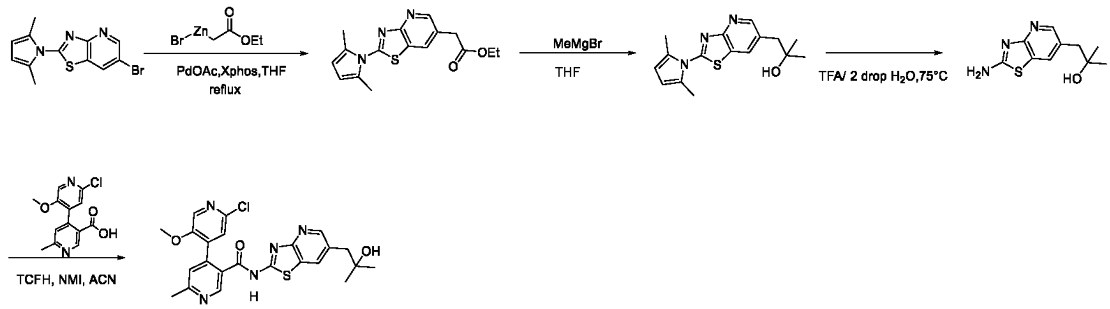


**Step 1: 2-(2-(2,5-dimethyl-1H-pyrrol-1-yl)thiazolo[4,5-b]pyridin-6-yl)ethyl acetate**

6-bromo-2-(2,5-dimethyl-1H-pyrrol-1-yl)thiazolo[4,5-b]pyridine (500 mg, 1.62 mmol) was dissolved in anhydrous tetrahydrofuran (5 mL); palladium acetate (27.0 mg, 0.16 mmol) and 2-dicyclohexylphosphin-2',4',6'-triisopropylbiphenyl (77.3 mg, 0.16 mmol) were added; then, (2-ethoxy-2-oxyethyl)zinc bromide (II) (15 mL, 16.22 mmol) was added; and under the protection of nitrogen, the resulting mixture was refluxed to react for 18 hours. After complete reaction, saturated ammonium chloride was slowly added to the reaction mixture for quenching; the mixture was extracted with ethyl acetate, concentrated under reduced pressure, and allowed to pass through the columns (eluent: ethyl acetate/petroleum ether=1/8, V/V), to obtain the title compound (white solid, 500 mg, yield: 97.7%). LC/MS (ESI) m/z: 316.2 [M+H]$^+$.

**Step 2: 1-(2-(2,5-dimethyl-1H-pyrrol-1-yl)thiazolo[4,5-b]pyridin-6-yl)-2-methylprop-2-ol**

2-(2-(2,5-dimethyl-1H-pyrrol-1-yl)thiazolo[4,5-b]pyridin-6-yl)ethyl acetate (500 mg, 1.59 mmol) was dissolved in anhydrous tetrahydrofuran (10 mL); under the protection of nitrogen and in an ice bath, 1 M methyl magnesium bromide (16 mL, 16 mmol) was added dropwise; and the mixture was allowed to react at room temperature for 18 hours. In the reaction mixture, saturated ammonium chloride was slowly added for quenching; and the mixture was extracted with ethyl acetate, concentrated under reduced pressure, and allowed to pass through the columns (eluent: methanol/dichloromethane = 1/20, V/V), to obtain crude title compound (yellow solid, 250 mg, yield: 52.3%). LC/MS (ESI) m/z: 302.2[M+H]$^+$.

**Step 3: 1-(2-aminothiazolo[4,5-b]pyridin-6-yl)-2-methylprop-2-ol**

The crude product 1-(2-(2,5-dimethyl-1H-pyrrol-1-yl)thiazolo[4,5-b]pyridin-6-yl)-2-methylprop-2-ol(250 mg, 0.83 mmol) was dissolved in trifluoroacetic acid (10 mL); and two drops of water were added; and the mixture was refluxed to allow for reaction for 2h. After complete reaction, the reaction mixture was directly spin-dried, added with dichloromethane, spin-dried three times to directly obtain crude title compound (yellow oil, 250 mg). LC/MS (ESI) m/z: 224.2 [M+H]$^+$.

**Step 4: 2'-chloro-N-(6-(2-hydroxy-2-methylpropyl)thiazolo[4,5-b]pyridin-2-yl)-5'-methoxy-6-methyl-[4,4'-bipyridine]-3-carboxamide**

The crude product 1-(2-aminothiazolo[4,5-b]pyridin-6-yl)-2-methylprop-2-ol (125 mg, 0.56 mmol), 2'-chloro-5'-methoxy-6-methyl-[4,4'-bipyridine]-3-carboxylic acid (171.7 mg, 8.4 mmol), and methylimidazole (229.9 mg, 2.8 mmol) were dissolved in 10 mL of acetonitrile, and stirred at room temperature for 10 minutes; and then N,N,N',N'-tetramethylchlorformamidine hexafluorphosphate (0.785 g, 2.8 mmol) was added, to allow for reaction at room temperature for 5 hours. The reaction mixture was concentrated under reduced pressure, and purified by prep-HPLC (eluent: acetonitrile/0.1% formic acid aqueous solution = 0%-70%, V/V) to obtain 2'-chloro-N-(6-(2-hydroxy-2-methylpropyl)thiazolo[4,5-b]pyridin-2-yl)-5'-methoxy-6-methyl-[4,4'-bipyridin e]-3-carboxamide (3.7 mg, white solid, yield: 1.4%). $^1$H NMR (400 MHz, DMSO-$d_6$) $\delta$ 13.14 (s, 1H), 8.86 (s, 1H), 8.39 (s, 1H), 8.24 (s, 1H), 8.15 (s, 1H), 7.59 (s, 1H), 7.46 (s, 1H), 4.45 (s, 1H),

3.60 (s, 3H), 2.76 (s, 2H), 2.60 (s, 3H), 1.09 (s, 6H). LC/MS (ESI) m/z: 484.2 [M+H]+.

**Example 94: 2'-chloro-5'-methoxy-6-methyl-N-(6-(methylsulfonyl)thiazolo[4,5-b]pyridin-2-yl)-[4,4'-bi pyridine]-3-carboxamide**

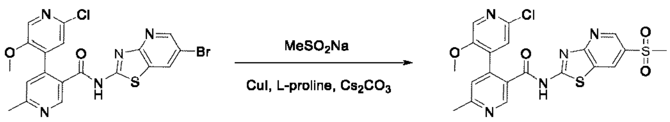


**Step 1: 2'-chloro-5'-methoxy-6-methyl-N-(6-(methylsulfonyl)thiazolo[4,5-b]pyridin-2-yl)-[4,4'-bipyrid ine]-3-carboxamide**

Sodium mesilate (166 mg, 0.816 mmol), L-proline (94 mg, 0.816 mmol), cuprous iodide (116 mg, 0.609 mmol) and cesium carbonate (266 mg, 0.816 mmol) were sequentially added to the solution of N-(6-bromothiazolo[4,5-b] pyridin-2-yl)-2'-chloro-5'-methoxy-6-methyl-[4,4'-bipyridine]-3-carboxamide (200 mg, 0.408 mmol) in dimethyl sulfoxide (10 mL). Then, the mixture was stirred at 100°C for 16 hours.After complete reaction, water was added; the resulting mixture was extracted with 10% methanol/dichloromethane; and the organic phase was washed with saturated saline, dried over anhydrous sodium sulfate, and concentrated under reduced pressure. The residue was purified by pre-HPLC to obtain the title compound (faint yellow solid,74.2 mg, yield: 37.2%). $^1$H NMR (400 MHz, DMSO-d6) δ 13.58 (s, 1H), 9.04 (s, 2H), 8.90 (s, 1H), 8.17 (s, 1H), 7.62 (s, 1H), 7.49 (s, 1H), 3.59 (s, 3H), 3.34 (s, 3H), 2.62 (s, 3H). LC/MS (ESI) m/z: 490.1 [M+H]+.

**Example 95: 2'-chloro-N-(6-(2-hydroxypropan-2-yl)thiazolo[4,5-b]pyridin-2-yl)-5'-methoxy-6-methyl -[4,4'-bipyridine]-3-carboxamide**

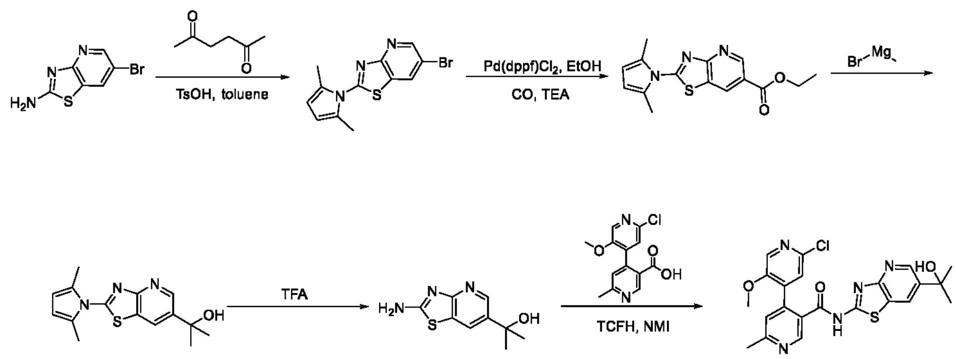


**Step 1: 6-bromo-2-(2,5-dimethyl-1H-pyrrol-1-yl)thiazolo[4,5-b]pyridine**

6-bromothiazolo[4,5-b]pyridin-2-amine (2.0 g, 8.69 mmol) was dissolved in anhydrous toluene (15 mL); hexan-2,5-dione (2.04 mL) and p-toluenesulfonic acid (165 mg, 0.958 mmol) were added; and then, under the protection of nitrogen, the mixture was allowed to react at 120°C for 24 hours. The mixture was cooled to room temperature, added with saturated sodium bicarbonate, and filtered; and the filtrate was extracted with ethyl acetate, washed with saturated saline, concentrated under reduced pressure, and allowed to pass through the columns (eluent: ethyl acetate/petroleum ether = 1/5, V/V), to obtain the title compound (white solid, 1.3 g, yield: 48.5%). LC/MS (ESI) m/z: 308.2 [M+H]+.

**Step 2: 2-(2,5-dimethyl-1H-pyrrol-1-yl)thiazolo[4,5-b]pyridin-6-ethyl formate**

6-bromo-2-(2,5-dimethyl-1H-pyrrol-1-yl)thiazolo[4,5-b]pyridine (500 mg, 1.62 mmol) was dissolved in anhydrous dimethylformamide (15 mL)/anhydrous ethanol (5 mL); [1,1'-bis(diphenylphosphino)ferrocen]palladium dichlorid (II) (116.1 mg, 0.16 mmol) and triethylamine (491.8 mg, 4.86 mmol) were added; and then, under the protection of carbon monoxide, the mixture was allowed to react at 100°C for 24 hours. The mixture was cooled to room temperature; saturated

ammonium chloride was slowly added to the reaction mixture; and the mixture was then extracted with ethyl acetate, washed with saturated saline, concentrated under reduced pressure, and allowed to pass through the columns (eluent: ethyl acetate/petroleum ether = 1/5, V/V) to obtain the title compound (white solid, 300 mg, yield: 61.4%). LC/MS (ESI) m/z: 302.1 $[M+H]^+$.

**Step 3: 2-(2-(2,5-dimethyl-1H-pyrrol-1-yl)thiazolo[4,5-b]pyridin-6-yl)prop-2-ol**

2-(2,5-dimethyl-1H-pyrrol-1-yl)thiazolo[4,5-b]pyridin-6-ethyl formate (300 mg, 0.995 mmol) was dissolved in anhydrous tetrahydrofuran (10 mL); under the protection of nitrogen and in an ice bath, methyl magnesium bromide (10 mL, 9.95 mmol) was added dropwise; and the mixture was allowed to react at room temperature for 18 hours. In the reaction mixture, saturated ammonium chloride was slowly added for quenching; and the mixture was extracted with ethyl acetate, concentrated under reduced pressure, and allowed to pass through the columns (eluent: methanol/dichloromethane = 1/20, V/V), to obtain crude title compound (yellow solid, 100 mg, yield: 35.0%). LC/MS (ESI) m/z: 288.2 $[M+H]^+$.

**Step 4: 2-(2-aminothiazolo[4,5-b]pyridin-6-yl)prop-2-ol**

2-(2-(2,5-dimethyl-1H-pyrrol-1-yl)thiazolo[4,5-b]pyridin-6-yl)prop-2-ol (100 mg, 0.478 mmol) was dissolved in trifluoroacetic acid (10 mL); two drops of water were added; and the mixture was refluxed to react for 2 hours. After complete reaction, the reaction mixture was directly spin-dried, added with dichloromethane, spin-dried three times to directly obtain crude title compound (yellow oil, 263.2 mg). LC/MS (ESI) m/z: 210.2 $[M+H]^+$.

**Step 5: 2'-chloro-N-(6-(2-hydroxypropan-2-yl)thiazolo[4,5-b]pyridin-2-yl)-5'-methoxy-6-methyl-[4,4'-bipyridine]-3-carboxamide**

The crude product 2-(2-aminothiazolo[4,5-b]pyridin-6-yl)prop-2-ol (263.2 mg, 0.56 mmol), 2'-chloro-5'-methoxy-6-methyl-[4,4'-bipyridine]-3-carboxylic acid (171.7 mg, 8.4 mmol), and methylimidazole (229.9 mg, 2.8 mmol) were dissolved in 10 mL of acetonitrile, and stirred at room temperature for 10 minutes; and then, N,N,N',N'-tetramethylchlorformamidine hexafluorphosphate (0.785 g, 2.8 mmol) was added to allow for reaction at room temperature for 5 hours. The reaction mixture was concentrated under reduced pressure, and purified by prep-HPLC (eluent: acetonitrile/0.1% trifluoroacetic acid in water = 0%-70%, V/V) to obtain 2'-chloro-N-(6-(2-hydroxypropan-2-yl)thiazolo[4,5-b]pyridin-2-yl)-5'-methoxy-6-methyl-[4,4'-bipyridine]-3-carboxamide (13.9 mg, yellow solid, yield: 2.4%). $^1H$ NMR (400 MHz, DMSO-$d_6$) $\delta$ 13.25 (s, 1H), 8.90 (s, 1H), 8.71 (s, 1H), 8.63 (s, 1H), 8.16 (s, 1H), 7.60 (s, 1H), 7.52 (s, 1H), 3.59 (s, 3H), 3.04 (s, 1H), 2.62 (s, 3H), 1.52 (s, 6H). LC/MS (ESI) m/z: 470.2 $[M+H]^+$.

**Example 96: 2'-chloro-N-(6-(4-hydroxypiperidin-1-carbonyl)thiazolo[4,5-b]pyridin-2-yl)-5'-methoxy-6-methyl-[4,4'-bipyridine]-3-carboxamide**

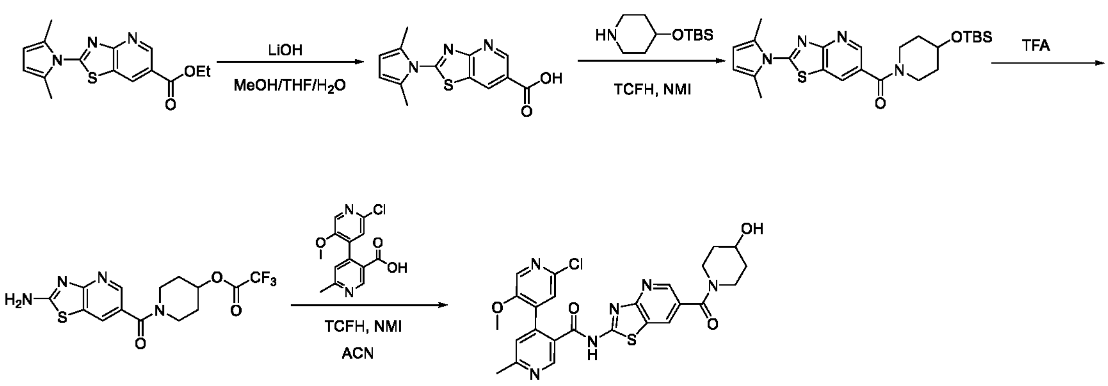


**Step 1: 2-(2,5-dimethyl-1H-pyrrol-1-yl)thiazolo[4,5-b]pyridin-6-formic acid**

2-(2,5-dimethyl-1H-pyrrol-1-yl)thiazolo[4,5-b]pyridin-6-ethyl formate (160 mg, 0.531 mmol) was dissolved in methanol (3 mL)/tetrahydrofuran (3 mL)/water (3 mL); and lithium hydroxide monohydrate (111.4 mg, 2.65 mmol) was added to allow for reaction at 30°C for 3 hours. After complete reaction, saturated ammonium chloride was added to the

reaction mixture for quenching to neutrality; and the reaction mixture was directly spin-dried to obtain a crude title compound (yellow solid, 300 mg). LC/MS (ESI) m/z: 274.2 [M+H]$^+$.

**Step 2: (4-((tert-butyldimethylsilyl)oxy)piperidin-1-yl)(2-(2,5-dimethyl-1H-pyrrol-1-yl)thiazolo[4,5-b] pyridin-6-yl)ketone**

The crude product 2-(2,5-dimethyl-1H-pyrrol-1-yl)thiazolo[4,5-b]pyridin-6-formic acid (300 mg, based on 0.531 mmol), 4-((tert-butyldimethylsilicyl)oxy)piperidine (228.8 mg, 1.06 mmol), and methylimidazole (229.9 mg, 2.8 mmol) were dissolved in 10 mL of acetonitrile, and stirred at room temperature for 10 minutes; and then, N,N,N',N'-tetramethylchlorformamidine hexafluorphosphate (0.785 g, 2.8 mmol) was added to allow for reaction at room temperature for 5 hours. The reaction mixture was concentrated under reduced pressure, subjected to sample blending, and allowed to pass through the columns (eluent: methanol/dichloromethane = 1/10, V/V) to obtain the title compound (yellow solid, 150 mg, yield: 60.0%). LC/MS (ESI) m/z: 471.2 [M+H]$^+$.

**Step 3: 1-(2-aminothiazolo[4,5-b]pyridin-6-carbonyl)piperidin-4-yl2,2,2-trifluoroacetate**

(4-((tert-butyldimethylsilyl)oxy)piperidin-1-yl)(2-(2,5-dimethyl-1H-pyrrol-1-yl)thiazolo[4,5-b]pyridin-6-yl) ketone (150 mg, 0.319 mmol) was dissolved in trifluoroacetic acid (10 mL); two drops of water were added; and the mixture was refluxed to react for 2 hours. After complete reaction, the reaction mixture was directly spin-dried, added with dichloromethane and spin-dried three times to directly obtain a crude title compound (yellow oil, 150 mg). LC/MS (ESI) m/z: 375.2 [M+H]$^+$.

**Step 4: 2'-chloro-N-(6-(4-hydroxypiperidin-1-carbonyl)thiazolo[4,5-b]pyridin-2-yl)-5'-methoxy-6-met hyl-[4,4'-bipyridine]-3-carboxamide**

The crude product 1-(2-aminothiazolo[4,5-b]pyridin-6-carbonyl)piperidin-4-yl2,2,2-trifluoroacetate (150 mg, 0.401 mmol), 2'-chloro-5'-methoxy-6-methyl-[4,4'-bipyridine]-3-carboxylic acid (55.7 mg, 2.00 mmol) and methylimidazole (164.2 mg, 2.00 mmol) were dissolved in 10 mL of acetonitrile, and stirred at room temperature for 10 minutes; and then, N,N,N',N'-tetramethylchlorformamidine hexafluorphosphate (560.7 mg, 2.0 mmol) was added to react at room temperature for 5 hours. The reaction mixture was concentrated under reduced pressure, subjected to sample blending, and allowed to pass through the columns (eluent: methanol/dichloromethane = 1/10, V/V) to obtain the crude title compound, which was then purified by prep-HPLC (eluent: acetonitrile/0.1% formic acid aqueous solution = 0%-70%, *V/V*) to obtain 2'-chloro-N-(6-(4-hydroxypiperidin-1-carbonyl)thiazolo[4,5-b]pyridin-2-yl)-5'-methoxy-6-methyl-[4,4'-bipyr idine]-3-carboxamide (34.3 mg, white solid, yield: 15.9%). $^1$H NMR (400 MHz, DMSO-d6) $\delta$ 13.30 (s, 1H), 8.88 (s, 1H), 8.57 (s, 1H), 8.51 (s, 1H), 8.15 (s, 1H), 7.59 (s, 1H), 7.47 (s, 1H), 4.85 - 4.77 (m, 1H), 4.00 (m, 1H), 3.76 (m, 1H), 3.60 (s, 3H), 2.87 (m, 3H), 2.61 (s, 3H), 1.76 (m, 2H), 1.40 (m, 2H). LC/MS (ESI) m/z: 539.2 [M+H]$^+$.

**Example 97: 2'-chloro-5'-methoxy-6-methyl-N-(6-(pyridin-2-vyl)imidazol[2,1-b][1,3,4]thiadiazol-2-yl)-[4,4'-bipyridine]-3-carboxamide**

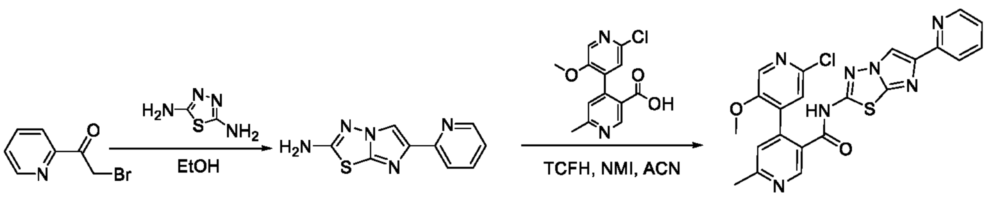


**Step 1: 6-(pyridin-2-yl)imidazo[2,1-b][1,3,4]thiadiazol-2-amine**

2-bromo-1-(2-pyridinyl)-1-ethanone hydrobromide (200 mg, 0.712 mmol) and 2,5-diamino-1,3,4-thiadiazol (82.5 mg, 0.712 mmol) were dissolved in anhydrous ethanol (10 mL); and under the protection of nitrogen, the mixture was stirred and refluxed overnight. After complete reaction, the reaction mixture was concentrated; and then, the residue was dissolved in water, and adjusted to alkaline pH by adding saturated sodium bicarbonate in water. The mixture was extracted with ethyl acetate; the organic phase was dried, filtered, and then spin-dried; and the residue was separated by column chromatography (dichloromethane/methanol system) to obtain a crude title compound (yellow solid, 100 mg).

LC/MS (ESI) m/z: 218.0 $[M+H]^+$.

**Step 2: 2'-chloro-5'-methoxy-6-methyl-N-(6-(pyridin-2-yl)imidazol[2,1-b][1,3,4]thiadiazol-2-yl)-[4,4'-bipyridine]-3-carboxamide**

2-(2,5-dimethyl-1H-pyrrol-1-yl)thiazolo[4,5-b]pyridin-6-formic acid (141 mg, 0.507 mmol), 6-(pyridin-2-yl)imidazo[2,1-b][1,3,4]thiadiazol-2-amine (100 mg, 0.46 mmol), and methylimidazole (188 mg, 2.3 mmol) were dissolved in 10 mL of acetonitrile, and stirred at 70°C for 5 minutes; and then, N,N,N',N'-tetramethylchlorformamidine hexafluorphosphate (142 mg, 0.507 mmol) was added. The mixture was allowed to react at 70°C for 2 hours. The reaction mixture was poured into water, and extracted with ethyl acetate; and the resulting organic phase was dried over anhydrous sodium sulfate, then filtered, and spin-dried. The residue was directly purified by preparative reversed-phase HPLC (acetonitrile-water system) to obtain the title compound (white solid, 3.4 mg, yield: 1.5%). $^1$H NMR (400 MHz, DMSO-$d_6$) δ 13.31 (s, 1H), 8.87 (s, 1H), 8.54 (d, J = 4.1 Hz, 1H), 8.47 (s, 1H), 8.20 (s, 1H), 7.94 (d, J = 7.9 Hz, 1H), 7.83 (t, J = 7.5 Hz, 1H), 7.59 (s, 1H), 7.47 (s, 1H), 7.31 - 7.21 (m, 1H), 3.68 (s, 3H), 2.61 (s, 3H). LC/MS (ESI) m/z: 478.1 $[M+H]^+$.

**Example 98: 2'-chloro-N-(6-(4-(hydroxymethyl)piperidin-1-yl)thiazolo[4,5-b]pyridin-2-yl)-5'-methox y-6-methyl-[4,4'-bipyridine]-3-carboxamide**

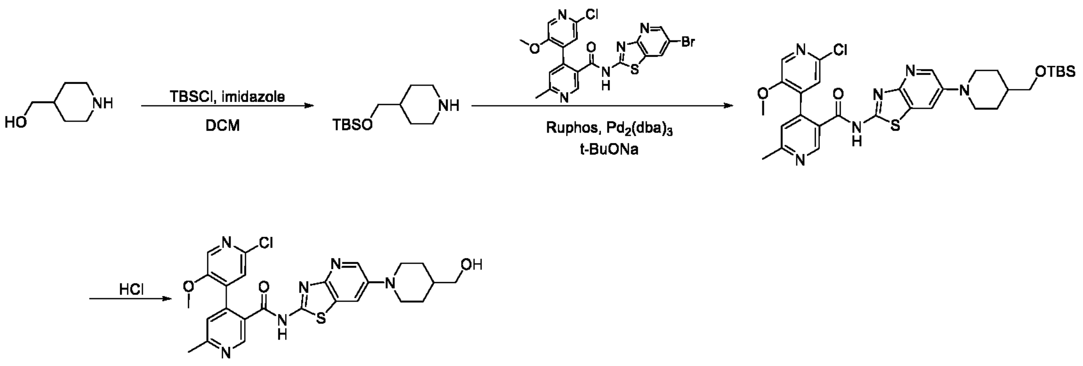


**Step 1: 4-(((tert-butyldimethylsilicyl)oxy)methyl)piperidine**

Imidazole (1.77 g, 26.0 mmol) and tert-butyldimethylchlorosilane (1.31 g, 8.68 mmol) were added to the solution of piperidin-4-ylmethanol (1 g, 8.68 mmol) in dichloromethane (10 mL). Then, the mixture was stirred at room temperature for 16 hours. After complete reaction, water was added; the resulting mixture was extracted with dichloromethane; and the organic phase was washed repeatedly with water till the complete removal of imidazole, dried over anhydrous sodium sulfate, and concentrated under reduced pressure to obtain the title compound (faint yellow oil, 1.35 g, yield: 67.8%). LC/MS (ESI) m/z: 230.3 $[M+H]^+$.

**Step 2: N-(6-(4-(((tert-butyldimethylsilyl)oxy)methyl)piperidin-1-yl)thiazolo[4,5-b]pyridin-2-yl)-2'-ch loro-5'-methoxy-6-methyl-[4,4'-bipyridine]-3-carboxamide**

4-(((tert-butyldimethylsilicyl)oxy)methyl)piperidine (285 mg, 1.22 mmol), sodium tert-butoxide (117 mg, 1.22 mmol), 2-dicyclohexylphosphino-2 ',6'-diisopropoxybiphenyl (57 mg, 0.122 mmol) and tris(dibenzylideneacetone)dipalladium (112 mg, 0.122 mmol) were sequentially added to the solution of N-(6-bromothiazolo[4,5-b]pyridin-2-yl)-2'-chloro-5'-methoxy-6-methyl-[4,4'-bipyridine]-3-carboxamide (300 mg, 0.611 mmol) in 1,4-dioxane (30 mL). Under the protection of nitrogen, the reaction mixture was stirred at 90°C to react for 2 hours. After complete reaction, the reaction mixture was concentrated under reduced pressure. The residue was purified by column chromatography (eluent: dichloromethane/methanol, gradient: 0-5% methanol) to obtain the title compound (yellow solid, 190 mg, yield: 28.0%). LC/MS (ESI) m/z: 639.3 $[M+H]^+$.

**Step 3: 2'-chloro-N-(6-(4-(hydroxymethyl)piperidin-1-yl)thiazolo[4,5-b]pyridin-2-yl)-5'-methoxy-6-m ethyl-[4,4'-bipyridine]-3-carboxamide**

4 mol/L dioxane hydrochloride solution (2 mL) was added to N-(6-(4-(((tert-butyldimethylsilyl)oxy)methyl)piperidin-1-yl)thiazolo[4,5-b]pyridin-2-yl)-2'-chloro-5'-metho xy-6-methyl-[4,4'-bipyridine]-3-carboxamide (190 mg, 0.297 mmol). The reaction mixture was stirred at room temperature to react for 2 hours. After complete reaction, the reaction mixture was concentrated under reduced pressure; and the residue was purified by pre-HPLC to obtain the title compound (yellow solid, 30.2 mg, yield: 19.4%). $^1$H NMR (400 MHz, DMSO-$d_6$)$\delta$12.95 (s, 1H), 8.85 (s, 1H), 8.35 (d,J= 2.8 Hz, 1H), 8.16 (s, 1H), 7.94 (d,J= 2.8 Hz, 1H), 7.57 (s, 1H), 7.45 (s, 1H), 4.49 (*t*,*J*= 5.0 Hz, 1H), 3.75 - 3.72 (m, 2H), 3.61 (s, 3H),3.31 - 3.23 (m, 2H), 2.78 - 2.67 (m, 2H), 2.60 (s, 3H), 1.82 - 1.72 (m, 2H), 1.60-1.47 (m, 1H), 1.36 - 1.22 (m, 2H).LC/MS (ESI) m/z: 525.3 [M+H]$^+$.

The example compounds in Table 8 were synthesized using commercially available raw materials with reference to the synthesis steps of Example **98.**

Table 8:

| Exam ples | Chemical structure | LC/MS(ESI ) m/z [M+H]$^+$ | $^1$H NMR |
|---|---|---|---|
| 99 | 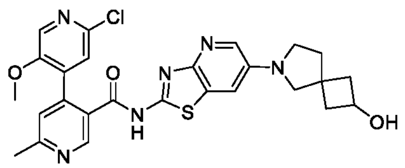 | 537.3 | $^1$H NMR (400 MHz, DMSO-$d_6$) $\delta$ 12.85 (s, 1H), 8.83 (s, 1H), 8.16 (s, 1H), 7.92 (s, 1H), 7.56 (s, 1H), 7.45 (s, 2H), 5.06 - 5.03 (m, 1H), 4.21 - 4.11 (m, 1H), 3.61 (s, 3H), 3.30 - 3.28 (m, 2H), 3.27 - 3.23 (m, 2H), 2.60 (s, 3H), 2.28 - 2.21 (m, 2H), 2.00 - 1.94 (m, 2H), 1.93 - 1.86 (m, 2H). |
| **100** | 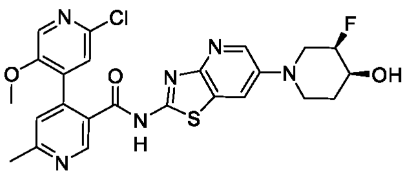 | 529.3 | $^1$H NMR (400 MHz, DMSO-$d_6$) $\delta$ 13.10 (s, 1H), 8.87 (s, 1H), 8.40 (d, *J* = 2.8 Hz, 1H), 8.17 (s, 1H), 8.11 (d, J= 2.8 Hz, 1H), 7.59 (s, 1H), 7.50 (s, 1H), 4.53 - 4.36 (m, 1H), 3.85 - 3.75 (m, 1H), 3.74 - 3.65 (m, 1H), 3.61 (s, 3H), 3.56 - 3.48 (m, 1H), 3.12 - 3.04 (m, 1H), 3.04 - 2.94 (m, 1H), 2.62 (s, 3H), 2.03 - 1.91 (m, 1H), 1.65 - 1.53 (m, 1H). |
| **101** | 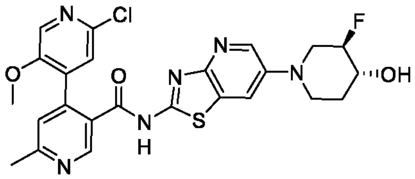 | 529.3 | $^1$H NMR (400 MHz, DMSO-$d_6$) $\delta$ 13.08 (s, 1H), 8.87 (s, 1H), 8.38 (d, *J* = 2.8 Hz, 1H), 8.17 (s, 1H), 8.10 (d, *J* = 2.8 Hz, 1H), 7.59 (s, 1H), 7.50 (s, 1H), 4.83 - 4.65 (m, 1H), 3.88 - 3.78 (m, 1H), 3.78 - 3.71 (m, 1H), 3.61 (s, 3H), 3.57 - 3.50 (m, 1H), 3.32 - 3.20 (m, 1H), 3.10 - 3.02 (m, 1H), 2.62 (s, 3H), 1.91 - 1.81 (m, 1H), 1.80 - 1.71 (m, 1H). |
| 102 | 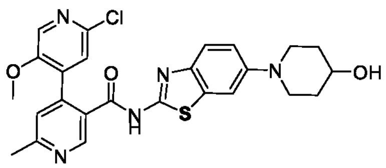 | 510.3 | $^1$H NMR (400 MHz, DMSO-$d_6$) $\delta$ 12.71 (s, 1H), 8.82 (s, 1H), 8.16 (s, 1H), 7.57 (d, *J* = 9.6 Hz, 1H), 7.56 (s, 1H), 7.46 - 7.44(m, 2H), 7.13 (dd, *J* = 9.0, 2.0 Hz, 1H), 4.68 (d, *J* = 3.0 Hz, 1H), 3.69 - 3.62 (m, 1H), 3.60 (s, 3H), 3.57 - 3.49 (m, 2H), 2.92 - 2.81 (m, 2H), 2.60 (s, 3H), 1.89 - 1.79 (m, 2H), 1.58 - 1.44 (m, 2H). |
| 103 | 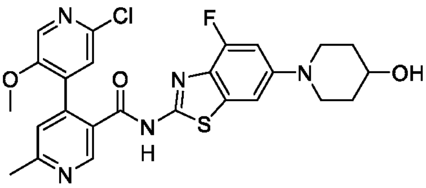 | 528.2 | $^1$H NMR (400 MHz, DMSO-$d_6$) $\delta$ 12.91 (s, 1H), 8.84 (s, 1H), 8.16 (s, 1H), 7.56 (s, 1H), 7.44 (s, 1H), 7.27 (s, 1H), 6.99 (d, *J* = 14.1 Hz, 1H), 4.68 (d, J = 3.2 Hz, 1H), 3.70 - 3.51 (m, 6H), 2.95 - 2.86 (m, 2H), 2.59 (s, 3H), 1.86 - 1.77 (m, 2H), 1.53 - 1.42 (m, 2H). |

(continued)

| Examples | Chemical structure | LC/MS(ESI) m/z [M+H]+ | $^1$H NMR |
|---|---|---|---|
| 104 | 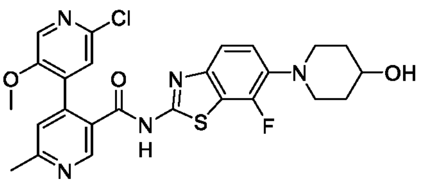 | 528.2 | $^1$H NMR (400 MHz, DMSO-$d_6$) $\delta$ 13.03 (s, 1H), 8.85 (s, 1H), 8.17 (s, 1H), 7.58 (s, 1H), 7.53 (d, $J$ = 8.6 Hz, 1H), 7.46 (s, 1H), 7.22 (t, $J$ = 8.7 Hz, 1H), 4.71 (d, $J$ = 4.0 Hz, 1H), 3.70 - 3.62 (m, 1H), 3.60 (s, 3H), 3.30 - 3.22 (m, 2H), 2.86 - 2.80 (m, 2H), 2.60 (s, 3H), 1.88 - 1.85 (m, 2H), 1.61 - 1.53 (m, 2H). |

**Example 105: (R)-2'-chloro-5'-methoxy-6-methyl-N-(6-(2-(3-methylpiperazin-1-yl)pyridin-4-yl)thiazo lo[4,5-b]pyridin-2-yl)-[4,4'-bipyridine]-3-carboxamide**

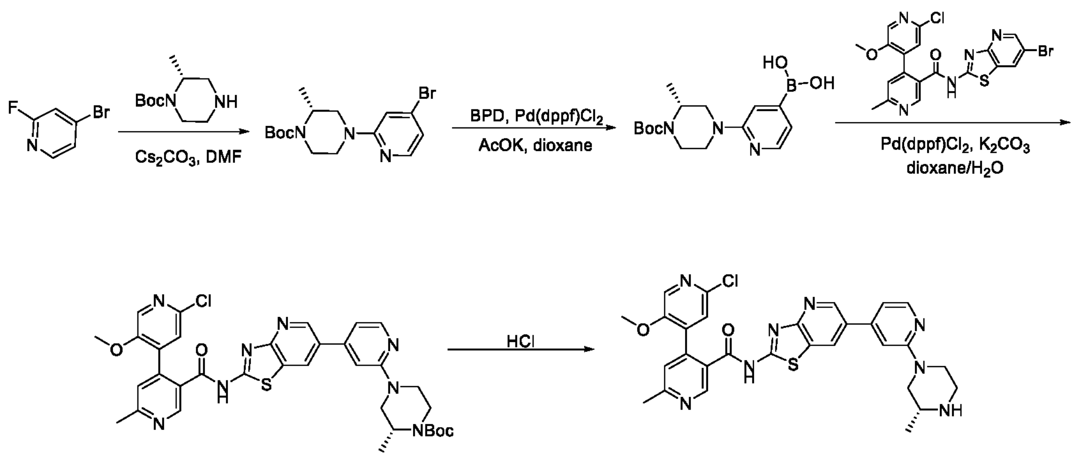


**Step 1: (R)-4-(4-bromopyridin-2-yl)-2-methylpiperazin-1-tert-butyl formate**

4-bromo-2-fluoropyridine (1.00 g, 5.68 mmol), (R)-2-methylpiperazin-1-tert-butyl formate (1.71 g, 8.52 mmol) and potassium carbonate (2.36 g, 17.0 mmol) were dissolved in dimethylsulfoxide (8 mL); and at room temperature, the resulting mixture was stirred to react for 3 hours. After complete reaction, the reaction mixture was extracted with water (50 mL) and ethyl acetate (50 mL); then, the organic phase was washed with saturated saline; and the residue was purified by column chromatography (eluent: dichloromethane/methanol, gradient: 0%-10% methanol) to obtain the title compound (1.6 g, white solid, yield: 79.0%). LC/MS (ESI) m/z: 356.2 [M+H]+.

**Step 2: (R)-(2-(4-(tert-butoxycarbonyl)-3-methylpiperazin-1-yl)pyridin-4-yl)boric acid**

(*R*)-4-(4-bromopyridin-2-yl)-2-methylpiperazin-1-tert-butyl formate (1.00 g, 2.807 mmol), potassium acetate (1.10 g, 11.2 mmol), bis(pinacolato)diboron (2.14 g, 8.42 mmol) and a DPPF-palladium dichloride complex (0.410 g, 0.561 mmol) were dissolved in 1,4-dioxane (25 mL); and the mixture was allowed to react at 85°C for 2 hours. After complete reaction, the reaction mixture was concentrated under reduced pressure; and the residue was purified by column chromatography (eluent: dichloromethane/methanol, gradient: 0%-10% methanol) to obtain the title compound (300 mg, brown solid, yield: 33.3%). LC/MS (ESI) m/z: 322.1 [M+H]+.

**Step 3: (*R*)-4-(4-(2-(2'-chloro-5'-methoxy-6-methyl-[4,4'-bipyridine]-3-carboxamide)thiazolo[4,5-b]py ridin-6-yl) pyridin-2-yl)-2-methylpiperazin-1-tert-butyl formate**

(*R*)-(2-(4-(tert-butoxycarbonyl)-3-methylpiperazin-1-yl)pyridin-4-yl)boric acid (235.60 mg, 0.734 mmol), N-(6-bromothiazolo[4,5-b]pyridin-2-yl)-2'-chloro-5'-methoxy-6-methyl-[4,4'-bipyridine]-3-carboxamide (360 mg, 0.734 mmol), potassium carbonate (304.13 mg, 2.201 mmol) and a DPPF-palladium dichloride complex (107.35 mg, 0.147 mmol) were dissolved in 1,4-dioxane (6 mL) and water (2 mL); and under the protection of nitrogen, the mixture was heated to 85°C to

react for 3 hours. After complete reaction, the reaction mixture was concentrated under reduced pressure; and the residue was purified by column chromatography (eluent: dichloromethane/methanol, gradient: 0%-10% methanol) to obtain the title compound (110 mg, brown solid, yield: 3.3%). LC/MS (ESI) m/z: 687.4 [M+H]$^+$.

**Step 4: (R)-2'-chloro-5'-methoxy-6-methyl-N-(6-(2-(3-methylpiperazin-1-yl)pyridin-4-yl)thiazolo[4,5-b]pyridin-2-yl)-[4,4'-bipyridine]-3-carboxamide**

(*R*)-4-(4-(2-(2'-chloro-5'-methoxy-6-methyl-[4,4'-bipyridine]-3-carboxamide)thiazolo[4,5-b]pyridin-6-yl)pyr idin-2-yl)-2-methylpiperazin-1-tert-butyl formate (110 mg, 0.205 mmol) was dissolved in 1,4-dioxane (5 mL); and hydrochloric acid-dioxane solution (3 mL) was added, to allow for reaction at room temperature for 3 hours. After complete reaction, the reaction mixture was concentrated under reduced pressure; and the residue was purified by reversed-phase preparation to obtain the title compound (25 mg, faint yellow solid, yield: 29.3%). $^1$H NMR (400 MHz, DMSO-$d_6$)δ8.99 (s, 1H), 8.85 (s, 1H), 8.64 (s, 1H), 8.21 - 8.18 (m, 2H), 8.15 (s, 1H), 7.49 (s, 1H), 7.33 (s, 1H), 7.22 (s, 1H), 7.08 (d, J = 5.1 Hz, 1H), 4.43 - 4.35 (m, 2H), 3.62 (s, 3H), 3.22 - 3.17 (m, 1H), 3.08 - 3.02 (m, 1H), 2.98 - 2.90 m, 2H), 2.75 - 2.62 (m, 1H), 2.58 (s, 3H), 1.18 (d, J = 6.4 Hz, 3H). LC/MS (ESI) m/z: 587.3 [M+H]$^+$.

**Example 106: 2'-chloro-N-(6-(4-hydroxy-4-(hydroxymethyl)piperidin-1-yl)thiazolo[4,5-b]pyridin-2-yl) -5'-methoxy-6-methyl-[4,4'-bipyridine]-3-carboxamide**

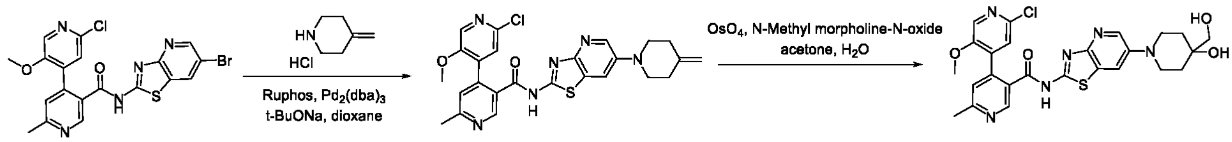


**Step 1: 2'-chloro-5'-methoxy-6-methyl-N-(6-(4-methoxy-piperidin-1-yl)thiazolo[4,5-b]pyridin-2-yl)-[4, 4'-bipyridine]-3-carboxamide**

N-(6-bromothiazolo[4,5-b]pyridin-2-yl)-2'-chloro-5'-methoxy-6-methyl-[4,4'-bipyridine]-3-carboxamide (500 mg, 1.02 mmol), 4-methylenepiperidine hydrochloride (136 mg, 1.02 mmol), tris(dibenzylideneacetone)dipalladium (93.3 mg, 0.102 mmol), 2-dicyclohexylphosphino-2',6'-diisopropoxy-1,1'-biphenyl (95.1 mg, 0.204 mmol) and sodium tert-butoxide (391 mg, 4.08 mmol) were dissolved in 1,4-dioxane (20 mL) to allow for reaction at 100°C for 4 hours under the protection of nitrogen. After the reaction was completed, the reaction mixture was added with water and extracted with ethyl acetate; and the organic phase was washed with saturated sodium chloride solution, dried over anhydrous sodium sulfate, filtered, and concentrated under reduced pressure to obtain residues, which were purified by silica-gel chromatography (eluent: petroleum ether/ethyl acetate, gradient: 50-70% ethyl acetate) to obtain 2'-chloro-5'-methoxy-6-methyl-N-(6-(4-methoxy-piperidin-1-yl)thiazolo[4,5-b]pyridin-2-yl)-[4,4'-bipyridine ]-3-carboxamide (yellow solid, 110 mg, yield: 21.3%). LC/MS (ESI) m/z: 507.0 [M+H]$^+$.

**Step 2: 2'-chloro-N-(6-(4-hydroxy-4-(hydroxymethyl)piperidin-1-yl)thiazolo[4,5-b]pyridin-2-yl)-5'-me thoxy-6-methyl-[4,4'-bipyridine]-3-carboxamide**

2'-chloro-5'-methoxy-6-methyl-N-(6-(4-methoxy-piperidin-1-yl)thiazolo[4,5-b]pyridin-2-yl)-[4,4'-bipyridine ]-3-carboxamide (100 mg, 0.197 mmol), osmium tetroxide (0.50 mg, 0.002 mmol) (0.25 mL of 4% aqueous solution, 0.04 mmol) and N-methylmorpholine oxide (69.3 mg, 0.592 mmol) were dissolved in acetone (4 mL) and water (1 mL), to allow for reaction at room temperature for 12 hours. The reaction mixture was concentrated under reduced pressure to obtain residues, which were purified by prep-HPLC (SilaSep™ C18 silica flash cartridge, 25%-85% MeCN in $H_2O$ with 0.01% FA) to obtain 2'-chloro-N-(6-(4-hydroxy-4-(hydroxymethyl)piperidin-1-yl)thiazolo[4,5-b]pyridin-2-yl)-5'-methoxy-6-meth yl-[4,4'-bipyridine]-3-carboxamide (yellow solid, 28.0 mg, yield: 26.4%). $^1$H NMR (400 MHz, DMSO-$d_6$) δ 8.85 (s, 1H), 8.34 (s, 1H), 8.16 (s, 1H), 7.93 (s, 1H), 7.56 (s, 1H), 7.43 (s, 1H), 4.58 (s, 1H), 4.20 (s, 1H), 3.61 (s, 3H), 3.51 - 3.45 (m, 2H), 3.22 (s, 2H), 3.10 (t, *J* = 11.3 Hz, 2H), 2.60 (s, 3H), 1.80 - 1.63 (m, 2H), 1.45 (d, *J* = 13.1 Hz, 2H). LC/MS (ESI) m/z: 541.2 [M+H]$^+$.

**Example 107: 2'-chloro-N-(5-(4-hydroxypiperidin-1-yl)thiazol[5,4-b]pyridin-2-yl)-5'-deuteromethoxy -6-methyl-[4,4'-bipyridine]-3-carboxamide**

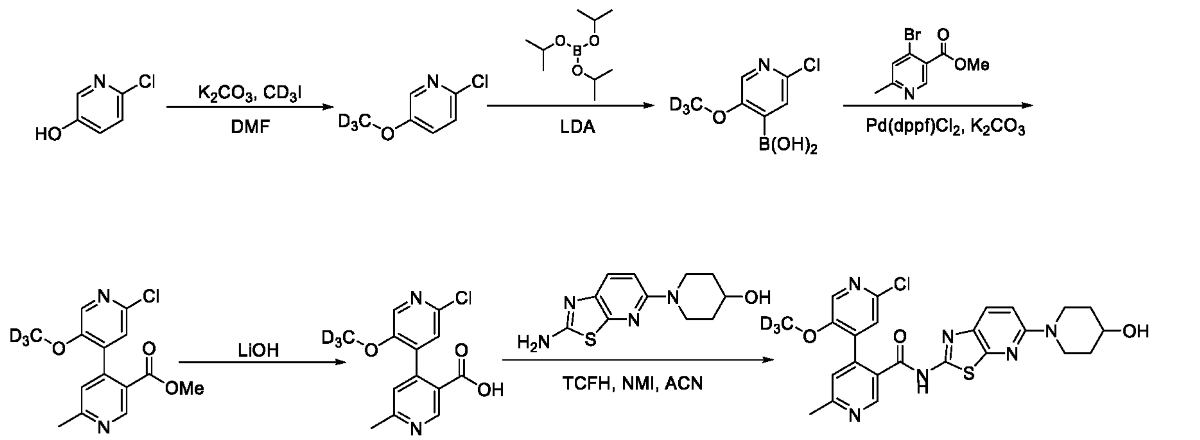


**Step 1: 2-chloro-5-deuteromethoxypyridine**

6-chloropyridin-3-ol (1 g, 7.72 mmol) and potassium carbonate (2.13 g, 15.4 mmol) were dissolved in 40 mL of N,N-dimethylformamide; deuteroiodomethane (1.34 g, 9.26 mmol) was added dropwise, to allow for reaction for 18 hours at room temperature; then, the reaction mixture was extracted with ethyl acetate and water; the organic phase was purified by silica-gel column chromatography (petroleum etherethyl acetate system: 0-25% ethyl acetate) to obtain the title compound (670 mg, white solid, yield: 59.3%). LC/MS (ESI) m/z: 147.2 [M+H] $^+$.

**Step 2: (2-chloro-5-deuteromethoxypyridin-4-yl)boric acid**

2-chloro-5-deuteromethoxypyridine (670 mg, 4.57 mmol) was dissolved in 15 mL of tetrahydrofuran; under the protection of nitrogen, the mixture was cooled to - 65°C; then, lithium diisopropylamide (4.57 mL, 2 mol/L) was added dropwise slowly; the above low temperature was held to allow for reaction for 2 hours; then, triisopropyl borate (1.72 g, 9.14 mmol) was added dropwise slowly; with the low temperature still held, the mixture was stirred for 1 hour; and then, the temperature was slowly increased to room temperature, and the mixture was stirred overnight. In an ice-water bath, 15 mL of water was added to the reaction mixture for quenching; the mixture was washed with ethyl acetate; and the aqueous phase was adjusted to pH of (5-6) with 1 M dilute hydrochloric acid aqueous solution, and filtered under reduced pressure; and the solid phase was dried to obtain the title compound (740 mg, white solid, yield: 85.1%). LC/MS (ESI) m/z: 191.1 [M+H] $^+$.

**Step 3: 2'-chloro-5'-deuteromethoxy-6-methyl-[4,4'-bipyridine]-3-methyl carboxylate**

4-bromo-6-methylmethyl nicotinate (700 mg, 3.04 mmol), (2-chloro-5-deuteromethoxypyridin-4-yl)boric acid (579 mg, 3.04 mmol), and potassium carbonate (1.26 g, 9.12 mmol) were dissolved in the mixed solution of 30 mL of 1,4-dioxane and 6 mL of water; and under the protection of nitrogen, catalyst bis(triphenylphosphine)palladium dichloride (111.3 mg, 0.152 mmol) was added; and the resulting mixture was heated to 80°C to react for 2 hours. The reaction mixture was cooled to room temperature, filtered, and then extracted with water and ethyl acetate; the organic phase was purified by silica-gel column chromatography (petroleum etherethyl acetate system: 0-50% ethyl acetate) to obtain the title compound (720 mg, white solid, yield: 80%). LC/MS (ESI) m/z: 296.1 [M+H] $^+$.

**Step 4: 2'-chloro-5'-deuteromethoxy-6-methyl-[4,4'-bipyridine]-3-carboxylic acid**

2'-chloro-5'-deuteromethoxy-6-methyl-[4,4'-bipyridine]-3-methyl carboxylate (720 mg, 2.44 mmol) was dissolved in 10 mL of tetrahydrofuran and 10 mL of water; lithium hydroxide (87.5 mg, 3.65 mmol) was added; the resulting mixture was stirred overnight at room temperature; the reaction mixture underwent rotary evaporation to remove most of tetrahydrofuran, and then extracted with ethyl acetate; the aqueous phase was adjusted to Ph of 5-6 with 1 M hydrochloric acid solution to allow for precipitation of white solids, which were filtered under reduced pressure; and the filter cake was dried to obtain the title compound (420 mg, white solid, yield: 60.9%). LC/MS (ESI) m/z: 282.1 [M+H] $^+$.

**Step 5: 2'-chloro-N-(5-(4-hydroxypiperidin-1-yl)thiazol[5,4-b]pyridin-2-yl)-5'-deuteromethoxy-6-met hyl-[4,4'-bipyridine]-3-carboxamide**

2'-chloro-5'-deuteromethoxy-6-methyl-[4,4'-bipyridine]-3-carboxylic acid (100 mg, 0.355 mmol), 1-(2-aminothiazol[5,4-b]pyridin-5-yl)piperidin-4-ol (88.8 mg, 0.355 mmol) and N-methylimidazole (87.4 mg, 1.07 mmol) were dissolved in 5 mL of acetonitrile and 1 mL of N,N-dimethylformamide, and stirred at 70°C for 15 minutes; then, N,N,N,N-tetramethylchlorourea hexafluorophosphate (99.6 mg, 0.355 mmol) was added; with the above temperature held, the mixture was stirred and refluxed for 2 hours; the reaction mixture was extracted with water and ethyl acetate; the organic phase was concentrated and then purified by pre-HPLC to obtain the title compound (71 mg, yellow solid, yield: 31.4%). $^1H$ NMR (400 MHz, DMSO-$d_6$) $\delta$ 12.68 (s, 1H), 8.81 (s, 1H), 8.16 (s, 1H), 7.85 (d, J = 9.1 Hz, 1H), 7.56 (s, 1H), 7.45 (s, 1H), 6.99 (d, J = 9.1 Hz, 1H), 4.70 (d, J = 4.4 Hz, 1H), 4.08 - 3.98 (m, 2H), 3.78 - 3.67 (m, 1H), 3.16 - 3.06 (m, 2H), 2.58 (s, 3H), 1.86 - 1.74 (m, 2H), 1.43 - 1.33 (m, 2H). LC/MS (ESI) m/z: 514.2 $[M+H]^+$.

**Example 108: 2'-chloro-N-(6-(4-hydroxycyclohex-1-en-1-yl)thiazolo[4,5-c]pyridin-2-yl)-5'-deuteromet hoxy-6-methyl-[4,4'-bipyridine]-3-carboxamide**

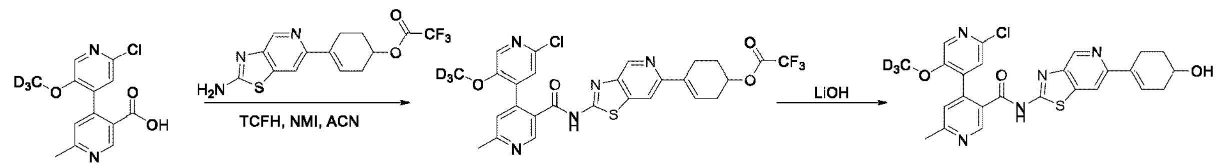


**Step 1: 4-(2-(2'-chloro-5'-deuteromethoxy-6-methyl-[4,4'-bipyridine]-3-formamido)thiazolo[4,5-c]pyr idin-6-yl) cyclohex-3-en-1-yl2,2,2-trifluoroacetate**

2'-chloro-5'-deuteromethoxy-6-methyl-[4,4'-bipyridine]-3-carboxylic acid (200 mg, 0.71 mmol), 4-(2-aminothiazolo[4,5-c]pyridin-6-yl)cyclohex-3-en-1-yl2,2,2-trifluoroacetate (243 mg, 0.355 mmol) and N-methylimidazole (174 mg, 2.13 mmol) were dissolved in 7 mL of acetonitrile and 2 mL N,N-dimethylformamide; at 70°C, the resulting mixture was stirred for 15 minutes; then, N,N,N,N-tetramethylchlorourea hexafluorophosphate (199 mg, 0.71 mmol) was added; with the above temperature held, the mixture was stirred and refluxed for 2 hours; the reaction mixture was extracted with water and ethyl acetate; and the organic phase was spin-dried to obtain the crude title compound (200 mg, yellow solid, yield: 27.8%). LC/MS (ESI) m/z: 607.2 $[M+H]^+$.

**Step 2: 2'-chloro-N-(6-(4-hydroxycyclohex-1-en-1-yl)thiazolo[4,5-c]pyridin-2-yl)-5'-deuteromethoxy-6 -methyl-[4,4'-bipyridine]-3-carboxamide**

4-(2-(2'-chloro-5'-deuteromethoxy-6-methyl-[4,4'-bipyridine]-3-formamido)thiazolo[4,5-c]pyridin-6-yl)cycl ohex-3-en-1-yl2,2,2-trifluoroacetate (200 mg, 0.329 mmol) and lithium hydroxide (138 mg, 3.29 mmol) were dissolved in 7 mL of methanol, and stirred at room temperature for 30 minutes; and the reaction mixture was purified by pre-HPLC to obtain the title compound (34.2 mg, yellow solid, yield: 21.3%). $^1H$ NMR (400 MHz, DMSO-$d_6$) $\delta$ 13.19 (s, 1H), 9.01 (s, 1H), 8.92 (s, 1H), 8.22 (s, 1H), 8.19 (s, 1H), 7.65 (s, 1H), 7.53 (s, 1H), 6.72 (s, 1H), 4.76 (d, *J* = 3.6 Hz, 1H), 3.91 - 3.82(m, 1H), 2.80-2.70 (m, 1H), 2.67 (s, 3H), 2.60-2.57 (m, 1H), 2.54 - 2.49(m, 1H), 2.23 - 2.06 (m, 1H), 2.05 - 1.93 (m, 1H), 1.72 - 1.63 (m, 1H). LC/MS (ESI) m/z: 511.2 $[M+H]^+$.

**Example 109: 2-chloro-N-(5-(4-hydroxypiperidin-1-yl)thiazolo[5,4-b]pyridin-2-yl)-5'-methoxy-6-deut eromethyl-[4,4'-bipyridine]-3-carboxamide**

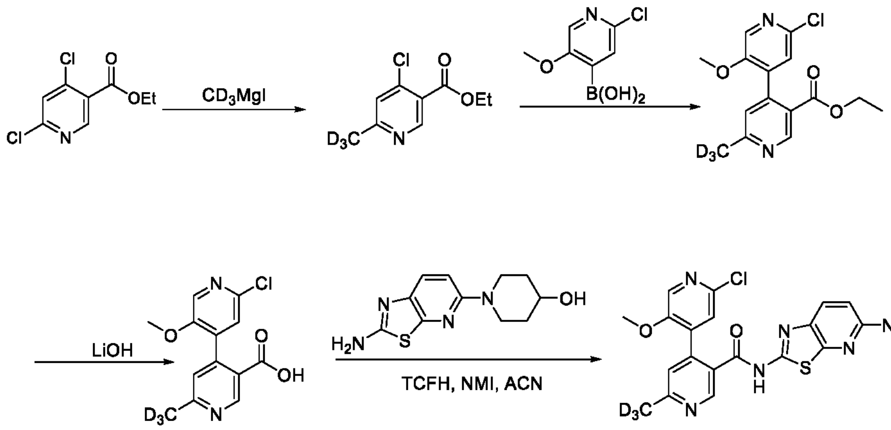


**Step 1: 4-chloro-6-deuteromethylethyl nicotinate**

4, 6-dichloroethyl nicotinate (2 g, 9.1 mmol) and ferric acetylacetonate (320 mg, 0.91 mmol) were dissolved in 30 mL of anhydrous tetrahydrofuran; and under the protection of nitrogen and with temperature controlled at room temperature, 1 mol/L deuteromethyl magnesium iodide (10.5 mL, 10.5mmol) was slowly dropped dropwise. The reaction was allowed to occur at room temperature for 1 hour. The reaction mixture was quenched with saturated ammonium chloride in water, extracted with ethyl acetate, washed with water, washed with saturated saline, and dried over anhydrous sodium sulfate; and the residue was purified by column chromatography (eluent: petroleum ether/ethyl acetate, gradient: 0-17% ethyl acetate) to obtain the title compound (faint yellow solid, 410 mg, yield: 22.3%). LC/MS (ESI) m/z: 203.0 $[M+H]^+$.

**Step 2: 2-chloro-5-methoxy-6-deuteromethyl-[4, 4'-bipyridine]-3-ethyl carboxylate**

4-chloro-6-deuteromethylethyl nicotinate (410 mg, 2.02 mmol), 2-chloro-5-methoxypyridin-4-boric acid (380 mg, 2.02 mmol), and potassium carbonate (838 mg, 6.06 mmol) were dissolved in 10 mL of 1,4-dioxane and 2 mL of water; 1,1-bis(diphenylphosphino)ferrocene palladium dichloride (148 mg, 0.2 mmol) was then added; and under the protection of nitrogen, the temperature was increased to 80°C to allow for reaction for 1 hour. The reaction mixture was filtered through diatomite; the filter cake was washed with methanol; the filtrate was concentrated under reduced pressure; and the resulting residue was purified by column chromatography (eluent: petroleum ether/ethyl acetate, gradient: 10%-40% ethyl acetate), to obtain the title compound (yellow solid, 175 mg, yield: 27.7%). LC/MS (ESI) m/z: 631.0 $[M+H]^+$.

**Step 3: 2-chloro-5-methoxy-6-deuteromethyl-[4, 4'-bipyridine]-3-carboxylic acid**

2-chloro-5-methoxy-6-deuteromethyl-[4, 4'-bipyridine]-3-ethyl carboxylate (175 mg, 0.56 mmol) was dissolved in 2 mL of water and 2 mL of tetrahydrofuran; and lithium hydroxide (34 mg, 0.84 mmol) was added, to allow for reaction at room temperature for 16 hours. The reaction mixture was concentrated under reduced pressure; and the residue was adjusted to pH of 4-5 using 2N hydrochloric acid, allowing for precipitation of solids, which were filtered and dried under reduced pressure to remove water, to obtain the title compound (white solid, 140 mg, yield: 87.5%). LC/MS (ESI) m/z: 282.0 $[M+H]^+$.

**Step 4: 2-chloro-N-(5-(4-hydroxypiperidin-1-yl)thiazolo[5,4-b]pyridin-2-yl)-5'-methoxy-6-deuteromet hyl-[4,4'-bipyridine]-3-carboxamide**

2-chloro-5-methoxy-6-deuteromethyl-[4, 4'-bipyridine]-3-carboxylic acid (20 mg, 0.07 mmol) and 1-(2-aminothiazolo[5, 4-b]pyridin-5-yl)piperidin-4-ol (19 mg, 0.07 mmol) were dissolved in 5 mL of acetonitrile and 3 mL of N,N-dimethylformamide; N-methylimidazole (17 mg, 0.21 mmol) was added, and stirred for 20 minutes; and then, N,N,N',N'-tetramethylchlorformamidine hexafluorphosphate (20 mg, 0.07 mmol) was added, to allow for reaction at room temperature for 2 hours. Ethyl acetate and water were added to the reaction mixture for stratification; the organic phase was washed with saturated sodium chloride solution, dried over anhydrous sodium sulfate, and filtered; the filtrate was concentrated under reduced pressure to obtain residues, which were treated by column chromatography; the reaction mixture was concentrated under reduced pressure; and the residue was purified by pre-HPLC (eluent: acetonitrile/0.1% formic acid aqueous solution, gradient: 25%-90%) to obtain the title compound (white solid, 12.7 mg, yield: 34.8%). $^1$HNMR (400MHz, DMSO-$d_6$) $\delta$ 12.66 (s, 1H), 8.82 (s, 1H), 8.16 (s, 1H), 7.83 (d, $J$ = 9.0 Hz, 1H), 7.55 (s, 1H), 7.43 (s, 1H),

6.98 (d, $J$ = 9.1 Hz, 1H), 4.70 (d, J = 4.0 Hz, 1H), 4.07 - 3.98 (m, 2H), 3.76 - 3.65 (m, 1H), 3.62 (s, 3H), 3.17 - 3.09 (m, 2H), 1.85 - 1.76 (m, 2H), 1.44 - 1.34 (m, 2H). LC/MS (ESI) m/z: 514.2 $[M+H]^+$.

**Example 110: 2'-chloro-N-(6-(4-hydroxycyclohex-1-en-1-yl)thiazolo[4,5-c]pyridin-2-yl)-5'-methoxy-6-(methyl-$d_3$)-[4,4'-bipyridine]-3-carboxamide**

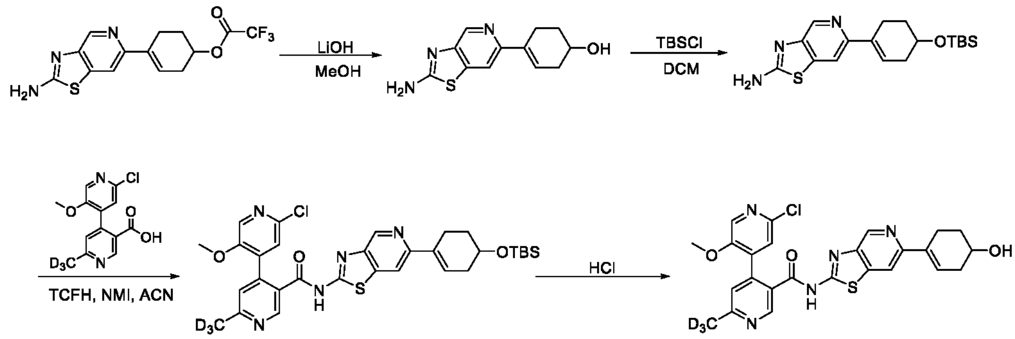


**Step 1: 4-(2-aminothiazolo[4,5-c]pyridin-6-yl)cyclohex-3-en-1-ol**

4-(2-aminothiazolo[4,5-c]pyridin-6-yl)cyclohex-3-en-1-yl2,2,2-trifluoroacetate (500 mg, 1.46 mmol) was dissolved in absolute methanol (10 mL); and lithium hydroxide (35 mg, 1.46 mmol) was added to allow for reaction at room temperature for 18 hours. After complete reaction, the reaction mixture was concentrated under reduced pressure and purified to obtain the title compound (white powder, 350 mg, yield: 97%). LC/MS (ESI) m/z: 247.9 $[M+H]^+$.

**Step 2: 6-(4-((tert-butyldimethylsilyl)oxy)cyclohex-1-en-1-yl)thiazolo[4,5-c]pyridin-2-amine**

4-(2-aminothiazolo[4,5-c]pyridin-6-yl)cyclohex-3-en-1-ol) (350 mg, 1.42 mmol) was dissolved in dichloromethane (10 mL); imidazole (483 mg, 7.1 mmol) and tert-butyldimethylchlorosilane (642 mg, 4.26 mmol) were added in sequence; and the mixture underwent reaction at room temperature overnight. A saturated ammonium chloride solution was added to the reaction mixture, which was then extracted with dichloromethane, washed with saturated saline, dried over anhydrous sodium sulfate, filtered, and concentrated; and the residue was purified by column chromatography (eluent: dichloromethane/methanol, gradient: 0%- 10% methanol) to obtain the target compound (white powder, 350 mg, yield: 68.4%). LC/MS (ESI) m/z: 362.1 $[M+H]^+$.

**Step 3: N-(6-(4-((tert-butyldimethylsilyl)oxy)cyclohex-1-en-1-yl)thiazolo[4,5-c]pyridin-2-yl)-2'-chloro-5'-methoxy-6-(methyl-d3)-[4,4'-bipyridine]-3-carboxamide**

6-(4-(tert-butyldimethylsiloxy)cyclohexyl-1-en-1-ylthiazolo[5, 4-b]pyridin-2-amine (50 mg, 0.14 mmol) and 2'-chloro-5'-methoxy-6-(methyl-d3)-[4,4'-bipyridine]-3-carboxylic acid (39 mg, 0.14 mmol) were dissolved in 3 mL of acetonitrile and 1 mL of N,N-dimethylformamide; N-methylimidazole (43 mg, 0.52 mmol) was added, and stirred for 10 minutes; and then, N,N,N',N'-tetramethylchlorformamidine hexafluorphosphate (39 mg, 0.14 mmol) was added, to allow for reaction at room temperature for 18 hours. Ethyl acetate and water were added to the reaction mixture for stratification; the organic phase was washed with saturated sodium chloride solution, dried over anhydrous sodium sulfate, and then filtered; and the filtrate was concentrated under reduced pressure to obtain residues, which were purified by column chromatography (eluent: petroleum ether/ethyl acetate, gradient: 30%-50% ethyl acetate) to obtain the title compound (white solid, 35 mg, yield: 40.5%). LC/MS (ESI) m/z: 625.3 $[M+H]^+$.

**Step 4: 2-chloro-6-(4-hydroxycyclohexyl-1-en-1-yl)thiazolopyridin-5,4-b-pyridin-2-yl-5'-methoxy-6-m ethyl-4, 4'-bipyridin-3-carboxamide**

N-(6-(4-((tert-butyldimethylsilyl)oxy)cyclohex-1-en-1-yl)thiazolo[4,5-c]pyridin-2-yl)-2'-chloro-5'-methoxy-6-(methyl-d3)-[4,4'-bipyridine]-3-carboxamide (40 mg, 0.064 mmol) was dissolved in 2 mL of dioxane; and 2 mL of 4 M dioxane hydrochloride solution was added to allow for reaction at room temperature for 3 hours. The reaction mixture was concentrated under reduced pressure; the residue was purified by pre-HPLC (eluent: acetonitrile/0.1% formic acid aqueous solution, gradient: 25%-90%) to obtain the title compound (white solid, 25 mg, yield: 76.4%). $^1H$ NMR (400 MHz,

DMSO-$d_6$) δ 13.08 (s, 1H), 8.93 (s, 1H), 8.87 (s, 1H), 8.16 (s, 1H), 8.10 (s, 1H), 7.57 (s, 1H), 7.45 (s, 1H), 6.67 - 6.63 (m, 1H), 4.69 (d, J = 3.6 Hz, 1H), 3.85 - 3.78 (m, 1H), 3.60 (s, 3H), 2.73 - 2.65 (m, 1H), 2.48 - 2.40 (m, 2H), 2.14 - 2.06 (m, 1H), 1.95 - 1.87 (m, 1H), 1.67 - 1.57 (m, 1H). LC/MS (ESI) m/z: 511.2 [M+H]$^+$.

**Example 111: 2'-chloro-N-(6-(4-hydroxycyclohex-1-en-1-yl-4-d)thiazolo[4,5-c]pyridin-2-yl)-5'-methox y-6-methyl-[4,4'-bipyridine]-3-carboxamide**

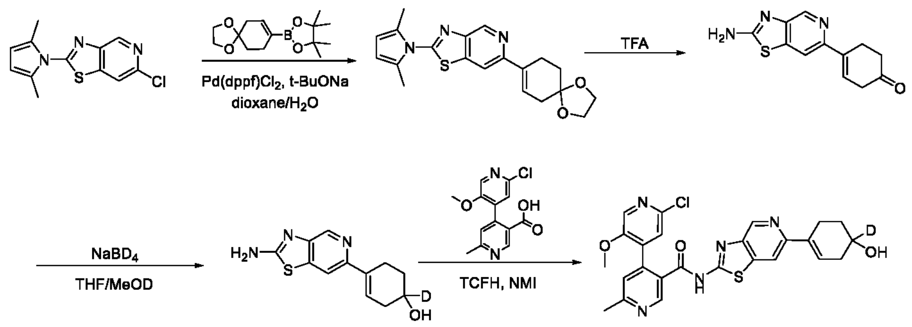


**Step 1: 2-(2,5-dimethyl-1H-pyrrol-1-yl)-6-(1,4-dioxyacetyl[4.5]dec-7-en-8-yl)thiazolo[4,5-c]pyridine**

6-chloro-2-(2,5-dimethyl-1H-pyrrol-1-yl)thiazolo[4,5-c]pyridine (1.7 g, 6.45 mmol) was dissolved in 1,4-dioxane (20 mL)/water (4 mL); 4,4,5,5-tetramethyl-2-(1,4-dioxypyrimidin[4.5]dec-7-en-8-yl)-1,3,2-dioxaborinan (2.57 g, 9.68 mmol), [1, 1'-bis(diphenylphosphino)ferrocen]palladium dichlorid (II) (94.4 mg, 0.129 mmol) and sodium tert-butoxide (1.86 g, 19.35 mmol) were added; and then, under the protection of nitrogen, the mixture was allowed to react at 95°C for 3 hours. The mixture was cooled to room temperature; saturated ammonium chloride was slowly added to the reaction mixture; and the mixture was then extracted with ethyl acetate, washed with saturated saline, concentrated under reduced pressure, and allowed to pass through the columns (eluent: ethyl acetate/petroleum ether = 1/1, V/V), to obtain the title compound (yellow solid, 1.2 g, yield: 50.6%). LC/MS (ESI) m/z: 368.2 [M+H]$^+$.

**Step 2: 4-(2-aminothiazolo[4,5-c]pyridin-6-yl)cyclohex-3-en-1-one**

2-(2,5-dimethyl-1H-pyrrol-1-yl)-6-(1,4-dioxyacetyl[4.5]dec-7-en-8-yl)thiazolo[4,5-c]pyridine (1.2 g, 3.27 mmol) was dissolved in trifluoroacetic acid (15 mL); two drops of water were added; and the mixture was refluxed to react for 30 minutes. After complete reaction, the reaction mixture was directly spin-dried, and subjected reversed-phase treatment (eluent: acetonitrile/water = 1/1, V/V) to obtain the title compound (yellow solid, 500 mg, yield: 62.5%). LC/MS (ESI) m/z: 246.1 [M+H]$^+$.

**Step 3: 4-(2-aminothiazolo[4,5-c]pyridin-6-yl)cyclohex-3-en-1-deuterium-1-ol**

4-(2-aminothiazolo[4,5-c]pyridin-6-yl)cyclohex-3-en-1-one (250 mg, 1.02 mmol) was dissolved in deuteromethanol (1 mL); 5 mL of tetrahydrofuran was added; in an ice bath, the mixture was stirred for 5 minutes; and then, sodium borodeuteride (51.4 mg, 1.22 mmol) was added to allow for reaction at room temperature for 30 minutes. After complete reaction, saturated ammonium chloride was added for quenching; the resulting mixture was extracted with ethyl acetate; the organic phase was washed with saturated sodium chloride, and dried; and the reaction mixture was directly spin-dried to obtain a crude title compound (yellow solid, 50 mg). LC/MS (ESI) m/z: 249.1 [M+H]$^+$.

**Step 4: 2'-chloro-N-(6-(4-hydroxycyclohex-1-en-1-yl-4-d)thiazolo[4,5-c]pyridin-2-yl)-5'-methoxy-6-me thyl-[4,4'-bipyridine]-3-carboxamide**

The crude product 4-(2-aminothiazolo[4,5-c]pyridin-6-yl)cyclohex-3-en-1-deuterium-1-ol (50 mg, 0.20 mmol), 2'-chloro-5'-methoxy-6-methyl-[4,4'-bipyridine]-3-carboxylic acid (84.2 mg, 0.30 mmol), and methylimidazole (0.5 mL) were dissolved in 10 mL of acetonitrile, and stirred at room temperature for 10 minutes; and then, N,N,N',N'-tetramethylchlorformamidine hexafluorphosphate (280.6 mg, 1.0 mmol) was added, to allow for reaction at room temperature for 1 hour. Saturated ammonium chloride was added for quenching; the resulting mixture was extracted with ethyl acetate; the organic phase was concentrated under reduced pressure, and purified by prep-HPLC (eluent: acetonitrile/0.1% trifluoroacetic acid in water = 0%-70%, V/V) to obtain the title compound (1.7 mg, white solid, yield: 1.7%). $^1$H NMR (400 MHz, DMSO-$d_6$) δ 13.18 (s, 1H), 8.98 (s, 1H), 8.86 (s, 1H), 8.18 (s, 1H), 8.17(s, 1H), 7.59 (s, 1H), 7.48 (s, 1H), 6.67 - 6.63 (m, 1H), 3.60 (s, 3H), 2.71 - 2.66 (m, 1H), 2.61 (s, 3H), 2.47 - 2.42 (m, 2H), 2.18 - 2.05 (m, 1H), 1.95 - 1.85 (m, 1H), 1.69 - 1.57 (m, 1H). LC/MS (ESI) m/z: 509.3 [M+H]$^+$.

**Example 112: 2'-chloro-N-(5-(4-hydroxypiperidin-1-yl-4-deuterium)thiazol[5,4-b]pyridin-2-yl)-5'-met hoxy-6-methyl-[4,4'-bipyridine]-3-carboxamide**

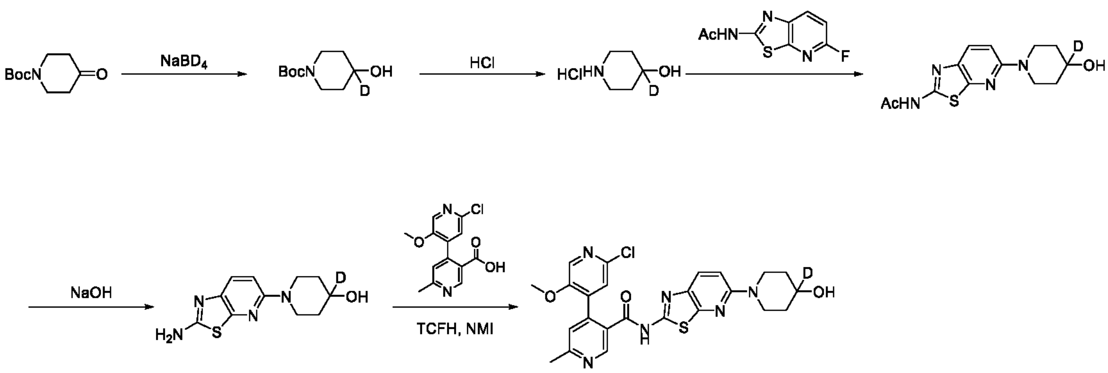


**Step 1: 4-hydroxypiperidin-1-tert-butyl carbamate-4-deuterium**

N-tert-butoxycarbonyl-4-piperidone (5 g, 25.1 mmol) was dissolved in 50 mL of methanol; under the protection of nitrogen, the mixture was cooled to 0-10°C; sodium borodeuteride (1.58 g, 37.6 mmol) was added portionwise, after which the ice bath was removed; and the mixture was stirred at room temperature for 2 hours. Water was added for quenching the reaction; the reaction mixture was concentrated to dryness under reduced pressure; water and dichloromethane were added for stratification; the aqueous phase was extracted with dichloromethane; and the organic phases were combined, washed with saturated saline, dried over anhydrous sodium sulfate, filtered, and concentrated to dryness under reduced pressure to obtain 4-hydroxypiperidin-1-tert-butyl carbamate-4-deuterium (4.5 g, yellow liquid, yield: 88.6%). LC/MS (ESI) m/z: 203.1 [M+H]$^+$.

**Step 2: piperidin-4-deuterium-4-ol hydrochloride**

4-hydroxypiperidin-1-tert-butyl formate-4-deuterium (4.5 g, 22.2 mmol) was dissolved in 20 mL of ethyl acetate; ethyl acetate hydrochloride (30 mL, 60.0 mmol) was added; and the mixture was stirred at room temperature for 18 hours. The reaction mixture was filtered, rinsed with ethyl acetate, and dried at 50°C in vacuum for 1 hour to obtain piperidin-4-deuterium-4-ol hydrochloride (3 g, white solid, yield: 38.9%). LC/MS (ESI) m/z: 103.1 [M+H]$^+$.

**Step 3: N-(5-(4-hydroxypiperidin-1-yl-4-deuterium)thiazol[5,4-b]pyridin-2-yl)acetamide**

N-(5-fluorothiazol[5,4-b]pyridin-2-yl)acetamide (250 mg, 1.18 mmol) and piperidin-4-deuterium-4-ol hydrochloride (328 mg, 2.37 mmol) were dissolved in 7 mL of N-methylpyrrolidone; N,N-diisopropylethylamine (765 mg, 5.92 mmol) was added; and the mixture was stirred at 120°C for 48 hours. The reaction mixture was concentrated under reduced pressure; the residue was purified by silica-gel column chromatography (eluent: dichloromethane/methanol = 20/1, V/V) to obtain N-(5-(4-hydroxypiperidin-1-yl-4-deuterium)thiazol[5,4-b]pyridin-2-yl)acetamide (135 mg, yellow solid, yield: 38.9%). LC/MS (ESI) m/z: 294.2 [M+H]$^+$.

**Step 4: 1-(2-aminothiazol[5,4-b]pyridin-5-yl)piperidin-4-deuterium-4-ol**

1-(2-aminothiazol[5,4-b]pyridin-5-yl)piperidin-4-deuterium-4-ol (135 mg, 0.460 mmol) and sodium hydroxide (184 mg, 4.60 mmol) were dissolved in 6 mL of methanol and 2 mL of water; and the mixture was stirred at 80°C for 3 hours. The reaction mixture was cooled to 0°C, adjusted to pH of 8-9 with 1 N hydrochloric acid, and concentrated under reduced pressure; and the residue was purified by silica-gel column chromatography (eluent: dichloromethane/methanol = 15/1, V/V) to obtain 1-(2-aminothiazol[5,4-b]pyridin-5-yl)piperidin-4-deuterium-4-ol (100 mg, yellow solid, yield: 86.5%). LC/MS (ESI) m/z: 252.2 [M+H]$^+$.

**Step 5: 2'-chloro-N-(5-(4-hydroxypiperidin-1-yl-4-deuterium)thiazol[5,4-b]pyridin-2-yl)-5'-methoxy-6 -methyl-[4,4'-bipyridine]-3-carboxamide**

1-(2-aminothiazol[5,4-b]pyridin-5-yl)piperidin-4-deuterium-4-ol (100 mg, 0.398 mmol), 2'-chloro-5'-methoxy-6-methyl-[4,4'-bipyridine]-3-carboxylic acid (111 mg, 0.398 mmol), and methylimidazole (98.1 mg, 1.20 mmol) were dissolved in 16 mL of acetonitrile and 4 mL of N,N-dimethylformamide, and stirred at 70°C for 20 minutes, then 4 mL of N,N,N',N'-tetramethylchlorformamidine hexafluorphosphate in acetonitrile solution (112 mg, 0.398 mmol) was added dropwise, and stirred at 70°C for 2 hours. The reaction mixture was concentrated under reduced pressure; water was added; the mixture was extracted with ethyl acetate; the organic phase was washed with water and with saline, and concentrated under reduced pressure; the residue was purified by prep-HPLC (eluent: acetonitrile/0.1% formic acid aqueous solution = 25%-90%, *V*/*V*) to obtain 2'-chloro-N-(5-(4-hydroxypiperidin-1-yl-4-deuterium)thiazol[5,4-b]pyridin-2-yl)-5'-methoxy-6-methyl-[4,4'-bipyridine]-3-carboxamide (100 mg, yellow solid, yield: 49.0%). $^1$H NMR (400 MHz, DMSO-$d_6$) $\delta$ 12.68 (s, 1H), 8.81 (s, 1H), 8.17 (s, 1H), 7.85 (d, $J$ = 9.1 Hz, 1H), 7.56 (s, 1H), 7.45 (s, 1H), 6.99 (d, $J$ = 9.1 Hz, 1H), 4.68 (s, 1H), 4.04 - 4.00 (m, 2H), 3.33 (s, 3H), 3.22 - 3.06 (m, 2H), 2.60 (s, 3H), 1.82 - 1.76 (m, 2H), 1.45 - 1.32 (m, 2H). LC/MS (ESI) m/z: 512.1 [M+H]$^+$.

**Example 113**: **2'-chloro-N-(7-fluoro-6-(4-hydroxypiperidin-1-yl-4-deuterium)benzoldlthiazol-2-yl)-5'-methoxy-6-methyl-[4,4'-bipyridine]-3-carboxamide**

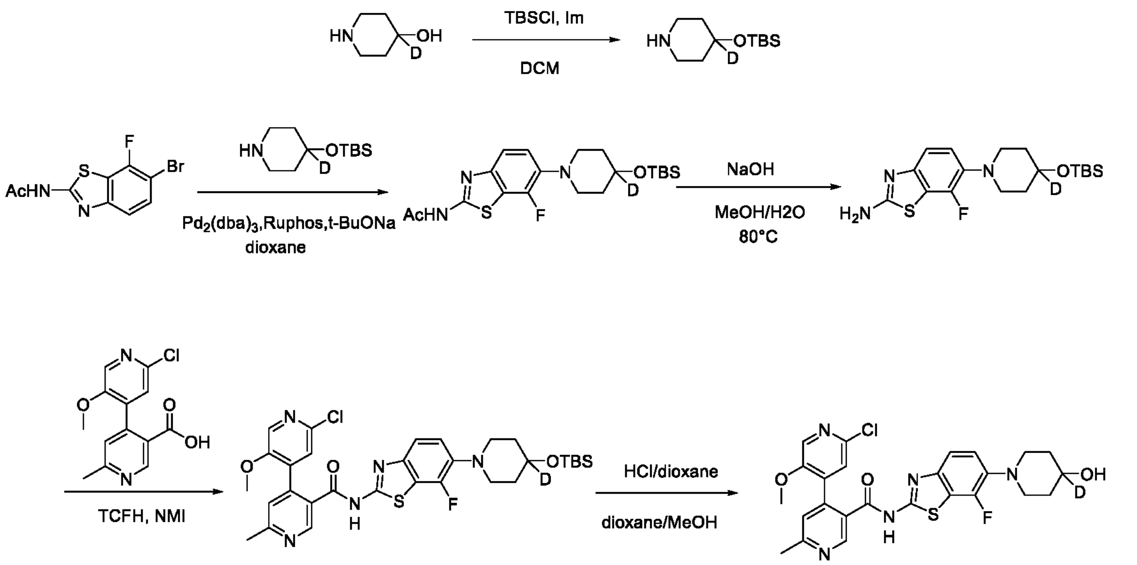


**Step 1: 4-((tert-butyldimethylsilicyl)oxy)piperidin-4-deuterium**

Piperidin-4-deuterium-4-ol (300 mg, 2.94 mmol) was dissolved in 20 mL of dichloromethane; at room temperature, imidazole (600.0 mg, 8.81 mmol) and tert-butyldimethylchlorosilane (443.1 mg, 2.94 mmol) were added; and the mixture was stirred at room temperature for 18 hours. 200 mL of dichloromethane was added; then, the mixture was repeatedly washed with water eight times; and the organic phase was dried, and concentrated under reduced pressure to obtain the title compound (350 mg, colorless oil, yield: 55.1%). LC/MS (ESI) m/z: 217.1 [M+H]$^+$.

**Step 2: N-(6-(4-((tert-butyldimethylsilyl)oxy)piperidin-1-yl-4-deuterium)-7-fluorobenzo[d]thiazol-2-yl) acetamide**

At room temperature, 4-((tert-butyldimethylsilicyl)oxy)piperidin-4-deuterium (250 mg, 1.16 mmol), N-(6-bromo-7-fluorobenzo[d]thiazol-2-yl)acetamide (400.8 mg, 1.39 mmol), bis(dibenzalacetone)palladium (II) (106.2 mg, 0.116 mmol), 2-dicyclohexylphosphino-2',6'-diisopropoxy-1,1'-biphenyl (108.3 mg, 0.232 mmol), and sodium tert-butoxide (334.4 mg, 3.48 mmol) were dissolved in the solution of anhydrous 1, 4-dioxane (20 mL), and nitrogen replacement was performed three times; and the mixture was stirred at 100°C for 1 hour. After the reaction was completed, the reaction mixture was directly concentrated under reduced pressure and treated by column chromatography (eluent: petroleum ether/ethyl acetate = 1/1, V/V) to obtain the title compound (faint yellow solid, 380 mg, yield: 77.5%). LC/MS (ESI) m/z:

425.1 [M+H]+.

**Step 3: 6-(4-((tert-butyldimethylsilicyl)oxy)piperidin-1-yl-4-deuterium)-7-fluorobenzo[d]thiazol-2-am ine**

N-(6-(4-((tert-butyldimethylsilyl)oxy)piperidin-1-yl-4-deuterium)-7-fluorobenzo[d]thiazol-2-yl)acetamide (380 mg, 0.895 mmol) and sodium hydroxide (179 mg, 4.47 mmol) were dissolved in 20 mL of methanol and 8 mL of water; and the mixture was stirred at 80°C for 3 hours. 100 mL of ethyl acetate was added; the mixture was washed with water twice; and the organic phase was dried, and concentrated under reduced pressure to obtain the title compound (300 mg, yellow solid, yield: 88.7%). LC/MS (ESI) m/z: 383.2 [M+H]+.

**Step 4: N-(6-(4-((tert-butyldimethylsilicyl)oxy)piperidin-1-yl-4-deuterium)-7-fluorobenzo[d]thiazol-2-yl)-2'-chloro-5'-methoxy-6-methyl-[4,4'-bipyridine]-3-carboxamide**

6-(4-((tert-butyldimethylsilicyl)oxy)piperidin-1-yl-4-deuterium)-7-fluorobenzo[d]thiazol-2-amine (300 mg, 0.784 mmol), 2'-chloro-5'-methoxy-6-methyl-[4,4'-bipyridine]-3-carboxylic acid (327.8 mg, 1.18 mmol), and methylimidazole (321.8 mg, 3.92 mmol) were dissolved in 16 mL of acetonitrile and 2 mL of N,N-dimethylformamide, and stirred at 75°C for 5 minutes; and N,N,N',N'-tetramethylchlorformamidine hexafluorphosphate (1.1 g, 3.92 mmol) was added, and stirred at 75°C for 30 minutes. Saturated ammonium chloride was added; the mixture was extracted with ethyl acetate; the organic phase was washed with water, washed with saline, concentrated under reduced pressure, and treated by column chromatography (eluent: dichloromethane/methanol = 10/1, *V*/*V*) to obtain the title compound (faint yellow solid, 300 mg, yield: 59.5%). LC/MS (ESI) m/z: 643.2 [M+H]+.

**Step 5: 2'-chloro-N-(7-fluoro-6-(4-hydroxypiperidin-1-yl-4-deuterium)benzo[d]thiazol-2-yl)-5'-metho xy-6-methyl-[4,4'-bipyridine]-3-carboxamide**

N-(6-(4-((tert-butyldimethylsilicyl)oxy)piperidin-1-yl-4-deuterium)-7-fluorobenzo[d]thiazol-2-yl)-2'-chloro-5'-methoxy-6-methyl-[4,4'-bipyridine]-3-carboxamide (300 mg, 0.466 mmol) was dissolved in 2 mL of methanol; in an ice bath, 4M hydrochloric acid-1,4-dioxane solution (10 mL) was added, and the resulting mixture was stirred at room temperature for 30 minutes; after complete reaction, the reaction mixture was concentrated; the residue was purified by prep-HPLC (eluent: acetonitrile/0.1 % formic acid aqueous solution = 20%-90%, V/V) to obtain 2'-chloro-N-(7-fluoro-6-(4-hydroxypiperidin-1-yl-4-deuterium)benzo[d]thiazol-2-yl)-5'-methoxy-6-methyl-[ 4,4'-bipyridine]-3-carboxamide (100 mg, faint yellow solid, yield: 40.5%). $^1H$ NMR (400 MHz, DMSO-$d_6$) δ 13.02 (s, 1H), 8.84 (s, 1H), 8.17 (s, 1H), 7.58 (s, 1H), 7.53 (d, *J* = 8.6 Hz, 1H), 7.46 (s, 1H), 7.22 (t, *J* = 8.7 Hz, 1H), 4.67 (s, 1H), 3.60 (s, 3H), 3.30 - 3.22 (m, 2H), 2.83 (t, *J* = 9.5 Hz, 2H), 2.59 (s, 3H), 1.90 -1.80 (m, 2H), 1.61 - 1.50 (m, 2H). LC/MS (ESI) m/z: 529.2 [M+H]+.

**Example 114 and Example 115: (R)-2'-chloro-N-(6-(4-hydroxycyclohex-1-en-1-yl)thiazolo[4,5-c]pyri din-2-yl)-5'-methoxy-6-methyl-[4.4'-bipyridine]-3-carboxamide (Example 114) and (S) -2'-chloro-N-(6-(4-hydroxycyclohex-1-en-1-yl)thiazolo[4,5-c]pyridin-2-yl)-5'-methoxy-6-methyl-[4,4'-bipyridine]-3-c arboxamide (Example 115)**

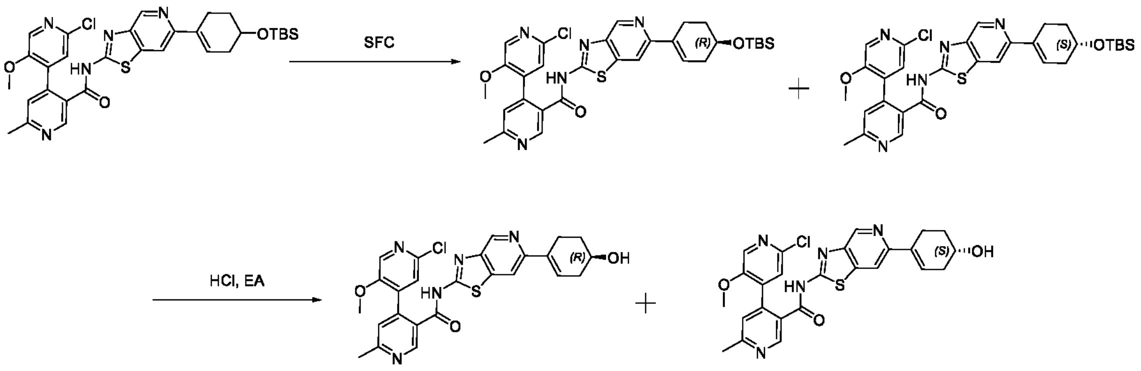

**Step 1: (R)-N-(6-(4-((tert-butyldimethylsilyl)oxy)cyclohex-1-en-1-yl)thiazolo[4,5-c]pyridin-2-yl)-2'-chl oro-5'-methoxy-6-methyl-[4,4'-bipyridine]-3-carboxamide and (S)-N-(6-(4-((tert-butyldimethylsilyl)o xy)cyclohex-1-en-1-yl)thiazolo[4.5-c]pyridin-2-yl)-2'-chloro-5'-methoxy-6-methyl-[4.4'-bipyridine]-3-c arboxamide**

N-(6-(4-((tert-butyldimethylsilyl)oxy)cyclohex-1-en-1-yl)thiazol[5,4-b]pyridin-2-yl)-2'-chloro-5'-methoxy-6 -methyl-[4,4'-bipyridine]-3-carboxamide (160 mg, 0.257 mmol) was separated by chiral resolution (OX chromatographic column, 30% methanol (containing 0.05% diethylamide: 70% supercritical carbon dioxide, 8 minutes), and then concentrated respectively to obtain (R)-N-(6-(4-((tert-butyldimethylsilyl)oxy)cyclohex-1-en-1-yl)thiazolo[4,5-c]pyridin-2-yl)-2'-chloro-5'-metho xy-6-methyl-[4,4'-bipyridine]-3-carboxamide (60 mg, 0.193 mmol, Rt = 4.046 min, yield: 37.5%) LC/MS (ESI) m/z: 623.2 $[M+H]^+$and (S)-N-(6-(4-((tert-butyldimethylsilyl)oxy)cyclohex-1-en-1-yl)thiazolo[4,5-c]pyridin-2-yl)-2'-chloro-5'-metho xy-6-methyl-[4,4'-bipyridine]-3-carboxamide (60 mg, 0.193 mmol, Rt=4.789 min, yield: 37.5% ) LC/MS (ESI) m/z: 623.2 $[M+H]^+$.

**Step 2: (R)-2'-chloro-N-(6-(4-hydroxycyclohex-1-en-1-yl)thiazolo[4,5-c]pyridin-2-yl)-5'-methoxy-6-me thyl-[4,4'-bipyridine]-3-carboxamide and (S) -2'-chloro-N-(6-(4-hydroxycyclohex-1-en-1-yl)thiazolo [4,5-c]pyridin-2-yl)-5'-methoxy-6-methyl-[4,4'-bipyridine]-3-carboxamide**

(R)-N-(6-(4-((tert-butyldimethylsilyl)oxy)cyclohex-1-en-1-yl)thiazolo[4,5-c]pyridin-2-yl)-2'-chloro-5'-metho xy-6-methyl-[4,4'-bipyridine]-3-carboxamide (50 mg, 0.18 mmol) was dissolved in 2 mL of acetonitrile; 2 mL of ethyl acetate -hydrochloric acid solution was added; and the mixture was stirred at room temperature for 3 hours. The reaction mixture was concentrated under reduced pressure; the residue was purified by pre-HPLC (eluent: acetonitrile/0.1% formic acid aqueous solution, gradient: 25%-90%) to obtain the title compound (white solid, 25 mg, yield: 61.2%). $^1$H NMR (400 MHz, DMSO-$d_6$)δ 13.08 (s, 1H), 8.94 (s, 1H), 8.86 (s, 1H), 8.18 (s, 1H), 8.12 (s, 1H), 7.59 (s, 1H), 7.47 (s, 1H), 6.66 - 6.62 (m, 1H), 4.74 - 4.62 (m, 1H), 3.85 - 3.78 (m, 1H), 3.61 (s, 3H), 2.72 - 2.65 (m, 1H), 2.61 (s, 3H), 2.52 - 2.43 (m, 2H), 2.15 - 2.05 (m, 1H), 1.96 - 1.88 (m, 1H), 1.69 - 1.57 (m, 1H). LC/MS (ESI) m/z: 508.2 $[M+H]^+$.

(S)-N-(6-(4-((tert-butyldimethylsilyl)oxy)cyclohex-1-en-1-yl)thiazolo[4,5-c]pyridin-2-yl)-2'-chloro-5'-metho xy-6-methyl-[4,4'-bipyridine]-3-carboxamide (50 mg, 0.18 mmol) was dissolved in 2 mL of acetonitrile; 2 mL of ethyl acetate -hydrochloric acid solution was added; and the mixture was stirred at room temperature for 3 hours. The reaction mixture was concentrated under reduced pressure; the residue was purified by pre-HPLC (eluent: acetonitrile/0.1% formic acid aqueous solution, gradient: 25%-90%) to obtain the title compound (white solid, 25 mg, yield: 61.2%). $^1$H NMR (400 MHz, DMSO-$d_6$)δ 13.07 (s, 1H), 8.93 (s, 1H), 8.85 (s, 1H), 8.17 (s, 1H), 8.12 (s, 1H), 7.58 (s, 1H), 7.47 (s, 1H), 6.67 - 6.63 (m, 1H), 4.73 - 4.62 (m, 1H), 3.84 - 3.78 (m, 1H), 3.60 (s, 3H), 2.72 - 2.65 (m, 1H), 2.61 (s, 3H), 2.52 - 2.43 (m, 2H), 2.16 - 2.06 (m, 1H), 1.96 - 1.88 (m, 1H), 1.67 - 1.57 (m, 1H). LC/MS (ESI) m/z: 508.2 $[M+H]^+$.

**Example 116**: **2'-chloro-N-(5-(4-hydroxy-3,3-dimethylpiperidin-1-yl)thiazol[5,4-b]pyridin-2-yl)-5'-me thoxy-6-methyl-[4,4'-bipyridine]-3-carboxamide**

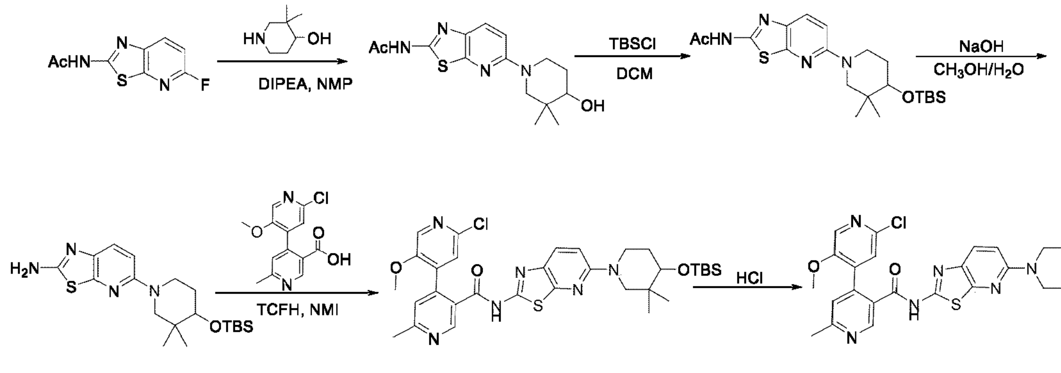


**Step 1: N-(5-(4-hydroxy-3,3-dimethylpiperidin-1-yl)thiazol[5,4-b]pyridin-2-yl)acetamide**

*N*-(5-fluorothiazol[5,4-b]pyridin-2-yl)acetamide (500 mg, 2.37 mmol) and 3,3-dimethylpiperidin-4-ol (459 mg, 3.55 mmol) were dissolved in 1-methyl-2-pyrrolidone (10 mL); *N,N*-diisopropylethylamine (2.14 g, 16.6 mmol) was added; and the mixture was allowed to react at 120°C for 48 hours. After complete reaction, the reaction mixture was extracted with water and ethyl acetate; then, the organic phase was washed with saturated saline; and the residue was purified by column

chromatography (eluent: dichloromethane/methanol, gradient: 0%-10% methanol) to obtain the title compound (400 mg, white solid, yield: 52.6%). LC/MS (ESI) m/z: 321.0 [M+H]$^+$.

**Step 2: N-(5-(4-((tert-butyldimethylsilicyl)oxy)-3,3-dimethylpiperidin-1-yl)thiazolo[5,4-b]pyridin-2-yl) acetamide**

N-(5-(4-hydroxy-3,3-dimethylpiperidin-1-yl)thiazol[5,4-b]pyridin-2-yl)acetamide (300 mg, 0.936 mmol) was dissolved in dichloromethane (5 mL); imidazole (637 mg, 9.36 mmol) and tert-butyldimethylchlorosilane (1.13 g, 7.49 mmol) were added; and the mixture was stirred at room temperature for 12 hours. After complete reaction, the reaction mixture was extracted with water and ethyl acetate; then, the organic phase was washed with saturated saline; and the residue was purified by column chromatography (eluent: petroleum ether/ethyl acetate, gradient: 10%-50% ethyl acetate) to obtain the title compound (200 mg, white solid, yield: 49.1%). LC/MS (ESI) m/z: 435.3 [M+H]$^+$.

**Step 3: 5-(4-((tert-butyldimethylsilicyl)oxy)-3,3-dimethylpiperidin-1-yl)thiazolo[5,4-b]pyridin-2-amine**

N-(5-(4-((tert-butyldimethylsilicyl)oxy)-3,3-dimethylpiperidin-1-yl)thiazolo[5,4-b]pyridin-2-yl)acetamide (200 mg, 0.460 mmol) and sodium hydroxide (184 mg, 4.60 mmol) were dissolved in methanol/water (6/2 mL), and heated to 80°C to react for 2 hours. After complete reaction, the reaction mixture was concentrated under reduced pressure; and the residue was purified by column chromatography (eluent: dichloromethane/methanol, gradient: 0%-10% methanol) to obtain the title compound (150 mg, white solid, yield: 83.0%).LC/MS (ESI) m/z: 393.3 [M+H]$^+$.

**Step 4: N-(5-(4-((tert-butyldimethylsilicyl)oxy)-3,3-dimethylpiperidin-1-yl)thiazolo[5,4-b]pyridin-2-yl) -2'-chloro-5'-methoxy-6-methyl-[4,4'-bipyridine]-3-carboxamide**

2'-chloro-5'-methoxy-6-methyl-[4,4'-bipyridine]-3-carboxylic acid (17.2 mg, 0.062 mmol), 5-(4-((tert-butyldimethylsilicyl)oxy)-3,3-dimethylpiperidin-1-yl)thiazolo[5,4-b]pyridin-2-amine (29.0 mg, 0.074 mmol) and 1-methylimidazole (22.2 mg, 0.277 mmol) were dissolved in acetonitrile/*N,N*-dimethylformamide (3/0.5 mL), and stirred at 75°C for 15 minutes. Then, *N, N, N', N'*-tetramethylchlorformamidine hexafluorphosphate (25.9 mg, 0.092 mmol) dissolved in acetonitrile (0.5 mL) was added; and the resulting mixture was stirred at 75°C for 1 hour. After complete reaction, water was added; the resulting mixture was extracted with ethyl acetate; and the organic phase was washed with saturated saline, dried over anhydrous sodium sulfate, and filtered; and the filtrate was concentrated under reduced pressure. The residue was purified by column chromatography (eluent: dichloromethane/methanol, gradient: 0%-10% methanol) to obtain the title compound (30 mg, yellow solid, yield: 74.6%). LC/MS (ESI) m/z: 653.3 [M+H]$^+$.

**Step 4: 2'-chloro-3'-fluoro-N-(6-(4-hydroxypiperidin-1-yl)thiazolo[4.5-b]pyridin-2-yl)-5'-methoxy-6-methyl-[4,4'-bipyridine]-3-carboxamide**

N-(5-(4-((tert-butyldimethylsilicyl)oxy)-3,3-dimethylpiperidin-1-yl)thiazolo[5,4-b]pyridin-2-yl)-2'-chloro-5'-methoxy-6-methyl-[4,4'-bipyridine]-3-carboxamide (30 mg, 0.046 mmol) was dissolved in hydrochloric acid in dioxane solution (3 mL); and the resulting mixture was allowed to react at room temperature for 2 hours. After complete reaction, the reaction mixture was concentrated under reduced pressure; and the residue was purified by reversed-phase preparation to obtain the title compound (18 mg, yellow solid, yield: 72.7%). $^1$H NMR (400 MHz, DMSO-$d_6$) δ 12.64 (s, 1H), 8.81 (s, 1H), 8.16 (s, 1H), 7.82 - 7.78 (m, 1H), 7.55 (s, 1H), 7.44 (s, 1H), 7.00-6.96 (m, 1H), 4.66 - 4.62 (m, 1H), 4.12 - 4.04 (m, 1H), 3.77 - 3.71 (m, 1H), 3.62 (s, 3H), 3.30 - 3.27(m, 1H), 3.11 - 3.05 (m, 1H), 2.86 - 2.79 (m, 1H), 2.59 (s, 3H), 1.77 - 1.65 (m, 1H), 1.58 - 1.45 (m, 1H), 0.93 (s, 3H), 0.82 (s, 3H). LC/MS (ESI) m/z: 539.2 [M+H]$^+$.

The example compounds in Table 9 were synthesized using commercially available raw materials with reference to the synthesis steps of Example **116**.

Table 9:

| | Chemical structure | LC/MS( ESI) m/z [M+H]$^+$ | $^1$H NMR |
|---|---|---|---|
| 117 | 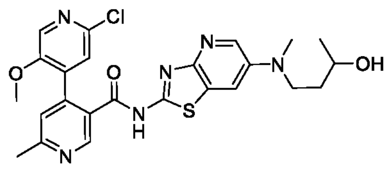 | 513 | $^1$H NMR (400 MHz, DMSO-$d_6$) δ 8.85 (s, 1H), 8.16 (s, 1H), 8.13 (d, *J* = 2.8 Hz, 1H), 7.64 (d, *J* = 2.8 Hz, 1H), 7.55 (s, 1H), 7.43 (s, 1H), 4.53 (d, *J* = 4.8 Hz, 1H), 3.69-3.63 (m, 1H), 3.61 (s, 3H), 3.50-3.39 (m, 2H), 2.93 (s, 3H), 2.59 (s, 3H), 1.66-1.56 (m, 1H), 1.56-1.45 (m, 1H), 1.09 (d, *J* = 6.2 Hz, 3H). |

(continued)

| | Chemical structure | LC/MS( ESI) m/z $[M+H]^+$ | $^1H$ NMR |
|---|---|---|---|
| 118 | 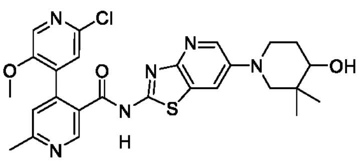 | 539.2 | $^1H$ NMR (400 MHz, DMSO-$d_6$) δ 12.94 (s, 1H), 8.85 (s, 1H), 8.31 (d, *J* = 2.5 Hz, 1H), 8.16 (s, 1H), 7.90 (d, *J* = 2.4 Hz, 1H), 7.57 (s, 1H), 7.44 (s, 1H), 4.63 (d, *J* = 4.6 Hz, 1H), 3.60 (s, 3H), 3.53 - 3.43 (m, 1H), 3.29 - 3.20 (m, 2H), 2.90 - 2.81 (m, 1H), 2.61 (s, 1H), 2.60 (s, 3H), 1.80 - 1.71 (m, 1H), 1.71 - 1.58 (m, 1H), 0.96 (s, 3H), 0.94 (s, 3H). |
| 119 | 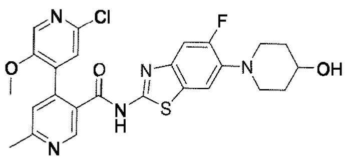 | 528.2 | $^1H$NMR(400MHz,DMSO-$d_6$) *δ* 12.86 (s, 1H), 8.82 (s, 1H), 8.16 (s, 1H), 7.65 (d, *J* = 8.5 Hz, 1H), 7.57 (s, 1H), 7.54 (d, *J* = 12.9 Hz, 1H), 7.45 (s, 1H), 4.71 (s, 1H), 3.65 - 3.61 (m, 1H), 3.59 (s, 3H), 3.26 - 3.22 (m, 2H), 2.7 2 - 2.68(m, 2H), 2.60 (s, 3H), 1.88 - 1.84 (m, 2H), 1.59 - 1.55 (m, 2H) |
| 120 | 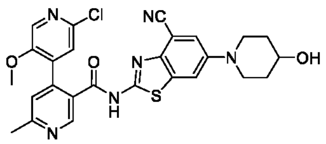 | 535.2 | $^1H$ NMR (400 MHz, DMSO-$d_6$) δ 13.21 (s, 1H), 8.86 (s, 1H), 8.16 (s, 1H), 7.86 (s, 1H), 7.57 (s, 2H), 7.45 (s, 1H), 4.70 (d, *J* = 4.0 Hz, 1H), 3.70 - 3.63 (m, 1H), 3.60 (s, 3H), 3.59 - 3.56 (m, 1H), 3.31 - 3.27 (m, 1H), 3.02 - 2.89 (m, 2H), 2.60 (s, 3H), 1.88 - 1.76 (m, 2H), 1.54 - 1.42 (m, 2H). |
| 121 | 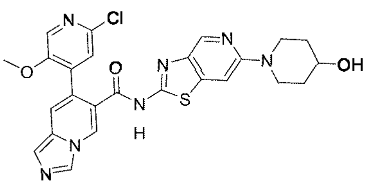 | 536.2 | $^1H$ NMR ( 400MHz, DMSO-$d_6$) δ 12.66 (s, 1H), 8.93 (s, 1H), 8.59 (s, 1H), 8.08 (s, 1H), 7.76 (s, 1H), 7.56 (d, J = 5.3 Hz, 2H), 6.99 (d, J = 9.1 Hz, 1H), 4.78 - 4.62 (m, 1H), 4.08 - 3.97 (m, 2H), 3.76 - 3.67 (m, 1H), 3.60 (s, 3H), 3.17 - 3.08 (m,2H), 1.85 - 1.75 (m, 2H), 1.43 - 1.32 (m, 2H) |
| 122 | 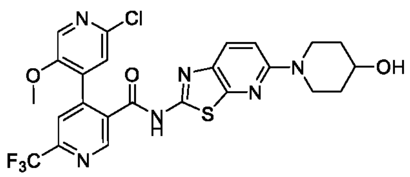 | 565.1 | $^1H$ NMR (400 MHz, DMSO-$d_6$) *δ* 12.94 (s, 1H), 9.15 (s, 1H), 8.22 (s, 1H), 8.14 (s, 1H), 7.87 (d, *J* = 9.1 Hz, 1H), 7.75 (s, 1H), 7.01 (d, *J* = 9.2 Hz, 1H), 4.71 (d, *J* = 4.2 Hz, 1H), 4.05 - 4.01 (m, 2H), 3.75 - 3.69 (m, 1H), 3.64 (s, 3H), 3.20 - 3.09 (m, 2H), 1.83 - 1.79 (m, 2H), 1.45 - 1.32 (m, 2H). |
| 123 | 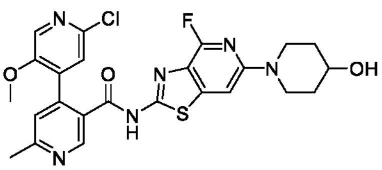 | 529.2 | $^1H$ NMR (400 MHz, DMSO-$d_6$) δ 13.01 (s, 1H), 8.83 (s, 1H), 8.17 (s, 1H), 7.57 (s, 1H), 7.45 (s, 1H), 7.24 (s, 1H), 4.71 (d, *J* = 4.1 Hz, 1H), 3.96 - 3.85 (m, 2H), 3.74 - 3.66 (m, 1H), 3.61 (s, 3H), 3.17 - 3.05 (m, 2H), 2.59 (s, 3H), 1.84 - 1.73 (m, 2H), 1.44 - 1.32 (m, 2H). |
| 124 | 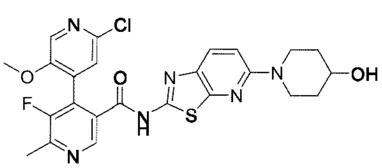 | 529.1 | $^1H$ NMR (400 MHz, DMSO- $d_6$) δ 12.83 (s, 1H), 8.77 (s, 1H), 8.27 (s, 1H), 7.84 (d, J = 9.1 Hz, 1H), 7.58 (s, 1H), 6.99 (d, J = 9.1 Hz, 1H), 4.70 (d, J = 4.2 Hz, 1H), 4.06 - 3.97 (m, 2H), 3.74 - 3.70(m, 1 H), 3.70 (s, 3H), 3.17 - 3.09 (m, 2H), 2.59 - 2.57 (m, 3H), 1.84 - 1.76 (m, 2H), 1.43 - 1.33 (m, 2H). |
| 125 | 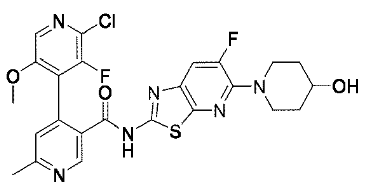 | 547.2 | $^1H$ NMR (400 MHz, DMSO-$d_6$) δ 9.04 (s, 1H), 8.16 (s, 1H), 7.84 (d, *J* = 13.7 Hz, 1H), 7.42 (s, 1H), 4.73 - 4.68 (m, 1H), 3.72 (s, 3H), 3.71-3.64 (m, 3H), 3.05 - 2.98 (m, 2H), 2.59 (s, 3H), 1.88 - 1.82 (m, 2H), 1.55-1.48 (m,2H). |

(continued)

| | Chemical structure | LC/MS( ESI) m/z [M+H]+ | 1H NMR |
|---|---|---|---|
| 126 | 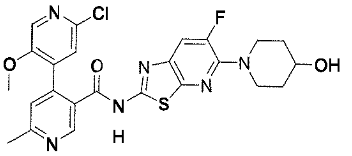 | 529.2 | 1H NMR (400 MHz, DMSO-$d_6$) δ 12.85 (s, 1H), 8.84 (s, 1H), 8.16 (s, 1H), 7.92 (d, *J* = 13.6 Hz, 1H), 7.55 (s, 1H), 7.43 (s, 1H), 4.72 (d, *J* = 3.8 Hz, 1H), 3.75 - 3.65 (m, 3H), 3.61 (s, 3H), 3.09 - 2.99 (m, 2H), 2.59 (s, 3H), 1.90 - 1.80 (m, 2H), 1.58 - 1.43 (m, 2H). |
| 127 | 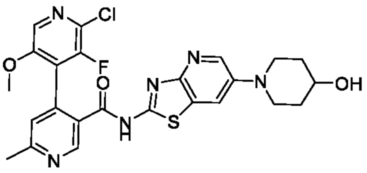 | 529.1 | 1H NMR (400 MHz, DMSO-$d_6$) δ 13.07(s, 1 H), 9.03 (s, 1H), 8.32 (s, 1H), 8.17 (s, 1H), 7.89 (s, 1H), 7.47 (s, 1H), 4.70 (d, J = 3.8 Hz, 1H), 3.72 (s, 3H), 3.67 - 3.61(s,1 H), 3.57 - 3.51 (m, 2H), 2.95 - 2.87 (m, 2H), 2.60 (s, 3H), 1.88 - 1.81 (m, 2H), 1.56 - 1.45 (m, 2H). |
| 128 | 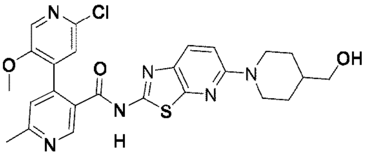 | 525.3 | 1H NMR (400 MHz, DMSO-$d_6$) δ 12.67 (s, 1H), 8.81 (s, 1H), 8.16 (s, 1H), 7.83 (d, *J* = 8.8 Hz, 1H), 7.55 (s, 1H), 7.44 (s, 1H), 6.98 (d, *J* = 9.2 Hz, 1H), 4.47 (t, *J* = 5.2 Hz, 1H), 4.36 - 4.25 (m, 2H), 3.62 (s, 3H), 3.28 (t, *J* = 5.8 Hz, 2H), 2.88 - 2.78 (m, 2H), 2.59 (s, 3H), 1.78 - 1.70 (m, 2H), 1.67 - 1.57 (m, 1H), 1.19 - 1.08 (m, 2H). |
| 129 | 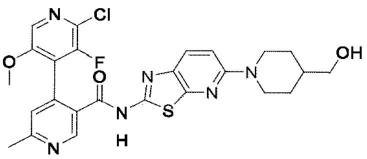 | 543.3 | 1H NMR (400 MHz, DMSO-$d_6$) δ 12.81 (s, 1H), 8.96 (s, 1H), 8.18 (s, 1H), 7.85 (d, *J* = 9.2 Hz, 1H), 7.50 (s, 1H), 6.99 (d, *J* = 9.2 Hz, 1H), 4.47 (t, *J* = 5.2 Hz, 1H), 4.35 - 4.27 (m, 2H), 3.73 (s, 3H), 3.27 (t, *J* = 5.8 Hz, 2H), 2.88 - 2.76 (m, 2H), 2.60 (s, 3H), 1.78 - 1.70 (m, 2H), 1.68 - 1.55 (m, 1H), 1.20 - 1.06 (m, 2H). |
| 130 | 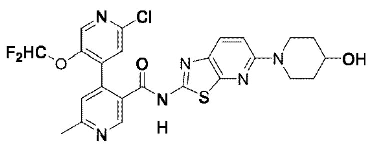 | 547.3 | 1H NMR (400 MHz, DMSO-$d_6$) δ 12.83 (s, 1H), 8.96 (s, 1H), 8.33 (s, 1H), 7.85 (d, *J* = 8.8 Hz, 1H), 7.74 (s, 1H), 7.47 (s, 1H), 7.14 (t, *J* = 72.4 Hz, 1H), 7.00 (d, *J* = 9.2 Hz, 1H), 4.70 (d, *J* = 4.4 Hz, 1H), 4.06 - 3.97 (m, 2H), 3.74 - 3.66 (m, 1H), 3.18 - 3.10 (m, 2H), 2.62 (s, 3H), 1.85 - 1.74 (m, 2H), 1.43 - 1.32 (m, 2H). |
| 131 | 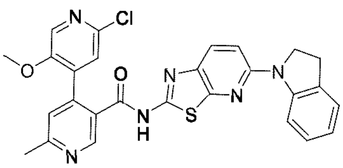 | 529.3 | 1H NMR (400 MHz, DMSO-$d_6$) δ 12.82 (s, 1H), 8.85 (s, 1H), 8.26 (d, *J* = 8.1 Hz, 1H), 8.17 (s, 1H), 8.05 (d, *J* = 8.9 Hz, 1H), 7.58 (s, 1H), 7.46 (s, 1H), 7.22 (d, *J* = 7.0 Hz, 1H), 7.16 (t, *J* = 7.7 Hz, 1H), 7.02 (d, *J* = 9.1 Hz, 1H), 6.87 (t, *J* = 7.3 Hz, 1H), 4.09 (t, *J* = 8.5 Hz, 2H), 3.64 (s, 3H), 3.22 (t, *J* = 8.5 Hz, 2H), 2.61 (s, 3H). |
| 132 | 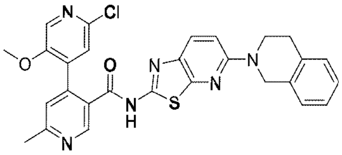 | 543.3 | 1H NMR (400 MHz, DMSO-$d_6$) δ 12.71 (s, 1H), 8.82 (s, 1H), 8.17 (s, 1H), 7.92 (d, *J* = 9.0 Hz, 1H), 7.57 (s, 1H), 7.45 (s, 1H), 7.29 -7.27 (m, 1H), 7.21 -7.18 (m, 3H), 7.05 (d, *J* = 9.1 Hz, 1H), 4.73 (s, 2H), 3.85 (t, *J* = 5.8 Hz, 2H), 3.62 (s, 3H), 2.93 (t, *J* = 5.6 Hz, 2H), 2.60 (s, 3H). |
| 133 | 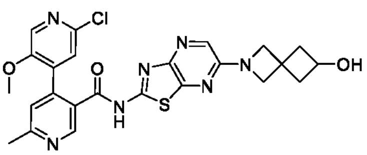 | 524.2 | 1H NMR (400 MHz, $CDCl_3$) δ 8.88 (s, 1H), 8.04 (s, 1H), 7.54 (s, 1H), 7.16 (s, 1H)7.15 (s, 1H), 4.34 - 4.28 (m, 1H), 4.10 (d, *J* = 5.6 Hz, 4H), 3.74 (s, 3H), 3.68 (s, 3H), 2.67 - 2.60 (m, 3H), 2.24 - 2.13 (m, 2H). |

(continued)

| | Chemical structure | LC/MS( ESI) m/z $[M+H]^+$ | $^1$H NMR |
|---|---|---|---|
| 134 | 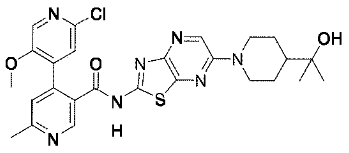 | 554.3 | $^1$H NMR (400 MHz, DMSO-$d_6$) δ 12.95 (s, 1H), 8.83 (s, 1H), 8.33 (s, 1H), 8.17 (s, 1H), 7.58 (s, 1H), 7.46 (s, 1H), 4.43 (d, *J* = 12.8 Hz, 2H), 4.15 (s, 1H), 3.62 (s, 3H), 2.87 - 2.78 (m, 2H), 2.60 (s, 3H), 1.81 (d, *J* = 11.6 Hz, 2H), 1.50 - 1.42 (m, 1H), 1.30 - 1.20 (m, 2H), 1.05 (s, 6H). |
| 135 | 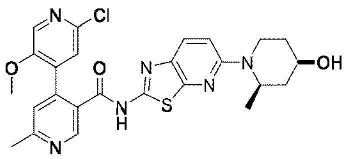 | 525.2 | $^1$H NMR (400 MHz, DMSO-$d_6$) δ 12.55(s, 1 H), 8.81 (s, 1H), 8.16 (s, 1H), 7.82 (d, *J* = 9.1 Hz, 1H), 7.55 (s, 1H), 7.44 (s, 1H), 6.90 (d, *J* = 9.2 Hz, 1H), 4.65 - 4.62 (m, 1H), 4.49 - 4.40 (m, 1H), 4.05 - 3.95 (m, 2H), 3.62 (s, 3H), 3.29 - 3.26 (m, 1H), 2.59 (s, 3H), 1.84 - 1.76 (s, 1H), 1.72 - 1.65 (s, 3H), 1.27 (d, *J* = 6.8 Hz, 3H). |
| 136 | 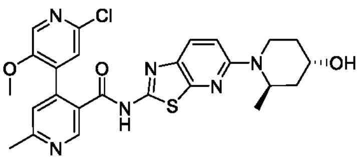 | 525.2 | $^1$H NMR (400 MHz, DMSO-$d_6$) δ 12.74 (s, 1H), 8.87 (s, 1H), 8.18 (s, 1H), 7.87 (d, *J* = 8.5 Hz, 1H), 7.58 (s, 1H), 7.54 (s, 1H),6.98 (s, 1H), 4.73 - 4.65 (m, 1H), 4.23 - 4.15 (m, 1H), 3.91 - 3.85 (m, 1H), 3.62 (s, 3H), 3.01 - 2.91 (m, 1H), 2.63 (s, 3H), 1.98 - 1.80 (m, 2H), 1.55 - 1.45 (m, 1H), 1.36 - 1.26 (m, 1H), 1.10 (d, *J* = 6.8 Hz, 3H). |
| 137 | 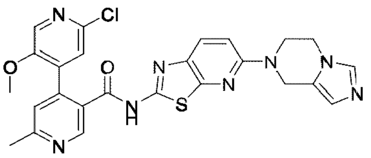 | 533.2 | $^1$H NMR (400 MHz, DMSO-$d_6$) δ 12.81 (s, 1H), 9.07 (s, 1H), 8.82 (s, 1H), 8.17 (s, 1H), 8.02 (d, *J* = 9.0 Hz, 1H), 7.60 (s, 1H), 7.58 (s, 1H), 7.46 (s, 1H), 7.18 (d, *J* = 9.1 Hz, 1H), 4.92 (s, 2H), 4.39 (t, *J* = 5.4 Hz, 2H), 4.14 - 4.08 (m, 2H), 3.61 (s, 3H), 2.60 (s, 3H). |
| 138 | 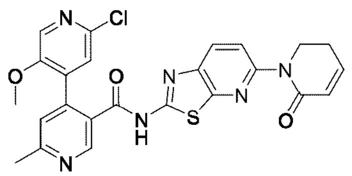 | 507.2 | $^1$H NMR (400 MHz, DMSO-$d_6$) δ 12.99 (s, 1H), 8.91 (s, 1H), 8.15 (s, 1H), 8.00 (s, 1H), 7.78 (s, 1H), 7.52 (s, 1H), 7.38 (s, 1H), 7.04 - 6.81 (m, 1H), 5.97 (d, *J* = 9.7 Hz, 1H), 4.10-4.04 (m, 2H), 3.62 (s, 3H), 2.58 (s, 3H), 2.49 - 2.47 (m, 2H). |
| 139 | 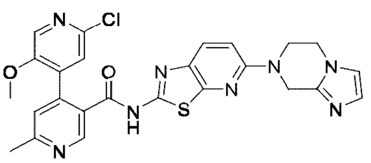 | 533.2 | $^1$HNMR(400MHz,DMSO-$d_6$) δ 12.77 (s, 1H), 8.82 (s, 1H), 8.16 (s, 1H), 7.97 (d, J = 9.0 Hz, 1H), 7.57 (s, 1H), 7.46 (s, 1H), 7.19 - 7.11 (m, 2H), 6.94 - 6.89 (m, 1H), 4.75 (s, 2H), 4.14 - 4.06 (m, 4H), 3.61 (s, 3H), 2.60 (s, 3H) |
| 140 | 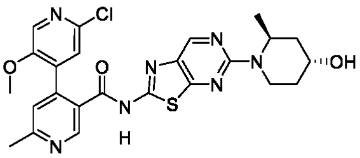 | 526.3 | $^1$H NMR (400 MHz, DMSO-$d_6$) δ 12.87 (s, 1H), 8.82 (s, 1H), 8.75 (s, 1H), 8.17 (s, 1H), 7.57 (s, 1H), 7.47 (s, 1H), 5.11 - 5.03 (m, 1H), 4.62 (d, *J* = 13.2 Hz, 1H), 3.92 - 3.86 (m, 1H), 3.62 (s, 3H), 3.51- 3.46 (m, 1H), 3.04 - 2.95 (m, 1H), 2.60 (s, 3H), 1.95 - 1.88 (m, 1H), 1.85 - 1.78 (m, 1H), 1.47 - 1.38 (m, 1H), 1.28 - 1.21 (m, 1H), 1.15 (d, *J* = 6.8 Hz, 3H). |
| 141 | 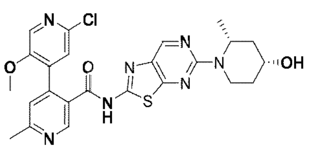 | 526.2 | $^1$H NMR (400 MHz, DMSO-$d_6$) δ 12.84 (s, 1H), 8.82 (s, 1H), 8.74 (s, 1H), 8.17 (s, 1H), 7.57 (s, 1H), 7.46 (s, 1H), 4.93 - 4.83 (m, 1H), 4.68 (d, *J* = 2.4 Hz, 1H), 4.40 - 4.41 (m, 1H), 4.04 - 3.97 (m, 1H), 3.63 (s, 3H), 3.31 - 3.25 (s, 1H), 2.60 (s, 3H), 1.78 - 1.58 (m, 4H), 1.32 (d, *J* = 6.8 Hz, 3H). |

(continued)

| | Chemical structure | LC/MS( ESI) m/z $[M+H]^+$ | $^1H$ NMR |
|---|---|---|---|
| 142 | 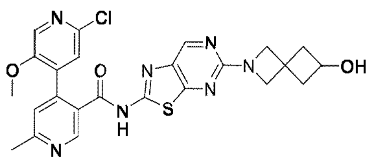 | 524.2 | $^1H$ NMR (400 MHz, DMSO-$d_6$) δ 12.85 (s, 1H), 8.83 (s, 1H), 8.69 (s, 1H), 8.17 (s, 1H), 7.56 (s, 1H), 7.44 (s, 1H), 5.02 - 4.98 (m, 1H), 4.18 - 4.06 (m, 1H), 3.80 - 3.72 (m, 2H), 3.63 (s, 3H), 3.52 - 3.46 (m, 2H), 2.59 (s, 3H), 2.29 - 2.21 (m, 1H), 2.13 - 2.06 (m, 1H), 1.88 - 1.82 (m, 1H), 1.70 - 1.64 (m, 1H). |
| 143 | 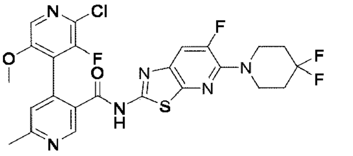 | 567.1 | $^1H$ NMR (400 MHz, DMSO-$d_6$) δ 13.06 (s, 1H), 8.99 (s, 1H), 8.18 (s, 1H), 8.03 (d, *J* = 13.4 Hz, 1H), 7.50 (s, 1H), 3.72 (s, 3H), 3.60 - 3.47 (m, 4H), 2.61 (s, 3H), 2.16 - 2.05 (m, 4H). |
| 144 | 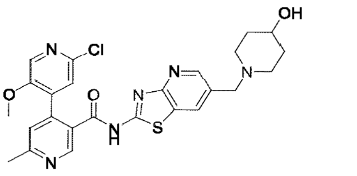 | 525.2 | $^1H$ NMR (400 MHz, DMSO-$d_6$) δ 13.38 (s, 1H), 9.45 (s, 1H), 8.89 (s, 1H), 8.68 (dd, J = 16.5, 2.0 Hz, 1H), 8.56 (dd, J = 13.6, 2.0 Hz, 1H), 8.17 (s, 1H), 7.61 (s, 1H), 7.49 (s, 1H), 4.49 - 4.41 (m, 2H), 3.60 (s, 3H), 3.44 - 3.36 (m, 2H), 3.25 - 3.20 (m, 2H), 3.05 - 2.95 (m, 1H), 2.62 (s, 3H), 1.92 - 1.94 (m, 1H), 1.85 - 1.75 (m, 2H), 1.59 - 1.51 (m, 1H). |
| 145 | 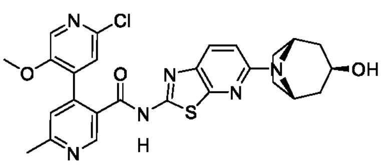 | 537.2 | $^1H$ NMR (400 MHz, DMSO-$d_6$) δ 12.65 (s, 1H), 8.82 (s, 1H), 8.16 (s, 1H), 7.85 (d, *J* = 8.9 Hz, 1H), 7.55 (s, 1H), 7.44 (s, 1H), 6.87 (d, *J* = 9.0 Hz, 1H), 4.54-4.48 (m, 2H), 4.44-4.36 (m, 1H), 4.05 - 3.90 (m, 1H), 3.62 (s, 3H), 2.59 (s, 3H), 2.00 - 1.88 (m, 2H), 1.79-1.71 (m, 4H), 1.50-1.44 (m, 2H) |
| 146 | 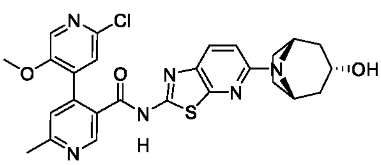 | 537.2 | $^1H$ NMR (400 MHz, DMSO-$d_6$) δ 12.66 (s, 1H), 8.82 (s, 1H), 8.16 (s, 1H), 7.85 (d, *J* = 8.9 Hz, 1H), 7.55 (s, 1H), 7.43 (s, 1H), 6.86 (d, *J* = 9.0 Hz, 1H), 4.51 (s, 2H), 4.44-4.36 (m, 1H) , 4.05 - 3.90 (m, 1H), 3.62 (s, 3H), 2.59 (s, 3H), 2.00 - 1.88 (m, 2H), 1.79-1.71 (m, 4H), 1.50-1.44 (m, 2H). |

**Example 147**: **2'-chloro-N-(5-(1,4-dimethyl-1H-1,2,3-triazol-5-yl)thiazolo[5,4-b]pyridin-2-yl)-5'-meth oxy-6-methyl-[4,4'-bipyridine]-3-carboxamide**

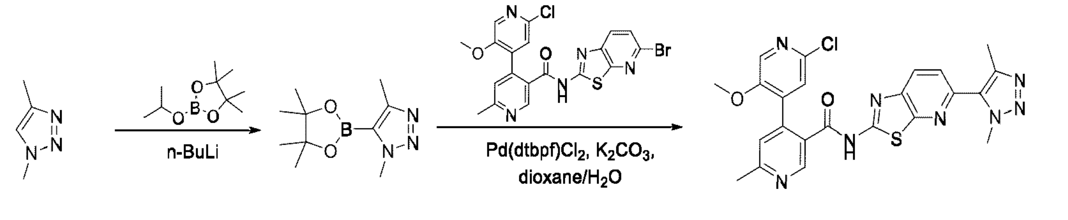


**Step 1: 1,4-dimethyl-5-(4,4,5,5-tetramethyl-1,3,2-dioxaborinan-2-yl)-1H-1,2,3-triazole**

At -78°C, 2.5M n-butyllithium in n-hexane solution (0.5 mL) was added to the solution of 1,4-dimethyl-1,2,3-triazole (100 mg, 1.030 mmol) in THF (10 mL); and the temperature held at 78°C, the reaction mixture was stirred for 1 hour. 4,4,5,5-tetramethyl-2-(prop-2-yloxy)-1,3,2-dioxaborinan (210 mg, 1.13 mmol) was added to the reaction solution at -78°C; and the reaction mixture was stirred at 25°C overnight. Saturated ammonium chloride in water was slowly added to quench

the reaction; the resulting mixture was extracted with ethyl acetate three times; the organic phases were combined, dried over anhydrous sodium sulfate, filtered, and concentrated to obtain a crude product (the crude product is brown oil, 100 mg, yield: 43.54%), which was directly used in the enxt step of reaction.

**Step 2**: **2'-chloro-N-(5-(1,4-dimethyl-1H-1,2,3-triazol-5-yl)thiazolo[5,4-b]pyridin-2-yl)-5'-methoxy-6-methyl-[4,4'-bipyridine]-3-carboxamide**

$K_2CO_3$ (186 mg, 1.35 mmol) and Pd(dppf)$Cl_2$ (33 mg, 0.045 mmol) were added to 1, 4-dimethyl-5-(4, 4, 5, 5-tetramethyl-1, 3, 2-dioxaborinan-2-yl)-1, 2, 3-triazole (100 mg, 0.448 mmol) and N-(5-bromo[1,3]thiazolo[5,

4-b]pyridin-2-yl)-4-(2-chloro-5-methoxypyridin-4-yl)-6-methylpyridin-3-carboxamide (220.0 mg, 0.448 mmol) in the mixed solution of 1,4-dioxane (30 mL) and $H_2O$ (6 mL). Under the protection of nitrogen, the temperature of the mixture was increased to 90°C and stirred to react for 3h. After complete reaction, water was added to the reaction mixture for quenching; and the mixture was extracted three times with ethyl acetate. The organic phase was concentrated to obtain a crude product, which was separated and purified by reversed-phase preparation (acetonitrile/water system), to obtain the title compound (white solid, 3 mg, yield: 1.32%). $^1$H NMR (400 MHz, DMSO-$d_6$) δ 13.21 (s, 1H), 8.82 (s, 1H), 8.29 (d, *J* = 8.4 Hz, 1H), 8.17 (s, 1H), 7.78 (d, *J* = 8.4 Hz, 1H), 7.60 (s, 1H), 7.48 (s, 1H), 4.15 (s, 3H), 3.63 (s, 3H), 2.62 (s, 3H), 2.39 (s, 3H). LC/MS (ESI) m/z: 507.2 $[M+H]^+$.

The example compounds in Table 10 were synthesized using commercially available raw materials with reference to the synthesis steps of Example **147.**

Table 10:

| Examples | Chemical structure | LC/MS(ESI) m/z $[M+H]^+$ | $^1$H NMR |
|---|---|---|---|
| 148 | 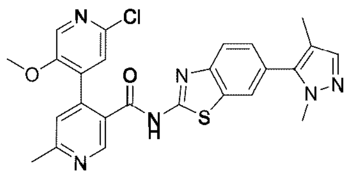 | 505.1 | $^1$H NMR (400 MHz, DMSO-$d_6$) $\delta$ 13.03 (s, 1H), 8.88 (s, 1H), 8.16 (s, 1H), 8.05 (s, 1H), 7.86 (d, *J* = 8.2 Hz, 1H), 7.58 (s, 1H), 7.48 - 7.42 (m, 2H), 7.35 (s, 1H), 3.73 (s, 3H), 3.62 (s, 3H), 2.61 (s, 3H), 1.99 (s, 3H). |
| 149 | 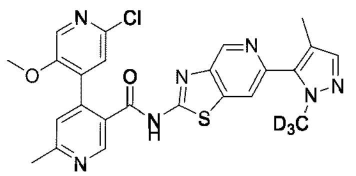 | 509.1 | $^1$H NMR (400 MHz, DMSO-$d_6$) δ 13.27 (s, 1H), 9.17 (s, 1H), 8.88 (s, 1H), 8.27 (s, 1H), 8.17 (s, 1H), 7.61 (s, 1H), 7.49 (s, 1H), 7.35 (s, 1H), 3.61 (s, 3H), 2.62 (s, 3H), 2.11 (s, 3H). |
| 150 | 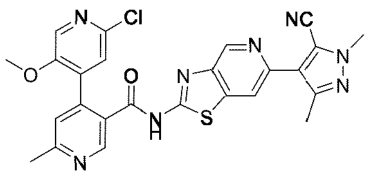 | 531.0 | $^1$H NMR (400 MHz, DMSO-$d_6$) $\delta$ 13.25 (s, 1H), 9.13 (s, 1H), 8.88 (s, 1H), 8.35 (s, 1H), 8.17 (s, 1H), 7.61 (s, 1H), 7.49 (s, 1H), 4.01 (s, 3H), 3.60 (s, 3H), 2.62 (s, 3H), 2.47 (s, 3H). |
| 151 | 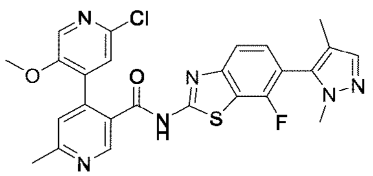 | 523.1 | $^1$H NMR (400 MHz, DMSO-$d_6$) δ 13.32 (s, 1H), 8.88 (s, 1H), 8.18 (s, 1H), 7.78 (d, *J* = 8.3 Hz, 1H), 7.60 (s, 1H), 7.55 - 7.44 (m, 2H), 7.40 (s, 1H), 3.67 (s, 3H), 3.62 (s, 3H), 2.62 (s, 3H), 1.93 (s, 3H). |
| 152 | 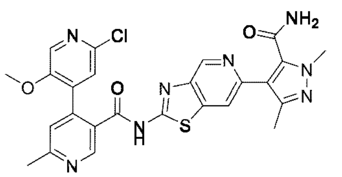 | 549.2 | $^1$H NMR (400 MHz, DMSO-$d_6$) $\delta$ 13.21 (s, 1H), 9.09 (s, 1H), 8.87 (s, 1H), 8.52 (s, 1H), 8.16 (d, *J* = 3.8 Hz, 2H), 7.73 (s, 1H), 7.60 (s, 1H), 7.49 (s, 1H), 3.92 (s, 3H), 3.61 (s, 3H), 2.61 (s, 3H), 2.28 (s, 3H). |

(continued)

| Examples | Chemical structure | LC/MS(ESI) m/z [M+H]+ | 1H NMR |
|---|---|---|---|
| 153 | 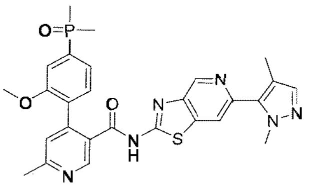 | 547.2 | 1HNMR(400MHz,DMSO-$d_6$) δ 13.21 (s, 1H), 9.17 (d, J = 0.8 Hz, 1H), 8.82 (s, 1H), 8.26 (d, J = 0.8 Hz, 1H), 7.59 - 7.54 (m, 1H), 7.54 - 7.47 (m, 1H), 7.44 (s, 1H), 7.35 (s, 1H), 7.30 (d, J = 11.8 Hz, 1H), 3.90 (s, 3H), 3.56 (s, 3H), 2.62 (s, 3H), 2.10 (s, 3H), 1.71 (s, 3H), 1.68 (s, 3H) |
| 154 | 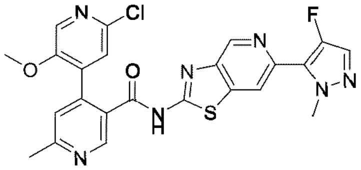 | 510.2 | 1H NMR (400 MHz, DMSO-$d_6$) δ 13.29 (s, 1H), 9.18 (s, 1H), 8.89 (s, 1H), 8.38 (s, 1H), 8.17 (s, 1H), 7.63 (d, J = 4.5 Hz, 1H), 7.60 (s, 1H), 7.48 (s, 1H), 4.06 (s, 3H), 3.60 (s, 3H), 2.62 (s, 3H). |
| 155 | 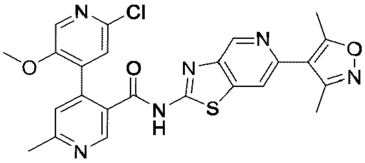 | 507 | 1H NMR (400 MHz, DMSO-$d_6$) δ 13.22 (s, 1H), 9.12 (s, 1H), 8.87 (s, 1H), 8.25 (s, 1H), 8.17 (s, 1H), 7.60 (s, 1H), 7.49 (s, 1H), 3.60 (s, 3H), 2.61 (s, 3H), 2.59 (s, 3H), 2.40 (s, 3H). |
| 156 | 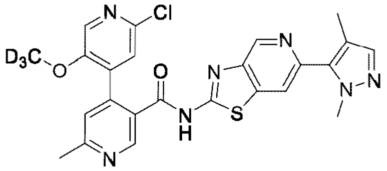 | 509.2 | 1H NMR (400 MHz, DMSO-$d_6$) δ 13.26 (s, 1H), 9.17 (s, 1H), 8.88 (s, 1H), 8.26 (s, 1H), 8.16 (s, 1H), 7.60 (s, 1H), 7.49 (s, 1H), 7.35 (s, 1H), 3.89 (s, 3H), 2.61 (s, 3H), 2.10 (s, 3H) |
| 157 | 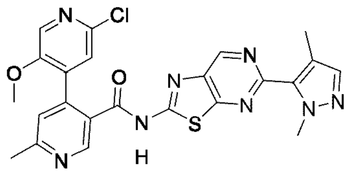 | 507.2 | 1H NMR (400 MHz, DMSO-$d_6$) δ 13.41 (s, 1H), 9.32 (s, 1H), 8.89 (s, 1H), 8.18 (s, 1H), 7.62 (s, 1H), 7.51 (s, 1H), 7.39 (s, 1H), 4.13 (s, 3H), 3.63 (s, 3H), 2.62 (s, 3H), 2.30 (s, 3H). |
| 158 | 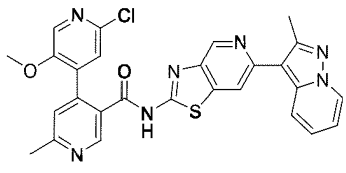 | 542.5 | 1H NMR (400 MHz, DMSO-$d_6$) δ 13.12 (s, 1H), 9.11 (s, 1H), 8.88 (s, 1H), 8.67 - 8.63 (m, 1H), 8.31 (s, 1H), 8.25 - 8.20 (m, 1H), 8.18 (s, 1H), 7.60 (s, 1H), 7.48 (s, 1H), 7.37 - 7.29 (m, 1H), 6.96 - 6.90 (m, 1H), 3.62 (s, 3H), 2.67 (s, 3H), 2.62 (s, 3H). |
| 159 | 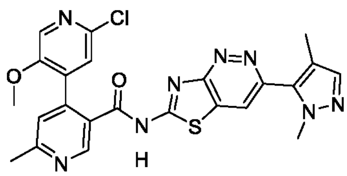 | 507.2 | 1H NMR (400 MHz, DMSO-$d_6$) δ 13.66 (s, 1H), 8.92 (s, 1H), 8.60 (s, 1H), 8.18 (s, 1H), 7.63 (s, 1H), 7.51 (s, 1H), 7.43 (s, 1H), 3.92 (s, 3H), 3.63 (s, 3H), 2.63 (s, 3H), 2.12 (s, 3H). |
| 160 | 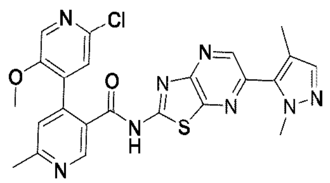 | 507.1 | 1H NMR (400 MHz, DMSO-$d_6$) δ 13.56 (s, 1H), 8.91 (s, 1H), 8.80 (s, 1H), 8.19 (s, 1H), 7.62 (s, 1H), 7.49 (s, 1H), 7.42 (s, 1H), 3.92 (s, 3H), 3.65 (s, 3H), 2.62 (s, 3H), 2.15 (s, 3H). |

(continued)

| Examples | Chemical structure | LC/MS(ESI) m/z [M+H]+ | 1H NMR |
|---|---|---|---|
| 161 | 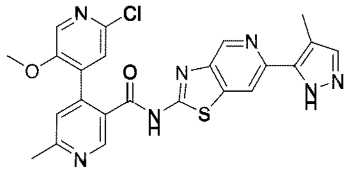 | 492.1 | 1H NMR (400 MHz, DMSO-$d_6$) δ 13.13 (s, 1H), 12.78 (s, 1H), 9.02 (s, 1H), 8.90 (s, 1H), 8.43 (s, 1H), 8.17 (s, 1H), 7.57 (s, 1H), 7.53 (s, 1H), 7.45 (s, 1H), 3.62 (s, 3H), 2.61 (s, 3H), 2.36 (s, 3H). |
| 162 | 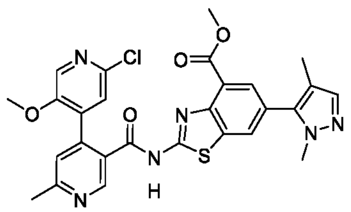 | 563.3 | 1H NMR (400 MHz, DMSO-$d_6$) δ 13.40 (s, 1H), 8.90 (s, 1H), 8.32 (s, 1H), 8.17 (s, 1H), 7.83 (d, *J* = 2.0 Hz, 1H), 7.59 (s, 1H), 7.46 (s, 1H), 7.37 (s, 1H), 3.91 (s, 3H), 3.73 (s, 3H), 3.61 (s, 3H), 2.61 (s, 3H), 1.99 (s, 3H). |
| 163 | 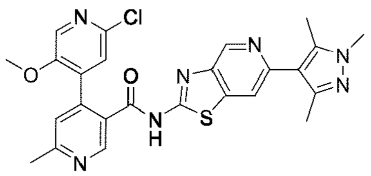 | 520.2 | 1H NMR(400 MHz, DMSO-$d_6$) δ 13.34 (s, 1H), 9.22 (s, 1H), 8.89 (s, 1H), 8.27 (s, 1H), 8.17 (s, 1H), 7.61 (s, 1H), 7.51 (s, 1H), 3.75 (s, 3H), 3.61 (s, 3H), 2.62 (s, 3H), 2.37 (s, 3H), 2.26 (s, 3H). |
| 164 | 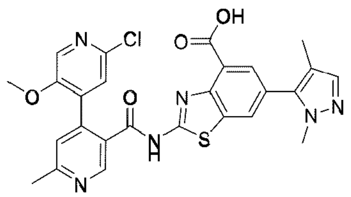 | 549.2 | 1H NMR (400 MHz, DMSO-$d_6$) δ 13.46 (s, 1H), 8.91 (s, 1H), 8.33 (d, *J* = 1.6 Hz, 1H), 8.18 (s, 1H), 7.87 (d, *J* = 1.6 Hz, 1H), 7.60 (s, 1H), 7.48 (s, 1H), 7.38 (s, 1H), 3.74 (s, 3H), 3.62 (s, 3H), 2.62 (s, 3H), 1.99 (s, 3H). |
| 165 | 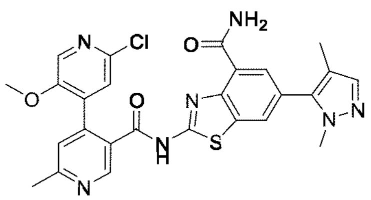 | 548.2 | 1H NMR (400 MHz, DMSO-$d_6$) δ 13.33 (s, 1H), 9.22 (s, 1H), 8.94 (s, 1H), 8.29 (d, *J* = 1.6 Hz, 1H), 8.19 (s, 1H), 8.08 (d, *J* = 2.8 Hz, 1H), 8.05 (d, *J* = 1.6 Hz, 1H), 7.61 (s, 1H), 7.50 (s, 1H), 7.38 (s, 1H), 3.73 (s, 3H), 3.63 (s, 3H), 2.63 (s, 3H), 1.99 (s, 3H). |
| 166 | 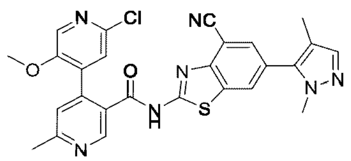 | 530.2 | 1H NMR (400 MHz, DMSO-$d_6$) δ 13.59 (s, 1H), 8.91 (s, 1H), 8.43 (s, 1H), 8.17 (s, 1H), 8.04 (s, 1H), 7.61 (s, 1H), 7.48 (s, 1H), 7.37 (s, 1H), 3.75 (s, 3H), 3.62 (s, 3H), 2.62 (s, 3H), 2.00 (s, 3H). |
| 167 | 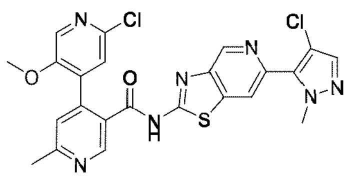 | 526.2 | 1H NMR (400 MHz, DMSO-$d_6$) δ 13.32 (s, 1H), 9.20 (s, 1H), 8.89 (s, 1H), 8.43 (s, 1H), 8.17 (s, 1H), 7.70 (s, 1H), 7.61 (s, 1H), 7.49 (s, 1H), 3.95 (s, 3H), 3.61 (s, 3H), 2.62 (s, 3H). |
| 168 | 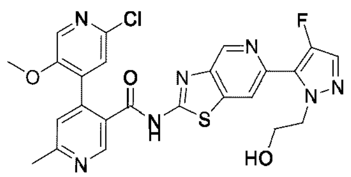 | 540.2 | 1H NMR (400 MHz, DMSO-$d_6$) δ 13.29 (s, 1H), 9.17 (s, 1H), 8.88 (s, 1H), 8.37 (s, 1H), 8.16 (s, 1H), 7.66 (d, *J* = 4.5 Hz, 1H), 7.60 (s, 1H), 7.49 (s, 1H), 4.54 (t, *J* = 6.0 Hz, 2H), 3.67 (t, *J* = 6.0 Hz, 2H), 3.60 (s, 3H), 2.62 (s, 3H). |

(continued)

| Exam ples | Chemical structure | LC/MS(ESI) m/z $[M+H]^+$ | $^1$H NMR |
|---|---|---|---|
| 169 | 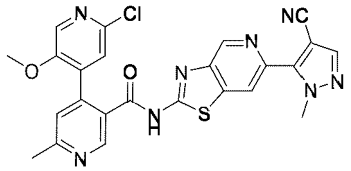 | 517.2 | $^1$H NMR (400 MHz, DMSO-$d_6$) δ 13.38 (s, 1H), 9.20 (s, 1H), 8.91 (s, 1H), 8.54 (s, 1H), 8.18 (s, 1H), 8.16 (s, 1H), 7.59 (s, 1H), 7.47 (s, 1H), 4.05 (s, 3H), 3.61 (s, 3H), 2.61 (s, 3H). |
| 170 | 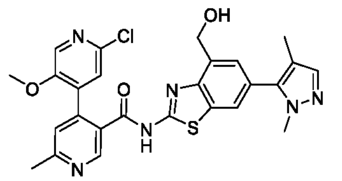 | 535.2 | $^1$H NMR (400 MHz, DMSO-$d_6$) δ 13.18 (s, 1H), 8.87 (s, 1H), 8.17 (s, 1H), 7.94 (d, *J* = 1.6 Hz, 1H), 7.58 (s, 1H), 7.48 (t, *J* = 0.8 Hz, 1H), 7.46 (s, 1H), 7.35 (s, 1H), 5.32 (t, *J* = 6.0 Hz, 1H), 4.99 (d, *J* = 5.6 Hz, 2H), 3.73 (s, 3H), 3.62 (s, 3H), 2.61 (s, 3H), 1.99 (s, 3H). |
| 171 | 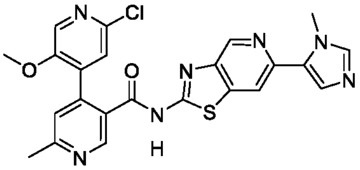 | 492.2 | $^1$H NMR (400 MHz, DMSO-$d_6$) δ 13.37 (s, 1H), 9.18 (s, 1H), 9.16 (s, 1H), 8.88 (s, 1H), 8.55 (s, 1H), 8.17 (s, 1H), 8.15 (s, 1H), 7.61 (s, 1H), 7.50 (s, 1H), 4.11 (s, 3H), 3.60 (s, 3H), 2.62 (s, 3H) |
| 172 | 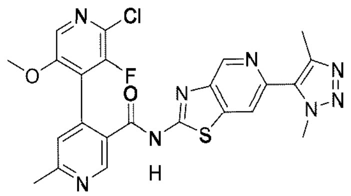 | 525.1 | $^1$H NMR (400 MHz, DMSO-$d_6$) δ 13.44 (s, 1H), 9.19 (s, 1H), 9.05 (s, 1H), 8.37 (s, 1H), 8.18 (s, 1H), 7.54 (s, 1H), 4.14 (s, 3H), 3.72 (s, 3H), 2.62 (s, 3H), 2.39 (s, 3H). |
| 173 | 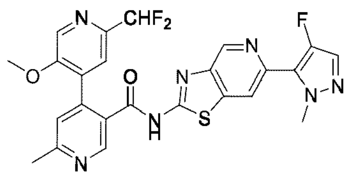 | 526.1 | $^1$H NMR (400 MHz, DMSO-$d_6$) δ 13.32 (s, 1H), 9.19 (s, 1H), 8.89 (s, 1H), 8.46 (s, 1H), 8.38 (s, 1H), 7.76 (s, 1H), 7.65 - 7.62 (m, 1H), 7.49 (s, 1H), 7.15 - 6.87 (m, 1H), 4.06 (s, 3H), 3.68 (s, 3H), 2.63 (s, 3H). |
| 174 | 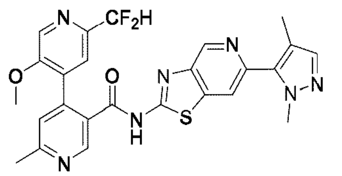 | 522.3 | $^1$HNMR(400MHz,DMSO-$d_6$) δ 13.28 (s, 1H), 9.17 (s, 1H), 8.88 (s, 1H), 8.46 (s, 1H), 8.27 - 8.24 (m, 1H), 7.76 (s, 1H), 7.49 (s, 1H), 7.35 (s, 1H), 7.00 (t, J = 55.1 Hz, 1H), 3.89 (s, 3H), 3.69 (s, 3H), 2.63 (s, 3H), 2.10 (s, 3H) |

**Example 175**: **2'-chloro-*N*-(6-(1,4-dimethyl-1*H*-1,2,3-triazol-5-yl)thiazolo[4,5-*c*]pyridin-2-yl)-5'-metho xy-6-methyl-[4,4'-bipyridine]-3-carboxamide**

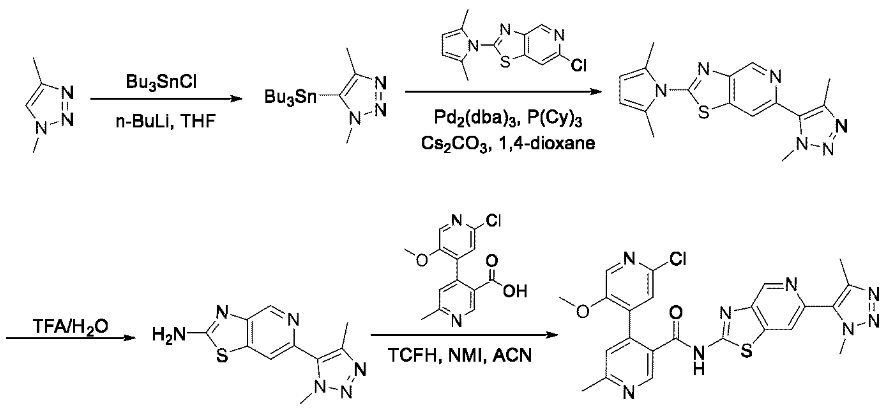


**Step 1: 1,4-dimethyl-5-(tributylstannyl)-1H-1,2,3-triazole**

-78°C, n-butyllithium (2.3 mL, 3.71 mmol, 1.6 mol/L) was added dropwise slowly to the solution of 1,4-dimethyl-1H-1,2,3-triazole (300 mg, 3.09 mmol) in tetrahydrofuran (30 mL). Under the protection of nitrogen, the reaction mixture was stirred at -78°C for 1 hour, and then tributyl tin chloride (0.922 mL, 3.40 mmol) was added. Under the protection of nitrogen, the reaction mixture was stirred at -78°C for 0.5 hours. After complete reaction, the reaction mixture was restored to room temperature, added with saturated ammonium chloride solution, extracted with ethyl acetate, washed with saturated saline, dried over anhydrous sodium sulfate, and concentrated under reduced pressure. The residue was purified by silica-gel chromatography (eluent: petroleum ether/ethyl acetate, gradient: 0-10% ethyl acetate) to obtain 1,4-dimethyl-5-(tributylstannyl)-1H-1,2,3-triazole (yellow oil, 1100 mg, yield: 92.2%). LC/MS (ESI) m/z: 388.2 $[M+H]^+$.

**Step 2: 6-(1,4-dimethyl-1*H*-1,2,3-triazol-5-yl)-2-(2,5-dimethyl-1*H*-pyrrol-1-yl)thiazolo[4,5-*c*]pyridine**

Tris(dibenzylideneacetone)dipalladium (355.7 mg, 0.39 mmol), tricyclohexylphosphine (217.9 mg, 0.78 mmol) and cesium carbonate (1687 mg, 5.18 mmol) were added to the solution of 1,4-dimethyl-5-(tributylstannyl)-1H-1,2,3-triazole (1000 mg, 2.59 mmol) and 6-chloro-2-(2,5-dimethyl-1H-pyrrol-1-yl)thiazolo[4,5-c]pyridine (683 mg, 2.59 mmol) in 1,4-dioxane (30 mL). Under the protection of nitrogen, the reaction mixture was stirred at 110°C to react for 5 hours. After complete reaction, the reaction mixture was cooled to room temperature; water was added; and the resulting mixture was extracted with ethyl acetate, washed with saturated saline, dried over anhydrous sodium sulfate, and concentrated under reduced pressure. The residue was purified by silica-gel chromatography (eluent: petroleum ether/ethyl acetate, gradient: 0-30% ethyl acetate) to obtain 6-(1,4-dimethyl-1*H*-1,2,3-triazol-5-yl)-2-(2,5-dimethyl-1*H*-pyrrol-1-yl)thiazolo[4,5-c]pyridine (yellow solid, 140 mg, yield: 16.7%). LC/MS (ESI) m/z: 325.1 $[M+H]^+$.

**Step 3**: **6-(1,4-dimethyl-1H-1,2,3-triazol-5-yl)thiazolo[4,5-c]pyridin-2-amine**

Trifluoroacetic acid (5.0 mL) and water (one drop) were added to 6-(1,4-dimethyl-1*H*-1,2,3-triazol-5-yl)-2-(2,5-dimethyl-1*H*-pyrrol-1-yl)thiazolo[4,5-c]pyridine (140 mg, 0.432 mmol), and stirred at 70°C to react for 1 hour. After complete reaction, the reaction mixture was concentrated under reduced pressure, to obtain a crude product 6-(1,4-dimethyl-1H-1,2,3-triazol-5-yl)thiazolo[4,5-*c*]pyridin-2-amine (yellow solid, 43.0 mg, yield: 40.5%), which was used directly in the next step. LC/MS (ESI) m/z: 247.1 $[M+H]^+$.

**Step 4: 2'-chloro-*N*-(6-(1,4-dimethyl-1*H*-1,2,3-triazol-5-yl)thiazolo[4,5-*c*]pyridin-2-yl)-5'-methoxy-6-methyl-[4,4'-bipyridine]-3-carboxamide**

6-(1,4-dimethyl-1H-1,2,3-triazol-5-yl)thiazolo[4,5-*c*]pyridin-2-amine (30.0 mg, 0.12 mmol), 2'-chloro-5'-methoxy-[3,4'-bipyridine]-4-carboxylic acid (34.0 mg, 0.12 mmol), N,N,N',N'-tetramethylchlorformamidine hexafluorphosphate (34.2 mg, 0.12 mmol) and N-methylimidazole (30.0 mg, 0.36 mmol) were dissolved in acetonitrile (10 mL); and the reaction mixture was stirred at 70°C to react for 4 hours. After the reaction was completed, the reaction mixture was concentrated under reduced pressure to obtain residues, which were purified by pre-HPLC to obtain 2'-chloro-*N*-(6-(1,4-dimethyl-1*H*-1,2,3-triazol-5-yl)thiazolo[4,5-*c*]pyridin-2-yl)-5'-methoxy-6-methyl-[4,4'-bi pyridine]-3-carboxamide (white solid, 12.2 mg, yield: 19.8%). $^1H$ NMR (400 MHz, DMSO-$d_6$) δ 13.30 (s, 1H), 9.20 (s, 1H), 8.88 (s, 1H), 8.40 (s, 1H), 8.17 (s, 1H), 7.61 (s, 1H), 7.49 (s, 1H), 4.14 (s, 3H), 3.60 (s, 3H), 2.62 (s, 3H), 2.39 (s, 3H). LC/MS (ESI) m/z: 507.2 $[M+H]^+$.

**Example 176: 2'-chloro-5'-methoxy-6-methyl-N-(6-(1-methyl-4-(methyl-d3)-1H-pyrazol-5-yl)thiazolo [4,5-c]pyridin-2-yl)-[4,4'-bipyridine]-3-carboxamide**

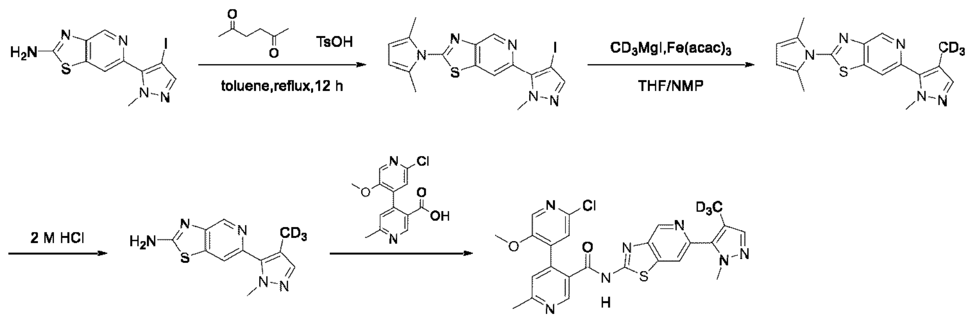


**Step 1: 2-(2,5-dimethyl-1H-pyrrol-1-yl)-6-(4-iodo-1-methyl-1H-pyrazol-5-yl)thiazolo[4,5-c]pyridine**

6-(4-iodo-1-methyl-1H-pyrazol-5-yl)thiazolo[4,5-c]pyridin-2-amine (602 mg, 1.69 mmol), acetonyl acetone (482 mg, 4.2 mmol), and p-toluenesulfonic acid (58.1 mg, 3.4 mmol) were dissolved in 10 mL of anhydrous toluene, to allow for reaction at 120°C for 18 hours. The reaction mixture was concentrated under reduced pressure, to obtain residues; and the residue was purified by column chromatography (eluent: petroleum ether/ethyl acetate, gradient: 5%-25% ethyl acetate), to obtain the title compound (brown solid, 440 mg, yield: 60%).LC/MS (ESI) m/z: 436.1 $[M+H]^+$.

**Step 2: 2-(2,5-dimethyl-1H-pyrrol-1-yl)-6-(1-methyl-4-(methyl-d3)-1H-pyrazol-5-yl)thiazolo[4,5-c]py ridine**

2-(2,5-dimethyl-1H-pyrrol-1-yl)-6-(4-iodo-1-methyl-1H-pyrazol-5-yl)thiazolo[4,5-c]pyridine (350 mg, 0.80 mmol) was dissolved in 10 mL of tetrahydrofuran and 1 mL of N-methylpyrrolidone; under the protection of nitrogen, ferric acetylacetonate (56.5 mg, 0.16 mmol) was added; and then, 1 M/L deuteromethyl magnesium iodide (2.4 mL) was added dropwise slowly. The reaction was allowed to occur at room temperature for 2 hours. Saturated ammonium chloride solution was added for quenching; the reaction mixture was extracted with ethyl acetate; the organic phase was washed with saturated saline, dried over anhydrous sodium sulfate, and filtered; and the filtrate was spin-dried to obtain residues, which were purified by column chromatography (eluent: petroleum ether/ethyl acetate, gradient: 5%-30% ethyl acetate), to obtain the crude title compound (brown solid, 50 mg, yield: 15.2%).LC/MS (ESI) m/z: 327.1 $[M+H]^+$.

**Step 3: 6-(1-methyl-4-(methyl-d3)-1H-pyrazol-5-yl)thiazolo[4,5-c]pyridin-2-amine**

2-(2,5-dimethyl-1H-pyrrol-1-yl)-6-(1-methyl-4-(methyl-d3)-1H-pyrazol-5-yl)thiazolo[4,5-c]pyridine (40 mg, 0.12 mmol) was dissolved in 2 M hydrochloric acid solution, to allow for reaction at 80°C for 1 hour. After complete reaction, the reaction mixture was concentrated under reduced pressure to obtain the title compound (white solid, 30 mg, yield: 99%).LC/MS (ESI) m/z: 248.8 $[M+H]^+$.

**Step 4: 2'-chloro-5'-methoxy-6-methyl-N-(6-(1-methyl-4-(methyl-d3)-1H-pyrazol-5-yl)thiazolo[4,5-c] pyridin-2-yl)-[4,4'-bipyridine]-3-carboxamide**

6-(1-methyl-4-(methyl-d3)-1H-pyrazol-5-yl)thiazolo[4,5-c]pyridin-2-amine (30 mg, 0.12 mmol) and 2'-chloro-5'-methoxy-6-methyl-[4,4'-bipyridine]-3-carboxylic acid (34 mg, 0.12 mmol) were dissolved in 5 mL of acetonitrile and 1 mL of N,N-dimethylformamide; N-methylimidazole (39 mg, 0.48 mmol) was added; the resulting mixture was stirred at 70°C for 10 minutes; and then, *N,N,N',N'*-tetramethylchlorformamidine hexafluorphosphate (50 mg, 0.18 mmol) was added to allow for reaction at 70°C 1 hour. Ethyl acetate and water were added to the reaction mixture for stratification; the organic phase was washed with saturated sodium chloride solution, dried over anhydrous sodium sulfate, and filtered; the filtrate was concentrated under reduced pressure; and the resulting residue was purified by pre-HPLC (eluent: acetonitrile/0.1% formic acid aqueous solution, gradient: 25%-90%) to obtain the title compound (white solid, 5.1mg, yield: 8.3%). $^1H$ NMR (400 MHz, $CDCl_3$) δ 8.93 (s, 1H), 8.86 (s, 1H), 8.07 (s, 1H), 7.85 (d, $J$ = 0.8 Hz, 1H), 7.39 (s, 1H), 7.26 (s, 1H), 7.22 (s, 1H), 3.98 (s, 3H), 3.77 (s, 3H), 2.69 (s, 3H). LC/MS (ESI) m/z: 509.2 $[M+H]^+$

**Example 177: 2'-chloro-*N*-(6-(1,4-dimethyl-1*H*-parazol-5-yl)-5-methoxythiazolo[4,5-*b*]pyridin-2-yl)-5' -methoxy-6-methyl-[4,4'-bipyridine]-3-carboxamide**

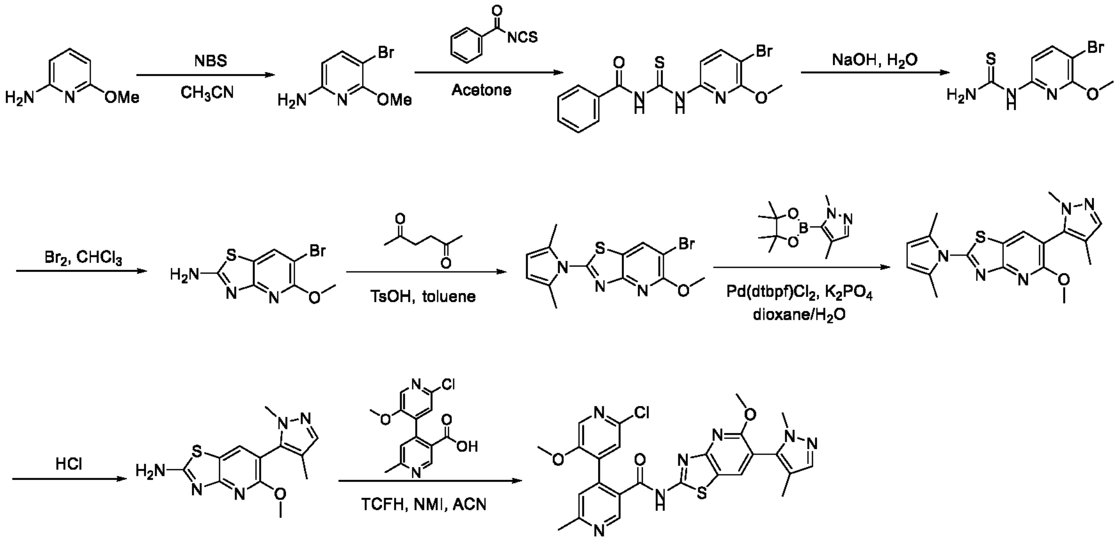


**Step 1: 5-bromo-6-methoxypyridin-2-amine**

N-bromosuccinimide (7.17 g, 40.28 mmol) was added to the solution of 2-amino-6-methoxypyridine (10.0 g, 80.55 mmol) in acetonitrile (80 mL); and the reaction mixture was stirred at room temperature for 1.5 hours. The solution of N-bromosuccinimide (7.17 g, 40.278 mmol) in acetonitrile (80 mL) was added to the reaction solution again; and the reaction mixture was continued with stirring at room temperature for 1.5 hours. After complete reaction, water was added; and the resulting mixture was extracted with ethyl acetate, washed with saturated saline, dried over anhydrous sodium sulfate, and concentrated under reduced pressure. The residue was purified by silica-gel chromatography (eluent: petroleum ether/ethyl acetate, gradient: 0-10% ethyl acetate) to obtain 5-bromo-6-methoxypyridin-2-amine (yellow solid, 7.5 g, yield: 45.8%). LC/MS (ESI) m/z: 203.0 $[M+H]^+$.

**Step 2: *N*- ((5-bromo-6-methoxypyridin-2-yl)carbamothioyl)benzamide**

Oxybenzoyl isothiocyanate (9.04 g, 55.41 mmol) was added to the solution of 5-bromo-6-methoxypyridin-2-amine (7.50 g, 36.94 mmol) in acetone (80 mL). Under the protection of nitrogen, the mixture was stirred at room temperature for 2 hours. After complete reaction, the reaction mixture was filtered; the filter cake was washed with acetone to obtain *N*-((5-bromo-6-methoxypyridin-2-yl)carbamothioyl)benzamide (yellow solid, 12.6 g, yield: 93.1%). LC/MS (ESI) m/z: 366.0 $[M+H]^+$.

**Step 3: 1-(5-bromo-6-methoxypyridin-2-yl)thiourea**

*N*-((5-bromo-6-methoxypyridin-2-yl)carbamothioyl)benzamide (12.6 g, 34.40 mmol) was added to sodium hydroxide (6.88 g, 172.0 mmol) in water (86 mL). The mixture was stirred at 100°C for 2 hours. After complete reaction, the reaction mixture was filtered; the filter cake was washed with water to obtain 1-(5-bromo-6-methoxypyridin-2-yl) thiourea (yellow solid, 9.0 g, yield: 99.8%). LC/MS (ESI) m/z: 262.0 $[M+H]^+$.

**Step 4: 6-bromo-5-methoxythiazolo[4,5-*b*]pyridin-2-amine**

Liquid bromine (3.5 mL, 68.66 mmol) was added to the solution of 1-(5-bromo-6-methoxypyridin-2-yl)thiourea (9.00 g, 34.33 mmol) in chloroform (90 mL). The mixture was stirred at 60°C for 12 hours. After complete reaction, the reaction mixture was concentrated under reduced pressure. The residue was pulped with dichloromethane to obtain 6-bromo-5-methoxythiazolo[4,5-*b*]pyridin-2-amine (yellow solid, 6.0 g, yield: 67.2%). LC/MS (ESI) m/z: 260.0 $[M+H]^+$.

**Step 5: 6-bromo-2-(2,5-dimethyl-1*H*-pyrrol-1-yl)-5-methoxythiazolo[4,5-*b*]pyridine**

2,5-hexanedione (2.63 g, 23.07 mmol) and p-toluenesulfonic acid (0.20 g, 1.15 mmol) were added to the solution of 6-bromo-5-methoxythiazolo[4,5-b]pyridin-2-amine (3.00 g, 11.53 mmol) in toluene (80 mL); and the reaction mixture was stirred at 120°C for 12 hours. After complete reaction, water was added; and the resulting mixture was extracted with ethyl acetate, washed with saturated saline, dried over anhydrous sodium sulfate, and concentrated under reduced pressure. The residue was purified by silica-gel chromatography (eluent: petroleum ether/ethyl acetate, gradient: 0-30% ethyl acetate) to obtain 6-bromo-2-(2,5-dimethyl-1*H*-pyrrol-1-yl)-5-methoxythiazolo[4,5-*b*]pyridine (white solid, 0.5 g, yield: 12.8%). LC/MS (ESI) m/z: 338.1 $[M+H]^+$.

**Step 6: 6-(1,4-dimethyl-1*H*-parazol-5-yl)-2-(2,5-dimethyl-1*H*-pyrrol-1-yl)-5-methoxythiazolo[4,5-*b*]py ridine**

[1,1'-bis(di-tert-butylphosphino)ferrocen]palladium dichloride (95.5 mg, 0.15 mmol) and potassium phosphate (941.4 mg, 4.44 mmol) were added to the solution of 6-bromo-2-(2,5-dimethyl-1*H*-pyrrol-1-yl)-5-methoxythiazolo[4,5-*b*] pyridine (500.0 mg, 1.48 mmol) and 1,4-dimethylpyrazol-5-pinacolboronate (393.9 mg, 1.77 mmol) in 1,4-dioxane/water (50 mL/10 mL). Under the protection of nitrogen, the reaction mixture was stirred at 90°C to react for 4 hours. After complete reaction, the reaction mixture was cooled to room temperature; water was added; and the resulting mixture was extracted with ethyl acetate, washed with saturated saline, dried over anhydrous sodium sulfate, and concentrated under reduced pressure. The residue was purified by silica-gel chromatography (eluent: petroleum ether/ethyl acetate, gradient: 20-60% ethyl acetate) to obtain 6-(1,4-dimethyl-1*H*-pyrazol-5-yl)-2-(2,5-dimethyl-1*H*-pyrrol-1-yl)-5-methoxythiazolo [4,5-*b*]pyridine (yellow solid, 330.0 mg, yield: 63.2%). LC/MS (ESI) m/z: 354.2 $[M+H]^+$.

**Step 7: 6-(1,4-dimethyl-1*H*-parazol-5-yl)-5-methoxythiazolo[4,5-*b*]pyridin-2-amine**

Hydrochloric acid solution (2.0 M, 10 mL) was added to 6-(1,4-dimethyl-1*H*-pyrazol-5-yl)-2-(2,5-dimethyl-1*H*-pyrrol-1-yl)-5-methoxythiazolo[4,5-*b*]pyridine (200 mg, 0.566 mmol), and stirred at 90°C for 1 hour. After complete reaction, the reaction mixture was concentrated under reduced pressure, to obtain a crude product 6-(1,4-dimethyl-1*H*-pyrazol-5-yl)-5-methoxythiazolo[4,5-*b*]pyridin-2-amine (yellow solid, 150.0 mg, yield: 96.3%), which was used directly in the next step. LC/MS (ESI) m/z: 276.1 $[M+H]^+$.

**Step 8**: **2'-chloro-*N*-(6-(1,4-dimethyl-1*H*-parazol-5-yl)-5-methoxythiazolo[4,5-*b*]pyridin-2-yl)-5'-meth oxy-6-methyl-[4,4'-bipyridine]-3-carboxamide**

6-(1,4-dimethyl-1*H*-pyrazol-5-yl)-5-methoxythiazolo[4,5-*b*]pyridin-2-amine (150 mg, 0.545 mmol), 2'-chloro-5'-methoxy-[3,4'-bipyridine]-4-carboxylic acid (151.83 mg, 0.545 mmol), N,N,N',N'-tetramethylchlorformamidine hexafluorphosphate (152.86 mg, 0.545 mmol) and N-methylimidazole (134.20 mg, 1.634 mmol) were dissolved in acetonitrile (20 mL); and the reaction mixture was stirred at 70°C to react for 4 hours. After the reaction was completed, the reaction mixture was concentrated under reduced pressure to obtain residues, which were purified by pre-HPLC to obtain 2'-chloro-*N*-(6-(1,4-dimethyl-1*H*-pyrazol-5-yl)-5-methoxythiazolo[4,5-*b*]pyridin-2-yl)-5'-methoxy-6-methyl-[4,4'-bipyridine]-3-carboxamide (white solid, 193.6 mg, yield: 66.3%). $^1$H NMR (400 MHz, DMSO-$d_6$) δ 13.16 (s, 1H), 8.86 (s, 1H), 8.32 (s, 1H), 8.17 (s, 1H), 7.60 (s, 1H), 7.49 (s, 1H), 7.32 (s, 1H), 3.95 (s, 3H), 3.61 (s, 3H), 3.59 (s, 3H), 2.62 (s, 3H), 1.88 (s, 3H). LC/MS (ESI) m/z: 536.1 $[M+H]^+$.

**Example 178: 2'-chloro-*N*-(6-(1,4-dimethyl-1*H*-parazol-5-yl)-5-oxo-4,5-dihydrothiazolo[4,5-*b*]pyridin-2-yl)-5'-methoxvy-6-methyl-[4,4'-bipyridine]-3-carboxamide**

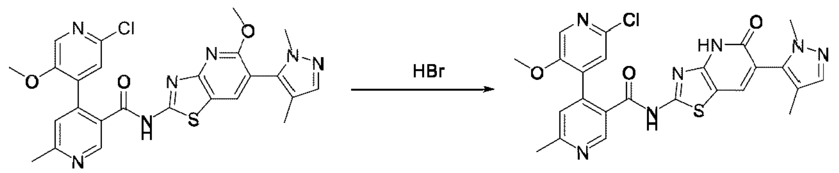


Hydrogen bromide solution (10 mL, 33% wt. in acetic acid) was added to 2'-chloro-*N*-(6-(1,4-dimethyl-1*H*-pyrazol-5-yl)-5-methoxythiazolo[4,5-*b*]pyridin-2-yl)-5'-methoxy-6-methyl-[4,4'-bipyridine]-3-carboxamide (100 mg, 0.187 mmol), and stirred at 50°C for 2 hours. After complete reaction, the reaction mixture was concentrated under

reduced pressure; the resulting residue was purified by pre-HPLC to obtain 2'-chloro-*N*-(6-(1,4-dimethyl-1*H*-pyrazol-5-yl)-5-oxo-4,5-dihydrothiazolo[4,5-*b*]pyridin-2-yl)-5'-methoxy-6-methyl-[4,4'-bipyridine]-3-carboxamide (white solid, 60.0 mg, yield: 61.6%). $^1$H NMR (400 MHz, DMSO-$d_6$) δ 13.35 (s, 1H), 8.90 (s, 1H), 8.19 (s, 1H), 8.10 (s, 1H), 7.60 (s, 1H), 7.57 (s, 1H), 7.31 (s, 1H), 3.64 (s, 3H), 3.62 (s, 3H), 2.64 (s, 3H), 1.91 (s, 3H). LC/MS (ESI) m/z: 522.1 [M+H]$^+$.

**Example 179: 2'-chloro-N-(6-(1,4-dimethyl-1H-parazol-5-yl)-4-oxo-4,5-dihydrothiazolo[4,5-c]pyridin-2-yl)-5'-methoxy-6-methyl-[4,4'-bipyridine]-3-carboxamide**

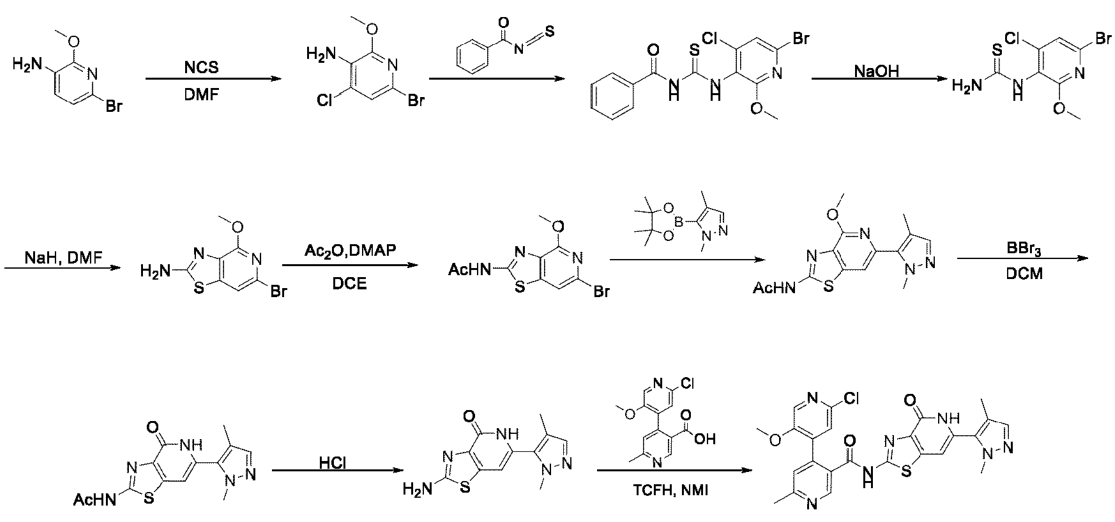


**Step 1: 6-bromo-4-chloro-2-methoxypyridin-3-amine**

6-bromo-2-methoxypyridin-3-amine (10 g, 49.2 mmol) was dissolved in N,N-dimethylformamide (20 mL); N-chlorosuccinimide (6.58 g, 49.2 mmol) was added; and the resulting mixture was stirred at room temperature for 3 hours. After complete reaction, the reaction mixture was extracted with water and ethyl acetate; the organic phase was concentrated under reduced pressure; and the residue was treated by column chromatography to obtain the title compound (8.4 g, creamy white solid, yield: 71.8%). LC/MS (ESI) m/z: 239.0 [M+H]$^+$.

**Step 2: N-((6-bromo-4-chloro-2-methoxypyridin-3-yl)carbamothioyl)benzamide**

6-bromo-4-chloro-2-methoxypyridin-3-amine (7.30 g, 30.7 mmol) was dissolved in acetone (50 mL); benzoyl isothiocyanate (7.52 g, 46.1 mmol) was added; and the mixture was stirred at room temperature for 3 hours.After complete reaction, the reaction mixture was filtered, washed, and dried to obtain the title compound (8.9 g, white solid, yield: 72.3%).LC/MS (ESI) m/z: 400.0 [M+H]$^+$.

**Step 3: 1-(6-bromo-4-chloro-2-methoxypyridin-3-yl)thiourea**

N-((6-bromo-4-chloro-2-methoxypyridin-3-yl)carbamothioyl)benzamide (8.70 g, 32.1 mmol) was dissolved in methanol/water (30/30 mL); sodium hydroxide (6.41 g, 160 mmol) was added; and the mixture was stirred at 60°C for 2 hours.After complete reaction, the reaction mixture was concentrated under reduced pressure; and the residue was adjusted to pH = 5, filtered, washed, and dried to obtain the title compound (6.0 g, pink solid, yield: 93.3%). LC/MS (ESI) m/z: 296.0 [M+H]$^+$.

**Step 4: 6-bromo-4-methoxythiazolo[4,5-c]pyridin-2-amine**

1-(6-bromo-4-chloro-2-methoxypyridin-3-yl)thiourea (3.00 g, 10.1 mmol) was dissolved in N,N-dimethylformamide (10 mL); sodium hydride (4.22 g, 29.1 mmol) was added; and the mixture was stirred at 60°C for 2 hours.After complete reaction, the reaction mixture was filtered, dried, washed and purified to obtain the title compound (2.0 g, pink solid, yield: 76.0%).LC/MS (ESI) m/z: 260.0 [M+H]$^+$.

**Step 5: N-(6-bromo-4-methoxythiazolo[4,5-c]pyridin-2-yl)acetamide**

6-bromo-4-methoxythiazolo[4,5-c]pyridin-2-amine (2 g, 7.69 mmol) was dissolved in dichloroethane (15 mL); 4-dimethylaminopyridine (0.190 g, 1.54 mmol) and di-tert-butyl dicarbonate (1.18 g, 11.5 mmol) were added; and the mixture was heated to 50°C and stirred for 2 hours. After complete reaction, the reaction mixture was concentrated under reduced pressure; and the residue was purified by column chromatography (eluent: petroleum ether/ethyl acetate, gradient: 30%-70% ethyl acetate) to obtain the title compound (1.3 g, pink solid, yield: 55.9%). LC/MS (ESI) m/z: 302.0 $[M+H]^+$.

**Step 5: N-(6-(1,4-dimethyl-1H-pyrazol-5-yl)-4-methoxythiazolo[4,5-c]pyridin-2-yl)acetamide**

N-(6-bromo-4-methoxythiazolo[4,5-c]pyridin-2-yl)acetamide (500 mg, 1.65 mmol), 1,4-dimethyl-5-(4,4,5,5-tetramethyl-1,3,2-dioxaborinan-2-yl)-1H-pyrazole (404 mg, 1.82 mmol), potassium carbonate (915 mg, 6.62 mmol) and dichloro[1,1'-di(diphenylphosphino)ferrocen]palladium (242 mg, 0.331 mmol) were dissolved in 1,4-dioxane/water (15/3 mL); and under the protection of nitrogen, the mixture was heated to 95°C to react for 2 hours. After complete reaction, the reaction mixture was concentrated under reduced pressure; and the residue was purified by column chromatography (eluent: dichloromethane/methanol, gradient: 0%-10% methanol) to obtain the title compound (120 mg, white solid, yield: 47.6%). LC/MS (ESI) m/z: 318.2 $[M+H]^+$.

**Step 6: N-(6-(1,4-dimethyl-1H-pyrazol-5-yl)-4-oxo-4,5-dihydrothiazolo[4,5-c]pyridin-2-yl)acetamide**

N-(6-(1,4-dimethyl-1H-pyrazol-5-yl)-4-methoxythiazolo[4,5-c]pyridin-2-yl)acetamide (100 mg, 0.379 mmol) was dissolved in hydrobromic acid in an acetic acid solution (5 mL), heated to100°C, and stirred for 4 hours. After complete reaction, the reaction mixture was filtered, washed, and dried to obtain the title compound (70 mg, yellow solid, yield: 53.4%). LC/MS (ESI) m/z: 304.1 $[M+H]^+$.

**Step 7: 2-amino-6-(1,4-dimethyl-1H-pyrazol-5-yl)thiazolo[4,5-c]pyridin-4(5H)-one**

N-(6-(1,4-dimethyl-1H-pyrazol-5-yl)-4-oxo-4,5-dihydrothiazolo[4,5-c]pyridin-2-yl)acetamide (170 mg, 0.231 mmol) was dissolved in methanol/water (3/1 mL); sodium hydroxide (46.2 mg, 1.15 mmol) was added; and the resulting mixture was heated to 60°C and stirred for 2 hours. After complete reaction, the reaction mixture was concentrated under reduced pressure; and the residue was purified by column chromatography (eluent: dichloromethane/methanol, gradient: 0%-10% methanol) to obtain the title compound (30 mg, white solid, yield: 49.8%). LC/MS (ESI) m/z: 262.2 $[M+H]^+$.

**Step 8: 2'-chloro-N-(6-(1,4-dimethyl-1H-pyrazol-5-yl)-4-oxo-4,5-dihydrothiazolo[4,5-c]pyridin-2-yl)-5 '-methoxy-6-methyl-[4,4'-bipyridine]-3-carboxamide**

2'-chloro-5'-methoxy-6-methyl-[4,4'-bipyridine]-3-carboxylic acid (30 mg, 0.108 mmol), 2-amino-6-(1,4-dimethyl-1H-pyrazol-5-yl)thiazolo[4,5-c]pyridin-4(5H)-one (28.1 mg, 0.108 mmol) and 1-methylimidazole (35.4 mg, 0.431 mmol) were dissolved in acetonitrile/N,N-dimethylformamide (4/1 mL); and the resulting mixture was stirred at 75°C for 15 minutes. Then, N,N,N',N'-tetramethylchlorformamidine hexafluorphosphate (33.2 mg, 0.118 mmol) dissolved in acetonitrile (1 mL) was added; and the mixture was stirred at 75°C for 1 hour. After complete reaction, the reaction mixture was concentrated under reduced pressure; and the residue was purified by reversed-phase preparation to obtain the title compound (4 mg, white solid, yield: 7.1%). $^1H$ NMR (400 MHz, DMSO-$d_6$) δ 13.23 (s, 1H), 11.70 (s, 1H), 8.89 (s, 1H), 8.16 (s, 1H), 7.54 (s, 1H), 7.41 (s, 1H), 7.34 (s, 1H), 6.90 (s, 1H), 3.75 (s, 3H), 3.62 (s, 3H), 2.59 (s, 3H), 2.00 (s, 3H). LC/MS (ESI) m/z: 522.2 $[M+H]^+$.

**Example 180: 2'-(difluoromethyl)-N-(6-(1,4-dimethyl-1H-1,2,3-triazol-5-yl)thiazolo[4,5-c]pyridin-2-yl) -5'-methoxy-6-methyl-[4,4'-bipyridine]-3-carboxamide**

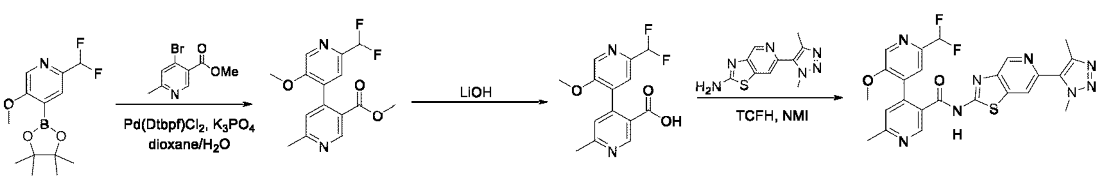

**Step 1: 2-difluoromethyl-5-methoxy-6-methyl-3-methyl carboxylate**

4-bromo-6-methylmethyl nicotinate (500 mg, 2.17 mmol), 2-difluoromethyl-5-methoxy-4-(4, 4, 5, 5-tetramethyl-1, 3, 2-dioxaboran-2-yl)pyridine (681.6 mg, 2.39 mmol), tripotassium phosphate (1.4 g, 6.52 mmol), and [1,1'-bis(di-tert-butylphosphino)ferrocen]palladium dichloride (140.3 mg, 0.22 mmol) were dissolved in 4 mL of water and 20 mL of 1,4-dioxane; and under the protection of nitrogen, the temperature was increased to 90°C, and the mixture was stirred for 2 hours. The reaction mixture was cooled to room temperature, and water was added to quench the reaction. The mixture was extracted with ethyl acetate three times; and the organic phases were combined, washed with saturated saline, dried over anhydrous sodium sulfate, and then filtered and concentrated. The residue was purified by column chromatography (eluent: petroleum ether/ethyl acetate, gradient: 0-60% ethyl acetate) to obtain the title compound (yellow solid, 600 mg, yield: 89.6%). LC/MS (ESI) m/z: 309 $[M+H]^+$.

**Step 2**: **2-difluoromethyl-5-methoxy-6-methyl-4,4-bipyridine-3-carboxylic acid**

2-difluoromethyl-5-methoxy-6-methyl-3-methyl carboxylate (400 mg, 1.3 mmol) was dissolved in 5 mL of water and 5 mL of tetrahydrofuran; and lithium hydroxide (77.9 mg, 1.95 mmol) was added, to allow for reaction at room temperature for 16 hours. The reaction mixture was concentrated under reduced pressure; and the residue was adjusted to pH of 4-5 using 2N hydrochloric acid, allowing for precipitation of solids, which were filtered and dried under reduced pressure to remove water, to obtain the title compound (white solid, 200 mg, yield: 52.4%). LC/MS (ESI) m/z: 295 $[M+H]^+$.

**Step 3: 2'-(difluoromethyl)-N-(6-(1,4-dimethyl-1H-1,2,3-triazol-5-yl)thiazolo[4,5-c]pyridin-2-yl)-5'-m ethoxy-6-methyl-[4,4'-bipyridine]-3-carboxamide**

6-(1,4-dimethyl-1H-1,2,3-triazol-5-yl)thiazolo[4, 5-c]pyridin-2-amine (50 mg, 0.2 mmol) and 2-difluoromethyl-5-methoxy-6-methyl-4,4-bipyridin-3-carboxylic acid (59.7 mg, 0.2 mmol) were dissolved in 5 mL of acetonitrile; N-methylimidazole (50 mg, 0.61 mmol) was added; with the temperature increased to 75°C, the resulting mixture was stirred for 20 minutes; then, N,N,N',N'-tetramethylchlorformamidine hexafluorphosphate (113.9 mg, 0.41 mmol) was added; and the temperature of the resulting mixture was increased to 75°C, and the mixture was stirred for 2 hours. Water was added to the reaction mixture to quench the reaction; the mixture was extracted with ethyl acetate three times; and the organic phases were combined. The organic phase was dried over anhydrous sodium sulfate, then filtered, and concentrated under reduced pressure; the residue was purified by reversed-phase preparation (eluent: acetonitrile/0.1% aqueous formic acid solution, gradient: 25%-90%) to obtain the title compound (white solid, 7 mg, yield: 6.6%). $^1$H NMR (400MHz,DMSO-$d_6$) δ 13.34 (s, 1H), 9.20 (d, $J$ = 0.7 Hz, 1H), 8.90 (s, 1H), 8.47 (s, 1H), 8.40 (d, $J$ = 0.8 Hz, 1H), 7.76 (s, 1H), 7.52 (s, 1H), 7.01 (t, $J$ = 55.0 Hz, 1H), 4.14 (s, 3H), 3.68 (s, 3H), 2.64 (s, 3H), 2.39 (s, 3H). LC/MS (ESI) m/z: 523.2 $[M+H]^+$.

**Example 181: N-(6-(1,2,4-oxadiazol-3-yl)thiazolo[4,5-c]pyridin-2-yl)-2'-chloro-5'-methoxy-6-methyl-[4,4'-bipyridine]-3-carboxamide**

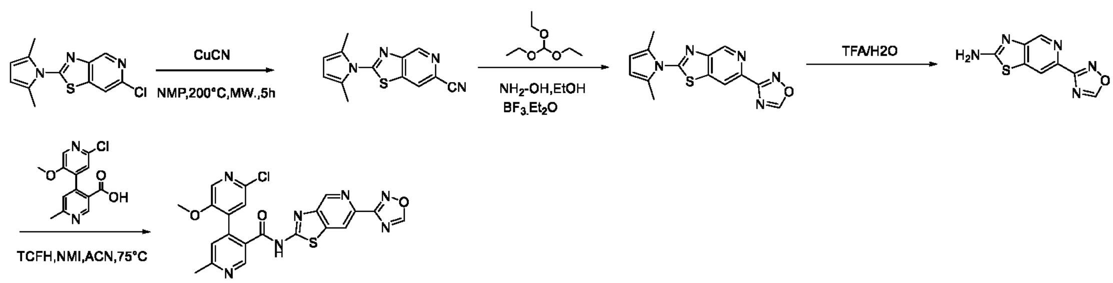


**Step 1: 2-(2,5-dimethyl-1H-pyrrol-1-yl)thiazolo[4,5-c]pyridin-6-nitrile**

At room temperature, 6-chloro-2-(2,5-dimethyl-1H-pyrrol-1-yl)thiazolo[4,5-c]pyridine (1.0 g, 3.79 mmol) was dissolved in N-methylpyrrolidone (10 mL); cuprous cyanide (0.68 g, 7.58 mmol) was added, to allow for reaction for 5 hours in the presence of microwaves at 200°C. After the reaction was completed, water was added; the reaction mixture was extracted with ethyl acetate, and washed with saturated saline; and the organic phase was directly concentrated under reduced pressure, subjected to sample blending, and allowed to pass through the columns (eluent: petroleum ether/ethyl acetate = 1/1, V/V) to obtain the title compound (faint yellow solid, 500 mg, yield: 46.7%). LC/MS (ESI) m/z: 255.2 $[M+H]^+$.

**Step 2: 3-(2-(2,5-dimethyl-1H-pyrrol-1-yl)thiazolo[4,5-c]pyridin-6-yl)-1,2,4-oxadiazole**

At room temperature, 2-(2,5-dimethyl-1H-pyrrol-1-yl)thiazolo[4,5-c]pyridin-6-nitrile (170 mg, 0.668 mmol) was dissolved in ethanol (2 mL); hydroxylamine (4 mL) was added; the resulting mixture was refluxed for 2 hours, and directly spin-dried; then, anhydrous dichloromethane, and triethyl orthoformate (396 mg, 2.67 mmol) were added; under the protection of nitrogen; boron trifluoride ethyl ether solution (9.49 mg, 0.067 mmol) was added, to allow the reaction to be continued for 2 hours at room temperature; after the reaction, water was added; the mixture was extracted with ethyl acetate, and washed with saturated saline; and the organic phase was directly concentrated under reduced pressure, subjected to sample blending, and allowed to pass through the columns (eluent: petroleum ether/ethyl acetate = 10/1, *V*/*V*) to obtain the title compound (faint yellow solid, 150 mg, yield: 75.5%). LC/MS (ESI) m/z: 298.1 $[M+H]^+$.

**Step 3: triethoxymethane 6-(1,2,4-oxadiazol-3-yl)thiazolo[4,5-c]pyridin-2-amine**

3-(2-(2,5-dimethyl-1H-pyrrol-1-yl)thiazolo[4,5-c]pyridin-6-yl)-1,2,4-oxadiazole (50 mg, 0.106 mmol) was dissolved in 5 mL of trifluoroacetic acid and 1 mL of water, to allow for reaction at 75°C for 1 hour. The reaction mixture was concentrated under reduced pressure, added with dichloromethane and concentrated, and this process was repeated three times to obtain the crude title compound (50 mg, yellow oil). LC/MS (ESI) m/z: 220.0 [M+H]+

**Step 4: N-(6-(1,2,4-oxadiazol-3-yl)thiazolo[4,5-c]pyridin-2-yl)-2'-chloro-5'-methoxy-6-methyl-[4,4'-bi pyridine]-3-carboxamide**

Triethoxymethane 6-(1,2,4-oxadiazol-3-yl)thiazolo[4,5-c]pyridin-2-amine (50 mg, 0.228 mmol), 2'-chloro-5'-methoxy-6-methyl-[4,4'-bipyridine]-3-carboxylic acid (95.3 mg, 0.342 mmol), and methylimidazole (56.2 mg, 0.684 mmol) were dissolved in 20 mL of acetonitrile and 1 mL of N,N-dimethylformamide to allow for reaction at 75°C for 5 minutes; and N,N,N',N'-tetramethylchlorformamidine hexafluorphosphate (96.0 mg, 0.342 mmol) was added to allow for reaction at 75°C for 60 minutes. Saturated ammonium chloride was added; the mixture was extracted with ethyl acetate; the organic phase was washed with water, washed with saline, and concentrated under reduced pressure; the residue was purified by prep-HPLC (eluent: acetonitrile/0.1% formic acid aqueous solution = 20%-90%, *V*/*V*) to obtain N-(6-(1,2,4-oxadiazol-3-yl) thiazolo[4,5-c]pyridin-2-yl)-2'-chloro-5'-methoxy-6-methyl-[4,4'-bipyridine]-3-c arboxamide (7.5 mg, white solid, yield: 6.8%). $^1$H NMR (400 MHz, DMSO-$d_6$) δ 13.39 (s, 1H), 9.75 (s, 1H), 9.18 (s, 1H), 8.88 (d, *J* = 12.3 Hz, 2H), 8.17 (s, 1H), 7.61 (s, 1H), 7.51 (s, 1H), 3.60 (s, 3H), 2.62 (s, 3H). LC/MS (ESI) m/z: 480.1 $[M+H]^+$.

**Example 182: N-(6-(1,4-dioxan-2-yl)thiazolo[4,5-b]pyridin-2-yl)-2'-chloro-5'-methoxy-6-methyl-[4,4'-bipyridine]-3-carboxamide**

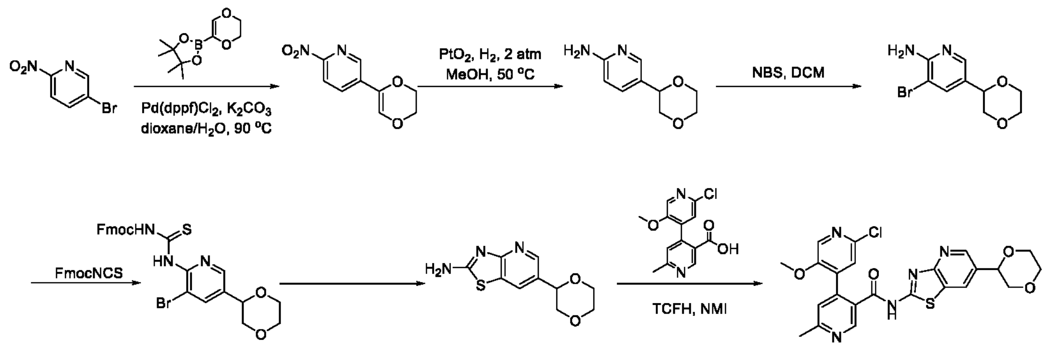


**Step 1: 5-(5,6-dihydro-1,4-dioxo-2-yl)-2-nitropyridine**

5-bromo-2-nitropyridine (500 mg, 2.46 mmol) and 2-(5,6-dihydro-1,4-dioxy-2-yl)-4,4,5,5-tetramethyl-1,3,2-dioxaborinan, potassium carbonate (1021 mg, 7.39 mmol) were dissolved in 25 mL of 1,4-dioxane and 5 mL of water; 1,1-bis(diphenylphosphino)ferrocene palladium dichloride (180 mg, 0.246 mmol) was added; and under the protection of nitrogen, the mixture was stirred at 90°C for 3 hours. The reaction mixture was concentrated under reduced pressure; ethyl acetate and water were added for stratification; the aqueous phase was extracted with ethyl acetate; and the organic phase was washed with water and with saline, and concentrated under reduced pressure to obtain residues, which were purified by silica-gel column chromatography (eluent: petroleum ether/ethyl acetate = 20, V/V) to obtain 5-(5,6-dihydro-1,4-dioxo-2-yl)-2-nitropyridine (550 mg of the crude product, yellow solid, yield: 107%). LC/MS (ESI) m/z: 209.1 $[M+H]^+$.

**Step 2**: **5-(1,4-dioxan-2-yl)pyridin-2-amine**

5-(5,6-dihydro-1,4-dioxo-2-yl)-2-nitropyridine (600 mg, 2.88 mmol) was dissolved in 50 mL of methanol; platinum dioxide (300 mg, 0.096 mmol) was added; and under the protection of a nitrogen balloon, the mixture was stirred at 50°C overnight. The reaction mixture was cooled to room temperature, and filtered through the padded diatomite; the filter cake was rinsed with methanol; and the filtrate was concentrated under reduced pressure to obtain 5-(1,4-dioxan-2-yl)pyridin-2-amine (500 mg, yellow oil, yield: 96.3%). LC/MS (ESI) m/z: 181.3 $[M+H]^+$.

**Step 3: 3-bromo-5-(1,4-dioxan-2-yl)pyridin-2-amine**

5-(1,4-dioxan-2-yl)pyridin-2-amine (400 mg, 2.22 mmol) was dissolved in 20 mL of dichloromethane; N-bromosuccinimide (435 mg, 2.44 mmol) was added; and the resulting mixture was stirred at room temperature for half an hour. The reaction mixture was concentrated under reduced pressure to obtain residues, which were purified by silica-gel column chromatography (eluent: dichloromethane/methanol = 50, V/V) to obtain 3-bromo-5-(1,4-dioxan-2-yl)pyridin-2-amine (420 mg, yellow solid, yield: 73%). LC/MS (ESI) m/z: 261.1 $[M+H]^+$.

**Step 4: 2-(3-(((9H-fluoren-9-yl)methoxy)carbonyl)thioureido)-3-bromo-5-(1,4-dioxan-2-yl)pyridine**

3-bromo-5-(1,4-dioxan-2-yl)pyridin-2-amine (420 mg, 1.62 mmol) was dissolved in 50 mL of acetone; 9-fmoc isothiocyanate (684 mg, 2.43 mmol) was added; and the resulting mixture was stirred at room temperature overnight. The reaction mixture was concentrated under reduced pressure to obtain residues, which were purified by silica-gel column chromatography (eluent: petroleum ether/ethyl acetate = 30, V/V) to obtain 2-(3-(((9H-fluoren-9-yl)methoxy)carbonyl)thioureido)-3-bromo-5-(1,4-dioxan-2-yl)pyridine (430 mg, yellow solid, yield: 30.4%). LC/MS (ESI) m/z: 540.1 $[M+H]^+$.

**Step 5: 6-(1,4-dioxan-2-yl)thiazolo[4,5-b]pyridin-2-amine**

2-(3-(((9H-fluoren-9-yl)methoxy)carbonyl)thioureido)-3-bromo-5-(1,4-dioxan-2-yl)pyridine (420 mg, 0.777 mmol), L-proline (89.5 mg, 0.777 mmol), and potassium carbonate (322 mg, 2.33 mmol) were dissolved in 50 mL of anhydrous 1,4-dioxane; cuprous iodide (74.00 mg, 0.389 mmol) was added; and under the protection of nitrogen, the mixture was stirred at 80°C for 3 hours. The reaction mixture was concentrated under reduced pressure to obtain residues, which were purified by silica-gel column chromatography (eluent: dichloromethane/methanol = 10, *V*/*V*) to obtain 6-(1,4-dioxan-2-yl)thiazolo[4,5-b]pyridin-2-amine (50 mg, yellow solid, yield: 27.1%). LC/MS (ESI) m/z: 238.3 $[M+H]^+$.

**Step 6: N-(6-(1,4-dioxan-2-yl)thiazolo[4,5-b]pyridin-2-yl)-2'-chloro-5'-methoxy-6-methyl-[4,4'-bipyrid ine]-3-carboxamide**

6-(1,4-dioxan-2-yl)thiazolo[4,5-b]pyridin-2-amine (50 mg, 0.211 mmol), 2'-chloro-5'-methoxy-6-methyl-[4,4'-bipyridine]-3-carboxylic acid (64.6 mg, 0.232 mmol) and methylimidazole (51.9 mg, 0.632 mmol) were dissolved in 4 mL of acetonitrile and 1 mL of N,N-dimethylformamide; the resulting mixture was stirred at 70°C for 5 minutes; 1 mL of N,N,N',N'-tetramethylchlorformamidine hexafluorphosphate in acetonitrile solution (65 mg, 0.232 mmol) was added dropwise; and the mixture was stirred at 70°C for 1 hour. The reaction mixture was purified by prep-HPLC (eluent: acetonitrile/0.1% formic acid aqueous solution = 0 - 50, V/V) to obtain N-(6-(1,4-dioxan-2-yl)thiazolo[4,5-b]pyridin-2-yl)-2'-chloro-5'-methoxy-6-methyl-[4,4'-bipyridine]-3-carbox amide (60 mg, white solid, yield: 57.2%). $^1H$ NMR (400 MHz, DMSO-$d_6$) $\delta$ 13.24 (s, 1H), 8.87 (s, 1H), 8.57 (d, $J$ = 2.0 Hz, 1H), 8.45 (d, $J$ = 1.9 Hz, 1H), 8.16 (s, 1H), 7.59 (s, 1H), 7.47 (s, 1H), 4.78 - 4.75 (m, 1H), 3.94 - 3.87 (m, 2H), 3.84 - 3.75 (m, 2H), 3.66 - 3.60 (m, 1H), 3.59 (s, 3H), 3.44 - 3.39 (m, 1H), 2.61 (s, 3H). LC/MS (ESI) m/z: 498.4 $[M+H]^+$.

**Example 183: 2'-chloro-N-(3-(4-hydroxycyclohexyl-1-en-1-yl)thiazolo[4,5-c]pyridazin-6-yl)-5'-methox y-6-methyl-[4,4'-bipyridine]-3-carboxamide**

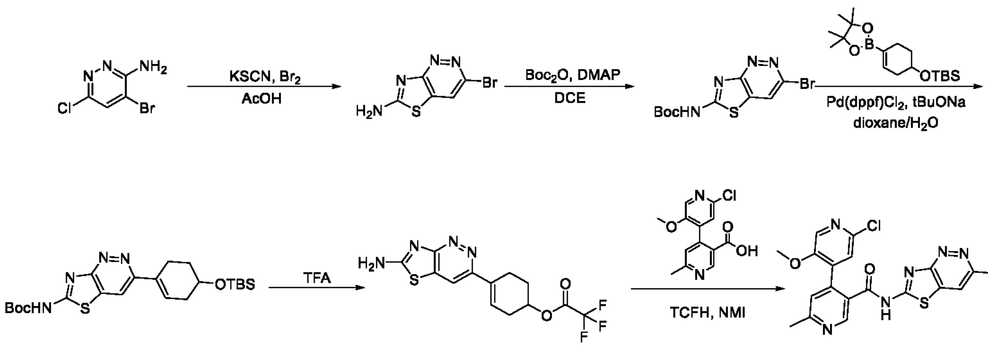


**Step 1: 3-bromothiazolo[4,5-c]pyridazin-6-amine**

Potassium thiocyanate (23.3 g, 240 mmol) and liquid bromine (3.1 mL, 60.5 mmol) were added to the solution of 4-bromo-6-chloropyridazin-3-amine (5 g, 24.0 mmol) in acetic acid (50 mL). Then, the mixture was stirred at 70°C for 64 hours. After complete reaction, the reaction mixture was filtered; the filter cake was rinsed with 50% methanol; and the filtrate was concentrated under reduced pressure to obtain the title compound (orange-yellow solid, 2.5 g, yield: 45.1%). LC/MS (ESI) m/z: 231.0 $[M+H]^+$.

**Step 2: (3-bromothiazolo[4,5-c]pyridazin-6-yl)aminotert-butyl carbamate**

Di-tert-butyl dicarbonate (4.72 g, 21.6 mmol) and 4-dimethylaminopyridine (0.26 g, 2.16 mmol) were added to the solution of 3-bromothiazolo[4,5-c]pyridazin-6-amine (2.5 g, 10.8 mmol) in dichloromethane (30 mL). Then, the resulting mixture was allowed to react at room temperature for 2 hours. After complete reaction, the reaction mixture was concentrated under reduced pressure; and the residue was subjected to reversed-phase purification (eluent: water/-acetonitrile, gradient: 0-100% acetonitrile) to obtain the title compound (yellow solid, 800 mg, yield: 22.4%). LC/MS (ESI) m/z: 333.0 $(M+2H)^+$.

**Step 3: tert-butyl(3-(4-((tert-butyldimethylsilicyl)oxy)cyclohex-1-en-1-yl)thiazolo[4,5-c]pyridazin-6-y l)carbamate**

Tert-butyldimethyl((4-(4,4,5,5-tetramethyl-1,3,2-dioxaborinan-2-yl)cyclohex-3-en-1-yl)oxy)silane (123 mg, 0.364 mmol), sodium tert-butoxide (116 mg, 1.21 mmol) and [1,1'-bis(diphenylphosphino)ferrocen]palladium dichloride (44 mg, 0.060 mmol) were sequentially added to (3-bromothiazolo[4,5-c]pyridazin-6-yl)aminotert-butyl carbamate (100 mg, 0.302 mmol) in the mixed solution of 1,4-dioxane (10 mL) and water (0.5 mL). Then, the mixture was stirred at 95°C for 2 hours in the presence of nitrogen. After complete reaction, the reaction mixture was filtered and concentrated under reduced pressure. The residue was purified by column chromatography (eluent: dichloromethane/methanol, gradient: 0-5% methanol) to obtain the title compound (faint yellow solid, 75 mg, yield: 53.7%). LC/MS (ESI) m/z: 463.2 $[M+H]^+$.

**Step 4: 4-(6-aminothiazolo[4,5-c]pyridazin-3-yl)cyclohex-3-en-1-yl2,2,2-trifluoroacetate**

Trifluoroacetic acid (2 mL, 26.1 mmol) was added to the solution of tert-butyl(3-(4-((tert-butyldimethylsilicyl)oxy) cyclohex-1-en-1-yl)thiazolo[4,5-c]pyridazin-6-yl)carbamate (75 mg, 0.162 mmol) in dichloromethane (2 mL). The reaction mixture was stirred at room temperature to react for 2 hours. After complete reaction, the reaction mixture was concentrated under reduced pressure. The residue was purified by column chromatography (eluent: dichloromethane/-methanol, gradient: 0-5% methanol) to obtain the title compound (faint yellow solid, 55 mg, yield: 98.5%). LC/MS (ESI) m/z: 345.1 $[M+H]^+$.

**Step 5: 2'-chloro-N-(3-(4-hydroxycyclohexyl-1-en-1-yl)thiazolo[4,5-c]pyridazin-6-yl)-5'-methoxy-6-me thyl-[4,4'-bipyridine]-3-carboxamide**

2'-chloro-5'-methoxy-6-methyl-[4,4'-bipyridine]-3-carboxylic acid (67 mg, 0.240 mmol) and N-methylimidazole (39 mg, 0.475 mmol) were sequentially added to 4-(6-aminothiazolo[4,5-c]pyridazin-3-yl)cyclohex-3-en-1-yl2,2,2-trifluoroacetate (55 mg, 0.16 mmol) in the mixed solution of N,N-dimethylformamide (3 mL) and acetonitrile (5 mL). Then, at 70°C,N,N,N',N'-tetramethylchlorformamidine hexafluorphosphate (54 mg, 0.192 mmol) was added to the mixture. The mixture was stirred at 70°C for 2 hours. After complete reaction, water was added; the resulting mixture was extracted with ethyl acetate; the organic phase was washed with saturated saline, dried over anhydrous sodium sulfate, and concentrated under reduced pressure. The residue was purified by pre-HPLC to obtain the title compound (white solid, 10.9 mg, yield: 13.4%). $^1$H NMR (400 MHz, DMSO-$d_6$) δ 13.51 (s, 1H), 8.90 (s, 1H), 8.52 (s, 1H), 8.17 (s, 1H), 7.61 (s, 1H), 7.49 (s, 1H), 6.64 (t, *J* = 3.6 Hz, 1H), 4.74 (d, *J* = 4.0 Hz, 1H), 3.91 - 3.81 (m, 1H), 3.62 (s, 3H), 2.87 - 2.76 (m, 1H), 2.62 (s, 3H), 2.60-2.52 (m, 2H), 2.21 - 2.10 (m, 1H), 1.99 - 1.89 (m, 1H), 1.73 - 1.61 (m, 1H). LC/MS (ESI) m/z: 509.2 [M+H]$^+$.

The example compounds in Table 11 were synthesized using commercially available raw materials with reference to the synthesis steps of Example 183.

Table 11:

| Examples | Chemical structure | LC/MS(ESI) m/z [M+H]$^+$ | $^1$H NMR |
|---|---|---|---|
| 184 | 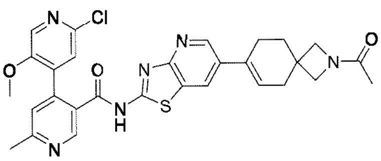 | 575.2 | $^1$H NMR (400 MHz, DMSO-$d_6$) δ 13.20 (s, 1H), 8.87 (s, 1H), 8.66 (d, *J* = 2.0 Hz, 1H), 8.46 (d, *J* = 2.0 Hz, 1H), 8.17 (s, 1H), 7.59 (s, 1H), 7.47 (s, 1H), 6.23 (t, *J* = 3.6 Hz, 1H), 3.91 (d, *J* = 8.0 Hz, 1H), 3.81 (d, *J* = 8.0 Hz, 1H), 3.64 (d, *J* = 9.2 Hz, 1H), 3.60 (s, 3H), 3.54 (d, *J* = 9.2 Hz, 1H), 2.61 (s, 3H), 2.59 - 2.53 (m, 2H), 2.49 - 2.44 (m, 2H), 1.97 - 1.88 (m, 2H), 1.76 (s, 3H). |
| 185 | 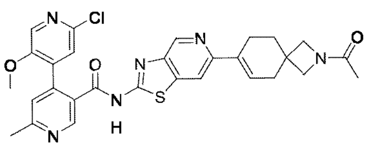 | 575.2 | $^1$H NMR (400 MHz, DMSO-$d_6$) δ13.14 (s, 1H), 8.95 (s, 1H), 8.87 (s, 1H), 8.17 (s, 1H), 8.15 (s, 1H), 7.58 (s, 1H), 7.47 (s, 1H), 6.72 (t, *J* = 3.6 Hz, 1H), 3.90 (d, *J* = 8.4 Hz, 1H), 3.80 (d, *J* = 8.0 Hz, 1H), 3.63 (d, *J* = 9.2 Hz, 1H), 3.60 (s, 3H), 3.53 (d, *J* = 9.2 Hz, 1H), 2.65 - 2.62 (m, 1H), 2.61 (s, 3H), 2.60 - 2.56 (m, 1H), 2.49 - 2.45 (m, 2H), 1.96 - 1.87 (m, 2H), 1.76 (s, 3H). |

**Example 186: 2-chloro-N-(6-(4-(diethylphospho)phenyl)thiazolo[4,5-b]pyridin-2-yl)-5'-methoxy-6-me thyl-[4,4'-bipyridine]-3-carboxamide**

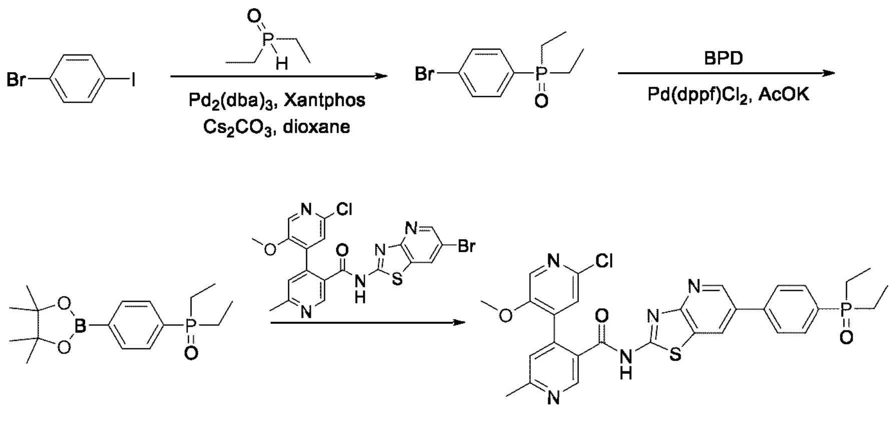


**Step 1: (4-bromophenyl)diethylphosphine oxide**

P-bromoiodobenzene (1g, 3.55mmol), diethylphosphine oxide (450mg, 4.24mmol), cesium carbonate (1.6g, 4.95mmol), 4,5-bisdiphenylphosphino-9,9-dimethylxanthene(150mg, 0.27mmol) and tris(dibenzylideneacetone)dipalladium (80 mg, 0.09 mmol) were dissolved in 50 mL of anhydrous 1,4-dioxane; and under the protection of nitrogen, with the temperature increased to 105°C, the mixture was stirred for 2-2.5 hours. The reaction mixture was cooled to room temperature, and water was added to quench the reaction. The mixture was extracted with ethyl acetate three times; and the organic phases were combined, washed with saturated saline, dried over anhydrous sodium sulfate, and then filtered and concentrated. The residue was purified by column chromatography (eluent: dichloromethane/methanol, gradient: 0-7% methanol, absorption peak monitoring at 220nm) to obtain the title compound (yellow solid, 900 mg, yield: 97.5%).

LC/MS (ESI) m/z:262.2 [M+H]+.

**Step 2: diethyl(4-(4,4,5,5-tetramethyl-1,3,2-dioxaborinan-2-yl)phenyl)phosphine oxide**

(4-bromophenyl)diethylphosphine oxide (850mg, 3.26 mmol), bis(pinacolato)diboron(1.65g, 6.51 mmol), potassium acetate (638.9mg, 6.51 mmol), and [1,1'-bis(diphenylphosphino)ferrocen]palladium dichloride (285.9mg, 0.39 mmol) were dissolved in 40 mL of anhydrous 1,4-dioxane; and under the protection of nitrogen, with the temperature increased to 100°C, the mixture was stirred for 3 hours. The reaction mixture was cooled to room temperature, and water was added to quench the reaction. The mixture was extracted with ethyl acetate three times; and the organic phases were combined, washed with saturated saline, dried over anhydrous sodium sulfate, and then filtered and concentrated. The residue was purified by column chromatography (eluent: dichloromethane/methanol, gradient: 0-5% methanol, absorption peak monitoring at 220nm) to obtain the title compound (yellow solid, 550 mg, yield: 55%). LC/MS (ESI) m/z: 309.0 [M+H]+.

**Step 3: 2-chloro-N-(6-(4-(diethylphospho)phenyl)thiazolo[4,5-b]pyridin-2-yl)-5'-methoxy-6-methyl-[4, 4'-bipyridine]-3-carboxamide**

N-(6-bromothiazolo[4, 5-b]pyridin-2-yl)-2'-chloro-5'-methoxy-6-methyl-[4, 4'-bipyridine]-3-carboxamide (100 mg, 0.2 mmol), diethyl(4-(4, 4, 5, 5-tetramethyl-1, 3, 2-dioxaborinan-2-yl)phenyl)phosphine oxide (69 mg, 0.22 mmol), tripotassium phosphate (129 mg, 0.60 mmol), and [1,1'-bis(di-tert-butylphosphino)ferrocen]palladium dichloride (13.1 mg, 0.02 mmol) were dissolved in 2 mL of water and 10 mL of 1,4-dioxane; and under the protection of nitrogen, with the temperature increased to 100°C, the mixture was stirred for 3 hours. The reaction mixture was cooled to room temperature, and water was added to quench the reaction. The mixture was extracted with ethyl acetate three times; and the organic phases were combined, washed with saturated saline, dried over anhydrous sodium sulfate, and then filtered and concentrated. The residue was purified by reversed-phase preparation (eluent: acetonitrile/0.1% formic acid aqueous solution, gradient: 25%-90%) to obtain the title compound (white solid, 42 mg, yield: 34.8%). 1HNMR(400MHz,DMSO-$d_6$) $\delta$ 13.29 (s, 1H), 8.94 (s, 1H), 8.90 (s, 1H), 8.81 (s, 1H), 8.18 (s, 1H), 7.94 - 7.91 (m, 2H), 7.88 - 7.83 (m, 2H), 7.60 (s, 1H), 7.47 (s, 1H), 3.62 (s, 3H), 2.61 (s, 3H), 2.03 - 1.92 (m, 4H), 1.01 - 0.92 m, 6H). LC/MS (ESI) m/z: 592.4 [M+H]+.

The example compounds in Table 12 were synthesized using commercially available raw materials with reference to the synthesis steps of Example 186.

Table 12:

| Examples | Chemical structure | LC/MS(ESI) m/z [M+H]+ | 1H NMR |
|---|---|---|---|
| 187 | 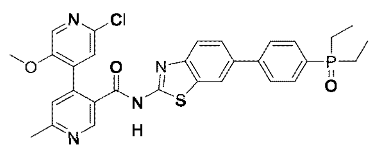 | 591.8 | 1HNMR(400MHz,DMSO-$d_6$) $\delta$ 13.02 (s, 1H), 8.88 (s, 1H), 8.38 (s, 1H), 8.17 (s, 1H), 7.91 - 7.88 (m, 1H), 7.87 - 7.86 (m, 1H), 7.85 - 7.83 (m, 2H), 7.82 - 7.77 (m, 2H), 7.58 (s, 1H), 7.46 (s, 1H), 3.62 (s, 3H), 2.61 (s, 3H), 2.05 - 1.85( m, 4H), 1.01 - 0.93 (m, 6H) |
| 188 | 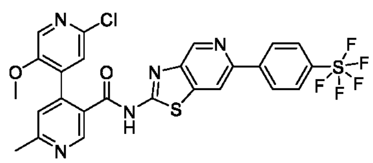 | 614.1 | 1HNMR(DMSO-$d_6$) $\delta$ 13.30 (s, 1H), 9.16 (s, 1H), 8.89 (s, 1H), 8.80 (s, 1H), 8.35 (d, *J* = 8.6 Hz, 2H), 8.18 (s, 1H), 8.04 (d, *J* = 9.0 Hz, 2H), 7.61 (s, 1H), 7.49 (s, 1H), 3.61 (s, 3H), 2.62 (s, 3H). |
| 189 | 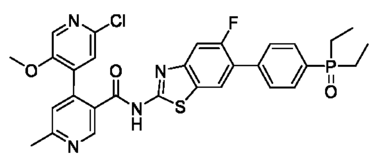 | 609.3 | 1HNMR(400MHz,DMSO-$d_6$) $\delta$ 13.10(s, 1H), 8.89 (s, 1H), 8.25 - 8.18 (m, 1H), 8.17 (s, 1H), 7.88 - 7.81 (m, 2H), 7.78 - 7.74 (m, 2H), 7.74 - 7.70 (m, 1H), 7.58 (s, 1H), 7.46 (s, 1H), 3.61 (s, 3H), 2.61 (s, 3H), 2.02 - 1.93 (m, 4H), 1.03 - 0.93 m, 6H) |
| 190 | 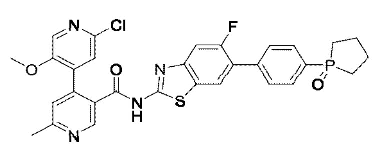 | 607.2 | 1HNMR(400MHz,DMSO-$d_6$) $\delta$ 13.11 (s, 1H), 8.88 (s, 1H), 8.21 (d, *J* = 7.6 Hz, 1H), 8.17 (s, 1H), 7.91 - 7.82 (m, 2H), 7.78 - 7.74 (m, 2H), 7.74 - 7.72 (m, 1H), 7.59 (s, 1H), 7.47 (s, 1H), 3.61 (s, 3H), 2.61 (s, 3H), 2.11 - 1.99 (m, 4H), 1.99 - 1.85 (m, 4H) |

**Example 191**: ***N*-(5-benzoyl-4.5.6,7-tetrahydrothiazolo[5.4]pyridin-2-yl)-2'-chloro-5'-methoxy-6-meth yl-[4,4'-bipyridine]-3-carboxamide**

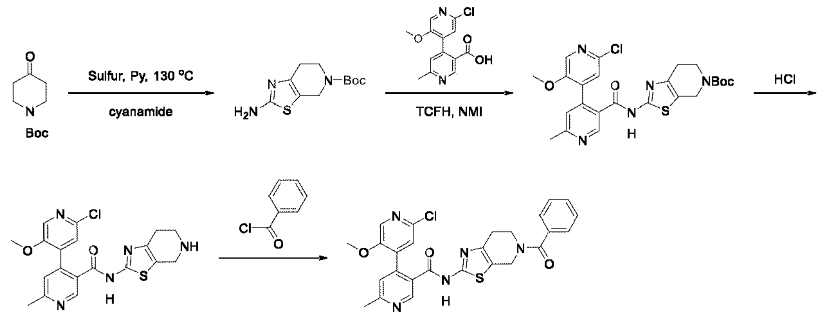


**Step 1: 2-amino-6,7-dihydrothiazol[5,4]pyridin-5(4H)-tert-butyl carboxylate**

Cyanamide (2.11 g, 50.2 mmol) and sulfur powder (1.61 g, 6.27 mmol) were added to the solution of 4-oxopiperidin-1-tert-butyl carbamate (5 g, 25.1 mmol) in pyridine (40 mL). The reaction mixture was stirred at 130°C to react for 1.5 hours. After complete reaction, the reaction mixture was filtered; the filter cake was rinsed with a small amount of ethyl acetate to obtain the title compound (white solid, 1.34 g, yield: 20.9%). LC/MS (ESI) m/z: 256.1 $[M+H]^+$.

**Step 2: 2-(2'-chloro-5'-methoxy-6-methyl-[4,4'-bipyridine]-3-formamido)-6,7-dihydrothiazolo[5,4-c]p yridin-5(4H)-tert-butyl carboxylate**

2'-chloro-5'-methoxy-6-methyl-[4,4'-bipyridine]-3-carboxylic acid (655 mg, 2.35 mmol) and N-methylimidazole (482 mg, 5.87 mmol) were sequentially added to 2-amino-6,7-dihydrothiazol[5,4]pyridin-5(4H)-tert-butyl carboxylate (500 mg, 1.96 mmol) in the mixed solution of N,N-dimethylformamide (12 mL) and acetonitrile (20 mL). Then, at 70°C, N,N,N',N'-tetramethylchlorformamidine hexafluorphosphate (659 mg, 2.35 mmol) was added to the mixture. The mixture was stirred at 70°C for 2 hours. After complete reaction, water was added; the resulting mixture was extracted with ethyl acetate; the organic phase was washed with saturated saline, dried over anhydrous sodium sulfate, and concentrated under reduced pressure. The residue was purified by column chromatography (eluent: dichloromethane/methanol, gradient: 0-10% methanol) to obtain the title compound (faint yellow solid, 1 g, yield: 99.0%). LC/MS (ESI) m/z: 516.3 $[M+H]^+$.

**Step 3**: **2'-chloro-5'-methoxy-6-methyl-N-(4,5,6,7-tetrahydrothiazolo[5,4-c]pyridin-2-yl)-[4,4'-bipyridi ne]-3-carboxamide**

The solution of 2-(2'-chloro-5'-methoxy-6-methyl-[4,4'-bipyridine]-3-formamido)-6,7-dihydrothiazolo[5,4-c]pyridin-5(4H)-t ert-butyl carboxylate (1 g, 1.938 mmol) in hydrochloric acid dioxane (10 mL, 4 M) was stirred at room temperature to allow for reaction for 2 hours. After complete reaction, the reaction mixture was concentrated under reduced pressure to obtain the title compound (yellow solid, 800 mg, yield: 99.3%). LC/MS (ESI) m/z: 416.1 $[M+H]^+$.

**Step 4: N-(5-benzoyl-4,5,6,7-tetrahydrothiazolo[5,4]pyridin-2-yl)-2'-chloro-5'-methoxy-6-methyl-[4,4' -bipyridine]-3-carboxamide**

Triethylamine (0.1 mL, 0.721 mmol) was added to the solution of 2'-chloro-5'-methoxy-6-methyl-N-(4,5,6,7-tetrahydrothiazolo[5,4-c]pyridin-2-yl)-[4,4'-bipyridine]-3-carboxa mide (100 mg, 0.240 mmol) in dichloromethane (5 mL); and then, benzoyl chloride (51 mg, 0.363 mmol) was added to the mixture at 0°C. The mixture was stirred at 0°C for 1 hour. After complete reaction, water was added; the resulting mixture was extracted with dichloromethane; and the organic phase was washed with saturated saline, dried over anhydrous sodium sulfate, and concentrated under reduced pressure. The residue was purified by pre-HPLC to obtain the title compound (white solid, 106.7 mg, yield: 85.3%). $^1H$ NMR (400 MHz, DMSO-$d_6$) δ 12.63 (s, 1H), 8.77 (s, 1H), 8.16 (s, 1H), 7.53 (s, 1H), 7.51 - 7.43 (m, 5H), 7.42 (s, 1H), 4.88 - 4.48 (m, 2H), 4.10-3.62 (m, 2H), 3.61 (s, 3H), 2.82 - 2.71 (m, 2H), 2.58 (s, 3H). LC/MS (ESI) m/z: 520.2 $[M+H]^+$.

**Example 192: 2'-chloro-5'-methoxy-6-methyl-N-(5-(pyridin-4-ylmethyl)-4,5,6,7-tetrahydrothiazolo[5, 4-c]pyridin-2-yl)-[4,4'-bipyridine]-3-carboxamide**

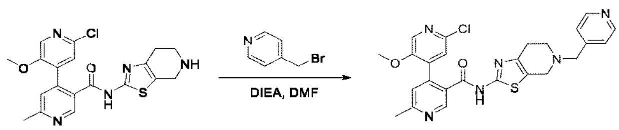


**Step 1: 2'-chloro-5'-methoxy-6-methyl-N-(5-(pyridin-4-ylmethyl)-4,5,6,7-tetrahydrothiazolo[5,4-c]pyr idin-2-yl)-[4,4'-bipyridine]-3-carboxamide**

N,N-diisopropylethylamine (186 mg, 1.44 mmol) and 4-(bromomethyl)pyridine hydrochloride (62 mg, 0.360 mmol) were added to the solution of 2'-chloro-5'-methoxy-6-methyl-N-(4,5,6,7-tetrahydrothiazolo[5,4-c]pyridin-2-yl)-[4,4'-bipyridine]-3-carboxa mide (100 mg, 0.240 mmol) in N,N-dimethylformamide (2 mL). The reaction mixture was stirred at room temperature to react for 5 hours. After complete reaction, water was added; the resulting mixture was extracted with ethyl acetate; and the organic phase was washed with saturated saline, dried over anhydrous sodium sulfate, and concentrated under reduced pressure. The residue was purified by pre-HPLC to obtain the title compound (yellow solid, 8.2 mg, yield: 6.83%). $^1$H NMR (400 MHz, DMSO-$d_6$) δ 12.54 (s, 1H), 8.77 (s, 1H), 8.52 (d, $J$ = 6.0 Hz, 2H), 8.16 (s, 1H), 7.51 (s, 1H), 7.41 (s, 1H), 7.37 (d, $J$ = 5.6 Hz, 2H), 3.74 (s, 2H), 3.61 (s, 3H), 3.60-3.53 (m, 2H), 2.83 - 2.75 (m, 2H), 2.71 - 2.66 (m, 2H), 2.58 (s, 3H). LC/MS (ESI) m/z: 507.2 [M+H]$^+$.

**Example 193: 2'-chloro-N-(6-(3,5-dimethylisooxazol-4-yl)thiazolo[4,5-c]pyridin-2-yl)-5'-methoxy-6-m ethyl-[4,4'-bipyridine]-3-carboxamide**

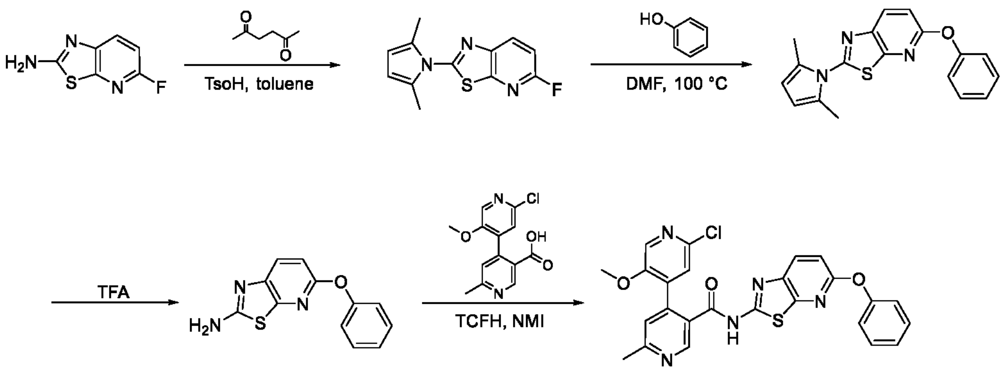


**Step 1: 2-(2,5-dimethyl-1H-pyrrol-1-yl)-5-fluorothiazolo[5,4-b]pyridine**

In the atmosphere of nitrogen, p-toluenesulfonic acid (0.20 g, 1.18 mmol) was added to the solution of 5-fluorothiazolo[5,4-b]pyridin-2-amine (2 g, 11.8 mmol) and 2,5-hexanedione (2.77 mL, 23.6 mmol) in dried toluene (10 mL); and the reaction mixture was stirred at 120°C for 16 hours under a water dispenser. After complete reaction, the reaction mixture was cooled to room temperature; sodium bicarbonate was added; and the resulting mixture was extracted with ethyl acetate, washed with saturated saline, dried over anhydrous sodium sulfate, and concentrated under reduced pressure. The residue was purified by column chromatography (eluent: petroleum ether/ethyl acetate, gradient: 0-10% ethyl acetate) to obtain the title compound (brown oil, 2.1 g, yield: 71.8%). LC/MS (ESI) m/z: 248 [M+H]$^+$

**Step 2: 2-(2,5-dimethyl-1H-pyrrol-1-yl)-5-phenoxythiazolo[5,4-b]pyridine**

Phenol (0.27 mL, 3.03 mmol) was added to the solution of 2-(2,5-dimethyl-1H-pyrrol-1-yl)-5-fluorothiazolo[5,4-b] pyridine (0.50 g, 2.02 mmol) and cesium carbonate (1.98 g, 6.07 mmol) in N-methylpyrrolidone (10 mL); and the reaction mixture was stirred at 100°C for 4 hours. After complete reaction, the reaction mixture was cooled to room temperature; water was added; and the resulting mixture was extracted with ethyl acetate, washed with saturated saline, dried over

anhydrous sodium sulfate, and concentrated under reduced pressure. The residue was purified by column chromatography (eluent: petroleum ether/ethyl acetate, gradient: 0-10% ethyl acetate) to obtain the title compound (colorless oil, 600 mg, yield: 92.3%). LC/MS (ESI) m/z: 322 $[M+H]^+$

**Step 3**: **6-(3,5-dimethylisooxazol-4-yl)thiazolo[4,5-c]pyridin-2-amine**

Trifluoroacetic acid (3 mL) was added to 2-(2,5-dimethyl-1H-pyrrol-1-yl)-5-phenoxythiazolo[5,4-b]pyridine (600 mg, 1.867 mmol) in water (3 mL). The mixture was stirred at 80°C to react for 2 hours. After complete reaction, the reaction mixture was cooled to room temperature; water was added; and the resulting mixture was extracted with ethyl acetate, washed with saturated saline, dried over anhydrous sodium sulfate, and concentrated under reduced pressure. The residue was purified by column chromatography (eluent: petroleum ether/ethyl acetate, gradient: 30-60% ethyl acetate) to obtain the title compound (brown solid, 400 mg, yield: 88.0%). LC/MS (ESI) m/z: 244 $[M+H]^+$

**Step 4: 2'-chloro-N-(6-(3,5-dimethylisooxazol-4-yl)thiazolo[4,5-c]pyridin-2-yl)-5'-methoxy-6-methyl-[4,4'-bipyridine]-3-carboxamide**

N-methylimidazole (203 mg, 2.48 mmol) was added to the solution of 2'-chloro-5'-methoxy-6-methyl-[4,4'-bipyridine]-3-carboxylic acid (230 mg, 0.825 mmol) and 5-phenoxythiazolo[5,4-b]pyridin-2-amine (200.78 mg, 0.825 mmol) in acetonitrile (10 mL); and the reaction mixture was stirred at 70°C for 10 minutes. Afterwards, N,N,N',N'-tetramethylchlorformamidine hexafluorphosphate (232 mg, 0.83 mmol) was added to the reaction mixture, and stirred at 70°C for 16 hours. After complete reaction, the reaction mixture was cooled to room temperature, and concentrated under reduced pressure. The residue was purified by C18 preparation to obtain the title compound (white solid, 69.3 mg, yield: 16.7%). LC/MS (ESI) m/z: 504 $[M+H]^+$; $^1$H NMR (400 MHz, DMSO-$d_6$) δ 12.98 (s, 1H), 8.83 (s, 1H), 8.21 (d, $J$ = 8.7 Hz, 1H), 8.15 (s, 1H), 7.57 (s, 1H), 7.47 - 7.42 (m, 3H), 7.24 (t, $J$ = 7.4 Hz, 1H), 7.20-7.16 (m, 2H), 7.14 (d, $J$ = 8.7 Hz, 1H), 3.60 (s, 3H), 2.60 (s, 3H).

**Example 194: 2'-chloro-5'-methoxy-6-methyl-*N*-(8-(1-methyl-1*H*-parazol-5-yl)-7,8-dihydro-6*H*-thiazo lo[5',4':4,5] benzo[1,2-b][1,4]oxazin-2-yl)-[4,4'-bipyridine]-3-carboxamide**

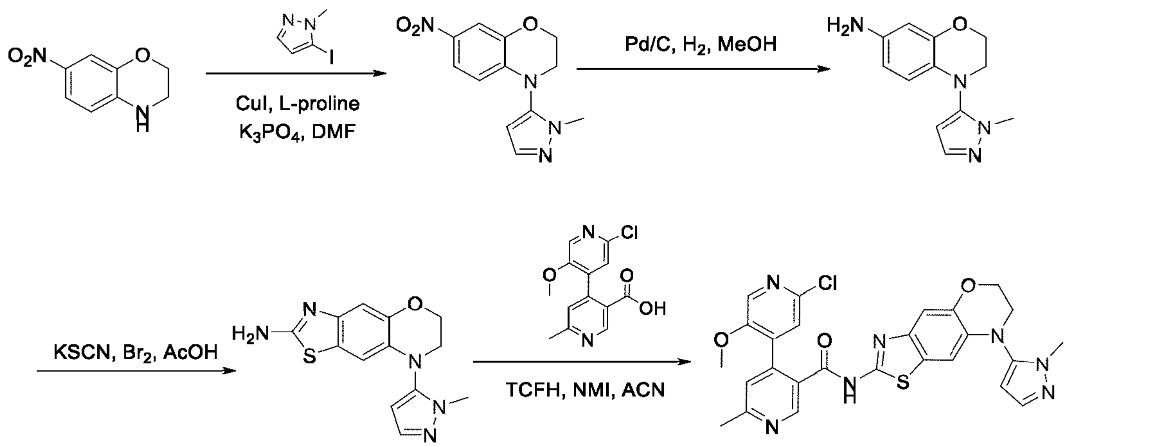


**Step 1: 4-(1-methyl-1*H*-parazol-5-yl)-7-nitro-3,4-dihydro-2*H*-benzo[*b*][1,4]oxazine**

Cuprous iodide (0.53 g, 2.78 mmol), L-proline (0.64 g, 5.55 mmol) and potassium phosphate (3.53 g, 16.65 mmol) were added to the solution of 7-nitro-3,4-dihydro-2H-1,4-benzoisooxazine (1.00 g, 5.55 mmol) in N,N-dimethylformamide (50 mL). Under the protection of nitrogen, the reaction mixture was stirred at 120°C to react for 12 hours. After complete reaction, the reaction mixture was cooled to room temperature; water was added; and the resulting mixture was extracted with ethyl acetate, washed with saturated saline, dried over anhydrous sodium sulfate, and concentrated under reduced pressure. The residue was purified by silica-gel chromatography (eluent: petroleum ether/ethyl acetate, gradient: 0-50% ethyl acetate) to obtain 4-(1-methyl-1*H*-pyrazol-5-yl)-7-nitro-3,4-dihydro-2H-benzo[b][1,4]oxazine (yellow solid, 0.60 g, yield: 41.7%). LC/MS (ESI) m/z: 261.1 $[M+H]^+$.

**Step 2**: **4-(1-methyl-1*H*-parazol-5-yl)-3,4-dihydro-2*H*-benzo[*b*][1,4]oxazin-7-amine**

Palladium on carbon (490.7 mg, 0.46 mmol) was added to the solution of 4-(1-methyl-1*H*-pyrazol-5-yl)-7-nitro-3,4-dihydro-2*H*-benzo[*b*][1,4]oxazine (600.0 mg, 2.31 mmol) in methanol; and in the atmosphere of hydrogen, the reaction mixture was stirred at room temperature for 5 hours. After complete reaction, the reaction mixture was concentrated under reduced pressure, to obtain a crude product 4-(1-methyl-1*H*-pyrazol-5-yl)-3,4-dihydro-2*H*-benzo[*b*][1,4]oxazin-7-amine (yellow solid, 470.0 mg, yield: 88.5%), which was used directly in the next step. LC/MS (ESI) m/z: 231.1 $[M+H]^+$.

**Step 3**: **8-(1-methyl-1*H*-parazol-5-yl)-7,8-dihydro-6*H*-thiazol[5',4':4,5]benzo[1,2-*b*][1,4]oxazin-2-amine**

At 0°C, 4-(1-methyl-1H-pyrazol-5-yl)-3,4-dihydro-2*H*-benzo[*b*][1,4]oxazin-7-amine (100 mg, 0.43 mmol) was added to the solution of potassium thiocyanate (211.0 mg, 2.17 mmol) in acetic acid (5 mL); and then, the solution of liquid bromine (138.8 mg, 0.87 mmol) in acetic acid (5 mL) was added dropwise slowly. The reaction mixture was stirred at room temperature for 12 hours. After complete reaction, water was added; and the resulting mixture was extracted with ethyl acetate, washed with saturated saline, dried over anhydrous sodium sulfate, and concentrated under reduced pressure. The residue was purified by silica-gel chromatography (eluent: dichloromethane/methanol, gradient: 10-30% methanol) to obtain 8-(1-methyl-1*H*-pyrazol-5-yl)-7,8-dihydro-6*H*-thiazol[5',4':4,5]benzo[1,2-*b*][1,4]oxazin-2-amine (yellow solid, 60.0 mg, yield: 48.1%). LC/MS (ESI) m/z: 288.0 $[M+H]^+$.

**Step 4: 2'-chloro-5'-methoxy-6-methyl-*N*-(8-(1-methyl-1*H*-parazol-5-yl)-7,8-dihydro-6*H*-thiazolo[5',4': 4,5]benzo [1,2-b][1,4]oxazin-2-yl)-[4,4'-bipyridine]-3-carboxamide**

8-(1-methyl-1*H*-pyrazol-5-yl)-7,8-dihydro-6*H*-thiazol[5',4':4,5]benzo[1,2-*b*][1,4]oxazin-2-amine (50.0 mg, 0.17 mmol), 2'-chloro-5'-methoxy-[3,4'-bipyridine]-4-carboxylic acid (48.5 mg, 0.17 mmol), N,N,N',N'-tetramethylchlorformamidine hexafluorphosphate (48.8 mg, 0.17 mmol) and N-methylimidazole (42.8 mg, 0.52 mmol) were dissolved in acetonitrile (20 mL); and the reaction mixture was stirred at 70°C to react for 4 hours. After the reaction was completed, the reaction mixture was concentrated under reduced pressure to obtain residues, which were purified by pre-HPLC to obtain 2'-chloro-5'-methoxy-6-methyl-*N*-(8-(1-methyl-1*H*-pyrazol-5-yl)-7,8-dihydro-6*H*-thiazolo[5',4':4,5]benzo[1, 2-b][1,4]oxazin-2-yl)-[4,4'-bipyridine]-3-carboxamide (white solid, 22.6 mg, yield: 23.7%). $^1H$ NMR (400 MHz, DMSO-$d_6$) $\delta$ 12.69 (s, 1H), 8.80 (s, 1H), 8.13 (s, 1H), 7.54 (s, 1H), 7.51 (d, *J* = 1.9 Hz, 1H), 7.43 (s, 1H), 7.20 (s, 1H), 6.69 (s, 1H), 6.20 (d, *J* = 1.9 Hz, 1H), 4.41 - 4.33 (m, 2H), 3.68 (s, 3H), 3.64 - 3.59 (m, 2H), 3.57 (s, 3H), 2.59 (s, 3H). LC/MS (ESI) m/z: 548.1 $[M+H]^+$.

**Example 195**: **2'-chloro-5'-methoxy-6-methyl-N-(6-(4-(methylsulfonamido)piperidin-1-yl)thiazolo[4,5 -c]pyridin-2-yl)-4,4'-bipyridine]-3-carboxamide**

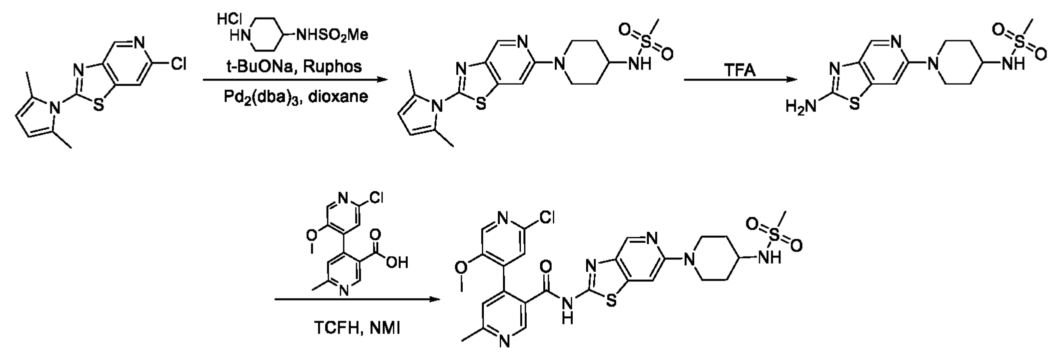


**Step 1: N-(1-(2-(2,5-dimethyl-1H-pyrrol-1-yl)thiazolo[4,5-c]pyridin-6-yl)piperidin-4-yl)methanesulfo namide**

$Pd_2(dba)_3$ (138.9 mg, 0.152 mmol), Ruphos (142 mg, 0.303 mmol) and sodium tert-butoxide (364 mg, 3.79 mmol) were added to the solution of 6-chloro-2-(2,5-dimethylpyrrol-1-yl)[1,3]thiazolo[4,5-c]pyridine (200 mg, 0.758 mmol) and N-(hexahydropyridin-4-yl)methanesulfonamide (270.4 mg, 1.517 mmol) in 1,4-dioxane (60 mL); and the reaction mixture was stirred at 100°C for 3 hours. After complete reaction, the reaction mixture was concentrated to obtain a crude product. The crude product was purified by column chromatography (eluent: dichloromethane/methanol, gradient: 0%-50%

methanol) to obtain the title compound (brown solid, 350 mg, yield: 91.1%).

**Step 2: N-(1-(2-aminothiazolo[4,5-c]pyridin-6-yl)piperidin-4-yl)methanesulfonamide**

Trifluoroacetic acid (5 mL) and $H_2O$ (5 mL) were added to the solution of N-(1-(2-(2,5-dimethyl-1H-pyrrol-1-yl) thiazolo[4,5-c]pyridin-6-yl)piperidin-4-yl)methanesulfonamide (100 mg, 0.247 mmol); and the reaction mixture was stirred at 80°C for 2 hours. After complete reaction, the reaction mixture was concentrated to obtain a crude product; the crude product was dissolved in methanol, added with triethylamine, and concentrated to obtain a crude product. The crude product was purified by silica-gel chromatography (eluent: dichloromethane/methanol, gradient: 0%- 50% methanol) to obtain the title compound (brown solid, 70 mg, yield: 86.7%).

**Step 3: 2'-chloro-5'-methoxy-6-methyl-N-(6-(4-(methylsulfonamido)piperidin-1-yl)thiazolo[4,5-c]pyri din-2-yl)-[4,4'-bipyridine]-3-carboxamide**

1-methylimidazole (52.7 mg, 0.641 mmol) was added to the solution of N-(1-(2-aminothiazolo[4,5-c]pyridin-6-yl) piperidin-4-yl)methanesulfonamide (70 mg, 0.214 mmol) and 4-(2-chloro-5-methoxypyridin-4-yl)-6-methylpyridin-3-carboxylic acid (65.5 mg, 0.235 mmol) in acetonitrile (5 mL) and DMF (3 mL); the reaction mixture was stirred at 70°C for 5 minutes; and then, TCFH (60 mg, 0.214 mmol) was added, and the reaction mixture was stirred at 70°C overnight. After complete reaction, water was added; the resulting mixture was extracted with ethyl acetate; and the organic phase was concentrated under reduced pressure, to obtain the residues, which were purified by reversed-phase preparation (acetonitrile/water system) to obtain the title compound (yellow solid, 10 mg, yield: 8.0%). $^1H$ NMR (400 MHz, DMSO-$d_6$) δ 12.84 (s, 1H), 8.83 (s, 1H), 8.60 (s, 1H), 8.17 (s, 1H), 7.57 (s, 1H), 7.46 (s, 1H), 7.41 (s, 1H), 7.10 (d, $J$ = 7.3 Hz, 1H), 4.20 (d, $J$ = 13.3 Hz, 2H), 3.61 (s, 3H), 3.46 - 3.38 (m, 1H), 3.01 - 2.93 (m, 5H), 2.60 (s, 3H), 1.94 - 1.86 (m, 2H), 1.51 - 1.39 (m, 2H). LC/MS (ESI) m/z: 588.2 $[M+H]^+$.

**Example 196: 2'-chloro-N-(6-(1,4-dimethyl-1H-1,2,3-triazol-5-yl)thiazolo[4,5-c]pyridin-2-yl)-5'-metho xy-6-(4-methyl-8-oxo-4,7-diazaspiro[2.5]oct-7-yl)-[4,4'-bipyridine]-3-carboxamide**

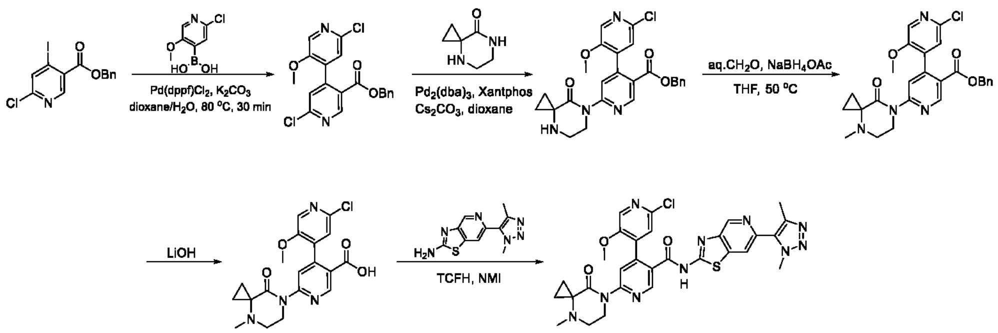


**Step 1: 2',6-dichloro-5'-methoxy-[4,4'-bipyridine]-3-benzyl carboxylate**

(2-chloro-5-methoxypyridin-4-yl)boric acid (552 mg, 2.94 mmol), potassium carbonate (1.22 g, 8.82 mmol) and 1,1'-bisdiphenylphosphinoferrocenpalladium dichloride (215 mg, 0.294 mmol) were sequentially added to the mixed solution of 6-chloro-4-iodobenzyl nicotinate (1.10 g, 2.94 mmol) in 1,4-dioxane (20 mL) and water (4 mL). Under the protection of nitrogen, the reaction mixture was stirred at 80°C to react for 30 minutes. After complete reaction, water was added; the resulting mixture was extracted with ethyl acetate; and the organic phase was washed with saturated saline, dried over anhydrous sodium sulfate, and concentrated under reduced pressure. The residue was purified by column chromatography (eluent: petroleum ether/ethyl acetate, gradient: 0-30% ethyl acetate) to obtain the title compound (white solid, 800 mg, yield: 69.6%). LC/MS (ESI) m/z: 389.1 $[M+H]^+$.

**Step 2: 2'-chloro-5'-methoxy-6-(8-oxo-4,7-diazaspiro[2.5]oct-7-yl)-[4,4'-bipyridine]-3-benzyl carboxy late**

4,7-diazaspiro[2.5]oct-8-one (81 mg, 0.642 mmol), cesium carbonate (628 mg, 1.927 mmol), 4,5-bisdiphenylphosphino-9,9-dimethylxanthene (37 mg, 0.064 mmol) and tris(dibenzylideneacetone)dipalladium (59 mg, 0.064 mmol) were sequentially added to the solution of 2', 6-dichloro-5'-methoxy-[4,4'-bipyridine]-3-benzyl carboxylate (250 mg, 0.642

mmol) in 1,4-dioxane (10 mL). Under the protection of nitrogen, the reaction mixture was stirred at 100°C to react for 3 hours. After complete reaction, the reaction mixture was concentrated under reduced pressure. The residue was purified by column chromatography (eluent: petroleum ether/ethyl acetate, gradient: 0-100% ethyl acetate) to obtain the title compound (yellow solid, 150 mg, yield: 48.8%). LC/MS (ESI) m/z: 479.2 $[M+H]^+$.

**Step 3**: **2'-chloro-5'-methoxy-6-(4-methyl-8-oxo-4,7-diazaspiro[2.5]oct-7-yl)-[4,4'-bipyridine]-3-benzyl carboxylate**

Formaldehyde in water (0.23 mL, 6.24 mmol) and sodium triacetoxyborohyride (198 mg, 0.939 mmol) were sequentially added to the solution of 2'-chloro-5'-methoxy-6-(8-oxo-4,7-diazaspiro[2.5]oct-7-yl)-[4,4'-bipyridine]-3-benzyl carboxylate (150 mg, 0.313 mmol) in tetrahydrofuran (5 mL). The reaction mixture was stirred at 50°C to react for 2 hours. With raw materials remained, formaldehyde in water (0.23 mL, 6.24 mmol) and sodium triacetoxyborohyride (198 mg, 0.939 mmol) were then supplemented; and the reaction mixture was continued with stirring at 50°C to react for 2 hours. After complete reaction, the saturated aqueous solution of sodium bicarbonate was added; the resulting mixture was extracted with ethyl acetate; and the organic phase was washed with saturated saline, dried over anhydrous sodium sulfate, and concentrated under reduced pressure. The residue was purified by column chromatography (eluent: petroleum ether/ethyl acetate, gradient: 0-100% ethyl acetate) to obtain the title compound (faint yellow oil, 100 mg, yield: 64.8%). LC/MS (ESI) m/z: 493.1 $[M+H]^+$.

**Step 4: 2'-chloro-5'-methoxy-6-(4-methyl-8-oxo-4,7-diazaspiro[2.5]oct-7-yl)-[4,4'-bipyridine]-3-carbo xylic acid**

Lithium hydroxide (17 mg, 0.405 mmol) was added to 2'-chloro-5'-methoxy-6-(4-methyl-8-oxo-4,7-diazaspiro [2.5]oct-7-yl)-[4,4'-bipyridine]-3-benzyl carboxylate (100 mg, 0.203 mmol) in the mixed solution of tetrahydrofuran (3 mL), methanol (1 mL) and water (1 mL). The reaction mixture was stirred at 40°C to react for 2 hours. After complete reaction, the reaction mixture was concentrated under reduced pressure to obtain the title compound (white solid, 80 mg, yield: 97.9%), which was used directly in the next step without purification. LC/MS (ESI) m/z: 403.2 $[M+H]^+$.

**Step 5: 2'-chloro-N-(6-(1,4-dimethyl-1H-1,2,3-triazol-5-yl)thiazolo[4,5-c]pyridin-2-yl)-5'-methoxy-6-(4-methyl-8-oxo-4,7-diazaspiro[2.5]oct-7-yl)-[4,4'-bipyridine]-3-carboxamide**

6-(1,4-dimethyl-1H-1,2,3-triazol-5-yl)thiazolo[4,5-c]pyridin-2-amine (59 mg, 0.240 mmol) and N-methylimidazole (49 mg, 0.597 mmol) were sequentially added to 2'-chloro-5'-methoxy-6-(4-methyl-8-oxo-4,7-diazaspiro[2.5]oct-7-yl)-[4,4'-bipyridine]-3-carboxylic acid (80 mg, 0.199 mmol) in the mixed solution of N,N-dimethylformamide (1.5 mL) and acetonitrile (2.5 mL). Then, at 70°C, *N,N,N',N'*-tetramethylchlorformamidine hexafluorphosphate (84 mg, 0.299 mmol) was added to the mixture. The mixture was stirred at 70°C for 2 hours. After complete reaction, water was added; the resulting mixture was extracted with ethyl acetate; the organic phase was washed with saturated saline, dried over anhydrous sodium sulfate, and concentrated under reduced pressure. The residue was purified by pre-HPLC to obtain the title compound (white solid, 11.4 mg, yield: 9.10%). $^1H$ NMR (400 MHz, DMSO-$d_6$) δ 13.36 (s, 1H), 9.21 (s, 1H), 8.91 (s, 1H), 8.41 (s, 1H), 8.19 (s, 1H), 8.09 (s, 1H), 7.52 (s, 1H), 4.27 (t, *J* = 5.6 Hz, 2H), 4.14 (s, 3H), 3.62 (s, 3H), 3.46 (t, *J* = 5.6 Hz, 2H), 2.58 (s, 3H), 2.40 (s, 3H), 1.36 - 1.29 (m, 2H), 1.17 - 1.05 (m, 2H). LC/MS (ESI) m/z: 631.4 $[M+H]^+$.

The example compounds in Table 13 were synthesized using commercially available raw materials with reference to the synthesis steps of Example 196.

Table 13:

| Examples | Chemical structure | LC/MS(ESI) m/z $[M+H]^+$ | $^1H$ NMR |
|---|---|---|---|
| 197 | 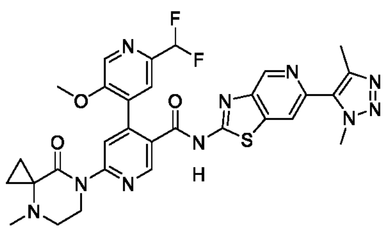 | 647.3 | $^1H$ NMR (400 MHz, DMSO-$d_6$) δ 13.37 (s, 1H), 9.21 (s, 1H), 8.91 (s, 1H), 8.48 (s, 1H), 8.40 (s, 1H), 8.11 (s, 1H), 7.69 (s, 1H), 7.02 (t, *J* = 55.2 Hz, 1H), 4.27 (t, *J* = 5.6 Hz, 2H), 4.14 (s, 3H), 3.69 (s, 3H), 3.35 (t, *J* = 5.6 Hz, 2H), 2.54 (s, 3H), 2.39 (s, 3H), 1.34 - 1.26 (m, 2H), 1.13 - 0.94 (m, 2H). |

**Example 198: 2'-(difluoromethyl)-N-(5-(4-hydroxypiperidin-1-yl)thiazolo[5,4-d]pyrimidin-2-yl)-5-me thoxy-6-methyl-[4,4'-bipyridine]-3-carboxamide**

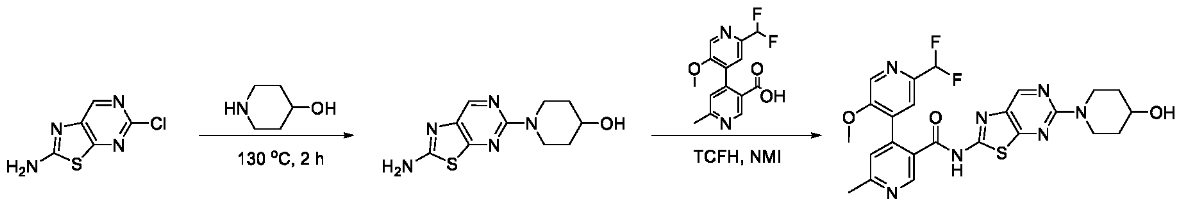


**Step 1: 1-(2-aminothiazolo[5,4-d]pyrimidin-5-yl)piperidin-4-ol**

The mixture of 5-chlorothiazol[5,4-d]pyrimidin-2-amine (2 g, 10.7 mmol) and piperidin-4-ol (3.25 g, 32.2 mmol) was stirred at 130° for 2 hours. After complete reaction, the residue was purified by column chromatography (eluent: ethyl acetate/methanol, gradient: 0-10% methanol) to obtain the title compound (faint yellow solid, 1.15 g, yield: 42.7%). LC/MS (ESI) m/z: 252.2 $[M+H]^+$.

**Step 2: 2'-(difluoromethyl)-N-(5-(4-hydroxypiperidin-1-yl)thiazolo[5,4-d]pyrimidin-2-yl)-5-methoxy-6-methyl-[4,4'-bipyridine]-3-carboxamide**

2'-(difluoromethyl)-5'-methoxy-6-methyl-[4,4'-bipyridine]-3-carboxylic acid (117 mg, 0.398 mmol) and N-methylimidazole (98 mg, 1.19 mmol) were sequentially added to 1-(2-aminothiazolo[5,4-d]pyrimidin-5-yl)piperidin-4-ol (100 mg, 0.398 mmol) in the mixed solution of N,N-dimethylformamide (3 mL) and acetonitrile (5 mL). Then, at 70°C, N,N,N',N'-tetramethylchlorformamidine hexafluorphosphate (167 mg, 0.595 mmol) was added to the mixture. The mixture was stirred at 70°C for 2 hours. After complete reaction, water was added; the resulting mixture was extracted with ethyl acetate; the organic phase was washed with saturated saline, dried over anhydrous sodium sulfate, and concentrated under reduced pressure. The residue was purified by pre-HPLC to obtain the title compound (yellow solid, 46.3 mg, yield: 22.1%). $^1$H NMR (400 MHz, DMSO) δ 12.89 (s, 1H), 8.82 (s, 1H), 8.75 (s, 1H), 8.46 (s, 1H), 7.72 (s, 1H), 7.46 (s, 1H), 6.99 (t, *J* = 54.8 Hz, 1H), 4.73 (d, *J* = 4.4 Hz, 1H), 4.32 - 4.25 (m, 2H), 3.79 - 3.72 (m, 1H), 3.70 (s, 3H), 3.30-3.26 (m, 2H), 2.61 (s, 3H), 1.82 - 1.76 (m, 2H), 1.39 - 1.31 (m, 2H). LC/MS (ESI) m/z: 528.2 $[M+H]^+$.

The example compounds in Table 14 were synthesized using commercially available raw materials with reference to the synthesis steps of Example **198.**

Table 14:

| Examples | Chemical structure | LC/MS(ESI) m/z $[M+H]^+$ | $^1$H NMR |
|---|---|---|---|
| 199 | 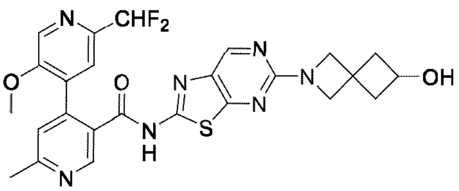 | 540.3 | $^1$H NMR (400 MHz, DMSO-$d_6$) δ 12.93 (s, 1H), 8.82 (s, 1H), 8.74 (s, 1H), 8.46 (s, 1H), 7.72 (s, 1H), 7.46 (s, 1H), 6.99 (t, *J* = 55.1 Hz, 1H), 5.05 (d, *J* = 6.2 Hz, 1H), 4.05 (s, 2H), 4.04 - 4.01 (m, 1H), 4.00 (s, 2H), 3.69 (s, 3H), 2.61 (s, 3H), 2.49 - 2.43 (m, 2H), 2.05 - 1.99 (m, 2H). |
| **200** | 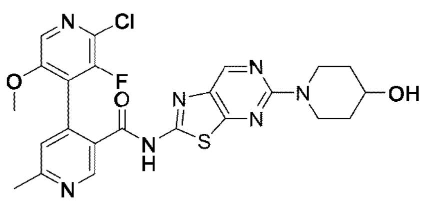 | 530.2 | $^1$H NMR (400 MHz, DMSO) δ 13.01 (s, 1H), 8.97 (s, 1H), 8.76 (s, 1H), 8.18 (s, 1H), 7.51 (s, 1H), 4.73 (d, *J* = 4.0 Hz, 1H), 4.33 - 4.24 (m, 2H), 3.80 - 3.74 (m, 1H), 3.73 (s, 3H), 3.36 - 3.34 (m, 1H), 3.30 - 3.26 (m, 1H), 2.61 (s, 3H), 1.82 - 1.74 (m, 2H), 1.41 - 1.31 (m, 2H). |

**Example 201**: **2'-(difluoromethyl)-5'-methoxy-6-methyl-N-(5-(2-oxopiperidin-1-yl)thiazolo[5,4-d]pyri midin-2-yl)-[4,4'-bipyridine]-3-carboxamide**

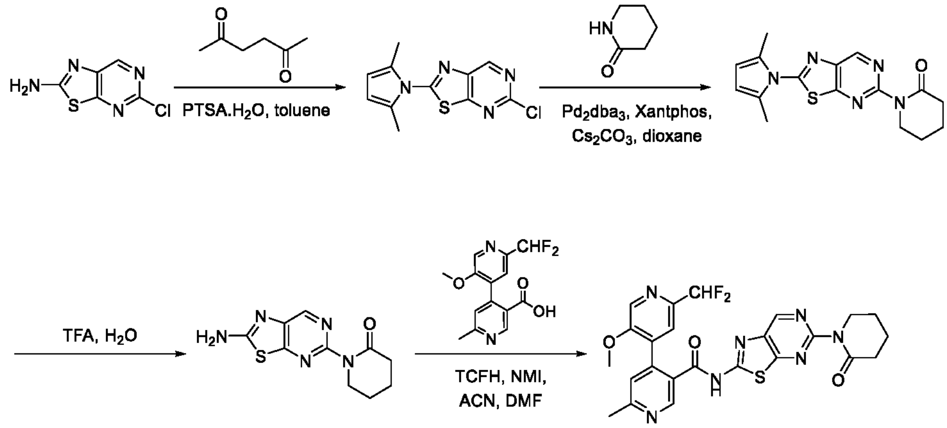


**Step 1: 5-chloro-2-(2,5-dimethyl-1H-pyrrol-1-yl)thiazolo[5,4-d]pyrimidine**

P-toluenesulfonic acid hydrate (1.02 g, 5.36 mmol) was added to the solution of 5-chloro[1,3]thiazolo[5,4-d]pyrimidin-2-amine (5 g, 26.8 mmol) and acetonyl acetone (6.12g, 53.6 nmol) in toluene (150 mL); and then, the mixture was stirred at 120°C for water dispensing overnight. The mixture was filtered; and the filter cake was rinsed with ethyl acetate. The filtrate was washed with sodium bicarbonate in water, washed with saturated saline, and concentrated under reduced pressure; the residue was purified by silica-gel column chromatography (eluent: petroleum ether/ethyl acetate =10, V/V) to obtain the title compound (yellow solid, 2.7 g, yield: 38.1%). LC/MS (ESI) m/z: 265.2 $[M+H]^+$.

**Step 2**: **1-(2-(2,5-dimethyl-1H-pyrrol-1-yl)thiazolo[5,4-d]pyrimidin-5-yl)piperidin-2-one**

Tris(dibenzylideneacetone)dipalladium (173 mg, 0.189 mmol) was added to the solution of 5-chloro-2-(2,5-dimethyl-1H-pyrrol-1-yl)thiazolo[5,4-d]pyrimidine (500 mg, 1.89 mmol), 2-piperidone (374 mg, 3.78 mmol), cesium carbonate (1.85 g, 5.67 mmol), and 4,5-bisdiphenylphosphino-9,9-dimethylxanthene (109 mg, 0.189 mmol) in 1,4-dioxane (30 mL); and under the protection of nitrogen, the mixture was stirred at 100°C for 2 hours. The mixture was concentrated under reduced pressure; and the residue was purified by silica-gel column chromatography (eluent: dichloromethane/methanol =30, V/V) to obtain the title compound (yellow solid, 500 mg, yield: 80.9%). LC/MS (ESI) m/z: 328.4 $[M+H]^+$.

**Step 3: 1-(2-aminothiazolo[5,4-d]pyrimidin-5-yl)piperidin-2-one**

1-(2-(2,5-dimethyl-1H-pyrrol-1-yl)thiazolo[5,4-d]pyrimidin-5-yl)piperidin-2-one (450 mg, 1.37 mmol) was dissolved in trifluoroacetic acid (18 mL) and water (0.18 mL); and the reaction mixture was stirred at 70°C for 2 hours. The mixture was concentrated under reduced pressure to obtain residues, which were dissolved in acetonitrile and then neutralized with ammonia. The mixture was concentrated under reduced pressure; and the residue was purified by silica-gel column chromatography (eluent: dichloromethane/0.1% ammonia methanol =10, V/V) to obtain the title compound (yellow solid, 150 mg, yield: 43.8%). LC/MS (ESI) m/z: 250.0 $[M+H]^+$.

**Step 4: 2'-(difluoromethyl)-5'-methoxy-6-methyl-N-(5-(2-oxopiperidin-1-yl)thiazolo[5,4-d]pyrimidin-2-yl)-[4,4'-bipyridine]-3-carboxamide**

1-(2-aminothiazolo[5,4-d]pyrimidin-5-yl)piperidin-2-one (120 mg, 0.481 mmol), 2'-(difluoromethyl)-5'-methoxy-6-methyl-[4,4'-bipyridine]-3-carboxylic acid (156 mg, 0.530 mmol), and methylimidazole (237 mg, 2.89 mmol) were dissolved in acetonitrile (9 mL) and N,N-dimethylformamide (2 mL), and stirred at 70°C for 5 minutes; and N,N,N',N'-tetramethylchlorformamidine hexafluorphosphate (270 mg, 0.963 mmol) in acetonitrile solution (1 mL) was added dropwise, and stirred at 70°C for 2 hours. The acetonitrile solution (1 mL) of methylimidazole (118 mg, 1.44 mmol) and N,N,N',N'-tetramethylchlorformamidine hexafluorphosphate (135 mg, 0.481 mmol) was supplemented, and stirred at 70°C for 2 hours. The reaction mixture was concentrated under reduced pressure to obtain residues, which were dissolved

in ethyl acetate; the organic phase was washed with water and with saline, and concentrated under reduced pressure to obtain a crude product; and the crude product was purified by prep-HPLC (eluent: methanol/0.1% formic acid aqueous solution = 0-50, V/V) to obtain the title compound (50 mg, white solid, yield: 19.8%). $^1$H NMR (400 MHz, DMSO-$d_6$) $\delta$ 13.39 (s, 1H), 9.18 (s, 1H), 8.88 (s, 1H), 8.46 (s, 1H), 7.76 (s, 1H), 7.49 (s, 1H), 7.00 (t, $J$ = 55.1 Hz, 1H), 3.85 - 3.78 (m, 2H), 3.69 (s, 3H), 2.63 (s, 3H), 2.47 - 2.44 (m, 2H), 1.92 - 1.86 (m, 4H). LC/MS (ESI) m/z: 526.2 [M+H]$^+$.

The example compounds in Table 15 were synthesized using commercially available raw materials with reference to the synthesis steps of Example **201**.

Table 15:

| Examples | Chemical structure | LC/MS(ESI) m/z [M+H]$^+$ | $^1$H NMR |
|---|---|---|---|
| 202 | 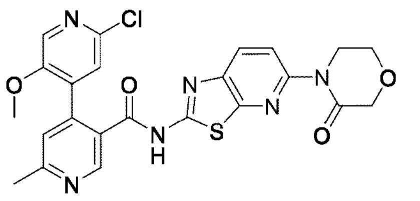 | 511.1 | $^1$H NMR (400 MHz, DMSO) δ 13.05 (s, 1H), 8.90 (s, 1H), 8.16 (s, 1H), 8.09 (s, 1H), 7.99 (s, 1H), 7.54 (s, 1H), 7.41 (s, 1H), 4.28 (s, 2H), 4.00 (s, 4H), 3.61 (s, 3H), 2.59 (s, 3H). |
| 203 | 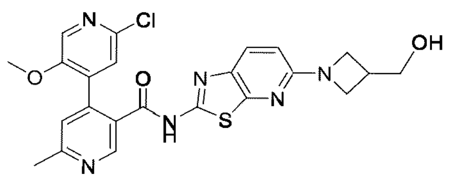 | 497.2 | $^1$H NMR (400 MHz, DMSO-$d_6$) δ 13.36 (s, 1H), 9.59 (s, 1H), 8.87 (s, 1H), 8.22 (d, $J$ = 9.4 Hz, 1H), 8.19 (s, 1H), 7.60 (s, 1H), 7.49 (s, 1H), 7.01 (d, $J$ = 9.4 Hz, 1H), 5.00 (s, 1H), 4.43 - 4.36 (m, 1H), 4.21 - 4.14 (m,1H), 3.62 (s, 3H), 3.55 - 3.53 (m, 2H), 3.37 - 3.20 (m, 2H), 2.61 (s, 3H), 2.45 - 2.37 (m, 1H). |
| 204 | 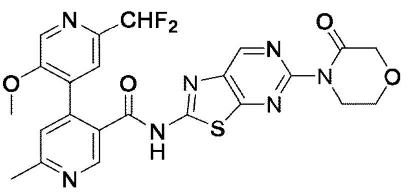 | 528.1 | $^1$H NMR (400 MHz, DMSO) δ 13.40 (s, 1H), 9.19 (s, 1H), 8.89 (s, 1H), 8.46 (s, 1H), 7.75 (s, 1H), 7.48 (s, 1H), 7.00 (t, $J$ = 55.1 Hz, 1H), 4.26 (s, 2H), 4.04 - 3.91 (m, 4H), 3.69 (s, 3H), 2.62 (s, 3H). |

**Example 205**: **2'-chloro-5'-methoxy-6-methyl-N-(6-(oxazol-2-yl)thiazolo[4,5-c]pyridin-2-yl)-[4,4'-bipy ridine]-3-carboxamide**

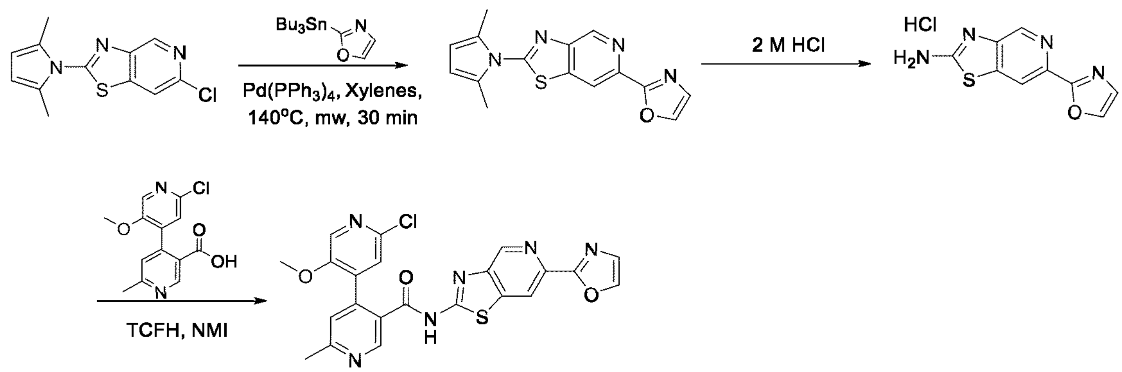


**Step 1: 2-(2-(2,5-dimethyl-1H-pyrrol-1-yl)thiazolo[4,5-c]pyridin-6-yl)oxazol**

Under the protection of nitrogen, 6-chloro-2-(2,5-dimethyl-1H-pyrrol-1-yl)thiazolo[4,5-c]pyridine (200 mg, 0.79 mmol), 2-(tributylstannyl)oxazole (299 mg, 0.83 mmol), and tetrakis(triphenylphosphine)palladium (96 mg, 0.08 mmol) were dissolved in ditoluene (5 mL). Under the protection of nitrogen, the reaction was allowed to occur at 140°C for 30 minutes in the presence of microwaves. After complete reaction, saturated potassium fluoride solution was added; and the

resulting mixture was stirred for 1 hour, extracted with ethyl acetate, washed with saturated saline, dried over anhydrous sodium sulfate, and concentrated under reduced pressure. The resulting residue was purified by column chromatography (eluent: petroleum ether, ethyl acetate, gradient: 20%-80% ethyl acetate) to obtain the title compound (yellow solid, 160 mg, yield: 71.2%). LC/MS (ESI) m/z: 296.9 $[M+H]^+$.

**Step 2: 6-(oxazol-2-yl)thiazolo[4,5-c]pyridin-2-amine hydrochloride**

2-(2-(2,5-dimethyl-1H-pyrrol-1-yl)thiazolo[4,5-c]pyridin-6-yl)oxazole (120 mg, 0.40 mmol) was dissolved in the solvent of 2 M hydrochloric acid (5 mL). The reaction was allowed to occur at 70°C for 1 hour. After complete reaction, the reaction mixture was concentrated under reduced pressure, added with dichloromethane (10 mL) for washing; and the solids were spin-dried to obtain the title compound (brown solid, 66 mg, yield: 58.4%). LC/MS (ESI) m/z: 219.1 $[M+H]^+$.

**Step 3: 2'-chloro-N-(5-(4,4-difluoropiperidin-1-yl)-6-fluorothiazolo[5,4-b]pyridin-2-yl)-3'-fluoro-5'-m ethoxy-6-methyl-[4,4'-bipyridine]-3-carboxamide**

6-(oxazol-2-yl)thiazolo[4,5-c]pyridin-2-amine hydrochloride (46 mg, 0.18 mmol) and 2'-chloro-5'-methoxy-6-methyl-[4,4'-bipyridine]-3-carboxylic acid (50 mg, 0.18 mmol) were dissolved in 5 mL of acetonitrile and 1 mL of N,N-dimethylformamide; N-methylimidazole (59.1 mg, 0.72 mmol) was added; the resulting mixture was stirred at 70°C for 10 minutes; and then, N,N,N',N'-tetramethylchlorformamidine hexafluorphosphate (76 mg, 0.27 mmol) was added to allow for reaction at 70°C for 1 hour. Ethyl acetate and water were added to the reaction mixture for stratification; the organic phase was washed with saturated sodium chloride solution, dried over anhydrous sodium sulfate, and filtered; the filtrate was concentrated under reduced pressure; and the resulting residue was purified by pre-HPLC (eluent: acetonitrile/0.1% formic acid aqueous solution, gradient: 25%-90%) to obtain the title compound (white solid, 25 mg, yield: 29%). $^1H$ NMR (400 MHz, DMSO) δ 13.34 (s, 1H), 9.12 (s, 1H), 8.89 (s, 1H), 8.82 (s, 1H), 8.30 (s, 1H), 8.17 (s, 1H), 7.60 (s, 1H), 7.48 (s, 1H), 7.46 (s, 1H), 3.61 (s, 3H), 2.62 (s, 3H). LC/MS (ESI) m/z: 479.1 $[M+H]^+$.

The example compounds in Table 16 were synthesized using commercially available raw materials with reference to the synthesis steps of Example **205.**

Table 16:

| Exam ples | Chemical structure | LC/MS(ESI) m/z $[M+H]^+$ | $^1H$ NMR |
|---|---|---|---|
| 206 | 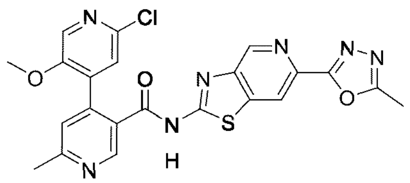 | 494.1 | $^1H$NMR (400 MHz, DMSO-$d_6$) δ 13.40 (s, 1H), 9.14 (s, 1H), 8.90 (s, 1H), 8.88 (s, 1H), 8.17 (s, 1H), 7.59 (s, 1H), 7.47 (s, 1H), 3.60 (s, 3H), 2.62 (s, 3H), 2.61 (s, 3H). |
| 207 | 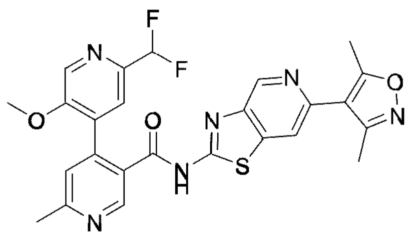 | 523.2 | $^1H$ NMR (400 MHz, DMSO) δ 13.25 (s, 1H), 9.13 (s, 1H), 8.88 (s, 1H), 8.46 (s, 1H), 8.25 (s, 1H), 7.76 (s, 1H), 7.50 (s, 1H), 7.00 (t, *J* = 55.0 Hz, 1H), 3.68 (s, 3H), 2.63 (s, 3H), 2.58 (s, 3H), 2.40 (s, 3H). |
| 208 | 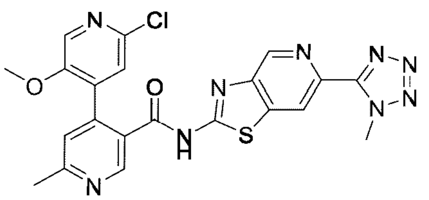 | 494.2 | $^1H$ NMR (400 MHz, DMSO) δ 13.40 (s, 1H), 9.23 (s, 1H), 8.99 (s, 1H), 8.89 (s, 1H), 8.17 (s, 1H), 7.61 (s, 1H), 7.50 (s, 1H), 4.45 (s, 3H), 3.60 (s, 3H), 2.62 (s, 3H). |

(continued)

| Examples | Chemical structure | LC/MS(ESI) m/z [M+H]+ | 1H NMR |
|---|---|---|---|
| 209 | 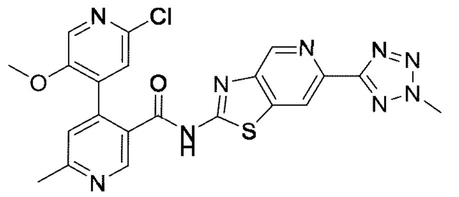 | 494.2 | 1H NMR (400 MHz, DMSO) δ 13.35 (s, 1H), 9.16 (s, 1H), 8.90 (s, 1H), 8.87 (s, 1H), 8.18 (s, 1H), 7.61 (s, 1H), 7.50 (s, 1H),4.46 (s, 3H), 3.61 (s, 3H), 2.62 (s, 3H). |
| 210 | 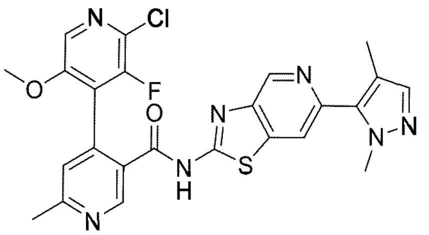 | 524.1 | 1H NMR (400MHz, DMSO-$d_6$) $\delta$ 13.42 (s, 1H), 9.18 (s, 1H), 9.04 (s, 1H), 8.26 (s, 1H), 8.19 (s, 1H), 7.56 (s, 1H), 7.36 (s, 1H), 3.89 (s, 3H), 3.72 (s, 3H), 2.63 (s, 3H), 2.10 (s, 3H). |
| 211 | 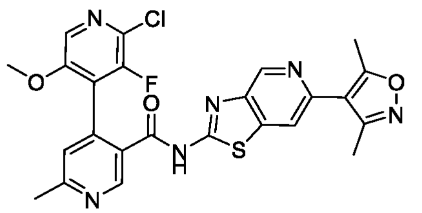 | 525.2 | 1H NMR (400MHz, DMSO-$d_6$) $\delta$ 13.35 (s, 1H), 9.13 (s, 1H), 9.03 (s, 1H), 8.23 (s, 1H), 8.18 (s, 1H), 7.54 (s, 1H), 3.72 (s, 3H), 2.62 (s, 3H), 2.58 (s, 3H), 2.40 (s, 3H). |
| 212 | 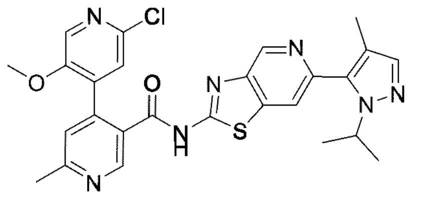 | 534.3 | 1H NMR (400MHz, DMSO-$d_6$) $\delta$ 13.26 (s, 1H), 9.18 (s, 1H), 8.88 (s, 1H), 8.22 (s, 1H), 8.17 (s, 1H), 7.61 (s, 1H), 7.49 (s, 1H), 7.39 (s, 1H), 4.80-4.88 (m, 1H), 3.62 (s, 3H), 2.62 (s, 3H), 2.05 (s, 3H), 1.35 (d, *J* = 8.0 Hz, 6H). |

**Example 213**: ***N*-(6-(1-cyclopropyl-4-methyl-1*H*-1,2,3-triazol-5-yl)thiazolo[4,5-*c*]pyridin-2-yl)-2'-(difluoromethyl)-5'-methoxy-6-methyl-[4,4'-bipyridine]-3-carboxamide**

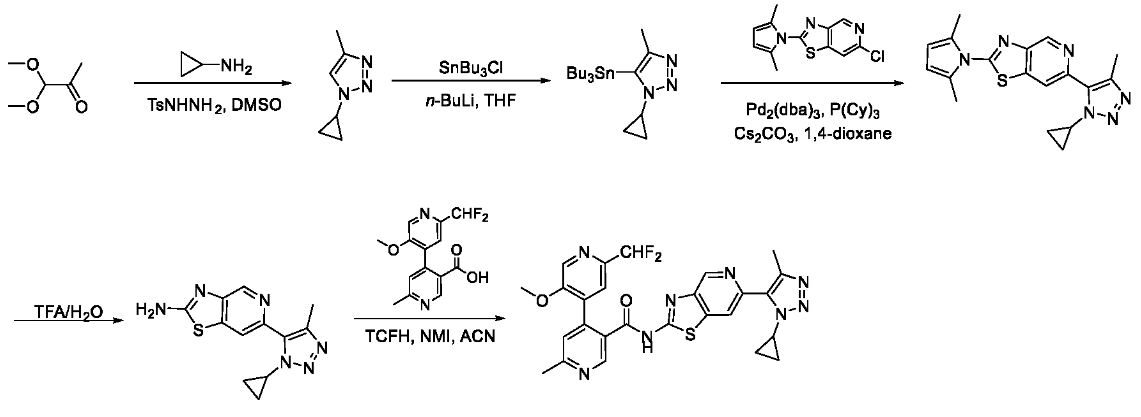


**Step 1: 1-cyclopropyl-4-methyl-1,2,3-triazole**

1,1-dimethoxyacetone (3.00 g, 25.40 mmol) and p-toluenesulfonhydrazide (4.73 g, 25.40 mmol) were dissolved in dimethyl sulfoxide (30 mL); the reaction mixture was stirred at room temperature for 1 hour; then, cyclopropylamine (1.52

g, 26.7 mmol) was added; and the reaction mixture was stirred at 80°C for 1 hour. After complete reaction, the reaction mixture was added with water, extracted with ethyl acetate, washed with saturated saline, dried over anhydrous sodium sulfate, and concentrated under reduced pressure to obtain a crude title compound (yellow oil, 1.00 g, yield: 31.9%), which was directly used in the next step of reaction. LC/MS (ESI) m/z: 124.2 [M+H]+.

**Step 2: 1-cyclopropyl-4-methyl-5-(tributylstannyl)-1H-1,2,3-triazole**

At -78°C, n-butyllithium (6.1 mL, 9.74 mmol, 1.6 mol/L) was added dropwise slowly to the solution of 1-cyclopropyl-4-methyl-1,2,3-triazole (1.0 g, 8.12 mmol) in tetrahydrofuran (20 mL). Under the protection of nitrogen, the reaction mixture was stirred at -78°C for 1 hour, and then, tributyl tin chloride (2.4 mL, 8.93 mmol) was added. Under the protection of nitrogen, the reaction mixture was stirred at -78°C for 0.5 hours. After complete reaction, the reaction mixture was restored to room temperature, added with saturated ammonium chloride solution, extracted with ethyl acetate, washed with saturated saline, dried over anhydrous sodium sulfate, and concentrated under reduced pressure. The residue was purified by silica-gel chromatography (eluent: petroleum ether/ethyl acetate, gradient: 0-30% ethyl acetate) to obtain the title compound (yellow oil, 300 mg, yield: 9.0%). LC/MS (ESI) m/z: 414.2 [M+H]+.

**Step 3: 6-(1-cyclopropyl-4-methyl-1*H*-1,2,3-triazol-5-yl)-2-(2,5-dimethyl-1*H*-pyrrol-1-yl)thiazolo[4,5-*c*] pyridine**

Tris(dibenzylideneacetone)dipalladium (83.3 mg, 0.91 mmol), tricyclohexylphosphine (51.0 mg, 0.18 mmol) and cesium carbonate (494 mg, 1.52 mmol) were added to the solution of 1-cyclopropyl-4-methyl-5-(tributylstannyl)-1H-1,2,3-triazole (250 mg, 0.61 mmol) and 6-chloro-2-(2,5-dimethyl-1H-pyrrol-1-yl)thiazolo[4,5-c]pyridine (160 mg, 0.61 mmol) in 1,4-dioxane (10 mL). Under the protection of nitrogen, the reaction mixture was stirred at 118°C to react for 16 hours. After complete reaction, the reaction mixture was cooled to room temperature; water was added; and the resulting mixture was extracted with ethyl acetate, washed with saturated saline, dried over anhydrous sodium sulfate, and concentrated under reduced pressure. The residue was purified by silica-gel chromatography (eluent: petroleum ether/ethyl acetate, gradient: 20-50% ethyl acetate) to obtain the title compound (yellow solid, 50 mg, yield: 23.5%). LC/MS (ESI) m/z: 351.1 [M+H]+.

**Step 4: 6-(1-cyclopropyl-4-methyl-1*H*-1,2,3-triazol-5-yl)thiazolo[4,5-c]pyridin-2-amine**

Trifluoroacetic acid (1.0 mL) and water (1 drop) were added to 6-(1-cyclopropyl-4-methyl-1H-1,2,3-triazol-5-yl)-2-(2,5-dimethyl-1H-pyrrol-1-yl)thiazolo[4,5-c]pyridine (50.0 mg, 0.14 mmol), and stirred at 70°C to react for 4 hours. After complete reaction, the reaction mixture was concentrated under reduced pressure, to obtain the crude title compound (yellow solid, 30.0 mg, yield: 77.2%), which was used directly in the next step. LC/MS (ESI) m/z: 273.1 [M+H]+.

**Step 5: *N*-(6-(1-cyclopropyl-4-methyl-1*H*-1,2,3-triazol-5-yl)thiazolo[4.5-*c*]pyridin-2-y1)-2'-(difluorome thyl)-5'-methoxy-6-methyl-[4.4'-bipyridine]-3-carboxamide**

6-(1-cyclopropyl-4-methyl-1H-1,2,3-triazol-5-yl)thiazolo[4,5-c]pyridin-2-amine (30.0 mg, 0.11 mmol), 2'-difluoromethyl-5'-methoxy-[3,4'-bipyridine]-4-carboxylic acid (32.4 mg, 0.11 mmol), N,N,N',N'-tetramethylchlorformamidine hexafluorphosphate (46.4 mg, 0.17 mmol) and N-methylimidazole (27.1 mg, 0.33 mmol) were dissolved in acetonitrile (10 mL); and the reaction mixture was stirred at 70°C to react for 12 hours. After the reaction was completed, the reaction mixture was concentrated under reduced pressure to obtain residues, which were purified by pre-HPLC to obtain the title compound (white solid, 10.0 mg, yield: 16.6%). $^1H$ NMR (400 MHz, DMSO) δ 13.33 (s, 1H), 9.20 (s, 1H), 8.89 (s, 1H), 8.46 (s, 1H), 8.43 (s, 1H), 7.76 (s, 1H), 7.49 (s, 1H), 7.00 (t, *J* = 55.1 Hz, 1H), 4.16 - 4.04 (m, 1H), 3.69 (s, 3H), 2.63 (s, 3H), 2.36 (s, 3H), 1.12 - 1.00 (m, 4H). LC/MS (ESI) m/z: 549.2 [M+H]+.

The example compounds in Table 17 were synthesized using commercially available raw materials with reference to the synthesis steps of Example **213.**

Table 17:

| Examples | Chemical structure | LC/MS(ESI) m/z [M+H]+ | $^1H$ NMR |
|---|---|---|---|
| 214 | 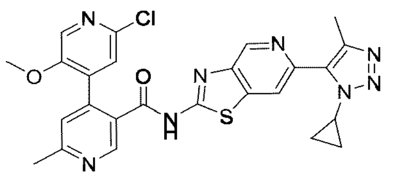 | 533.2 | $^1H$ NMR (400 MHz, DMSO) δ 13.32 (s, 1H), 9.21 (s, 1H), 8.90 (s, 1H), 8.45 (s, 1H), 8.18 (s, 1H), 7.61 (s, 1H), 7.52 (s, 1H), 4.14 - 4.08 (m, 1H), 3.61 (s, 3H), 2.62 (s, 3H), 2.36 (s, 3H), 1.12 - 0.99 (m, 4H). |

**Example 215: 2'-(difluoromethyl)-N-(6-(1,4-dimethyl-1H-1,2,3-triazol-5-yl)-7-fluorothiazolo[4,5-c]pyr idin-2-yl)-5'-methoxy-6-methyl-[4,4'-bipyridine]-3-carboxamide**

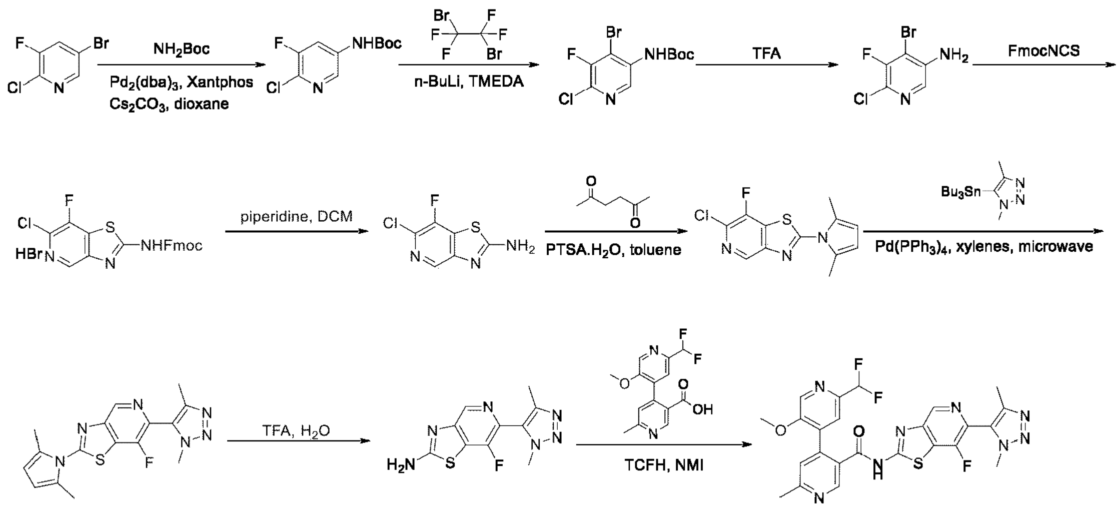


**Step 1: tert-butyl (6-chloro-5-fluoropyridin-3-yl) carbamate**

Tris(dibenzylideneacetone)dipalladium (130 mg, 0.143 mmol) was added to the solution of 2-chloro-3-fluoro-5-bromopyridine (1.00 g, 4.75 mmol), tert-Butyl carbamate (610 mg, 5.23 mmol), cesium carbonate (3.10 g, 9.50 mmol), and 4,5-bisdiphenylphosphino-9,9-dimethylxanthene (110 mg, 0.190 mmol) in 1,4-dioxane (20 mL); and under the protection of nitrogen, the mixture was stirred at 85°C for 20 hours. The mixture was concentrated under reduced pressure; the residue was purified by silica-gel column chromatography (eluent: petroleum ether/ethyl acetate =4, V/V) to obtain the title compound (yellow solid, 920 mg, yield: 78.5%). LC/MS (ESI) m/z: 246.9 $[M+H]^+$.

**Step 2: (4-bromo-6-chloro-5-fluoropyridine)-3-aminotert-butyl carbamate**

**tert-butyl**(6-chloro-5-fluoropyridin-3-yl)carbamate (920 mg, 3.73 mmol) and N,N,N',N'-tetramethylethyledediamine (1.3 g, 11.2 mmol) were dissolved in diethyl ether (20 mL). Under the protection of nitrogen; the reaction mixture was cooled to -60°C; and 1.6 M n-butyllithium (6.99 mL, 11.2 mmol) was added dropwise, after which the temperature of the mixture was increased to -20°C, and the mixture was then stirred for 1.5 hours. The reaction mixture was cooled to -60°C; and 1,2-dibromotetrafluoroethane (3004 mg, 11.6 mmol) was added dropwise, after which the temperature of the mixture was slowly increased to room temperature over 1 hour. 1 N HCl (9.2 ml) was used to quench the reaction; the mixture was extracted with ethyl acetate; the organic phase was washed with water and with saline, dried over anhydrous sodium sulfate, and filtered. The mixture was concentrated under reduced pressure to obtain the title compound (yellow solid, 1.1 g, yield: 90.6%). LC/MS (ESI) m/z: 270.1 $(M-56)^+$.

**Step 3: 4-bromo-6-chloro-5-fluoropyridin-3-amine**

(4-bromo-6-chloro-5-fluoropyridine)-3-aminotert-butyl carbamate (1.1 g, 3.38 mmol) was dissolved in trifluoroacetic acid (15 mL) and water (7.5 mL); and the reaction mixture was stirred at room temperature for 1 hour. The mixture was concentrated under reduced pressure to obtain residues, which were then dissolved in ethyl acetate and neutralized with saturated sodium bicarbonate in water; the mixture was extracted with ethyl acetate; the organic phase was washed with water and with saline, dried over anhydrous sodium sulfate, and filtered. The mixture was concentrated under reduced pressure to obtain the title compound (yellow oil, 800 mg, yield: 105%). LC/MS (ESI) m/z: 226.8 $[M+H]^+$.

**Step 4: (9H-fluoren-9-yl)methyl (6-chloro-7-fluorothiazolol4.5-c]pyridin-2-yl)carbamate hydrobromide**

4-bromo-6-chloro-5-fluoropyridin-3-amine (800 mg, 3.55 mmol) was dissolved in acetone (10 mL); O-((9H-fluoren-9-yl)methyl)isothiocyanate carbonate (1200 mg, 4.26 mmol) was added. The reaction mixture was stirred at 50°C overnight, then cooled to room temperature, and filtered; the filter cake was rinsed with acetone; and the solids were dried under reduced pressure to obtain the title compound (1 g, white solid, yield: 55.6%). LC/MS (ESI) m/z: 425.9 $[M+H]^+$.

**Step 5: 6-chloro-7-fluorothiazolo[4,5-c]pyridin-2-amine**

Piperidine (1.51 g, 17.8 mmol) was added to the solution of (9H-fluoren-9-yl)methyl (6-chloro-7-fluorothiazolo [4,5-c]pyridin-2-yl)carbamate hydrobromide (900 mg, 1.78 mmol) in dichloromethane (30 mL); and then, the mixture was stirred at room temperature for 1 hour. The mixture was concentrated under reduced pressure; and the residue was purified by silica-gel column chromatography (eluent: dichloromethane/methanol = 30, V/V) to obtain the title compound (white solid, 360 mg, yield: 99.6%). $^1$H NMR (400 MHz, DMSO-$d_6$) δ 8.27 (s, 1H), 8.24 (s, 2H). LC/MS (ESI) m/z: 203.8 $[M+H]^+$.

**Step 6: 6-chloro-2-(2,5-dimethyl-1H-pyrrol-1-yl)-7-fluorothiazolo[4,5-c]pyridine**

P-toluenesulfonic acid hydrate (67.3 mg, 0.354 mmol) was added to the solution of 6-chloro-7-fluorothiazolo[4,5-c]pyridin-2-amine (360 mg, 1.77 mmol) and acetonyl acetone (404 mg, 3.54 mmol) in toluene (20 mL); and then, the mixture was refluxed at 130°C and subjected to water dispensing for 2 hours. The reaction mixture was cooled to room temperature, added with ethyl acetate, washed with sodium bicarbonate solution, then washed with saturated saline, and concentrated under reduced pressure; and the residue was purified by silica-gel column chromatography (eluent: petroleum ether/ethyl acetate = 10, V/V) to obtain the title compound (white solid, 300 mg, yield: 60.2%). $^1$H NMR (400 MHz, $CDCl_3$) δ 8.89 (s, 1H), 5.99 (s, 2H), 2.41 (s, 6H). LC/MS (ESI) m/z: 282.0 $[M+H]^+$.

**Step 7: 6-(1,4-dimethyl-1H-1,2,3-triazol-5-yl)-2-(2,5-dimethyl-1H-pyrrol-1-yl)-7-fluorothiazolo[4,5-c] pyridine**

Tetrakis(triphenylphosphine)palladium (308 mg, 0.266 mmol) was added to the ditoluene solution (10 mL) of 6-(1,4-dimethyl-1H-1,2,3-triazol-5-yl)-2-(2,5-dimethyl-1H-pyrrol-1-yl)-7-fluorothiazolo[4,5-c]pyridine (250 mg, 0.887 mmol) and 1,4-dimethyl-5-(tributylstannyl)-1H-1,2,3-triazole (514 mg, 1.33 mmol); and under the protection of nitrogen, the mixture was treated with microwaves at 140°C to react for half an hour. The mixture was concentrated under reduced pressure; and the residue was purified by silica-gel column chromatography (eluent: petroleum ether/ethyl acetate = 3, V/V) to obtain the title compound (white solid, 290 mg, yield: 95.4%). $^1$H NMR (400 MHz, DMSO-$d_6$) δ 9.35 (d, $J$ = 1.9 Hz, 1H), 6.05 (s, 2H), 4.06 (s, 3H), 2.40 (s, 6H), 2.29 (d, $J$ = 2.0 Hz, 3H). LC/MS (ESI) m/z: 343.0 $[M+H]^+$.

**Step 8: 6-(1,4-dimethyl-1H-1,2,3-triazol-5-yl)-7-fluorothiazolo[4,5-c]pyridin-2-amine**

6-(1,4-dimethyl-1H-1,2,3-triazol-5-yl)-2-(2,5-dimethyl-1H-pyrrol-1-yl)-7-fluorothiazolo[4,5-c]pyridine (270 mg, 0.789 mmol) was dissolved in trifluoroacetic acid (15 mL) and water (0.5 mL); and the reaction mixture was stirred at 70°C for 3 hours. The mixture was concentrated under reduced pressure to obtain residues, which were dissolved in acetonitrile and then neutralized with ammonia. The mixture was concentrated under reduced pressure; and the residue was purified by silica-gel column chromatography (eluent: dichloromethane/0.1% ammonia methanol =10, V/V) to obtain the title compound (yellow solid, 130 mg, yield: 62.4%). $^1$H NMR (400 MHz, DMSO-$d_6$) δ 8.66 (d, $J$ = 2.0 Hz, 1H), 7.26 (s, 5H), 4.01 (s, 3H), 2.25 (d, $J$ = 1.8 Hz, 3H). LC/MS (ESI) m/z: 265.3 $[M+H]^+$.

**Step 9: 2'-(difluoromethyl)-N-(6-(1,4-dimethyl-1H-1,2,3-triazol-5-yl)-7-fluorothiazolo[4.5-c]pvridin-2-yl)-5'-methoxy-6-methyl-[4,4'-bipyridine]-3-carboxamide**

6-(1,4-dimethyl-1H-1,2,3-triazol-5-yl)-7-fluorothiazolo[4,5-c]pyridin-2-amine (50 mg, 0.189 mmol), 2'-(difluoromethyl)-5'-methoxy-6-methyl-[4,4'-bipyridine]-3-carboxylic acid (61.2 mg, 0.208 mmol), and methylimidazole (93.2 mg, 1.14 mmol) were dissolved in acetonitrile (4 mL) and N,N-dimethylformamide (1 mL), and stirred at 70°C for 5 minutes; N,N,N',N'-tetramethylchlorformamidine hexafluorphosphate (106 mg, 0.378 mmol) in acetonitrile solution (1 mL) was added dropwise, and stirred at 70°C for 2 hours. The reaction mixture was concentrated under reduced pressure to obtain residues, which were dissolved in ethyl acetate; the organic phase was washed with water and with saline, and concentrated under reduced pressure to obtain a crude product; and the crude product was purified by prep-HPLC (eluent: acetonitrile/0.1% formic acid aqueous solution = 0-50, *V*/*V*) to obtain the title compound (11 mg, white solid, yield: 10.8%). $^1$H NMR (400 MHz, DMSO-$d_6$) $\delta$ 13.65 (s, 1H), 9.14 (d, $J$ = 1.7 Hz, 1H), 8.91 (s, 1H), 8.47 (s, 1H), 7.77 (s, 1H), 7.51 (s, 1H), 7.01 (t, $J$ = 55.0 Hz, 1H), 4.03 (s, 3H), 3.69 (s, 3H), 2.63 (s, 3H), 2.26 (s, 3H). LC/MS (ESI) m/z: 541.2 $[M+H]^+$.

The example compounds in Table 18 were synthesized using commercially available raw materials with reference to the synthesis steps of Example **215.**

Table 18:

| Examples | Chemical structure | LC/MS(ESI) m/z [M+H]+ | 1H NMR |
|---|---|---|---|
| 216 | 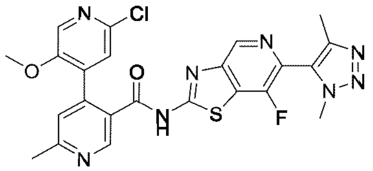 | 525.1 | 1H NMR (400 MHz, DMSO-$d_6$) δ 13.64 (s, 1H), 9.14 (d, $J$ = 1.7 Hz, 1H), 8.91 (s, 1H), 8.18 (s, 1H), 7.62 (s, 1H), 7.51 (s, 1H), 4.03 (s, 3H), 3.62 (s, 3H), 2.63 (s, 3H), 2.27 (d, $J$ = 1.4 Hz, 3H). |
| 217 | 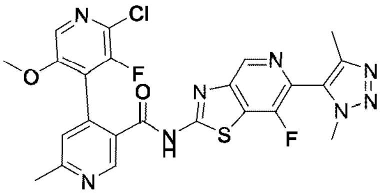 | 543.4 | 1H NMR (400 MHz, DMSO-$d_6$) δ 13.76 (s, 1H), 9.15 (s, 1H), 9.06 (s, 1H), 8.20 (s, 1H), 7.57 (s, 1H), 4.02 (s, 3H), 3.73 (s, 3H), 2.64 (s, 3H), 2.07 (s, 3H). |

**Example 218: 2'-(difluoromethyl)-5'-methoxy-6-methyl-N-(6-(4-methyl-4H-1,2,4-triazol-3-yl)thiazolo [4.5-c]pyridin-2-y])-[4.4'-bipyridine]-3-carboxamide**

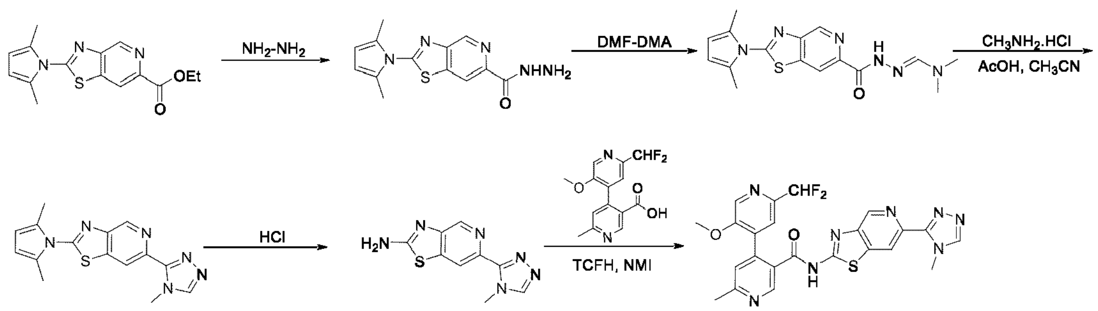


**Step 1: 2-(2,5-dimethyl-1H-pyrrol-1-yl)thiazolo[4,5-c]pyridin-6-formylhydrazine**

2-(2,5-dimethyl-1H-pyrrol-1-yl)thiazolo[4,5-c]pyridin-6-ethyl formate (1.5 g, 4.98 mmol) was dissolved in ethanol (20 mL) solvent. 80% hydrazine hydrate (2 mL) was added. The reaction was allowed to occur at 85°C for 2 hours. After complete reaction, the reaction mixture was filtered; the filter cake was washed with ethanol (10 mL), and dried to obtain the title compound (white solid, 1.2 g, yield: 83.9%). LC/MS (ESI) m/z: 288.1 [M+H]+.

**Step 2: N'-(2-(2,5-dimethyl-1H-pyrrol-1-yl)thiazolo[4,5-c]pyridin-6-carbonyl)-N, N-dimethyl formyl hydrazine**

2-(2,5-dimethyl-1H-pyrrol-1-yl)thiazolo[4,5-c]pyridin-6-formylhydrazine (1.2 g, 4.18 mmol) was dissolved in the mixed solvent of N,N-dimethylformamide dimethylacetal (5 mL) and dichloromethane (10 mL). The reaction was allowed to occur at 40°C for 2 hours. After complete reaction, the reaction mixture was concentrated under reduced pressure; ethyl acetate (20 mL) was added; and the resulting mixture was stirred for half an hour. The mixture was filtered under reduced pressure; and the filter cake was concentrated and evaporated to dryness to obtain the title compound (white solid, 1.1 g, yield: 76.9%). LC/MS (ESI) m/z: 343.1 [M+H]+.

**Step 3: 2-(2,5-dimethyl-1H-pyrrol-1-yl)-6-(4-methyl-4H-1,2,4-triazol-3-yl)thiazolo[4,5-c]pyridine**

N'-(2-(2,5-dimethyl-1H-pyrrol-1-yl)thiazolo[4,5-c]pyridin-6-carbonyl)-N, N-dimethylformylhydrazine (1.1 g, 3.21 mmol) was dissolved in the mixed solvent of 10 mL of acetonitrile and 1 mL of acetic acid; and methylamine hydrochloride (433 mg, 6.42 mmol) was added to allow reaction at 60°C for 2 hours. After complete reaction, ethyl acetate and water were added to the reaction mixture for stratification; the organic phase was washed with saturated sodium chloride solution; the organic phase was dried over anhydrous sodium sulfate, and filtered; and the filtrate was concentrated under reduced pressure to obtain residues, which were purified by column chromatography (eluent: dichloromethane, methanol,

gradient: 0%-10% methanol) to obtain the title compound (yellow solid, 650 mg, yield: 65.1%). LC/MS (ESI) m/z: 311.1 $[M+H]^+$.

**Step 4: 2-(2,5-dimethyl-1H-pyrrol-1-yl)-6-(4-methyl-4H-1,2,4-triazol-3-yl)thiazolo[4,5-c]pyridine hyd rochloride**

2-(2,5-dimethyl-1H-pyrrol-1-yl)-6-(4-methyl-4H-1,2,4-triazol-3-yl)thiazolo[4,5-c]pyridine (1.1 g, 3.21 mmol) was dissolved in 10 mL of 2 M hydrochloric acid, to allow for reaction at 70°C for 30 minutes. After complete reaction, the reaction mixture was concentrated under reduced pressure; 5 mL of dichloromethane was added; the mixture was stirred for half an hour, and then filtered; and the filter cake was evaporated to dryness under reduced pressure to obtain the title compound (yellow solid, 200 mg, yield: 77.1%). LC/MS (ESI) m/z: 233.1 $[M+H]^+$

**Step 5: 2'-(difluoromethyl)-5'-methoxy-6-methyl-N-(6-(4-methyl-4H-1,2,4-triazol-3-yl)thiazolo[4,5-c]p yridin-2-yl)-[4,4'-bipyridine]-3-carboxamide**

2-(2,5-dimethyl-1H-pyrrol-1-yl)-6-(4-methyl-4H-1,2,4-triazol-3-yl)thiazolo[4,5-c]pyridine hydrochloride (75 mg, 0.28 mmol) and 2'-(difluoromethyl)-5'-methoxy-6-methyl-[4,4'-bipyridine]-3-carboxylic acid (3982 mg, 0.28 mmol) were dissolved in 5 mL of acetonitrile and 1 mL of N,N-dimethylformamide; N-methylimidazole (92 mg, 1.12 mmol) was added; the resulting mixture was stirred at 70°C for 10 minutes; and then, N,N,N',N'-tetramethylchlorformamidine hexafluorphosphate (118 mg, 0.42 mmol) was added to allow for reaction at 70°C for 1 hour. Ethyl acetate and water were added to the reaction mixture for stratification; the organic phase was washed with saturated sodium chloride solution; the organic phase was dried over anhydrous sodium sulfate, and filtered; and the filtrate was concentrated under reduced pressure; and the resulting residue was treated by pre-HPLC to obtain the title compound (white solid, 35 mg, yield: 24.7%). $^1H$ NMR (400 MHz, DMSO) δ 13.33 (s, 1H), 9.15 (d, *J* = 0.7 Hz, 1H), 8.89 (s, 1H), 8.82 (d, *J* = 0.7 Hz, 1H), 8.62 (s, 1H), 8.47 (s, 1H), 7.75 (s, 1H), 7.49 (s, 1H), 7.00 (t, *J* = 55.1 Hz, 1H), 4.03 (s, 3H), 3.69 (s, 3H), 2.63 (s, 3H). LC/MS (ESI) m/z: 509.2 $[M+H]^+$.

**Example 219: 2'-chloro-N-(6-(1-(2-(dimethylamino)ethyl)-4-methyl-1H-parazol-5-yl)thiazolo[4,5-c]py ridin-2-yl)-5'-methoxy-6-methyl-[4.4'-bipyridine]-3-carboxamide trifluoroacetate**

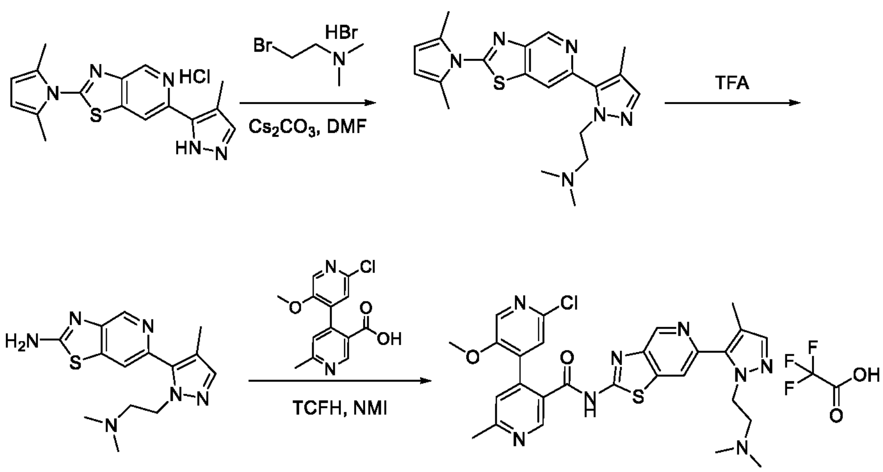


**Step 1: 2-(5-(2-(2,5-dimethyl-1H-pyrrol-1-yl)thiazolo[4.5-c]pyridin-6-yl)-4-methyl-1H-pyrazol-1-yl)-N, N-dimethylethan-1-amine**

Cesium carbonate $Cs_2CO_3$ (1.55 g, 4.77 mmol) was added to the solution of 2-(2,5-dimethyl-1H-pyrrol-1-yl)-6-(4-methyl-1H-pyrazol-5-yl)thiazolo[4,5-c]pyridine hydrochloride (330 mg, 0.954 mmol) and N,N-dimethylamine bromoethane hydrobromide (289 mg, 1.24 mmol) in N,N-dimethylformamide (10 mL); and then, the mixture was stirred at 100°C for 3 hours. The mixture was concentrated under reduced pressure, added with ethyl acetate, washed with water and with saturated saline, and concentrated under reduced pressure; the residue was purified by silica-gel column chromatography (eluent: dichloromethane/methanol = 10, V/V) to obtain a crude product; and the crude product was purified by prep-HPLC (eluent: acetonitrile/0.1% trifluoroacetic acid in water = 0-50, V/V) to obtain the title compound (20 mg, yellow solid, yield: 5.25%). LC/MS (ESI) m/z: 381.1 $[M+H]^+$.

**Step 2: 6-(1-(2-(dimethylamino)ethyl)-4-methyl-1H-pyrazol-5-yl)thiazolo[4,5-c]pyridin-2-amine**

2-(5-(2-(2,5-dimethyl-1H-pyrrol-1-yl)thiazolo[4,5-c]pyridin-6-yl)-4-methyl-1H-pyrazol-1-yl)-N,N-dimethyl ethan-1-amine (20 mg, 0.053 mmol) was dissolved in trifluoroacetic acid (2 mL); and water (0.04 mL) was added. The resulting mixture was stirred at 70°C for 2 hours. The reaction mixture was concentrated to dryness under reduced pressure to obtain residues, which were dissolved in acetonitrile, neutralized with ammonia, and concentrated under reduced pressure; the residue was purified by reversed-phase chromatographic columns (C18, eluent: acetonitrile/0.1% formic acid aqueous solution = 0-30, V/V) to obtain the title compound (yellow solid, 10 mg, yield: 62.9%). LC/MS (ESI) m/z: 303.0 $[M+H]^+$.

**Step 3: 2'-chloro-N-(6-(1-(2-(dimethylamino)ethyl)-4-methyl-1H-pyrazol-5-yl)thiazolo[4,5-c]pyridin-2 -yl)-5'-methoxy-6-methyl-[4,4'-bipyridine]-3-carboxamide trifluoroacetate**

6-(1-(2-(dimethylamino)ethyl)-4-methyl-1H-pyrazol-5-yl)thiazolo[4,5-c]pyridin-2-amine (10 mg, 0.033 mmol), 2'-chloro-5'-methoxy-6-methyl-[4,4'-bipyridine]-3-carboxylic acid (10.1 mg, 0.036 mmol), and methylimidazole (16.3 mg, 0.198 mmol) were dissolved in acetonitrile (3 mL), and stirred at 70°C for 5 minutes; N,N,N',N'-tetramethylchlorformamidine hexafluorphosphate (27.8 mg, 0.099 mmol) in acetonitrile solution (1 mL) was added dropwise, and stirred at 70°C for four hours. The reaction mixture was concentrated under reduced pressure to obtain residues, which were dissolved in ethyl acetate; the organic phase was washed with water and with saline, and concentrated under reduced pressure to obtain a crude product; and the crude product was purified by prep-HPLC (eluent: acetonitrile/0.1% trifluoroacetic acid in water = 0-50, V/V) to obtain the title compound (2 mg, grey solid, yield: 10.7%). $^1$H NMR (400 MHz, DMSO-$d_6$) $\delta$ 13.30 (s, 1H), 9.55 (s, 1H), 9.20 (s, 1H), 8.88 (s, 1H), 8.34 (s, 1H), 8.18 (s, 1H), 7.61 (s, 1H), 7.54 (s, 1H), 7.50 (s, 1H), 4.64 (t, *J* = 6.2 Hz, 2H), 3.64 - 3.55 (m, 5H), 2.84 (s, 6H), 2.62 (s, 3H), 2.16 (s, 3H). LC/MS (ESI) m/z: 563.2 $[M+H]^+$.

The example compounds in Table 19 were synthesized using commercially available raw materials with reference to the synthesis steps of Example **219.**

Table 19:

| Examples | Chemical structure | LC/MS(ESI) m/z $[M+H]^+$ | $^1$H NMR |
|---|---|---|---|
| 220 | 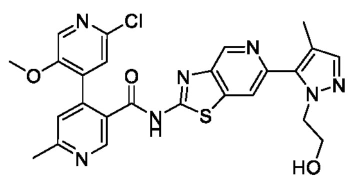 | 536.2 | $^1$H NMR (400 MHz, DMSO-$d_6$) $\delta$ 13.27 (s, 1H), 9.16 (s, 1H), 8.88 (s, 1H), 8.26 (s, 1H), 8.17 (s, 1H), 7.60 (s, 1H), 7.49 (s, 1H), 7.39 (s, 1H), 4.84 (t, *J* = 5.5 Hz, 1H), 4.31 (t, *J* = 6.3 Hz, 2H), 3.68 - 3.63 (m, 2H), 3.62 (s, 3H), 2.62 (s, 3H), 2.09 (s, 3H). |
| 221 | 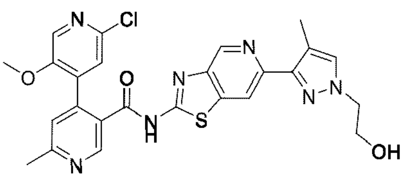 | 536.2 | $^1$H NMR (400 MHz, DMSO-$d_6$) $\delta$ 13.14 (s, 1H), 9.03 (s, 1H), 8.88 (s, 1H), 8.47 (s, 1H), 8.18 (s, 1H), 7.59 (s, 2H), 7.47 (s, 1H), 4.92 (t, *J* = 5.3 Hz, 1H), 4.14 (t, *J* = 5.6 Hz, 2H), 3.77 (q, *J* = 5.5 Hz, 2H), 3.62 (s, 3H), 2.61 (s, 3H), 2.36 (s, 3H). |

**Example 222: 2'-chloro-*N*-(6-(1,4-dimethyl-1*H*-1,2,3-triazol-5-yl)benzo[*d*]lthiazol-2-yl)-5'-methoxy-6-methyl-[4.4'-bipyridine]-3-carboxamide**

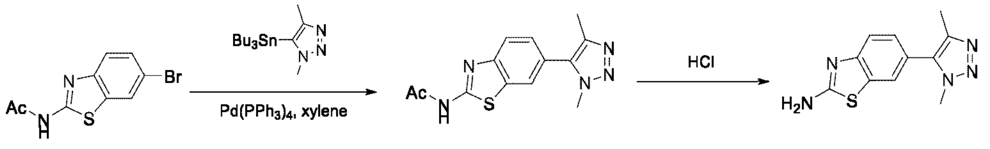


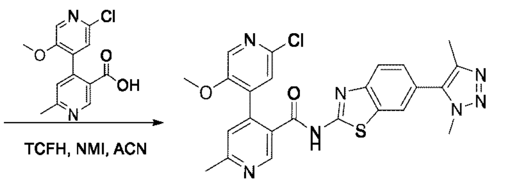


**Step 1: *N*-(6-(1,4-dimethyl-1*H*-1,2,3-triazol-5-yl)benzo[*d*]thiazol-2-yl)acetamide**

Tetrakis(triphenylphosphine)palladium (639 mg, 0.55 mmol) was added to the solution of N-(6-bromobenzo[d]thiazol-2-yl)acetamide (500 mg, 1.84 mmol) and 1,4-dimethyl-5-(tributylstannyl)-1H-1,2,3-triazole (854 mg, 2.21 mmol) in ditoluene (10 mL). The reaction mixture was stirred at 160°C for 2 hours in a microwave reactor. After complete reaction, the reaction mixture was cooled to room temperature; water was added; and the resulting mixture was extracted with ethyl acetate, washed with saturated saline, dried over anhydrous sodium sulfate, and concentrated under reduced pressure. The residue was purified by silica-gel chromatography (eluent: petroleum ether/ethyl acetate, gradient: 30-70% ethyl acetate), to obtain the title compound (yellow solid, 180 mg, yield: 34.0%). LC/MS (ESI) m/z: 288.1 $[M+H]^+$.

**Step 2: 6-(1,4-dimethyl-1*H*-1,2,3-triazol-5-yl)benzo[*d*]thiazol-2-amine**

Hydrochloric acid solution (6.0 M, 10 mL) was added to *N*-(6-(1,4-dimethyl-1H-1,2,3-triazol-5-yl)benzo[d]thiazol-2-yl)acetamide (180 mg, 0.63 mmol), and stirred at 100°C for 2 hours. After complete reaction, the reaction mixture was concentrated under reduced pressure; the residue was pulped and purified to obtain the title compound (yellow solid, 120 mg, yield: 78.1%). LC/MS (ESI) m/z: 246.1 $[M+H]^+$.

**Step 3: 2'-chloro-*N*-(6-(1,4-dimethyl-1*H*-1,2,3-triazol-5-yl)benzo[*d*]thiazol-2-yl)-5'-methoxy-6-methyl-[4,4'-bipyridine]-3-carboxamide**

6-(1,4-dimethyl-1H-1,2,3-triazol-5-yl)benzo[d]thiazol-2-amine (50.0 mg, 0.20 mmol), 2'-chloro-5'-methoxy-6-methyl-[4,4'-bipyridine]-3-carboxylic acid (56.8 mg, 0.20 mmol), N,N,N',N'-tetramethylchlorformamidine hexafluorphosphate (85.8 mg, 0.31 mmol) and N-methylimidazole (50.2 mg, 0.61 mmol) were dissolved in acetonitrile (10 mL); and the reaction mixture was stirred at 70°C for 4 hours. After the reaction was completed, the reaction mixture was concentrated under reduced pressure to obtain residues, which were purified by pre-HPLC to obtain the title compound (white solid, 12.0 mg, yield: 11.6%). $^1$H NMR (400 MHz, DMSO) δ 13.08 (s, 1H), 8.86 (s, 1H), 8.17 (s, 2H), 7.91 (d, *J* = 8.3 Hz, 1H), 7.59 (s, 1H), 7.55 (dd, *J* = 8.4, 1.8 Hz, 1H), 7.48 (s, 1H), 3.95 (s, 3H), 3.61 (s, 3H), 2.61 (s, 3H), 2.25 (s, 3H). LC/MS (ESI) m/z: 506.1 $[M+H]^+$.

The example compounds in Table 20 were synthesized using commercially available raw materials with reference to the synthesis steps of Example **222.**

Table 20:

| Examples | Chemical structure | LC/MS(ESI) m/z $[M+H]^+$ | $^1$H NMR |
|---|---|---|---|
| 223 | 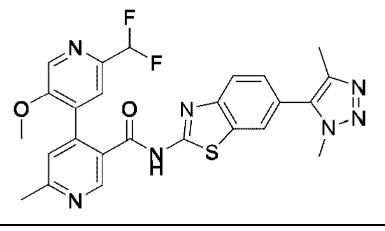 | 522.1 | $^1$H NMR (400 MHz, DMSO) δ 13.11 (s, 1H), 8.87 (s, 1H), 8.46 (s, 1H), 8.15 (s, 1H), 7.91 (d, *J* = 8.4 Hz, 1H), 7.75 (s, 1H), 7.55 (dd, *J* = 8.4, 1.7 Hz, 1H), 7.49 (s, 1H), 7.00 (t, *J* = 55.1 Hz, 1H), 3.95 (s, 3H), 3.69 (s, 3H), 2.62 (s, 3H), 2.25 (s, 3H). |

(continued)

| Examples | Chemical structure | LC/MS(ESI) m/z [M+H]+ | $^1$H NMR |
|---|---|---|---|
| 224 | 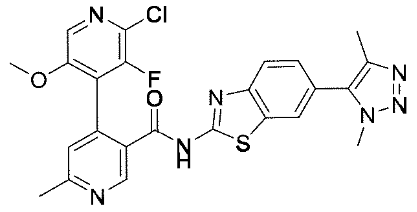 | 524.1 | $^1$H NMR (400 MHz, DMSO) δ 13.22 (s, 1H), 9.02 (s, 1H), 8.18 (s, 1H), 8.15 (s, 1H), 7.92 (d, *J* = 8.2 Hz, 1H), 7.56 (dd, *J* = 8.4, 1.8 Hz, 1H), 7.53 (s, 1H), 3.95 (s, 3H), 3.72 (s, 3H), 2.62 (s, 3H), 2.25 (s, 3H). |

**Example 225: 2'-chloro-5'-methoxy-6-methyl-N-(6-methyl-5-oxo-6,7,8,9-tetrahydro-5H-thiazolo[4',5': 4,5]benzo [1, 2-c]aza-2-yl)-[4,4'-bipyridine]-3-carboxamide**

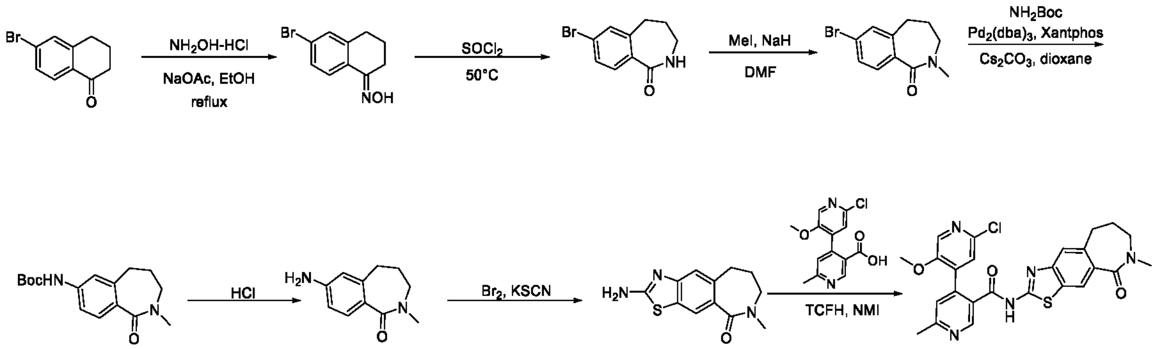


**Step 1: 6-bromo-3,4-dihydronaphthalen-1(2H)-ketoxime**

At room temperature, hydroxylamine hydrochloride (1.16 g, 16.7 mmol) was dissolved in anhydrous ethanol (25 mL); sodium acetate (1.37 g, 16.7 mmol) and 6-bromo-3,4-dihydronaphthalen-1(2H)-one (2.5 g, 11.1 mmol) were added; and the mixture was heated and refluxed for 3 hours. After complete reaction, the reaction mixture was cooled to room temperature; water was added; the resulting mixture was filtered; the filter cake was washed with a small amount of water; and the solids were dried to obtain the title compound (faint yellow solid, 2.6 g, yield: 97.5%). LC/MS (ESI) m/z: 240.0 [M+H]+.

**Step 2: 7-bromo-2,3,4,5-tetrahydro-1H-benzo[c]aza-1-one**

At room temperature, 6-bromo-3,4-dihydronaphthalen-1(2H)-ketoxime (2.6 g, 10.8 mmol) was dissolved in sulfoxide chloride (25 mL); and the mixture was heated to 50°C and stirred for 2 hours. After the reaction was completed, the reaction mixture was directly spin-dried, diluted with ethyl acetate, then poured into saturated sodium bicarbonate solution to weak alkalinity, extracted with ethyl acetate, washed with saturated saline, and dried; and the organic phase was directly concentrated under reduced pressure, subjected to sample blending, and allowed to pass through the columns (eluent: dichloromethane/methanol = 10/1, V/V) to obtain the title compound (light green solid, 1.5 g, yield: 57.7%). LC/MS (ESI) m/z: 239.9 [M+H]+.

**Step 3: 7-bromo-2-methyl-2,3,4,5-tetrahydro-1H-benzo[c]aza-1-one**

7-bromo-2,3,4,5-tetrahydro-1H-benzo[c]aza-1-one (1.4 g, 5.83 mmol) was dissolved in anhydrous N,N-dimethylformamide (20 mL); under the protection of nitrogen and in an ice bath, 60% sodium hydrogen (0.47 g, 11.7 mmol) was added portionwise, and with the temperature held, the mixture was stirred for 30 min; and then, iodomethane (1.24 g, 8.75 mmol) was added dropwise slowly at the current temperature, after which the temperature was restored to room temperature, and the mixture continued to undergo reaction for 2 hours. After the reaction was completed, the reaction mixture was quenched with saturated ammonium chloride, extracted with ethyl acetate, washed with saturated saline, and dried; and the organic phase was directly concentrated under reduced pressure, subjected to sample blending,

and allowed to pass through the columns (eluent: petroleum ether/ethyl acetate = 1/1, V/V) to obtain the title compound (white solid, 1.2 g, yield: 81.0%). LC/MS (ESI) m/z: 254.0 $[M+H]^+$.

**Step 4:(2-methyl-1-oxo-2,3,4,5-tetrahydro-1H-benzo[c]aza-7-yl)aminotert-butyl carbamate**

At room temperature, 7-bromo-2-methyl-2,3,4,5-tetrahydro-1H-benzo[c]aza-1-one (100 mg, 0.393 mmol) was dissolved in 1,4-dioxane (15 mL); aminotert-butyl carbamate (69.2 mg, 0.59 mmol), cesium carbonate (385 mg, 1.18 mmol), bis(dibenzalacetone)palladium (II) (18.0 mg, 0.02 mmol) and 4,5-bisdiphenylphosphino-9,9-dimethylxanthene (23 mg, 0.039 mmol) were added; and under the protection of nitrogen, the mixture was stirred at 95°C for 16 hours. After the reaction was completed, saturated ammonium chloride was added; the reaction mixture was extracted with ethyl acetate, and washed with saturated saline; and the organic phase was directly concentrated under reduced pressure, subjected to sample blending, and allowed to pass through the columns (eluent: petroleum ether/ethyl acetate = 1/1, V/V) to obtain the title compound (faint yellow solid, 100 mg, yield: 87.5 %). LC/MS (ESI) m/z: 291.2 $[M+H]^+$.

**Step 5: 7-amino-2-methyl-2,3,4,5-tetrahydro-1H-benzo[c]aza-1-one**

In an ice bath, (2-methyl-1-oxo-2,3,4,5-tetrahydro-1H-benzo[c]aza-7-yl)aminotert-butyl carbamate (100 mg, 0.344 mmol) was dissolved in 2 M ethyl acetate hydrochloride solution (10 mL) to react at room temperature for 16 hours. After the reaction was completed, the reaction mixture was directly concentrated under reduced pressure to obtain the title compound (faint yellow solid, 64 mg, yield: 98%). LC/MS (ESI) m/z: 191.2 $[M+H]^+$.

**Step 6: 2-amino-6-methyl-6,7,8,9-tetrahydro-5H-thiazolo[4',5':4,5]benzo[1,2-c]azolin-5-one**

7-amino-2-methyl-2,3,4,5-tetrahydro-1H-benzo[c]aza-1-one (300 mg, 1.58 mmol) was dissolved in 10 ml of acetic acid; at room temperature, potassium thiocyanate (613 mg, 6.31 mmol) was added; then, liquid bromine (252 mg, 1.58 mmol) dissolved in acetic acid was added dropwise at room temperature, after which the mixture was allowed to react at room temperature for 2 hours. After the reaction was completed, saturated sodium bicarbonate was added for quenching; the reaction mixture was adjusted to the pH of weak alkalinity, and extracted by adding ethyl acetate; the organic phase was spin-dried; the residue was treated by prep-HPLC (eluent: acetonitrile/0.1% formic acid aqueous solution = 20%-90%, V/V) to obtain the title compound (70 mg, white solid, yield: 17.9%). LC/MS (ESI) m/z: 248.1 $[M+H]^+$

**Step 7: 2'-chloro-5'-methoxy-6-methyl-N-(6-methyl-5-oxo-6,7,8,9-tetrahydro-5H-thiazolo[4', 5':4,5]b enzo[1,2-c] aza-2-yl)-[4,4'-bipyridine]-3-carboxamide**

2-amino-6-methyl-6,7,8,9-tetrahydro-5H-thiazolo[4',5':4,5]benzo[1,2-c]azolin-5-one (30 mg, 0.121 mmol), 2'-chloro-5'-methoxy-6-methyl-[4,4'-bipyridine]-3-carboxylic acid (37.2 mg, 0.133 mmol), and methylimidazole (29.9 mg, 0.364 mmol) were dissolved in 16 mL of acetonitrile and 1 mL of N,N-dimethylformamide, to allow for reaction at 75°C for 5 minutes; and N,N,N',N'-tetramethylchlorformamidine hexafluorphosphate (170 mg, 0.607 mmol) was added to allow for reaction at 75°C for 60 minutes. Saturated ammonium chloride was added; the mixture was extracted with ethyl acetate; the organic phase was directly spin-dried and concentrated; the residue was treated by prep-HPLC (eluent: acetonitrile/0.1% formic acid aqueous solution = 20%-90%, V/V) to obtain two title compounds (13 mg, faint yellow solid, yield: 21.1%). $^1H$ NMR (400 MHz, DMSO-*d*6) $\delta$ 13.02 (s, 1H), 8.85 (s, 1H), 8.15 (s, 1H), 8.09 (s, 1H), 7.61 (s, 1H), 7.56 (s, 1H), 7.45 (s, 1H), 3.59 (s, 3H), 3.17 (t, *J* = 6.1 Hz, 2H), 3.07 (s, 3H), 2.80 (t, *J* = 6.8 Hz, 2H), 2.60 (s, 3H), 2.10 - 1.92 (m, 2H). LC/MS (ESI) m/z: 508.2 $[M+H]^+$.

**Example 226: 2'-chloro-*N*-(6-(4-(difluoromethyl)-1-methyl-1*H*-1,2,3-triazol-5-yl)thiazolo[4,5-c]pyridi n-2-yl)-5'-methoxv-6-methyl-[4.4'-bipyridine]-3-carboxamide**

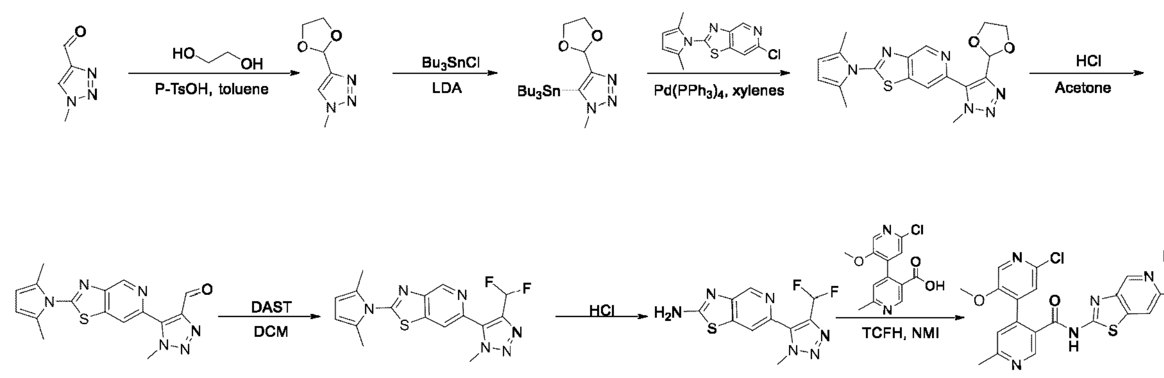


**Step 1: 4-(1,3-dioxolam-2-yl)-1-methyl-1H-1,2,3-triazole**

Ethylene glycol (2 mL, 35.9 mmol) and p-toluenesulfonic acid (0.62 g, 3.60 mmol) were added to the solution of 1-methyl-1H-1,2,3-triazol-4-formaldehyde (2 g, 18.0 mmol) in toluene (20 mL). The reaction mixture was stirred at 120°C to react for 5 hours. After complete reaction, the reaction mixture was concentrated under reduced pressure; saturated sodium bicarbonate solution was added; the resulting mixture was extracted with ethyl acetate; and the organic phase was washed with saturated saline, dried over anhydrous sodium sulfate, and concentrated under reduced pressure. The residue was purified by column chromatography (eluent: petroleum ether/ethyl acetate, gradient: 0-100% ethyl acetate) to obtain the title compound (white solid, 960 mg, yield: 34.4%). LC/MS (ESI) m/z: 156.1 $[M+H]^+$.

**Step 2: 4-(1,3-dioxolam-2-yl)-1-methyl-5-(tributylstannyl)-1H-1,2,3-triazole**

At -78°C, lithium diisopropylamide (2.9 mL, 5.80 mmol, 2 M) was added dropwise slowly to the solution of 4-(1,3-dioxolam-2-yl)-1-methyl-1H-1,2,3-triazole (750 mg, 4.83 mmol) in tetrahydrofuran (15 mL). Under the protection of nitrogen, the reaction mixture was stirred at -78 °C to react for 1 hour. Then, tributyl tin chloride (1.4 mL, 5.16 mmol) were added to the reaction mixture, and stirred to react for half an hour. After complete reaction, the saturated solution of ammonium chloride was added; the resulting mixture was extracted with ethyl acetate; and the organic phase was washed with saturated saline, dried over anhydrous sodium sulfate, and concentrated under reduced pressure. The residue was purified by column chromatography (eluent: petroleum ether/ethyl acetate, gradient: 0-50% ethyl acetate) to obtain the title compound (yellow oil, 660 mg, yield: 30.7%). LC/MS (ESI) m/z: 446.2 $[M+H]^+$.

**Step 3: 6-(4-(1,3-dioxolam-2-yl)-1-methyl-1H-1,2,3-triazol-5-yl)-2-(2,5-dimethyl-1H-pyrrol-1-yl)thiazo lo[4,5-c] pyridine**

4-(1,3-dioxolam-2-yl)-1-methyl-5-(tributylstannyl)-1H-1,2,3-triazole (660 mg, 1.49 mmol) and tetrakis(triphenylphosphine)palladium (460 mg, 0.398 mmol) were added to the solution of 6-chloro-2-(2,5-dimethyl-1H-pyrrol-1-yl)thiazolo [4,5-c]pyridine (350 mg, 1.33 mmol) in ditoluene (5 mL). Under the protection of nitrogen, the reaction mixture was stirred at 160°C in the presence of microwaves to react for half an hour. After complete reaction, the reaction mixture was concentrated under reduced pressure. The residue was purified by column chromatography (eluent: petroleum ether/ethyl acetate, gradient: 0-100% ethyl acetate) to obtain the title compound (yellow oil, 175 mg, yield: 34.5%). LC/MS (ESI) m/z: 383.2 $[M+H]^+$.

**Step 4: 5-(2-(2,5-dimethyl-1H-pyrrol-1-yl)thiazolo[4,5-c]pyridin-6-yl)-1-methyl-1H-1,2,3-triazol-4-for maldehyde**

1 N hydrochloric acid aqueous solution (1.6 mL) was added to the solution of 6-(4-(1,3-dioxolam-2-yl)-1-methyl-1H-1,2,3-triazol-5-yl)-2-(2,5-dimethyl-1H-pyrrol-1-yl)thiazolo[4,5-c]pyr idine (150 mg, 0.392 mmol) in acetone (5 mL). The reaction mixture was stirred at room temperature to react for half an hour. After complete reaction, saturated sodium bicarbonate solution was added; the resulting mixture was extracted with ethyl acetate; and the organic phase was washed with saturated saline, dried over anhydrous sodium sulfate, and concentrated under reduced pressure. The residue was purified by column chromatography (eluent: petroleum ether/ethyl acetate, gradient: 0-40% ethyl acetate) to obtain the title compound (white solid, 85 mg, yield: 64.0%). LC/MS (ESI) m/z: 339.2 $[M+H]^+$.

**Step 5: 6-(4-(difluoromethyl)-1-methyl-1H-1,2,3-triazol-5-yl)-2-(2,5-dimethyl-1H-pyrrol-1-yl)thiazolo [4,5-c]pyridine**

At 0°C, diethylaminosulfur trifluoride (121 mg, 0.751 mmol) was added to the solution of 5-(2-(2,5-dimethyl-1H-pyrrol-1-yl)thiazolo[4,5-c]pyridin-6-yl)-1-methyl-1H-1,2,3-triazol-4-formaldehyde (85 mg, 0.251 mmol) in dichloromethane (10 mL). The reaction mixture was stirred at room temperature to react for 2 hours. After complete reaction, the reaction mixture was poured into sodium bicarbonate in ice water; the mixture was extracted with dichloromethane; and the organic phase was washed with saturated saline, dried over anhydrous sodium sulfate, and concentrated under reduced pressure. The residue was purified by column chromatography (eluent: petroleum ether/ethyl acetate, gradient: 0-20% ethyl acetate) to obtain the title compound (white solid, 20 mg, yield: 22.2%). LC/MS (ESI) m/z: 361.1 $[M+H]^+$.

**Step 6: 6-(4-(difluoromethyl)-1-methyl-1H-1,2,3-triazol-5-yl)thiazolo[4,5-c]pyridin-2-amine**

6-(4-(difluoromethyl)-1-methyl-1H-1,2,3-triazol-5-yl)-2-(2,5-dimethyl-1H-pyrrol-1-yl)thiazolo[4,5-c]pyridin e (20 mg, 0.055 mmol) in 2 N hydrochloric acid aqueous solution (2 mL) was stirred at 80°C to react for 2 hours. After complete reaction, the reaction mixture was concentrated under reduced pressure to obtain the title compound (faint yellow solid, 15 mg, yield: 95.8%). LC/MS (ESI) m/z: 283.1 $[M+H]^+$.

**Step 7: 2'-chloro-N-(6-(4-(difluoromethyl)-1-methyl-1H-1,2,3-triazol-5-yl)thiazolo[4,5-c]pyridin-2-yl)-5'-methoxy-6-methyl-[4,4'-bipyridine]-3-carboxamide**

2'-chloro-5'-methoxy-6-methyl-[4,4'-bipyridine]-3-carboxylic acid (16 mg, 0.057 mmol) and *N*-methylimidazole (22 mg, 0.268 mmol) were sequentially added to 6-(4-(difluoromethyl)-1-methyl-1H-1,2,3-triazol-5-yl)thiazolo[4,5-c] pyridin-2-amine (15 mg, 0.053 mmol) in the mixed solution of *N,N*-dimethylformamide (0.6 mL) and acetonitrile (1 mL). Then, at 70°C, N,N,N',N'-tetramethylchlorformamidine hexafluorphosphate (30 mg, 0.107 mmol) was added to the mixture. The mixture was stirred at 70°C for 2 hours. After complete reaction, water was added; the resulting mixture was extracted with ethyl acetate; and the organic phase was washed with saturated saline, dried over anhydrous sodium sulfate, and concentrated under reduced pressure. The residue was purified by prep-HPLC (eluent: acetonitrile/0.1% formic acid aqueous solution = 20-95%, V/V) to obtain the title compound (white solid, 3.2 mg, yield: 11.1%). $^1H$ NMR (400 MHz, DMSO-$d_6$) $\delta$ 13.39 (s, 1H), 9.21 (s, 1H), 8.90 (s, 1H), 8.45 (s, 1H), 8.17 (s, 1H), 7.60 (s, 1H), 7.48 (s, 1H), 7.29 (t, J = 53.2 Hz, 1H), 4.19 (s, 3H), 3.60 (s, 3H), 2.62 (s, 3H). LC/MS (ESI) m/z: 543.2 $[M+H]^+$.

**Example 227: 2'-chloro-N-(5-(1,4-dimethyl-1H-1,2,3-triazol-5-yl)-6-fluorothiazolo[5,4-b]pyridin-2-yl) -5'-methoxy-6-methyl-[4,4'-bipyridine]-3-carboxamide**

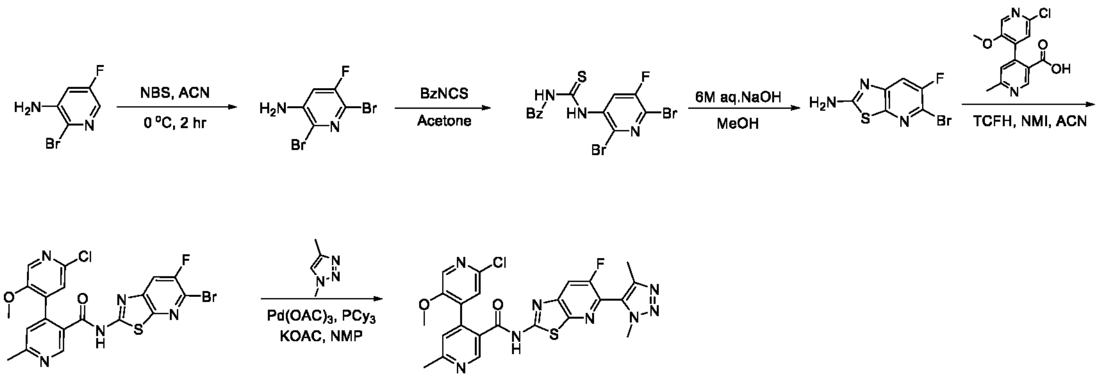


**Step 1: 2,6-dibromo-5-fluoropyridin-3-amine**

Under the protection of nitrogen, 2-bromo-5-fluoropyridin-3-amine (2 g, 10.5 mmol) was dissolved in acetonitrile (20 mL); in an ice bath, N-bromosuccinimide (2.05 g, 11.5 mmol) was slowly added; and the reaction mixture was stirred at room temperature to react for 2 hours. After the reaction was completed, water was added; the mixture was extracted with ethyl acetate; the organic phase was washed with saturated saline, dried over anhydrous sodium sulfate, filtered; and the filtrate was concentrated under reduced pressure. The residue was purified by column chromatography (eluent: petroleum ether/ethyl acetate, gradient: 15%-50% ethyl acetate) to obtain the title compound (brown solid, 2.6 g, yield: 92%). LC/MS

(ESI) m/z: 269 [M+H]+.

**Step 2: N-((2,6-dibromo-5-fluoropyridin-3-yl)carbamothioyl)benzamide**

At room temperature, 2,6-dibromo-5-fluoropyridin-3-amine (2.6 g, 9.7 mmol) was dissolved in acetone (20 mL); benzoyl isothiocyanate (2.4 g, 14.5 mmol) was added portionwise slowly; and the reaction mixture was stirred at 60°C to react for 4 hours. After the reaction was completed; the reaction mixture was cooled to 0°C, filtered; and the filter cake was washed with acetone. The filter cake was dried under reduced pressure to obtain the title compound (white solid, 3.5 g, yield: 83.5%). LC/MS (ESI) m/z: 432 [M+H]+.

**Step 3: 5-bromo-6-fluorothiazolo[5,4-b]pyridin-2-amine**

At room temperature, N-((2,6-dibromo-5-fluoropyridin-3-yl)carbamothioyl)benzamide (3.5 g, 8.1 mmol) was dissolved in 6 M sodium hydroxide in water (15 mL) and methanol (15 mL); and the mixture was stirred at 70°C to react for 5 hours. After the reaction was completed, the reaction mixture was cooled to 0°C, and filtered; and the filter cake was washed with water, dried under reduced pressure to obtain the title compound (white solid, 1.7 g, yield: 84.8%). LC/MS (ESI) m/z: 248 [M+H]+.

**Step 4: N-(5-bromo-6-fluorothiazolo[5,4-b]pyridin-2-yl)-2'-chloro-5'-methoxy-6-methyl-[4,4'-bipyridi ne]-3-carboxamide**

At room temperature and under the protection of nitrogen, 5-bromo-6-fluorothiazolo[5,4-b]pyridin-2-amine (142.4 mg, 0.6 mmol) and 2'-chloro-5'-methoxy-6-methyl-[4,4'-bipyridine]-3-carboxylic acid (80 mg, 0.3 mmol) were dissolved in acetonitrile (2 mL); N-methylimidazole (94.3 mg, 1.1 mmol) was added; the mixture was stirred at 70°C for 10 minutes; then, N,N,N',N'-tetramethylchlorformamidine hexafluorphosphate (88.6 mg, 0.3 mmol) was added; and the reaction mixture was continued with stirring at 70°C to react for 1 hour. Ethyl acetate and water were added to the reaction mixture for stratification; the organic phase was washed with saturated saline, dried over anhydrous sodium sulfate, and filtered; and the filtrate was concentrated under reduced pressure. The residue was purified by column chromatography (eluent: dichloromethane/methanol, gradient: 1%-10% methanol) to obtain the title compound (white solid, 40 mg, yield: 27.4%). LC/MS (ESI) m/z: 508 [M+H]+.

**Step 5: 2'-chloro-N-(5-(1,4-dimethyl-1H-1,2,3-triazol-5-yl)-6-fluorothiazolo[5,4-b]pyridin-2-yl)-5'-met hoxy-6-methyl-[4,4'-bipyridine]-3-carboxamide**

Under the protection of nitrogen, N-(5-bromo-6-fluorothiazolo[5,4-b]pyridin-2-yl)-2'-chloro-5'-methoxy-6-methyl-[4,4'-bipyridine]-3-carboxa mide (40 mg, 0.08 mmol), 1,4-dimethyl-1H-1,2,3-triazole (15.3 mg, 0.16 mmol), potassium acetate (15.4 mg, 0.16 mmol), tricyclohexylphosphine (8.8 mg, 0.03 mmol) and palladium acetate (3.5 mg, 0.02 mmol) were dissolved in N-methylpyrrolidone (2 mL) solvent. The mixture was subjected to nitrogen replacement three times, and stirred at 120°C under the protection of nitrogen to react for 12 hours. The reaction mixture was diluted with ethyl acetate, and washed with water and saturated saline in sequence; and the organic phase was dried over anhydrous sodium sulfate, and concentrated under reduced pressure. The residue was purified by pre-HPLC to obtain the title compound (white solid, 2.47 mg, yield: 6.0 %). $^1H$ NMR (400 MHz, DMSO-$d_6$) δ 13.36 (s, 1H), 8.88 (s, 1H), 8.40 (d, *J* = 10.7 Hz, 1H), 8.18 (s, 1H), 7.61 (s, 1H), 7.50 (s, 1H), 4.03 (s, 3H), 3.63 (s, 3H), 2.62 (s, 3H), 2.25 (d, *J* = 1.8 Hz, 3H). LC/MS (ESI) m/z: 525 [M+H]+.

**Example 228: 2'-chloro-N-(7-cyano-6-(1,4-dimethyl-1H-1,2,3-triazol-5-yl)thiazolo[4,5-c]pyridin-2-yl)-5'-methoxy-6-methyl-[4,4'-bipyridine]-3-carboxamide**

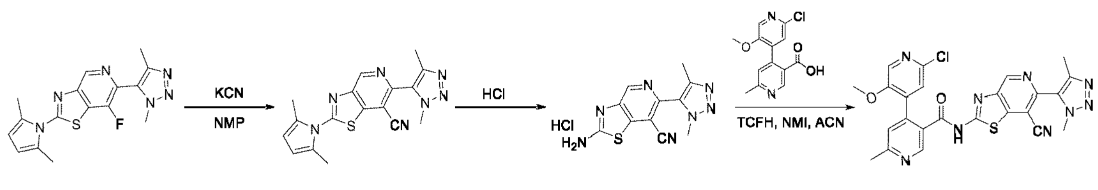

**Step 1: 6-(1,4-dimethyl-1H-1,2,3-triazol-5-yl)-2-(2,5-dimethyl-1H-pyrrol-1-yl)thiazolo[4,5-c]pyridin-7 -nitrile**

At room temperature, potassium cyanide (105 mg, 1.61 mmol) was added to the solution of 6-(1,4-dimethyl-1H-1,2,3-triazol-5-yl)-2-(2,5-dimethyl-1H-pyrrol-1-yl)-7-fluorothiazolo[4,5-c]pyridine (500 mg, 1.46 mmol) in N-methylpyrrolidone (5 mL); and then, the mixture was stirred at 100 °C to react for 4 hours. The reaction mixture was cooled to room temperature, added with ethyl acetate, sequentially washed with water and saturated saline, dried over anhydrous sodium sulfate, and filtered; and the filtrate was concentrated under reduced pressure. The residue was purified by column chromatography (eluent: dichloromethane/methanol, gradient: 0-5% methanol) to obtain the title compound (yellow solid, 25 mg, yield: 4.9%). LC/MS (ESI) m/z: 350.2 $[M+H]^+$.

**Step 2: 2-amino-6-(1,4-dimethyl-1H-1,2,3-triazol-5-yl)thiazolo[4,5-c]pyridin-7-nitrile hydrochloride**

At room temperature, 6-(1,4-dimethyl-1H-1,2,3-triazol-5-yl)-2-(2,5-dimethyl-1H-pyrrol-1-yl)thiazolo[4,5-c]pyridin-7-nitrile (25 mg, 0.072 mmol) was dissolved in 1,4-dioxane (2 mL) and 4 N hydrochloric acid (2 mL); and the reaction mixture was stirred at 80°C to react for 2 hours. The mixture was concentrated under reduced pressure to obtain the title compound (yellow solid, 8 mg, yield: 36.1%). LC/MS (ESI) m/z: 272.2 $[M+H]^+$.

**Step 3: 2'-chloro-N-(7-cyano-6-(1,4-dimethyl-1H-1,2,3-triazol-5-yl)thiazolo[4,5-c]pyridin-2-yl)-5'-met hoxy-6-methyl-[4,4'-bipyridine]-3-carboxamide**

At room temperature, 2-amino-6-(1,4-dimethyl-1H-1,2,3-triazol-5-yl)thiazolo[4,5-c]pyridin-7-nitrile hydrochloride (8 mg, 0.026 mmol), 2'-chloro-5'-methoxy-6-methyl-[4,4'-bipyridine]-3-carboxylic acid (8 mg, 0.029 mmol), and N-methylimidazole (12.8 mg, 0.16 mmol) were dissolved in acetonitrile (1 mL); the mixture was stirred at 70°C for 5 minutes; then, the solution of N,N,N',N'-tetramethylchlorformamidine hexafluorphosphate (14.6 mg, 0.052 mmol) in acetonitrile (1 mL) was added to the above solution; and the reaction mixture was stirred at 70°C to react for 2 hours. The mixture was filtered, and then purified by pre-HPLC to obtain the title compound (2.3 mg, white solid, yield: 16.6%). $^1$H NMR (400 MHz, DMSO-$d_6$) $\delta$ 13.75 (s, 1H), 9.40 (s, 1H), 8.94 (s, 1H), 8.18 (s, 1H), 7.60 (s, 1H), 7.48 (s, 1H), 4.03 (s, 3H), 3.62 (s, 3H), 2.62 (s, 3H), 2.32 (s, 3H). LC/MS (ESI) m/z: 532.1 $[M+H]^+$.

**Example 229: 2'-chloro-N-(6-(1,4-dimethyl-1H-1,2,3-triazol-5-yl)-7-methoxythiazolo[4,5-c]pyridin-2-yl)-5'-methoxy-6-methyl-[4,4'-bipyridine]-3-carboxamide**

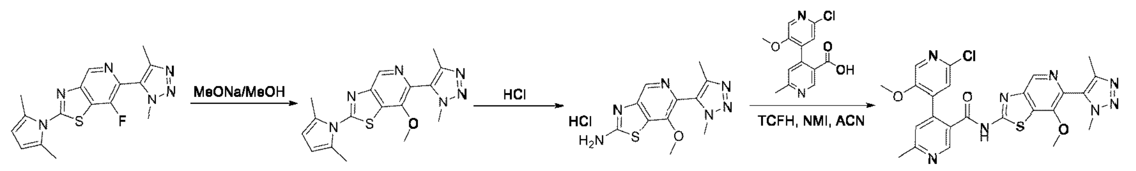


**Step 1: 6-(1,4-dimethyl-1H-1,2,3-triazol-5-yl)-2-(2,5-dimethyl-1H-pyrrol-1-yl)-7-methoxythiazolo[4,5-c]pyridine**

At room temperature, 30% methanol solution of sodium methylate (10 mL) was added to the solution of 6-(1,4-dimethyl-1H-1,2,3-triazol-5-yl)-2-(2,5-dimethyl-1H-pyrrol-1-yl)-7-fluorothiazolo[4,5-c]pyridine (1 g, 2.92 mmol) in methanol (10 mL); and the mixture was stirred at 80°C to react for 2 hours. The reaction mixture was concentrated under reduced pressure; and the residue was purified by silica-gel column chromatography (eluent: dichloromethane/methanol, gradient: 0-5% methanol) to obtain the title compound (yellow solid, 920 mg, yield: 88.9%). LC/MS (ESI) m/z: 355.0 $[M+H]^+$.

**Step 2: 6-(1,4-dimethyl-1H-1,2,3-triazol-5-yl)-7-methoxythiazolo[4,5-c]pyridin-2-amine hydrochloride**

At room temperature, 6-(1,4-dimethyl-1H-1,2,3-triazol-5-yl)-2-(2,5-dimethyl-1H-pyrrol-1-yl)-7-methoxythiazolo [4,5-c]pyridine (920 mg, 2.596 mmol) was dissolved in 1,4-dioxane (15 mL) and 4 N hydrochloric acid (15 mL); and the reaction mixture was stirred at 80°C to react for 1 hour. The reaction mixture was concentrated under reduced pressure; and the residue was pulped with acetonitrile, and dried under reduced pressure to obtain the title compound (yellow solid, 420 mg, yield: 51.7%). LC/MS (ESI) m/z: 276.9 $[M+H]^+$.

**Step 3: 2'-chloro-N-(6-(1,4-dimethyl-1H-1,2,3-triazol-5-yl)-7-methoxythiazolo[4,5-c]pyridin-2-yl)-5'-methoxy-6-methyl-[4,4'-bipyridine]-3-carboxamide**

At room temperature, 6-(1,4-dimethyl-1H-1,2,3-triazol-5-yl)-7-methoxythiazolo[4,5-c]pyridin-2-amine hydrochloride (100 mg, 0.32 mmol), 2'-chloro-5'-methoxy-6-methyl-[4,4'-bipyridine]-3-carboxylic acid (98 mg, 0.35 mmol), and N-methylimidazole (158 mg, 1.92 mmol) were dissolved in acetonitrile (3 mL); the mixture was stirred at 70°C for 5 minutes; and then, the solution of N,N,N',N'-tetramethylchlorformamidine hexafluorphosphate (153 mg, 0.54 mmol) in acetonitrile (1 mL) was added to the reaction mixture, which was stirred at 70°C to react for 1 hour. The reaction mixture was diluted with ethyl acetate, washed with water and saturated saline in sequence, dried over anhydrous sodium sulfate, and concentrated under reduced pressure. The residue was purified by pre-HPLC to obtain the title compound (white solid, 60 mg, yield: 35%). $^1$H NMR (400 MHz, DMSO-*d*$_6$) δ 13.44 (s, 1H), 8.94 (s, 1H), 8.89 (s, 1H), 8.19 (s, 1H), 7.61 (s, 1H), 7.49 (s, 1H), 3.95 (s, 3H), 3.93 (s, 3H), 3.63 (s, 3H), 2.62 (s, 3H), 2.19 (s, 3H). LC/MS (ESI) m/z: 537.1 [M+H]$^+$.

**Example 230: 2'-chloro-N-(6-(1,4-dimethyl-1H-1,2,3-triazol-5-yl)-7-fluorothiazolo[4,5-c]pyridin-2-yl)-5'-(methoxy-d3)-6-methyl-[4,4'-bipyridine]-3-carboxamide**

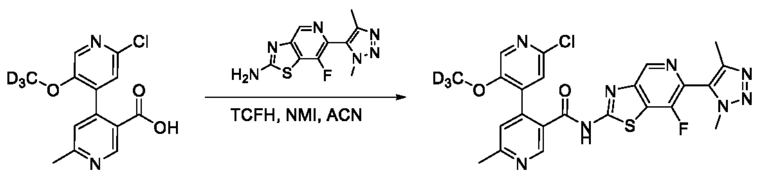


At room temperature, 6-(1,4-dimethyl-1H-1,2,3-triazol-5-yl)-7-fluorothiazolo[4,5-c]pyridin-2-amine (11 mg, 0.04 mol), 2'-chloro-5'-(methoxy-d3)-6-methyl-[4,4'-bipyridine]-3-carboxylic acid (12.8 mg, 0.045 mol), and N-methylimidazole (16.5 mg, 0.2 mmol) were dissolved in acetonitrile (2 mL); the mixture was stirred at 70°C for 5 minutes; then, N,N,N',N'-tetramethylchlorformamidine hexafluorphosphate (22.4 mg, 0.08 mol) was added; and the reaction mixture was stirred at 70°C to react for 2 hours. The reaction mixture was cooled to room temperature and diluted with water; the aqueous phase was extracted with ethyl acetate twice; the organic phases were combined, washed with saturated saline, dried over anhydrous sodium sulfate, and filtered. The filtrate was concentrated under reduced pressure; and the residue was purified by pre-HPLC to obtain the title compound (faint yellow solid, 2.2 mg, yield: 11%). $^1$H NMR (400 MHz, DMSO-*d*$_6$) δ 9.01 (s, 1H), 8.96 (s, 1H), 8.16 (s, 1H), 7.54 (s, 1H), 7.41 (s, 1H), 4.01 (s, 3H), 2.60 (s, 3H), 2.25 (d, *J* = 1.7 Hz, 3H). LC/MS (ESI) (m/z): 528 [M+H]$^+$.

**Example 231: 2'-chloro-N-(6-(1,4-dimethyl-1H-1,2,3-triazol-5-yl)-7-fluorothiazolo[4,5-c]pyridin-2-yl)-5'-(methoxy-d3)-6-(methyl-d3)-[4,4'-bipyridine]-3-carboxamide**

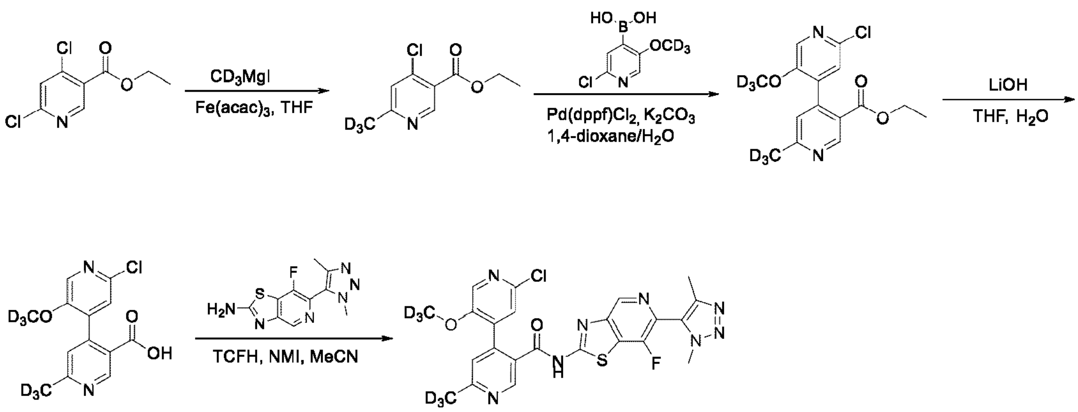


**Step 1: 4-chloro-6-(methyl-d3)ethyl nicotinate**

Under the protection of nitrogen, 4,6-dichloropyridin-3-ethyl carboxylate (2 g, 9.09 mmol) and ferric acetylacetonate (320 mg, 0.91 mmol) were dissolved in anhydrous tetrahydrofuran (30 mL); and at room temperature, the solution of 1 M deuteromethyl magnesium iodide (10 mL, 9.99 mmol) was dropped slowly. The reaction mixture was stirred at room temperature to react for 1 hour, quenched with saturated ammonium chloride in water, and extracted with ethyl acetate. The organic phase was washed with water and saturated saline in sequence, dried over anhydrous sodium sulfate, and filtered; and the filtrate was concentrated under reduced pressure. The residue was purified by column chromatography (eluent: petroleum ether/ethyl acetate, gradient: 0-15% ethyl acetate) to obtain the title compound (yellow-green oil, 440 mg, yield: 23.8%). LC/MS (ESI) m/z: 203.2 $[M+H]^+$.

**Step 2: 2'-chloro-5'-(methoxy-d3)-6-(methyl-d3)-[4,4'-bipyridine]-3-ethyl carboxylate**

At room temperature and under the protection of nitrogen, 4-chloro-6-deuteromethylethyl nicotinate (410 mg, 2.02 mmol), (2-chloro-5-(methoxy-d3)pyridin-4-yl)boric acid (380 mg, 2.02 mmol), and potassium carbonate (838 mg, 6.06 mmol) were dissolved in 1,4-dioxane (20 mL) and water (4 mL); and then, 1,1-bis(diphenylphosphino)ferrocene palladium dichloride (148 mg, 0.2 mmol) was added. The reaction mixture was subjected to nitrogen replacement three times, and was stirred at 80°C under the protection of nitrogen to react for 1 hour. The reaction mixture was filtered through diatomite; the filter cake was washed with methanol; the filtrate was concentrated under reduced pressure; and the resulting residue was purified by column chromatography (eluent: petroleum ether/ethyl acetate, gradient: 10%-30% ethyl acetate) to obtain the title compound (white solid, 350 mg, yield: 45.3%). LC/MS (ESI) m/z: 313.2 $[M+H]^+$.

**Step 3: 2'-chloro-5'-(methoxy-d3)-6-(methyl-d3)-[4,4'-bipyridine]-3-carboxylic acid**

At room temperature, 2'-chloro-5'-(methoxy-d3)-6-(methyl-d3)-[4,4'-bipyridine]-3-ethyl carboxylate (350 mg, 1.12 mmol) was dissolved in water (5 mL) and tetrahydrofuran (5 mL); then, lithium hydroxide (94 mg, 2.24 mmol) was added; and the reaction mixture was stirred at room temperature to react for 16 hours. The reaction mixture was concentrated under reduced pressure to remove tetrahydrofuran; the residual aqueous phase was adjusted to pH of 4-5 with 2N hydrochloric acid to allow for the precipitation of solids, and filtered; and the filter cake was dried under reduced pressure to obtain the title compound (white solid, 280 mg, yield: 87.8%). LC/MS (ESI) m/z: 285.0 $[M+H]^+$.

**Step 4:**

**2'-chloro-N-(6-(1,4-dimethyl-1H-1,2,3-triazol-5-yl)-7-fluorothiazolo[4,5-c]pyridin-2-yl)-5'-(methoxy-d3 )-6-(methyl-d3)-[4,4'-bipyridine]-3-carboxamide**

At room temperature, 2'-chloro-5'-(methoxy-d3)-6-(methyl-d3)-[4,4'-bipyridine]-3-carboxylic acid (280 mg, 0.98 mmol) and 6-(3,5-dimethyl-1,2,3-triazol-4-yl)-7-fluoro[1,3]thiazolo[4,5-c]pyridin-2-amine (19 mg, 0.07 mmol) were dissolved in 10 mL of acetonitrile; N-methylimidazole (726.6 mg, 8.85 mmol) was added; with the temperature increased to 70°C, the mixture was stirred for 5 minutes; then, N,N,N',N'-tetramethylchlorformamidine hexafluorphosphate (827.7 mg, 2.95 mmol) was added; and the reaction mixture was continued with stirring at 70°C to react for 16 hours. Ethyl acetate and water were added to the reaction mixture for stratification; the organic phase was washed with saturated sodium chloride solution, dried over anhydrous sodium sulfate, and filtered; the filtrate was concentrated under reduced pressure. The residue was purified by pre-HPLC (eluent: acetonitrile/0.1% formic acid aqueous solution, gradient: 25%-90%) to obtain the title compound (white solid, 73.19 mg, yield: 14.0%). $^1H$ NMR (400 MHz, DMSO-$d_6$) $\delta$ 13.64 (s, 1H), 9.15 (d, $J$ = 1.9 Hz, 1H), 8.91 (s, 1H), 8.18 (s, 1H), 7.63 (s, 1H), 7.52 (s, 1H), 4.03 (s, 3H), 2.27 (s, 3H). LC/MS (ESI) m/z: 531 $[M+H]^+$.

**Example 232: 2'-chloro-N-(6-(1,4-dimethyl-1H-1,2,3-triazol-5-yl)-5-fluorothiazolo[4,5-b]pyridin-2-yl) -5-methoxy-6-methyl-[4,4'-bipyridine]-3-carboxamide**

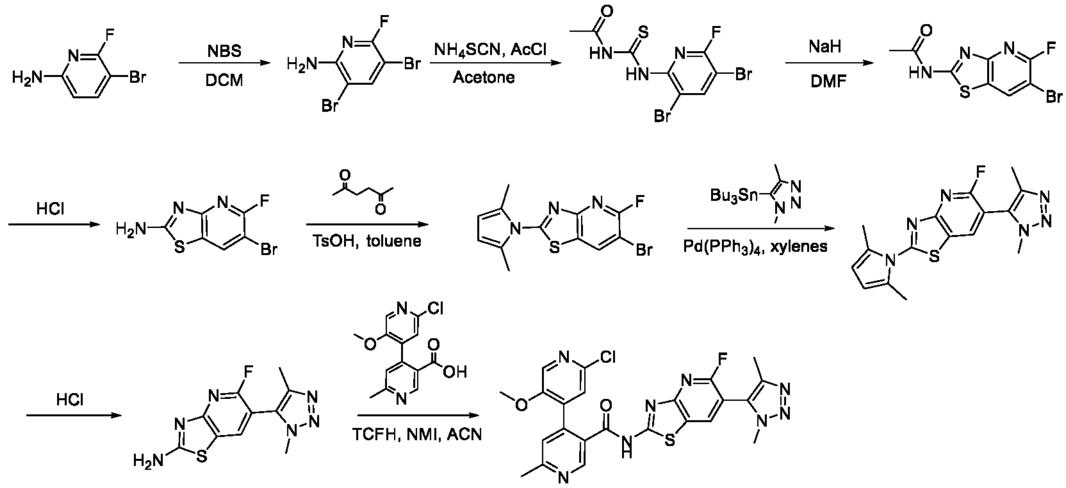


**Step 1: 3,5-dibromo-6-fluoropyridin-2-amine**

5-bromo-6-fluoropyridin-2-amine (3 g, 15.7 mmol) was dissolved in dichloromethane (10 mL); N-bromosuccinimide (3.08 g, 17.3 mmol) was added; and the reaction mixture was stirred at room temperature to react for 2 hours. The mixture was concentrated under reduced pressure; and the residue was purified by silica-gel column chromatography (eluent: petroleum ether/ethyl acetate, gradient: 10-20% ethyl acetate) to obtain the title compound (yellow solid, 3.7 g, yield: 87.3%). LC/MS (ESI) m/z: 270.9 $[M+H]^+$.

**Step 2: N-((3,5-dibromo-6-fluoropyridin-2-yl)carbamothioyl)acetamide**

Ammonium thiocyanate (0.85 g, 11.1 mmol) was dissolved in acetone (20 mL); acetyl chloride (0.8 mL, 11.3 mmol) was added; and the reaction mixture was stirred at 50°C for half an hour. At 50°C, 3,5-dibromo-6-fluoropyridin-2-amine (2.5 g, 9.26 mmol) was slowly added to the above reaction mixture, which was then continued with stirring to react for 16 hours. The mixture was concentrated under reduced pressure; and the residue was purified by silica-gel column chromatography (eluent: dichloromethane/ethyl acetate, gradient: 0-50% ethyl acetate) to obtain the title compound (yellow solid, 600 mg, yield: 17.4%). LC/MS (ESI) m/z: 369.9 $[M+H]^+$.

**Step 3: N-(6-bromo-5-fluorothiazolo[4,5-b]pyridin-2-yl)acetamide**

At room temperature and under the protection of nitrogen, N-((3,5-dibromo-6-fluoropyridin-2-yl)carbamothioyl) acetamide (600 mg, 1.62 mmol) was dissolved in N,N-dimethylformamide (10 mL); 60% sodium hydride (129 mg, 3.23 mmol) was added; and the reaction mixture was stirred at 80°C to react for 3 hours. The reaction mixture was quenched at 0°C with saturated ammonium chloride solution; the product was precipitated from the mixture, and filtered; and the filter cake was purified by silica-gel column chromatography (eluent: dichloromethane/methanol, gradient: 10-15% methanol) to obtain the title compound (yellow solid, 130 mg, yield: 27.7%). LC/MS (ESI) m/z: 290.0 $[M+H]^+$.

**Step 4: 6-bromo-5-fluorothiazolo[4,5-b]pyridin-2-amine hydrochloride**

At room temperature, N-(6-bromo-5-fluorothiazolo[4,5-b]pyridin-2-yl)acetamide (130 mg, 0.448 mmol) was dissolved in the solution of 6 N hydrochloric acid (5 mL); and the reaction mixture was stirred at 120°C to react for 2 hours. The reaction mixture was cooled to room temperature to allow for the precipitation of solids, and filtered; the filter cake was pulped with acetonitrile to obtain the title compound (100 mg, brown solid, yield: 90%). LC/MS (ESI) m/z: 248.0 $[M+H]^+$.

**Step 5: 6-bromo-2-(2,5-dimethyl-1H-pyrrol-1-yl)-5-fluorothiazolo[4,5-b]pyridine**

At room temperature, 2,5-hexanedione (64 mg, 0.562 mmol) and p-toluenesulfonic acid hydrate (10 mg, 0.056 mmol) were sequentially added to the solution of 6-bromo-5-fluorothiazolo[4,5-b]pyridin-2-amine hydrochloride (80 mg, 0.281 mmol) in toluene (5 mL); and then, the mixture was stirred at 120°C to react for 16 hours. The reaction mixture was cooled to room temperature, diluted with ethyl acetate, sequentially washed with sodium bicarbonate in water and saturated saline, dried over anhydrous sodium sulfate, and filtered; and the filtrate was concentrated under reduced

pressure. The residue was purified by silica-gel column chromatography (eluent: petroleum ether/ethyl acetate, gradient: 0-10% ethyl acetate) to obtain the title compound (pink solid, 40 mg, yield: 43.6%). LC/MS (ESI) m/z: 326.0 $[M+H]^+$.

**Step 6: 6-(1,4-dimethyl-1H-1,2,3-triazol-5-yl)-2-(2,5-dimethyl-1H-pyrrol-1-yl)-5-fluorothiazolo[4,5-b] pyridine**

At room temperature and under the protection of nitrogen, tetrakis(triphenylphosphine)palladium (43 mg, 0.037 mmol) was added to the solution of 6-bromo-2-(2,5-dimethyl-1H-pyrrol-1-yl)-5-fluorothiazolo[4,5-b]pyridine (40 mg, 0.123 mmol) and 1,4-dimethyl-5-(tributylstannyl)-1H-1,2,3-triazole (71 mg, 0.184 mmol) in ditoluene (3 mL); the mixture was subjected to nitrogen replacement three times; and under the protection of nitrogen, the mixture was stirred at 150°C to react for 3 hours. The mixture was concentrated under reduced pressure; and the residue was purified by silica-gel column chromatography (eluent: petroleum ether/ethyl acetate, gradient: 90-100% ethyl acetate) to obtain the title compound (faint yellow solid, 35 mg, yield: 83.4%). LC/MS (ESI) m/z: 343.2 $[M+H]^+$.

**Step 7: 6-(1,4-dimethyl-1H-1,2,3-triazol-5-yl)-5-fluorothiazolo[4,5-b]pyridin-2-amine hydrochloride**

At room temperature, 6-(1,4-dimethyl-1H-1,2,3-triazol-5-yl)-2-(2,5-dimethyl-1H-pyrrol-1-yl)-5-fluorothiazolo [4,5-b]pyridine (35 mg, 0.102 mmol) was dissolved in 2 N hydrochloric acid (3 mL); and the reaction mixture was stirred at 80°C to react for 2 hours. The reaction mixture was concentrated under reduced pressure; and the residue was pulped with acetonitrile to obtain the title compound (brown solid, 20 mg, yield: 65.2%). LC/MS (ESI) m/z: 265.2 $[M+H]^+$.

**Step 8: 2'-chloro-N-(6-(1,4-dimethyl-1H-1,2,3-triazol-5-yl)-5-fluorothiazolo[4,5-b]pyridin-2-yl)-5-met hoxy-6-methyl-[4,4'-bipyridine]-3-carboxamide**

At room temperature, 6-(1,4-dimethyl-1H-1,2,3-triazol-5-yl)-5-fluorothiazolo[4,5-b]pyridin-2-amine hydrochloride (20 mg, 0.067 mmol), 2'-chloro-5'-methoxy-6-methyl-[4,4'-bipyridine]-3-carboxylic acid (21 mg, 0.073 mmol), and N-methylimidazole (22 mg, 0.268 mmol) were dissolved in acetonitrile (3 mL), and stirred at 70°C for 5 minutes; then, N,N,N',N'-tetramethylchlorformamidine hexafluorphosphate (28 mg, 0.10 mmol) was added to the reaction mixture, which was then basically clarified; and then the reaction mixture was stirred at 70°C to react for 2 hours. The reaction mixture was filtered; and the filtrate was purified by pre-HPLC to obtain the title compound (16.63 mg, white solid, yield: 41.9%). $^1H$ NMR (400 MHz, DMSO-$d_6$) δ 13.53 (s, 1H), 8.90 (s, 1H), 8.75 (d, $J$ = 9.3 Hz, 1H), 8.18 (s, 1H), 7.61 (s, 1H), 7.49 (s, 1H), 3.93 (s, 3H), 3.61 (s, 3H), 2.62 (s, 3H), 2.20 (s, 3H). LC/MS (ESI) m/z: 525.2 $[M+H]^+$.

**Example 233: 2'-chloro-N-(6-(1,4-dimethyl-1H-1,2,3-triazol-5-yl)-7-methylthiazolo[4,5-c]pyridin-2-yl) -5'-methoxy-6-methyl-[4,4'-bipyridine]-3-carboxamide**

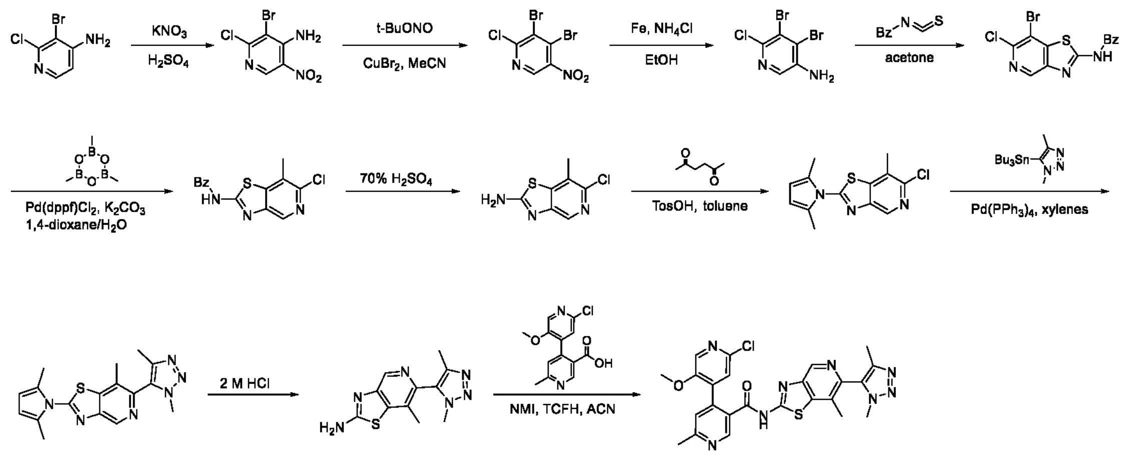


**Step 1: 3-bromo-2-chloro-5-nitropyridin-4-amine**

Under the protection of nitrogen, 3-bromo-2-chloropyridin-4-amine (5 g, 24.1 mmol) was dissolved in concentrated sulfuric acid (50 mL); the reaction mixture was cooled to 0°C; potassium nitrate (4.9 g, 48.5 mmol) was added portionwise; and the reaction mixture was continued with stirring at 90°C for 3 hours. The reaction mixture was cooled to

room temperature, and slowly poured into ice blocks, allowing for the precipitation of yellow solids, which were then filtered. The filter cake was dried under reduced pressure to obtain the title compound (yellow solid, 4.6 g, yield: 75.6%). LC/MS (ESI) m/z: 252/254 $[M+H]^+$.

**Step 2: 3,4-dibromo-2-chloro-5-nitropyridine**

Under the protection of nitrogen, 3-bromo-2-chloro-5-nitropyridin-4-amine (3.4 g, 13.5 mmol) and copper bromide (4.5 g, 20.1 mmol) were dissolved in acetonitrile (50 mL); the reaction mixture was cooled to 0°C; tert-butyl nitrite (2.0 g, 19.6 mmol), was added dropwise slowly; and under the protection of nitrogen, the reaction mixture was continued with stirring at 65°C to react for 1 hour. At 0°C, the reaction mixture was quenched with saturated sodium thiosulfate solution (100 mL); the mixture was extracted with ethyl acetate twice; the organic phase was sequentially washed with water and saturated saline, dried over anhydrous sodium sulfate, and filtered. The filtrate was concentrated under reduced pressure; and the residue was purified by silica-gel column chromatography (eluent: petroleum ether/ethyl acetate, gradient: 3-10% ethyl acetate) to obtain the title compound (white solid, 2.6 g, yield: 61%). LC/MS (ESI) m/z: 316/318 $[M+H]^+$.

**Step 3: 4,5-dibromo-6-chloropyridin-3-amine**

Under the protection of nitrogen, 3,4-dibromo-2-chloro-5-nitropyridine (2.6 g, 8.2 mmol) was dissolved in ethanol (30 mL) and saturated ammonium chloride (5 mL); iron powder (1.0 g, 17.8 mmol) was added; and under the protection of nitrogen, the mixture was stirred at 80°C to react for 2 hours. The reaction mixture was filtered through diatomite; the filtrate was concentrated under reduced pressure; and the residue was purified by silica-gel column chromatography (eluent: petroleum ether/ethyl acetate, gradient: 3-20% ethyl acetate) to obtain the title compound (white solid, 1.9 g, yield: 80.7%). $^1H$ NMR (400 MHz, $CDCl_3$) $\delta$ 7.84 (s, 1H), 4.30 (s, 2H). LC/MS (ESI) m/z: 286 $[M+H]^+$.

**Step 4: N-(7-bromo-6-chlorothiazolo[4,5-c]pyridin-2-yl)benzamide hydrobromide**

At room temperature, benzoyl isothiocyanate (2.2 g, 13.5 mmol) was added portionwise to the solution of 4,5-dibromo-6-chloropyridin-3-amine (1.9 g, 6.6 mmol) in acetone (30 mL); and the reaction mixture was stirred at 50°C to react for 3 hours. The reaction mixture was cooled to room temperature, and filtered. The filter cake was washed with acetone, and then dried under reduced pressure to obtain the title compound (2.0 g, yellow solid, yield: 81.8%). LC/MS (ESI) m/z: 368/370 $[M+H]^+$.

**Step 5: N-(6-chloro-7-methylthiazolo[4,5-c]pyridin-2-yl)benzamide**

At room temperature and under the protection of nitrogen, [1,1'-bis(diphenylphosphino)ferrocen]palladium dichlorid (75 mg, 0.1 mol) was added to the solution of N-(7-bromo-6-chlorothiazolo[4,5-c]pyridin-2-yl)benzamide hydrobromide (500 mg, 1.4 mmol), trimethylboroxine (0.6 mL, 2.1 mol, 3.5 M tetrahydrofuran solution), and potassium carbonate (560 mg, 4.2 mol) in 1,4-dioxane (10 mL) and (2 mL); the mixture was subjected to nitrogen replacement three times; and under the protection of nitrogen, the mixture was stirred at 80°C to react for 3 hours. The reaction mixture was cooled to room temperature, and then filtered through diatomite; and the filter cake was washed with ethyl acetate twice. The filtrate was sequentially washed with water and saturated saline, dried over anhydrous sodium sulfate, and filtered; and the filtrate was concentrated under reduced pressure. The residue was purified by silica-gel column chromatography (eluent: petroleum ether/ethyl acetate, gradient: 3-30% ethyl acetate) to obtain the title compound (yellow solid, 170 mg, yield: 41.3%). LC/MS (ESI) m/z: 304 $[M+H]^+$.

**Step 6: 6-chloro-7-methylthiazolo[4,5-c]pyridin-2-amine**

At room temperature, N-(6-chloro-7-methylthiazolo[4,5-c]pyridin-2-yl)benzamide (170 mg, 0.55 mol) was added to 70% $H_2SO_4$ (10 mL); the reaction mixture was stirred at 110°C to react for 3 hours; and the reaction mixture was first clarified gradually, allowing for slow precipitation of solids. The reaction mixture was cooled to 0°C, and neutralized to neutrality with 6 M sodium hydroxide in water. The mixture was filtered; the filter cake was washed twice with water and methanol respectively, and dried under reduced pressure to obtain the title compound (90 mg, yellow solid, yield: 80.5%). LC/MS (ESI) m/z: 200 $[M+H]^+$.

**Step 7: 6-chloro-2-(2,5-dimethyl-1H-pyrrol-1-yl)-7-methylthiazolo[4,5-c]pyridine**

At room temperature, acetonyl acetone (0.5 mL, 5.0 mol) and p-toluenesulfonic acid hydrate (30 mg, 0.2 mol)

were sequentially added to the solution of 6-chloro-7-methylthiazolo[4,5-c]pyridin-2-amine (90 mg, 0.45 mol) in toluene (5 mL); and then, the mixture was stirred at 140°C to react for 5 hours. The reaction mixture was cooled to room temperature, and filtered; and the filter cake was washed with ethyl acetate twice. The filtrate was sequentially washed with sodium bicarbonate in water and saturated saline, dried over anhydrous sodium sulfate, and concentrated under reduced pressure. The residue was purified by silica-gel column chromatography (eluent: petroleum ether/ethyl acetate, gradient: 5-20% ethyl acetate) to obtain the title compound (yellow solid, 50 mg, yield: 39.9%). LC/MS (ESI) m/z: 278 $[M+H]^+$.

**Step 8: 6-(1,4-dimethyl-1H-1,2,3-triazol-5-yl)-2-(2,5-dimethyl-1H-pyrrol-1-yl)-7-methylthiazolo[4,5-c] pyridine**

At room temperature and under the protection of nitrogen, tetrakis(triphenylphosphine)palladium (15 mg, 0.1 mmol) was added to the solution of 6-chloro-2-(2,5-dimethyl-1H-pyrrol-1-yl)-7-methylthiazolo[4,5-c]pyridine (50 mg, 0.2 mol) and 1,4-dimethyl-5-(tributylstannyl)-1H-1,2,3-triazole (300 mg, 0.8 mol) in ditoluene (5 mL); and the mixture was subjected to nitrogen replacement three times; and under the protection of nitrogen, the mixture was stirred at 140°C to react for four hours. The mixture was concentrated under reduced pressure; and the residue was purified by silica-gel column chromatography (eluent: petroleum ether/ethyl acetate, gradient: 1-30% ethyl acetate) to obtain the title compound (yellow solid, 30 mg, yield: 49.2%). $^1H$ NMR (400 MHz, $CDCl_3$) $\delta$ 9.25 (s, 1H), 6.00 (s, 2H), 3.99 (s, 3H), 2.46 (s, 3H), 2.43 (s, 6H), 2.30 (s, 3H). LC/MS (ESI) m/z: 339.0 $[M+H]^+$.

**Step 9: 6-(1,4-dimethyl-1H-1,2,3-triazol-5-yl)-7-methylthiazolo[4,5-c]pyridin-2-amine**

At room temperature, 6-(1,4-dimethyl-1H-1,2,3-triazol-5-yl)-2-(2,5-dimethyl-1H-pyrrol-1-yl)-7-methylthiazolo [4,5-c]pyridine (30 mg, 0.1 mol) was dissolved in 2 N hydrochloric acid (5 mL); and the reaction mixture was stirred at 80°C to react for 2 hours. The mixture was concentrated under reduced pressure; the residue was washed with saturated sodium bicarbonate, and extracted with dichloromethane; the organic phase was dried over anhydrous sodium sulfate, and concentrated under reduced pressure to obtain the title compound (faint yellow solid, 20 mg, yield: 80%). LC/MS (ESI) m/z: 261 $[M+H]^+$.

**Step 10: 2'-chloro-N-(6-(1,4-dimethyl-1H-1,2,3-triazol-5-yl)-7-methylthiazolo[4,5-c]pyridin-2-yl)-5'-m ethoxy-6-methyl-[4,4'-bipyridine]-3-carboxamide**

At room temperature, 6-(1,4-dimethyl-1H-1,2,3-triazol-5-yl)-7-methylthiazolo[4,5-c]pyridin-2-amine (20 mg, 0.08 mol), 2'-chloro-5'-methoxy-6-methyl-[4,4'-bipyridine]-3-carboxylic acid (35 mg, 0.1 mol), and N-methylimidazole (250 mg, 3.0 m mol) were dissolved in acetonitrile (2 mL), and stirred at 70°C for 5 minutes; then, N,N,N',N'-tetramethylchlorformamidine hexafluorphosphate (65 mg, 0.24 mmol) was added to the reaction mixture, which was then basically clarified; and then the reaction mixture was stirred at 70°C to react for 3 hours, filtered, separated by pre-HPLC, and freeze-dried to obtain the title compound (0.72 mg, white solid, yield: 1.8%). $^1H$ NMR (400 MHz, DMSO-$d_6$) $\delta$ 13.37 (s, 1H), 9.07 (s, 1H), 8.89 (s, 1H), 8.18 (s, 1H), 7.61 (s, 1H), 7.49 (s, 1H), 3.85 (s, 3H), 3.62 (s, 3H), 2.62 (s, 3H), 2.36 (s, 3H), 2.14 (s, 3H). LC/MS (ESI) m/z: 521 $[M+H]^+$.

**Comparative Example 1:**

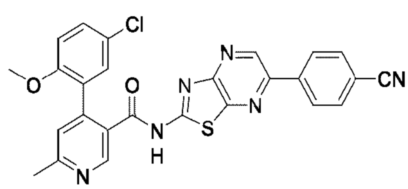

Comparative Example 1 was conducted according to the synthesis method in Example 23 of Patent WO2023134739.

**Comparative Example 2:**

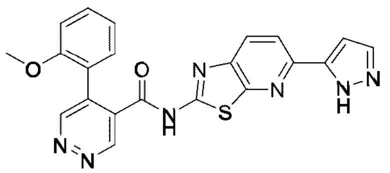

Comparative Example 2 was conducted according to the synthesis method in Example 47 of Patent WO2023134708.

## Effect Example

### Experimental Approaches:

### 1. Biochemical Activity Assay (ADP Glo Assay) of Pol θ Inhibitor

The N-terminus of the Pol θ protein contains a helicase domain, which has ATPase activity and can hydrolyze ATP into ADP. The ATPase activity of Pol θ protein and the inhibitory effect of small molecule compounds on the protein can be analyzed by ADP-Glo™ Kinase Assay (Promega, V9102). The specific assay method was as follows:

1) The helicase domain of the Pol θ protein was purified from insect protein expression system with a purity of >90%. ssDNA (5'-CCAGTGAATTGTTGCTCGGTACCTGCTAAC-3', Zhejiang Youkang Biotechnology Co., Ltd.) was ordered as the substrate for the Pol θ protein. Reaction buffer containing 10 mM DTT (Dithiothreitol, DTT), 20 mM $MgCl_2$, Tris-HCl pH 7.5 was prepared.
2) The 2× ssDNA-Pol θ premix was prepared. The reaction was conducted in a 384-well plate. A control group with only buffer is set, and the premix was added to the rest of the groups. Compounds were added with liquid dispenser (Thermo, Multidrop 8) and diluted at a start concentration of 10 μM and at a dilution ratio of 1/3; and a total of 9 points were set, with 2 replicates in each group.
3) The 2× ATP solution was prepared, and all wells were added with 2× ATP solution in equal volume, and allowed to stand at the room temperature for 60 min.
4) Following the instructions of Promega Kit, ADP-Glo Detection Reagent was added to the reaction system. Then the reaction was incubated at the room temperature for 60 min.
5) Following the instructions of Promega Kit, Kinase Detection Reagent was added to the reaction system. Then the reaction was incubated at room temperature for 60 min. Chemiluminescence signals were detected by a microplate reader (Thermo, VarioskanLUX). The reading interval for each well was set to 1000 ms.

Data analysis was conducted on the measurement results: the CV% of the control group should be less than 10%, and the z' value should be greater than 0.5. Data meeting the above quality control were forwarded to calculate the inhibition rate of the compound (inhibition rate (%) = 100 × (average value of control group - average value of experimental group)/(average value of control group - average value of blank control group). GraphPad Prism8 was used to perform nonlinear regression to fit the curves of the inhibition rates of the compounds to obtain $IC_{50}$ values.

### 2. Cell Viability Assay of Pol θ Inhibitor

The cell viability assay, i.e., the commonly used CTG assay, was conducted for the efficacy of the inhibitor *in vitro* using the CellTiter Glo reagent (Promega, G7573). The specific assay method was as follows:

1) DLD1 and DLD1$^{-/-}$ cells were cultured, digested with trypsin (0.025% Trypsin-EDTA, Hyclone) one day before the assays, and centrifuged at 1000 rpm for 3 min to collect. The cells were counted with a cell counter (Shanghai Mengwei Biomedical Technology Co., Ltd., SmartCell600A.SC1006), and seeded into 96-well white culture plates at an appropriate seeding density.
2) 24 hours after cell plating, dosing treatment was conducted, with the day denoted as Day 0. Compounds were added using liquid dispenser (Thermo, Multidrop 8). The plate was set as a 96-well plate and the start concentration was set to 30 μM. Serial dilution was conducted with the dilution ratio of 1/3, and a total of 9 points were set with 2 replicates set in each group. Cells were incubated in a 5% $CO_2$ incubator at 37°C.
3) On Day 3 and Day 7, the culture medium was changed, and the compounds were added according to Step 2.
4) On Day 10, the cell culture plates were taken out from incubator. Equal volume of CTG reagent (the CTG reagent needed to be restored in temperature for premixing in advance according to the reagent instructions) was added, and

gently and evenly mixed on a thermomixer (Hangzhou Allsheng Instrument Co., Ltd., MSC-100) for 10 min (speed: 300). Chemiluminescence detection was conducted using the microplate reader (Thermo, VarioskanLUX).

5) Data analysis was conduct on the measurement results: the CV% of the control group should be less than 20%, and the value z' should be greater than 0.5. Data satisfying the above quality control were used to calculate the cell viability: (inhibition rate (%) = 100 * (average value of control group - average value of experimental group)/(average value of control group - average value of blank control group). GraphPad Prism8 was used to perform nonlinear regression to fit the curves of the inhibition rates of the compounds to obtain $IC_{50}$ values.

**Data list:**

Biochemical assay:
++++≤50 nM, 50 nM < +++ ≤200 nM, 200 nM < ++ ≤500 nM, + >500 nM;
Cell viability assay:
++++≤ 100 nM, 100 nM< +++ ≤500 nM, 500 nM < ++ ≤1 μM, + >1 μM;
NT = not detected;

| Examples | Biochemical assay $IC_{50}$ (nM) | Cell viability assay $IC_{50}$ (nM) | Examples | Biochemical assay $IC_{50}$ (nM) | Cell viability assay $IC_{50}$ (nM) |
|---|---|---|---|---|---|
| 1 | ++++ | ++ | 59 | ++++ | ++++ |
| 2 | +++ | + | 60 | ++++ | +++ |
| 3 | ++++ | ++++ | 61 | ++++ | +++ |
| 4 | ++++ | ++ | 62 | ++++ | +++ |
| 5 | +++ | +++ | 63 | ++++ | + |
| 6 | +++ | ++ | 64 | ++++ | ++ |
| 7 | ++++ | ++++ | 65 | ++++ | ++++ |
| 8 | ++++ | ++++ | 66 | ++++ | +++ |
| 9 | ++++ | ++++ | 67 | ++++ | ++++ |
| 10 | ++++ | +++ | 68 | ++++ | + |
| 11 | ++++ | ++++ | 69 | ++++ | ++++ |
| 12 | ++++ | ++++ | 70 | ++++ | ++++ |
| 13 | ++++ | ++++ | 71 | ++++ | +++ |
| 14 | ++++ | ++++ | 72 | ++++ | +++ |
| 15 | ++++ | ++++ | 73 | ++++ | +++ |
| 16 | ++++ | + | 74 | ++++ | +++ |
| 17 | ++++ | +++ | 75 | ++++ | ++ |
| 18 | ++++ | ++++ | 76 | ++++ | + |
| 19 | +++ | +++ | 77 | ++++ | ++++ |
| 20 | ++++ | ++++ | 78 | ++++ | ++++ |
| 21 | ++++ | ++++ | 79 | ++++ | ++++ |
| 22 | +++ | + | 80 | ++++ | ++++ |
| 23 | ++++ | ++++ | 81 | ++++ | ++++ |
| 24 | ++++ | +++ | 82 | ++++ | +++ |
| 25 | ++++ | +++ | 83 | ++++ | ++++ |
| 26 | ++++ | +++ | 84 | ++++ | ++++ |
| 27 | ++++ | ++ | 85 | ++++ | ++++ |

(continued)

| Examples | Biochemical assay $IC_{50}$ (nM) | Cell viability assay $IC_{50}$ (nM) | Examples | Biochemical assay $IC_{50}$ (nM) | Cell viability assay $IC_{50}$ (nM) |
|---|---|---|---|---|---|
| 28 | ++++ | +++ | 86 | ++++ | ++++ |
| 29 | ++++ | +++ | 87 | ++++ | ++++ |
| 30 | ++++ | ++++ | 88 | ++++ | ++++ |
| 31 | +++ | + | 89 | ++++ | +++ |
| 32 | +++ | + | 90 | + | + |
| 33 | ++++ | +++ | 91 | ++++ | ++++ |
| 34 | ++++ | + | 92 | +++ | +++ |
| 35 | ++ | + | 93 | ++++ | +++ |
| 36 | ++ | ++ | 94 | ++++ | ++ |
| 37 | ++++ | ++++ | 95 | ++++ | +++ |
| 38 | ++++ | +++ | 96 | ++++ | + |
| 39 | ++++ | +++ | 97 | ++++ | + |
| 40 | +++ | + | 98 | ++++ | ++++ |
| 41 | + | + | 99 | ++++ | +++ |
| 42 | ++ | + | 100 | ++++ | +++ |
| 43 | +++ | +++ | 101 | ++++ | +++ |
| 44 | ++++ | ++++ | 102 | ++++ | ++++ |
| 45 | ++++ | +++ | 103 | ++++ | ++++ |
| 46 | ++++ | ++++ | 104 | ++++ | ++++ |
| 47 | +++ | ++ | 105 | ++++ | +++ |
| 48 | ++++ | +++ | 106 | ++++ | +++ |
| 49 | ++++ | ++++ | 107 | ++++ | ++++ |
| 50 | ++++ | ++ | 108 | ++++ | ++++ |
| 51 | ++++ | ++++ | 109 | ++++ | ++++ |
| 52 | ++++ | ++++ | 110 | ++++ | ++++ |
| 53 | ++++ | ++++ | 111 | ++++ | ++++ |
| 54 | ++++ | ++ | 112 | ++++ | ++++ |
| 55 | ++++ | +++ | 113 | ++++ | ++++ |
| 56 | ++ | + | 114 | ++++ | ++++ |
| 57 | ++++ | +++ | 115 | ++++ | ++++ |
| 58 | ++++ | + | | | |

Specific data of biochemical assays and cell viability assays:

| Exam ples | Biochemical assay $IC_{50}$ (nM) | Cell viability assay $IC_{50}$ (nM) | Examp les | Biochemical assay $IC_{50}$ (nM) | Cell viability assay $IC_{50}$ (nM) |
|---|---|---|---|---|---|
| **1** | 7.07 | 131 | **119** | 5.46 | 10.7 |

(continued)

| Exam ples | Biochemical assay $IC_{50}$ (nM) | Cell viability assay $IC_{50}$ (nM) | Examp les | Biochemical assay $IC_{50}$ (nM) | Cell viability assay $IC_{50}$ (nM) |
|---|---|---|---|---|---|
| **2** | 118 | 3162 | **120** | 5.93 | 14.2 |
| **3** | 2.51 | 13.6 | **121** | 51.9 | 2512 |
| **4** | 19.1 | 736 | **122** | 145 | 2712 |
| **5** | 81.5 | 153 | **123** | 10.5 | 24.2 |
| **6** | 193 | 794 | **124** | 163 | 584 |
| **7** | 26.4 | 73.6 | **125** | 10 | 3.98 |
| **8** | 1.09 | 24.2 | **126** | 7.35 | 9.26 |
| **9** | 1.96 | 3.83 | **127** | 3.52 | 15.8 |
| **10** | 33.1 | 446 | **128** | 9.69 | 39.8 |
| **11** | 3.11 | 11.2 | **129** | 8.06 | 9.68 |
| **12** | 2.41 | 15.3 | **130** | 6.91 | 11 |
| **13** | 2.89 | 5.84 | **131** | 149 | 736 |
| **14** | 2.74 | 31.6 | **132** | 163 | 158 |
| **15** | 3.55 | 14.7 | **133** | 6.92 | 26.1 |
| **16** | 13.5 | 2326 | **134** | 4.11 | 5.41 |
| **17** | 8.57 | 103 | **135** | 5.41 | 19.2 |
| **18** | 5.69 | 44.7 | **136** | 25.8 | 121 |
| **19** | 72.1 | 261 | **137** | 25.1 | 171 |
| **20** | 2.95 | 7.64 | **138** | 7.58 | 26.1 |
| **21** | 3.48 | 76.4 | **139** | 19.1 | 121 |
| **22** | 115 | 1585 | **140** | 15.8 | 68.1 |
| **23** | 2.84 | 15.3 | **141** | 8.23 | 21.5 |
| **24** | 15.2 | 192 | **142** | 4.49 | 104 |
| **25** | 3.94 | 138 | **143** | 23.6 | 43 |
| **26** | 5.69 | 158 | **144** | 36.7 | 2224 |
| **27** | 10.3 | 736 | **145** | 46.4 | NT |
| **28** | 12.1 | 178 | **146** | 49.4 | NT |
| **29** | 12.9 | 131 | **147** | 17.6 | 82.5 |
| **30** | 1.62 | 19.2 | **148** | 4.93 | 4.13 |
| **31** | 66.4 | NT | **149** | 2.29 | 3.54 |
| **32** | 196 | 7080 | **150** | 5.58 | 21.5 |
| **33** | 24.9 | 171 | **151** | 8.57 | 3.98 |
| **34** | 48.9 | 1995 | **152** | 3.02 | 17.2 |
| **35** | 251 | 3286 | **153** | 24.4 | 545 |
| **36** | 236 | 584 | **154** | 2.36 | 17.8 |
| **37** | 4.36 | 44.7 | **155** | 5.75 | 19.2 |
| **38** | 17.3 | 482 | **156** | 2.75 | 5.62 |
| **39** | 8.57 | 108 | **157** | 5.08 | 32.9 |

(continued)

| Exam ples | Biochemical assay $IC_{50}$ (nM) | Cell viability assay $IC_{50}$ (nM) | Examp les | Biochemical assay $IC_{50}$ (nM) | Cell viability assay $IC_{50}$ (nM) |
|---|---|---|---|---|---|
| **40** | 14.9 | 4467 | **158** | 6.31 | 4.64 |
| **41** | 1243 | NT | **159** | 2.75 | 1166 |
| **42** | 200 | NT | **160** | 5.75 | 82.5 |
| **43** | 51.9 | 293 | **161** | 3.41 | 121 |
| **44** | 5.81 | 9.26 | **162** | 63.1 | 233 |
| **45** | 25.4 | 141 | **163** | 2.51 | 3.41 |
| **46** | 8.31 | 60.7 | **164** | 8.32 | NT |
| **47** | 53.0 | 631 | **165** | 16.9 | 446 |
| **48** | 26.4 | 165 | **166** | 25.1 | 68.1 |
| **49** | 7.58 | 54.1 | **167** | 3.74 | 5.41 |
| **50** | 8.39 | 541 | **168** | 1.91 | 4.46 |
| **51** | 9.50 | 85.8 | **169** | 8.84 | 89.1 |
| **52** | 2.62 | 20.7 | **170** | 7.13 | 825 |
| **53** | 1.96 | 9.38 | **171** | 4.93 | 13.1 |
| **54** | 40.6 | 794 | **172** | 2.84 | NT |
| **55** | 28.7 | 112 | **173** | 2.93 | NT |
| **56** | 222 | 9623 | **174** | 2.93 | NT |
| **57** | 2.51 | 126 | **175** | 1.84 | 10 |
| **58** | 11 | 3415 | **176** | 4.64 | 4.29 |
| **59** | 3.63 | 82.5 | **177** | 13.6 | 121 |
| **60** | 9.89 | 501 | **178** | 1.63 | 35.5 |
| **61** | 10.5 | 141 | **179** | 2.43 | 562 |
| **62** | 16.9 | 631 | **180** | 1.63 | 5.62 |
| **63** | 5.58 | 1711 | **181** | 6.71 | NT |
| **64** | 25.4 | 681 | **182** | 2.08 | 39.8 |
| **65** | 2.72 | 42.2 | **183** | 3.41 | 1259 |
| **66** | 4.98 | 192 | **184** | 15.2 | 482 |
| **67** | 6.18 | 46.4 | **185** | 8.94 | 35.9 |
| **68** | 1.39 | 1122 | **186** | 5.58 | 1525 |
| **69** | 4.28 | 92.6 | **187** | 8.75 | 271 |
| **70** | 2.73 | 14.2 | **188** | 36.7 | <1.52 |
| **71** | 4.46 | 142 | **189** | 8.06 | 12.9 |
| **72** | 3.15 | 190 | **190** | 5.08 | 3.28 |
| **73** | 1.67 | 209 | **191** | 29.3 | 521 |
| **74** | 6.44 | 383 | **192** | 103 | 4823 |
| **75** | 2.51 | 681 | **193** | 10.6 | 92.6 |
| **76** | 5.14 | NT | **194** | 22.9 | 65.6 |
| **77** | 3.94 | 7.36 | **195** | 6.18 | 34.2 |

(continued)

| Exam ples | Biochemical assay $IC_{50}$ (nM) | Cell viability assay $IC_{50}$ (nM) | Examp les | Biochemical assay $IC_{50}$ (nM) | Cell viability assay $IC_{50}$ (nM) |
|---|---|---|---|---|---|
| **78** | 2.89 | 8.21 | **196** | 11.7 | 3.83 |
| **79** | 3.35 | 92.6 | **197** | 8.57 | NT |
| **80** | 9.12 | 30.3 | **198** | 4.23 | 23.3 |
| **81** | 2.51 | 30.4 | **199** | 7.82 | 31.6 |
| **82** | 6.71 | 104 | **200** | 6.51 | 7.07 |
| **83** | 3.21 | 29.3 | **201** | 5.24 | 224 |
| **84** | 1.81 | 2.42 | **202** | 6.11 | 38.8 |
| **85** | 1.81 | 44.7 | **203** | NT | NT |
| **86** | 2.67 | 23.3 | **204** | 8.57 | 541 |
| **87** | 1.91 | 6.81 | **205** | 5.24 | 20.0 |
| **88** | 1.15 | 52.1 | **206** | 2.36 | 13.6 |
| **89** | 36.7 | 171 | **207** | 3.41 | 8.57 |
| **90** | NT | NT | **208** | 36.3 | 141 |
| **91** | 1.88 | 11.7 | **209** | 1.93 | 6.82 |
| **92** | 59.9 | 316 | **210** | 2.43 | <1.52 |
| **93** | 5.93 | 355 | **211** | 2.51 | 3.28 |
| **94** | 6.98 | 631 | **212** | 3.48 | 2.23 |
| **95** | 8.94 | 383 | **213** | 3.63 | 4.13 |
| **96** | 5.58 | 7644 | **214** | 6.71 | 27 |
| **97** | 26.4 | 1212 | **215** | 3.11 | 2.92 |
| **98** | 2.96 | 10.6 | **216** | 3.31 | <1.52 |
| **99** | 14.3 | 138 | **217** | 3.63 | <1.52 |
| **100** | 2.95 | 207 | **218** | 31.1 | 656 |
| **101** | 3.78 | 389 | **219** | 3.31 | 63.1 |
| **102** | 8.06 | 178 | **220** | 2.51 | 22.2 |
| **103** | 2.67 | 22.4 | **221** | 4.78 | 1848 |
| **104** | 1.56 | 5.62 | **222** | 4.36 | 9.62 |
| **105** | 6.99 | 152 | **223** | 2.51 | 10.0 |
| **106** | 6.18 | 450 | **224** | 3.98 | 3.16 |
| **107** | 10.1 | 207 | **225** | 21.1 | 56.2 |
| **108** | 1.88 | 8.79 | **226** | 4.02 | 8.46 |
| **109** | 6.71 | 147 | **227** | 20.7 | 112 |
| **110** | 2.61 | 2.41 | **228** | 111 | NT |
| **111** | 3.78 | 13.1 | **229** | 36.7 | NT |
| **112** | 10 | 24.2 | **230** | 2.84 | NT |
| **113** | 4.36 | 4.82 | **231** | 3.08 | NT |
| **114** | 3.98 | 1.84 | **232** | 2.51 | NT |
| **115** | 3.21 | 2.61 | **233** | 8.58 | NT |

(continued)

| Exam ples | Biochemical assay $IC_{50}$ (nM) | Cell viability assay $IC_{50}$ (nM) | Examp les | Biochemical assay $IC_{50}$ (nM) | Cell viability assay $IC_{50}$ (nM) |
|---|---|---|---|---|---|
| **116** | 10.3 | 36.9 | **Comp arativ e Exam ple 1** | 6.91 | NT |
| **117** | 7.28 | 799 | **Comp arativ e Exam ple 2** | 307 | NT |
| **118** | 4.02 | 23 | | | |

**3. Solubility Assay**

1) Prepare 0.1 M $Na_2PO_4$ buffer (pH 7.4):

11 g of $Na_2HPO_4$ (FW: 141.96) and 3.5 g of $NaH_2PO_4$_$2H_2O$ (FW: 156.03) were added to 1L of Mili-Q water, with pH adjusted to 7.4 using phosphoric acid or sodium hydroxide.

2) 10 μL of the compound to be tested (concentration: 10 mM in DMSO) was added to 990 μL of $Na_2PO_4$ buffer (final DMSO concentration: 1%) prepared in Step 1).

3) The sample tube was shaken at room temperature for 2 hours (1000 rpm/min).

4) Prepare the calibration curve:

a) Prepare 300 μM spiking solution (SS):

6 μL (10 mM in DMSO) of the stock solution of the compound to be tested was added to 194 μL of MeOH/ACN (4:1); and

b) Draw the standard curve.

| | MeOH:ACN(4:1)mixture (μL) | STD ID | STD Conc. (μM) |
|---|---|---|---|
| 100 μL SS (300 μM) | 400 | STD 8 | 60 |
| 100 μL STD 8 | 200 | STD 7 | 20 |
| 100 μL STD 7 | 100 | STD 6 | 10 |
| 100 μL STD 6 | 150 | STD 5 | 4 |
| 100 μL STD 5 | 400 | STD 4 | 0.8 |
| 100 μL STD 4 | 300 | STD 3 | 0.2 |
| 100 μL STD 3 | 100 | STD 2 | 0.1 |
| 100 μL STD 2 | 400 | STD 1 | 0.02 |

5) The samples were centrifuged (10 min, 12,000 rpm) to precipitate insoluble particles. The supernatant was filtered with a 0.22 μm filter membrane, and then transferred to a new centrifuge tube.

6) The supernatant was diluted 10-fold with 100 mM buffer.

The supernatant (10 μL) was added to the buffer (100 mM, 90 μL) and diluted 10-fold.

7) The sample was prepared under LC-MS/MS (API 4000) detection.

10 μL of the sample (10-fold dilution) and a standard curve sample were added to 400 μL of (MeOH: ACN=1:1) solution, and detected on the instrument, and the solubility value was calculated according to the standard curve.

**Experimental Results:**

| **Examples** | Solubility (μM) | **Examples** | Solubility (μM) |
|---|---|---|---|
| **11** | 76.4 | **175** | 4.61 |
| **46** | 61.5 | **180** | 91.1 |
| **70** | 70.6 | **207** | 58.7 |
| **87** | 79.7 | **216** | 61.8 |

(continued)

| Examples | Solubility (μM) | Examples | Solubility (μM) |
|---|---|---|---|
| **155** | 7.81 | **217** | 82.6 |
| **172** | 63.7 | **Comparative Example 1** | 2.35 |

**4. Metabolic Stability of Mouse Liver Microsome Assay**

**1) Preparation of experimental reagents**

**a) Phosphate buffer solution (PBS):** 100 mM, pH=7.4±0.1.

73.21 g of dipotassium phosphate trihydrate $K_2HPO_4 \cdot 3H_2O$ (MW=228.22) and 10.78 g of potassium dihydrogen phosphate $KH_2PO_4$ (MW=136.09) were dissolved in 4000 mL of pure water, with pH adjusted to 7.4 ± 0.1 using phosphoric acid or potassium hydroxide.

**b) Microsome working solution:** 1.0 mg/mL.

The purchased mouse liver microsomes (IPHASE, Cat. No.: 0121E1.01, Lot. No.: 22F001) were of 20 mg/mL (10 mg, 0.5 mL), with 225 μL added to 4275 μL of PBS solution to prepare 1.0 mg/mL microsome working solution.

**c) Working solution for NADPH regeneration system:** 2 mM NADPH solution and 6 mM magnesium chloride mixture

I, 6.8 mg of NADPH (MW=833.35, 98%) powder was weighed and added to 2000 μL of the PBS solution, to produce NADPH stock solution with a solubility of 40 mM.

II, 24.64 mg of magnesium chloride hexahydrate (MV=203.3, 99%) solids were weighed, and added to and dissolved in 10 mL of the PBS solution, to prepare magnesium chloride stock solution with a concentration of 12 mM. (Note whether the precipitation occurred)

III, 200 μL of the 40 mM NADPH stock solution, 2.0 mL of the 12 mM magnesium chloride stock solution, and 1.80 mL of the PBS solution were used to prepare the working solution of the NADPH regeneration system, containing the 2 mM NADPH solution and the 6 mM magnesium chloride mixture.

**d) Stop solution:** containing 200 ng/mL tolbutamide acetonitrile solution.

I, 6.006 mg of tolbutamide (MV=270.35, 99.9%) solids were weighed, and 30 mL of acetonitrile was added to prepare the 200 μg/mL of tolbutamide stock solution.

II, 500 mL of acetonitrile was added to 500 μL of the 200 μg/mL tolbutamide stock solution to obtain the tolbutamide acetonitrile solution with a concentration of 200 ng/mL.

**e) Test sample/reference working solution:** 100 μM test sample/reference working solution

I, 1 mL of 1.0 mg/mL testosterone (MV=288.42) solution was purchased, and 155.8 μL of ACN was added to prepare a testosterone stock solution with a concentration of 3 mM.

II, 1450 μL of acetonitrile was added to 50 μL of the 3 mM testosterone stock solution to prepare the 100 μM testosterone working solution.

III, 6.98 mg of propafenone hydrochloride was weighed, and 1.8433 mL of acetonitrile was added to dissolve the propafenone hydrochloride; and 5.67 mg of diclofenac sodium was weighed, and 1.783 mL of acetonitrile was added to dissolve the diclofenac sodium. 10 mM propafenone stock solution and 10 mM diclofenac stock solution were prepared respectively.

IV, 10 μL of the propafenone stock solution and 10 mL of the diclofenac stock solution were each added with 990 μL of acetonitrile to prepare 100 μM propafenone working solution and 100 μM diclofenac working solutions.

**2 Experimental Procedures**

a) 8 incubation plates were prepared, named T0, T5, T15, T30, T45, T60, Blank60 and NCF60, respectively.

b) 100 μL of microsome working solution was added to each of T0, T5, T15, T30, T45, T60, Blank60 and NCF60; 2 μL of the test (reference) working solution was added to each of T0, T5, T15, T30, T45, T60 and NCF60; and 2 μL of acetonitrile was added to Blank60.

c) T5, T15, T30, T45, T60, and Blank60 were pre-incubated in a 37°C water bath for 10 minutes.

d) 600 μL of the stop solution was added to the sample T0 to stop the reaction; and 98 μL of phosphate buffer solution (PBS) was added to NCF60, and NCF60 samples without the NADPH regeneration system were taken as negative control.

e) 98 μL of the working solution of the NADPH regeneration system was supplemented to the sample T0 that is stopped in the previous step; and after pre-incubation, 98 μL of the working solution of the NADPH regeneration system was added to each of T5, T15, T30, T45, T60, and Blank60 to initiate the reaction. Therefore, the incubation system was 200 μL in total, the dose concentration of the test sample (reference substance) was 1 μM, the microsome concentration was 0.5 mg/mL, and the concentration of the NADPH regeneration system was 1 mM.

f) The above samples T5, T15, T30, T45, T60, Blank60 and NCF60 were each incubated in a water bath at 37°C for the corresponding time: 5 min, 15 min, 30 min, 45 min, 60 min, 60 min, and 60 min.

g) After incubation for the corresponding time, 600 μL of the stop solution was added to the samples T5, T15, T30, T45, T60, Blank60 and NCF60 to stop the reaction.

h) After the reaction was stopped, the samples T0, T5, T15, T30, T45, T60, Blank60 and NCF60 were shaken well and then centrifuged for 5 min at 14000 rpm and 4°C. 100 μL of the supernatant was analyzed by LC-MS/MS in each case.

**3. Data Processing:**

(1) The formula for calculating the half-life was as follows:

$$T_{1/2} = \frac{Ln2}{-k_e} = \frac{0.693}{-k_e}$$

$$CL_{int(mic)} = \frac{0.693}{In\ vitro\ T_{1/2}} \bullet \frac{1}{mg/mL\ microsomal\ \ protein\ in\ reaction\ system} \qquad \mu L/min/mg$$

$$CL_{int(liver)} = CL_{int(mic)} \bullet \frac{mg\ microsomes}{g\ liver} \bullet \frac{g\ liver}{kg\ body\ weight} \qquad mL/min/kg$$

$$CL_{int(hep)} = \frac{CL_{int(liver)} \bullet \text{Hepatic Blood Flow}}{CL_{int(liver)} + \text{Hepatic Blood Flow}} \qquad mL/min/kg$$

**Experimental Results:**

| **Examples** | Half-life ($T_{1/2}$, min) | **Examples** | Half-life ($T_{1/2}$, min) |
|---|---|---|---|
| **11** | 225 | **175** | 630 |
| **46** | 474 | **180** | ∞ |
| **70** | 533 | **207** | 866 |
| **87** | 866 | **216** | 1733 |
| **155** | 693 | **217** | ∞ |
| **172** | ∞ | | |

In the table above, "∞" indicates infinity.

### 5. Mouse PK Assay

The objective of this experiment was to study the pharmacokinetics of the test compound in ICR mice (male, 18-22 g, 4-6 weeks old, Beijing Vital River Laboratory Animal Technology Co., Ltd.) after oral administration. The specific experimental procedures were as follows:

1) The compound was dissolved in 5% DMSO + 40% PEG + 20% (10% TPGS) in water + 35% water at a concentration of 0.5 mg/mL, and then administered orally (5 mL/Kg); and
2) Blood samples were collected, with 0.03 mL collected at 0.25 h, 0.5 h, 1 h, 2 h, 4 h, 8 h, and 24 h, and EDTA-K2 was added. CD1 mice: Approximately 0.03 mL of blood was collected at each time point. The blood was centrifuged for 5 min at 4000 g in a centrifuge at 4°C to collect plasma. Plasma samples were aliquoted into clean polyethylene microcentrifuge tubes and then stored in a freezer at -75 ± 15°C before analysis; and
3) Samples were analyzed, and the concentration of the compound in each plasma sample was analyzed by LC-MS/MS. For plasma measurements, pharmacokinetic parameters were calculated using WinNonlin (PhoenixTM, version 8.3) or other similar software.

**Experimental Results:**

| **Examples** | **Administration dose (mg/kg)** | **Half-life (hr)** | **Mean residence time (hr)** | **Time to peak (hr)** | **Peak concentration (ng/mL)** | **Area under curve (0-t; hr * ng/mL)** | **Bioavailability (%)** |
|---|---|---|---|---|---|---|---|
| **11** | 25 | 2.19 | 3.15 | 0.83 | 7407 | 26324 | -- |
| **46** | 25 | 1.59 | 4.13 | 2.0 | 7217 | 46041 | 127 |
| **70** | 100 | 2.91 | 2.2 | 0.5 | 22767 | 60434 | -- |
| **87** | 100 | 2.12 | 3.45 | 0.25 | 74833 | 300735 | -- |
| **155** | 30 | 2.26 | 4.56 | 1.0 | 43083 | 321917 | 513 |
| **172** | 30 | 5.10 | 5.91 | 0.25 | 43555 | 429051 | 206 |
| **175** | 30 | 2.68 | 4.58 | 1.67 | 42933 | 293209 | 151 |
| **180** | 30 | 1.75 | 2.88 | 1.08 | 44133 | 176788 | -- |
| **207** | 30 | 1.97 | 5.13 | 1.67 | 52444 | 581387 | 311 |
| **216** | 30 | 2.20 | 3.38 | 0.75 | 89917 | 385297 | 178 |
| **217** | 30 | 2.16 | 3.95 | 0.67 | 156130 | 852390 | 259 |

## Claims

1. A compound of formula I, a pharmaceutically acceptable salt thereof, or an isotopic compound thereof,

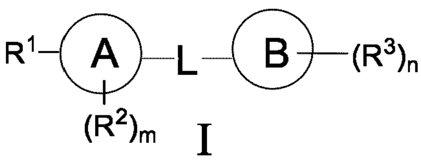

I

wherein:

m is 0, 1, 2, or 3; n is 1, 2, or 3;
L is

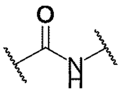, 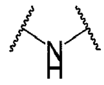, 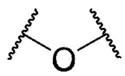, -CO- or 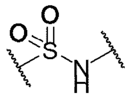;

ring A is a 6-12 membered aromatic ring or a 5-12 membered heteroaromatic ring; the heteroatom in said 5-12 membered heteroaromatic ring is selected from the group consisting of N, O, and S, and the number of heteroatom(s) is 1, 2, or 3;

$R^1$ is a 6-12 membered aryl unsubstituted or substituted by one or more $R^{1-1}$, or 5-12 membered heteroaryl unsubstituted or substituted by one or more $R^{1-2}$; the heteroatom in said 5-12 membered heteroaryl is selected from the group consisting of N, O, and S, and the number of heteroatom(s) is 1, 2, or 3;

$R^{1-1}$ and $R^{1-2}$ are each independently selected from the group consisting of deuterium, halogen, cyano, amino, hydroxyl, $C_1$-$C_6$ alkyl unsubstituted or substituted by one or more $R^{1-1-1}$, $C_1$-$C_6$ alkoxy unsubstituted or substituted by one or more $R^{1-1-2}$, $C_2$-$C_6$ alkenyl unsubstituted or substituted by one or more $R^{1-1-3}$, $C_2$-$C_6$ alkynyl unsubstituted or substituted by one or more $R^{1-1-4}$, 3-6 membered cycloalkyl unsubstituted or substituted by one or more $R^{1-1-5}$, 4-6 membered heterocyclyl unsubstituted or substituted by one or more $R^{1-1-6}$, -COOR$^{1-1-7}$, -C(O)R$^{1-1-8}$, -C(O)NR$^{1-1-9}$R$^{1-1-9}$

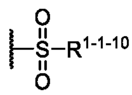, and 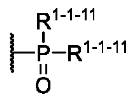

; the heteroatom in said 4-6 membered heterocyclyl is selected from the group consisting of N, O, and S, and the number of heteroatom(s) is 1, 2, or 3;

$R^{1-1-1}$, $R^{1-1-2}$, $R^{1-1-3}$ , $R^{1-1-4}$ , $R^{1-1-5}$ and $R^{1-1-6}$ are each independently selected from the group consisting of deuterium, hydroxyl, cyano and halogen;

$R^{1-1-7}$, $R^{1-1-8}$ and $R^{1-1-9}$ are each independently selected from hydrogen or $C_1$-$C_6$ alkyl;

$R^{1-1-10}$ and $R^{1-1-11}$ are each independently selected from hydroxyl or $C_1$-$C_6$ alkyl;

$R^2$ is each independently selected from the group consisting of

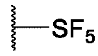,

deuterium, halogen, cyano, amino, hydroxyl, $C_1$-$C_6$ alkyl unsubstituted or substituted by one or more $R^{2-1}$, $C_1$-$C_6$ alkoxy unsubstituted or substituted by one or more $R^{2-2}$, $C_2$-$C_6$ alkenyl unsubstituted or substituted by one or more $R^{2-3}$, $C_2$-$C_6$ alkynyl unsubstituted or substituted by one or more $R^{2-4}$, 3-6 membered cycloalkyl unsubstituted or substituted by one or more $R^{2-5}$ and 4-6 membered heterocyclyl unsubstituted or substituted by one or more $R^{2-6}$; the heteroatom in said 4-6 membered heterocyclyl is selected from the group consisting of N, O, and S, and the number of heteroatom(s) is 1, 2, or 3;

$R^{2-1}$, $R^{2-2}$, $R^{2-3}$, $R^{2-4}$, $R^{2-5}$ and $R^{2-6}$ are each independently selected from the group consisting of deuterium, hydroxyl, cyano and halogen;

the ring B is a 5-20 membered fused heteroaromatic ring unsubstituted or substituted by one or more $R^{b-1}$;

the heteroatom in said 5-20 membered fused heteroaromatic ring is selected from the group consisting of N, O, and S, and the number of heteroatom(s) is 1, 2, 3, 4, 5, 6, 7 or 8, a single heteroaromatic ring in said 5-20 membered fused heteroaromatic ring is a 5-7 membered heteroaromatic ring, and the number of rings in said 5-20 membered fused heteroaromatic ring is 2, 3 or 4;

$R^{b-1}$ is each independently selected from the group consisting of deuterium, oxo(=O), halogen, cyano, hydroxyl, $C_1$-$C_6$ alkyl unsubstituted or substituted by one or more $R^{b-1-1}$, $C_1$-$C_6$ alkoxy unsubstituted or substituted by one or more $R^{b-1-2}$, -NR$^{b-1-3}$R$^{b-1-3}$, -C(O)NR$^{b-1-4}$R$^{b-1-4}$, -COOR$^{b-1-5}$,

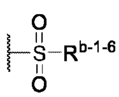

and

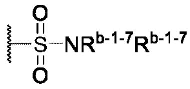
;

$R^{b-1-1}$ and $R^{b-1-2}$ are each independently selected from the group consisting of hydroxyl, halogen and $-NR^{b-1-1-1}R^{b-1-1-1}$;
$R^{b-1-3}$, $R^{b-1-4}$, $R^{b-1-5}$ and $R^{b-1-7}$ are each independently selected from hydrogen or $C_1$-$C_6$ alkyl;
$R^{b-1-6}$ is each independently selected from hydroxyl or $C_1$-$C_6$ alkyl;
$R^{b-1-1-1}$ is each independently selected from the group consisting of hydrogen, $C_1$-$C_6$ alkyl and $-C(O)R^{b-1-1-1-1}$;
$R^{b-1-1-1-1}$ is $C_1$-$C_6$ alkyl;
$R^3$ is hydrogen,

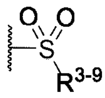

, 3-12 membered cycloalkyl unsubstituted or substituted by one or more $R^{3-1}$, 4-12 membered heterocyclyl unsubstituted or substituted by one or more $R^{3-2}$, 4-12 membered cycloalkenyl unsubstituted or substituted by one or more $R^{3-3}$, 4-12 membered heterocycloalkenyl unsubstituted or substituted by one or more $R^{3-4}$, 6-10 membered aryl unsubstituted or substituted by one or more $R^{3-5}$, 5-10 membered heteroaryl unsubstituted or substituted by one or more $R^{3-6}$, $-C(O)R^4$, $-NH(CH_2)_pR^5$, $-O(CH_2)_qR^6$, $C_1$-$C_6$ alkyl unsubstituted or substituted by one or more $R^{3-7}$, or $-OR^7$ unsubstituted or substituted by one or more $R^{3-8}$; the heteroatom in each of said 4-12 membered heterocyclyl, 4-12 membered heterocycloalkenyl and 5-10 membered heteroaryl is selected from the group consisting of N, O, and S, and the number of heteroatom(s) is 1, 2, 3 or 4;
p is 0, 1, 2, or 3;
q is 1, 2, or 3;
$R^{3-1}$, $R^{3-2}$, $R^{3-3}$, $R^{3-4}$, $R^{3-7}$ and $R^{3-8}$ are each independently selected from the group consisting of deuterium, hydroxyl, halogen, cyano, amino, $-NHC(O)R^{3-2-1}$, $C_1$-$C_6$ alkyl unsubstituted or substituted by one or more $R^{3-2-2}$, $C_1$-$C_6$ alkoxy, $C_2$-$C_6$ alkenyl, $C_2$-$C_6$ alkynyl, oxo(=O), $-C(O)NH_2$, 3-6 membered cycloalkyl unsubstituted or substituted by one or more $R^{3-2-3}$, 4-6 membered heterocyclyl, $-S(O)_2R^{3-4-1}$, $-NR^{3-4-2}R^{3-4-2}$,

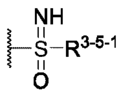 and 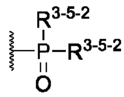;

the heteroatom in said 4-6 membered heterocyclyl selected from the group consisting of N, O, and S, and the number of heteroatom(s) is 1 or 2;
$R^{3-5}$ and $R^{3-6}$ are each independently selected from the group consisting of deuterium,

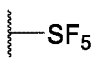,

hydroxyl, halogen, cyano, amino, $-NHC(O)R^{3-2-1}$, $C_1$-$C_6$ alkyl unsubstituted or substituted by one or more $R^{3-2-2}$, $C_2$-$C_6$ alkenyl, $C_2$-$C_6$ alkynyl, $-C(O)NH_2$, 3-6 membered cycloalkyl unsubstituted or substituted by one or more $R^{3-2-3}$, 4-6 membered heterocyclyl unsubstituted or substituted by one or more $R^{3-6-1}$, $-S(O)_2R^{3-4-1}$, $-NR^{3-4-2}R^{3-4-2}$,

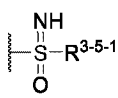 and 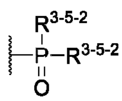;

the heteroatom in said 4-6 membered heterocyclyl is one or more selected from N, O, and S, and the number of heteroatom(s) is 1 or 2;
$R^{3-2-1}$ and $R^{3-4-2}$ are each independently selected from the group consisting of hydrogen, $-S(O)_2R^{3-4-1}$, 3-6 membered cycloalkyl unsubstituted or substituted by one or more $R^{3-2-1-1}$, and $C_1$-$C_6$ alkyl;
$R^{3-2-1-1}$ is each independently selected from $C_1$-$C_6$ alkyl;
$R^{3-2-3}$ and $R^{3-5-1}$ are each independently selected from hydroxyl or $C_1$-$C_6$ alkyl;

$R^{3-5-2}$ is each independently selected from the group consisting of hydroxyl, $C_1$-$C_6$ alkyl, 1-10 membered heteroalkyl, and two $R^{3-5-2}$s form 5-10 membered heterocyclyl; the heteroatom in said 1-10 membered heteroalkyl comprises, in addition to phosphorus, selected from the group consisting of N, O, and S, and the number of heteroatom(s) is 1, 2, 3 or 4; the heteroatom in said 5-10 membered heterocyclyl comprises, in addition to phosphorus, selected from the group consisting of N, O, and S, and the number of heteroatom(s) is 1, 2, 3 or 4;
$R^3$ is each independently selected from $C_1$-$C_6$ alkyl;
$R^{3-2-2}$ is each independently selected from the group consisting of deuterium, hydroxyl and halogen (for example, $R^{3-2-2}$ is each independently selected from hydroxyl or halogen);
$R^{3-4-1}$ is hydroxyl, amino or $C_1$-$C_6$ alkyl;
$R^{3-9}$ is $C_1$-$C_6$ alkyl;
$R^4$ is 4-6 membered heterocyclyl unsubstituted or substituted by one or more $R^{4-1}$; the heteroatom in said 4-6 membered heterocyclyl is selected from the group consisting of N, O, and S, and the number of heteroatom(s) is 1 or 2;
$R^{4-1}$ is each independently selected from hydroxyl;
$R^5$ is 3-6 membered cycloalkyl unsubstituted or substituted by one or more $R^{5-1}$ , $-S(O)_2R^{5-2}$, or 4-6 membered heterocyclyl unsubstituted or substituted by one or more $R^{5-3}$; the heteroatom in said 4-6 membered heterocyclyl is selected from the group consisting of N, O, and S, and the number of heteroatom(s) is 1 or 2;
$R^{5-2}$ is $C_1$-$C_6$ alkyl;
$R^{5-3}$ is each independently selected from $-S(O)_2R^{5-3-1}$; $R^{5-3-1}$ is each independently selected from $C_1$-$C_6$ alkyl;
$R^6$ is 3-6 membered cycloalkyl unsubstituted or substituted by one or more $R^{6-1}$;
$R^{5-1}$ and $R^{6-1}$ are each independently selected from hydroxyl; and
$R^7$ is 3-6 membered cycloalkyl.

**2.** The compound of formula I, the pharmaceutically acceptable salt thereof, or the isotopic compound thereof according to claim 1, wherein:

m is 0, 1, 2, or 3; n is 1, 2, or 3;
L is

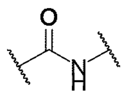 , 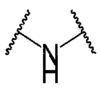 , 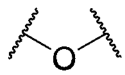 , -CO- or 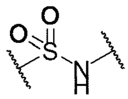 ;

the ring A is a 6-12 membered aromatic ring or a 5-12 membered heteroaromatic ring; the heteroatom in said 5-12 membered heteroaromatic ring is selected from the group consisting of N, O, and S, and the number of heteroatom(s) is 1, 2, or 3;
$R^1$ is 6-12 membered aryl unsubstituted or substituted by one or more $R^{1-1}$ or 5-12 membered heteroaryl unsubstituted or substituted by one or more $R^{1-2}$; the heteroatom in said 5-12 membered heteroaryl is selected from the group consisting of N, O, and S, and the number of heteroatom(s) is 1, 2, or 3;
$R^{1-1}$ and $R^{1-2}$ are each independently selected from the group consisting of deuterium, halogen, cyano, amino, hydroxyl, $C_1$-$C_6$ alkyl unsubstituted or substituted by one or more $R^{1-1-1}$, $C_1$-$C_6$ alkoxy unsubstituted or substituted by one or more $R^{1-1-2}$, $C_2$-$C_6$ alkenyl unsubstituted or substituted by one or more $R^{1-1-3}$, $C_2$-$C_6$ alkynyl unsubstituted or substituted by one or more $R^{1-1-4}$, 3-6 membered cycloalkyl unsubstituted or substituted by one or more $R^{1-1-5}$, 4-6 membered heterocyclyl unsubstituted or substituted by one or more $R^{1-1-6}$, $-COOR^{1-1-7}$, $-C(O)R^{1-1-8}$, $-C(O)NR^{1-1-9}R^{1-1-9}$,

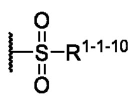 and 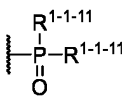 ;

the heteroatom in said 4-6 membered heterocyclyl is selected from the group consisting of N, O, and S, and the number of heteroatom(s) is 1, 2, or 3;
$R^{1-1-1}$, $R^{1-1-2}$, $R^{1-1-3}$, $R^{1-1-4}$, $R^{1-1-5}$ and $R^{1-1-6}$ are each independently selected from the group consisting of deuterium, hydroxyl, cyano and halogen;
$R^{1-1-7}$, $R^{1-1-8}$ and $R^{1-1-9}$ are each independently selected from hydrogen or $C_1$-$C_6$ alkyl;
$R^{1-1-10}$ and $R^{1-1-11}$ are each independently selected from hydroxyl or $C_1$-$C_6$ alkyl;
$R^2$ is each independently selected from the group consisting of deuterium, halogen, cyano, amino, hydroxyl,

$C_1$-$C_6$ alkyl unsubstituted or substituted by one or more $R^{2-1}$, $C_1$-$C_6$ alkoxy unsubstituted or substituted by one or more $R^{2-2}$, $C_2$-$C_6$ alkenyl unsubstituted or substituted by one or more $R^{2-3}$, $C_2$-$C_6$ alkynyl unsubstituted or substituted by one or more $R^{2-4}$, 3-6 membered cycloalkyl unsubstituted or substituted by one or more $R^{2-5}$ and 4-6 membered heterocyclyl unsubstituted or substituted by one or more $R^{2-6}$; the heteroatom in said 4-6 membered heterocyclyl is selected from the group consisting of N, O, and S, and the number of heteroatom(s) is 1, 2, or 3;

$R^{2-1}$, $R^{2-2}$, $R^{2-3}$, $R^{2-4}$, $R^{2-5}$ and $R^{2-6}$ are each independently selected from the group consisting of deuterium, hydroxyl, cyano and halogen;

the ring B is a 5-20 membered fused heteroaromatic ring unsubstituted or substituted by one or more $R^{b-1}$; the heteroatom in said 5-20 membered fused heteroaromatic ring is selected from the group consisting of N, O, and S, and the number of heteroatom(s) is 1, 2, 3, 4, 5, or 6, a single heteroaromatic ring in said 5-20 membered fused heteroaromatic ring is a 5-7 membered heteroaromatic ring, and the number of rings in said 5-20 membered fused heteroaromatic ring is 2, 3 or 4;

$R^{b-1}$ is each independently selected from the group consisting of deuterium, oxo(=O), halogen, cyano, hydroxyl, $C_1$-$C_6$ alkyl unsubstituted or substituted by one or more $R^{b-1-1}$, $C_1$-$C_6$ alkoxy unsubstituted or substituted by one or more $R^{b-1-2}$, $-NR^{b-1-3}R^{b-1-3}$, $-C(O)NR^{b-1-4}R^{b-1-4}$, $-COOR^{b-1-5}$,

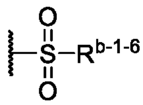

and

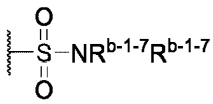

;

$R^{b-1-1}$ and $R^{b-1-2}$ are each independently selected from the group consisting of hydroxyl, halogen and-$NR^{b-1-1-1}R^{b-1-1}$;

$R^{b-1-3}$, $R^{b-1-4}$, $R^{b-1-5}$ and $R^{b-1-7}$ are each independently selected from hydrogen or $C_1$-$C_6$ alkyl;

$R^{b-1-6}$ is each independently selected from hydroxyl or $C_1$-$C_6$ alkyl;

$R^{b-1-1-1}$ is each independently selected from the group consisting of hydrogen, $C_1$-$C_6$ alkyl and $-C(O)R^{b-1-1-1-1}$;

$R^{b-1-1-1-1}$ is $C_1$-$C_6$ alkyl;

$R^3$ is hydrogen, 3-12 membered cycloalkyl unsubstituted or substituted by one or more $R^{3-1}$, 4-12 membered heterocyclyl unsubstituted or substituted by one or more $R^{3-2}$, 4-12 membered cycloalkenyl unsubstituted or substituted by one or more $R^{3-3}$, 4-12 membered heterocycloalkenyl unsubstituted or substituted by one or more $R^{3-4}$, 6-10 membered aryl unsubstituted or substituted by one or more $R^{3-5}$, 5-10 membered heteroaryl unsubstituted or substituted by one or more $R^{3-6}$, $-C(O)R^4$, $-NH(CH_2)_pR^5$, $-O(CH_2)_qR^6$, $C_1$-$C_6$ alkyl unsubstituted or substituted by one or more $R^{3-7}$, or $-OR^7$ unsubstituted or substituted by one or more $R^{3-8}$;

the heteroatom in each of said 4-12 membered heterocyclyl, said 4-12 membered heterocycloalkenyl and said 5-10 membered heteroaryl is selected from the group consisting of N, O, and S, and the number of heteroatom(s) is 1, 2, 3 or 4;

p is 0, 1, 2, or 3;

q is 1, 2, or 3;

$R^{3-1}$, $R^{3-2}$, $R^{3-3}$, $R^{3-4}$, $R^{3-7}$ and $R^{3-8}$ are each independently selected from the group consisting of deuterium, hydroxyl, halogen, cyano, $-NHC(O)R^{3-2-1}$, $C_1$-$C_6$ alkyl unsubstituted or substituted by one or more $R^{3-2-2}$, $C_2$-$C_6$ alkenyl, $C_2$-$C_6$ alkynyl, oxo(=O), $-C(O)NH_2$, 3-6 membered cycloalkyl unsubstituted or substituted by one or more $R^{3-2-3}$, 4-6 membered heterocyclyl, $-S(O)_2R^{3-4-1}$ , $-NR^{3-4-2}R^{3-4-2}$,

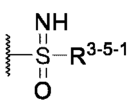 and 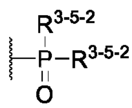 ;

the heteroatom in said 4-6 membered heterocyclyl is selected from the group consisting of N, O, and S, and the number of heteroatom(s) is 1 or 2;

$R^{3-5}$ and $R^{3-6}$ are each independently selected from the group consisting of deuterium, hydroxyl, halogen, cyano,

amino, $-NHC(O)R^{3-2-1}$, $C_1$-$C_6$ alkyl unsubstituted or substituted by one or more $R^{3-2-2}$, $C_2$-$C_6$ alkenyl, $C_2$-$C_6$ alkynyl, $-C(O)NH_2$, 3-6 membered cycloalkyl unsubstituted or substituted by one or more $R^{3-2-3}$, 4-6 membered heterocyclyl, $-S(O)_2R^{3-4-1}$ , $NR^{3-4-2}$ $R^{3-4-2}$,

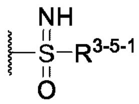 and 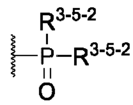 ;

the heteroatom in said 4-6 membered heterocyclyl is selected from the group consisting of N, O, and S, and the number of heteroatom(s) is 1 or 2;
$R^{3-2-1}$ and $R^{3-4-2}$ are each independently selected from hydrogen or $C_1$-$C_6$ alkyl;
$R^{3-2-3}$, $R^{3-5-1}$ and $R^{3-5-2}$ are each independently selected from hydroxyl or $C_1$-$C_6$ alkyl;
$R^{3-2-2}$ is each independently selected from hydroxyl or halogen;
$R^{3-4-1}$ is hydroxyl, amino or $C_1$-$C_6$ alkyl;
$R^4$ is 4-6 membered heterocyclyl; the heteroatom in said 4-6 membered heterocyclyl is selected from the group consisting of N, O, and S, and the number of heteroatom(s) is 1 or 2;
$R^5$ is 3-6 membered cycloalkyl unsubstituted or substituted by one or more $R^{5-1}$
$R^6$ is 3-6 membered cycloalkyl unsubstituted or substituted by one or more $R^{6-1}$;
$R^{5-1}$ and $R^{6-1}$ are each independently selected from hydroxyl; and
$R^7$ is 3-6 membered cycloalkyl.

**3.** The compound of formula I, the pharmaceutically acceptable salt thereof, or the isotopic compound thereof according to claim 1, wherein the compound of formula I is a compound as shown in formula I',

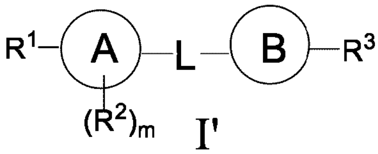

I'

.

**4.** The compound of formula I, the pharmaceutically acceptable salt thereof, or the isotopic compound thereof according to claim 1, wherein the compound of formula I satisfies one or more of the following conditions:

(1) in the ring A, said 5-12 membered heteroaromatic ring is a 5-10 membered heteroaromatic ring, and the heteroatom in said 5-10 membered heteroaromatic ring may be selected from one or two of N, O or S; said 5-10 membered heteroaromatic ring is preferably a 5-6 membered heteroaromatic ring, a 5 membered-fused-6 membered heteroaromatic ring or a 6 membered-fused-6 membered heteroaromatic ring, preferably,

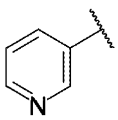 , 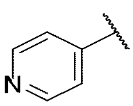 , 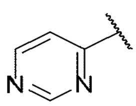 , 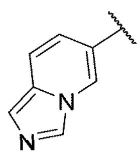 , 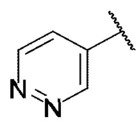 , 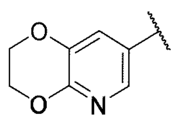 , 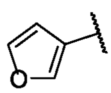 ,

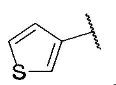 , 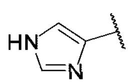 , 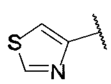 , 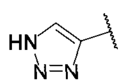 or 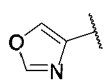 ,

for example,

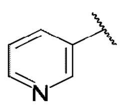 , 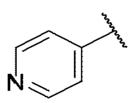 ,

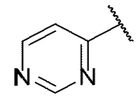 or 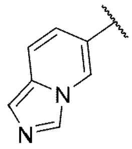 ;

(2) in $R^1$, said 6-12 membered aryl is 6-10 membered aryl, and can be phenyl or naphthyl, for example, phenyl;
(3) in $R^1$, said 5-12 membered heteroaryl is 5-10 membered heteroaryl, the heteroatom in said 5-10 membered heteroaryl can be N and/or O, and the number of heteroatom(s) can be 1 or 2; said 5-12 membered heteroaryl is preferably 5-6 membered heteroaryl or 5 membered-fused-6 membered heteroaryl, more preferably

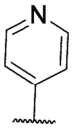 , 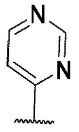 , 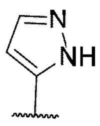 , 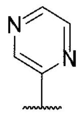 , 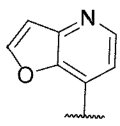 or 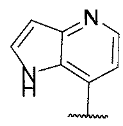 ,

for example,

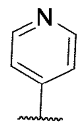 ;

(4) in each $R^{1-1}$, said halogen is fluorine, chlorine, bromine or iodine, for example, fluorine;
(5) in each $R^{1-1}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl;
(6) in each $R^{1-1}$, said $C_1$-$C_6$ alkoxy is $C_1$-$C_4$ alkoxy, and can be methoxy, ethoxy, n-propoxy, isopropoxy, n-butoxy, isobutoxy, pr-butoxy, sec-butoxy or tert-butoxy, for example, methoxy;
(7) in each $R^{1-2}$, said halogen is fluorine, chlorine, bromine or iodine, for example, fluorine or chlorine;
(8) in each $R^{1-2}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, preferably methyl;
(9) in each $R^{1-2}$, said $C_1$-$C_6$ alkoxy is $C_1$-$C_4$ alkoxy, and can be methoxy, ethoxy, n-propoxy, isopropoxy, n-butoxy, isobutoxy, pr-butoxy, sec-butoxy or tert-butoxy, for example, methoxy;
(10) in each $R^{1-1-1}$, said halogen is fluorine, chlorine, bromine or iodine, preferably fluorine;
(11) in each $R^{1-1-2}$, said halogen is fluorine, chlorine, bromine or iodine, for example, fluorine;
(12) in each $R^2$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, for example, methyl;
(13) in the ring B, the number of heteroatom(s) in said 5-20 membered fused heteroaromatic ring is 1, 2, 3 or 4; said 5-20 membered fused heteroaromatic ring is preferably 5 membered-fused-5 membered heteroaromatic ring, a 5 membered-fused-6 membered heteroaromatic ring, a 6 membered-fused-6 membered heteroaromatic ring, a 5 membered-fused-6 membered-fused-6 membered-fused-6 membered heteroaromatic ring or a 5 membered-fused-6 membered-fused-7 membered-fused-6 membered heteroaromatic ring, more preferably

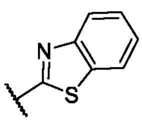 , 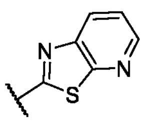 , 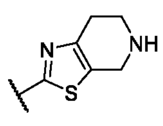 , 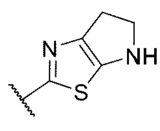 ,

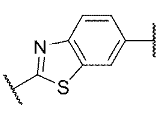 , 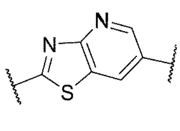 , 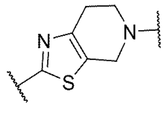 , 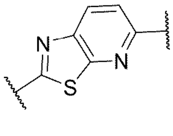 , 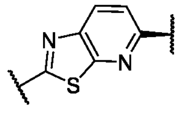 , 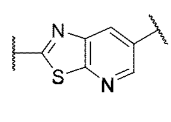 ,

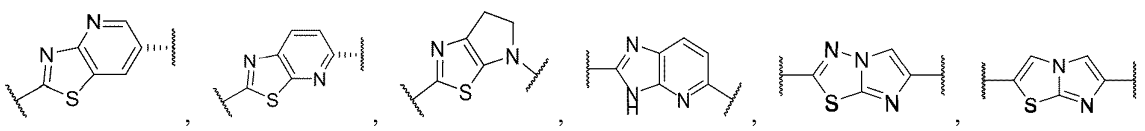
, , , , , ,

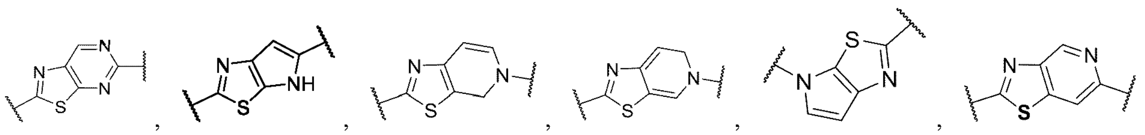
, , , , , ,

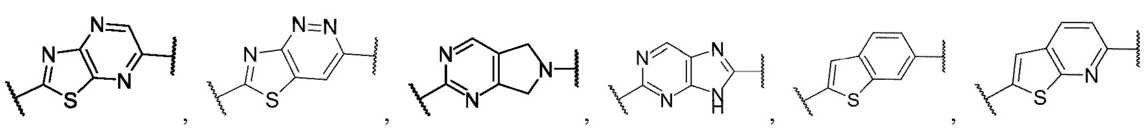
, , , , , ,

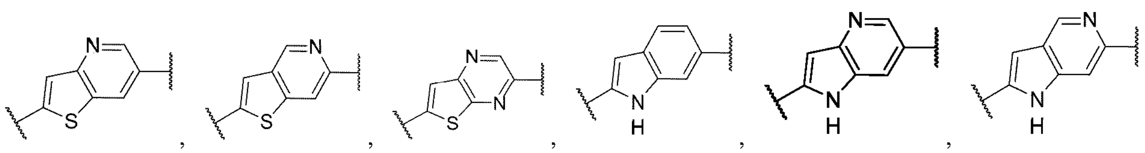
, , , , , ,

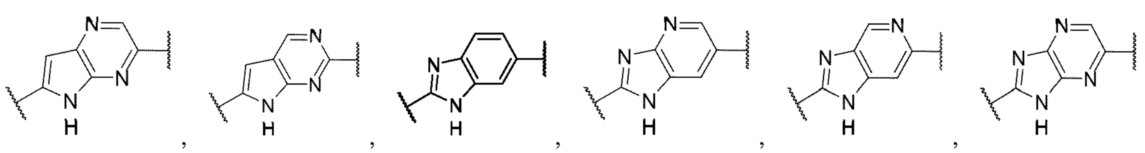
, , , , , ,

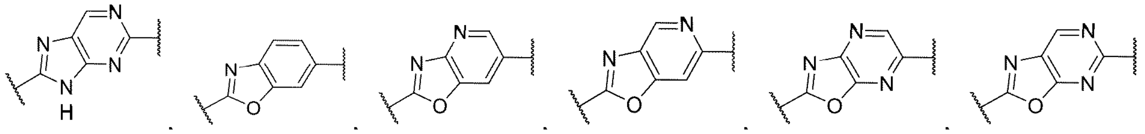
, , , , , ,

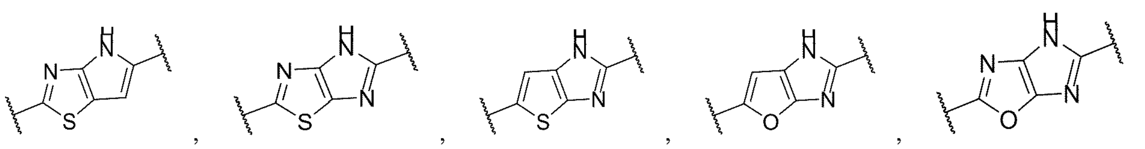
, , , , ,

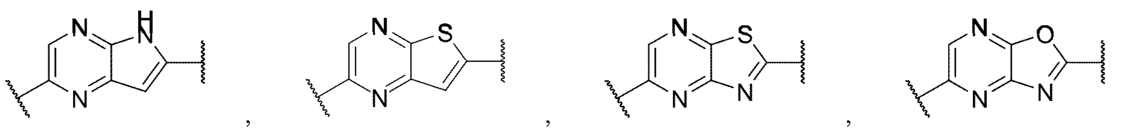
, , , ,

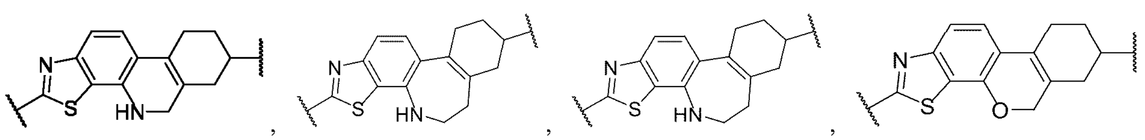
, , , ,

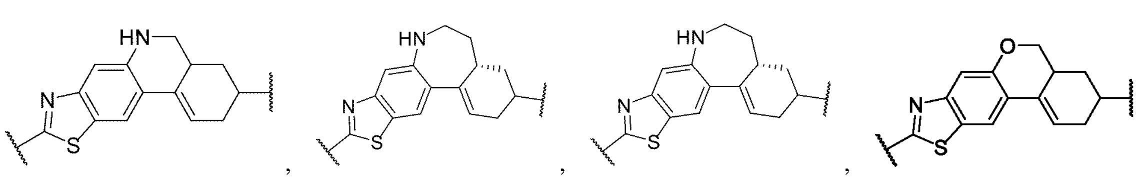
, , , ,

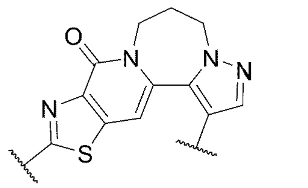 , 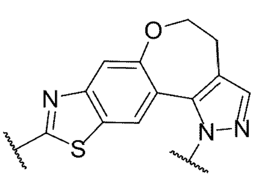 , 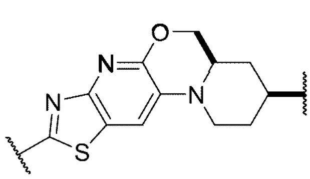 ,

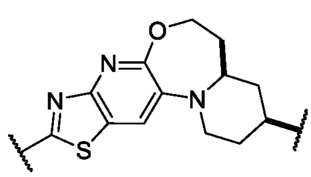 , 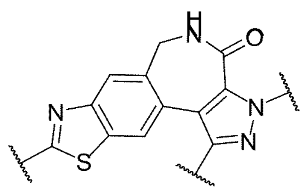 , 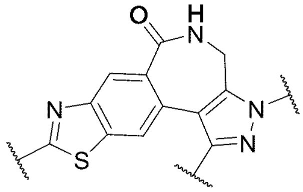 ,

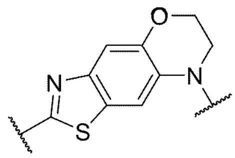 , 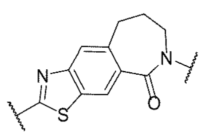 , 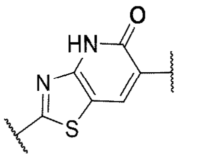 , 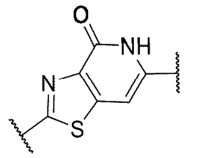 ,

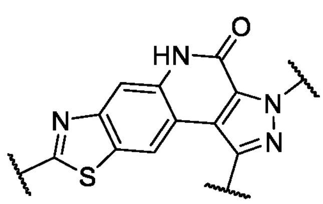 or 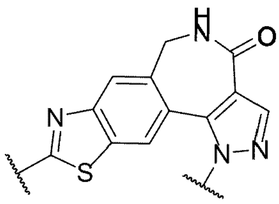 ,

, for example,

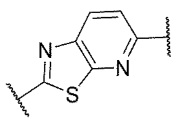 , 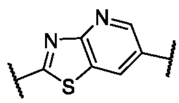 ,

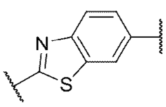 , 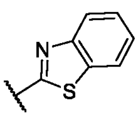 , 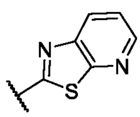 , 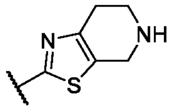 , 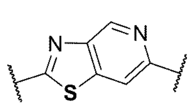 or 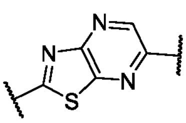

(for another example,

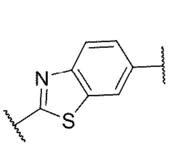 , 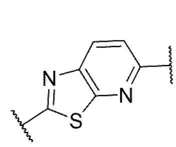 , 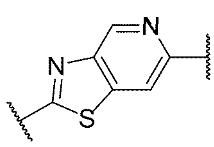 , 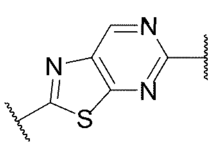 ,

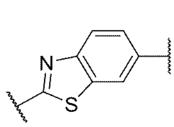 , 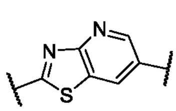 , 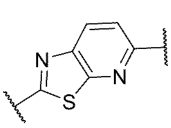

);

(14) in each $R^{b-1}$, said halogen is fluorine, chlorine, bromine or iodine, for example, fluorine;

(15) in each $R^{b-1}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, for example, methyl;

(16) in each $R^{b-1}$, said $C_1$-$C_6$ alkoxy is $C_1$-$C_4$ alkoxy, and can be methoxy, ethoxy, n-propoxy, isopropoxy, n-butoxy, isobutoxy, pr-butoxy, sec-butoxy or tert-butoxy, for example, methoxy or ethoxy;

(17) in each $R^{b-1-1}$, said halogen is fluorine, chlorine, bromine or iodine;

(18) in each $R^{b-1-2}$, said halogen is fluorine, chlorine, bromine or iodine;

(19) in each $R^{b-1-2-1}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, preferably methyl;

(20) in $R^3$, said 3-12 membered cycloalkyl is 3-6 membered cycloalkyl, and can be cyclopropyl, cyclobutyl, cyclopentyl or cyclohexyl, preferably cyclobutyl or cyclohexyl, for example, cyclohexyl;

(21) in $R^3$, a ring in said 4-12 membered heterocyclyl is a monocyclic, spiro, fused or bridged ring, the number of rings in said spiro, fused or bridged ring can be 2, said spiro ring can be a 3 membered-spiro-5 membered heterocyclyl, 3 membered-spiro-6 membered heterocyclyl, 4 membered-spiro-5 membered heterocyclyl, 4 membered-spiro-6 membered heterocyclyl, 4 membered-spiro-4 membered heterocyclyl, 4 membered-spiro-7 membered heterocyclyl, 5 membered-spiro-4 membered heterocyclyl, 5 membered-spiro-7 membered heterocyclyl, 5 membered-spiro-5 membered heterocyclyl or 5 membered-spiro-6 membered heterocyclyl, said fused ring can be 3 membered-fused-5 membered heterocyclyl, 4 membered-fused-5 membered heterocyclyl or 5 membered-fused-5 membered heterocyclyl, said bridged ring can be 5 membered-bridged-6 membered heterocyclyl, and the number of heteroatom(s) in said 4-12 membered heterocyclyl can be 1 or 2; the monocyclic ring is preferably 4-7 membered heterocyclyl, and the number of heteroatom(s) can be 1 or 2; a heteroatom is preferably N and/or O, preferably

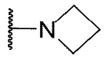, 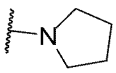, 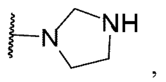, 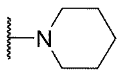, 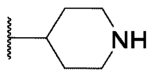, 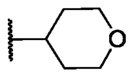, 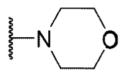,

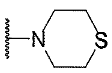, 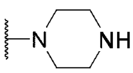, 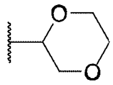, 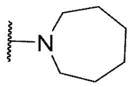, 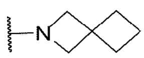, 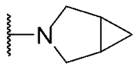, 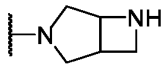,

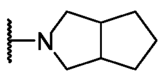, 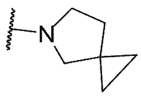, 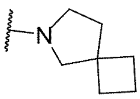, 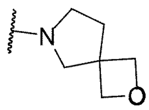, 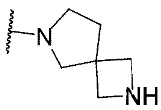, 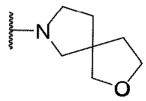,

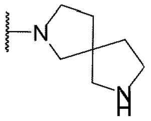, 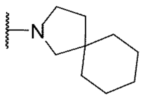, 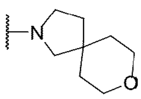, 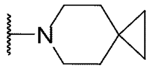, 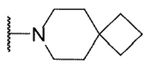, 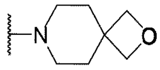,

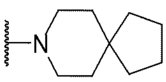, 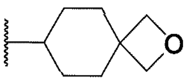, 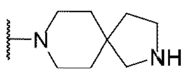 or 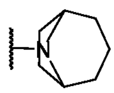

, for example,

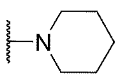, 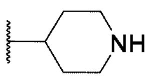,

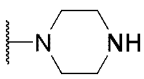 or 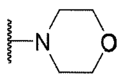,

, for another example,

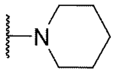 ;

(22) in $R^3$, a ring in said 4-10 membered cycloalkenyl is a monocyclic, spiro, fused or bridged ring, the number of rings in said spiro, fused or bridged ring can be 2, said spiro ring can be 4 membered-spiro-4 membered cycloalkenyl, 4 membered-spiro-5 membered cycloalkenyl or 4 membered-spiro-6 membered cycloalkenyl, said fused ring can be 5 membered-fused-5 membered cycloalkenyl or 5 membered-fused-6 membered cycloalkenyl, said bridged ring can be 5 membered-bridged-7 membered cycloalkenyl, preferably

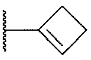 , 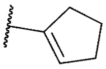 , 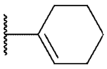 , 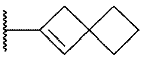 , 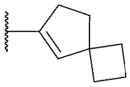 , 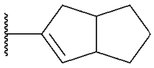 , 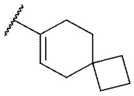 ,

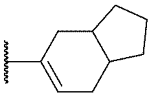 or 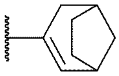 ,

, for example,

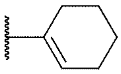 ;

(23) in $R^3$, a ring in said 4-10 membered heterocycloalkenyl is a monocyclic or spiro ring, the number of rings in said spiro ring can be 2, said spiro ring can be 4 membered-spiro-4 membered heterocycloalkenyl, 4 membered-spiro-5 membered heterocycloalkenyl or 4 membered-spiro-6 membered heterocycloalkenyl, and the number of heteroatom in said 4-10 membered heterocycloalkenyl can be 1;

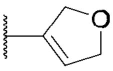 , 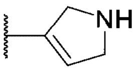 ,

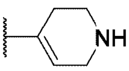 , 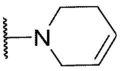 , 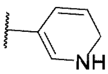 , 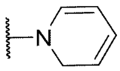 , 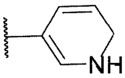 , 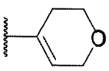 , 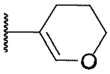 , 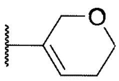 ,

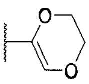 , 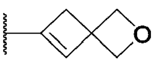 , 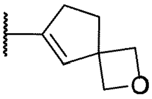 , 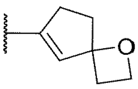 , 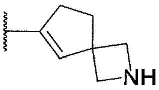 , 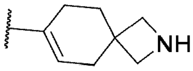 or

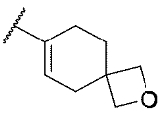

is preferred, for example,

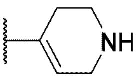 or 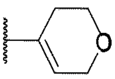 ;

(24) in $R^3$, said 6-10 membered aryl is aryl or naphthyl, preferably aryl;

(25) in $R^3$, said 5-10 membered heteroaryl is monocyclic- or fused heteroaryl, the number of rings in said fused ring can be 2, said fused ring can be 5 membered-fused-5 membered heteroaryl or 5 membered-fused-6 membered heteroaryl, the heteroatom in said 5-10 membered heteroaryl can be N and/or O, and the number of

heteroatom can be 1;

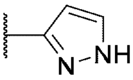, 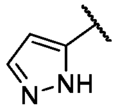, 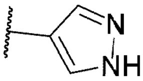, 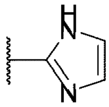, 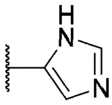,

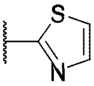, 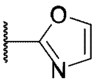, 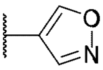, 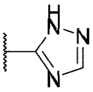, 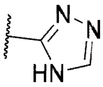, 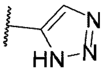, 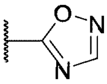, 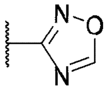, 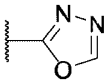,

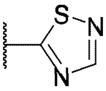, 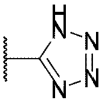, 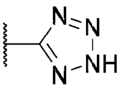, 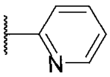, 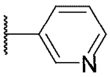, 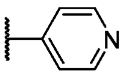, 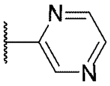, 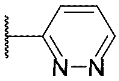,

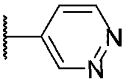, 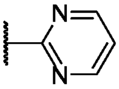, 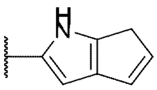, 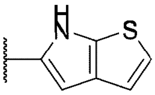, 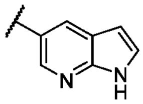, 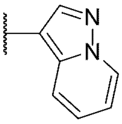, 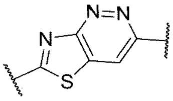,

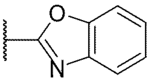, 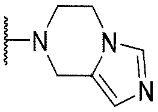, 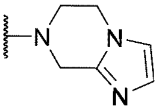, 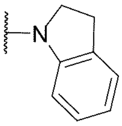, 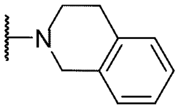 or 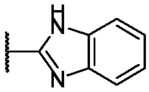 is preferred, for example,

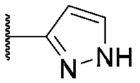 or 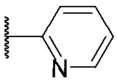

, for another example,

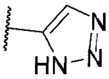;

(26) in $R^3$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, preferably methyl or isopropyl, for example, methyl;

(27) in each $R^{3-1}$, said halogen is fluorine, chlorine, bromine or iodine, preferably fluorine;

(28) in each $R^{3-1}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, preferably methyl;

(29) in each $R^{3-2}$, said halogen is fluorine, chlorine, bromine or iodine, for example, fluorine;

(30) in each $R^{3-2}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, preferably methyl or isopropyl, for example, methyl;

(31) in each $R^{3-2}$, said $C_2$-$C_6$ alkenyl is $C_2$-$C_4$ alkenyl, and can be

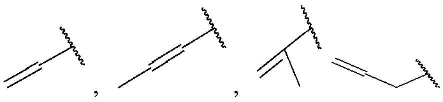

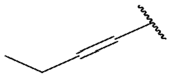, 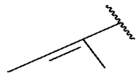, 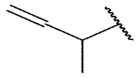 or 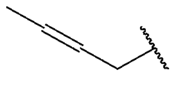,

preferably

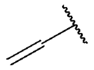;

(32) in each $R^{3-2}$, said $C_2$-$C_6$ alkynyl is $C_2$-$C_4$ alkynyl, and can be

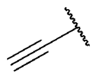, 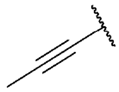, 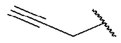,

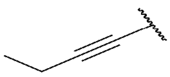, 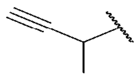 or 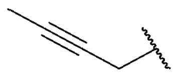,

, preferably

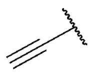;

(33) in each $R^{3-2}$, said 3-6 membered cycloalkyl is cyclopropyl, cyclobutyl, cyclopentyl or cyclohexyl, preferably cyclobutyl;
(34) in each $R^{3-2}$, the number of heteroatom(s) in the 4-6 membered heterocyclyl can be 1 or 2, and the heteroatom in said 4-6 membered heterocyclyl is preferably

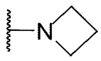, 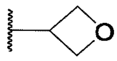, 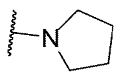,

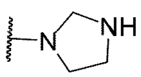, 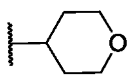, 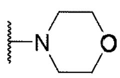, 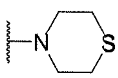, 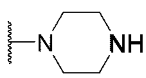, 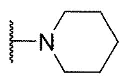 or 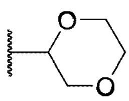;

(35) in $R^{3-2-1}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, preferably methyl;
(36) in each $R^{3-2-2}$, said halogen is fluorine, chlorine, bromine or iodine, preferably fluorine;
(37) in each $R^{3-2-3}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, preferably methyl;
(38) in each $R^{3-3}$, said halogen is fluorine, chlorine, bromine or iodine, preferably, fluorine;
(39) in each $R^{3-4}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, preferably methyl;
(40) in each $R^{3-4}$, said halogen is fluorine, chlorine, bromine or iodine;
(41) in each $R^{3-4}$, the number of heteroatom(s) in the 4-6 membered heterocyclyl can be 1 or 2, or the heteroatom in said 4-6 membered heterocyclyl can be

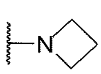, 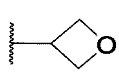, 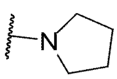, 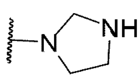,

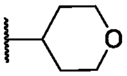, 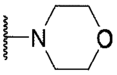, 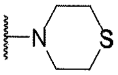, 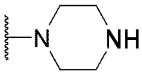, 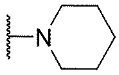, or 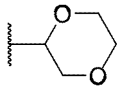

, preferably

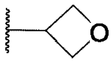;

(42) in $R^{3-4-1}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, preferably methyl;
(43) in each $R^{3-4-2}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, preferably methyl;
(44) in $R^{3-5-1}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, preferably methyl;
(45) in each $R^{3-6}$, said halogen is fluorine, chlorine, bromine or iodine, preferably chlorine;
(46) in each $R^{3-6}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, preferably methyl;
(47) in each $R^{3-7}$, said halogen is fluorine, chlorine, bromine or iodine;
(48) in each $R^{3-7}$, the number of heteroatom(s) in said 4-6 membered heterocyclyl can be 1 or 2, or the heteroatom in said 4-6 membered heterocyclyl can be

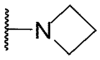, 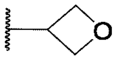, 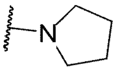, 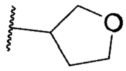,

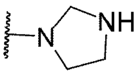, 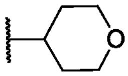, 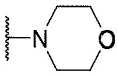, 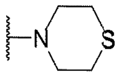, 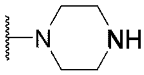, 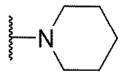 or 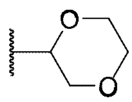,

for example

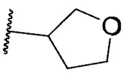;

(49) in $R^4$, the number of heteroatom(s) in said 4-6 membered heterocyclyl can be 1 or 2, or the heteroatom in said 4-6 membered heterocyclyl can be

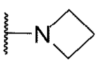, 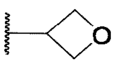, 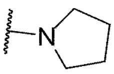, 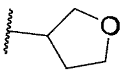, 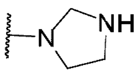,

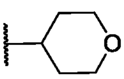, 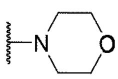, 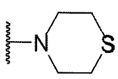, 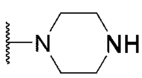, 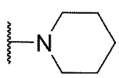 or 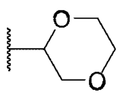

, for example,

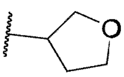;

(50) in $R^5$, said 3-6 membered cycloalkyl is cyclopropyl, cyclobutyl, cyclopentyl or cyclohexyl, preferably cyclobutyl;
(51) in $R^6$, said 3-6 membered cycloalkyl is cyclopropyl, cyclobutyl, cyclopentyl or cyclohexyl, preferably cyclohexyl;

(52) in $R^7$, said 3-6 membered cycloalkyl is cyclopropyl, cyclobutyl, cyclopentyl or cyclohexyl, preferably cyclohexyl;
(53) in each $R^{3-2-1-1}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, preferably methyl;
(54) in each $R^{3-6-1}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, preferably methyl;
(55) in each $R^{3-9}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, preferably methyl;
(56) in $R^5$, the heteroatom in said 4-6 membered heterocyclyl is selected from the group consisting of N, O, and S, and the number of heteroatom(s) can be 1 or 2, for example,

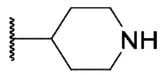 ;

(57) in each $R^{5-2}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, preferably methyl;
(58) in each $R^{5-3-1}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, preferably methyl;
(59) in each $R^{3-5-2}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, preferably methyl or ethyl;
(60) in $R^{3-5-2}$, said 1-10 membered heteroalkyl can be 1-4 membered heteroalkyl;
(61) in $R^{3-5-2}$, the heteroatom in said 1-10 membered heteroalkyl comprises, in addition to phosphorus, selected from N and/or O;
(62) in $R^{3-5-2}$, the number of heteroatom(s) in said 1-10 membered heteroalkyl is 3;
(63) in $R^{3-5-2}$, said 4-10 membered heterocyclyl can be 4-8 membered heteroalkyl, for example,

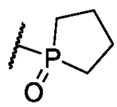 ;

(64) in $R^{3-5-2}$, the heteroatom in said 4-10 membered heterocyclyl comprises, in addition to phosphorus, selected from N and/or O;
(65) in $R^{3-5-2}$, the number of heteroatom(s) in said 4-10 membered heterocyclyl is 1 or 3;
(66) in $R^3$, said 3-12 membered cycloalkyl is 7-10 membered cycloalkyl, a ring in said 7-10 membered cycloalkyl can be a spiro, fused or bridged ring, the number of rings in said spiro, fused or bridged ring can be 2, said spiro ring can be 4 membered-spiro-4 membered cycloalkyl, 4 membered-spiro-5 membered cycloalkyl or 4 membered-spiro-6 membered cycloalkyl, said fused ring can be 5 membered-fused-5 membered cycloalkyl or 5 membered-fused-6 membered cycloalkyl, and the bridged ring can be 5 membered-bridged-7 membered cycloalkyl, preferably

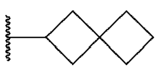 , 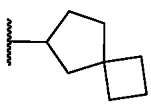 , 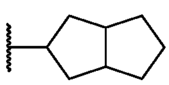 ,

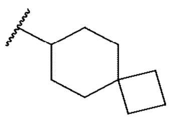 , 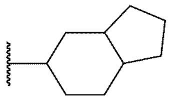 or 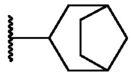 ;

(67) said pharmaceutically acceptable salt is trifluoride hydrochloride or formate;
(68) in each $R^2$, said halogen can be fluorine, chlorine, bromine or iodine, for example, fluorine;
(69) in each of $R^{b-1-4}$ and $R^{b-1-5}$, said $C_1$-$C_6$ alkyl can be $C_1$-$C_4$ alkyl, or can be methyl, ethyl, n-propyl, isopropyl, n-butyl, isobutyl, pr-butyl, sec-butyl or tert-butyl, preferably methyl;
(70) in $R^3$, the heteroatom in said 5-10 membered heteroaryl can be N, and the number of heteroatom(s)can be 1, 2, or 3;
(71) in each $R^{3-6}$, the heteroatom in said 4-6 membered heterocyclyl may be selected from N, and the number of

heteroatom(s) can be 1 or 2, for example,

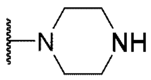;

and (72) in each $R^{3-6}$, said 3-6 membered cycloalkyl can be cyclopropyl, cyclobutyl, cyclopentyl or cyclohexyl, preferably cyclopropyl.

**5.** The compound of formula I, the pharmaceutically acceptable salt thereof, or the isotopic compound thereof according to any one of claims 1-4, wherein the compound of formula I satisfies one or more of the following conditions:

(1) the ring A is a 5-12 membered heteroaromatic ring; preferably, the ring A is a 5-10 membered heteroaromatic ring; the heteroatom in said 5-10 membered heteroaromatic ring is N, and the number of heteroatom(s) is 1 or 2;
(2) $R^1$ is phenyl unsubstituted or substituted by one or more $R^{1-1}$ or 5-10 membered heteroaryl unsubstituted or substituted by one or more $R^{1-2}$; the heteroatom in said 5-10 membered heteroaryl is N and/or O, and the number of heteroatom(s) is 1 or 2;
(3) $R^{1-1}$ is each independently selected from deuterium, halogen, cyano, amino, $C_1$-$C_6$ alkyl unsubstituted or substituted by one or more $R^{1-1-1}$ or $C_1$-$C_6$ alkoxy unsubstituted or substituted by one or more $R^{1-1-2}$; preferably, each $R^{1-1}$ is each independently selected from halogen or $C_1$-$C_6$ alkoxy unsubstituted or substituted by one or more $R^{1-1-2}$;
(4) $R^{1-2}$ is each independently selected from the group consisting of deuterium, halogen, cyano, amino, $C_1$-$C_6$ alkyl unsubstituted or substituted by one or more $R^{1-1-1}$ and $C_1$-$C_6$ alkoxy unsubstituted or substituted by one or more $R^{1-1-2}$; preferably, $R^{1-2}$ is each independently selected from the group consisting of deuterium, halogen, $C_1$-$C_6$ alkyl unsubstituted or substituted by one or more $R^{1-1-1}$ and $C_1$-$C_6$ alkoxy unsubstituted or substituted by one or more $R^{1-1-2}$; more preferably, $R^{1-2}$ is each independently selected from halogen or unsubstituted or $C_1$-$C_6$ alkoxy substituted by one or more $R^{1-1-2}$;
(5) $R^{1-1-1}$ is each independently selected from deuterium or halogen;
(6) $R^{1-1-2}$ is each independently selected from deuterium or halogen; preferably, $R^{1-1-2}$ is each independently selected from halogen;
(7) $R^2$ is each independently selected from deuterium, or $C_1$-$C_6$ alkane unsubstituted or substituted by one or more $R^{2-1}$; preferably, $R^2$ is each independently selected from deuterium or $C_1$-$C_6$ alkyl, or is $C_1$-$C_6$ alkane unsubstituted or substituted by one or more $R^{2-1}$; preferably, $R^2$ is each independently selected from $C_1$-$C_6$ alkyl;
(8) m is 0 or 1;
(9) the ring B is a 5-12 membered fused heteroaromatic ring unsubstituted or substituted by one or more $R^{b-1}$; the heteroatom in said 5-12 membered fused heteroaromatic ring is N and/or S, the number of heteroatom(s) is 1, 2, 3 or 4, a single heteroaryl ring in said 5-12 membered fused heteroaromatic ring is a 5-7 membered heteroaromatic ring, the number of rings in said 5-12 membered fused heteroaromatic ring is 2,

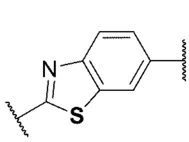 , 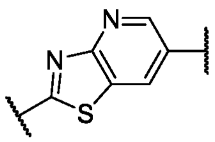 or 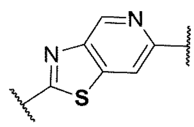

is preferred; preferably, the ring B is

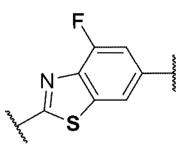 , 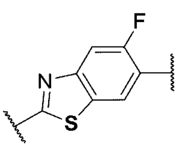 , 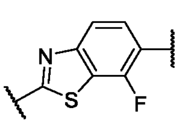 , 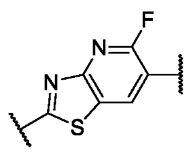 , 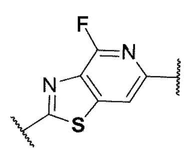 ,

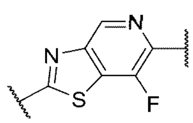 or 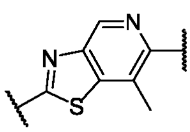 ;

(10) $R^{b-1}$ is each independently selected from the group consisting of deuterium, oxo(=O), halogen, cyano, hydroxyl, $C_1$-$C_6$ alkyl unsubstituted or substituted by one or more $R^{b-1-1}$ or $C_1$-$C_6$ alkoxy unsubstituted and substituted by one or more $R^{b-1-2}$; preferably, $R^{b-1}$ is each independently selected from halogen or $C_1$-$C_6$ alkyl;

(11) $R^{b-1-1}$ is each independently selected from the group consisting of hydroxyl, halogen and amino unsubstituted or substituted by one or two $R^{b-1-2-1}$;

(12) $R^{b-1-2}$ is each independently selected from the group consisting of hydroxyl, halogen and amino unsubstituted or substituted by one or two $R^{b-1-2-1}$;

(13) $R^{b-1-2-1}$ is each independently selected from hydrogen or $C_1$-$C_6$ alkyl;

(14) $R^3$ is hydrogen, 3-6 membered cycloalkyl unsubstituted or substituted by one or more $R^{3-1}$, 4-12 membered heterocyclyl unsubstituted or substituted by one or more $R^{3-2}$, 4-10 membered cycloalkenyl unsubstituted or substituted by one or more $R^{3-3}$, 4-10 membered heterocycloalkenyl unsubstituted or substituted by one or more $R^{3-4}$, 6-10 membered aryl unsubstituted or substituted by one or more $R^{3-5}$, 5-10 membered heteroaryl unsubstituted or substituted by one or more $R^{3-6}$, $-C(O)R^4$, $-NH(CH_2)_pR^5$, $-O(CH_2)_qR^6$, $C_1$-$C_6$ alkyl unsubstituted or substituted by one or more $R^{3-7}$ or $-OR^7$ unsubstituted or substituted by one or more $R^{3-8}$; the heteroatom in said 4-12 membered heterocyclyl, 4-10 membered heterocycloalkenyl or 5-10 membered heteroaryl is one or more of N, O, and S, and the number of heteroatom(s) is 1, 2, 3 or 4; preferably, $R^3$ is hydrogen, 3-6 membered cycloalkyl unsubstituted or substituted by one or more $R^{3-1}$, 4-6 membered heterocyclyl unsubstituted or substituted by one or more $R^{3-2}$, 4-6 membered cycloalkenyl unsubstituted or substituted by one or more $R^{3-3}$, 4-6 membered heterocycloalkenyl unsubstituted or substituted by one or more $R^{3-4}$, phenyl unsubstituted or substituted by one or more $R^{3-5}$, 5-6 membered heteroaryl unsubstituted or substituted by one or more $R^{3-6}$, $-C(O)R^4$ or $C_1$-$C_6$ alkyl unsubstituted or substituted by one or more $R^{3-7}$; the heteroatom in said 4-6 membered heterocyclyl, 4-6 membered heterocycloalkenyl or 5-6 membered heteroaryl is N and/or O, and the number of heteroatom(s) is 1 or 2;

(15) p is 0, 1, 2, or 3; q is 1, 2, or 3;

(16) $R^{3-1}$ is each independently selected from the group consisting of deuterium, hydroxyl, halogen and $C_1$-$C_6$ alkyl; preferably, $R^{3-1}$ is each independently selected from hydroxyl or deuterium, for example, $R^{3-1}$ is each independently selected from hydroxyl;

(17) $R^{3-2}$ is each independently selected from the group consisting of deuterium, hydroxyl, halogen, cyano, amino, $-NHC(O)R^{3-2-1}$, $C_1$-$C_6$ alkyl unsubstituted or substituted by one or more $R^{3-2-2}$, $C_2$-$C_6$ alkenyl, $C_2$-$C_6$ alkynyl, oxo(=O), $-C(O)NH_2$, 3-6 membered cycloalkyl unsubstituted or substituted by one or more $R^{3-2-3}$ and 4-6 membered heterocyclyl; the heteroatom in said 4-6 membered heterocyclyl is selected from the group consisting of N, O, and S, and the number of heteroatom(s) is 1 or 2; preferably, $R^{3-2}$ is each independently selected from the group consisting of hydroxyl, deuterium and $C_1$-$C_6$ alkyl, for example, $R^{3-2}$ is each independently selected from hydroxyl or $C_1$-$C_6$ alkyl;

(18) $R^{3-2-1}$ is $C_1$-$C_6$ alkyl;

(19) $R^{3-2-2}$ is each independently selected from the group consisting of deuterium, hydroxyl and halogen, preferably, $R^{3-2-2}$ is each independently selected from hydroxyl or halogen; for example, $R^{3-2-2}$ is each independently selected from deuterium;

(20) $R^{3-2-3}$ is each independently selected from $C_1$-$C_6$ alkyl or hydroxyl;

(21) $R^{3-3}$ is each independently selected from the group consisting of deuterium, hydroxyl, halogen, cyano, amino, $-S(O)_2R^{3-4-1}$ and $-NR^{3-2}R^{3-2}$; preferably, $R^{3-3}$ is each independently selected from hydroxyl or deuterium; for example, $R^{3-3}$ is each independently selected from hydroxyl;

(22) $R^{3-4}$ is each independently selected from the group consisting of deuterium, hydroxyl, $C_1$-$C_6$ alkyl, oxo(=O), cyano, halogen, 4-6 membered heterocyclyl, $-S(O)_2R^{3-4-1}$ and $-NR^{3-4-2}R^{3-4-2}$ ; preferably, $R^{3-4}$ is each independently selected from $C_1$-$C_6$ alkyl;

(23) $R^{3-4-1}$ is $C_1$-$C_6$ alkyl;

(24) $R^{3-4-2}$ is each independently selected from hydrogen or $C_1$-$C_6$ alkyl;

(25) $R^{3-5}$ is each independently selected from deuterium,

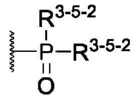

or

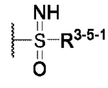

; preferably, $R^{3-5}$ is each independently selected from

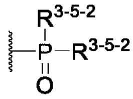 or 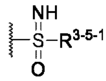

, or $R^{3-5}$ is each independently selected from deuterium or

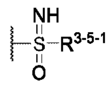;

preferably, $R^{3-5}$ is each independently selected from

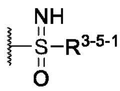;

(26) $R^{3-5-1}$ and $R^{3-5-2}$ are each independently selected from $C_1$-$C_6$ alkyl, preferably, $R^{3-5-1}$ is $C_1$-$C_6$ alkyl;
(27) $R^{3-6}$ is each independently selected from the group consisting of deuterium, halogen and $C_1$-$C_6$ alkyl unsubstituted or substituted by one or more $R^{3-2-2}$, preferably $R^{3-6}$ is each independently selected from the group consisting of deuterium, halogen and $C_1$-$C_6$ alkyl; for example, $R^{3-6}$ is each independently selected from halogen or $C_1$-$C_6$ alkyl;
(28) $R^{3-7}$ is each independently selected from the group consisting of deuterium, hydroxyl, halogen and 4-6 membered heterocyclyl; the heteroatom in said 4-6 membered heterocyclyl is O, and the number of heteroatom(s) is 1; preferably, $R^{3-7}$ is each independently selected from 4-6 membered heterocyclyl; the heteroatom in said 4-6 membered heterocyclyl is O, and the number of heteroatom(s) is 1;
(29) $R^{3-8}$ is hydroxyl;
(30) $R^4$ is 4-6 membered heterocyclyl; the heteroatom in said 4-6 membered heterocyclyl is O, and the number of heteroatom(s) is 1;
(31) $R^5$ is 3-6 membered cycloalkyl substituted by one or more $R^{5-1}$;
(32) $R^6$ is 3-6 membered cycloalkyl substituted by one or more $R^{6-1}$;

and (33) $R^{2-1}$ is each independently selected from deuterium.

**6.** The compound of formula I, the pharmaceutically acceptable salt thereof, or the isotopic compound thereof according to any one of claims 1-4, wherein the compound of formula I satisfies one or more of the following conditions:

(1) the ring A is a 6-12 membered aromatic ring or a 5-6 membered heteroaromatic ring; the heteroatom in said 5-6 membered heteroaromatic ring is one or two selected from N, O, and S, and the number of heteroatom(s) is 1, 2, or 3;
(2) $R^1$ is 6-12 membered aryl unsubstituted or substituted by one or more $R^{1-1}$ or 5-6 membered heteroaryl unsubstituted or substituted by one or more $R^{1-2}$; the heteroatom in said 5-6 membered heteroaromatic ring is one or two selected from N, O, and S, and the number of heteroatom(s) is 1, 2, or 3;
(3) $R^{1-1}$ and $R^{1-2}$ are each independently selected from halogen or $C_1$-$C_6$ alkoxy;
(4) the ring B is a 5-20 membered fused heteroaromatic ring unsubstituted or substituted by one or more $R^{b-1}$; the heteroatom in said 5-20 membered fused heteroaromatic ring is selected from the group consisting of N, O, and S, and the number of heteroatom(s) is 1, 2, 3, 4, 5, 6, 7 or 8, a single heteroaromatic ring in said 5-20 membered fused heteroaromatic ring is a 5-7 membered heteroaromatic ring, and the number of rings in said 5-20 membered fused heteroaromatic ring is 3 or 4;
(5) $R^{b-1}$ is each independently selected from the group consisting of deuterium, cyano, halogen and $C_1$-$C_6$ alkyl unsubstituted or substituted by one or more $R^{b-1-1}$;
(6) $R^3$ is hydrogen,

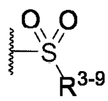,

3-6 membered cycloalkyl unsubstituted or substituted by one or more $R^{3-1}$, 4-9 membered heterocyclyl unsubstituted or substituted by one or more $R^{3-2}$, 4-6 membered cycloalkenyl unsubstituted or substituted by one or more $R^{3-3}$, 4-6 membered heterocycloalkenyl unsubstituted or substituted by one or more $R^{3-4}$, phenyl unsubstituted or substituted by one or more $R^{3-5}$, 5-10 membered heteroaryl unsubstituted or substituted by one or more $R^{3-6}$, -C(O)$R^4$ or $C_1$-$C_6$ alkyl unsubstituted or substituted by one or more $R^{3-7}$; the heteroatom in said 4-6 membered heterocycloalkenyl and 5-10 membered heteroaryl is N and/or O, and the number of heteroatom(s) is 1 or 2; the heteroatom in said 4-9 membered heterocyclyl is one or two selected from N, O, and S, and the number of heteroatom(s) is 1 or 2;

(7) $R^{3-2}$ is each independently selected from the group consisting of deuterium, oxo, halogen, $C_1$-$C_6$ alkoxy, -N$R^{3-4-2}R^{3-4-2}$, -S(O)$_2R^{3-4-1}$, hydroxyl and $C_1$-$C_6$ alkyl unsubstituted or substituted by one or more $R^{3-2-2}$;

(8) $R^{3-3}$ is each independently selected from the group consisting of deuterium, oxo, amino and hydroxyl;

(9) $R^{3-4}$ is each independently selected from the group consisting of deuterium, oxo and $C_1$-$C_6$ alkyl;

(10) $R^{3-4-1}$ is $C_1$-$C_6$ alkyl;

(11) $R^{3-4-2}$ is each independently selected from the group consisting of -S(O)$_2R^{3-4-1}$, hydrogen and $C_1$-$C_6$ alkyl;

(12) $R^{3-6}$ is each independently selected from the group consisting of deuterium, halogen, $C_1$-$C_6$ alkyl unsubstituted or substituted by one or more $R^{3-2-2}$ and 4-6 membered heterocyclyl unsubstituted or substituted by one or more $R^{3-6-1}$; the heteroatom in said 4-6 membered heterocyclyl is one or two selected from N, O, and S, and the number of heteroatom(s) is 1 or 2;

(13) $R^{3-6-1}$ is each independently selected from $C_1$-$C_6$ alkyl;

(14) $R^{3-2-2}$ is each independently selected from deuterium or hydroxyl;

(15) $R^{3-7}$ is each independently selected from the group consisting of deuterium, hydroxyl, -S(O)$_2R^{3-4-1}$ and 4-6 membered heterocyclyl; the heteroatom in said 4-6 membered heterocyclyl is O, and the number of heteroatom(s) is 1;

(16) $R^{3-5}$ is each independently selected from the group consisting of deuterium,

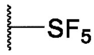
,
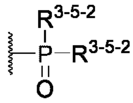

and

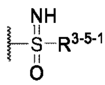
;

and (17) $R^{3-5-2}$ is each independently selected from the group consisting of hydroxyl, $C_1$-$C_6$ alkyl, 1-10 membered heteroalkyl and two $R^{3-5-2}$s form 4-10 membered heterocyclyl; the heteroatom in said 1-10 membered heteroalkyl comprises, in addition to phosphorus, selected from the group consisting of N, O, and S, and the number of heteroatom(s) is 1, 2, 3 or 4; the heteroatom in said 4-10 membered heterocyclyl comprises, in addition to phosphorus, selected from the group consisting of N, O, and S, and the number of heteroatom(s) is 1, 2, 3 or 4.

**7.** The compound of formula I, the pharmaceutically acceptable salt thereof, or the isotopic compound thereof according to any one of claims 1-4, wherein in the compound of formula I, $R^3$ satisfies one of the following conditions:

(1) $R^3$ is hydrogen, 3-6 membered cycloalkyl unsubstituted or substituted by one or more $R^{3-1}$, 4-6 membered heterocyclyl unsubstituted or substituted by one or more $R^{3-2}$, 4-6 membered cycloalkenyl unsubstituted or substituted by one or more $R^{3-3}$, 4-6 membered heterocycloalkenyl unsubstituted or substituted by one or more $R^{3-4}$, phenyl unsubstituted or substituted by one or more $R^{3-5}$, 5-6 membered heteroaryl unsubstituted or substituted by one or more $R^{3-6}$, -C(O)$R^4$ or $C_1$-$C_6$ alkyl unsubstituted or substituted by one or more $R^{3-7}$; the heteroatom in said 4-6 membered heterocyclyl, 4-6 membered heterocycloalkenyl or 5-6 membered heteroaryl is N and/or O, and the number of heteroatom(s) is 1, 2 or 3;

(2) $R^3$ is hydrogen, 3-6 membered cycloalkyl unsubstituted or substituted by one or more $R^{3-1}$, 4-6 membered heterocyclyl unsubstituted or substituted by one or more $R^{3-2}$, 4-6 membered cycloalkenyl unsubstituted or substituted by one or more $R^{3-3}$, 4-6 membered heterocycloalkenyl unsubstituted or substituted by one or more $R^{3-4}$, phenyl unsubstituted or substituted by one or more $R^{3-5}$, 5-6 membered heteroaryl unsubstituted or substituted by one or more $R^{3-6}$, -C(O)$R^4$ or $C_1$-$C_6$ alkyl unsubstituted or substituted by one or more $R^{3-7}$; the heteroatom in said 4-6 membered heterocyclyl or 4-6 membered heterocycloalkenyl is N and/or O, and the

number of heteroatom(s) is 1 or 2; or the heteroatom in said 5-6 membered heteroaryl is N and/or O, and the number of heteroatom(s) is 1, 2 or 3;

(3) $R^3$ is hydrogen,

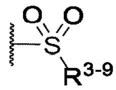,

3-6 membered cycloalkyl unsubstituted or substituted by one or more $R^{3-1}$, 4-9 membered heterocyclyl unsubstituted or substituted by one or more $R^{3-2}$, 4-6 membered cycloalkenyl unsubstituted or substituted by one or more $R^{3-3}$, 4-6 membered heterocycloalkenyl unsubstituted or substituted by one or more $R^{3-4}$, phenyl unsubstituted or substituted by one or more $R^{3-5}$, 5-10 membered heteroaryl unsubstituted or substituted by one or more $R^{3-6}$, -C(O)$R^4$ or $C_1$-$C_6$ alkyl unsubstituted or substituted by one or more $R^{3-7}$; the heteroatom in said 4-6 membered heterocycloalkenyl and 5-10 membered heteroaryl is N and/or O, and the number of heteroatom(s) is 1, 2 or 3; the heteroatom in said 4-9 membered heterocyclyl is one or two selected from N, O, and S, and the number of heteroatom(s) is 1 or 2;

(4) $R^3$ is hydrogen,

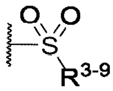,

, 3-6 membered cycloalkyl unsubstituted or substituted by one or more $R^{3-1}$, 4-9 membered heterocyclyl unsubstituted or substituted by one or more $R^{3-2}$, 4-6 membered cycloalkenyl unsubstituted or substituted by one or more $R^{3-3}$, 4-6 membered heterocycloalkenyl unsubstituted or substituted by one or more $R^{3-4}$, phenyl unsubstituted or substituted by one or more $R^{3-5}$, 5-10 membered heteroaryl unsubstituted or substituted by one or more $R^{3-6}$, -C(O)$R^4$ or $C_1$-$C_6$ alkyl unsubstituted or substituted by one or more $R^{3-7}$; the heteroatom in said 4-6 membered heterocycloalkenyl is N and/or O, and the number of heteroatom(s) is 1 or 2; the heteroatom in said 5-10 membered heteroaryl is N and/or O, and the number of heteroatom(s) is 1, 2 or 3; the heteroatom in said 4-9 membered heterocyclyl is one or two selected from N, O, and S, and the number of heteroatom(s) is 1 or 2;

and (5) $R^3$ is hydrogen, 3-6 membered cycloalkyl unsubstituted or substituted by one or more $R^{3-1}$, 4-12 membered heterocyclyl unsubstituted or substituted by one or more $R^{3-2}$, 4-10 membered cycloalkenyl unsubstituted or substituted by one or more $R^{3-3}$, 4-10 membered heterocycloalkenyl unsubstituted or substituted by one or more $R^{3-4}$, 6-10 membered aryl unsubstituted or substituted by one or more $R^{3-5}$, 5-10 membered heteroaryl unsubstituted or substituted by one or more $R^{3-6}$, -C(O)$R^4$, $C_1$-$C_6$ alkyl unsubstituted or substituted by one or more $R^{3-7}$ or -O$R^7$ unsubstituted or substituted by one or more $R^{3-8}$; the heteroatom in said 4-12 membered heterocyclyl or 4-10 membered heterocycloalkenyl is one or more of N, O, and S, and the number of heteroatom(s) is 1, or 2; the heteroatom in said 5-10 membered heteroaryl is N and/or O, and the number of heteroatom(s) is 1, 2, 3 or 4.

**8.** The compound of formula I, the pharmaceutically acceptable salt thereof, or the isotopic compound thereof according to claim 1, wherein the compound of formula I satisfies one or two of the following conditions:

(1) the ring B is a 5-20 membered fused heteroaromatic ring unsubstituted or substituted by one or more $R^{b-1}$; the heteroatom in said 5-20 membered fused heteroaromatic ring is selected from the group consisting of N, O, and S, and the number of heteroatom(s) is 1, 2, 3, 4, 5, 6, 7 or 8, a single heteroaromatic ring in said 5-20 membered fused heteroaromatic ring is a 5-7 membered heteroaromatic ring, and the number of rings in said 5-20 membered fused heteroaromatic ring is 2, 3 or 4, the unsubstituted 5-20 membered fused heteroaromatic ring is

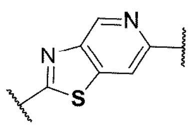;

; preferably, the ring B is a 5-20 membered fused heteroaromatic ring substituted by one or more $R^{b-1}$; the heteroatom in said 5-20 membered fused heteroaromatic ring is selected from the group consisting of N, O, and S, the number of heteroatom(s) is 1, 2, 3, 4, 5, 6, 7 or 8, a single heteroaromatic ring in said 5-20 membered fused

heteroaromatic ring is a 5-7 membered heteroaromatic ring, and the number of rings in said 5-20 membered fused heteroaromatic ring is 2, 3 or 4;
(2) the ring A is a 5-12 membered heteroaromatic ring; the heteroatom in said 5-12 membered heteroaromatic ring is one or more of N, O, and S, and the number of heteroatom(s) is 1, 2, or 3;

$R^1$ is 5-12 membered heteroaryl unsubstituted or substituted by one or more $R^{1-2}$; the heteroatom in said 5-12 membered heteroaromatic ring is one or two selected from N, O, and S, and the number of heteroatom(s) is 1, 2 or 3;
$R^3$ is hydrogen,

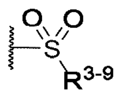

, 3-12 membered cycloalkyl unsubstituted or substituted by one or more $R^{3-1}$, 4-12 membered heterocyclyl substituted by one or more $R^{3-2}$, 4-12 membered cycloalkenyl unsubstituted or substituted by one or more $R^{3-3}$, 4-12 membered heterocycloalkenyl unsubstituted or substituted by one or more $R^{3-4}$, 6-10 membered aryl unsubstituted or substituted by one or more $R^{3-5}$, 5-10 membered heteroaryl unsubstituted or substituted by one or more $R^{3-6}$, $-C(O)R^4$, $-NH(CH_2)_pR^5$, $-O(CH_2)_qR^6$, $C_1$-$C_6$ alkyl, or $-OR^7$ unsubstituted or substituted by one or more $R^{3-8}$; the heteroatom in each of said 4-12 membered heterocyclyl, said 4-12 membered heterocycloalkenyl and said 5-10 membered heteroaryl is selected from the group consisting of N, O, and S, and the number of heteroatom(s) is 1, 2, 3 or 4;
$R^{3-5}$ and $R^{3-6}$ are each independently selected from the group consisting of deuterium,

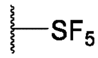 ,

hydroxyl, amino, $-NHC(O)R^{3-2-1}$, $C_1$-$C_6$ alkyl, $C_2$-$C_6$ alkenyl, $C_2$-$C_6$ alkynyl, $-C(O)NH_2$, 3-6 membered cycloalkyl substituted by one or more $R^{3-2-3}$, 4-6 membered heterocyclyl unsubstituted or substituted by one or more $R^{3-6-1}$, $-S(O)_2R^{3-4-1}$, $-NR^{3-4-2}R^{3-4-2}$,

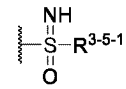 and 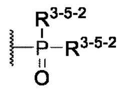 ;

and the heteroatom in said 4-6 membered heterocyclyl is selected from the group consisting of N, O, and S, and the number of heteroatom(s) is 1 or 2.

**9.** The compound of formula I, the pharmaceutically acceptable salt thereof, or the isotopic compound thereof according to claim 1, wherein the compound of formula I is a compound as shown in formula Ia, Ib, Ic, Id or Ie,

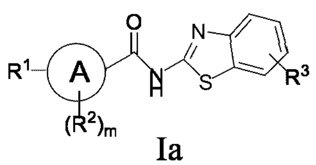

Ia ,

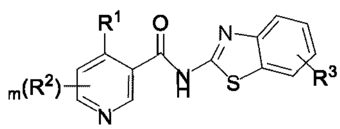

Ib ,

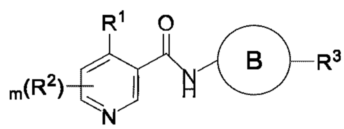

Ic

or

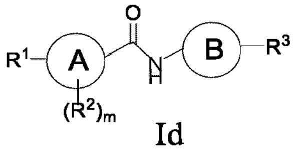
Id

or

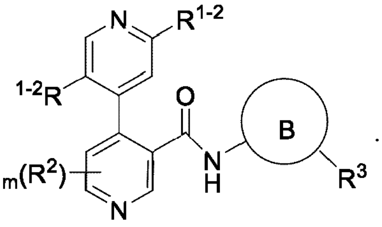
Ie

10. The compound of formula I, the pharmaceutically acceptable salt thereof, or the isotopic compound thereof according to claim 1, wherein the compound of formula I is as defined in a scheme 1, a scheme 1', a scheme 2, a scheme 2', a scheme 3, a scheme 3', a scheme 4, a scheme 4', a scheme 5, a scheme 6, a scheme 7, a scheme 8, a scheme 8', a scheme 9, a scheme 9', a scheme 10, a scheme 11 or a scheme 12;
in the scheme 1,

L is

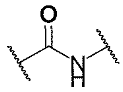
;

the ring A is a 5-12 membered heteroaromatic ring; the heteroatom in said 5-12 membered heteroaromatic ring is one or more of N, O, and S, and the number of heteroatom(s) is 1, 2, or 3;
$R^1$ is a 6-12 membered aryl unsubstituted or substituted by one or more $R^{1-1}$, or 5-12 membered heteroaryl unsubstituted or substituted by one or more $R^{1-2}$; the heteroatom in said 5-12 membered heteroaryl is selected from the group consisting of N, O, and S, and the number of heteroatom(s) is 1, 2, or 3;
$R^{1-1}$ and $R^{1-2}$ are each independently selected from the group consisting of deuterium, halogen, cyano, amino, $C_1$-$C_6$ alkyl unsubstituted or substituted by one or more $R^{1-1-1}$ and $C_1$-$C_6$ alkoxy unsubstituted or substituted by one or more $R^{1-1-2}$;
$R^{1-1-1}$ and $R^{1-1-2}$ are each independently selected from deuterium or halogen;
$R^2$ is each independently selected from deuterium or $C_1$-$C_6$ alkyl;
m is 0, 1, 2, or 3;
the ring B is a 5-20 membered fused heteroaromatic ring unsubstituted or substituted by one or more $R^{b-1}$; the heteroatom in said 5-20 membered fused heteroaromatic ring is one or more of N, O, and S, and the number of heteroatom(s) is 1, 2, 3, 4, 5 or 6, a single heteroaromatic ring in said 5-20 membered fused heteroaromatic ring is a 5-7 membered heteroaryl ring, and the number of rings in said 5-20 membered fused heteroaromatic ring is 2, 3 or 4;
$R^{b-1}$ is each independently selected from the group consisting of deuterium, oxo(=O), halogen, cyano, hydroxyl, $C_1$-$C_6$ alkyl unsubstituted or substituted by one or more $R^{b-1-1}$ and $C_1$-$C_6$ alkoxy unsubstituted or substituted by one or more $R^{b-1-2}$;
$R^{b-1-1}$ and $R^{b-1-2}$ are each independently selected from the group consisting of hydroxyl, halogen and amino unsubstituted or substituted by1 or 2$R^{b-1-2-1}$;
$R^{b-1-2-1}$ is each independently selected from hydrogen or $C_1$-$C_6$ alkyl;
$R^3$ is hydrogen, 3-6 membered cycloalkyl unsubstituted or substituted by one or more $R^{3-1}$, 4-12 membered heterocyclyl unsubstituted or substituted by one or more $R^{3-2}$, 4-10 membered cycloalkenyl unsubstituted or substituted by one or more $R^{3-3}$, 4-10 membered heterocycloalkenyl unsubstituted or substituted by one or more $R^{3-4}$, 6-10 membered aryl unsubstituted or substituted by one or more $R^{3-5}$, 5-10 membered heteroaryl unsubstituted or substituted by one or more $R^{3-6}$, $-C(O)R^4$, $-NH(CH_2)_pR^5$, $-O(CH_2)_qR^6$, $C_1$-$C_6$ alkyl unsubstituted or substituted by one or more $R^{3-7}$, or $-OR^7$ unsubstituted or substituted by one or more $R^{3-8}$; the heteroatom in said 4-12 membered heterocyclyl, 4-10 membered heterocycloalkenyl or 5-10 membered heteroaryl is one or more selected from N, O, and S, and the number of heteroatom(s) is 1, 2 or 3; p is 0, 1, 2, or 3;
q is 1, 2, or 3;
$R^{3-1}$ is each independently selected from the group consisting of deuterium, hydroxyl, halogen and $C_1$-$C_6$ alkyl;
$R^{3-2}$ is each independently selected from the group consisting of deuterium, hydroxyl, halogen, cyano, amino, $-NHC(O)R^{3-2-1}$, $C_1$-$C_6$ alkyl unsubstituted or substituted by one or more $R^{3-2-2}$, $C_2$-$C_6$ alkenyl, $C_2$-$C_6$ alkynyl, oxo(=O), $-C(O)NH_2$, 3-6 membered cycloalkyl unsubstituted or substituted by one or more $R^{3-2-3}$ and 4-6 membered heterocyclyl; the heteroatom in said 4-6 membered heterocyclyl is selected from the group consisting

of N, O, and S, and the number of heteroatom(s) is 1 or 2;
$R^{3-2-1}$ is $C_1$-$C_6$ alkyl;
$R^{3-2-2}$ is each independently selected from hydroxyl or halogen;
$R^{3-2-3}$ is each independently selected from $C_1$-$C_6$ alkyl or hydroxyl;
$R^{3-3}$ is each independently selected from the group consisting of deuterium, hydroxyl, halogen, cyano, amino, $-S(O)_2R^{3-4-1}$ and-$NR^{3-4-2}R^{3-4-2}$;
$R^{3-4}$ is each independently selected from the group consisting of deuterium, hydroxyl, $C_1$-$C_6$ alkyl, oxo(=O), cyano, halogen, 4-6 membered heterocyclyl, $-S(O)_2R^{3-4-1}$ and $-NR^{3-4-2}R^{3-4-2}$;
$R^{3-4-1}$ is $C_1$-$C_6$ alkyl;
$R^{3-4-2}$ is each independently selected from hydrogen or $C_1$-$C_6$ alkyl;
$R^{3-5}$ is each independently selected from deuterium or

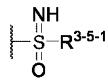
;

$R^{3-5-1}$ is $C_1$-$C_6$ alkyl;
$R^{3-6}$ is each independently selected from the group consisting of deuterium, halogen and $C_1$-$C_6$ alkyl;
$R^{3-7}$ is each independently selected from the group consisting of deuterium, hydroxyl, halogen and 4-6 membered heterocyclyl; the heteroatom in said 4-6 membered heterocyclyl is O, and the number of heteroatom(s) is 1;
$R^{3-8}$ is hydroxyl;
$R^4$ is 4-6 membered heterocyclyl; the heteroatom in said 4-6 membered heterocyclyl is selected from the group consisting of N, O, and S, and the number of heteroatom(s) is 1 or 2;
$R^5$ is 3-6 membered cycloalkyl unsubstituted or substituted by one or more $R^{5-1}$;
$R^{5-1}$ is each independently selected from hydroxyl;
$R^6$ is 3-6 membered cycloalkyl unsubstituted or substituted by one or more $R^{6-1}$;
$R^{6-1}$ is each independently selected from hydroxyl;
$R^7$ is 3-6 membered cycloalkyl;
in the scheme 1',
L is

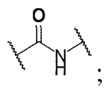
;

the ring A is a 5-10 membered heteroaromatic ring; the heteroatom in said 5-10 membered heteroaromatic ring is N, and the number of heteroatom(s) is 1 or 2;
$R^1$ is 6-10 membered aryl unsubstituted or substituted by one or more $R^{1-1}$ or 5-6 membered heteroaryl unsubstituted or substituted by one or more $R^{1-2}$; the heteroatom in said 5-6 membered heteroaryl is N, and the number of heteroatom(s) is 1 or 2;
$R^{1-1}$ and $R^{1-2}$ are each independently selected from the group consisting of deuterium, halogen, $C_1$-$C_6$ alkyl unsubstituted or substituted by one or more $R^{1-1-1}$, $C_1$-$C_6$ alkoxy unsubstituted or substituted by one or more $R^{1-1-2}$ and

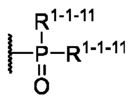
;

$R^{1-1-1}$ and $R^{1-1-2}$ are each independently selected from halogen;
$R^{1-1-11}$ is each independently selected from hydroxyl or $C_1$-$C_6$ alkyl;
$R^2$ is independently deuterium or $C_1$-$C_6$ alkyl unsubstituted or substituted by one or more $R^{2-1}$;
$R^{2-1}$ is each independently selected from halogen or deuterium;
m is 0 or 1;
the ring B is a 5-12 membered fused heteroaromatic ring unsubstituted or substituted by one or more $R^{b-1}$; the heteroatom in said 5-12 membered fused heteroaromatic ring is N and/or S, the number of heteroatom(s) is 1, 2, 3 or 4, and the number of rings in said 5-12 membered fused heteroaromatic ring is 2;
$R^{b-1}$ is each independently selected from the group consisting of deuterium, cyano, halogen and $C_1$-$C_6$ alkyl

unsubstituted or substituted by one or more $R^{b-1-1}$;
$R^{b-1-1}$ is each independently selected from halogen;
$R^3$ is hydrogen,

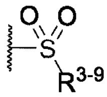

3-6 membered cycloalkyl unsubstituted or substituted by one or more $R^{3-1}$, 4-9 membered heterocyclyl unsubstituted or substituted by one or more $R^{3-2}$, 4-6 membered cycloalkenyl unsubstituted or substituted by one or more $R^{3-3}$, 4-6 membered heterocycloalkenyl unsubstituted or substituted by one or more $R^{3-4}$, phenyl unsubstituted or substituted by one or more $R^{3-5}$, 5-10 membered heteroaryl unsubstituted or substituted by one or more $R^{3-6}$, -C(O)$R^4$ or $C_1$-$C_6$ alkyl unsubstituted or substituted by one or more $R^{3-7}$; the heteroatom in said 4-6 membered heterocycloalkenyl and 5-10 membered heteroaryl is N and/or O, and the number of heteroatom(s) is 1 or 2; the heteroatom in said 4-9 membered heterocyclyl is one or two selected from N, O, and S, and the number of heteroatom(s) is 1 or 2; $R^{3-1}$ is each independently selected from deuterium or hydroxyl;
$R^{3-2}$ is each independently selected from the group consisting of deuterium, oxo, halogen, $C_1$-$C_6$ alkoxy, -N$R^{3-4-2}R^{3-4-2}$, -S(O)$_2R^{3-4-1}$, hydroxyl and $C_1$-$C_6$ alkyl unsubstituted or substituted by one or more $R^{3-2-2}$;
$R^{3-3}$ is each independently selected from the group consisting of deuterium, oxo, amino and hydroxyl; $R^{3-4}$ is each independently selected from the group consisting of deuterium, oxo and $C_1$-$C_6$ alkyl;
$R^{3-4-1}$ is $C_1$-$C_6$ alkyl;
$R^{3-4-2}$ is each independently selected from the group consisting of -S(O)$_2R^{3-4-1}$, hydrogen and $C_1$-$C_6$ alkyl;
$R^{3-5}$ is each independently selected from the group consisting of deuterium,

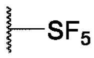
,
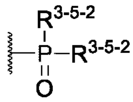

and

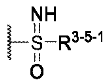
;

$R^{3-5-1}$ is independently $C_1$-$C_6$ alkyl;
$R^{3-5-2}$ is each independently selected from the group consisting of hydroxyl, $C_1$-$C_6$ alkyl, 1-10 membered heteroalkyl, and two $R^{3-5-2}$s form 4-10 membered heterocyclyl; the heteroatom in said 1-10 membered heteroalkyl comprises, in addition to phosphorus, selected from the group consisting of N, O, and S, and the number of heteroatom(s) is 1, 2, 3 or 4; the heteroatom in said 4-10 membered heterocyclyl comprises, in addition to phosphorus, selected from the group consisting of N, O, and S, and the number of heteroatom(s) **is 1, 2, 3 or 4;**
$R^{3-6}$ is each independently selected from the group consisting of deuterium, halogen, $C_1$-$C_6$ alkyl unsubstituted or substituted by one or more $R^{3-2-2}$ and 4-6 membered heterocyclyl unsubstituted or substituted by one or more $R^{3-6-1}$; the heteroatom in said 4-6 membered heterocyclyl is one or two selected from N, O, and S, the number of heteroatom(s) is 1 or 2;
$R^{3-6-1}$ is each independently selected from $C_1$-$C_6$ alkyl;
$R^{3-2-2}$ is each independently selected from deuterium or hydroxyl;
$R^{3-7}$ is each independently selected from the group consisting of deuterium, hydroxyl, -S(O)$_2R^{3-4-1}$ and 4-6 membered heterocyclyl; the heteroatom in said 4-6 membered heterocyclyl is O, and the number of heteroatom(s) is 1;
$R^4$ is 4-6 membered heterocyclyl; the heteroatom in said 4-6 membered heterocyclyl is O, and the number of heteroatom(s) is 1; $R^4$ is 4-6 membered heterocyclyl; the heteroatom in said 4-6 membered heterocyclyl is selected from the group consisting of N, O, and S, and the number of heteroatom(s) is 1 or 2;
$R^5$ is 3-6 membered cycloalkyl unsubstituted or substituted by one or more $R^{5-1}$;
$R^{5-1}$ is each independently selected from hydroxyl;
$R^6$ is 3-6 membered cycloalkyl unsubstituted or substituted by one or more $R^{6-1}$;
$R^{6-1}$ is each independently selected from hydroxyl;

$R^7$ is 3-6 membered cycloalkyl;
in the scheme 2,
L is

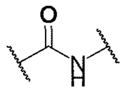;

the ring A is a 5-10 membered heteroaromatic ring; the heteroatom in said 5-10 membered heteroaromatic ring is N, and the number of heteroatom(s) is 1 or 2;
$R^1$ is 6-10 membered aryl unsubstituted or substituted by one or more $R^{1-1}$ or 5-6 membered heteroaryl unsubstituted or substituted by one or more $R^{1-2}$; the heteroatom in said 5-6 membered heteroaryl is N, and the number of heteroatom(s) is 1 or 2;
$R^{1-1}$ and $R^{1-2}$ are each independently selected from the group consisting of deuterium, halogen, $C_1$-$C_6$ alkyl unsubstituted or substituted by one or more $R^{1-1-1}$, $C_1$-$C_6$ alkoxy unsubstituted or substituted by one or more $R^{1-1-2}$ and

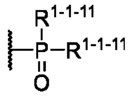;

$R^{1-1-1}$ and $R^{1-1-2}$ are each independently selected from halogen;
$R^{1-1-11}$ is each independently selected from hydroxyl or $C_1$-$C_6$ alkyl;
$R^2$ is independently deuterium or $C_1$-$C_6$ alkyl unsubstituted or substituted by one or more $R^{2-1}$;
$R^{2-1-2}$ is each independently selected from halogen;
m is 0 or 1;
the ring B is 5-12 membered fused heteroaromatic ring unsubstituted or substituted by one or more $R^{b-1}$; the heteroatom in said 5-12 membered fused heteroaromatic ring is N and/or S, the number of heteroatom(s) is 1, 2, 3 or 4, and the number of rings in said 5-12 membered fused heteroaromatic ring is 2;
$R^{b-1}$ is each independently selected from the group consisting of deuterium, halogen and $C_1$-$C_6$ alkyl unsubstituted or substituted by one or more $R^{b-1-1}$;
$R^{b-1-1}$ is each independently selected from halogen;
$R^3$ is hydrogen, 3-6 membered cycloalkyl unsubstituted or substituted by one or more $R^{3-1}$, 4-6 membered heterocyclyl unsubstituted or substituted by one or more $R^{3-2}$, 4-6 membered cycloalkenyl unsubstituted or substituted by one or more $R^{3-3}$, 4-6 membered heterocycloalkenyl unsubstituted or substituted by one or more $R^{3-4}$, phenyl unsubstituted or substituted by one or more $R^{3-5}$, 5-6 membered heteroaryl unsubstituted or substituted by one or more $R^{3-6}$, -C(O)$R^4$ or $C_1$-$C_6$ alkyl unsubstituted or substituted by one or more $R^{3-7}$; the heteroatom in said 4-6 membered heterocyclyl, 4-6 membered heterocycloalkenyl or 5-6 membered heteroaryl is N and/or O, and the number of heteroatom(s) is 1 or 2;
$R^{3-1}$ is each independently selected from deuterium or hydroxyl;
$R^{3-2}$ is each independently selected from the group consisting of deuterium, hydroxyl and $C_1$-$C_6$ alkyl;
$R^{3-3}$ is each independently selected from deuterium or hydroxyl;
$R^{3-4}$ is each independently selected from deuterium or $C_1$-$C_6$ alkyl;
$R^{3-5}$ is each independently selected from deuterium or

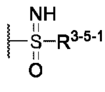;

$R^{3-5-1}$ is $C_1$-$C_6$ alkyl;
$R^{3-6}$ is each independently selected from the group consisting of deuterium, halogen and $C_1$-$C_6$ alkyl;
$R^{3-7}$ is each independently selected from deuterium, or 4-6 membered heterocyclyl; the heteroatom in said 4-6 membered heterocyclyl is O, and the number of heteroatom(s) is 1;
$R^4$ is 4-6 membered heterocyclyl; the heteroatom in said 4-6 membered heterocyclyl is O, and the number of heteroatom(s) is 1;
in the scheme 2',
L is

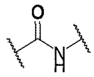;

the ring A is a 5-10 membered heteroaromatic ring; the heteroatom in said 5-10 membered heteroaromatic ring is N, and the number of heteroatom(s) is 1 or 2;
$R^1$ is 6-10 membered aryl unsubstituted or substituted by one or more $R^{1-1}$ or 5-6 membered heteroaryl unsubstituted or substituted by one or more $R^{1-2}$; the heteroatom in said 5-6 membered heteroaryl is N, and the number of heteroatom(s) is 1 or 2;
$R^{1-1}$ and $R^{1-2}$ are each independently selected from the group consisting of deuterium, halogen, $C_1$-$C_6$ alkyl unsubstituted or substituted by one or more $R^{1-1-1}$, $C_1$-$C_6$ alkoxy unsubstituted or substituted by one or more $R^{1-1-2}$ and

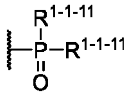;

$R^{1-1-1}$ and $R^{1-1-2}$ are each independently selected from halogen or deuterium;
$R^{1-1-11}$ is each independently selected from hydroxyl or $C_1$-$C_6$ alkyl;
$R^2$ is independently deuterium or $C_1$-$C_6$ alkyl unsubstituted or substituted by one or more $R^{2-1}$;
$R^{2-1}$ is each independently selected from halogen or deuterium;
m is 0 or 1;
the ring B is 5-12 membered fused heteroaromatic ring unsubstituted or substituted by one or more $R^{b-1}$; the heteroatom in said 5-12 membered fused heteroaromatic ring is N and/or S, the number of heteroatom(s) is 1, 2, 3 or 4, and the number of rings in said 5-12 membered fused heteroaromatic ring is 2;
$R^{b-1}$ is each independently selected from the group consisting of deuterium, cyano, halogen and $C_1$-$C_6$ alkyl unsubstituted or substituted by one or more $R^{b-1-1}$;
$R^{b-1-1}$ is each independently selected from halogen;
$R^3$ is hydrogen,

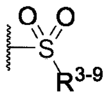,

3-6 membered cycloalkyl unsubstituted or substituted by one or more $R^{3-1}$, 4-9 membered heterocyclyl unsubstituted or substituted by one or more $R^{3-2}$, 4-6 membered cycloalkenyl unsubstituted or substituted by one or more $R^{3-3}$, 4-6 membered heterocycloalkenyl unsubstituted or substituted by one or more $R^{3-4}$, phenyl unsubstituted or substituted by one or more $R^{3-5}$, 5-10 membered heteroaryl unsubstituted or substituted by one or more $R^{3-6}$, -C(O)$R^4$ or $C_1$-$C_6$ alkyl unsubstituted or substituted by one or more $R^{3-7}$; the heteroatom in said 4-6 membered heterocycloalkenyl or 5-10 membered heteroaryl is N and/or O, and the number of heteroatom(s) is 1 or 2; the heteroatom in said 4-9 membered heterocyclyl is one or two selected from N, O, and S, and the number of heteroatom(s) is 1 or 2;
$R^{3-1}$ is each independently selected from deuterium or hydroxyl;
$R^{3-2}$ is each independently selected from the group consisting of deuterium, oxo, halogen, $C_1$-$C_6$ alkoxy, -N$R^{3-4-2}R^{3-4-2}$, -S(O)$_2R^{3-4-1}$, hydroxyl and $C_1$-$C_6$ alkyl unsubstituted or substituted by one or more $R^{3-2-2}$;
$R^{3-3}$ is each independently selected from the group consisting of deuterium, oxo, amino and hydroxyl;
$R^{3-4}$ is each independently selected from the group consisting of deuterium, oxo and $C_1$-$C_6$ alkyl;
$R^{3-4-1}$ is $C_1$-$C_6$ alkyl;
$R^{3-4-2}$ is each independently selected from the group consisting of -S(O)$_2R^{3-4-1}$, hydrogen and $C_1$-$C_6$ alkyl;
$R^{3-5}$ is each independently selected from the group consisting of deuterium,

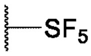, 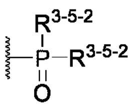

and

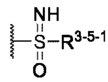

$R^{3-5-1}$ is $C_1$-$C_6$ alkyl;

$R^{3-5-2}$ is each independently selected from the group consisting of hydroxyl, $C_1$-$C_6$ alkyl, 1-10 membered heteroalkyl and two $R^{3-5-2}$s form 4-10 membered heterocyclyl; the heteroatom in said 1-10 membered heteroalkyl comprises, in addition to phosphorus, selected from the group consisting of N, O, and S, and the number of heteroatom(s) is 1, 2, 3 or 4; the heteroatom in said 4-10 membered heterocyclyl comprises, in addition to phosphorus, selected from the group consisting of N, O, and S, and the number of heteroatom(s) **is 1, 2, 3 or 4;**

$R^{3-6}$ is each independently selected from the group consisting of deuterium, halogen, $C_1$-$C_6$ alkyl unsubstituted or substituted by one or more $R^{3-2-2}$ and 4-6 membered heterocyclyl unsubstituted or substituted by one or more $R^{3-6-1}$; the heteroatom in said 4-6 membered heterocyclyl is one or two selected from N, O, and S, the number of heteroatom(s) is 1 or 2;

$R^{3-6-1}$ is each independently selected from $C_1$-$C_6$ alkyl;

$R^{3-2-2}$ is each independently selected from deuterium or hydroxyl;

$R^{3-7}$ is each independently selected from the group consisting of deuterium, hydroxyl, -$S(O)_2R^{3-4-1}$ and 4-6 membered heterocyclyl; the heteroatom in said 4-6 membered heterocyclyl is O, and the number of heteroatom(s) is 1;

$R^4$ is 4-6 membered heterocyclyl; the heteroatom in said 4-6 membered heterocyclyl is O, and the number of heteroatom(s) is 1;

in the scheme 3,

L is

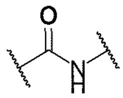

;

the ring A is a 5-10 membered heteroaromatic ring; the heteroatom in said 5-10 membered heteroaromatic ring is N, and the number of heteroatom(s) is 1 or 2;

$R^1$ is 6-10 membered aryl unsubstituted or substituted by one or more $R^{1-1}$ or 5-6 membered heteroaryl unsubstituted or substituted by one or more $R^{1-2}$; the heteroatom in said 5-6 membered heteroaryl is N, and the number of heteroatom(s) is 1;

$R^{1-1}$ and $R^{1-2}$ are each independently selected from halogen or $C_1$-$C_6$ alkoxy unsubstituted or substituted by one or more $R^{1-1-2}$;

$R^{1-1-2}$ is each independently selected from halogen;

$R^2$ is independently $C_1$-$C_6$ alkyl;

m is 0 or 1;

the ring B is a 5-12 membered fused heteroaromatic ring unsubstituted or substituted by one or more $R^{b-1}$; the heteroatom in said 5-12 membered fused heteroaromatic ring is N and/or S, and the number of heteroatom(s) is 1, 2, 3 or 4, a single heteroaryl ring in said 5-12 membered fused heteroaromatic ring is a 5-7 membered heteroaryl ring, and the number of rings in said 5-12 membered fused heteroaromatic ring is 2;

$R^{b-1}$ is each independently selected from halogen;

$R^3$ is hydrogen, 3-6 membered cycloalkyl unsubstituted or substituted by one or more $R^{3-1}$, 4-6 membered heterocyclyl unsubstituted or substituted by one or more $R^{3-2}$, 4-6 membered cycloalkenyl unsubstituted or substituted by one or more $R^{3-3}$, 4-6 membered heterocycloalkenyl unsubstituted or substituted by one or more $R^{3-4}$, phenyl unsubstituted or substituted by one or more $R^{3-5}$, 5-6 membered heteroaryl unsubstituted or substituted by one or more $R^{3-6}$, or -$C(O)R^4$; the heteroatom in said 4-6 membered heterocyclyl, 4-6 membered heterocycloalkenyl or 5-6 membered heteroaryl is N and/or O, and the number of heteroatom(s) is 1 or 2;

$R^{3-1}$ is each independently selected from hydroxyl;

$R^{3-2}$ is each independently selected from hydroxyl or $C_1$-$C_6$ alkyl;

$R^{3-3}$ is each independently selected from hydroxyl;

$R^{3-4}$ is each independently selected from $C_1$-$C_6$ alkyl;

$R^{3-5}$ is each independently selected from

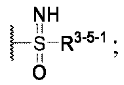

$R^{3-5-1}$ is $C_1$-$C_6$ alkyl;
$R^{3-6}$ is each independently selected from halogen or $C_1$-$C_6$ alkyl;
$R^4$ is 4-6 membered heterocyclyl; the heteroatom in said 4-6 membered heterocyclyl is O, and the number of heteroatom(s) is 1;
in the scheme 3',
L is

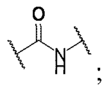

the ring A is a 5-10 membered heteroaromatic ring; the heteroatom in said 5-10 membered heteroaromatic ring is N, and the number of heteroatom(s) is 1 or 2;
$R^1$ is 6-10 membered aryl unsubstituted or substituted by one or more $R^{1-1}$ or 5-6 membered heteroaryl unsubstituted or substituted by one or more $R^{1-2}$; the heteroatom in said 5-6 membered heteroaryl is N, and the number of heteroatom(s) is 1;
$R^{1-1}$ and $R^{1-2}$ are each independently selected from halogen or $C_1$-$C_6$ alkoxy unsubstituted or substituted by one or more $R^{1-1-2}$;
$R^{1-1-2}$ is each independently selected from deuterium or halogen;
$R^2$ is each independently selected from $C_1$-$C_6$ alkane unsubstituted or substituted by one or more $R^{2-1}$;
$R^{2-1}$ is each independently selected from deuterium;
m is 0 or 1;
the ring B is a 5-12 membered fused heteroaromatic ring unsubstituted or substituted by one or more $R^{b-1}$; the heteroatom in said 5-12 membered fused heteroaromatic ring is N and/or S, and the number of heteroatom(s) is 1, 2, 3 or 4, a single heteroaryl ring in said 5-12 membered fused heteroaromatic ring is a 5-7 membered heteroaromatic ring, and the number of rings in said 5-12 membered fused heteroaromatic ring is 2;
$R^{b-1}$ is each independently selected from halogen;
$R^3$ is hydrogen, 3-6 membered cycloalkyl unsubstituted or substituted by one or more $R^{3-1}$, 4-6 membered heterocyclyl unsubstituted or substituted by one or more $R^{3-2}$, 4-6 membered cycloalkenyl unsubstituted or substituted by one or more $R^{3-3}$, 4-6 membered heterocycloalkenyl unsubstituted or substituted by one or more $R^{3-4}$, phenyl unsubstituted or substituted by one or more $R^{3-5}$, 5-6 membered heteroaryl unsubstituted or substituted by one or more $R^{3-6}$, or -C(O)$R^4$; the heteroatom in said 4-6 membered heterocyclyl, 4-6 membered heterocycloalkenyl or 5-6 membered heteroaryl is N and/or O, and the number of heteroatom(s) is 1 or 2;
$R^{3-1}$ is each independently selected from hydroxyl or deuterium;
$R^{3-2}$ is each independently selected from hydroxyl, deuterium or $C_1$-$C_6$ alkyl;
$R^{3-3}$ is each independently selected from hydroxyl or deuterium;
$R^{3-4}$ is each independently selected from $C_1$-$C_6$ alkyl;
$R^{3-5}$ is each independently selected from the group consisting of

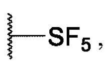 , 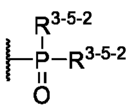 and 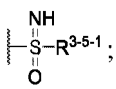

$R^{3-5-1}$ is $C_1$-$C_6$ alkyl;
$R^{3-5-2}$ is each independently selected from hydroxyl, $C_1$-$C_6$ alkyl or 1-10 membered heteroalkyl, or two $R^{3-5-2}$s form 4-10 membered heterocyclyl; the heteroatom in said 1-10 membered heteroalkyl comprises, in addition to phosphorus, selected from the group consisting of N, O, and S, and the number of heteroatom(s) is 1, 2, 3 or 4; the heteroatom in said 4-10 membered heterocyclyl comprises, in addition to phosphorus, selected from the group consisting of N, O, and S, and the number of heteroatom(s) is 1, 2, 3 or 4;
$R^{3-6}$ is each independently selected from halogen or is unsubstituted or substituted by one or more $R^{3-2-2}$; $R^{3-2-2}$ is each independently selected from deuterium;
$R^4$ is 4-6 membered heterocyclyl; the heteroatom in said 4-6 membered heterocyclyl is O, and the number of heteroatom(s) is 1;
in the scheme 4,

L is

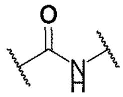

the ring A is a 5-6 membered heteroaromatic ring; the heteroatom in said 5-6 membered heteroaromatic ring is N, and the number of heteroatom(s) is 1 or 2;
$R^1$ is phenyl unsubstituted or substituted by one or more $R^{1-1}$ or 5-6 membered heteroaryl unsubstituted or substituted by one or more $R^{1-2}$; the heteroatom in said 5-6 membered heteroaryl is N, and the number of heteroatom(s) is 1;
$R^{1-1}$ and $R^{1-2}$ are each independently selected from halogen or $C_1$-$C_6$ alkoxy unsubstituted or substituted by one or more $R^{1-1-2}$;
$R^{1-1-2}$ is each independently selected from halogen;
$R^2$ is independently $C_1$-$C_6$ alkyl;
m is 0 or 1;
the ring B is a 5 membered-fused-6 membered heteroaromatic ring; the heteroatom in said 5 membered-fused-6 membered heteroaromatic ring is N and/or S, and the number of heteroatom(s) is 1 or 3;
$R^3$ is hydrogen, 3-6 membered cycloalkyl unsubstituted or substituted by one or more $R^{3-1}$, 4-6 membered heterocyclyl unsubstituted or substituted by one or more $R^{3-2}$ or 4-6 membered cycloalkenyl unsubstituted or substituted by one or more $R^{3-3}$; the heteroatom in said 4-6 membered heterocyclyl is N and/or O, and the number of heteroatom(s) is 1 or 2;
$R^{3-1}$ is each independently selected from hydroxyl;
$R^{3-2}$ is each independently selected from hydroxyl or $C_1$-$C_6$ alkyl;
$R^{3-3}$ is each independently selected from hydroxyl;
in the scheme 4',
L is

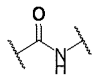

the ring A is a 5-6 membered heteroaromatic ring; the heteroatom in said 5-6 membered heteroaromatic ring is N, and the number of heteroatom(s) is 1;
$R^1$ is phenyl unsubstituted or substituted by one or more $R^{1-1}$ or 5-6 membered heteroaryl unsubstituted or substituted by one or more $R^{1-2}$; the heteroatom in said 5-6 membered heteroaryl is N, and the number of heteroatom(s) is 1;
$R^{1-1}$ and $R^{1-2}$ are each independently selected from halogen or $C_1$-$C_6$ alkoxy unsubstituted or substituted by one or more $R^{1-1-2}$;
$R^{1-1-2}$ is each independently selected from halogen or deuterium;
$R^2$ is independently $C_1$-$C_6$ alkane unsubstituted or substituted by one or more $R^{2-1}$;
$R^{2-1}$ is each independently selected from deuterium;
m is 0 or 1;
the ring B is 5 membered-fused-6 membered cycloheteroaromatic ring; the heteroatom in said 5 membered-fused-6 membered heteroaromatic ring is N and/or S, and the number of heteroatom(s) is 1, 2, 3 or 4;
$R^3$ is 3-6 membered cycloalkyl unsubstituted or substituted by one or more $R^{3-1}$, 4-6 membered cyclene unsubstituted or substituted by one or more $R^{3-3}$, phenyl unsubstituted or substituted by one or more $R^{3-5}$ or 5-6 membered heteroaryl unsubstituted or substituted by one or more $R^{3-6}$; the heteroatom in said 4-6 membered heterocyclyl is N and/or O, and the number of heteroatom(s) is 1 or 2; the heteroatom in said 5-6 membered heteroaromatic ring is N, and the number of heteroatom(s) is 2;
$R^{3-1}$ is each independently selected from hydroxyl;
$R^{3-3}$ is each independently selected from hydroxyl;
$R^{3-5}$ is each independently selected from

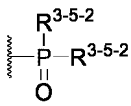 or 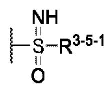;

$R^{3-5-1}$ and $R^{3-5-2}$ are each independently selected from $C_1$-$C_6$ alkyl;
$R^{3-6}$ is each independently selected from halogen or is unsubstituted or substituted by one or more $R^{3-2-2}$; $R^{3-2-2}$ is each independently selected from deuterium;
in the scheme 5,
L is

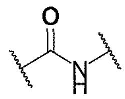
;

the ring A is a 5-6 membered heteroaromatic ring; the heteroatom in said 5-6 membered heteroaromatic ring is N, and the number of heteroatom(s) is 1;
$R^1$ is phenyl unsubstituted or substituted by one or more $R^{1-1}$ or 5-6 membered heteroaryl unsubstituted or substituted by one or more $R^{1-2}$; the heteroatom in said 5-6 membered heteroaryl is N, and the number of heteroatom(s) is 1;
$R^{1-1}$ and $R^{1-2}$ are each independently selected from halogen or $C_1$-$C_6$ alkoxy unsubstituted or substituted by one or more $R^{1-1-2}$;
$R^{1-1-2}$ is each independently selected from halogen;
$R^2$ is independently $C_1$-$C_6$ alkyl;
m is 0 or 1;
the ring B is a 5 membered-fused-6 membered heteroaromatic ring; the heteroatom in said 5 membered-fused-6 membered heteroaromatic ring is N and/or S, and the number of heteroatom(s) is 1 or 3;
$R^3$ is 3-6 membered cycloalkyl unsubstituted or substituted by one or more $R^{3-1}$ or 4-6 membered cycloalkenyl unsubstituted or substituted by one or more $R^{3-3}$;
$R^{3-1}$ is each independently selected from hydroxyl;
$R^{3-3}$ is each independently selected from hydroxyl;
in the scheme 6,
L is

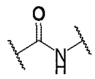
;

the ring A is a 5-6 membered heteroaromatic ring; the heteroatom in said 5-6 membered heteroaromatic ring is N, and the number of heteroatom(s) is 1;
$R^1$ is phenyl unsubstituted or substituted by one or more $R^{1-1}$ or 5-6 membered heteroaryl unsubstituted or substituted by one or more $R^{1-2}$; the heteroatom in said 5-6 membered heteroaryl is N, and the number of heteroatom(s) is 1;
$R^{1-1}$ and $R^{1-2}$ are each independently selected from halogen or $C_1$-$C_6$ alkoxy unsubstituted or substituted by one or more $R^{1-1-2}$;
$R^{1-1-2}$ is each independently selected from halogen or deuterium;
$R^2$ is independently $C_1$-$C_6$ alkane unsubstituted or substituted by one or more $R^{2-1}$;
$R^{2-1}$ is each independently selected from deuterium;
m is 0 or 1;
the ring B is a 5-12 membered fused heteroaromatic ring unsubstituted or substituted by one or more $R^{b-1}$; said 5-12 membered fused heteroaromatic ring is

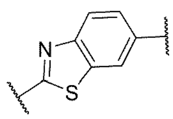
,
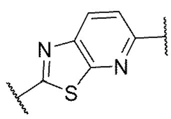
,
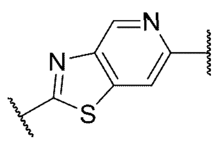
,

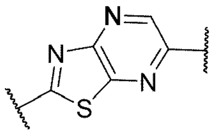, 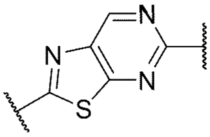, 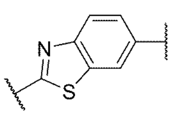, 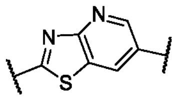, 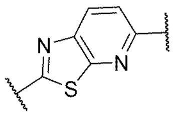;

$R^{b-1}$is each independently selected from halogen;

$R^3$ is 3-6 membered cycloalkyl unsubstituted or substituted by one or more $R^{3-1}$, 4-6 membered cyclene unsubstituted or substituted by one or more $R^{3-3}$, phenyl unsubstituted or substituted by one or more $R^{3-5}$ or 5-6 membered heteroaryl unsubstituted or substituted by one or more $R^{3-6}$; the heteroatom in said 4-6 membered heterocyclyl is N and/or O, and the number of heteroatom(s) is 1 or 2; the heteroatom in said 5-6 membered heteroaromatic ring is N, and the number of heteroatom(s) is 2;

$R^{3-1}$ is each independently selected from hydroxyl;

$R^{3-3}$ is each independently selected from hydroxyl;

$R^{3-5}$ is each independently selected from

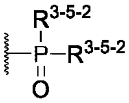 or 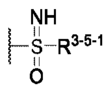;

$R^{3-5-1}$ and $R^{3-5-2}$ are each independently selected from $C_1$-$C_6$ alkyl;

$R^{3-6}$ is each independently selected from halogen or is unsubstituted or substituted by one or more $R^{3-2-2}$; $R^{3-2-2}$ is each independently selected from deuterium;

In the scheme 7,

L is

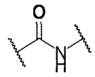;

the ring A is a 6-12 membered aromatic ring or a 5-6 membered heteroaromatic ring; the heteroatom in said 5-6 membered heteroaromatic ring is one or two selected from N, O, and S, and the number of heteroatom(s) is 1, 2, or 3;

$R^1$ is 6-12 membered aryl unsubstituted or substituted by one or more $R^{1-1}$ or 5-6 membered heteroaryl unsubstituted or substituted by one or more $R^{1-2}$; the heteroatom in said 5-6 membered heteroaromatic ring is one or two selected from N, O, and S, and the number of heteroatom(s) is 1, 2, or 3;

$R^{1-1}$ and $R^{1-2}$ are each independently selected from halogen or $C_1$-$C_6$ alkoxy;

the ring B is a 5-20 membered fused heteroaromatic ring unsubstituted or substituted by one or more $R^{b-1}$; the heteroatom in said 5-20 membered fused heteroaromatic ring is selected from the group consisting of N, O, and S, and the number of heteroatom(s) is 1, 2, 3, 4, 5, 6, 7 or 8, a single heteroaromatic ring in said 5-20 membered fused heteroaromatic ring is a 5-7 membered heteroaromatic ring, and the number of rings in said 5-20 membered fused heteroaromatic ring is 3 or 4;

$R^3$ is hydrogen;

in the scheme 8,

L is

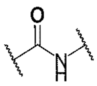;

the ring A is a 5-10 membered heteroaromatic ring; the heteroatom in said 5-10 membered heteroaromatic ring is N, and the number of heteroatom(s) is 1 or 2;

$R^1$ is 6-10 membered aryl unsubstituted or substituted by one or more $R^{1-1}$ or 5-6 membered heteroaryl unsubstituted or substituted by one or more $R^{1-2}$; the heteroatom in said 5-6 membered heteroaryl is N, and the number of heteroatom(s) is 1 or 2;

$R^{1-1}$ and $R^{1-2}$ are each independently selected from the group consisting of deuterium, halogen, $C_1$-$C_6$ alkyl unsubstituted or substituted by one or more $R^{1-1-1}$, $C_1$-$C_6$ alkoxy unsubstituted or substituted by one or more $R^{1-1-2}$ and

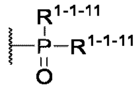

;

$R^{1-1-1}$ and $R^{1-1-2}$ are each independently selected from halogen or deuterium;

$R^{1-1-11}$ is each independently selected from hydroxyl or $C_1$-$C_6$ alkyl;

$R^2$ is independently deuterium or $C_1$-$C_6$ alkyl unsubstituted or substituted by one or more $R^{2-1}$;

$R^{2-1}$ is each independently selected from halogen or deuterium;

m is 0 or 1;

the ring B is a 5-12 membered fused heteroaromatic ring substituted by one or more $R^{b-1}$; the heteroatom in said 5-12 membered fused heteroaromatic ring is N and/or S, the number of heteroatom(s) is 1, 2, 3 or 4, and the number of rings in said 5-12 membered fused heteroaromatic ring is 2;

$R^{b-1}$ is each independently selected from halogen, or $C_1$-$C_6$ alkyl unsubstituted or substituted by one or more $R^{b-1-1}$;

$R^{b-1-1}$ is each independently selected from halogen;

$R^3$ is hydrogen, 3-6 membered cycloalkyl unsubstituted or substituted by one or more $R^{3-1}$, 4-12 membered heterocyclyl unsubstituted or substituted by one or more $R^{3-2}$, 4-10 membered cycloalkenyl unsubstituted or substituted by one or more $R^{3-3}$, 4-10 membered heterocycloalkenyl unsubstituted or substituted by one or more $R^{3-4}$, 6-10 membered aryl unsubstituted or substituted by one or more $R^{3-5}$, 5-10 membered heteroaryl unsubstituted or substituted by one or more $R^{3-6}$, -C(O)$R^4$, $C_1$-$C_6$ alkyl unsubstituted or substituted by one or more $R^{3-7}$ or -O$R^7$ unsubstituted or substituted by one or more $R^{3-8}$; the heteroatom in said 4-12 membered heterocyclyl or 4-10 membered heterocycloalkenyl is selected from the group consisting of N, O, and S, and the number of heteroatom(s) is 1, or 2; the heteroatom in said 5-10 membered heteroaryl is N and/or O, and the number of heteroatom(s) is 1, 2, 3 or 4;

$R^{3-1}$ is each independently selected from deuterium or hydroxyl;

$R^{3-2}$ is each independently selected from the group consisting of deuterium, oxo, halogen, $C_1$-$C_6$ alkoxy, -N$R^{3-4-2}R^{3-4-2}$, -S(O)$_2R^{3-4-1}$, hydroxyl and $C_1$-$C_6$ alkyl unsubstituted or substituted by one or more $R^{3-2-2}$;

$R^{3-3}$ is each independently selected from the group consisting of deuterium, oxo, amino and hydroxyl;

$R^{3-4}$ is each independently selected from the group consisting of deuterium, oxo and $C_1$-$C_6$ alkyl;

$R^{3-4-1}$ is $C_1$-$C_6$ alkyl;

$R^{3-4-2}$ is each independently selected from the group consisting of -S(O)$_2R^{3-4-1}$, hydrogen and $C_1$-$C_6$ alkyl;

$R^{3-5}$ is each independently selected from the group consisting of deuterium,

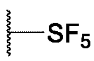, 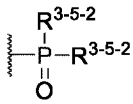

and

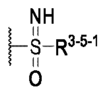;

$R^{3-5-1}$ is $C_1$-$C_6$ alkyl;

$R^{3-6}$ is each independently selected from the group consisting of deuterium, halogen, $C_1$-$C_6$ alkyl unsubstituted or substituted by one or more $R^{3-2-2}$ and 4-6 membered heterocyclyl unsubstituted or substituted by one or more $R^{3-6-1}$; the heteroatom in said 4-6 membered heterocyclyl is one or two selected from N, O, and S, the number of heteroatom(s) is 1 or 2;

$R^{3-5-2}$ is each independently selected from the group consisting of hydroxyl, $C_1$-$C_6$ alkyl, 1-10 membered heteroalkyl and two $R^{3-5-2}$s form 4-10 membered heterocyclyl; the heteroatom in said 1-10 membered heteroalkyl comprises, in addition to phosphorus, selected from the group consisting of N, O, and S, and the number of heteroatom(s) is 1, 2, 3 or 4; the heteroatom in said 4-10 membered heterocyclyl comprises, in addition to phosphorus, selected from the group consisting of N, O, and S, and the number of heteroatom(s) is 1, 2, 3 or 4;

$R^{3-6-1}$ is each independently selected from $C_1$-$C_6$ alkyl;

$R^{3-2-2}$ is each independently selected from deuterium or hydroxyl;

$R^{3-7}$ is each independently selected from the group consisting of deuterium, hydroxyl, -S(O)$_2R^{3-4-1}$ and 4-6 membered heterocyclyl; the heteroatom in said 4-6 membered heterocyclyl is O, and the number of heteroa-

tom(s) is 1;
$R^{3-8}$ is each independently selected from deuterium or hydroxyl;
$R^4$ is 4-6 membered heterocyclyl; the heteroatom in said 4-6 membered heterocyclyl is O, and the number of heteroatom(s) is 1;
$R^7$ is 3-6 membered cycloalkyl;
in the scheme 8',
L is

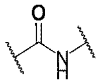;

the ring A is a 5-10 membered heteroaromatic ring; the heteroatom in said 5-10 membered heteroaromatic ring is N, and the number of heteroatom(s) is 1 or 2;
$R^1$ is 6-10 membered aryl unsubstituted or substituted by one or more $R^{1-1}$ or 5-6 membered heteroaryl unsubstituted or substituted by one or more $R^{1-2}$; the heteroatom in said 5-6 membered heteroaryl is N, and the number of heteroatom(s) is 1 or 2;
$R^{1-1}$ and $R^{1-2}$ are each independently selected from the group consisting of deuterium, halogen, $C_1$-$C_6$ alkyl unsubstituted or substituted by one or more $R^{1-1-1}$ and $C_1$-$C_6$ alkoxy unsubstituted or substituted by one or more $R^{1-1-2}$;
$R^{1-1-1}$ and $R^{1-1-2}$ are each independently selected from halogen or deuterium;
$R^{1-1-11}$ is each independently selected from hydroxyl or $C_1$-$C_6$ alkyl;
$R^2$ is independently deuterium or $C_1$-$C_6$ alkyl unsubstituted or substituted by one or more $R^{2-1}$;
$R^{2-1}$ is each independently selected from halogen or deuterium;
m is 0 or 1;
the ring B is a 5-12 membered fused heteroaromatic ring substituted by one or more $R^{b-1}$; the heteroatom in said 5-12 membered fused heteroaromatic ring is N and/or S, the number of heteroatom(s) is 1, 2, 3 or 4, and the number of rings in said 5-12 membered fused heteroaromatic ring is 2;
$R^{b-1}$ is each independently selected from halogen, or $C_1$-$C_6$ alkyl unsubstituted or substituted by one or more $R^{b-1-1}$;
$R^{b-1-1}$ is each independently selected from halogen;
$R^3$ is hydrogen, 3-6 membered cycloalkyl unsubstituted or substituted by one or more $R^{3-1}$, 4-12 membered heterocyclyl unsubstituted or substituted by one or more $R^{3-2}$, 4-10 membered cycloalkenyl unsubstituted or substituted by one or more $R^{3-3}$, 4-10 membered heterocycloalkenyl unsubstituted or substituted by one or more $R^{3-4}$, 6-10 membered aryl unsubstituted or substituted by one or more $R^{3-5}$, 5-10 membered heteroaryl unsubstituted or substituted by one or more $R^{3-6}$, -C(O)$R^4$, $C_1$-$C_6$ alkyl unsubstituted or substituted by one or more $R^{3-7}$ or -O$R^7$ unsubstituted or substituted by one or more $R^{3-8}$; the heteroatom in said 4-12 membered heterocyclyl or 4-10 membered heterocycloalkenyl is selected from the group consisting of N, O, and S, and the number of heteroatom(s) is 1, or 2; the heteroatom in said 5-10 membered heteroaryl is N and/or O, and the number of heteroatom(s) is 1, 2, 3 or 4;
$R^{3-1}$ is each independently selected from deuterium or hydroxyl;
$R^{3-2}$ is each independently selected from the group consisting of deuterium, oxo, halogen, $C_1$-$C_6$ alkoxy, -N$R^{3-4-2}R^{3-4-2}$, -S(O)$_2R^{3-4-1}$, hydroxyl and $C_1$-$C_6$ alkyl unsubstituted or substituted by one or more $R^{3-2-2}$;
$R^{3-3}$ is each independently selected from the group consisting of deuterium, oxo, amino and hydroxyl;
$R^{3-4}$ is each independently selected from the group consisting of deuterium, oxo and $C_1$-$C_6$ alkyl;
$R^{3-4-1}$ is $C_1$-$C_6$ alkyl;
$R^{3-4-2}$ is each independently selected from the group consisting of -S(O)$_2R^{3-4-1}$, hydrogen and $C_1$-$C_6$ alkyl;
$R^{3-5}$ is each independently selected from the group consisting of deuterium,

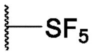, 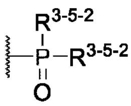

and

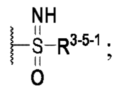

$R^{3-5-1}$ is $C_1$-$C_6$ alkyl;
$R^{3-6}$ is each independently selected from the group consisting of deuterium, halogen, $C_1$-$C_6$ alkyl unsubstituted or substituted by one or more $R^{3-2-2}$ and 4-6 membered heterocyclyl unsubstituted or substituted by one or more $R^{3-6-1}$; the heteroatom in said 4-6 membered heterocyclyl is one or two selected from N, O, and S, the number of heteroatom(s) is 1 or 2;
$R^{3-5-2}$ is each independently selected from the group consisting of hydroxyl, $C_1$-$C_6$ alkyl, 1-10 membered heteroalkyl and two $R^{3-5-2}$s form 4-10 membered heterocyclyl; the heteroatom in said 1-10 membered heteroalkyl comprises, in addition to phosphorus, selected from the group consisting of N, O, and S, and the number of heteroatom(s) is 1, 2, 3 or 4; the heteroatom in said 4-10 membered heterocyclyl comprises, in addition to phosphorus, selected from the group consisting of N, O, and S, and the number of heteroatom(s) is 1, 2, 3 or 4;
$R^{3-6-1}$ is each independently selected from $C_1$-$C_6$ alkyl;
$R^{3-2-2}$ is each independently selected from deuterium or hydroxyl;
$R^{3-7}$ is each independently selected from the group consisting of deuterium, hydroxyl, $-S(O)_2R^{3-4-1}$ and 4-6 membered heterocyclyl; the heteroatom in said 4-6 membered heterocyclyl is O, and the number of heteroatom(s) is 1;
$R^{3-8}$ is each independently selected from deuterium or hydroxyl;
$R^4$ is 4-6 membered heterocyclyl; the heteroatom in said 4-6 membered heterocyclyl is O, and the number of heteroatom(s) is 1;
$R^7$ is 3-6 membered cycloalkyl;
in the scheme 9,
L is

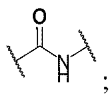

the ring A is a 5-6 membered heteroaromatic ring; the heteroatom in said 5-6 membered heteroaromatic ring is N, and the number of heteroatom(s) is 1;
$R^1$ is 5-6 membered heteroaryl unsubstituted or substituted by one or more $R^{1-2}$; the heteroatom in said 5-6 membered heteroaryl is N, and the number of heteroatom(s) is 1 or 2;
$R^{1-2}$ is each independently selected from the group consisting of deuterium, halogen, $C_1$-$C_6$ alkyl unsubstituted or substituted by one or more $R^{1-1-1}$, $C_1$-$C_6$ alkoxy unsubstituted or substituted by one or more $R^{1-1-2}$ and

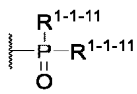

;

$R^{1-1-1}$ and $R^{1-1-2}$ are each independently selected from halogen or deuterium;
$R^{1-1-11}$ is each independently selected from hydroxyl or $C_1$-$C_6$ alkyl;
$R^2$ is independently deuterium or $C_1$-$C_6$ alkyl unsubstituted or substituted by one or more $R^{2-1}$;
$R^{2-1}$ is each independently selected from halogen or deuterium;
m is 0 or 1;
the ring B is a 5-12 membered fused heteroaromatic ring substituted by one or more $R^{b-1}$; the heteroatom in said 5-12 membered fused heteroaromatic ring is N and/or S, the number of heteroatom(s) is 1, 2, 3 or 4, and the number of rings in said 5-12 membered fused heteroaromatic ring is 2;
$R^{b-1}$ is each independently selected from halogen, or $C_1$-$C_6$ alkyl unsubstituted or substituted by one or more $R^{b-1-1}$;
$R^{b-1-1}$ is each independently selected from halogen;
$R^3$ is 3-6 membered cycloalkyl unsubstituted or substituted by one or more $R^{3-1}$, 4-9 membered heterocyclyl unsubstituted or substituted by one or more $R^{3-2}$, 4-6 membered cycloalkenyl unsubstituted or substituted by one or more $R^{3-3}$, 4-6 membered heterocycloalkenyl unsubstituted or substituted by one or more $R^{3-4}$, phenyl unsubstituted or substituted by one or more $R^{3-5}$, or 5-10 membered heteroaryl unsubstituted or substituted by one or more $R^{3-6}$; the heteroatom in said 4-9 membered heterocyclyl or 4-6 membered heterocycloalkenyl is N

and/or O, and the number of heteroatom(s) is 1 or 2; the heteroatom in said 5-10 membered heteroaryl is selected from N, and the number of heteroatom(s) is 1, 2, 3 or 4;
$R^{3-1}$ is each independently selected from deuterium or hydroxyl;
$R^{3-2}$ is each independently selected from the group consisting of deuterium, oxo, halogen, $C_1$-$C_6$ alkoxy, $-NR^{3-4-2}R^{3-4-2}$, $-S(O)_2R^{3-4-1}$, hydroxyl and $C_1$-$C_6$ alkyl unsubstituted or substituted by one or more $R^{3-2-2}$;
$R^{3-3}$ is each independently selected from the group consisting of deuterium, oxo, amino and hydroxyl;
$R^{3-4}$ is each independently selected from deuterium, oxo or $C_1$-$C_6$ alkyl;
$R^{3-4-1}$ is $C_1$-$C_6$ alkyl;
$R^{3-4-2}$ is each independently selected from the group consisting of $-S(O)_2R^{3-4-1}$, hydrogen and $C_1$-$C_6$ alkyl;
$R^{3-5}$ is each independently selected from the group consisting of deuterium,

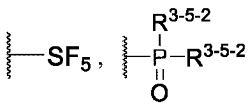

and

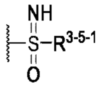

;

$R^{3-5-1}$ is $C_1$-$C_6$ alkyl;
$R^{3-6}$ is each independently selected from the group consisting of deuterium, halogen, $C_1$-$C_6$ alkyl unsubstituted or substituted by one or more $R^{3-2-2}$ and 4-6 membered heterocyclyl unsubstituted or substituted by one or more $R^{3-6-1}$; the heteroatom in said 4-6 membered heterocyclyl is one or two selected from N, O, and S, the number of heteroatom(s) is 1 or 2;
$R^{3-5-2}$ is each independently selected from the group consisting of hydroxyl, $C_1$-$C_6$ alkyl, 1-10 membered heteroalkyl and two $R^{3-5-2}$s form 4-10 membered heterocyclyl; the heteroatom in said 1-10 membered heteroalkyl comprises, in addition to phosphorus, selected from the group consisting of N, O, and S, and the number of heteroatom(s) is 1, 2, 3 or 4; the heteroatom in said 4-10 membered heterocyclyl comprises, in addition to phosphorus, selected from the group consisting of N, O, and S, and the number of heteroatom(s) is 1, 2, 3 or 4;
$R^{3-6-1}$ is each independently selected from $C_1$-$C_6$ alkyl;
$R^{3-2-2}$ is each independently selected from deuterium or hydroxyl;
in the scheme 9',
L is

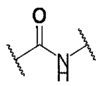

;

the ring A is a 5-6 membered heteroaromatic ring; the heteroatom in said 5-6 membered heteroaromatic ring is N, and the number of heteroatom(s) is 1;
$R^1$ is 5-6 membered heteroaryl unsubstituted or substituted by one or more $R^{1-2}$; the heteroatom in said 5-6 membered heteroaryl is N, and the number of heteroatom(s) is 1 or 2;
$R^{1-2}$ is each independently selected from the group consisting of deuterium, halogen, $C_1$-$C_6$ alkyl unsubstituted or substituted by one or more $R^{1-1-1}$ and $C_1$-$C_6$ alkoxy unsubstituted or substituted by one or more $R^{1-1-2}$;
$R^{1-1-1}$ and $R^{1-1-2}$ are each independently selected from halogen or deuterium;
$R^{1-1-11}$ is each independently selected from hydroxyl or $C_1$-$C_6$ alkyl;
$R^2$ is independently deuterium or $C_1$-$C_6$ alkyl unsubstituted or substituted by one or more $R^{2-1}$;
$R^{2-1}$ is each independently selected from halogen or deuterium;
m is 0 or 1;
the ring B is a 5-12 membered fused heteroaromatic ring substituted by one or more $R^{b-1}$; the heteroatom in said 5-12 membered fused heteroaromatic ring is N and/or S, the number of heteroatom(s) is 1, 2, 3 or 4, and the number of rings in said 5-12 membered fused heteroaromatic ring is 2;
$R^{b-1}$ is each independently selected from halogen, or $C_1$-$C_6$ alkyl unsubstituted or substituted by one or more $R^{b-1-1}$;
$R^{b-1-1}$ is each independently selected from halogen;

$R^3$ is 3-6 membered cycloalkyl unsubstituted or substituted by one or more $R^{3-1}$, 4-9 membered heterocyclyl unsubstituted or substituted by one or more $R^{3-2}$, 4-6 membered cycloalkenyl unsubstituted or substituted by one or more $R^{3-3}$, 4-6 membered heterocycloalkenyl unsubstituted or substituted by one or more $R^{3-4}$, phenyl unsubstituted or substituted by one or more $R^{3-5}$, or 5-10 membered heteroaryl unsubstituted or substituted by one or more $R^{3-6}$; the heteroatom in said 4-9 membered heterocyclyl or 4-6 membered heterocycloalkenyl is N and/or O, and the number of heteroatom(s) is 1 or 2; the heteroatom in said 5-10 membered heteroaryl is selected from N, and the number of heteroatom(s) is 1, 2, 3 or 4;
$R^{3-1}$ is each independently selected from deuterium or hydroxyl;
$R^{3-2}$ is each independently selected from the group consisting of deuterium, oxo, halogen, $C_1$-$C_6$ alkoxy, $-NR^{3-2}R^{3-4-2}$, $-S(O)_2R^{3-4-1}$, hydroxyl and $C_1$-$C_6$ alkyl unsubstituted or substituted by one or more $R^{3-2-2}$;
$R^{3-3}$ is each independently selected from the group consisting of deuterium, oxo, amino and hydroxyl;
$R^{3-4}$ is each independently selected from the group consisting of deuterium, oxo and $C_1$-$C_6$ alkyl;
$R^{3-4-1}$ is $C_1$-$C_6$ alkyl;
$R^{3-4-2}$ is each independently selected from the group consisting of $-S(O)_2R^{3-4-1}$, hydrogen and $C_1$-$C_6$ alkyl;
$R^{3-5}$ is each independently selected from the group consisting of deuterium,

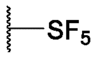 , 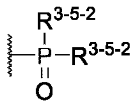

and

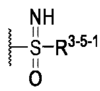 ;

$R^{3-5-1}$ is $C_1$-$C_6$ alkyl;
$R^{3-6}$ is each independently selected from the group consisting of deuterium, halogen, $C_1$-$C_6$ alkyl unsubstituted or substituted by one or more $R^{3-2-2}$ and 4-6 membered heterocyclyl unsubstituted or substituted by one or more $R^{3-6-1}$; the heteroatom in said 4-6 membered heterocyclyl is one or two selected from N, O, and S, the number of heteroatom(s) is 1 or 2;
$R^{3-5-2}$ is each independently selected from the group consisting of hydroxyl, $C_1$-$C_6$ alkyl,1-10 membered heteroalkyl and two $R^{3-5-2}$s form 4-10 membered heterocyclyl; the heteroatom in said 1-10 membered heteroalkyl comprises, in addition to phosphorus, selected from the group consisting of N, O, and S, and the number of heteroatom(s) is 1, 2, 3 or 4; the heteroatom in said 4-10 membered heterocyclyl comprises, in addition to phosphorus, selected from the group consisting of N, O, and S, and the number of heteroatom(s) is 1, 2, 3 or 4;
$R^{3-6-1}$ is each independently selected from $C_1$-$C_6$ alkyl;
$R^{3-2-2}$ is each independently selected from deuterium or hydroxyl;
in the scheme 10,
m is 0 or 1; n is 1;
L is

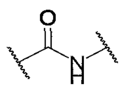 ;

the ring A is a 5-6 membered heteroaromatic ring; the heteroatom in said 5-6 membered heteroaromatic ring is N, and the number of heteroatom(s) is 1;
$R^1$ is 5-6 membered heteroaryl unsubstituted or substituted by one or more $R^{1-2}$; the heteroatom in said 5-6 membered heteroaryl is N, and the number of heteroatom(s) is 1, 2 or 3;
$R^{1-2}$ is each independently selected from the group consisting of deuterium, halogen, cyano, amino, hydroxyl, $C_1$-$C_6$ alkyl unsubstituted or substituted by one or more $R^{1-1-1}$, $C_1$-$C_6$ alkoxy unsubstituted or substituted by one or more $R^{1-1-2}$, $C_2$-$C_6$ alkenyl unsubstituted or substituted by one or more $R^{1-1-3}$ and $C_2$-$C_6$ alkynyl unsubstituted or substituted by one or more $R^{1-1-4}$;
$R^{1-1-1}$, $R^{1-1-2}$, $R^{1-1-3}$ and $R^{1-1-4}$ are each independently selected from the group consisting of deuterium, hydroxyl, cyano and halogen;
$R^2$ is each independently selected from the group consisting of deuterium, halogen, cyano, amino, hydroxyl,

$C_1$-$C_6$ alkyl unsubstituted or substituted by one or more $R^{2-1}$, $C_1$-$C_6$ alkoxy unsubstituted or substituted by one or more $R^{2-2}$, $C_2$-$C_6$ alkenyl unsubstituted or substituted by one or more $R^{2-3}$, $C_2$-$C_6$ alkynyl unsubstituted or substituted by one or more $R^{2-4}$, 3-6 membered cycloalkyl unsubstituted or substituted by one or more $R^{2-5}$ and 4-6 membered heterocyclyl unsubstituted or substituted by one or more $R^{2-6}$; the heteroatom in said 4-12 membered heterocyclyl is one or more selected from N, and the number of heteroatom(s) is 1, 2, or 3;
$R^{2-1}$, $R^{2-2}$, $R^{2-3}$, $R^{2-4}$, $R^{2-5}$ and $R^{2-6}$ are each independently selected from the group consisting of deuterium, hydroxyl, cyano and halogen;
the ring B is a 5-20 membered fused heteroaromatic ring unsubstituted or substituted by one or more $R^{b-1}$; the heteroatom in said 5-20 membered fused heteroaromatic ring is selected from the group consisting of N, O, and S, the number of heteroatom(s) is 1, 2, 3, 4, 5, 6, 7 or 8, a single heteroaromatic ring in said 5-20 membered fused heteroaromatic ring is a 5-7 membered heteroaromatic ring, and the number of rings in said 5-20 membered fused heteroaromatic ring is 2 or 3; and when the ring number in said 5-20 membered fused heteroaromatic ring is 2, the ring B is a 5-20 membered fused heteroaromatic ring substituted by one or more $R^{b-1}$;
$R^{b-1}$ is each independently selected from halogen, or $C_1$-$C_6$ alkyl unsubstituted or substituted by one or more $R^{b-1-1}$;
$R^{b-1-1}$ is each independently selected from hydroxyl or halogen;
$R^3$ is hydrogen, 3-12 membered cycloalkyl unsubstituted or substituted by one or more $R^{3-1}$, 4-12 membered heterocyclyl unsubstituted or substituted by one or more $R^{3-2}$, 4-12 membered cycloalkenyl unsubstituted or substituted by one or more $R^{3-3}$, 4-12 membered heterocycloalkenyl unsubstituted or substituted by one or more $R^{3-4}$, 6-10 membered aryl unsubstituted or substituted by one or more $R^{3-5}$, 5-10 membered heteroaryl unsubstituted or substituted by one or more $R^{3-6}$, -C(O)$R^4$, $C_1$-$C_6$ alkyl unsubstituted or substituted by one or more $R^{3-7}$ or -O$R^7$ unsubstituted or substituted by one or more $R^{3-8}$; the heteroatom in said 4-12 membered heterocyclyl, 4-12 membered heterocycloalkenyl or 5-10 membered heteroaryl is selected from the group consisting of N, O, and S, and the number of heteroatom(s) is 1, 2, 3 or 4;
$R^{3-1}$, $R^{3-2}$, $R^{3-3}$ and $R^{3-4}$ are each independently selected from the group consisting of deuterium, hydroxyl, halogen, cyano, amino, -NHC(O)$R^{3-2-1}$, $C_1$-$C_6$ alkyl unsubstituted or substituted by one or more $R^{3-2-2}$, $C_1$-$C_6$ alkoxy, $C_2$-$C_6$ alkenyl, $C_2$-$C_6$ alkynyl, oxo(=O), -C(O)$NH_2$, 3-6 membered cycloalkyl unsubstituted or substituted by one or more $R^{3-2-3}$, 4-6 membered heterocyclyl, -S(O)$_2R^{3-4-1}$, -N$R^{3-4-2}R^{3-4-2}$,

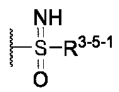

and

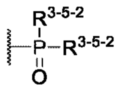 ;

the heteroatom in said 4-6 membered heterocyclyl is selected from the group consisting of N, O, and S, and the number of heteroatom(s) is 1 or 2; the heteroatom in said 5-6 membered heteroaryl is N, and the number of heteroatom(s) is 1 or 2;
$R^{3-7}$ is each independently selected from the group consisting of deuterium, hydroxyl, halogen, cyano and amino;
$R^{3-8}$ is each independently selected from hydroxyl or 4-6 membered heterocyclyl;
$R^{3-5}$ and $R^{3-6}$ are each independently selected from the group consisting of deuterium,

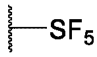 ,

hydroxyl, halogen, cyano, amino, -NHC(O)$R^{3-2-1}$, $C_1$-$C_6$ alkyl unsubstituted or substituted by one or more $R^{3-2-2}$, $C_2$-$C_6$ alkenyl, $C_2$-$C_6$ alkynyl, -C(O)$NH_2$, 3-6 membered cycloalkyl unsubstituted or substituted by one or more $R^{3-2-3}$, 4-6 membered heterocyclyl unsubstituted or substituted by one or more $R^{3-6-1}$,

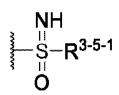

and

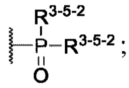

the heteroatom in said 4-6 membered heterocyclyl is selected from the group consisting of N, O, and S, and the number of heteroatom(s) is 1 or 2;
$R^{3-2-1}$ and $R^{3-4-2}$ are each independently selected from hydrogen or $C_1$-$C_6$ alkyl;
$R^{3-2-3}$ and $R^{3-5-1}$ are each independently selected from hydroxyl or $C_1$-$C_6$ alkyl;
$R^{3-5-2}$ is each independently selected from the group consisting of hydroxyl, $C_1$-$C_6$ alkyl, 1-10 membered heteroalkyl and two $R^{3-5-2}$s form 4-10 membered heterocyclyl; the heteroatom in said 1-10 membered heteroalkyl comprises, in addition to phosphorus, selected from the group consisting of N, O, and S, and the number of heteroatom(s) is 1, 2, 3 or 4; the heteroatom in said 4-10 membered heterocyclyl comprises, in addition to phosphorus, selected from the group consisting of N, O, and S, and the number of heteroatom(s) is 1, 2, 3 or 4;
$R^{3-6-1}$ is each independently selected from $C_1$-$C_6$ alkyl;
$R^{3-2-2}$ is each independently selected from the group consisting of deuterium, hydroxyl and halogen;
$R^{3-4-1}$ is hydroxyl, amino or $C_1$-$C_6$ alkyl;
$R^4$ is 4-6 membered heterocyclyl unsubstituted or substituted by one or more $R^{4-1}$; the heteroatom in said 4-6 membered heterocyclyl is selected from N, and the number of heteroatom(s) is 1 or 2;
$R^{4-1}$ is each independently selected from hydroxyl;
$R^7$ is 3-6 membered cycloalkyl;
in the scheme 11,
m is 0 or 1; n is 1;
L is

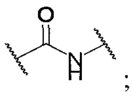

the ring A is a 5-6 membered heteroaromatic ring; the heteroatom in said 5-6 membered heteroaromatic ring is N, and the number of heteroatom(s) is 1;
$R^1$ is 5-6 membered heteroaryl unsubstituted or substituted by one or more $R^{1-2}$; the heteroatom in said 5-6 membered heteroaryl is N, and the number of heteroatom(s) is 1;
$R^{1-2}$ is each independently selected from the group consisting of deuterium, halogen and $C_1$-$C_6$ alkoxy unsubstituted or substituted by one or more $R^{1-1-2}$;
$R^{1-1-2}$ is each independently selected from the group consisting of deuterium, hydroxyl, cyano and halogen; $R^2$ is each independently selected from deuterium, or $C_1$-$C_6$ alkyl unsubstituted or substituted by one or more $R^{2-1}$;
$R^{2-1}$ is each independently selected from the group consisting of deuterium, hydroxyl, cyano and halogen; the ring B is a 5-20 membered fused heteroaromatic ring unsubstituted or substituted by one or more $R^{b-1}$; the heteroatom in said 5-20 membered fused heteroaromatic ring is selected from the group consisting of N, O, and S, the number of heteroatom(s) is 1, 2, 3, 4, 5, 6, 7 or 8, a single heteroaromatic ring in said 5-20 membered fused heteroaromatic ring is a 5-7 membered heteroaromatic ring, and the number of rings in said 5-20 membered fused heteroaromatic ring is 2 or 3; and when the ring number in said 5-20 membered fused heteroaromatic ring is 2, the ring B is a 5-20 membered fused heteroaromatic ring substituted by one or more $R^{b-1}$;
$R^{b-1}$ is each independently selected from halogen or $C_1$-$C_6$ alkyl;
$R^3$ is 3-12 membered cycloalkyl unsubstituted or substituted by one or more $R^{3-1}$, 4-12 membered heterocyclyl unsubstituted or substituted by one or more $R^{3-2}$, 4-12 membered cycloalkenyl unsubstituted or substituted by one or more $R^{3-3}$, 4-12 membered heterocycloalkenyl unsubstituted or substituted by one or more $R^{3-4}$, 6-10 membered aryl unsubstituted or substituted by one or more $R^{3-5}$, 5-10 membered heteroaryl unsubstituted or substituted by one or more $R^{3-6}$, or -$OR^7$ unsubstituted or substituted by one or more $R^{3-8}$; the heteroatom in each of said 4-12 membered heterocyclyl, said 4-12 membered heterocycloalkenyl and said 5-10 membered heteroaryl is selected from the group consisting of N, O, and S, and the number of heteroatom(s) is 1, 2, 3 or 4;
$R^{3-1}$, $R^{3-2}$, $R^{3-3}$ and $R^{3-4}$ are each independently selected from the group consisting of deuterium, hydroxyl, halogen, cyano, amino, -NHC(O)$R^{3-2-1}$, $C_1$-$C_6$ alkyl unsubstituted or substituted by one or more $R^{3-2-2}$, $C_1$-$C_6$ alkoxy, $C_2$-$C_6$ alkenyl, $C_2$-$C_6$ alkynyl, oxo(=O),

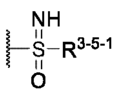 and 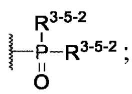

$R^{3-8}$ is each independently selected from hydroxyl or 4-6 membered heterocyclyl;
$R^{3-5}$ and $R^{3-6}$ are each independently selected from the group consisting of deuterium,

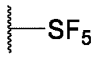 ,

hydroxyl, halogen, cyano, amino, $C_1$-$C_6$ alkyl unsubstituted or substituted by one or more $R^{3-2-2}$, $C_2$-$C_6$ alkenyl, $C_2$-$C_6$ alkynyl, -C(O)$NH_2$, 3-6 membered cycloalkyl unsubstituted or substituted by one or more $R^{3-2-3}$, 4-6 membered heterocyclyl unsubstituted or substituted by one or more $R^{3-6-1}$,

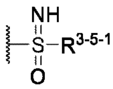 or 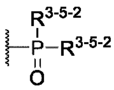 ;

the heteroatom in said 4-6 membered heterocyclyl is selected from the group consisting of N, O, and S, and the number of heteroatom(s) is 1 or 2;
$R^{3-2-1}$ is each independently selected from hydrogen or $C_1$-$C_6$ alkyl;
$R^{3-2-3}$ and $R^{3-5-1}$ are each independently selected from hydroxyl or $C_1$-$C_6$ alkyl;
$R^{3-5-2}$ is each independently selected from the group consisting of hydroxyl, $C_1$-$C_6$ alkyl or 1-10 membered heteroalkyl, and two $R^{3-5-2}$s form 4-10 membered heterocyclyl; the heteroatom in said 1-10 membered heteroalkyl comprises, in addition to phosphorus, selected from the group consisting of N, O, and S, and the number of heteroatom(s) is 1, 2, 3 or 4; the heteroatom in said 4-10 membered heterocyclyl comprises, in addition to phosphorus, selected from the group consisting of N, O, and S, and the number of heteroatom(s) is 1, 2, 3 or 4;
$R^{3-6-1}$ is each independently selected from $C_1$-$C_6$ alkyl;
$R^{3-2-2}$ is each independently selected from the group consisting of deuterium, hydroxyl and halogen;
$R^7$ is 3-6 membered cycloalkyl;
in the scheme 12,
m is 0 or 1; n is 1;
L is

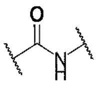 ;

the ring A is a 5-6 membered heteroaromatic ring; the heteroatom in said 5-6 membered heteroaromatic ring is N, and the number of heteroatom(s) is 1;
$R^1$ is 5-6 membered heteroaryl unsubstituted or substituted by one or more $R^{1-2}$; the heteroatom in said 5-6 membered heteroaryl is N, and the number of heteroatom(s) is 1;
$R^{1-2}$ is each independently selected from the group consisting of deuterium, halogen, and $C_1$-$C_6$ alkoxy unsubstituted or substituted by one or more $R^{1-1-2}$;
$R^{1-1-2}$ is each independently selected from the group consisting of deuterium, hydroxyl, cyano and halogen;
$R^2$ is each independently selected from deuterium, or $C_1$-$C_6$ alkyl unsubstituted or substituted by one or more $R^{2-1}$;
$R^{2-1}$ is each independently selected from the group consisting of deuterium, hydroxyl and cyano or halogen; the ring B is 5-12 membered fused heteroaromatic ring substituted by one or more $R^{b-1}$, the heteroatom in said 5-12 membered fused heteroaromatic ring is selected from N and S, and the number of heteroatom(s) is 1, 2, 3 or 4, (for example,

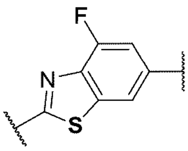 , 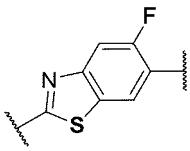 , 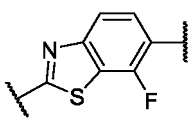 , 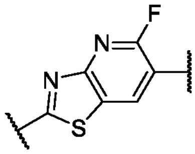 ,

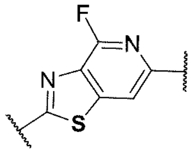, 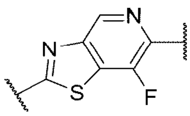 or 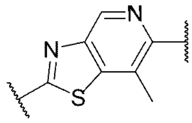

);

$R^{b-1}$ is each independently selected from halogen or $C_1$-$C_6$ alkyl (for example, fluorine or methyl);

$R^3$ is 3-6 membered cycloalkyl unsubstituted or substituted by one or more $R^{3-1}$, 4-6 membered heterocyclyl unsubstituted or substituted by one or more $R^{3-2}$, 4-6 membered cycloalkenyl unsubstituted or substituted by one or more $R^{3-3}$, 4-6 membered heterocycloalkenyl unsubstituted or substituted by one or more $R^{3-4}$, phenyl unsubstituted or substituted by one or more $R^{3-5}$, or 5-6 membered heteroaryl unsubstituted or substituted by one or more $R^{3-6}$; the heteroatom in said 4-6 membered heterocyclyl or 4-6 membered heterocycloalkenyl is N and/or O, and the number of heteroatom(s) is 1 or 2; the heteroatom in said 5-6 membered heteroaryl is N, and the number of heteroatom(s) is 1, 2 or 3;

$R^{3-1}$, $R^{3-2}$, $R^{3-3}$ and $R^{3-4}$ are each independently selected from the group consisting of deuterium, hydroxyl and $C_1$-$C_6$ alkyl;

$R^{3-5}$ is each independently selected from the group consisting of

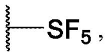, 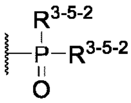 and 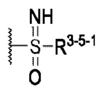;

$R^{3-5-1}$ is $C_1$-$C_6$ alkyl;

$R^{3-5-2}$ is each independently selected from hydroxyl or $C_1$-$C_6$ alkyl;

$R^{3-6}$ is each independently selected from halogen, or $C_1$-$C_6$ alkoxy unsubstituted or substituted by one or more $R^{3-2-2}$; and

$R^{3-2-2}$ is each independently selected from deuterium.

**11.** The compound of formula I, the pharmaceutically acceptable salt thereof, or the isotopic compound thereof according to claim 1, wherein the compound of formula I satisfies one or more of the following conditions:

(1) $R^1$ is

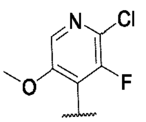, 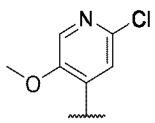, 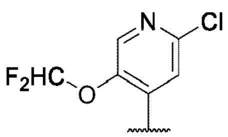, 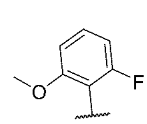, 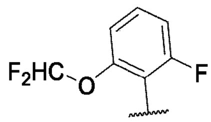,

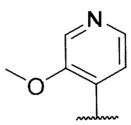, 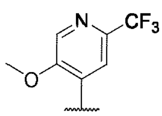, 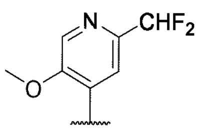, 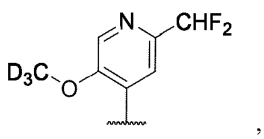, 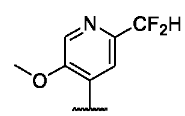,

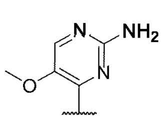, 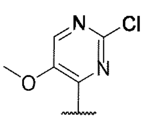, 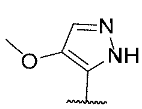, 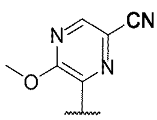, 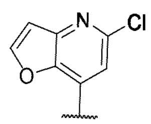, 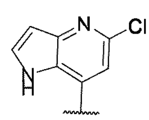,

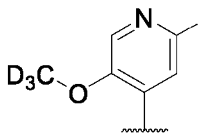 , 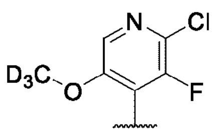 , 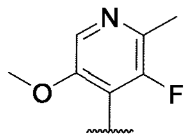 or 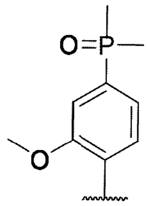 ;

(2)

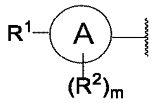

is

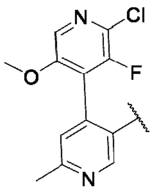 , 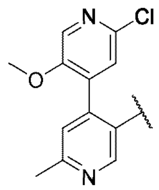 , 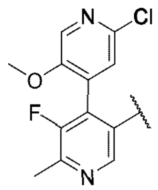 , 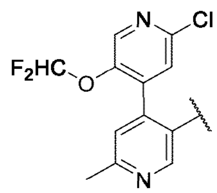 , 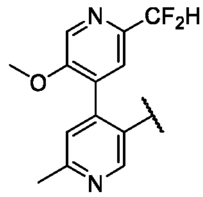 ,

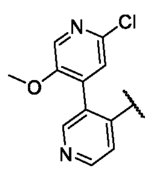 , 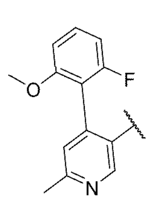 , 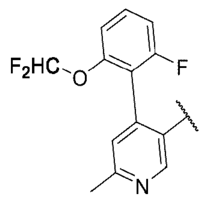 , 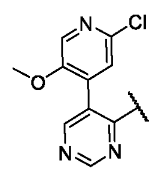 , 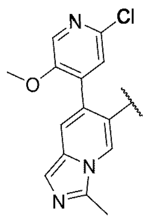 , 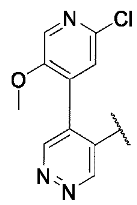 ,

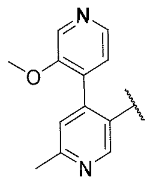 , 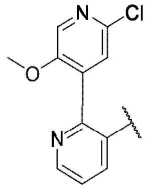 , 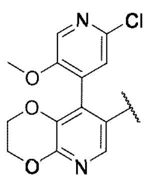 , 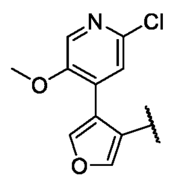 , 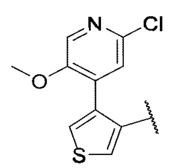 , 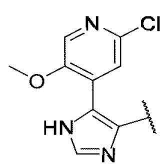 ,

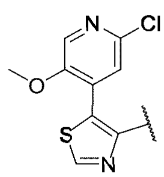 , 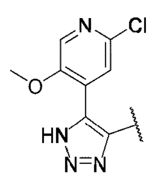 , 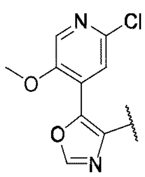 , 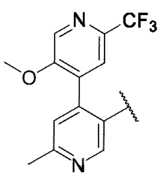 , 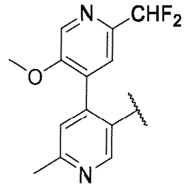 , 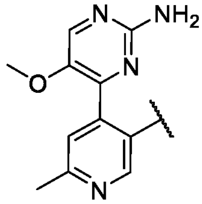 ,

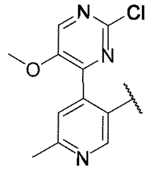 , 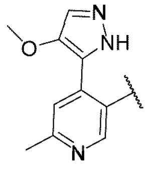 , 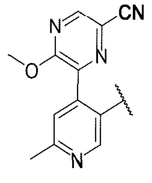 , 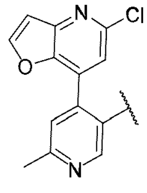 , 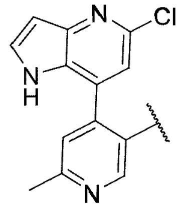 , 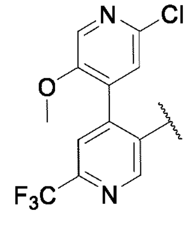 ,

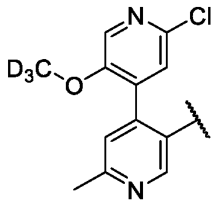, 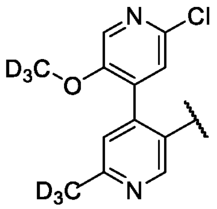, 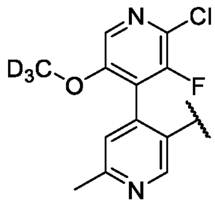, 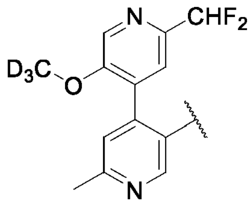,

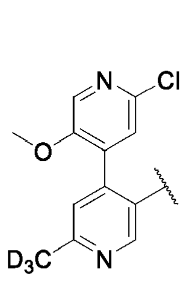, 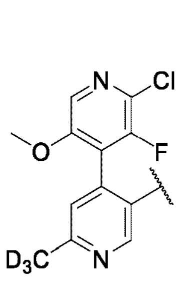, 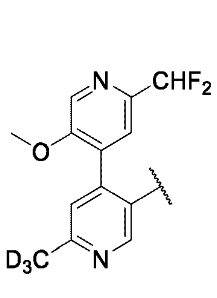, 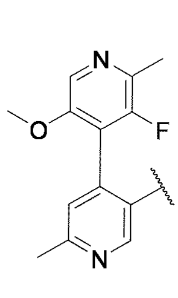, 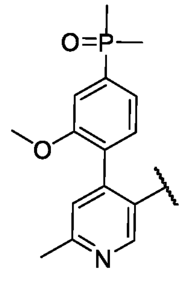,

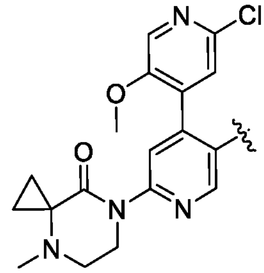, 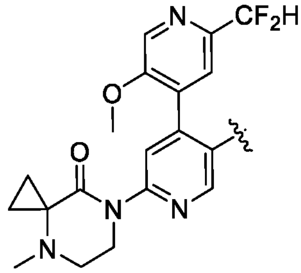 or 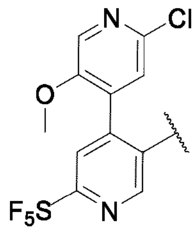;

(3)

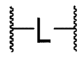

is

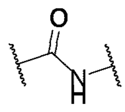;

(4)

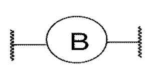

is

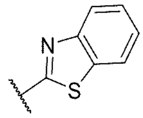, 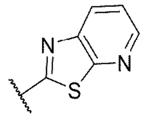, 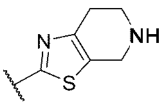, 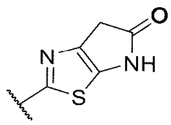, 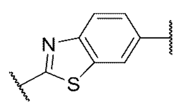,

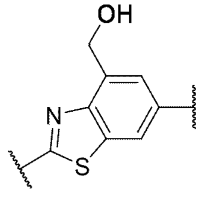, 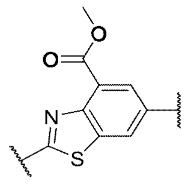, 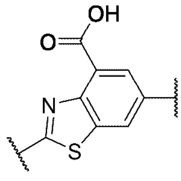, 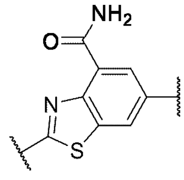, 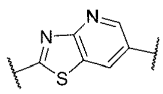,

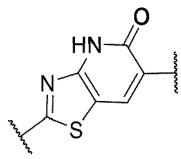, 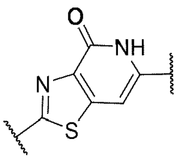, 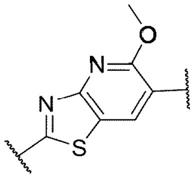, 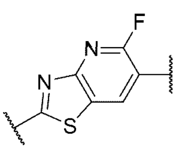, 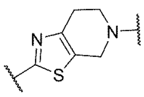, 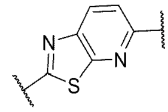,

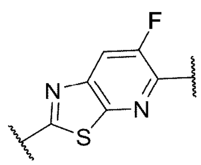, 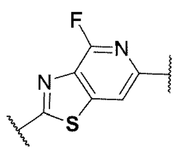, 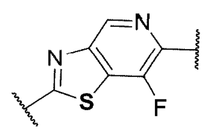, 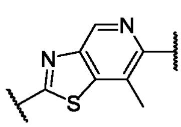, 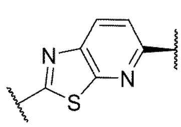,

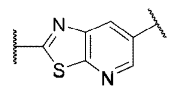, 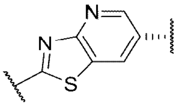, 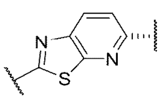, 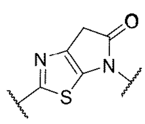, 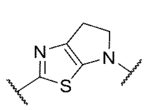, 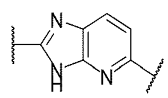,

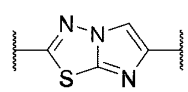, 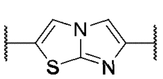, 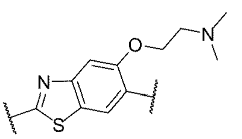, 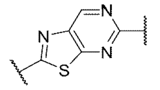, 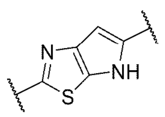,

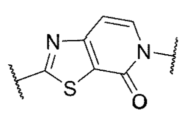, 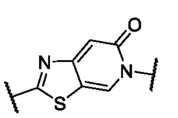, 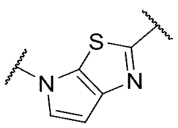, 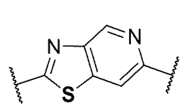, 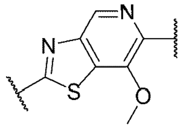,

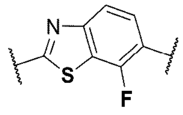, 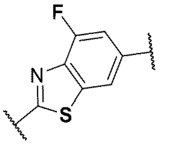, 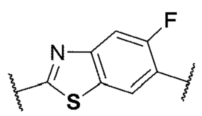, 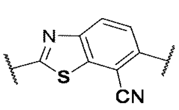, 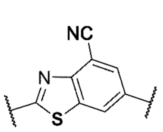,

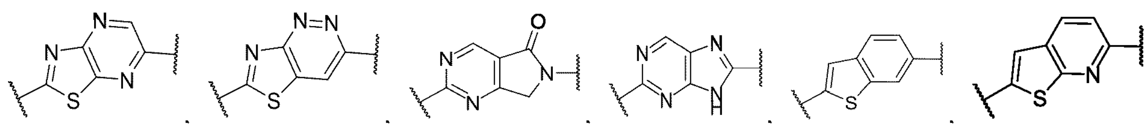, , , , , ,

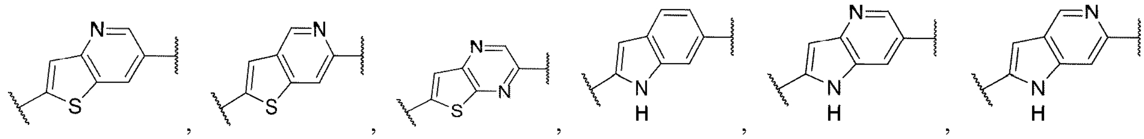, , , , , ,

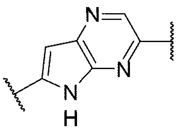, 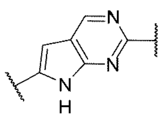, 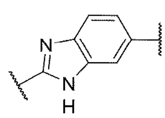, 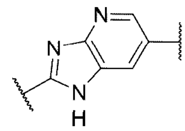, 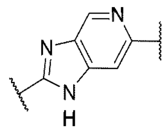, 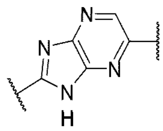,

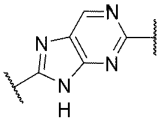, 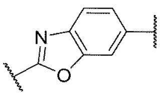, 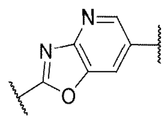, 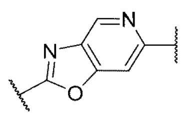, 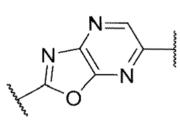, 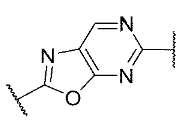,

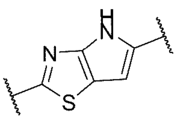, 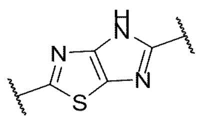, 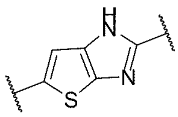, 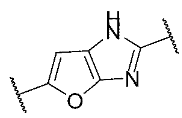, 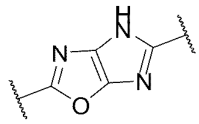,

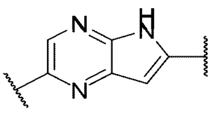, 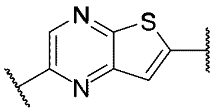, 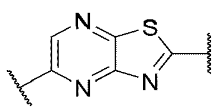, 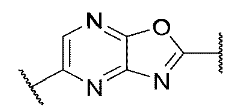,

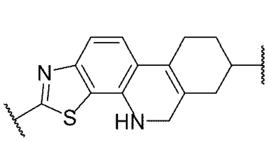, 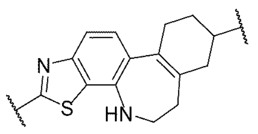, 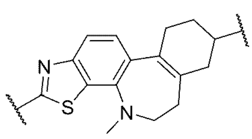, 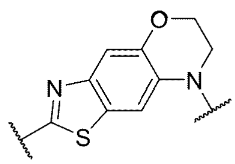,

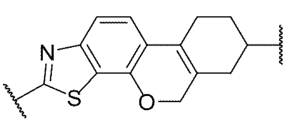, 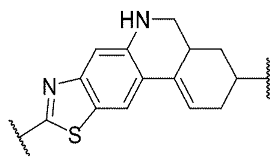, 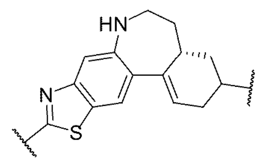, 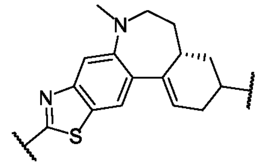,

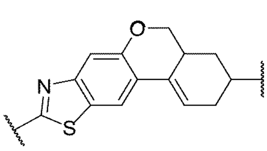, 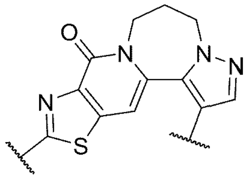, 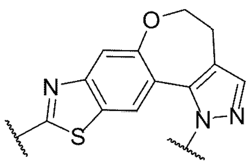, 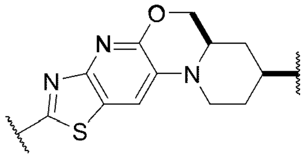,

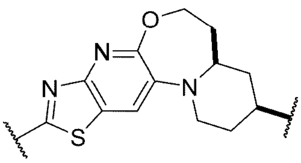, 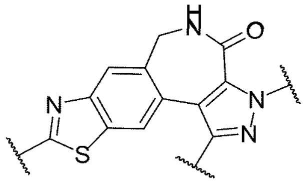, 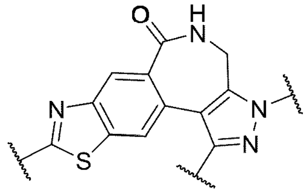,

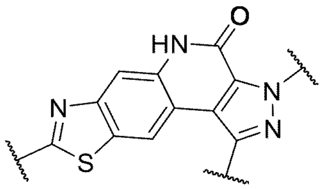, 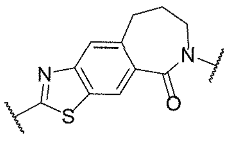 or 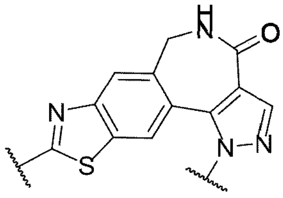;

and (5) $R^3$ is hydrogen, methyl,

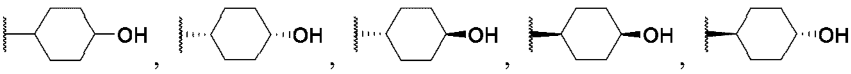,

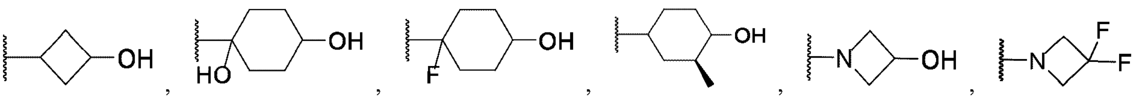,

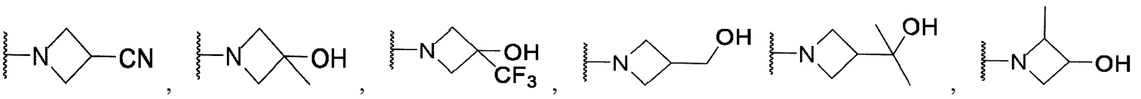,

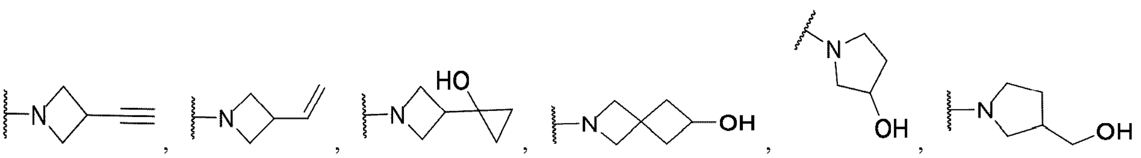,

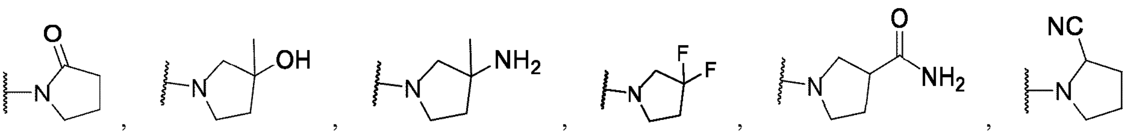,

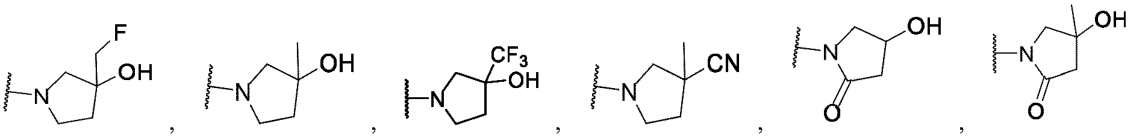,

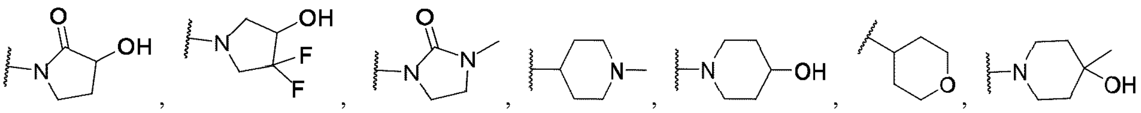,

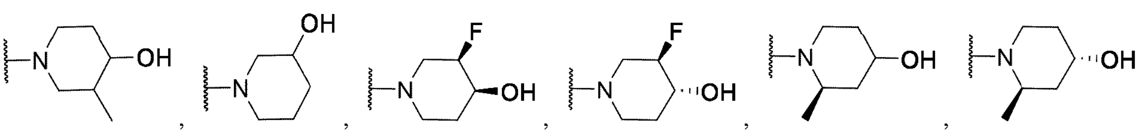,

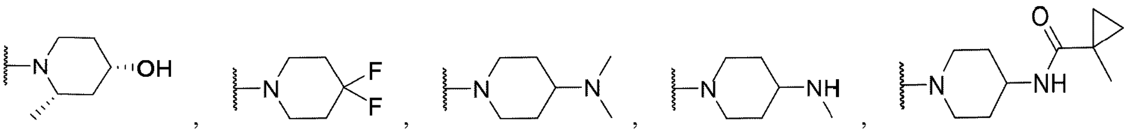,

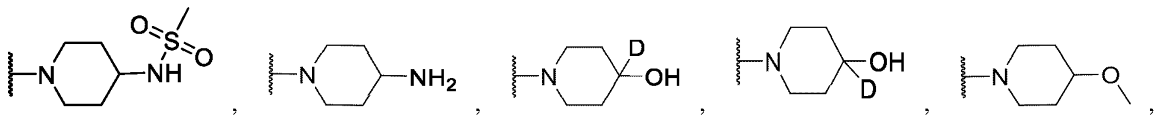

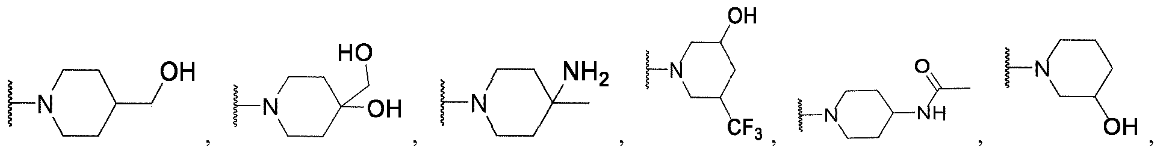

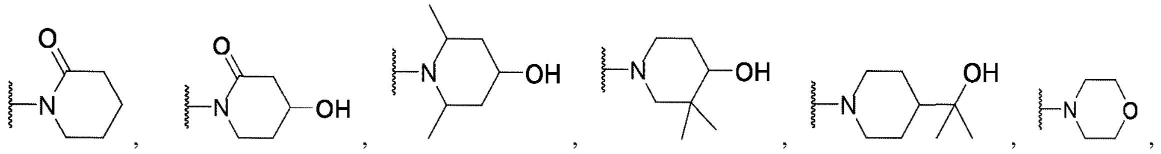

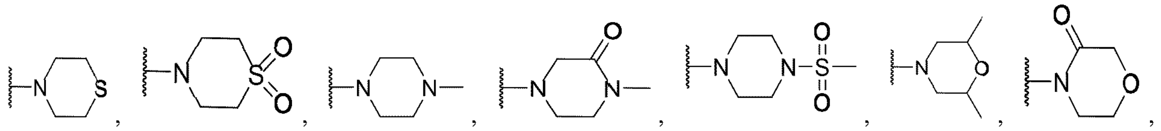

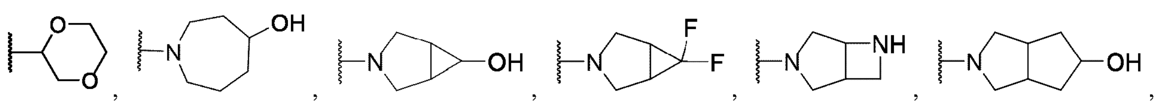

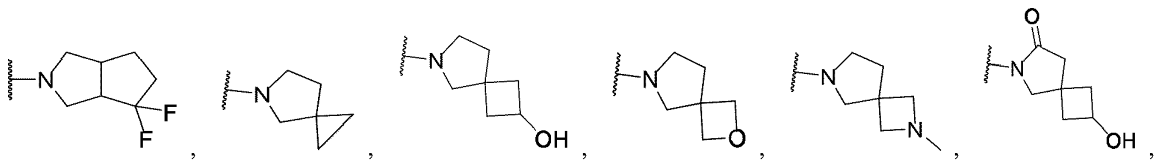

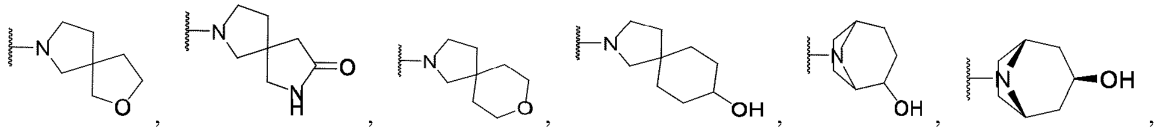

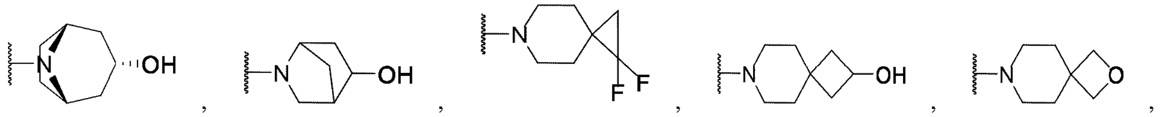

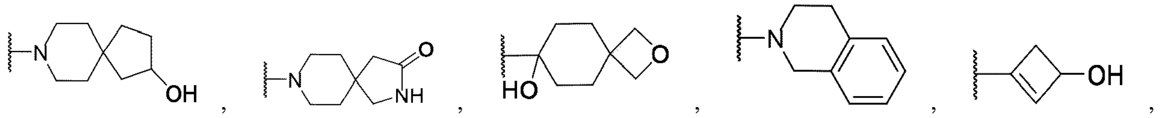

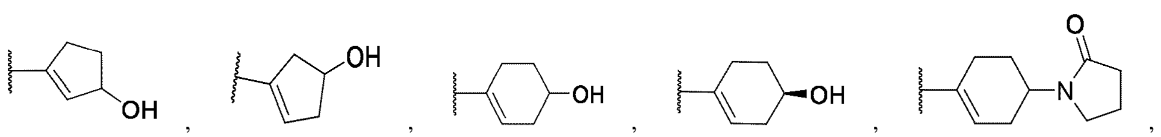

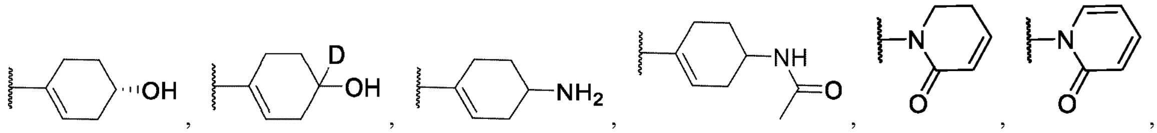
, , , , , ,

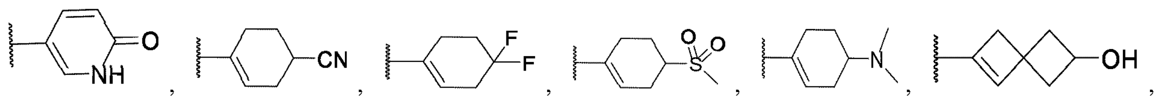
, , , , , ,

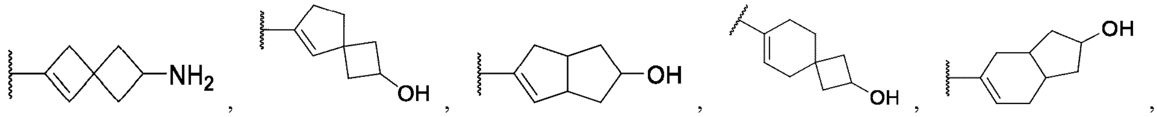
, , , , ,

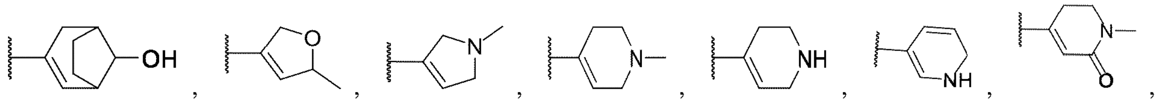
, , , , , , ,

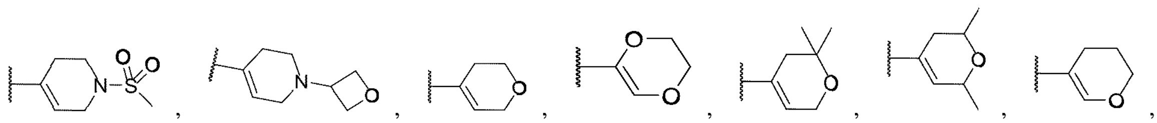
, , , , , , ,

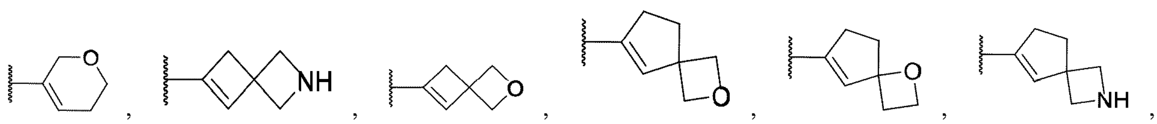
, , , , , ,

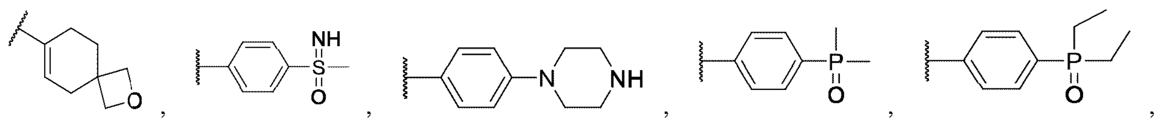
, , , , ,

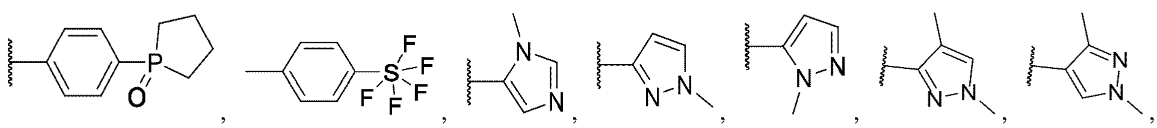
, , , , , , ,

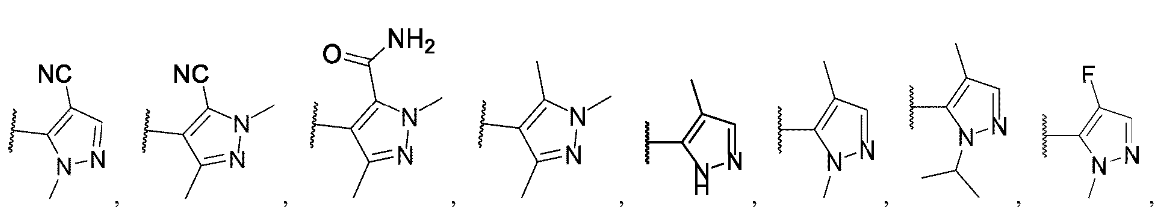
, , , , , , , ,

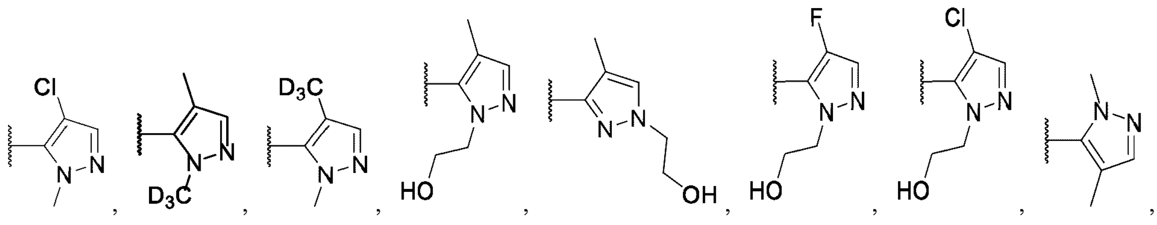
, , , , , , , ,

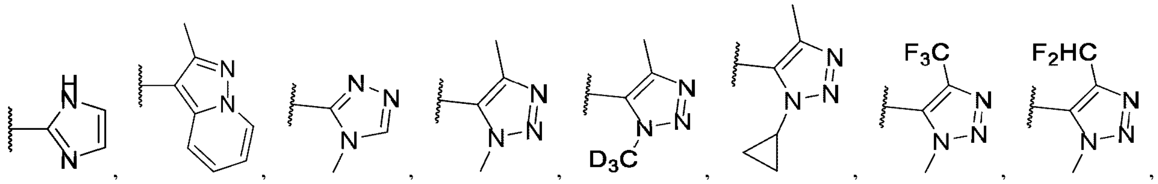

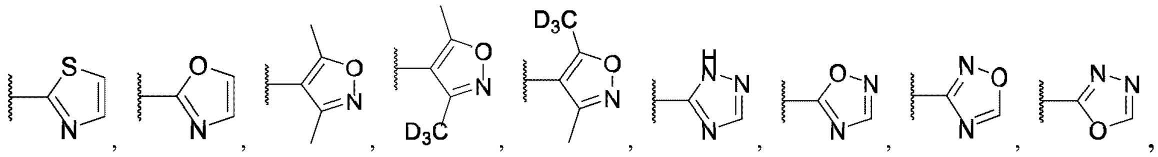

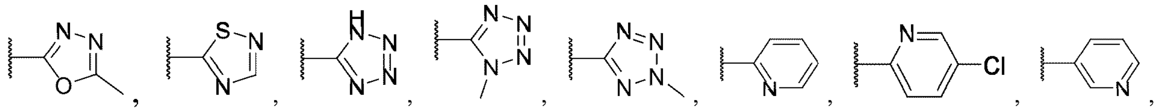

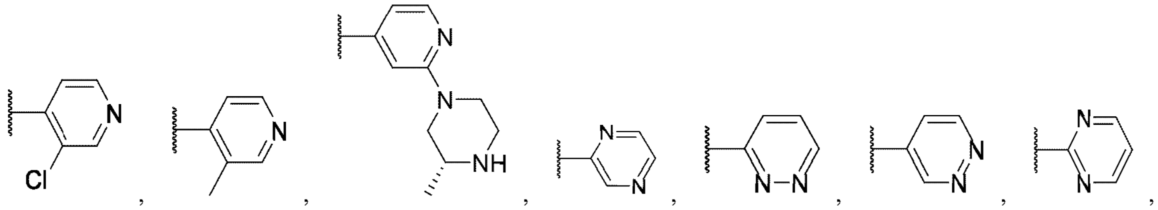

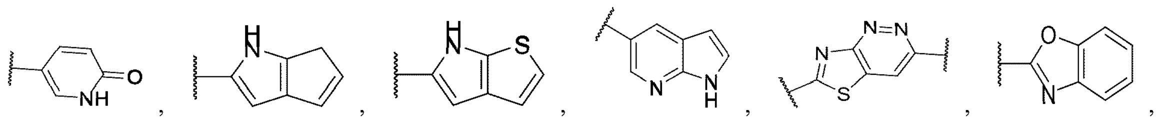

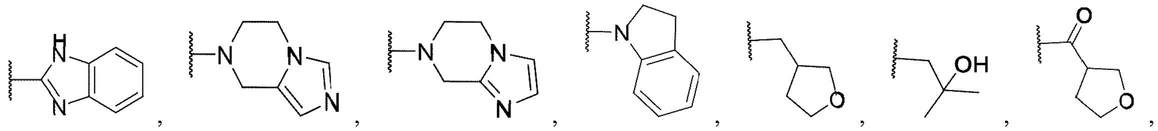

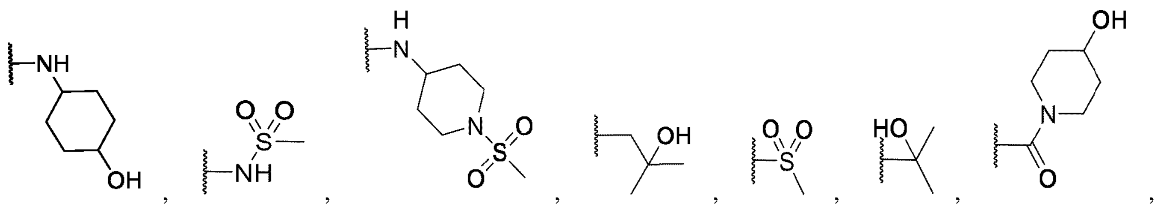

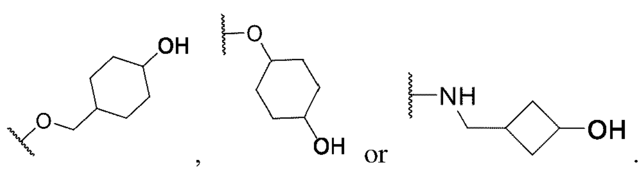

12. The compound of formula I, the pharmaceutically acceptable salt thereof, or the isotopic compound thereof according to claim 1, wherein the compound of formula I is any compound shown below:

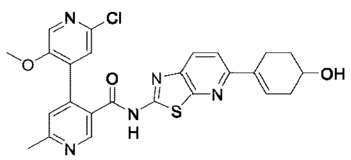 ,
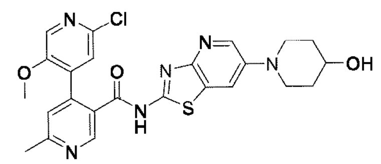 ,
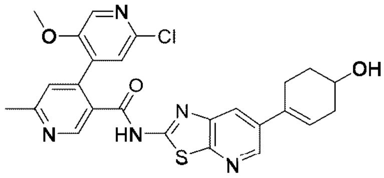 ,
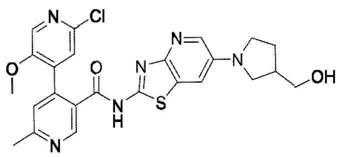 ,
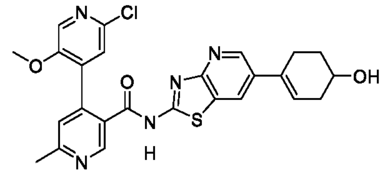 ,
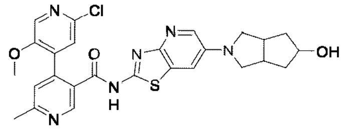 ,
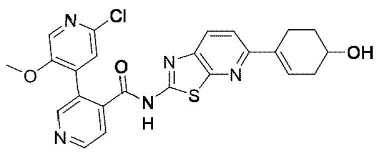 ,
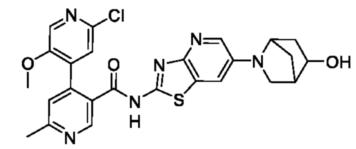 ,
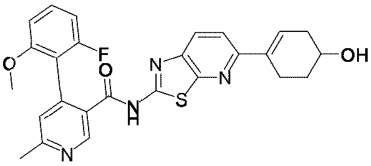 ,
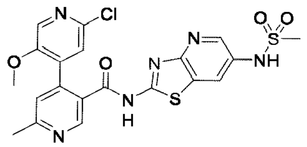 ,
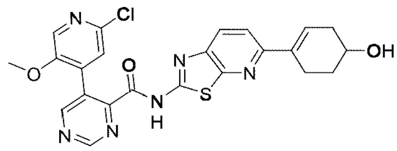 ,
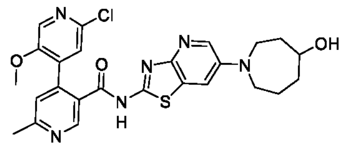 ,
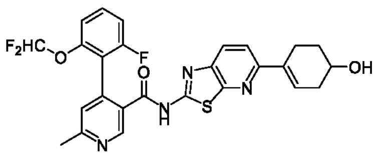 ,
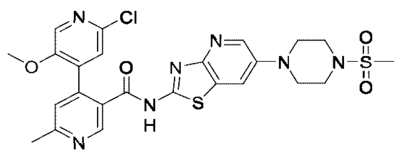 ,
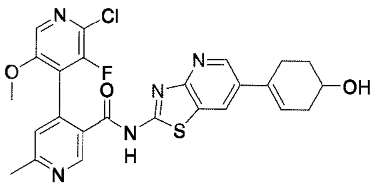 ,
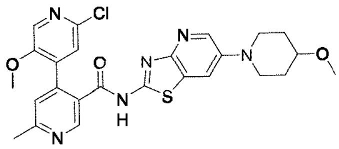 ,
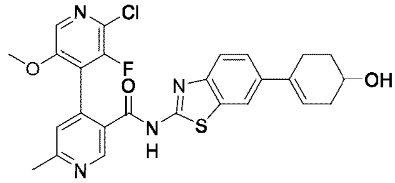 ,
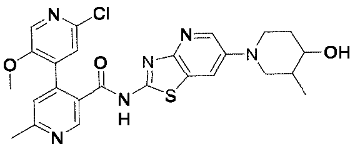 ,
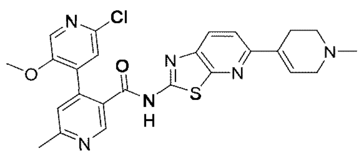 ,
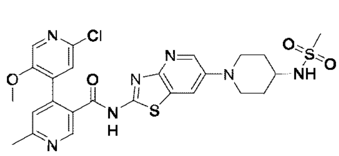 ,
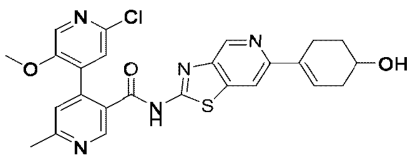 ,
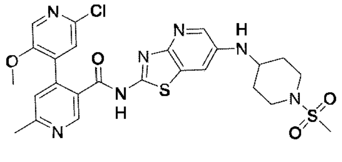 ,
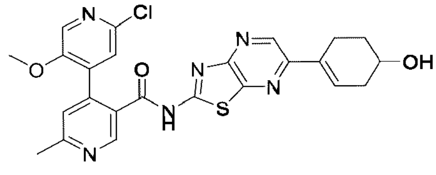 ,
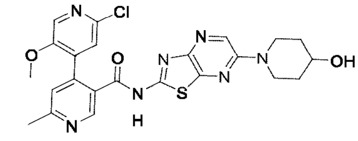 ,

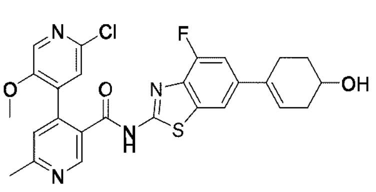,
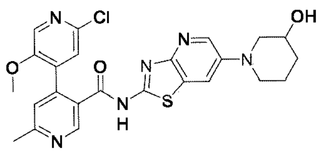,
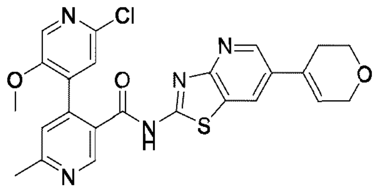,

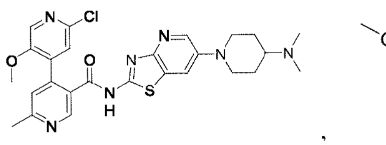,
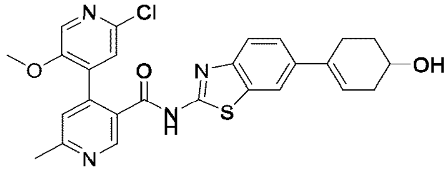,
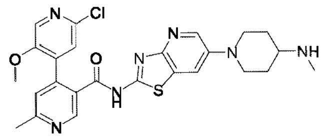,

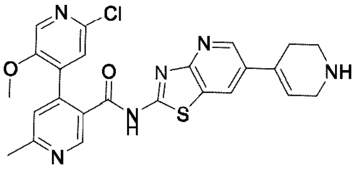,
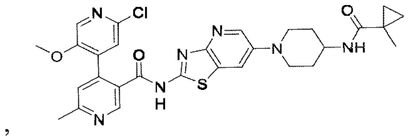,
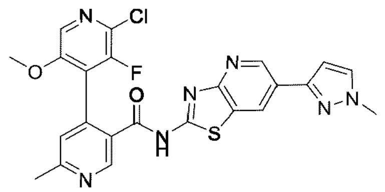,

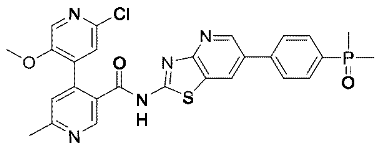,
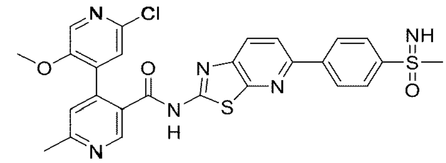,
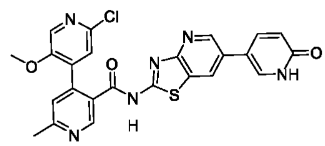,

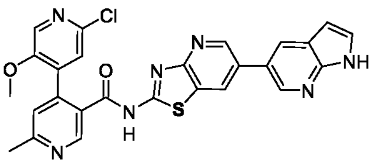,
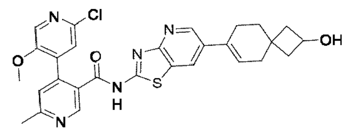,
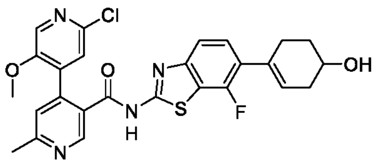,

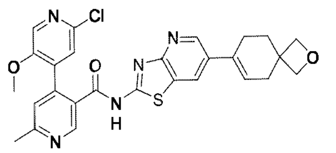,
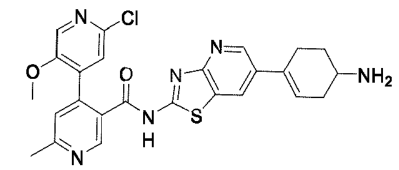,
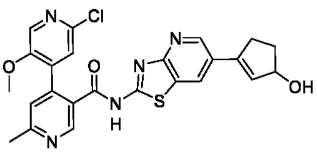,

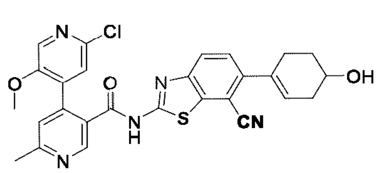,
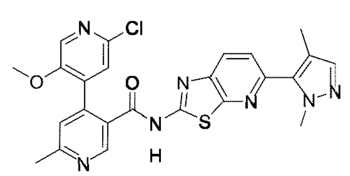,
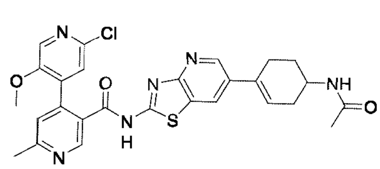,

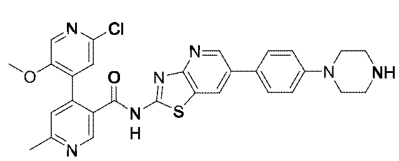,
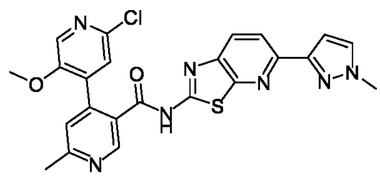,
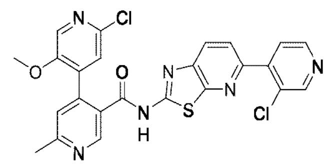,

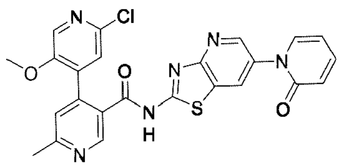 , 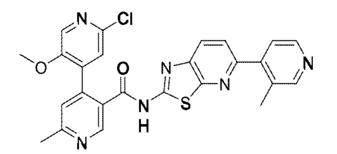 , 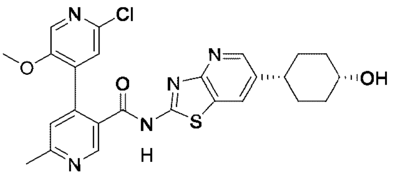 ,

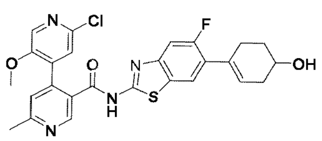 , 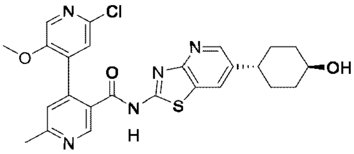 , 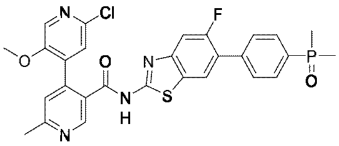 ,

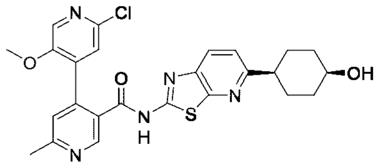 , 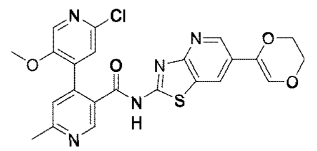 , 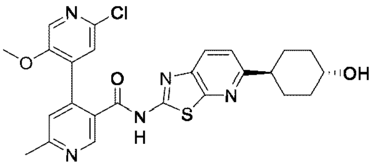 ,

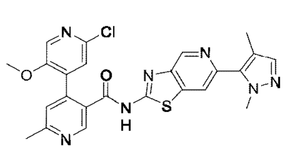 , 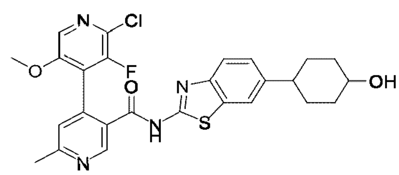 , 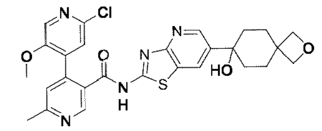 ,

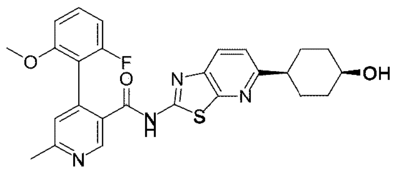 , 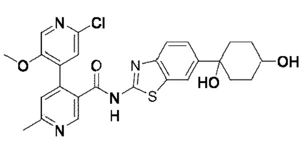 , 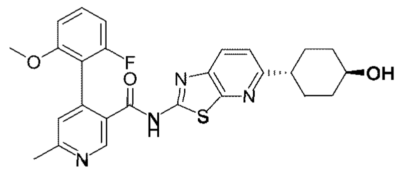 ,

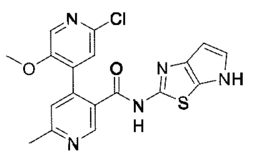 , 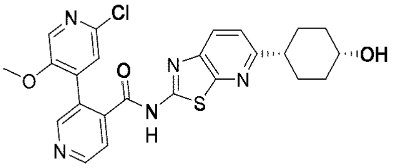 , 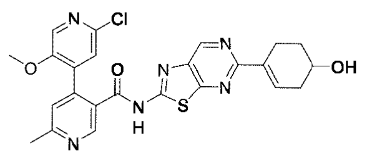 ,

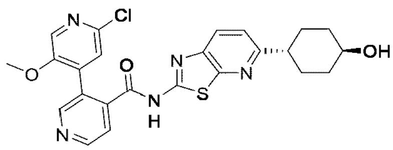 , 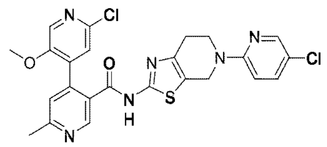 , 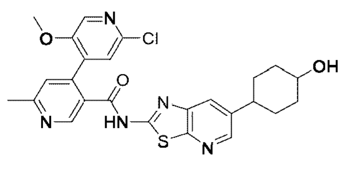 ,

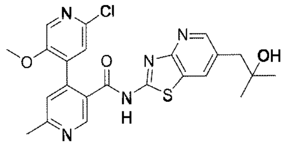 . 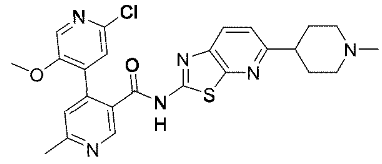 . 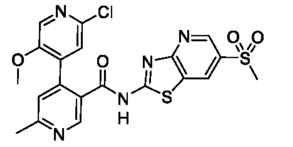 .

, , ,

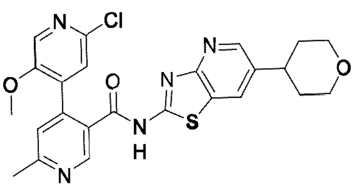, 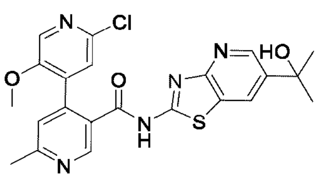, 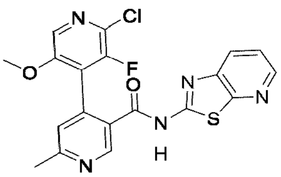,

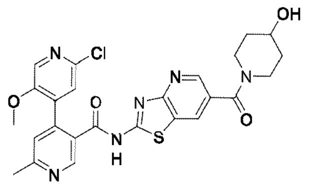, 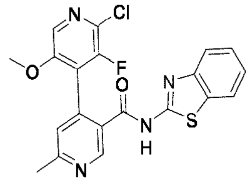, 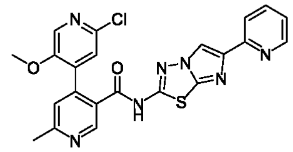,

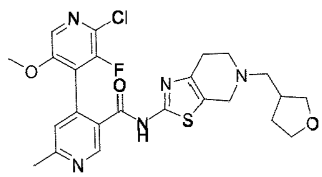, 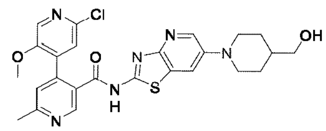, 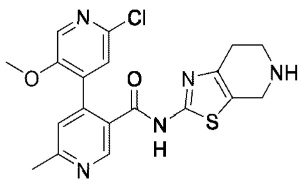,

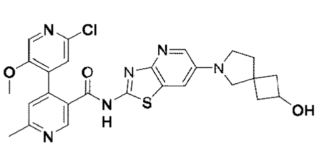, 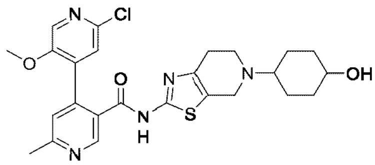, 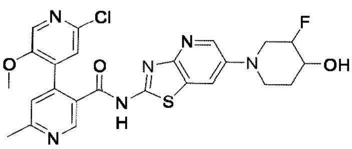,

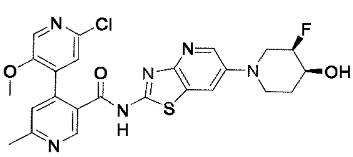, 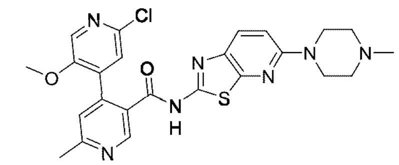, 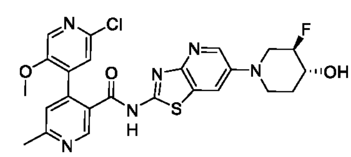,

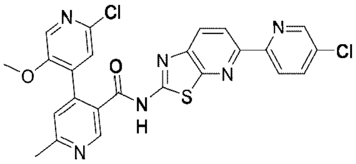, 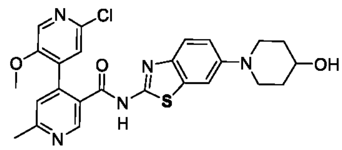, 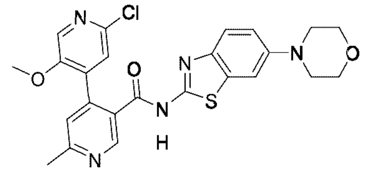,

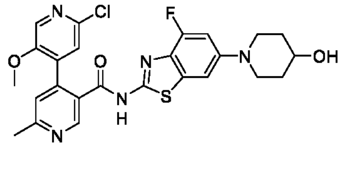, 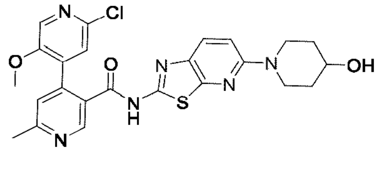, 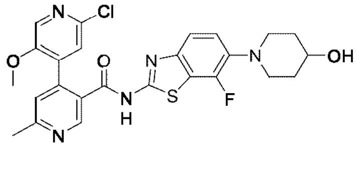,

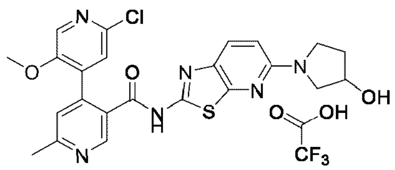 , 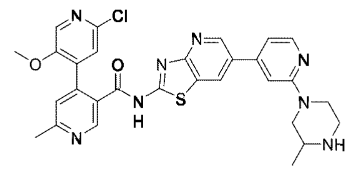 , 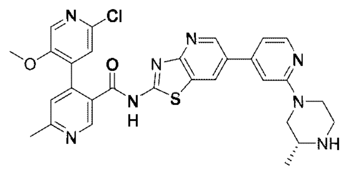 ,

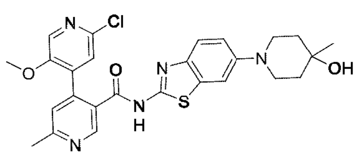 , 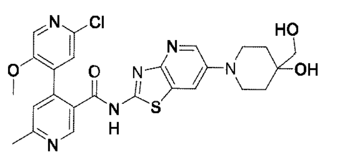 , 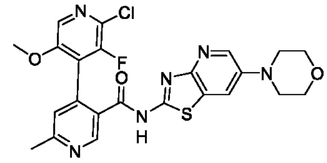 ,

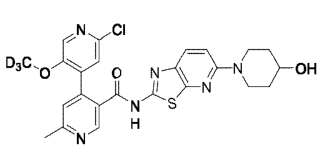 , 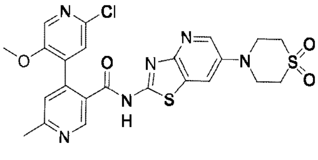 , 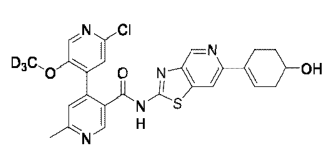 ,

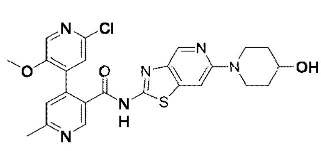 , 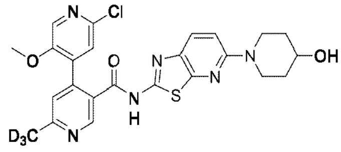 , 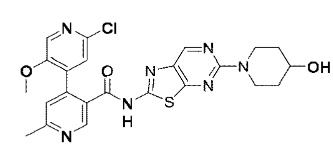 ,

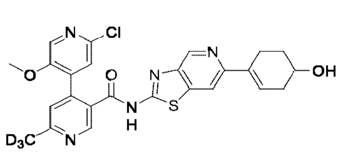 , 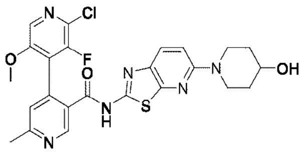 , 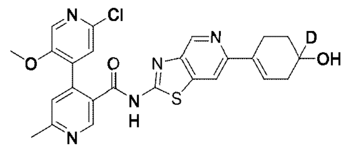 ,

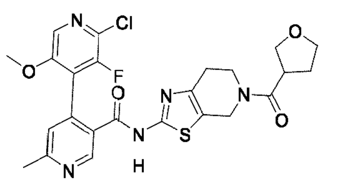 , 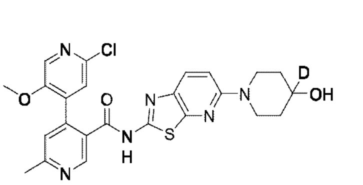 , 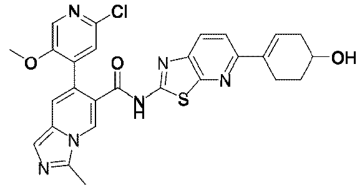 ,

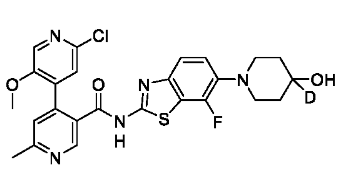 , 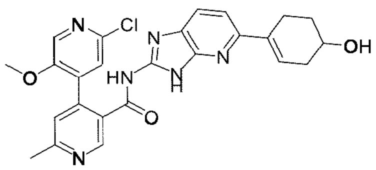 , 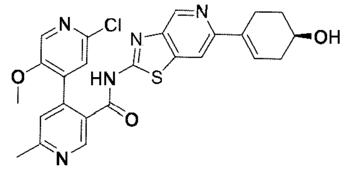 ,

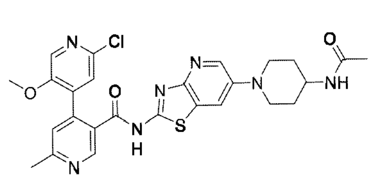 , 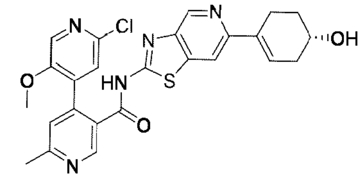 , 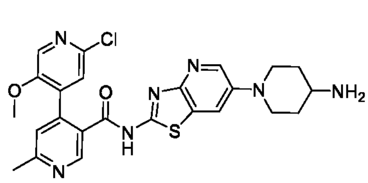 ,

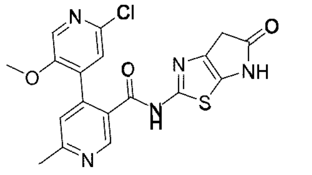,
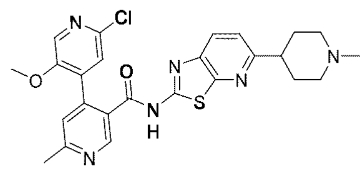,
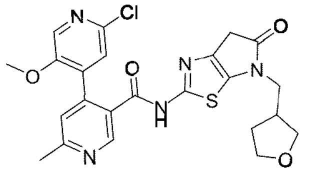,
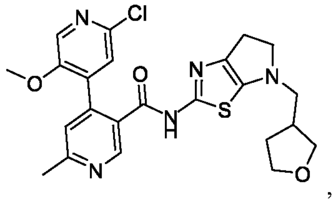,
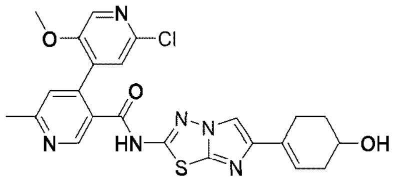,
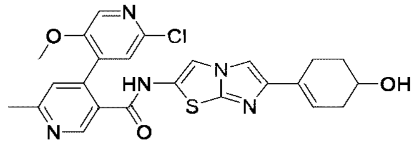,
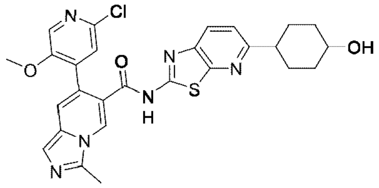,
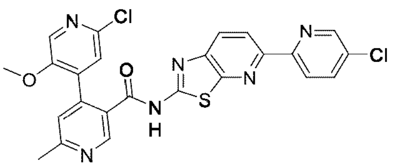,
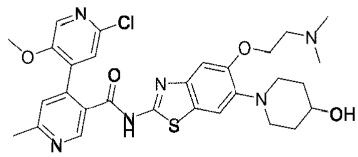,
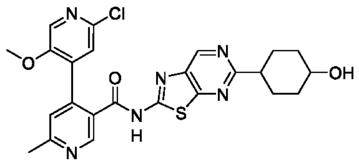,
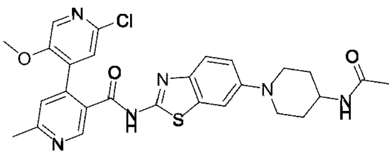,
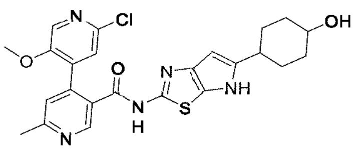,
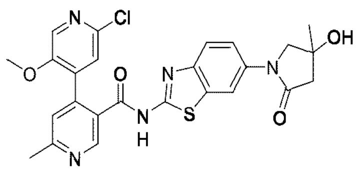,
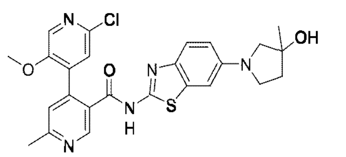,
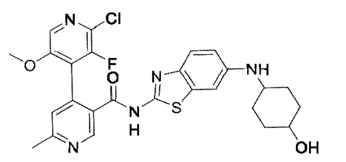,
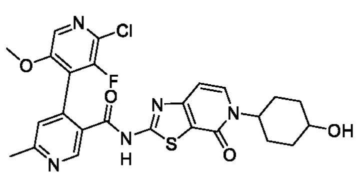,
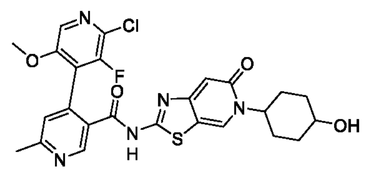,
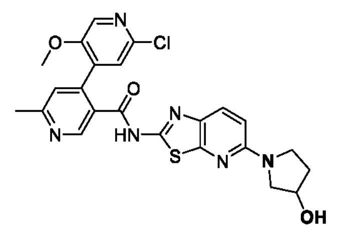,
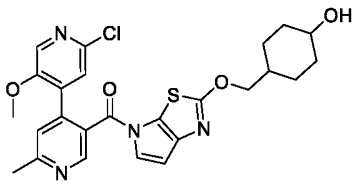,
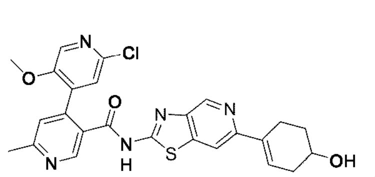,
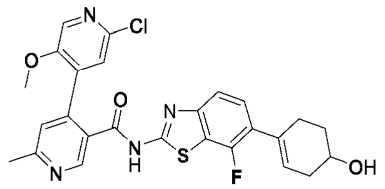,

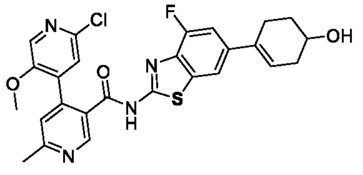,
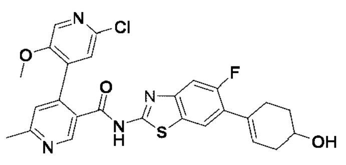,
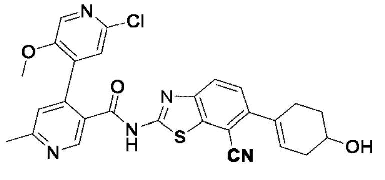,

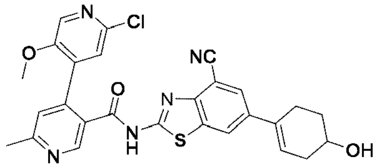,
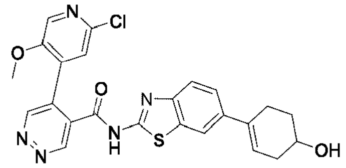,
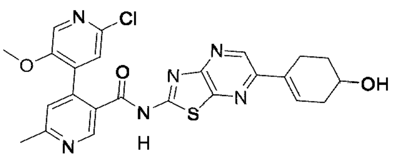,

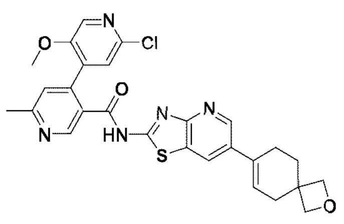,
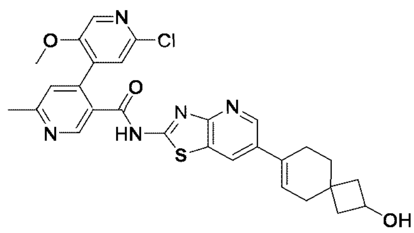,
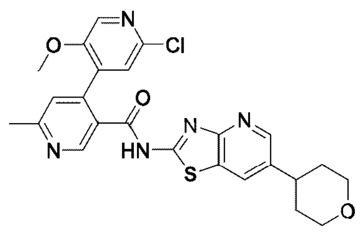,

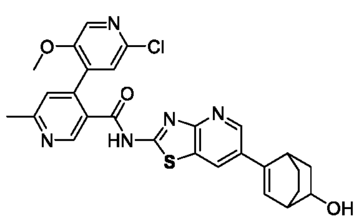,
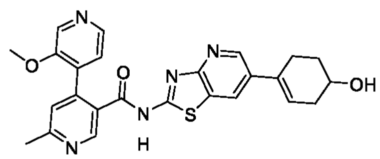,
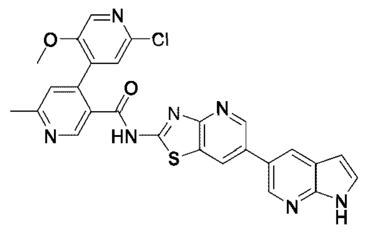,

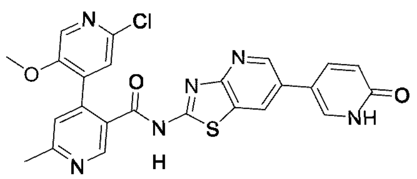,
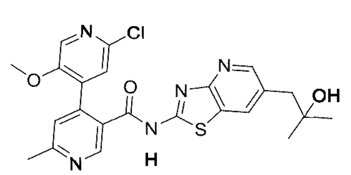,
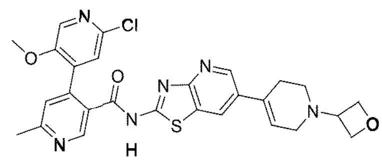,

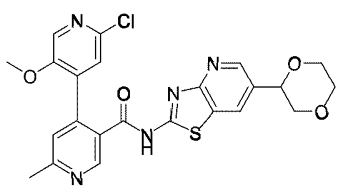,
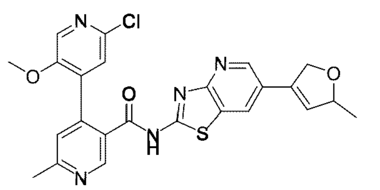,
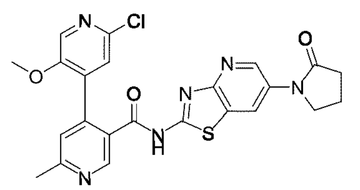,

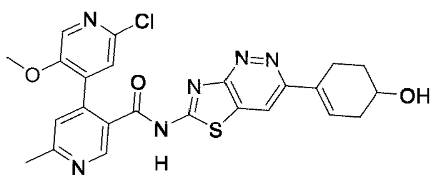,
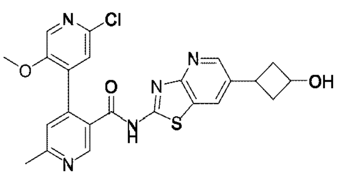,
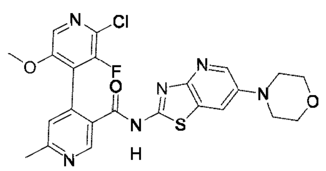,

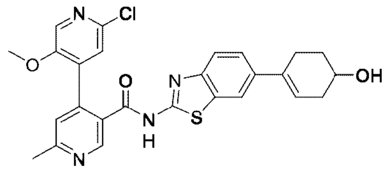,
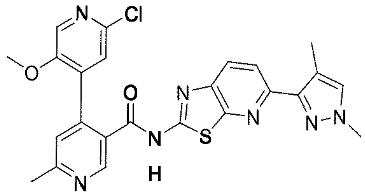,
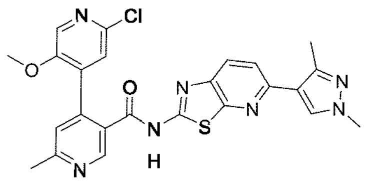,

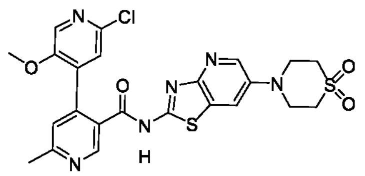,
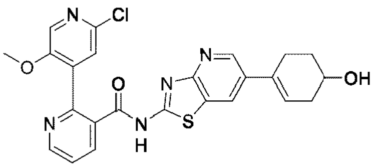,
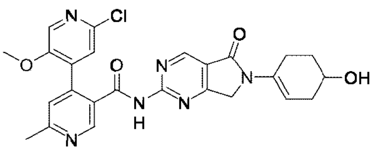,

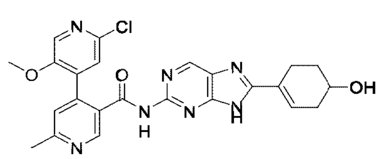,
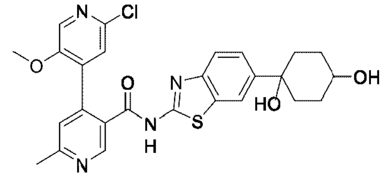,
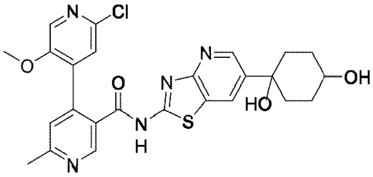,

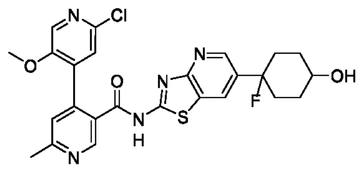,
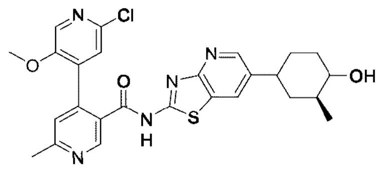,
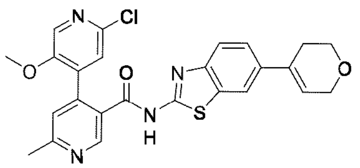,

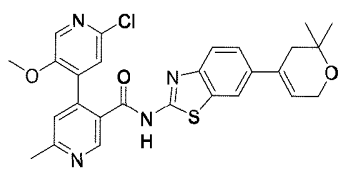,
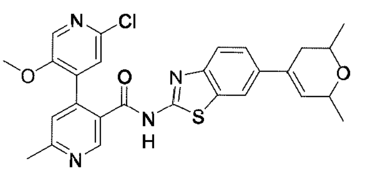,
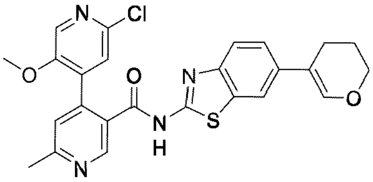,

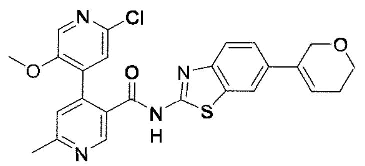,
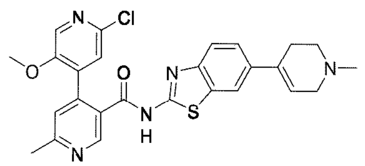,
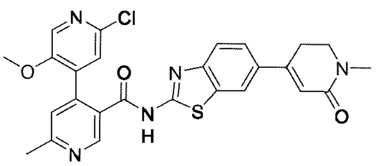,

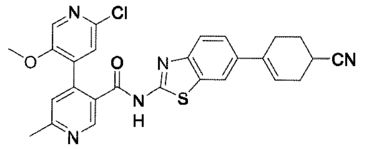,
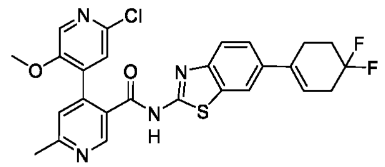,
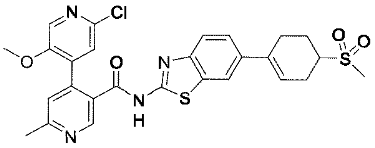,

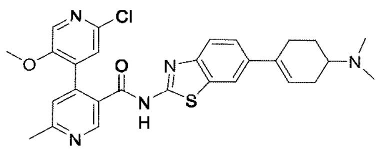 , 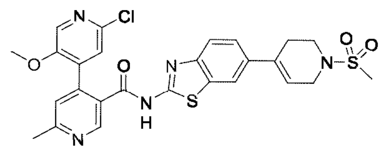 , 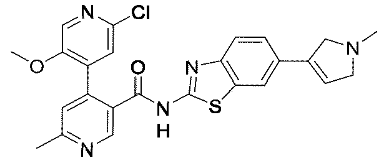 ,

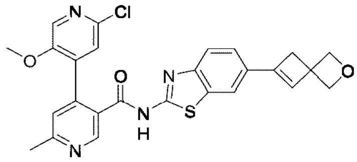 , 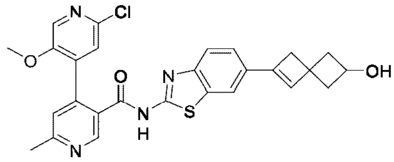 , 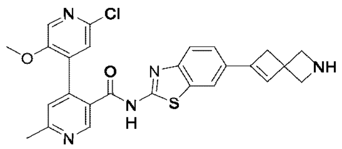 ,

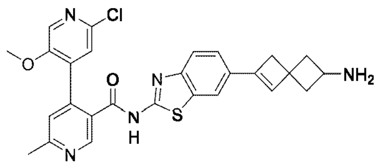 , 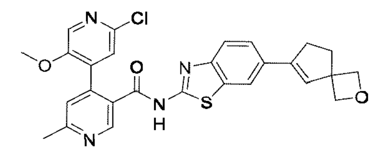 , 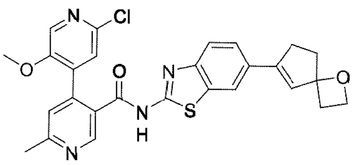 ,

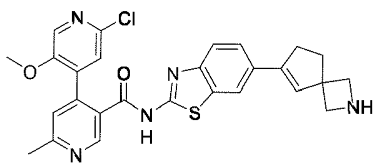 , 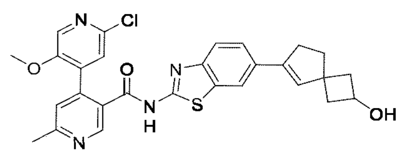 , 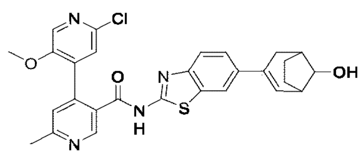 ,

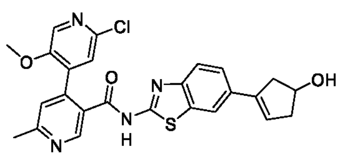 , 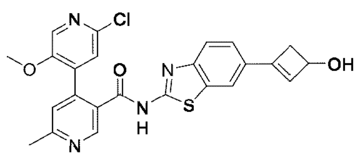 , 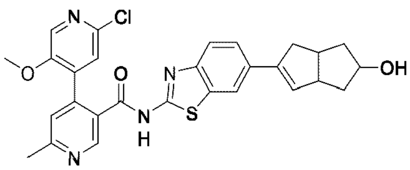 ,

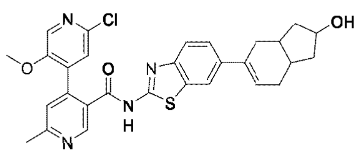 , 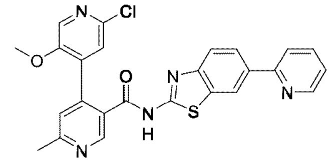 , 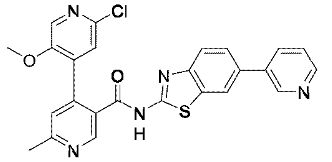 ,

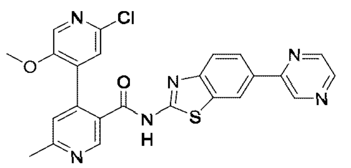 , 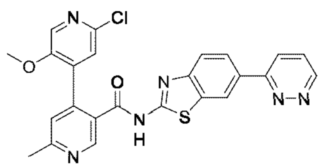 , 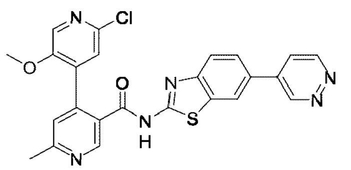 ,

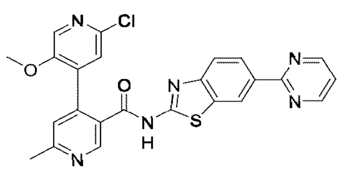 , 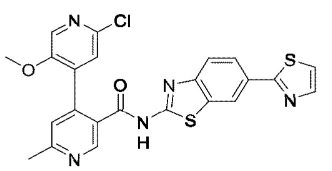 , 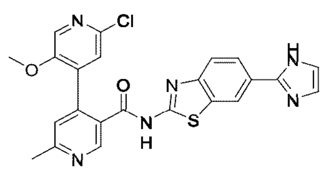 ,

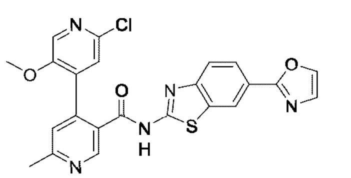,
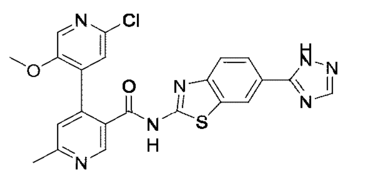,
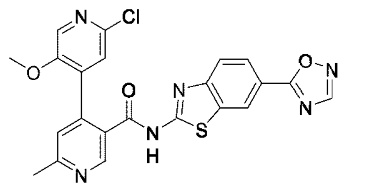,

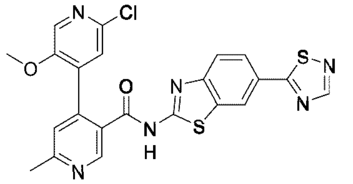,
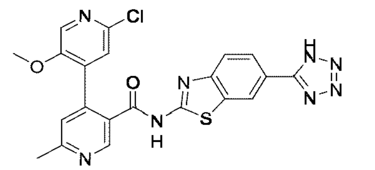,
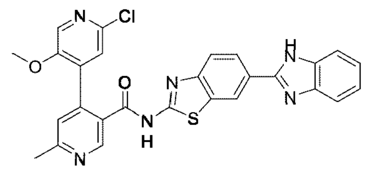,

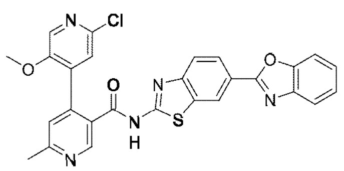,
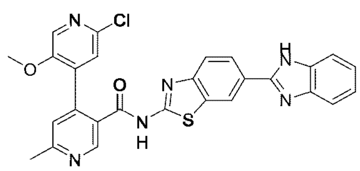,
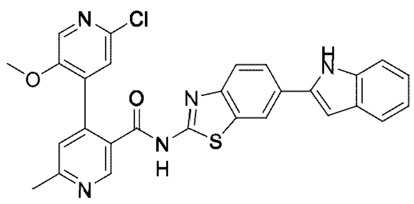,

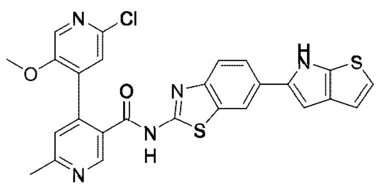,
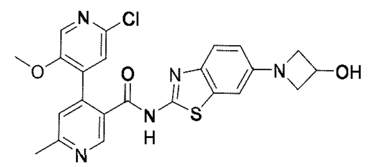,
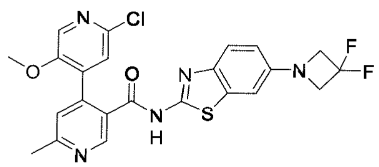,

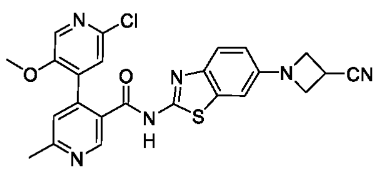,
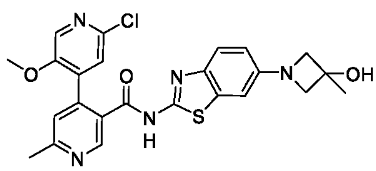,
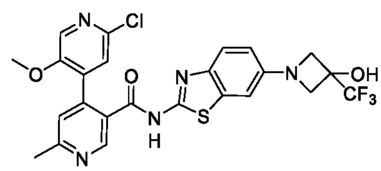,

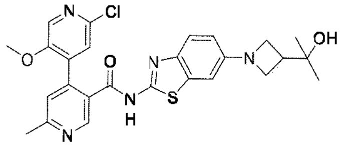,
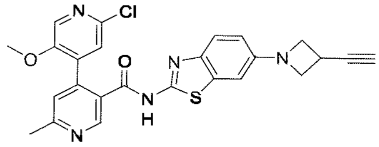,
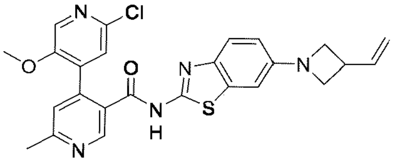,

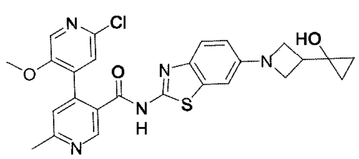,
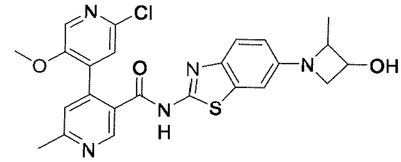,
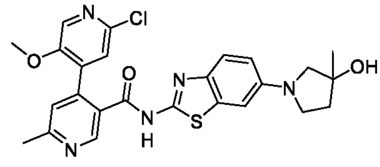,

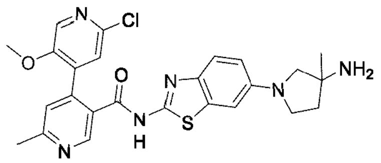,
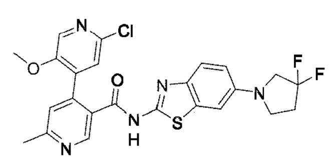,
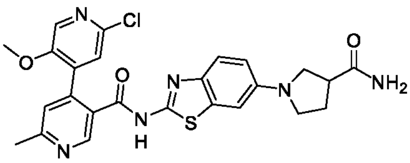,

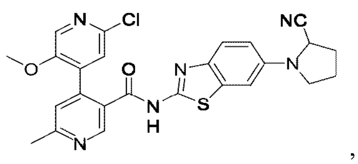,
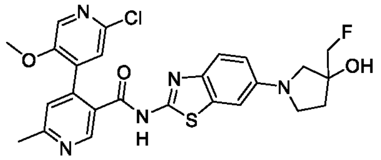,
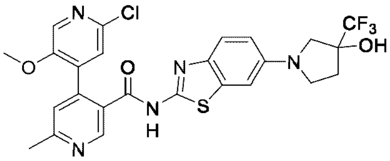,

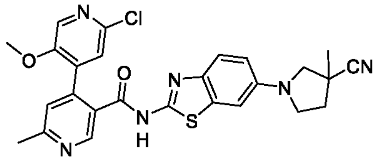,
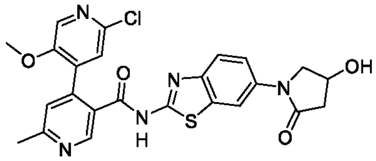,
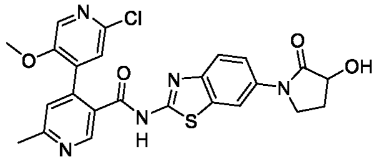,

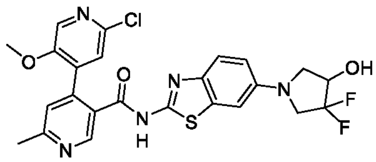,
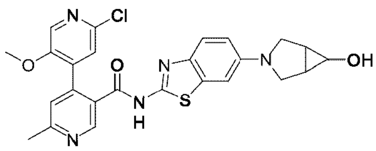,
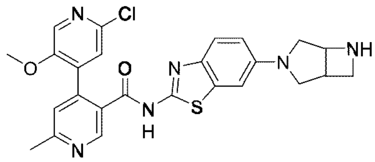,

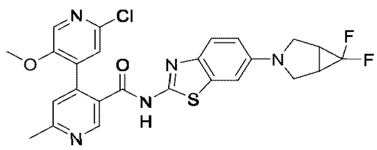,
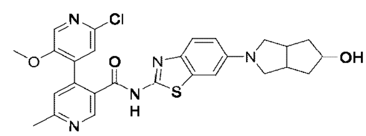,
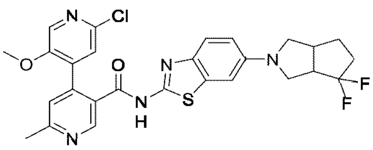,

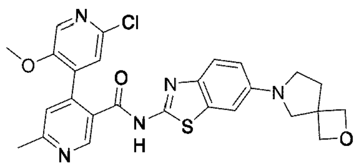,
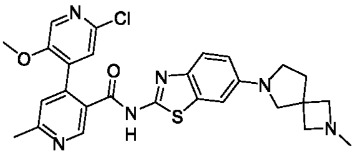,
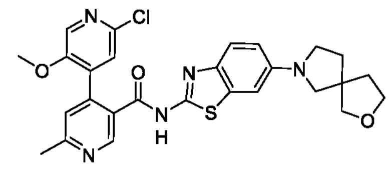,

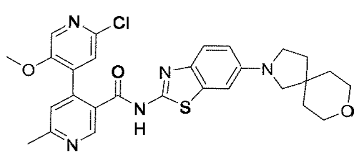,
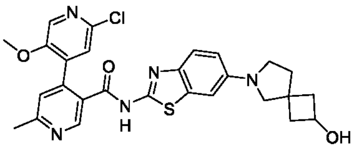,
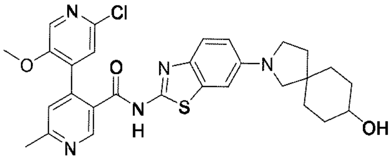,

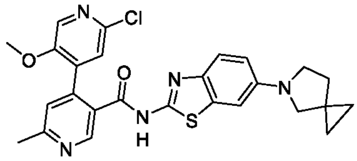,
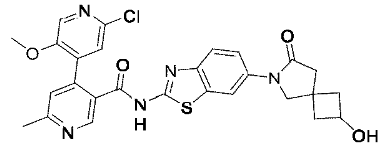,
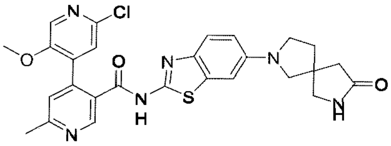,

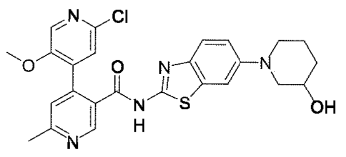 , 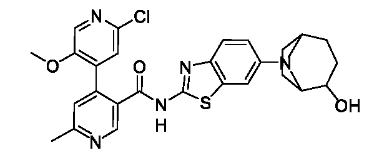 , 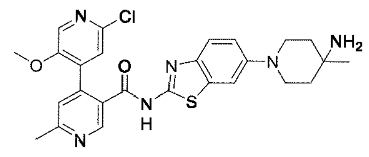 ,

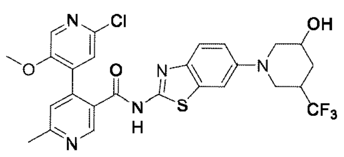 , 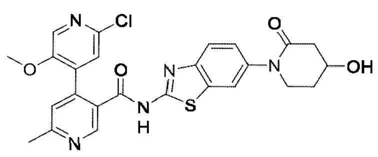 , 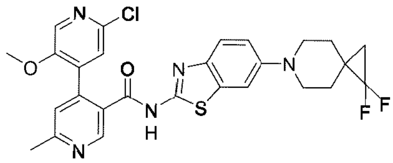 ,

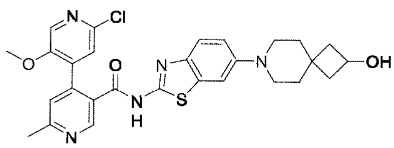 , 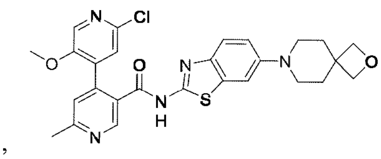 , 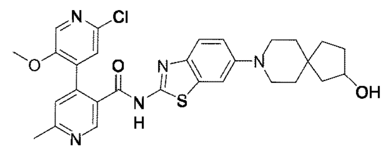 ,

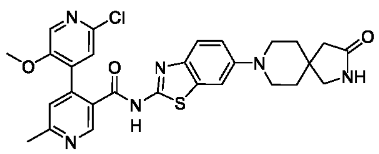 , 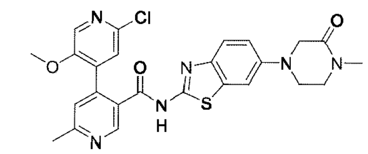 , 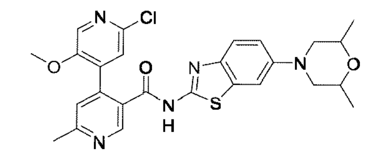 ,

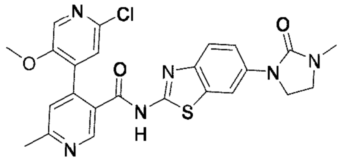 , 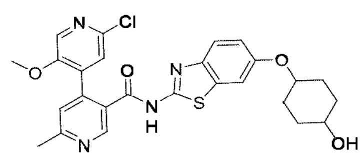 , 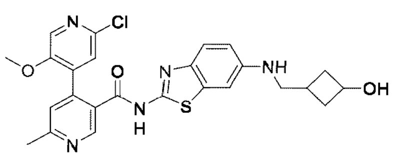 ,

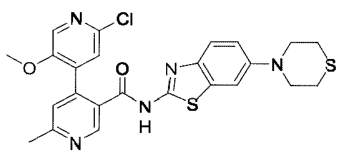 , 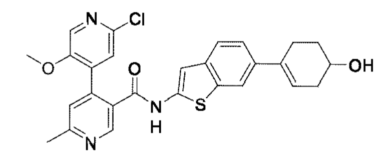 , 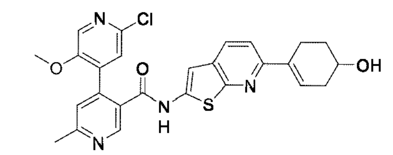 ,

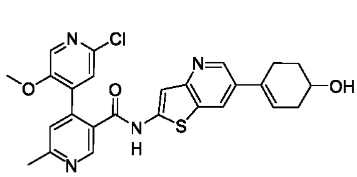 , 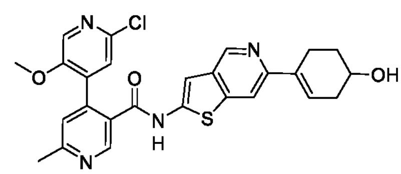 , 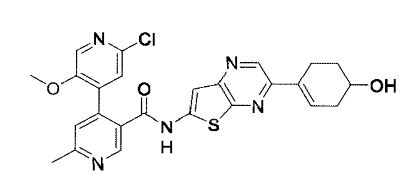 ,

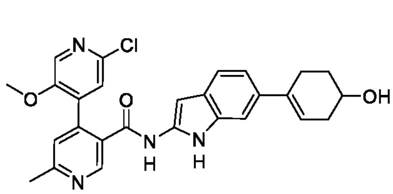 , 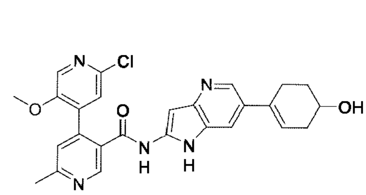 , 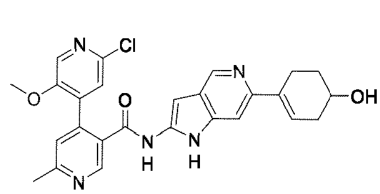 ,

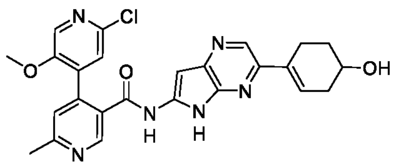 , 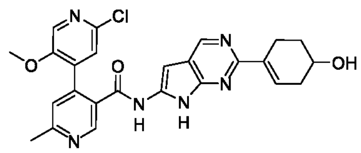 , 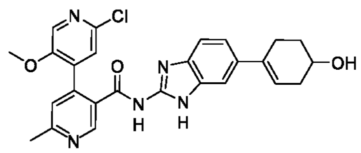 ,

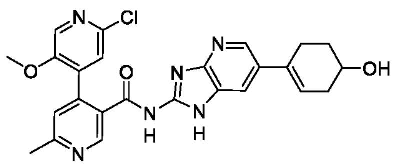 , 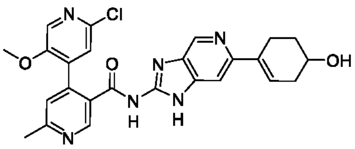 , 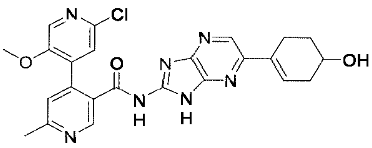 ,

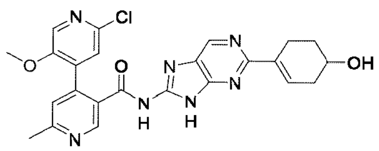 , 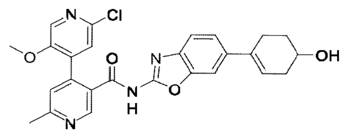 , 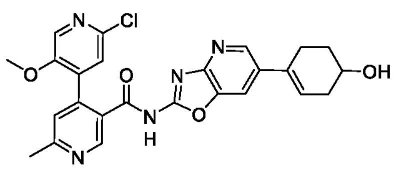 ,

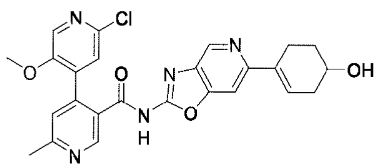 , 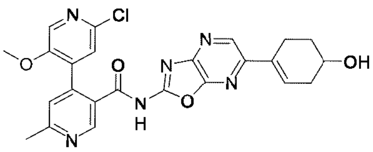 , 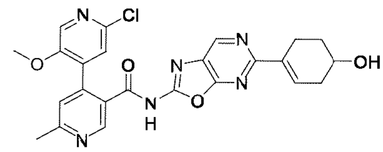 ,

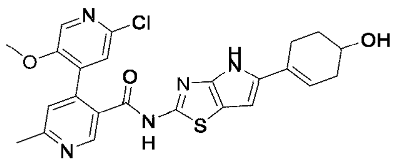 , 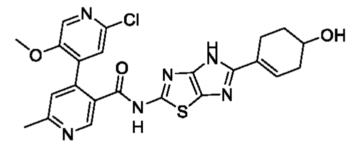 , 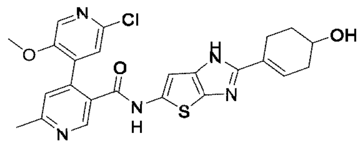 ,

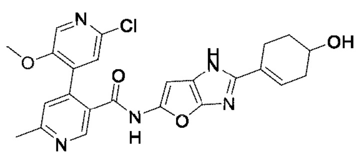 , 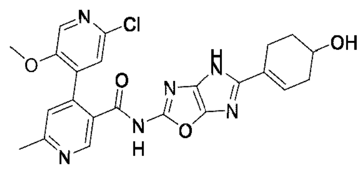 , 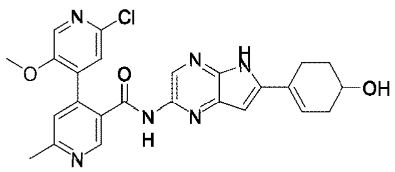 ,

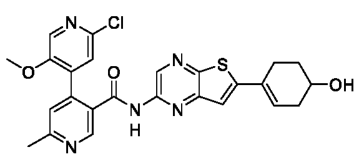 , 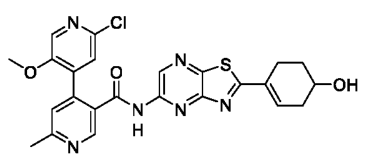 , 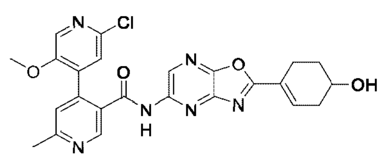 ,

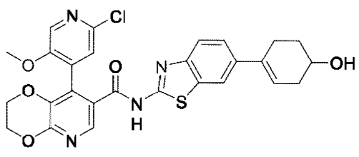 , 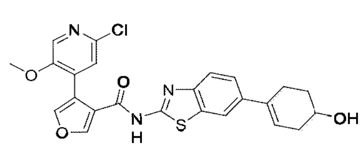 , 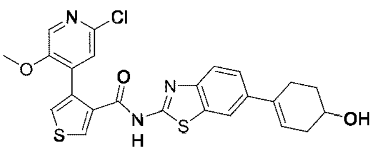 ,

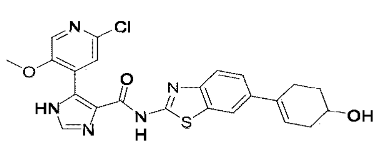,
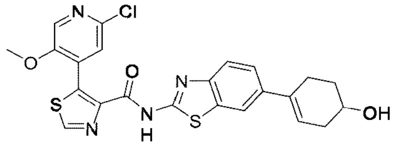,
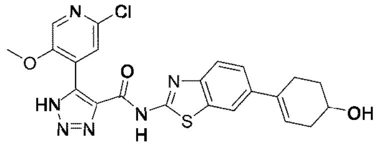,

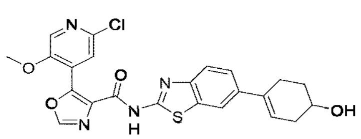,
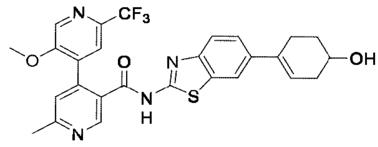,
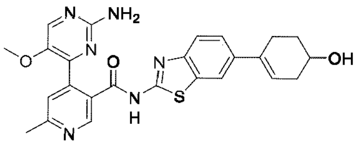,

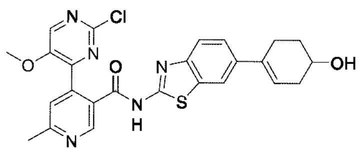,
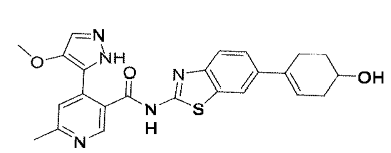,
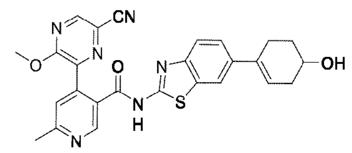,

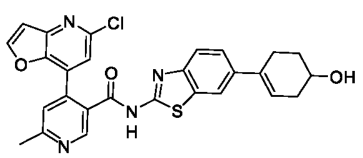,
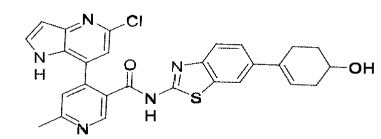,
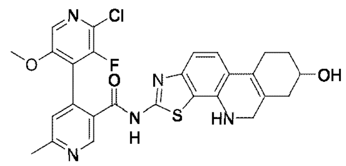,

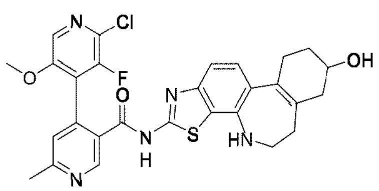,
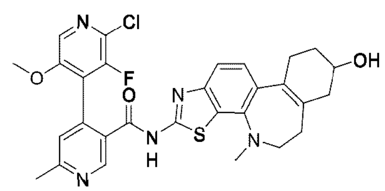,
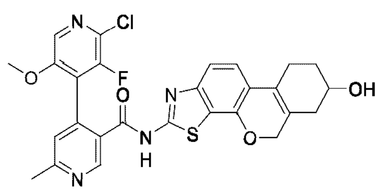,

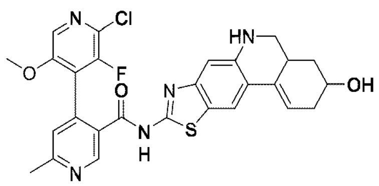,
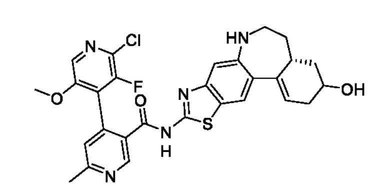,
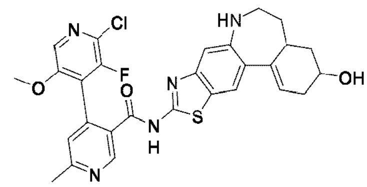,

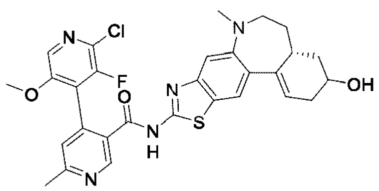,
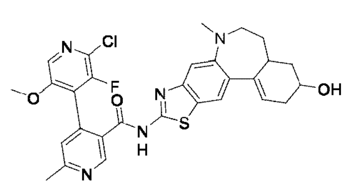,
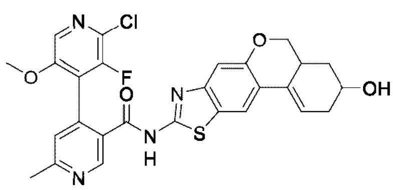,

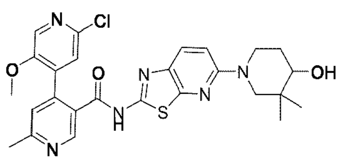,
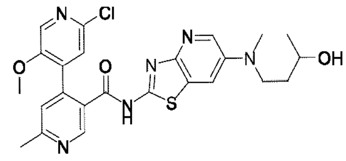,
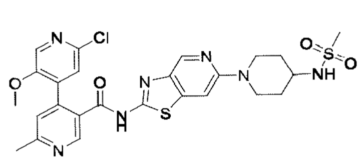,

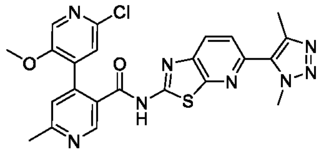,
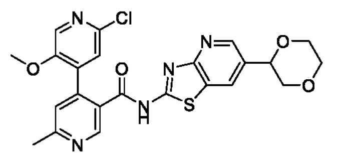,
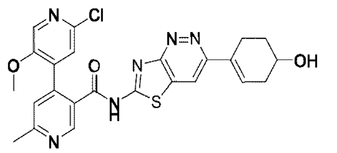,
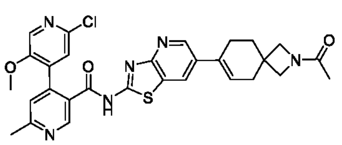,
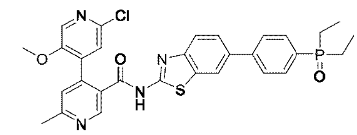,
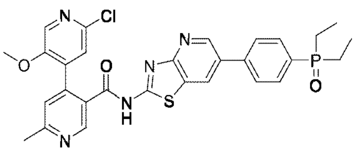,
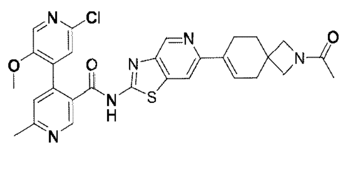,
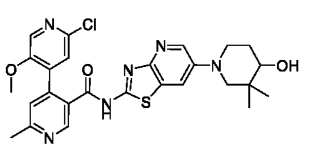,
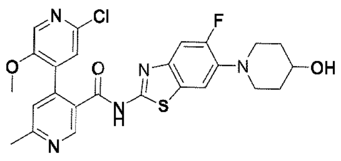,
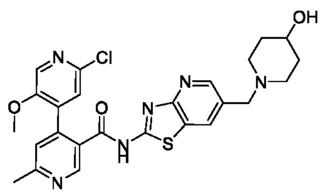,
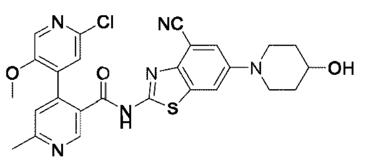,
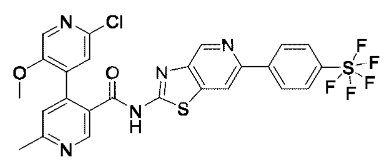,
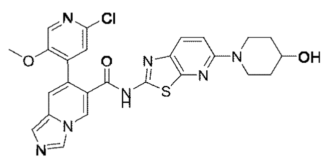,
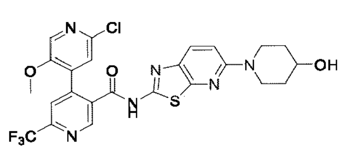,
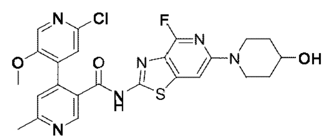,
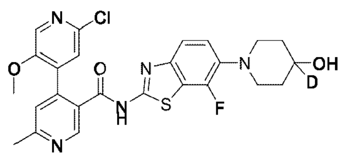,
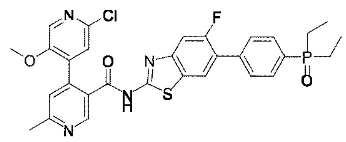,
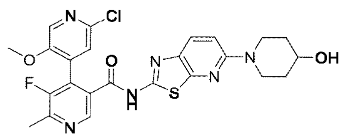,
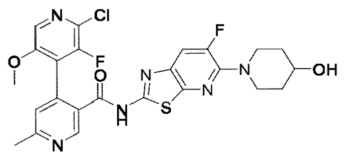,
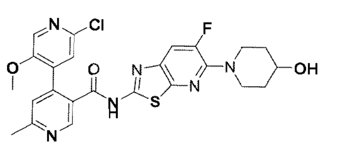,
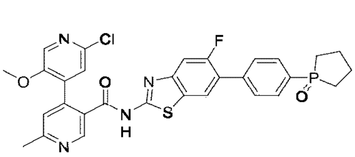,
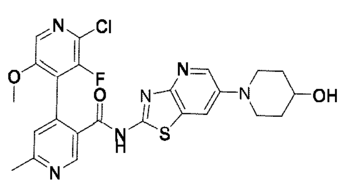,
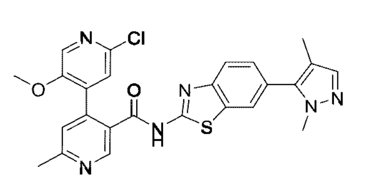,
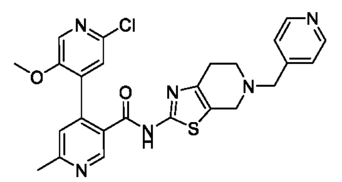,

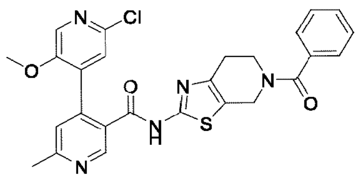, 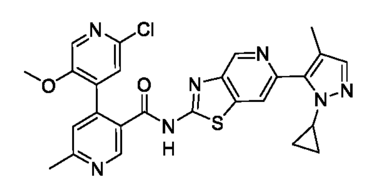, 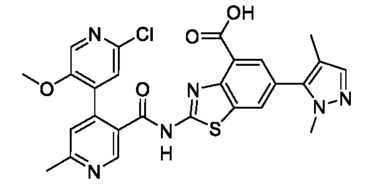,

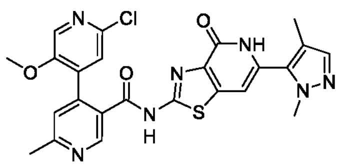, 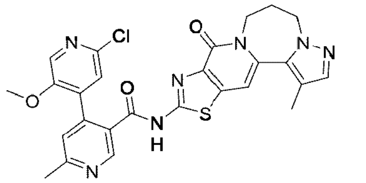, 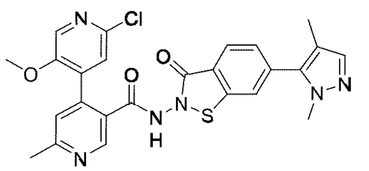,

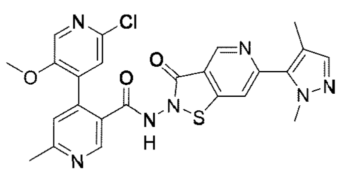, 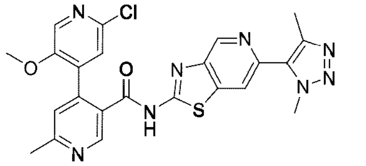, 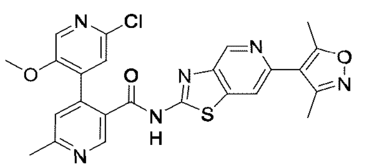,

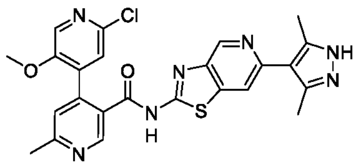, 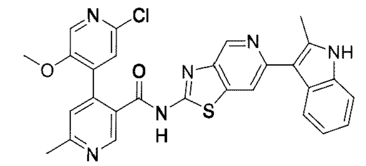, 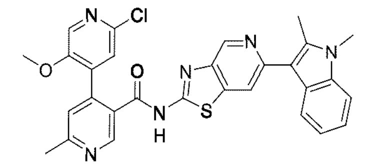,

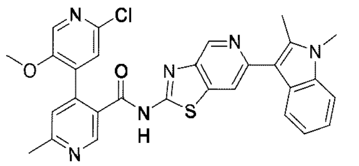, 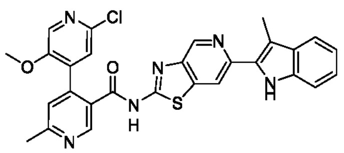, 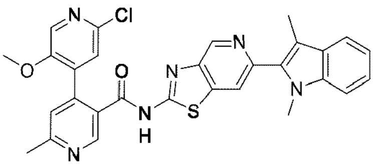,

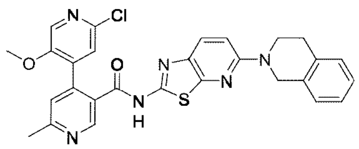, 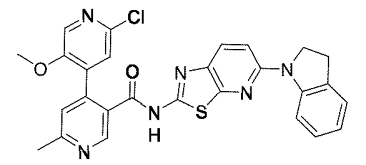, 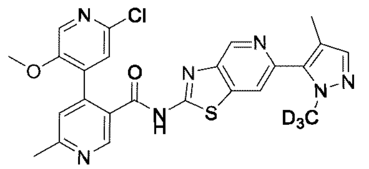,

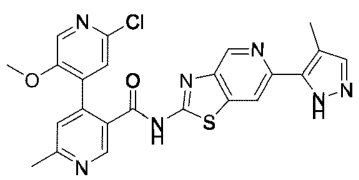, 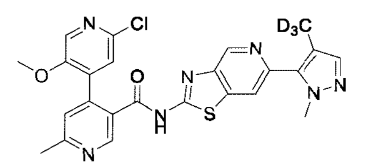, 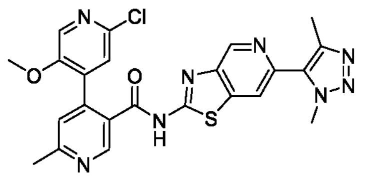,

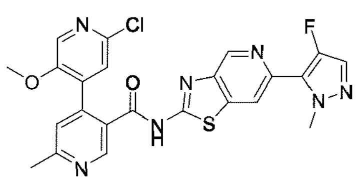, 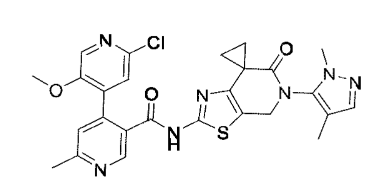, 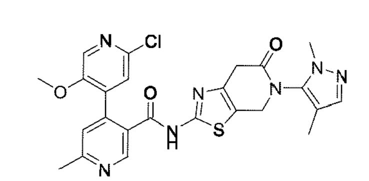,

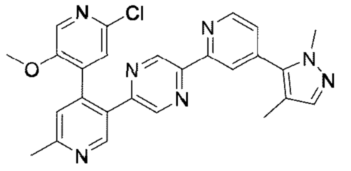 , 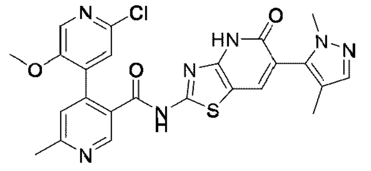 , 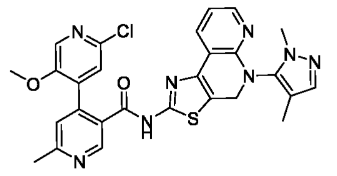 ,

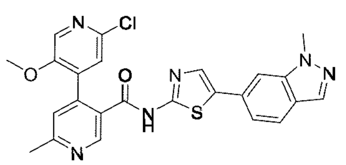 , 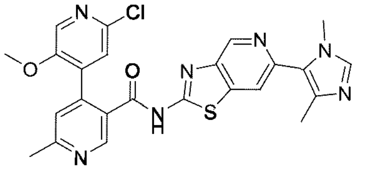 , 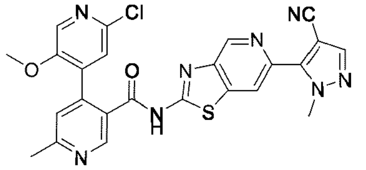 ,

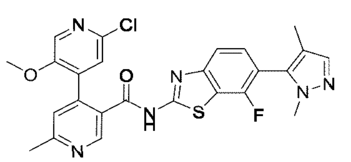 , 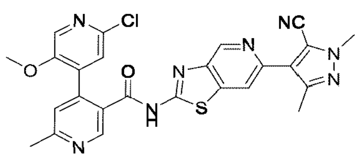 , 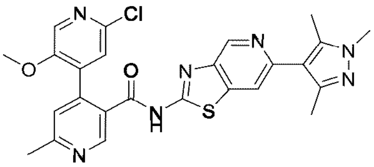 ,

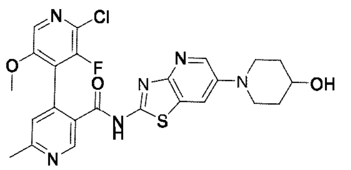 , 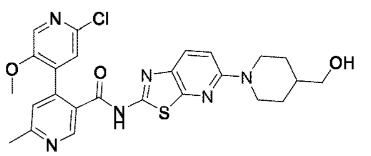 , 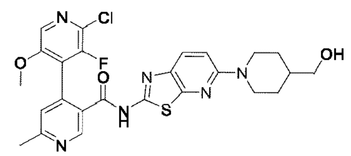 ,

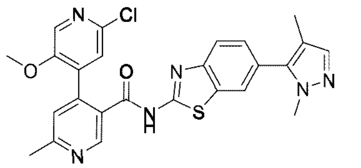 , 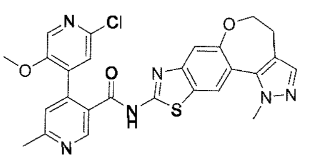 , 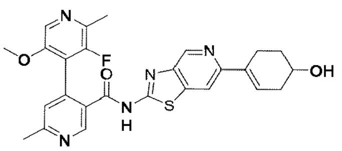 ,

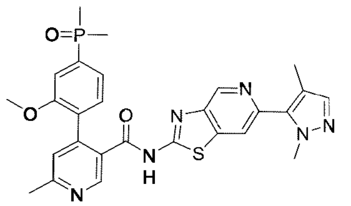 , 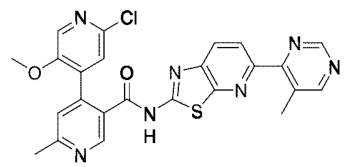 , 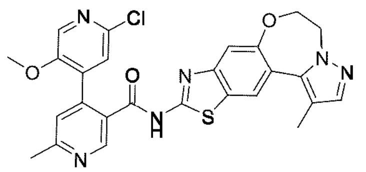 ,

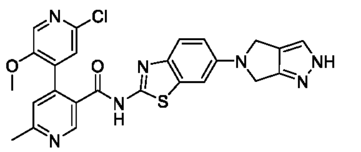 , 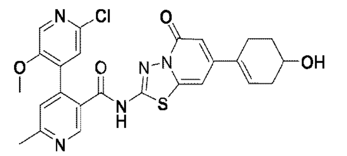 , 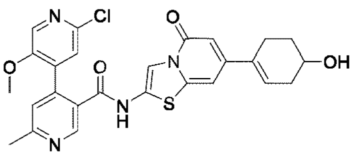 ,

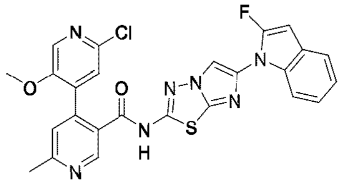 , 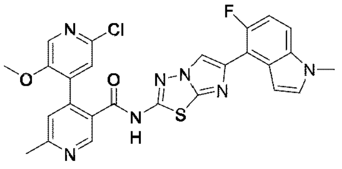 , 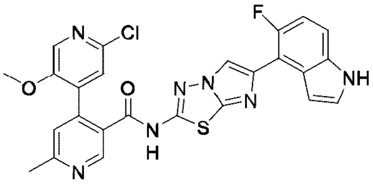 ,

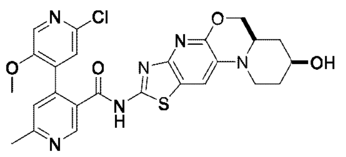,
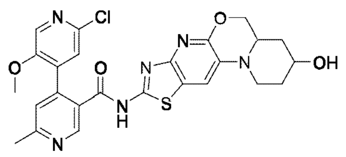,
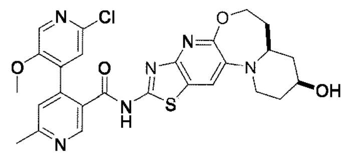,

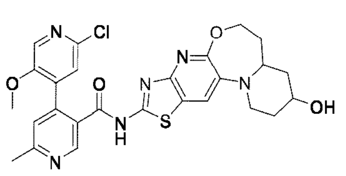,
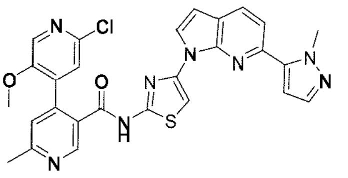,
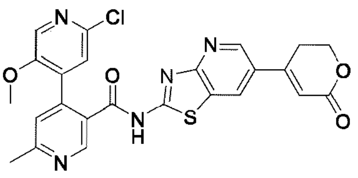,

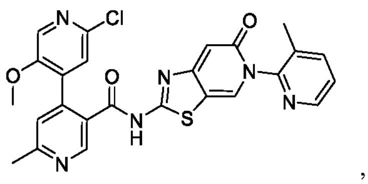,
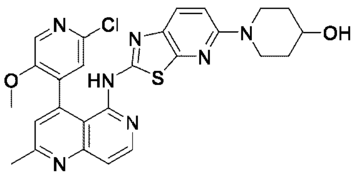,
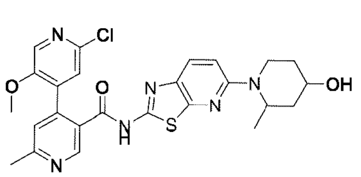,

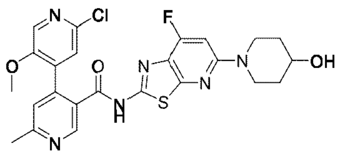,
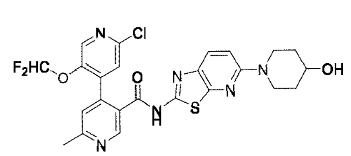,
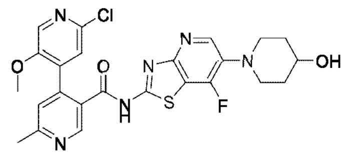,

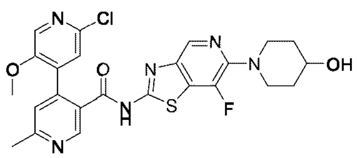,
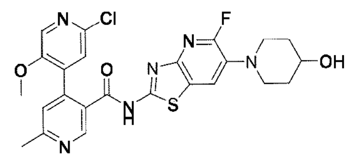,
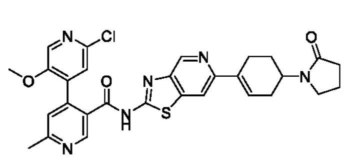,

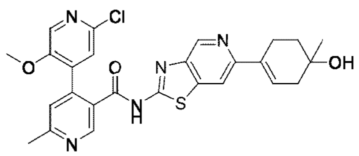,
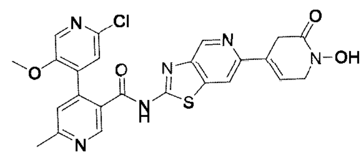,
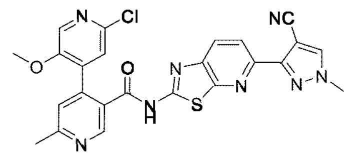,

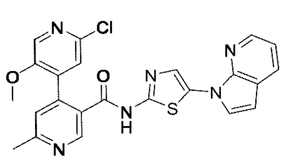,
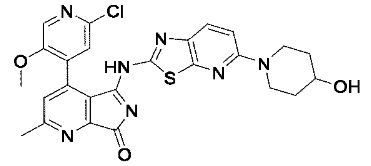,
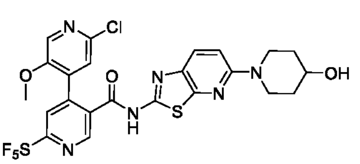,

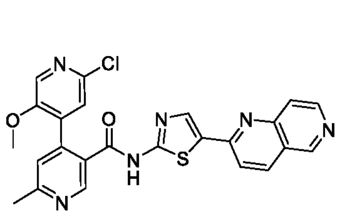,
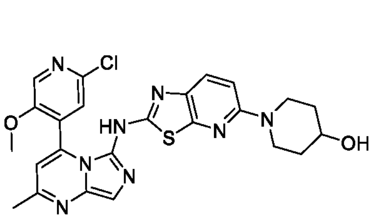,
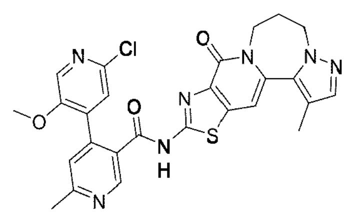,

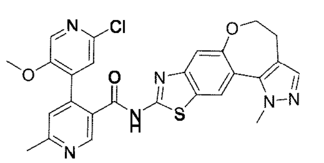 , 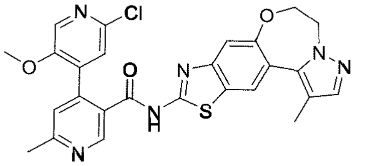 , 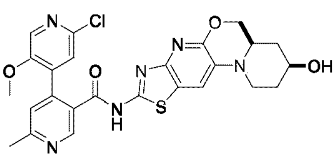 ,

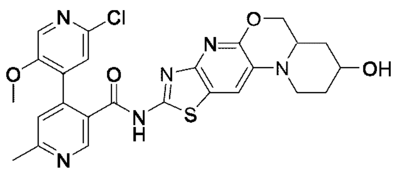 , 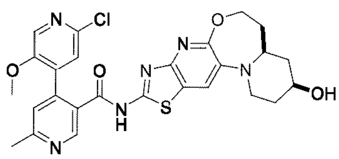 , 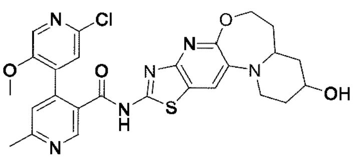 ,

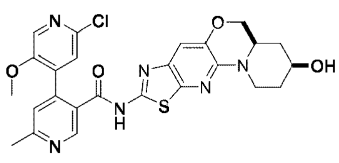 , 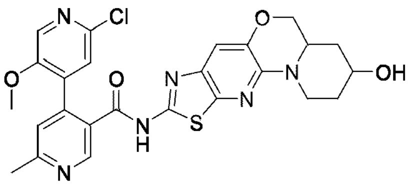 , 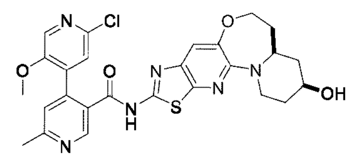 ,

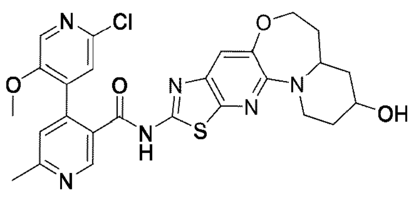 , 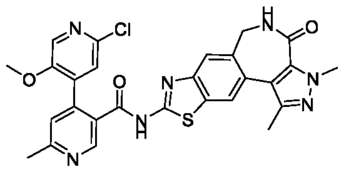 , 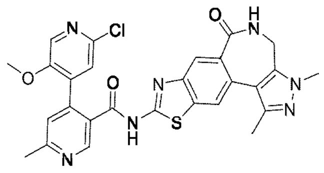 ,

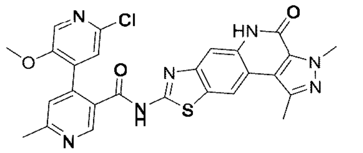 , 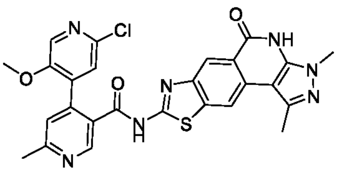 , 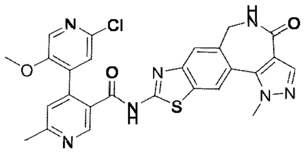 ,

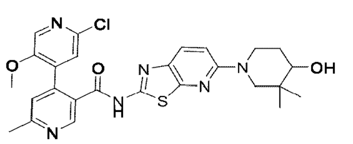 , 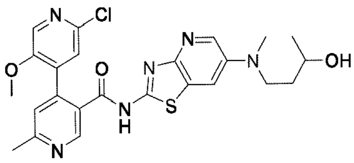 , 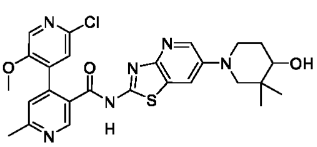 ,

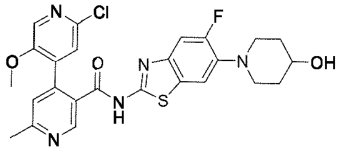 , 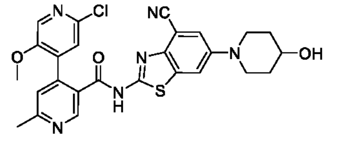 , 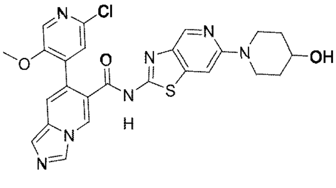 ,

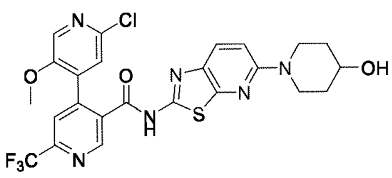 , 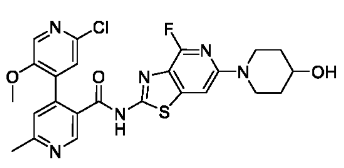 , 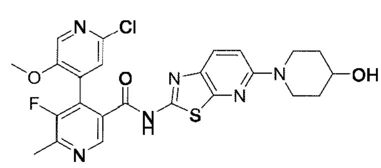 ,

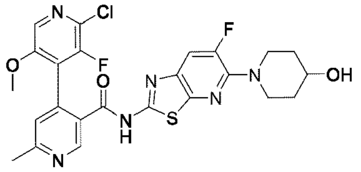,
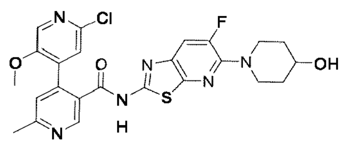,
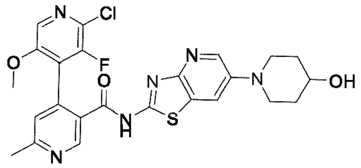,
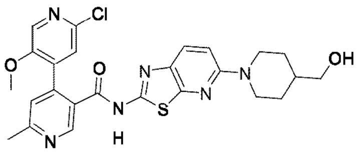,
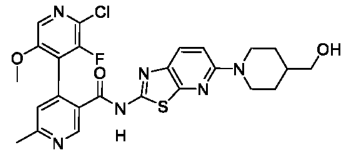,
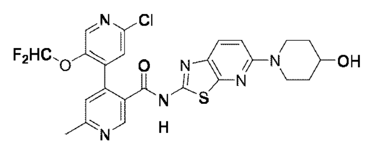,
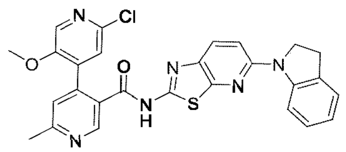,
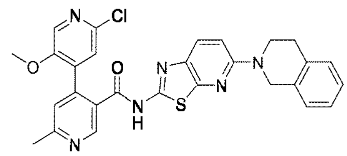,
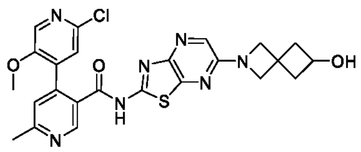,
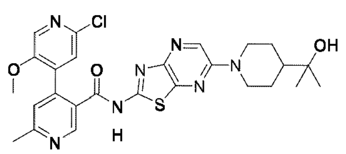,
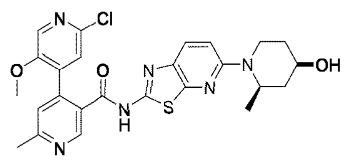,
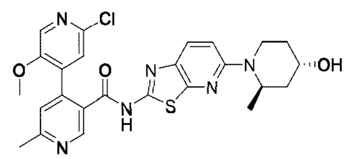,
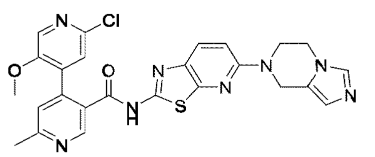,
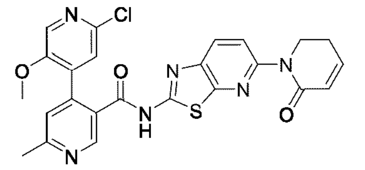,
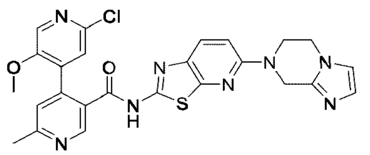,
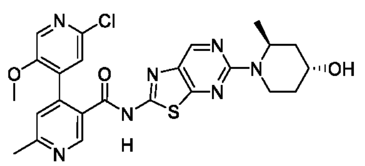,
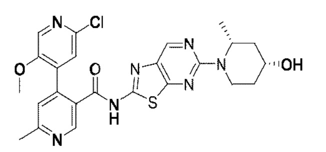,
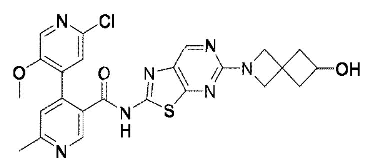,
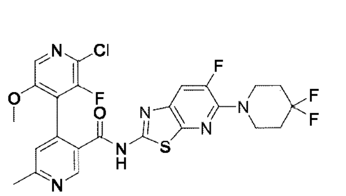,
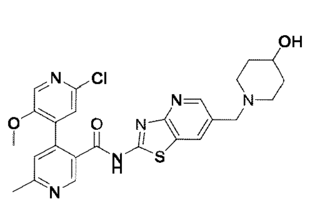,
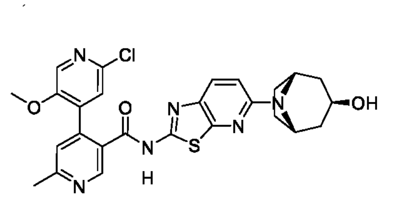,
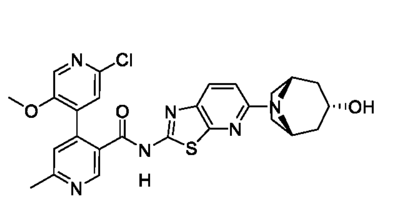,
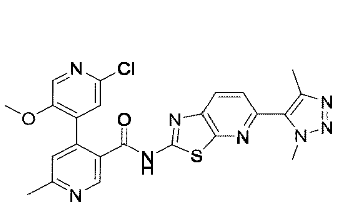,
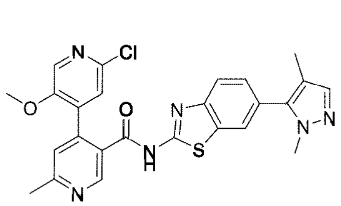,

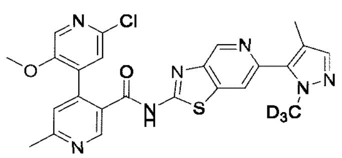
,
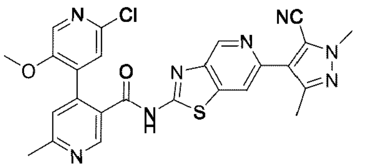
,
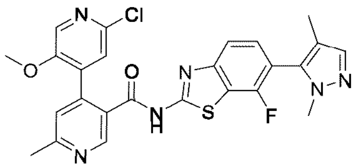
,

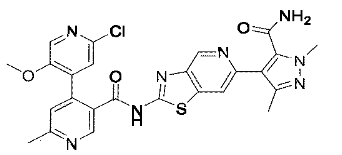
,
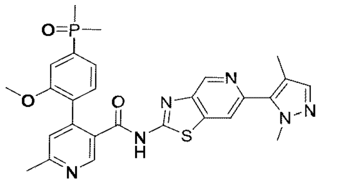
,
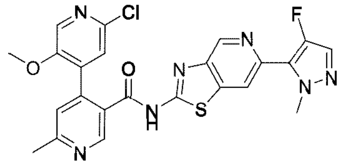
,

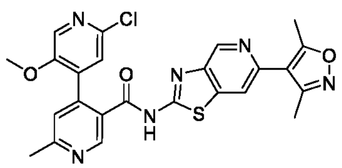
,
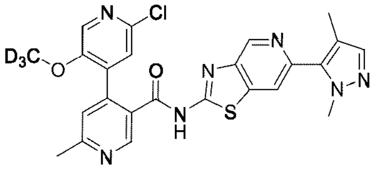
,
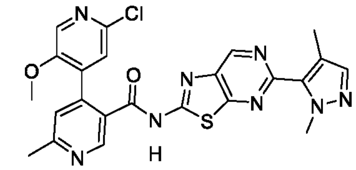
,

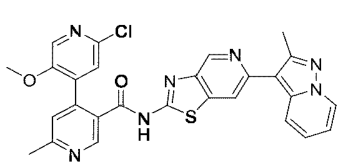
,
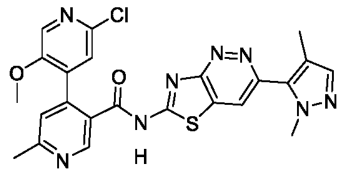
,
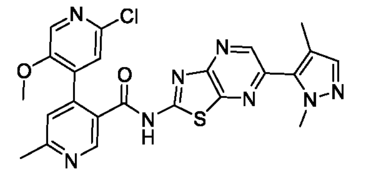
,

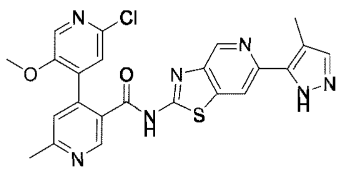
,
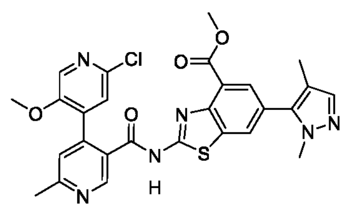
,
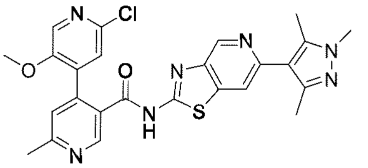
,

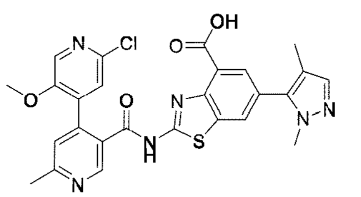
,
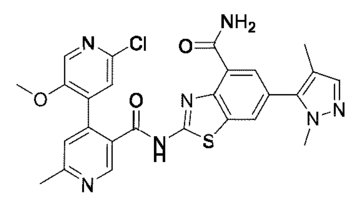
,
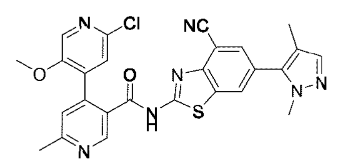
,

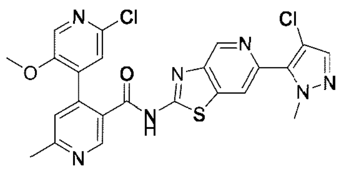
,
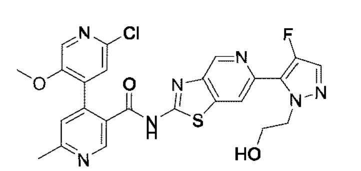
,
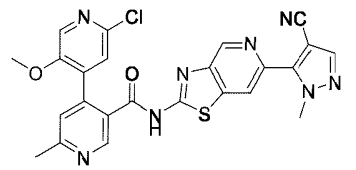
,

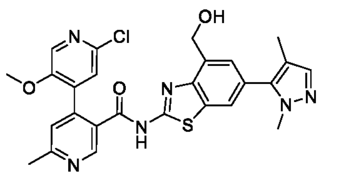, 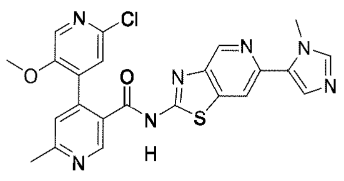, 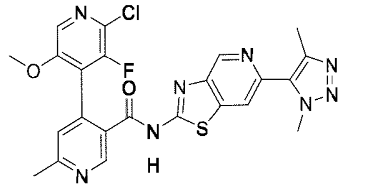,

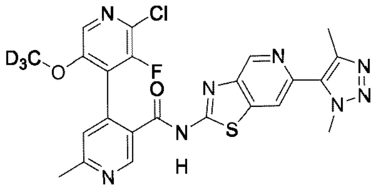, 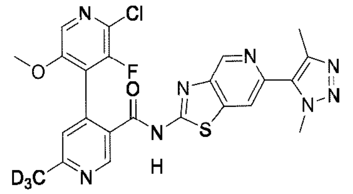, 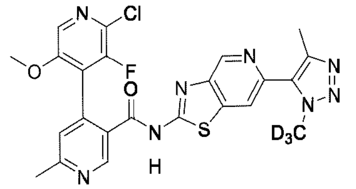,

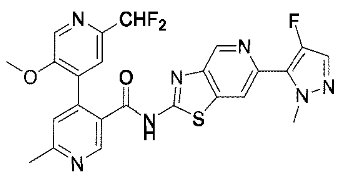, 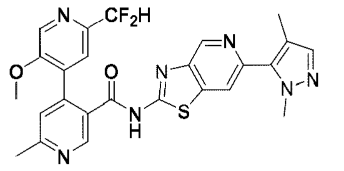, 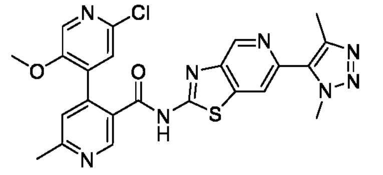,

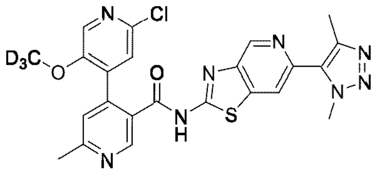, 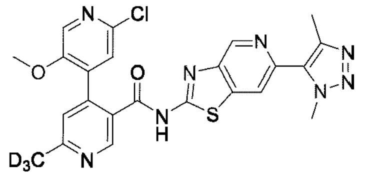, 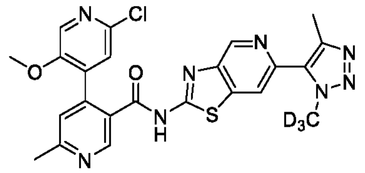,

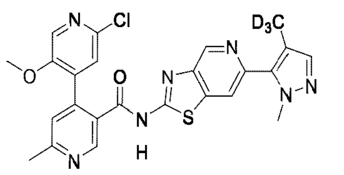, 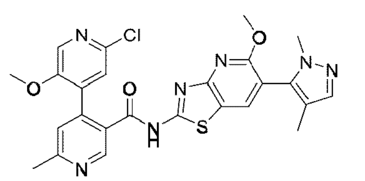, 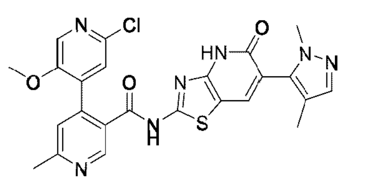,

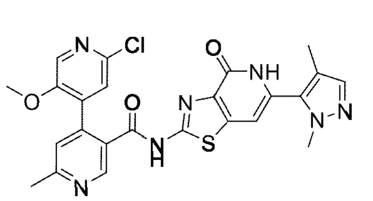, 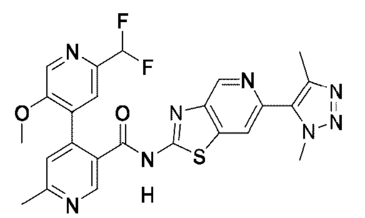, 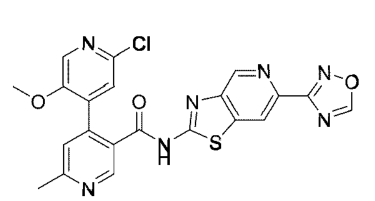,

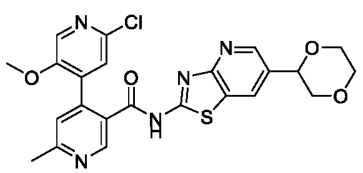, 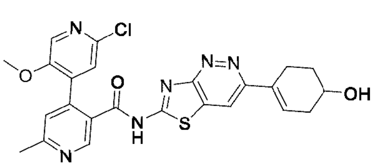, 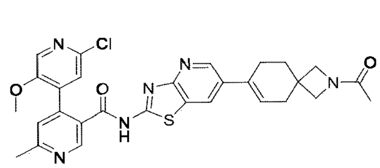,

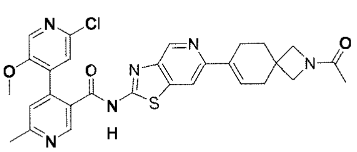,
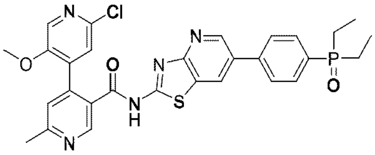,
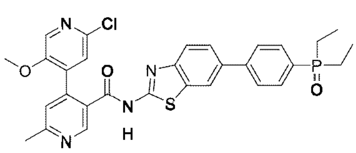,
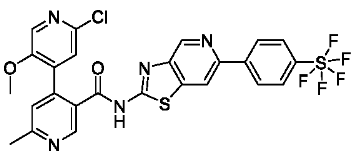,
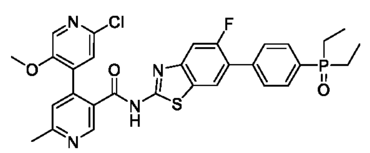,
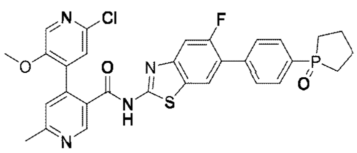,
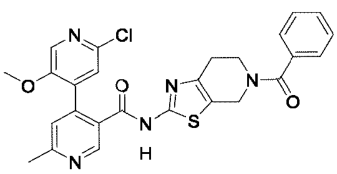,
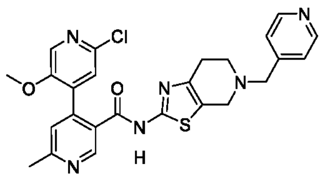,
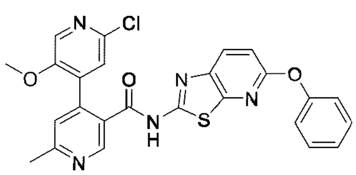,
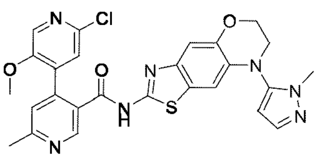,
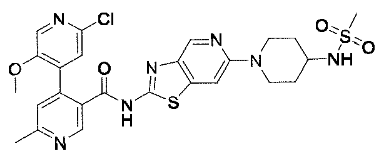,
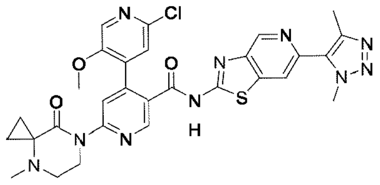,
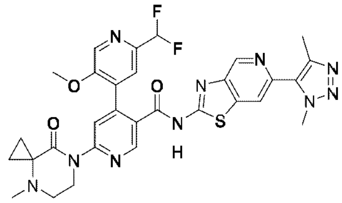,
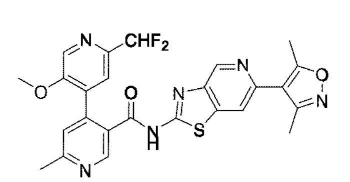,
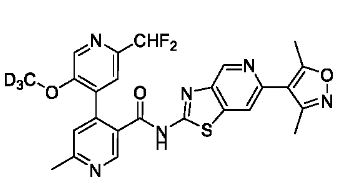,
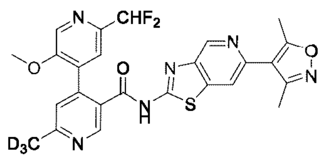,
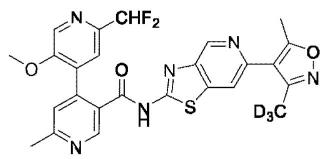,
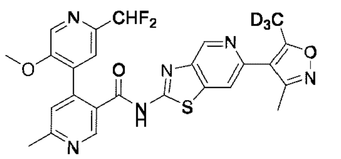,
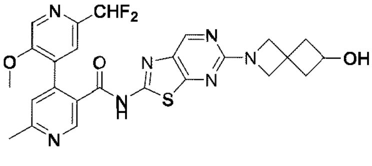,
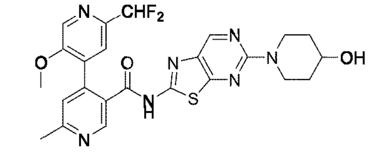,
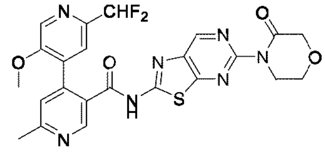,
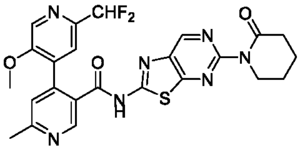,
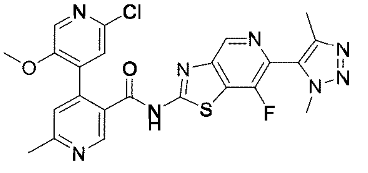,
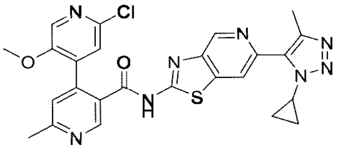,

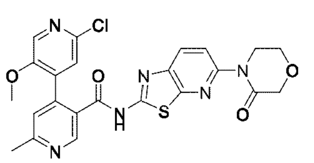,
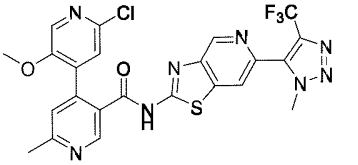,
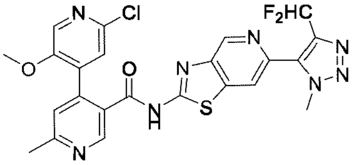,

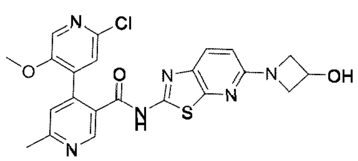,
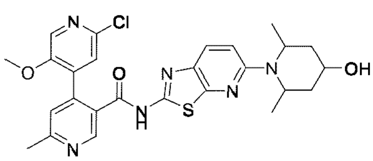,
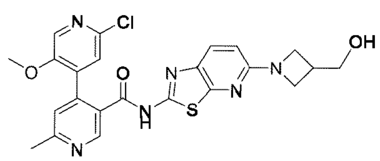,

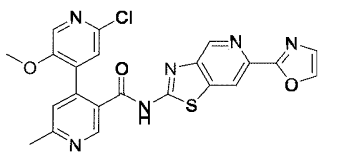,
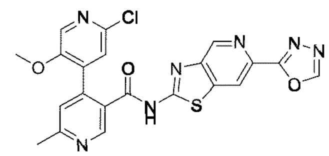,
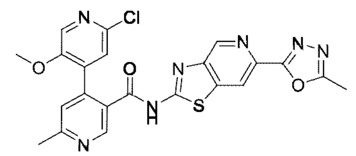,

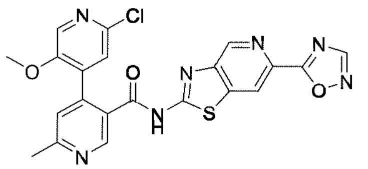,
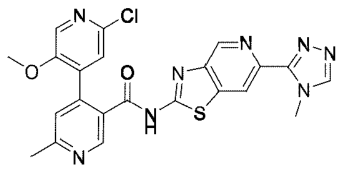,
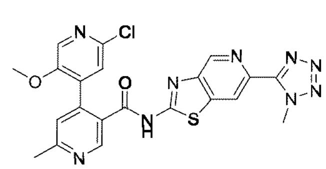,

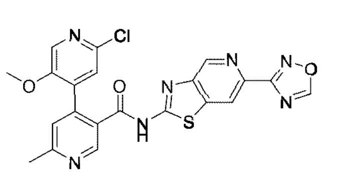,
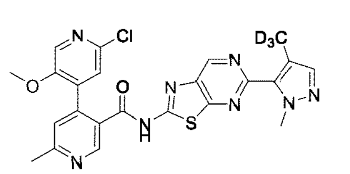,
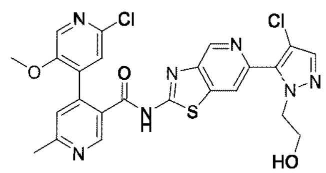,

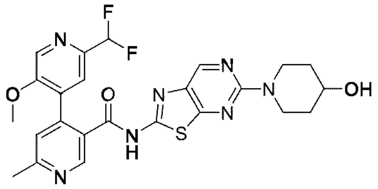,
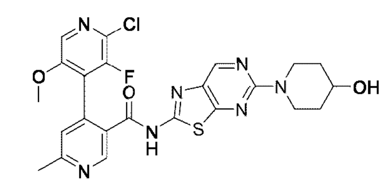,
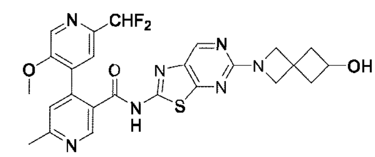,

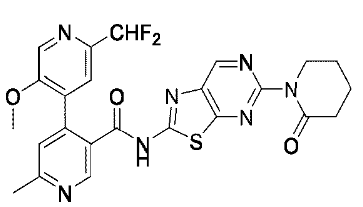,
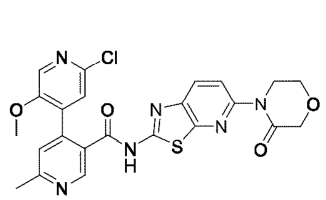,
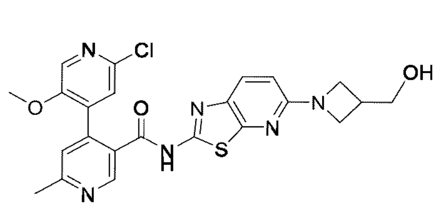,

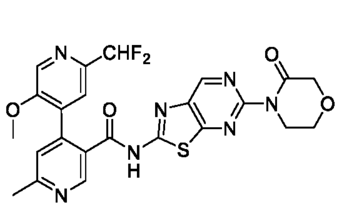,
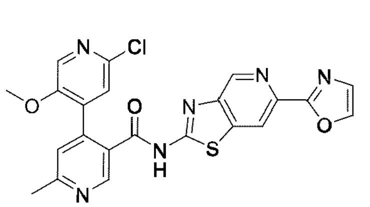,
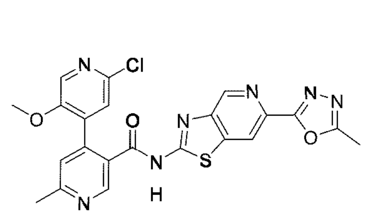,

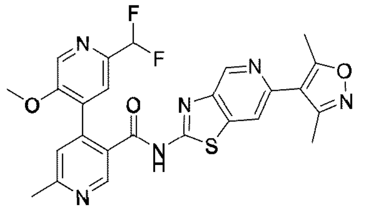,
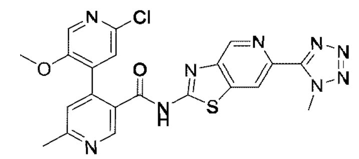,
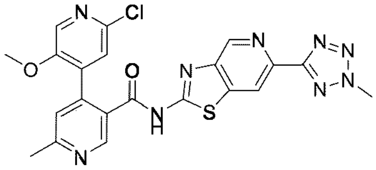,

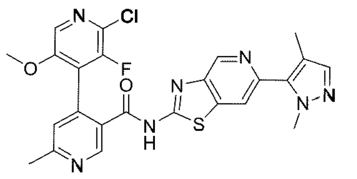,
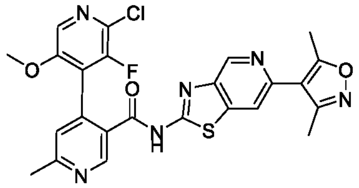,
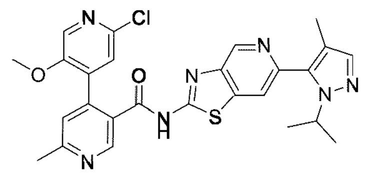,

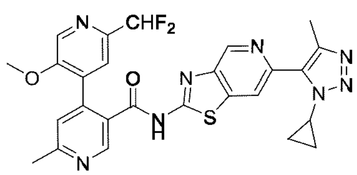,
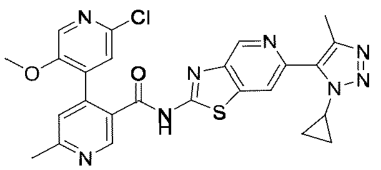,
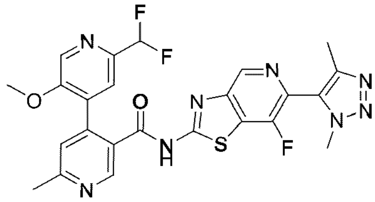,

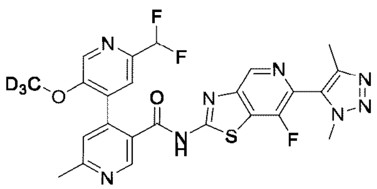,
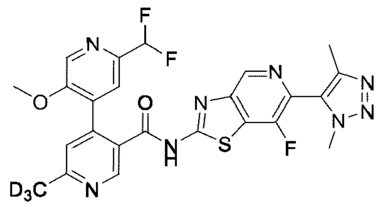,
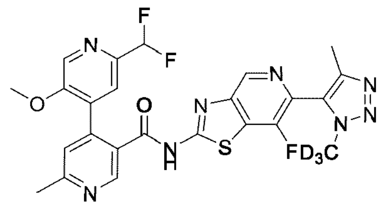,

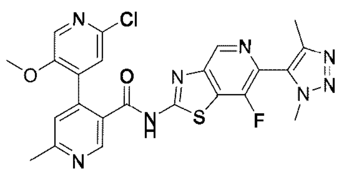,
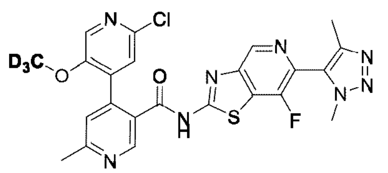,
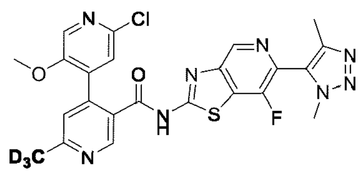,

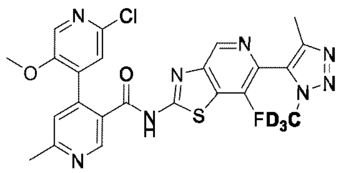,
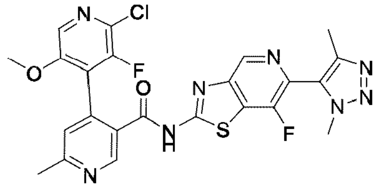,
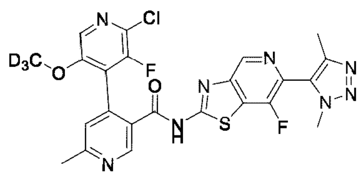,

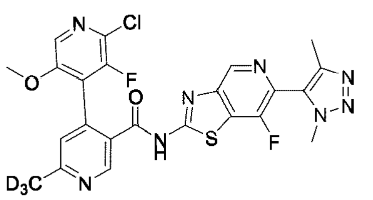,
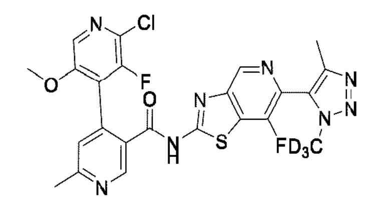,
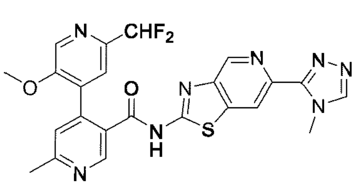,

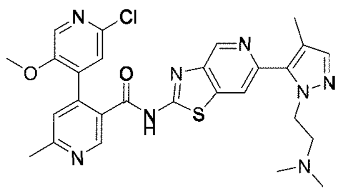, 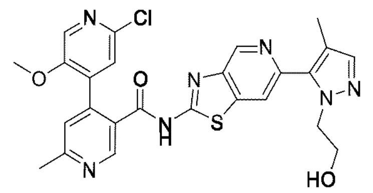, 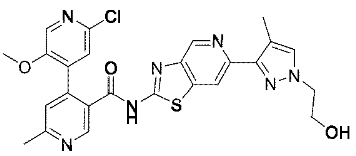,

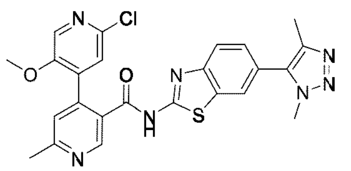, 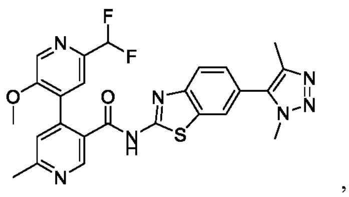, 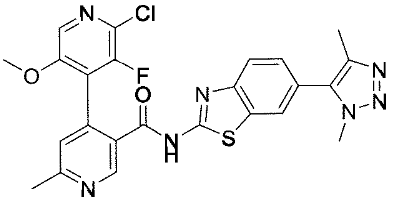,

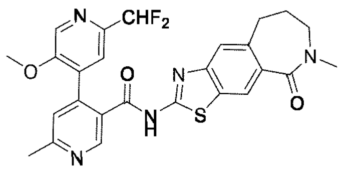, 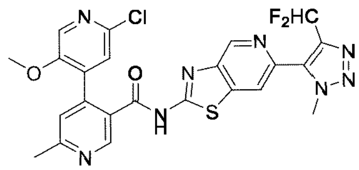, 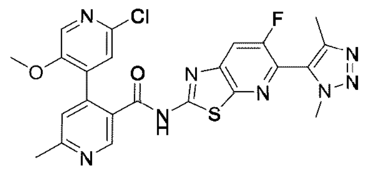,

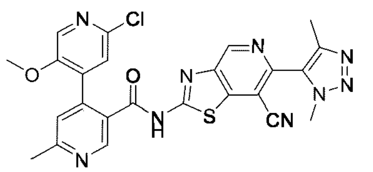, 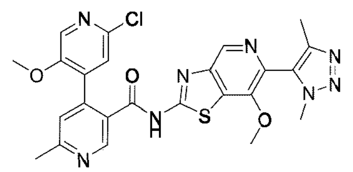, 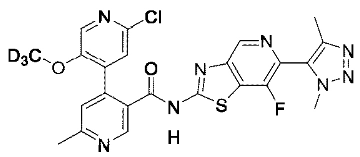,

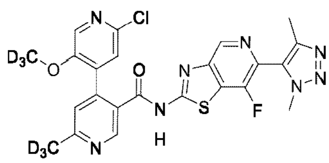, 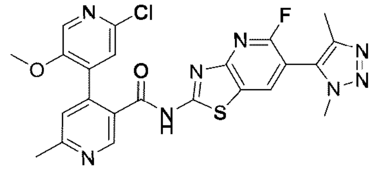 and 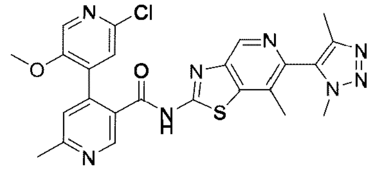.

**13.** A method for preparing a compound of formula I', comprising a scheme a, a scheme b or a scheme c;

the scheme a comprises the step of: subjecting a compound of formula II and a compound of formula III in a solvent to a condensation reaction shown below to obtain the compound of formula I',

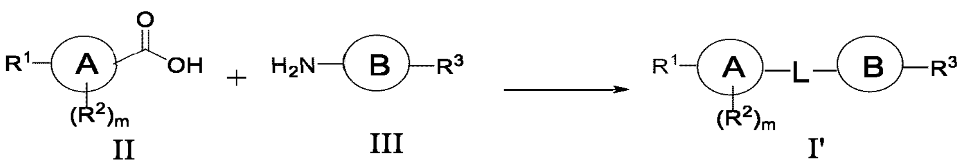

II III I' ;

the scheme b comprises the steps of: (a) subjecting a compound of formula II and a compound of formula III' in a solvent to a condensation reaction shown below to obtain a compound of formula I",

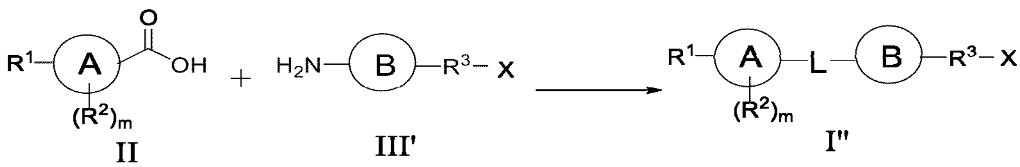

II III' I"

wherein X is tert-Butyldimethylsilyl ether group or tert-butoxycarbonyl, and
(b) subjecting the compound of formula I" prepared by step (a) in a solvent in the presence of an acid to a deprotection reaction shown below to obtain the compound of formula I';

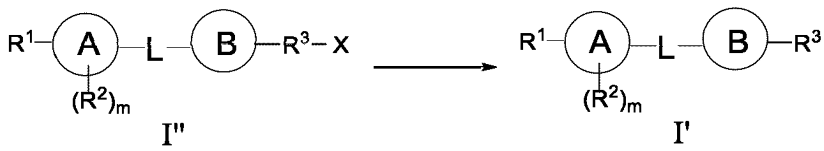

the scheme c comprises the steps of: subjecting a compound of formula IV and a compound of formula V in a solvent in the presence of a condensing agent to a condensation reaction, to botain the compound of formula I',

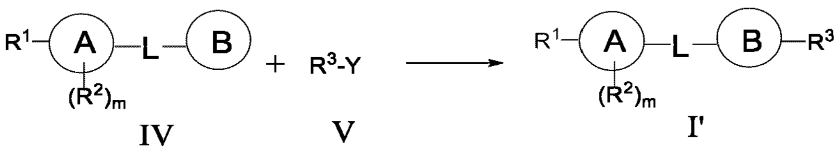

Y is hydrogen or carbonyl;
wherein $R^1$, $R^2$, m, a ring A, L, a ring B and $R^3$ are as defined in any one of claims 1-10;
preferably, the preparation method for a compound of formula I satisfies one or more of the following conditions:

(1) in the scheme a, in said condensation reaction, a molar ratio of said compound of formula II to said compound of formula III is 1:(0.5-1.5), for example, 1:1, 1:1.35 or 1:0.83;
(2) in the scheme b, in said condensation reaction, a molar ratio of said compound of formula II to said compound of formula III is 1:(0.5-1.5), for example, 1:1 or 0.67;
(3) in the scheme a or b, in said condensation reaction, the solvent is an amide solvent and/or a cyanogen solvent; the amide solvent is preferably N,N-dimethyl formamide; and the cyanogen solvent is preferably acetonitrile;
(4) in the scheme a or b, in said condensation reaction, the condensing agent is N,N,N',N'-tetramethylchlorformamidine hexafluorphosphate and N-methylimidazole;
(5) in the scheme a or b, in said condensation reaction, a molar ratio of said compound of formula II to the condensing agent is 1:(2-5), for example, 1:4 or 1:4.3, 1:3.7 or 1:4.1;
(6) in the scheme a or b, in said condensation reaction, a reaction temperature for the condensation reaction is room temperature;
(7) in the scheme a or b, the condensation reaction may further comprise post-treatment, which comprises the steps of: one or more of concentrating, washing with water, extracting, drying, column chromatography, and preparative chromatography;
(8) in the scheme c, in said condensation reaction, when Y is carbonyl, a molar ratio of said compound of formula IV to said compound of formula V is 1:(0.3-0.6), for example, 1:0.46;
(9) in the scheme c, in said condensation reaction, when Y is carbonyl, the solvent is an alcohol solvent, for example, methanol;
(10) in the scheme c, in said condensation reaction, when Y is carbonyl, the condensing agent is sodium cyanoborohydride and tetraethyl titanate;
(11) in the scheme c, in said condensation reaction, when Y is carbonyl, a molar ratio of said compound of formula IV to the condensing agent is 1:(2-5), for example, 1:3.2;
(12) in the scheme c, in said condensation reaction, when Y is carbonyl, a reaction temperature for the condensation reaction is room temperature;
(13) in the scheme c, in said condensation reaction, when Y is carbonyl, the condensation reaction further comprises post-treatment, which comprises the steps of: one or more of filtering, concentrating and preparative chromatography;
(14) in the scheme c, in said condensation reaction, when Y is hydrogen, a molar ratio of said compound of formula IV to said compound of formula V is 1:(1-1.5), for example, 1:1.49;
(15) in the scheme c, in said condensation reaction, when Y is hydrogen, the solvent is an amide solvent and/or a cyanogen solvent; the amide solvent is preferably N,N-dimethyl formamide; and the cyanogen solvent is preferably acetonitrile;
(16) in the scheme c, in said condensation reaction, when Y is hydrogen, the condensing agent is N,N,N',N'-tetramethylchlorformamidine hexafluorphosphate and N-methylimidazole;
(17) in the scheme c, in said condensation reaction, when Y is hydrogen, a molar ratio of said compound of formula IV to the condensing agent is 1:(2-5), for example, 1:4.2;
(18) in the scheme c, in said condensation reaction, when Y is hydrogen, a reaction temperature for the condensation reaction is room temperature;

and (19) in the scheme c, in said condensation reaction, when Y is hydrogen, the condensation reaction further comprises post-treatment, which comprises the steps of: one or more of extracting, drying, concentrating and preparative chromatography.

**14.** A pharmaceutical composition, comprising:

(1) a (therapeutically effective amount of) substance A, wherein said substance A is the compound, the pharmaceutically acceptable salt thereof or the isotopic compound thereof according to any one of claims 1-12, and
(2) a pharmaceutically acceptable excipient.

**15.** Use of a substance A in preparation of a polymerase theta inhibitor, wherein said substance A is the compound, the pharmaceutically acceptable salt thereof or the isotopic compound thereof according to any one of claims 1-12.

**16.** Use of a substance A in preparation of a medicament, wherein said substance A is the compound, the pharmaceutically acceptable salt thereof or the isotopic compound thereof according to any one of claims 1-12; and the medicament is for use in treatment of lung cancer, breast cancer, HR-deficient ovarian cancer, gastric cancer, prostate cancer, pancreatic cancer, or colon cancer.

**17.** A compound of any one of the following:

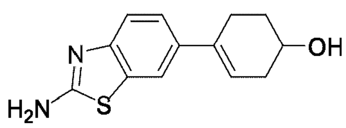 , 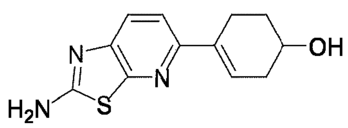 , 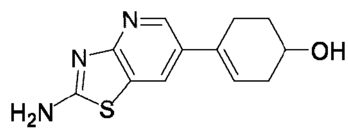 ,

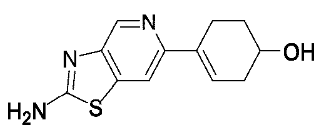 , 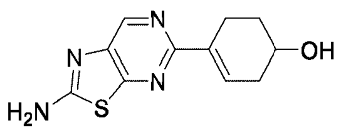 , 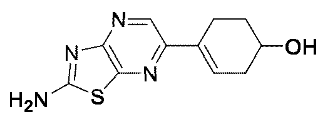 ,

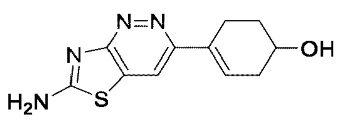 , 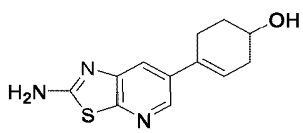 , 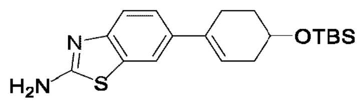 ,

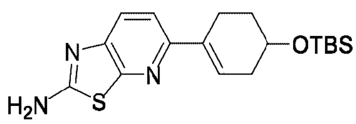 , 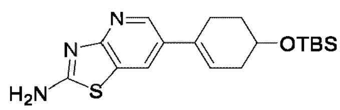 , 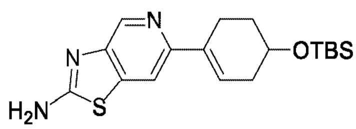 ,

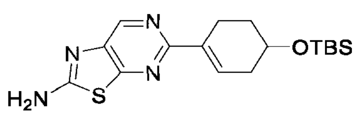 , 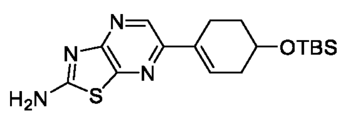 , 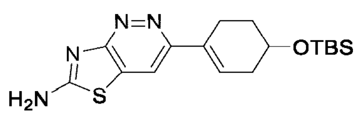 ,

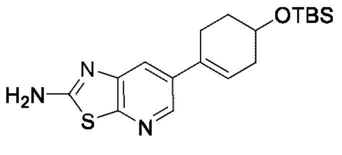 , 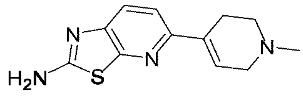 , 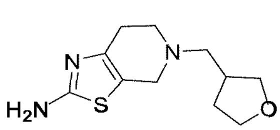 ,

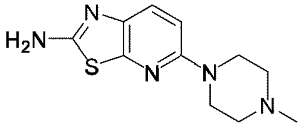 , 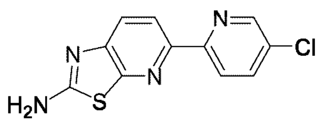 , 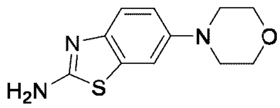 ,

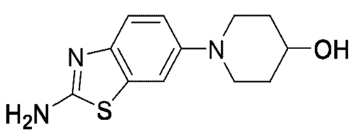 , 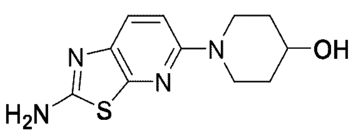 , 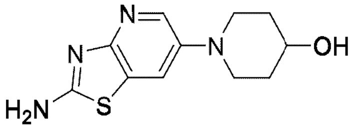 ,

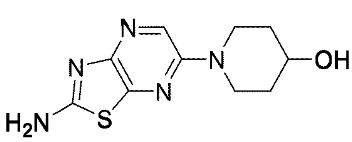 , 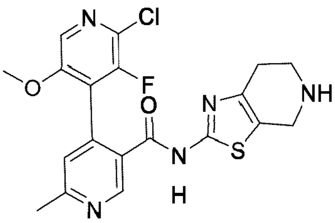 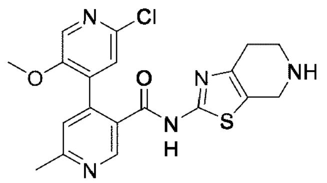 ,

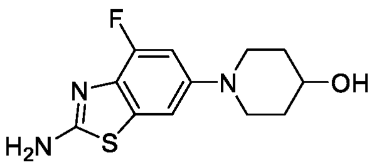 , 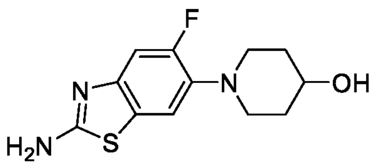 , 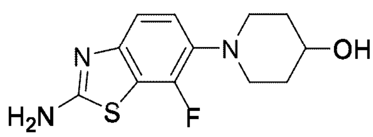 ,

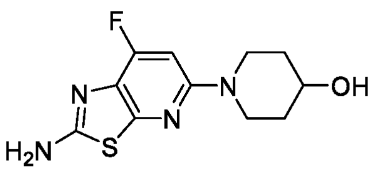 , 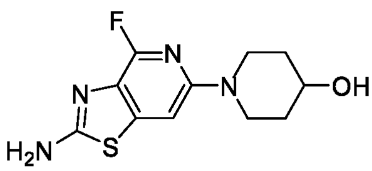 , 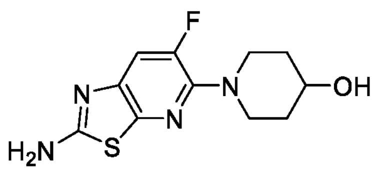 ,

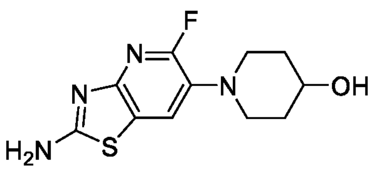 , 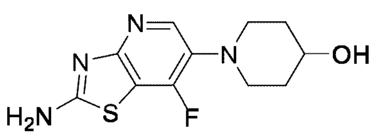 , 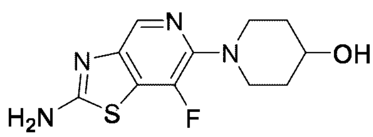 ,

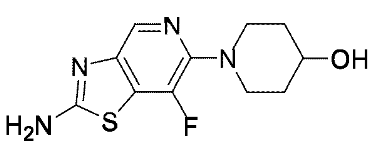 , 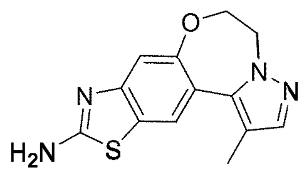 , 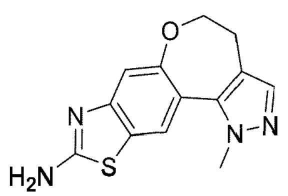 ,

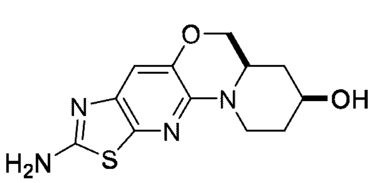 , 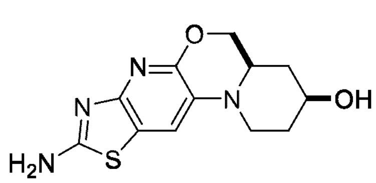 , 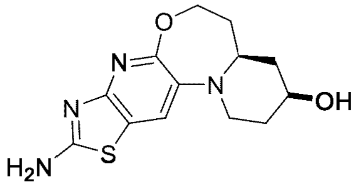 ,

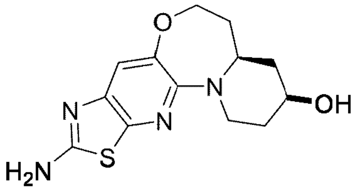 , 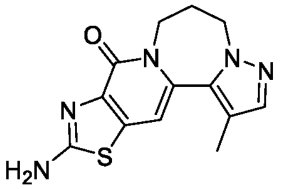 , 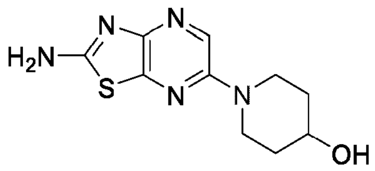 ,

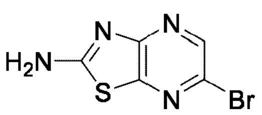 , 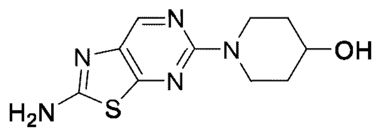 , 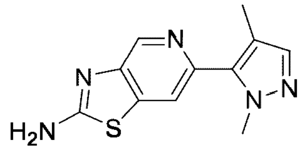 ,

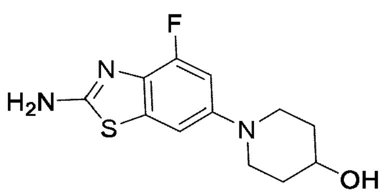 . , 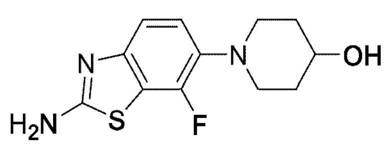 ,

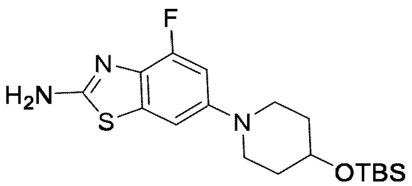 , 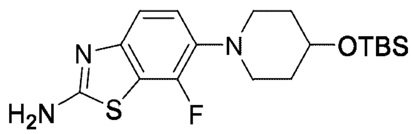 ,

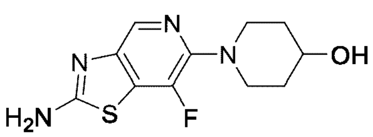 , 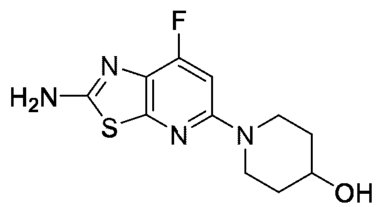 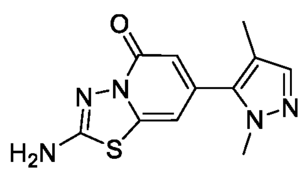 ,

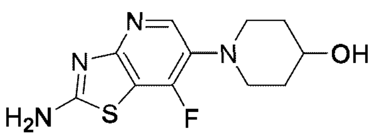 , 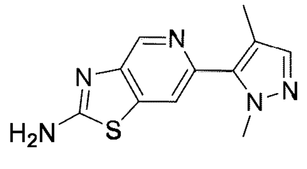 , 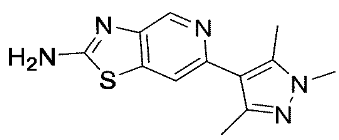 ,

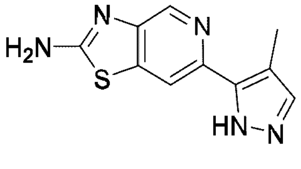 , 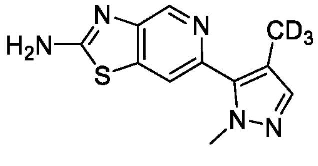 , 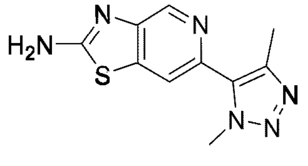 ,

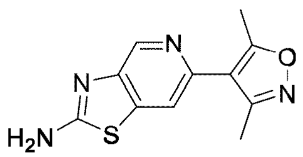 , 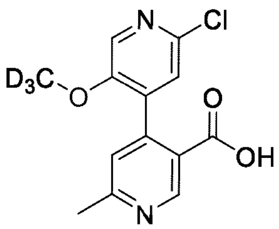 , 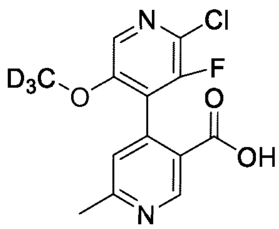 , 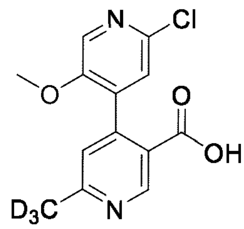 ,

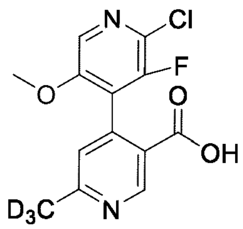 , 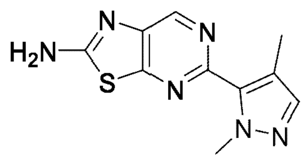 , 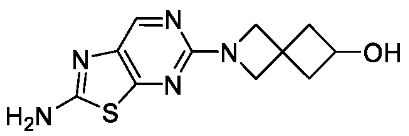 ,

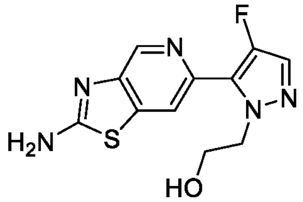 , 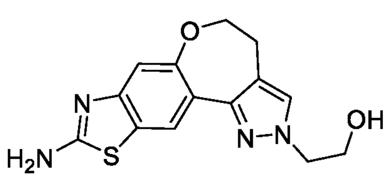 , 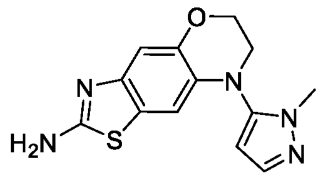 ,

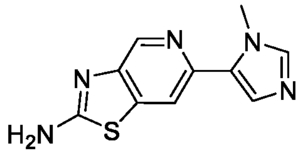 , 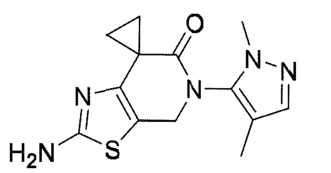 , 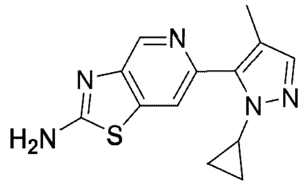 ,

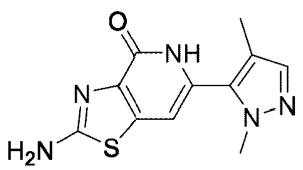 , 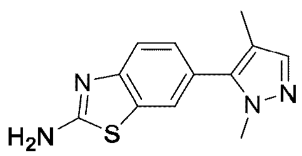 , 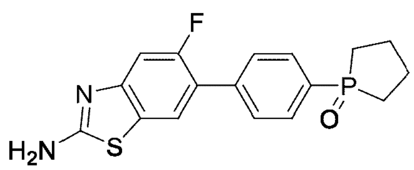 ,

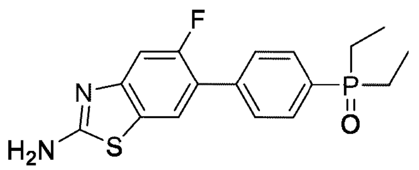 , 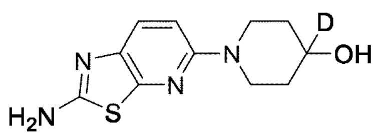 ,

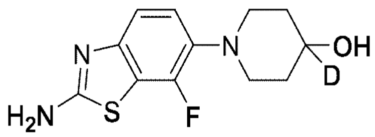 , 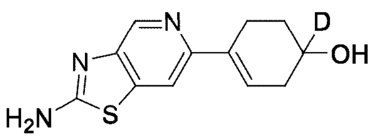 , 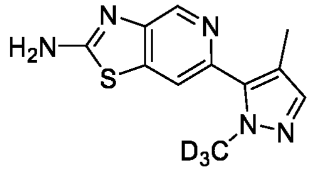 ,

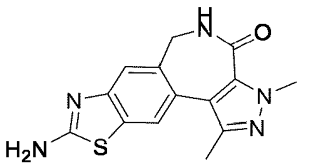 , 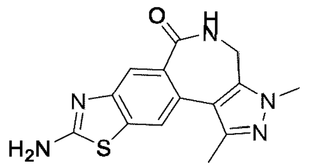 , 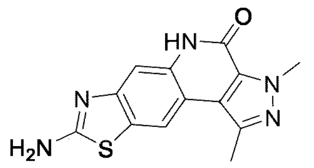 ,

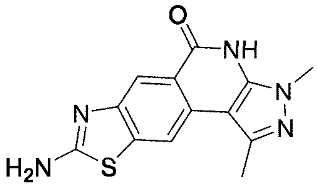 , 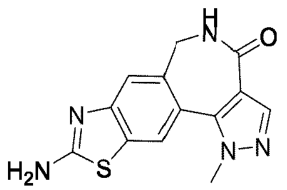 , 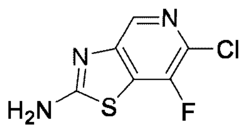 , 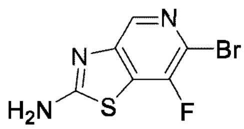 ,

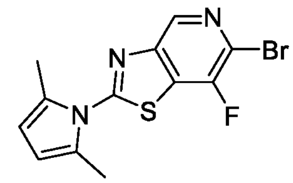 , 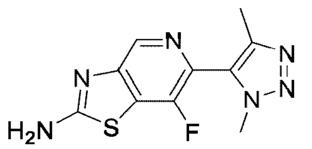 , 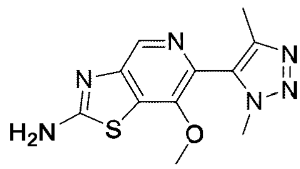 ,

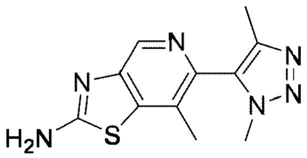 or 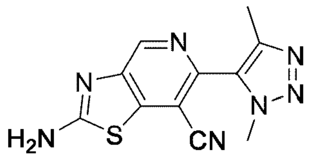 .

**18.** A compound of formula VI,

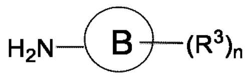

VI

,

wherein ring B is 5-20 membered fused heteroaromatic ring unsubstituted or substituted by one or more $R^{b-1}$; the heteroatom in said 5-20 membered fused heteroaromatic ring is selected from the group consisting of N, O, and S, the number of heteroatom(s) is 1, 2, 3, 4, 5, 6, 7 or 8, a single heteroaromatic ring in said 5-20 membered fused heteroaromatic ring is a 5-7 membered heteroaromatic ring, and the number of rings in said 5-20 membered fused heteroaromatic ring is 3 or 4; and $R^3$ and n are as defined in any one of claims 1-11.

## INTERNATIONAL SEARCH REPORT

International application No.
**PCT/CN2023/127292**

### A. CLASSIFICATION OF SUBJECT MATTER

C07D513/04(2006.01)i； C07D513/14(2006.01)i； C07D417/14(2006.01)i； C07D417/04(2006.01)i； C07D401/04(2006.01)i； A61K31/429(2006.01)i； A61K31/428(2006.01)i； A61P35/00(2006.01)i

According to International Patent Classification (IPC) or to both national classification and IPC

### B. FIELDS SEARCHED

Minimum documentation searched (classification system followed by classification symbols)

IPC:C07D,A61K,A61P

Documentation searched other than minimum documentation to the extent that such documents are included in the fields searched

Electronic data base consulted during the international search (name of data base and, where practicable, search terms used)

DWPI, CNTXT, STN(REGISTRY, CAPLUS): 杭州圣域, 聚合酶, 噻唑, 苯并, 吡啶并, 肿瘤, 癌症, Pol, theta, POLQ, ATPase, thiazol+, benzo, pyrido, tumor, cancer; STN中结构式检索, structural formula search in STN

### C. DOCUMENTS CONSIDERED TO BE RELEVANT

| Category* | Citation of document, with indication, where appropriate, of the relevant passages | Relevant to claim No. |
|---|---|---|
| PX | WO 2023134708 A1 (BEIGENE, LTD. et al.) 20 July 2023 (2023-07-20)<br>claims 1-29, and description, pages 38-39, embodiments 1-177 | 1-18 |
| PX | WO 2023134739 A1 (NANJING ZAIMING PHARMACEUTICAL CO., LTD.) 20 July 2023 (2023-07-20)<br>claims 1-22, and description, page 13, embodiments 1-94 | 1-18 |
| PY | WO 2022259204 A1 (IDEAYA BIOSCIENCES, INC. et al.) 15 December 2022 (2022-12-15)<br>claims 1-67, description, embodiments 1-490, and pages 89-98 | 1-18 |
| Y | WO 2022118210 A1 (IDEAYA BIOSCIENCES, INC. et al.) 09 June 2022 (2022-06-09)<br>claims 1-77, and description, embodiments 1-271, and pages 85-89 | 1-18 |
| Y | WO 2020243459 A1 (IDEAYA BIOSCIENCES, INC.) 03 December 2020 (2020-12-03)<br>claims 1-72, and description, embodiments 1-232, and pages 83-84 | 1-18 |
| Y | WO 2020160213 A1 (IDEAYA BIOSCIENCES, INC.) 06 August 2020 (2020-08-06)<br>claims 1-22, and description, embodiments 1-27, and paragraphs [0020] and [0055] | 1-18 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **18 January 2024** | **25 January 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

INTERNATIONAL SEARCH REPORT

International application No.
**PCT/CN2023/127292**

**C. DOCUMENTS CONSIDERED TO BE RELEVANT**

| Category* | Citation of document, with indication, where appropriate, of the relevant passages | Relevant to claim No. |
|---|---|---|
| A | WO 2012111016 A1 (COUNCIL OF SCIENTIC & INDUSTRIAL RESEARCH et al.) 23 August 2012 (2012-08-23)<br>entire document | 1-18 |
| A | WO 2021242955 A1 (SENDA BIOSCIENCES, INC.) 02 December 2021 (2021-12-02)<br>entire document | 1-18 |

Form PCT/ISA/210 (second sheet) (July 2022)

INTERNATIONAL SEARCH REPORT

International application No.
**PCT/CN2023/127292**

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☑ Claims Nos.: **1-12, 17-18**
because they relate to parts of the international application that do not comply with the prescribed requirements to such an extent that no meaningful international search can be carried out, specifically:

The compounds involved in claims 1-12 cover a very broad range, and it is difficult for the examiner to carry out an exhaustive search of the prior art with regard to the current scope of the claims. The present search report is provided on the basis that the compounds in claims 1-12 are limited to the compound as shown in formula Ib in claim 9. The search for intermediates of claims 17-18 is also performed on the basis of an intermediate corresponding to the compound as shown in formula Ib.

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2023/127292**

| Patent document cited in search report | Publication date (day/month/year) | Patent family member(s) | Publication date (day/month/year) |
|---|---|---|---|
| WO 2023134708 A1 | 20 July 2023 | TW 202329955 A | 01 August 2023 |
| WO 2023134739 A1 | 20 July 2023 | TW 202334169 A | 01 September 2023 |
| WO 2022259204 A1 | 15 December 2022 | TW 202315618 A | 16 April 2023 |
| | | AU 2022289762 A1 | 14 December 2023 |
| | | CA 3222078 A1 | 15 December 2022 |
| WO 2022118210 A1 | 09 June 2022 | CA 3200006 A1 | 09 June 2022 |
| | | TW 202237595 A | 01 October 2022 |
| | | JP 2023552764 A | 19 December 2023 |
| | | EP 4255573 A1 | 11 October 2023 |
| | | AU 2021390194 A1 | 22 June 2023 |
| WO 2020243459 A1 | 03 December 2020 | TW 202110834 A | 16 March 2021 |
| | | CA 3141134 A1 | 03 December 2020 |
| | | US 2023078112 A1 | 16 March 2023 |
| | | JP 2022535227 A | 05 August 2022 |
| | | AU 2020282768 A1 | 23 December 2021 |
| | | AU 2020282768 B2 | 14 September 2023 |
| | | EP 3976608 A1 | 06 April 2022 |
| | | EP 3976608 C0 | 08 November 2023 |
| | | BR 112021024101 A2 | 08 February 2022 |
| WO 2020160213 A1 | 06 August 2020 | EP 3917919 A1 | 08 December 2021 |
| | | AU 2020215042 A1 | 19 August 2021 |
| | | CA 3127642 A1 | 06 August 2020 |
| | | US 2021387968 A1 | 16 December 2021 |
| | | JP 2022519535 A | 24 March 2022 |
| WO 2012111016 A1 | 23 August 2012 | None | |
| WO 2021242955 A1 | 02 December 2021 | US 2023234967 A1 | 27 July 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 2022113390715
- CN 2023100413978
- CN 2023100486339
- CN 2023101385833
- CN 2023106346553
- CN 2023108473638
- CN 2023113518111
- WO 2023134739 A
- WO 2023134708 A


### Non-patent literature cited in the description


- **P. HEINRICH STAHL** ; **CAMILLE G**. Handbook of Pharmaceutical Salts: Properties, Selection, and Use. Wermuth, 2011